# EUROPEAN PATENT APPLICATION

(11) **EP 2 514 766 A2**
(43) Date of publication of application: **24.10.2012**
(21) Application number: 12165562.5
(22) Date of filing: 27.03.2008
(51) Int. Cl.: C07K 16/28, C07K 16/30, C07K 16/40, A61K 39/395, A61P 35/00, G01N 33/53

(54) **Antibodies, methods and kits for diagnosing and treating melanoma**

(30) Priority: 29.03.2007 US 907343 P; 21.05.2007 US 924549 P
(62) Divisional of application: 08738150.5
(71) Applicant: Technion Research & Development Foundation Ltd., 32000 Haifa (IL)
(72) Inventor: Reiter, Yoram, 34987 Haifa (IL); Cohen, Cyril, 49379 Petach-Tikva (IL); Klechevsky, Eynav, 34817 Haifa (IL); Michaeli, Yael, 30500 Binyamina (IL); Haus-Cohen, Maya, 34750 Haifa (IL); Denkberg, Galit, 36001 Nofit (IL)
(74) Representative: Vossius & Partner

(57) **Abstract**

The present invention relates to a method of identifying if a subject is suitable for TCRL-based epitope directed therapy, comprising determining a level of epitope presentation on at least one cell of the subject using an antibody comprising an antigen recognition domain capable of binding to an MHC-I molecule being complexed with a peptide fragment of said antigen, wherein the antibody does not bind said MHC-I in the absence of said complexed peptide fragment of said antigen, and wherein the antibody does not bind said peptide fragment of said antigen in an absence of said MHC, wherein a level value higher than a predetermined threshold is indicative of an individual being suitable for TCRL-based epitope directed therapy. The present invention further relates to a method of identifying if a subject is suitable for CTL-based epitope directed therapy, comprising determining a level of epitope presentation on at least one cell of the subject using an antibody comprising an antigen recognition domain capable of binding to an MHC-I molecule being complexed with a peptide fragment of said antigen, wherein the antibody does not bind said MHC-I in the absence of said complexed peptide fragment of said antigen, and wherein the antibody does not bind said peptide fragment of said antigen in an absence of said MHC, wherein a level value lower than a predetermined threshold is indicative of an individual being suitable for CTL-based epitope directed therapy.

SEQUENCE LISTING

<110>  Technion Research & Development Foundation Ltd.
       Reiter, Yoram
       Cohen, Cyril
       Klechevsky, Eynav
       Michaeli, Yael
       Haus-Cohen, Maya
       Denkberg, Galit

<120>  ANTIBODIES, METHODS AND KITS FOR DIAGNOSING AND TREATING MELANOMA

<130>  U1305 EP/1

<150>  US 60/907,343
<151>  2007-03-29

<150>  US 60/924,549
<151>  2007-05-21

<160>  910

<170>  PatentIn version 3.5

<210>  1
<211>  9
<212>  PRT
<213>  Artificial sequence

<220>
<223>  Tyrosinase D 369-377 derived peptide

<400>  1

Tyr Met Asp Gly Thr Met Ser Gln Val
1               5


<210>  2
<211>  9
<212>  PRT
<213>  Artificial sequence

<220>
<223>  Gag derived peptide

<400>  2

Ser Leu Tyr Asn Thr Val Ala Thr Leu
1               5


<210>  3
<211>  9
<212>  PRT
<213>  Artificial sequence

<220>
<223>  Tyr N derived peptide

<400>  3

Tyr Met Asn Gly Thr Met Ser Gln Val
1               5

<210>  4
<211>  9
<212>  PRT
<213>  Artificial sequence

<220>
<223>  gp100 209-217 derived peptide (G9-209 2M)

<400>  4

Ile Met Asp Gln Val Pro Phe Ser Val
1               5


<210>  5
<211>  9
<212>  PRT
<213>  Artificial sequence

<220>
<223>  gp100 280-288 derived peptide (G9-280)

<400>  5

Tyr Leu Glu Pro Gly Pro Val Thr Ala
1               5


<210>  6
<211>  9
<212>  PRT
<213>  Artificial sequence

<220>
<223>  hTERT 540-548 derived peptide

<400>  6

Ile Leu Ala Lys Phe Leu His Trp Leu
1               5


<210>  7
<211>  9
<212>  PRT
<213>  Artificial sequence

<220>
<223>  TARP 29-37 peptide

<400>  7

Phe Leu Arg Asn Phe Ser Leu Met Leu
1               5


<210>  8
<211>  21
<212>  DNA
<213>  Artificial sequence

<220>
<223>  Single strand DNA oligonucleotide

<400>  8
gtgccctgac cctacaagat g                                                21


<210>  9
<211>  21
<212>  DNA
<213>  Artificial sequence

<220>
<223>  Single strand DNA oligonucleotide

<400>  9
cataagcagg tggagcattg g                                                21


<210>  10
<211>  20
<212>  DNA
<213>  Artificial sequence

<220>
<223>  Single strand DNA oligonucleotide

<400>  10
tattgaaagt gccgagatcc                                                  20


<210>  11
<211>  19
<212>  DNA
<213>  Artificial sequence

<220>
<223>  Single strand DNA oligonucleotide

<400>  11
tgcaaggacc acagccatc                                                   19


<210>  12
<211>  24
<212>  DNA
<213>  Artificial sequence

<220>
<223>  Single strand DNA oligonucleotide

<400>  12
atggaacgcc cgagggacct ttac                                             24


<210>  13
<211>  24
<212>  DNA
<213>  Artificial sequence

<220>
<223>  Single strand DNA oligonucleotide

<400>  13
tgagaggcat ccgctatccc agta                                             24


<210>  14

<211>  20
<212>  DNA
<213>  Artificial sequence

<220>
<223>  Single strand DNA oligonucleotide

<400>  14
tattgggcgc ctggtcacca                                                    20


<210>  15
<211>  20
<212>  DNA
<213>  Artificial sequence

<220>
<223>  Single strand DNA oligonucleotide

<400>  15
ccaccttctt gatgtcatca                                                    20


<210>  16
<211>  660
<212>  DNA
<213>  Artificial sequence

<220>
<223>  TA2-VLCL coding sequence

<400>  16
atggatgttg tgatgactca gtctccactc tccctgcccg tcacccctgg agagccggcc        60

tccatctcct gcaggtctag tcagagcctc ctgcatagta atggacacaa ctatttggat       120

tggtacctgc agaagccagg gcagtctcca cagctcctga tctatttggg ttctaatcgg       180

gcctccgggg tccctgacag gttcagtggc agtggatcag gcacagattt tacactgaaa       240

atcagcagag tggaggctga ggatgttggg gtttattact gcatgcaaac tctacaaact       300

cctctcactt tcggccctgg gaccaaagtg gatatcaaac gaactgtggc tgcaccatct       360

gtcttcatct tcccgccatc tgatgagcag ttgaaatctg gaactgcctc tgttgtgtgc       420

ctgctgaata acttctatcc cagagaggcc aaagtacagt ggaaggtgga taacgccctc       480

caatcgggta actcccagga gagtgtcaca gagcaggaca gcaaggacag cacctacagc       540

ctcagcagca ccctgacgct gagcaaagca gactacgaga aacacaaact ctacgcctgc       600

gaagtcaccc atcagggcct gagctcgccc gtcacaaaga gcttcaacag gggagagtgt       660


<210>  17
<211>  219
<212>  PRT
<213>  Artificial sequence

<220>
<223>  TA2-VLCL(Human kappa subgroup II)


<220>

<221>  misc_feature
<222>  (1)..(112)
<223>  VL

<220>
<221>  misc_feature
<222>  (113)..(219)
<223>  CL

<400>  17

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly His Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Thr
                85                  90                  95

Leu Gln Thr Pro Leu Thr Phe Gly Pro Gly Thr Lys Val Asp Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Leu Tyr Ala Cys Glu Val Thr His Gln Gly Leu Ser Ser
        195                 200                 205

Pro Val Thr Lys Ser Phe Asn Arg Gly Glu Cys
    210                 215

<210>  18
<211>  690
<212>  DNA
<213>  Artificial sequence

<220>
<223>  TA2-VH CH coding sequence

<400>  18
atggaggtcc agctggtgga gaccggggga ggcgtggtcc agcctgggag gtccctgaga      60

ctctcctgtg cagcctctgg attcaccttc agtagctatg gcatgcactg ggtccgccag      120

gctccaggca aggggctgga gtgggtggca gttatatcat atgatggaag taataaatac      180

tatgcagact ccgtgaaggg ccgattcacc atctccagag acaattccaa gaacacgctg      240

tatctgcaaa tgaacagcct gagagctgag gacacggctg tgtattactg tgcgaaagat      300

cgttatggct ggggatcgtc ctttgggcac tttgactact ggggccaggg aaccctggtc      360

accgtctcaa gcgcctccac caagggccca tcggtcttcc ccctggcacc ctcctccaag      420

agcacctctg ggggcacagc ggccctgggc tgcctggtca aggactactt ccccgaaccg      480

gtgacggtgt cgtggaactc aggcgccctg accagcggcg tccacacctt cccggctgtc      540

ctacagtcct caggactcta ctccctcagc agcgtagtga ccgtgccctc cagcagcttg      600

ggcacccaga cctacatctg caacgtgaat cacaagccca gcaacaccaa ggtggacaag      660

aaagttgagc ccaaatcttg tgcggccgca                                      690


<210>  19
<211>  229
<212>  PRT
<213>  Artificial sequence

<220>
<223>  TA2-VH CH


<220>
<221>  misc_feature
<222>  (1)..(123)
<223>  VH

<220>
<221>  misc_feature
<222>  (124)..(229)
<223>  CH

<400>  19

Glu Val Gln Leu Val Glu Thr Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Tyr Gly Trp Gly Ser Ser Phe Gly His Phe Asp Tyr
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Ala Ala Ala
225

<210>  20
<211>  9
<212>  PRT
<213>  Artificial sequence

<220>
<223>  gp100 154-162 peptide

<400>  20

Lys Thr Trp Gly Gln Tyr Trp Gln Val
1               5

<210>  21
<211>  10
<212>  PRT
<213>  Artificial sequence

<220>
<223>  MART1 26-35  peptide

<400>  21

Glu Ala Ala Gly Ile Gly Ile Leu Thr Val
1               5                   10


<210>  22
<211>  9
<212>  PRT
<213>  Artificial sequence

<220>
<223>  MART1 27-35  peptide

<400>  22

Ala Ala Gly Ile Gly Ile Leu Thr Val
1               5


<210>  23
<211>  15
<212>  PRT
<213>  Artificial sequence

<220>
<223>  Flexible peptide linker

<400>  23

Gly Gly Gly Gly Ser Gly Gly Gly Gly Ser Gly Gly Gly Gly Ser
1               5                   10                  15


<210>  24
<211>  9
<212>  PRT
<213>  Artificial sequence

<220>
<223>  EBV BMLF-1 280-288 derived peptide

<400>  24

Gly Leu Cys Thr Leu Val Ala Met Leu
1               5


<210>  25
<211>  9
<212>  PRT
<213>  Artificial sequence

<220>
<223>  CMV P65 495-503 derived peptide

<400>  25

Asn Leu Val Pro Met Val Ala Thr Val
1               5

<210>  26
<211>  9
<212>  PRT
<213>  Artificial sequence

<220>
<223>  HTLV-1 TAX1 1-19 derived peptide

<400>  26

Leu Leu Phe Gly Tyr Pro Val Tyr Val
1               5

<210>  27
<211>  10
<212>  PRT
<213>  Artificial sequence

<220>
<223>  Mart-1 27L peptide

<400>  27

Glu Leu Ala Gly Ile Gly Ile Leu Thr Val
1               5                   10

<210>  28
<211>  9
<212>  PRT
<213>  Artificial sequence

<220>
<223>  gp100 280M peptide

<400>  28

Tyr Leu Glu Pro Gly Pro Val Thr Val
1               5

<210>  29
<211>  9
<212>  PRT
<213>  Artificial sequence

<220>
<223>  hTERT865-873 derived peptide

<400>  29

Arg Leu Val Asp Asp Phe Leu Leu Val
1               5

<210>  30
<211>  10
<212>  PRT

<213>  Artificial sequence

<220>
<223>  XAGE-1 peptide

<400>  30

Gly Val Phe Pro Ser Ala Pro Ser Pro Val
1               5                   10


<210>  31
<211>  216
<212>  PRT
<213>  Artificial sequence

<220>
<223>  CLA12-VLCL (Human lambda subgruop I)


<220>
<221>  misc_feature
<222>  (1)..(111)
<223>  VL

<220>
<221>  misc_feature
<222>  (112)..(216)
<223>  CL

<400>  31

Gln Ser Val Val Thr Gln Pro Pro Ser Val Ser Ala Ala Pro Gly Arg
1               5                   10                  15

Lys Val Thr Ile Ser Cys Ser Gly Ser Ser Ser Asn Ile Gly Ser Asn
            20                  25                  30

Tyr Val Ser Trp Tyr Gln Gln Val Pro Gly Thr Ala Pro Lys Leu Leu
        35                  40                  45

Ile Tyr Glu Asp Asp Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Phe Cys Ala Thr Trp Asp Arg Thr Val
                85                  90                  95

Asn Val Val Arg Phe Gly Gly Gly Thr Arg Leu Thr Val His Ser Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr

130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  32
<211>  221
<212>  PRT
<213>  Artificial sequence

<220>
<223>  CLA12-VHCH (subgroup II)

<220>
<221>  misc_feature
<222>  (1)..(118)
<223>  VH

<220>
<221>  misc_feature
<222>  (119)..(221)
<223>  CH

<400>  32

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ser
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr

85                  90                  95

Cys Ala Arg His Val Gly His Glu Leu Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys
    210                 215                 220

<210>  33
<211>  217
<212>  PRT
<213>  Artificial sequence

<220>
<223>  CAG10- VLCL (Human lambda subgroup I)

<220>
<221>  misc_feature
<222>  (1)..(112)
<223>  VL

<220>
<221>  misc_feature
<222>  (113)..(217)
<223>  CL

<400>  33

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Asp Val His Trp Tyr Gln Gln Leu Pro Gly Thr Ala Pro Lys Leu

35                  40                  45

Leu Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Asn Ser
                85                  90                  95

Leu Ser Ser Trp Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  34
<211>  222
<212>  PRT
<213>  Artificial sequence

<220>
<223>  CAG10-VHCH (subgroup  I)

<220>
<221>  misc_feature
<222>  (1)..(120)
<223>  VH

<220>
<221>  misc_feature
<222>  (121)..(222)
<223>  CH

<400>  34

Glu Val Gln Leu Gln Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Arg Ile Ile Pro Ile Leu Gly Ile Ala Asn Tyr Ala Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Val Gly Ser Gly Ser Tyr Ser Leu Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Ser Cys
    210                 215                 220

<210>  35
<211>  648
<212>  DNA
<213>  Artificial sequence

<220>

<223>  CLA12-VLCL (Human lambda subgroup I)  coding sequence

<400>  35
cagtctgtcg tgacgcagcc gccctcagtg tctgcggccc caggacggaa ggtcaccatc      60

tcctgctctg gaagcagctc caacattggg agtaattatg tgtcctggta ccagcaagtc     120

ccagggacag cccccaaact cctcatctat gaagatgata agcgaccctc agggattcct     180

gaccgattct ctggctccaa gtctgggacg tccgccaccc tgggcatcac cggactacag     240

actggggacg aggccgatta tttctgcgcc acgtgggata ggactgtcaa tgttgtacgt     300

ttcggcggag ggaccaggct gaccgtccac agtcagccca aggctgcccc ctcggtcact     360

ctgttcccgc cctcctctga ggagcttcaa gccaacaagg ccacactggt gtgtctcata     420

agtgacttct acccgggagc cgtgacagtg gcctggaagg cagatggcag ccccgtcaag     480

gcgggagtgg agaccaccaa accctccaaa cagagcaaca acaagtacgc ggccagcagc     540

tacctgagcc tgacgcccga gcagtggaag tcccacagaa gctacagctg ccaggtcacg     600

catgaaggga gcaccgtgga gaagacagtg gcccctacag aatgttca                  648

<210>  36
<211>  663
<212>  DNA
<213>  Artificial sequence

<220>
<223>  CLA12-VHCH (Human lambda subgroup I)  coding sequence

<400>  36
cagctgcagc tgcaggagtc gggcccagga ctggtgaagc cttcggagac cctgtccctc      60

acctgcactg tctctggtgg ctccatcagc agtagtagtt actactgggg ctggatccgc     120

cagcccccag ggaaggggct ggagtggatt gggagtatct attatagtgg gagcacctac     180

tacaacccgt ccctcaagag tcgagtcacc atatccgtag acacgtccaa gaaccagttc     240

tccctgaagc tgagctctgt gaccgccgca gacacggctg tgtattactg tgcgagacat     300

gtgggccacg aacttgacta ctggggccag ggaaccctgg tcaccgtctc aagcgcctcc     360

accaagggcc catcggtctt ccccctggca ccctcctcca agagcacctc tgggggcaca     420

gcggccctgg gctgcctggt caaggactac ttccccgaac cggtgacggt gtcgtggaac     480

tcaggcgccc tgaccagcgg cgtccacacc ttcccggctg tcctacagtc ctcaggactc     540

tactccctca gcagcgtagt gaccgtgccc tccagcagct tgggcaccca gacctacatc     600

tgcaacgtga atcacaagcc cagcaacacc aaggtggaca agaaagttga gcccaaatct     660

tgt                                                                   663

<210>  37
<211>  672
<212>  DNA
<213>  Artificial sequence

<220>
<223>  CAG10- VLCL (Human lambda subgroup I) coding sequence

<400>  37
gaaggagata tacatatggc acagtctgtg ttgacgcagc cgccctcagt gtctggggcc      60

ccagggcaga gggtcaccat ctcctgcact gggagcagct ccaacatcgg ggcaggttat     120

gatgtacact ggtaccagca gcttccggga acagccccca aactcctcat ctatggtaac     180

agcaatcggc cctcaggggt ccctgaccga ttctctggct ccaagtctgg cacctcagcc     240

tccctggcca tcactgggct ccaggctgag gatgaggctg attattactg ccagtcctat     300

gacaacagcc tgagtagttg ggtgttcggc ggagggacca agctgaccgt cctaggtcag     360

cccaaggctg ccccctcggt cactctgttc ccgccctcct ctgaggagct tcaagccaac     420

aaggccacac tggtgtgtct cataagtgac ttctacccgg gagccgtgac agtggcctgg     480

aaggcagata gcagccccgt caaggcggga gtggagacca ccacaccctc caaacaaagc     540

aacaacaagt acgcggccag cagctatctg agcctgacgc ctgagcagtg gaagtcccac     600

agaagctaca gctgccaggt cacgcatgaa gggagcaccg tggagaagac agtggcccct     660

acagaatgtt ca                                                         672

<210>  38
<211>  660
<212>  DNA
<213>  Artificial sequence

<220>
<223>  CAG10-VHCH (subgroup  I) coding sequence

<400>  38
gaggtacagc tgcagcagtc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggaagg atcatcccta tccttggtat agcaaactac     180

gcacagaagt tccagggcag agtcacgatt accgcggaca aatccacgag cacagcctac     240

atggagctga gcagcctgag atctgaggac acggccgtgt attactgtgc gagagatgtt     300

gggtcgggga gttattccct tgactactgg ggccagggaa ccctggtcac cgtctcaagc     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtc cacaccttcc cggctgtcct acagtcctca     540

ggactctact ccctcagcag cgtagtgacc gtgccctcca gcagcttggg cacccagacc     600

tacatctgca acgtgaatca caagcccagc aacaccaagg tggacaagaa agttgagccc     660

<210>  39
<211>  744
<212>  DNA
<213>  Artificial sequence

<220>
<223>  CAG10- VLCL-BirA Tag coding sequence

<400>  39
gaaggagata tacatatggc acagtctgtg ttgacgcagc cgccctcagt gtctggggcc      60

ccagggcaga gggtcaccat ctcctgcact gggagcagct ccaacatcgg ggcaggttat     120

gatgtacact ggtaccagca gcttccggga acagccccca aactcctcat ctatggtaac     180

agcaatcggc cctcaggggt ccctgaccga ttctctggct ccaagtctgg cacctcagcc     240

tccctggcca tcactgggct ccaggctgag gatgaggctg attattactg ccagtcctat     300

gacaacagcc tgagtagttg ggtgttcggc ggagggacca agctgaccgt cctaggtcag     360

cccaaggctg ccccctcggt cactctgttc ccgccctcct ctgaggagct tcaagccaac     420

aaggccacac tggtgtgtct cataagtgac ttctacccgg gagccgtgac agtggcctgg     480

aaggcagata gcagccccgt caaggcggga gtggagacca ccacaccctc caaacaaagc     540

aacaacaagt acgcggccag cagctatctg agcctgacgc ctgagcagtg gaagtcccac     600

agaagctaca gctgccaggt cacgcatgaa gggagcaccg tggagaagac agtggcccct     660

acagaatgtt cacccggatc cctgcaccat atcctggacg cccagaagat ggtgtggaat     720

caccgctaat gaattcggct gcta                                            744

<210>  40
<211>  242
<212>  PRT
<213>  Artificial sequence

<220>
<223>  CAG10- VLCL-BirA Tag


<220>
<221>  misc_feature
<222>  (1)..(224)
<223>  VL-CL

<220>
<221>  misc_feature
<222>  (225)..(242)
<223>  BirA tag

<400>  40

Glu Gly Asp Ile His Met Ala Gln Ser Val Leu Thr Gln Pro Pro Ser
1               5                   10                  15


Val Ser Gly Ala Pro Gly Gln Arg Val Thr Ile Ser Cys Thr Gly Ser
            20                  25                  30


Ser Ser Asn Ile Gly Ala Gly Tyr Asp Val His Trp Tyr Gln Gln Leu
        35                  40                  45

Pro Gly Thr Ala Pro Lys Leu Leu Ile Tyr Gly Asn Ser Asn Arg Pro
    50                  55                  60

Ser Gly Val Pro Asp Arg Phe Ser Gly Ser Lys Ser Gly Thr Ser Ala
65                  70                  75                  80

Ser Leu Ala Ile Thr Gly Leu Gln Ala Glu Asp Glu Ala Asp Tyr Tyr
                85                  90                  95

Cys Gln Ser Tyr Asp Asn Ser Leu Ser Ser Trp Val Phe Gly Gly Gly
            100                 105                 110

Thr Lys Leu Thr Val Leu Gly Gln Pro Lys Ala Ala Pro Ser Val Thr
        115                 120                 125

Leu Phe Pro Pro Ser Ser Glu Glu Leu Gln Ala Asn Lys Ala Thr Leu
    130                 135                 140

Val Cys Leu Ile Ser Asp Phe Tyr Pro Gly Ala Val Thr Val Ala Trp
145                 150                 155                 160

Lys Ala Asp Ser Ser Pro Val Lys Ala Gly Val Glu Thr Thr Thr Pro
                165                 170                 175

Ser Lys Gln Ser Asn Asn Lys Tyr Ala Ala Ser Ser Tyr Leu Ser Leu
            180                 185                 190

Thr Pro Glu Gln Trp Lys Ser His Arg Ser Tyr Ser Cys Gln Val Thr
        195                 200                 205

His Glu Gly Ser Thr Val Glu Lys Thr Val Ala Pro Thr Glu Cys Ser
    210                 215                 220

Pro Gly Ser Leu His His Ile Leu Asp Ala Gln Lys Met Val Trp Asn
225                 230                 235                 240

His Arg

<210>  41
<211>  768
<212>  DNA
<213>  Artificial sequence

<220>
<223>  CLA12-VLCL-BirA Tag

<400>  41
caacggtttc cctctagaaa taattttgtt taactttaag aaggagatat acatatggca      60

cagtctgtcg tgacgcagcc gccctcagtg tctgcggccc caggacggaa ggtcaccatc     120

tcctgctctg gaagcagctc caacattggg agtaattatg tgtcctggta ccagcaagtc 180

ccagggacag cccccaaact cctcatctat gaagatgata agcgaccctc agggattcct 240

gaccgattct ctggctccaa gtctgggacg tccgccaccc tgggcatcac cggactacag 300

actggggacg aggccgatta tttctgcgcc acgtgggata ggactgtcaa tgttgtacgt 360

ttcggcggag ggaccaggct gaccgtccac agtcagccca aggctgcccc ctcggtcact 420

ctgttcccgc cctcctctga ggagcttcaa gccaacaagg ccacactggt gtgtctcata 480

agtgacttct acccgggagc cgtgacagtg gcctggaagg cagatggcag ccccgtcaag 540

gcgggagtgg agaccaccaa accctccaaa cagagcaaca acaagtacgc ggccagcagc 600

tacctgagcc tgacgcccga gcagtggaag tcccacagaa gctacagctg ccaggtcacg 660

catgaaggga gcaccgtgga gaagacagtg gcccctacag aatgttcacc cggatccctg 720

caccatatcc tggacgccca gaagatggtg tggaatcacc gctaatga 768

<210> 42
<211> 236
<212> PRT
<213> Artificial sequence

<220>
<223> CLA12-VLCL-BirA Tag


<220>
<221> misc_feature
<222> (2)..(2)
<223> Addition of Ala

<220>
<221> misc_feature
<222> (3)..(109)
<223> VL

<220>
<221> misc_feature
<222> (110)..(218)
<223> CL

<220>
<221> misc_feature
<222> (219)..(221)
<223> Linker

<220>
<221> misc_feature
<222> (222)..(236)
<223> BirA tag

<400> 42

Met Ala Gln Ser Val Val Thr Gln Pro Pro Ser Val Ser Ala Ala Pro
1               5                   10                  15


Gly Arg Lys Val Thr Ile Ser Cys Ser Gly Ser Ser Ser Asn Ile Gly
            20                  25                  30

Ser Asn Tyr Val Ser Trp Tyr Gln Gln Val Pro Gly Thr Ala Pro Lys
        35                  40                  45

Leu Leu Ile Tyr Glu Asp Asp Lys Arg Pro Ser Gly Ile Pro Asp Arg
    50                  55                  60

Phe Ser Gly Ser Lys Ser Gly Thr Ser Ala Thr Leu Gly Ile Thr Gly
65                  70                  75                  80

Leu Gln Thr Gly Asp Glu Ala Asp Tyr Phe Cys Ala Thr Trp Asp Arg
                85                  90                  95

Thr Val Asn Val Val Arg Phe Gly Gly Gly Thr Arg Leu Thr Val His
            100                 105                 110

Ser Gln Pro Lys Ala Ala Pro Ser Val Thr Leu Phe Pro Pro Ser Ser
        115                 120                 125

Glu Glu Leu Gln Ala Asn Lys Ala Thr Leu Val Cys Leu Ile Ser Asp
    130                 135                 140

Phe Tyr Pro Gly Ala Val Thr Val Ala Trp Lys Ala Asp Gly Ser Pro
145                 150                 155                 160

Val Lys Ala Gly Val Glu Thr Thr Lys Pro Ser Lys Gln Ser Asn Asn
                165                 170                 175

Lys Tyr Ala Ala Ser Ser Tyr Leu Ser Leu Thr Pro Glu Gln Trp Lys
            180                 185                 190

Ser His Arg Ser Tyr Ser Cys Gln Val Thr His Glu Gly Ser Thr Val
        195                 200                 205

Glu Lys Thr Val Ala Pro Thr Glu Cys Ser Pro Gly Ser Leu His His
    210                 215                 220

Ile Leu Asp Ala Gln Lys Met Val Trp Asn His Arg
225                 230                 235

<210>  43
<211>  807
<212>  DNA
<213>  Artificial sequence

<220>
<223>  CLA12-VHCH-His MYC Tag coding sequence

<400>  43
caacggtttc cctctagaaa taattttgtt taactttaag aaggagatat acatatggcc        60

gagctgcagc tgcaggagtc gggcccagga ctggtgaagc cttcggagac cctgtccctc      120

acctgcactg tctctggtgg ctccatcagc agtagtagtt actactgggg ctggatccgc      180

cagcccccag ggaaggggct ggagtggatt gggagtatct attatagtgg gagcacctac      240

tacaacccgt ccctcaagag tcgagtcacc atatccgtag acacgtccaa gaaccagttc      300

tccctgaagc tgagctctgt gaccgccgca gacacggctg tgtattactg tgcgagacat      360

gtgggccacg aacttgacta ctggggccag ggaaccctgg tcaccgtctc aagcgcctcc      420

accaagggcc catcggtctt ccccctggca ccctcctcca agagcacctc tgggggcaca      480

gcggccctgg gctgcctggt caaggactac ttccccgaac cggtgacggt gtcgtggaac      540

tcaggcgccc tgaccagcgg cgtccacacc ttcccggctg tcctacagtc ctcaggactc      600

tactccctca gcagcgtagt gaccgtgccc tccagcagct tgggcaccca gacctacatc      660

tgcaacgtga atcacaagcc cagcaacacc aaggtggaca agaaagttga gcccaaatct      720

tgtgcggccg cacatcatca tcaccatcac ggggccgcag aacaaaaact catctcagaa      780

gaggatctga atggggccgc ataatga                                          807

<210>  44
<211>  249
<212>  PRT
<213>  Artificial sequence

<220>
<223>  -VHCH-His MYC Tag


<220>
<221>  misc_feature
<222>  (2)..(2)
<223>  Addition of A

<220>
<221>  misc_feature
<222>  (3)..(3)
<223>  E Changed from the original Q

<220>
<221>  misc_feature
<222>  (4)..(3)
<223>  vh

<220>
<221>  misc_feature
<222>  (4)..(115)
<223>  VH

<220>
<221>  misc_feature
<222>  (116)..(223)
<223>  CH

<220>
<221>  misc_feature
<222>  (227)..(232)
<223>  His tag

<220>
<221>  misc_feature
<222>  (233)..(249)
<223>  Myc tag

<400>  44

Met Ala Glu Leu Gln Leu Gln Glu Ser Gly Pro Gly Leu Val Lys Pro
1               5                   10                  15

Ser Glu Thr Leu Ser Leu Thr Cys Thr Val Ser Gly Gly Ser Ile Ser
            20                  25                  30

Ser Ser Ser Tyr Tyr Trp Gly Trp Ile Arg Gln Pro Pro Gly Lys Gly
        35                  40                  45

Leu Glu Trp Ile Gly Ser Ile Tyr Tyr Ser Gly Ser Thr Tyr Tyr Asn
    50                  55                  60

Pro Ser Leu Lys Ser Arg Val Thr Ile Ser Val Asp Thr Ser Lys Asn
65                  70                  75                  80

Gln Phe Ser Leu Lys Leu Ser Ser Val Thr Ala Ala Asp Thr Ala Val
                85                  90                  95

Tyr Tyr Cys Ala Arg His Val Gly His Glu Leu Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Ala
    210                 215                 220

Ala Ala His His His His His His Gly Ala Ala Glu Gln Lys Leu Ile

225                 230                 235                 240

Ser Glu Glu Asp Leu Asn Gly Ala Ala
                245

<210>  45
<211>  759
<212>  DNA
<213>  Artificial sequence

<220>
<223>  CAG10-VHCH-His MYC Tag coding sequence

<220>
<221>  misc_feature
<222>  (693)..(693)
<223>  n is a, c, g, or t

<400>  45
taactttaag aaggagatat acatatggcc gaggtacagc tgcagcagtc tggggctgag      60

gtgaagaagc ctgggtcctc ggtgaaggtc tcctgcaagg cttctggagg caccttcagc     120

agctatgcta tcagctgggt gcgacaggcc cctggacaag ggcttgagtg gatgggaagg     180

atcatcccta tccttggtat agcaaactac gcacagaagt tccagggcag agtcacgatt     240

accgcggaca aatccacgag cacagcctac atggagctga gcagcctgag atctgaggac     300

acggccgtgt attactgtgc gagagatgtt gggtcgggga gttattccct tgactactgg     360

ggccagggaa ccctggtcac cgtctcaagc gcctccacca agggcccatc ggtcttcccc     420

ctggcaccct cctccaagag cacctctggg ggcacagcgg ccctgggctg cctggtcaag     480

gactacttcc ccgaaccggt gacggtgtcg tggaactcag gcgccctgac cagcggcgtc     540

cacaccttcc cggctgtcct acagtcctca ggactctact ccctcagcag cgtagtgacc     600

gtgccctcca gcagcttggg cacccagacc tacatctgca acgtgaatca caagcccagc     660

aacaccaagg tggacaagaa agttgagccc aantcttgtg cggccgcaca tcatcatcac     720

catcacgggg ccgcagaaca aaaactcatc tcagaagag                            759

<210>  46
<211>  244
<212>  PRT
<213>  Artificial sequence

<220>
<223>  CAG10-VHCH-His MYC Tag

<220>
<221>  misc_feature
<222>  (1)..(221)
<223>  VH-CH

<220>
<221>  misc_feature

<222>  (228)..(233)
<223>  His tag

<220>
<221>  misc_feature
<222>  (234)..(244)
<223>  Myc tag

<400>  46

Met Ala Glu Val Gln Leu Gln Gln Ser Gly Ala Glu Val Lys Lys Pro
1               5                   10                  15

Gly Ser Ser Val Lys Val Ser Cys Lys Ala Ser Gly Gly Thr Phe Ser
            20                  25                  30

Ser Tyr Ala Ile Ser Trp Val Arg Gln Ala Pro Gly Gln Gly Leu Glu
        35                  40                  45

Trp Met Gly Arg Ile Ile Pro Ile Leu Gly Ile Ala Asn Tyr Ala Gln
    50                  55                  60

Lys Phe Gln Gly Arg Val Thr Ile Thr Ala Asp Lys Ser Thr Ser Thr
65                  70                  75                  80

Ala Tyr Met Glu Leu Ser Ser Leu Arg Ser Glu Asp Thr Ala Val Tyr
                85                  90                  95

Tyr Cys Ala Arg Asp Val Gly Ser Gly Ser Tyr Ser Leu Asp Tyr Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Ser Cys
    210                 215                 220

Ala Ala Ala His His His His His His Gly Ala Ala Glu Gln Lys Leu
225                 230                 235                 240

Ile Ser Glu Glu

<210>  47
<211>  13
<212>  PRT
<213>  Artificial sequence

<220>
<223>  CLA12-VLCL CDR

<400>  47

Ser Gly Ser Ser Ser Asn Ile Gly Ser Asn Tyr Val Ser
1               5                   10

<210>  48
<211>  7
<212>  PRT
<213>  Artificial sequence

<220>
<223>  CLA12-VLCL CDR

<400>  48

Glu Asp Asp Lys Arg Pro Ser
1               5

<210>  49
<211>  11
<212>  PRT
<213>  Artificial sequence

<220>
<223>  CLA12-VLCL CDR

<400>  49

Ala Thr Trp Asp Arg Thr Val Asn Val Val Arg
1               5                   10

<210>  50
<211>  7
<212>  PRT
<213>  Artificial sequence

<220>
<223>  CLA12-VHCH CDR

<400>  50

Ser Ser Ser Tyr Tyr Trp Gly
1               5

<210>  51
<211>  16
<212>  PRT
<213>  Artificial sequence

<220>
<223>  CLA12-VHCH CDR

<400>  51

Ser Ile Tyr Tyr Ser Gly Ser Thr Tyr Tyr Asn Pro Ser Leu Lys Ser
1               5                   10                  15


<210>  52
<211>  8
<212>  PRT
<213>  Artificial sequence

<220>
<223>  CLA12-VHCH CDR

<400>  52

His Val Gly His Glu Leu Asp Tyr
1               5


<210>  53
<211>  14
<212>  PRT
<213>  Artificial sequence

<220>
<223>  CAG10- VLCL CDR

<400>  53

Thr Gly Ser Ser Ser Asn Ile Gly Ala Gly Tyr Asp Val His
1               5                   10


<210>  54
<211>  7
<212>  PRT
<213>  Artificial sequence

<220>
<223>  CAG10- VLCL CDR

<400>  54

Gly Asn Ser Asn Arg Pro Ser
1               5


<210>  55
<211>  11
<212>  PRT
<213>  Artificial sequence

<220>
<223>  CAG10- VLCL CDR

<400>  55

Gln Ser Tyr Asp Asn Ser Leu Ser Ser Trp Val
1               5                   10

<210>  56
<211>  5
<212>  PRT
<213>  Artificial sequence

<220>
<223>  CAG10-VHCH CDR

<400>  56

Ser Tyr Ala Ile Ser
1               5

<210>  57
<211>  17
<212>  PRT
<213>  Artificial sequence

<220>
<223>  CAG10-VHCH CDR

<400>  57

Arg Ile Ile Pro Ile Leu Gly Ile Ala Asn Tyr Ala Gln Lys Phe Gln
1               5                   10                  15

Gly

<210>  58
<211>  11
<212>  PRT
<213>  Artificial sequence

<220>
<223>  CAG10-VHCH CDR

<400>  58

Asp Val Gly Ser Gly Ser Tyr Ser Leu Asp Tyr
1               5                   10

<210>  59
<211>  16
<212>  PRT
<213>  Artificial sequence

<220>
<223>  TA2-VLCL CDR

<400>  59

Arg Ser Ser Gln Ser Leu Leu His Ser Asn Gly His Asn Tyr Leu Asp
1               5                   10                  15

<210>  60
<211>  7
<212>  PRT
<213>  Artificial sequence

<220>
<223>  TA2-VLCL CDR

<400>  60

Leu Gly Ser Asn Arg Ala Ser
1               5


<210>  61
<211>  9
<212>  PRT
<213>  Artificial sequence

<220>
<223>  TA2-VLCL CDR

<400>  61

Met Gln Thr Leu Gln Thr Pro Leu Thr
1               5


<210>  62
<211>  5
<212>  PRT
<213>  Artificial sequence

<220>
<223>  TA2-VHCH CDR

<400>  62

Ser Tyr Gly Met His
1               5


<210>  63
<211>  17
<212>  PRT
<213>  Artificial sequence

<220>
<223>  TA2-VHCH CDR

<400>  63

Val Ile Ser Tyr Asp Gly Ser Asn Lys Tyr Tyr Ala Asp Ser Val Lys
1               5                   10                  15


Gly



<210>  64
<211>  14
<212>  PRT
<213>  Artificial sequence

<220>
<223>  TA2-VHCH CDR

<400>  64

Asp Arg Tyr Gly Trp Gly Ser Ser Phe Gly His Phe Asp Tyr
1               5                   10


<210>  65
<211>  8
<212>  PRT
<213>  Artificial sequence

<220>
<223>  Pol derived peptide

<400>  65

Ile Leu Glu Pro Val His Gly Val
1               5


<210>  66
<211>  54
<212>  DNA
<213>  Artificial sequence

<220>
<223>  Bir A tag expressing sequence

<400>  66
cccggatccc tgcaccatat cctggacgcc cagaagatgg tgtggaatca ccgc          54


<210>  67
<211>  529
<212>  PRT
<213>  Artificial sequence

<220>
<223>  Single strand DNA oligonucleotide

<400>  67

Met Leu Leu Ala Val Leu Tyr Cys Leu Leu Trp Ser Phe Gln Thr Ser
1               5                   10                  15


Ala Gly His Phe Pro Arg Ala Cys Val Ser Ser Lys Asn Leu Met Glu
            20                  25                  30


Lys Glu Cys Cys Pro Pro Trp Ser Gly Asp Arg Ser Pro Cys Gly Gln
        35                  40                  45


Leu Ser Gly Arg Gly Ser Cys Gln Asn Ile Leu Leu Ser Asn Ala Pro
    50                  55                  60


Leu Gly Pro Gln Phe Pro Phe Thr Gly Val Asp Asp Arg Glu Ser Trp
65                  70                  75                  80

Pro Ser Val Phe Tyr Asn Arg Thr Cys Gln Cys Ser Gly Asn Phe Met
                85                  90                  95
Gly Phe Asn Cys Gly Asn Cys Lys Phe Gly Phe Trp Gly Pro Asn Cys
            100                 105                 110
Thr Glu Arg Arg Leu Leu Val Arg Arg Asn Ile Phe Asp Leu Ser Ala
        115                 120                 125
Pro Glu Lys Asp Lys Phe Phe Ala Tyr Leu Thr Leu Ala Lys His Thr
    130                 135                 140
Ile Ser Ser Asp Tyr Val Ile Pro Ile Gly Thr Tyr Gly Gln Met Lys
145                 150                 155                 160
Asn Gly Ser Thr Pro Met Phe Asn Asp Ile Asn Ile Tyr Asp Leu Phe
                165                 170                 175
Val Trp Ile His Tyr Tyr Val Ser Met Asp Ala Leu Leu Gly Gly Tyr
            180                 185                 190
Glu Ile Trp Arg Asp Ile Asp Phe Ala His Glu Ala Pro Ala Phe Leu
        195                 200                 205
Pro Trp His Arg Leu Phe Leu Leu Arg Trp Glu Gln Glu Ile Gln Lys
    210                 215                 220
Leu Thr Gly Asp Glu Asn Phe Thr Ile Pro Tyr Trp Asp Trp Arg Asp
225                 230                 235                 240
Ala Glu Lys Cys Asp Ile Cys Thr Asp Glu Tyr Met Gly Gly Gln His
                245                 250                 255
Pro Thr Asn Pro Asn Leu Leu Ser Pro Ala Ser Phe Phe Ser Ser Trp
            260                 265                 270
Gln Ile Val Cys Ser Arg Leu Glu Glu Tyr Asn Ser His Gln Ser Leu
        275                 280                 285
Cys Asn Gly Thr Pro Glu Gly Pro Leu Arg Arg Asn Pro Gly Asn His
    290                 295                 300
Asp Lys Ser Arg Thr Pro Arg Leu Pro Ser Ser Ala Asp Val Glu Phe
305                 310                 315                 320
Cys Leu Ser Leu Thr Gln Tyr Glu Ser Gly Ser Met Asp Lys Ala Ala
                325                 330                 335
Asn Phe Ser Phe Arg Asn Thr Leu Glu Gly Phe Ala Ser Pro Leu Thr

340                 345                 350

Gly Ile Ala Asp Ala Ser Gln Ser Ser Met His Asn Ala Leu His Ile
        355                 360                 365

Tyr Met Asn Gly Thr Met Ser Gln Val Gln Gly Ser Ala Asn Asp Pro
    370                 375                 380

Ile Phe Leu Leu His His Ala Phe Val Asp Ser Ile Phe Glu Gln Trp
385                 390                 395                 400

Leu Arg Arg His Arg Pro Leu Gln Glu Val Tyr Pro Glu Ala Asn Ala
                405                 410                 415

Pro Ile Gly His Asn Arg Glu Ser Tyr Met Val Pro Phe Ile Pro Leu
            420                 425                 430

Tyr Arg Asn Gly Asp Phe Phe Ile Ser Ser Lys Asp Leu Gly Tyr Asp
        435                 440                 445

Tyr Ser Tyr Leu Gln Asp Ser Asp Pro Asp Ser Phe Gln Asp Tyr Ile
    450                 455                 460

Lys Ser Tyr Leu Glu Gln Ala Ser Arg Ile Trp Ser Trp Leu Leu Gly
465                 470                 475                 480

Ala Ala Met Val Gly Ala Val Leu Thr Ala Leu Leu Ala Gly Leu Val
                485                 490                 495

Ser Leu Leu Cys Arg His Lys Arg Lys Gln Leu Pro Glu Glu Lys Gln
            500                 505                 510

Pro Leu Leu Met Glu Lys Glu Asp Tyr His Ser Leu Tyr Gln Ser His
        515                 520                 525

Leu

<210>  68
<211>  529
<212>  PRT
<213>  Artificial sequence

<220>
<223>  Single strand DNA oligonucleotide

<220>
<221>  misc_feature
<223>  N371D mutant

<400>  68

Met Leu Leu Ala Val Leu Tyr Cys Leu Leu Trp Ser Phe Gln Thr Ser
1               5                   10                  15

Ala Gly His Phe Pro Arg Ala Cys Val Ser Ser Lys Asn Leu Met Glu
            20                  25                  30

Lys Glu Cys Cys Pro Pro Trp Ser Gly Asp Arg Ser Pro Cys Gly Gln
        35                  40                  45

Leu Ser Gly Arg Gly Ser Cys Gln Asn Ile Leu Leu Ser Asn Ala Pro
    50                  55                  60

Leu Gly Pro Gln Phe Pro Phe Thr Gly Val Asp Asp Arg Glu Ser Trp
65                  70                  75                  80

Pro Ser Val Phe Tyr Asn Arg Thr Cys Gln Cys Ser Gly Asn Phe Met
                85                  90                  95

Gly Phe Asn Cys Gly Asn Cys Lys Phe Gly Phe Trp Gly Pro Asn Cys
            100                 105                 110

Thr Glu Arg Arg Leu Leu Val Arg Arg Asn Ile Phe Asp Leu Ser Ala
        115                 120                 125

Pro Glu Lys Asp Lys Phe Phe Ala Tyr Leu Thr Leu Ala Lys His Thr
    130                 135                 140

Ile Ser Ser Asp Tyr Val Ile Pro Ile Gly Thr Tyr Gly Gln Met Lys
145                 150                 155                 160

Asn Gly Ser Thr Pro Met Phe Asn Asp Ile Asn Ile Tyr Asp Leu Phe
                165                 170                 175

Val Trp Ile His Tyr Tyr Val Ser Met Asp Ala Leu Leu Gly Gly Tyr
            180                 185                 190

Glu Ile Trp Arg Asp Ile Asp Phe Ala His Glu Ala Pro Ala Phe Leu
        195                 200                 205

Pro Trp His Arg Leu Phe Leu Leu Arg Trp Glu Gln Glu Ile Gln Lys
    210                 215                 220

Leu Thr Gly Asp Glu Asn Phe Thr Ile Pro Tyr Trp Asp Trp Arg Asp
225                 230                 235                 240

Ala Glu Lys Cys Asp Ile Cys Thr Asp Glu Tyr Met Gly Gly Gln His
                245                 250                 255

Pro Thr Asn Pro Asn Leu Leu Ser Pro Ala Ser Phe Phe Ser Ser Trp
            260                 265                 270

Gln Ile Val Cys Ser Arg Leu Glu Glu Tyr Asn Ser His Gln Ser Leu
        275                 280                 285

Cys Asn Gly Thr Pro Glu Gly Pro Leu Arg Arg Asn Pro Gly Asn His
    290                 295                 300

Asp Lys Ser Arg Thr Pro Arg Leu Pro Ser Ser Ala Asp Val Glu Phe
305                 310                 315                 320

Cys Leu Ser Leu Thr Gln Tyr Glu Ser Gly Ser Met Asp Lys Ala Ala
                325                 330                 335

Asn Phe Ser Phe Arg Asn Thr Leu Glu Gly Phe Ala Ser Pro Leu Thr
            340                 345                 350

Gly Ile Ala Asp Ala Ser Gln Ser Ser Met His Asn Ala Leu His Ile
        355                 360                 365

Tyr Met Asp Gly Thr Met Ser Gln Val Gln Gly Ser Ala Asn Asp Pro
    370                 375                 380

Ile Phe Leu Leu His His Ala Phe Val Asp Ser Ile Phe Glu Gln Trp
385                 390                 395                 400

Leu Arg Arg His Arg Pro Leu Gln Glu Val Tyr Pro Glu Ala Asn Ala
                405                 410                 415

Pro Ile Gly His Asn Arg Glu Ser Tyr Met Val Pro Phe Ile Pro Leu
            420                 425                 430

Tyr Arg Asn Gly Asp Phe Phe Ile Ser Ser Lys Asp Leu Gly Tyr Asp
        435                 440                 445

Tyr Ser Tyr Leu Gln Asp Ser Asp Pro Asp Ser Phe Gln Asp Tyr Ile
    450                 455                 460

Lys Ser Tyr Leu Glu Gln Ala Ser Arg Ile Trp Ser Trp Leu Leu Gly
465                 470                 475                 480

Ala Ala Met Val Gly Ala Val Leu Thr Ala Leu Leu Ala Gly Leu Val
                485                 490                 495

Ser Leu Leu Cys Arg His Lys Arg Lys Gln Leu Pro Glu Glu Lys Gln
            500                 505                 510

Pro Leu Leu Met Glu Lys Glu Asp Tyr His Ser Leu Tyr Gln Ser His

515                    520                    525

Leu

<210>  69
<211>  9
<212>  PRT
<213>  Artificial sequence

<220>
<223>  HLA restricted peptide

<400>  69

Ala Ala Met Val Gly Ala Val Leu Thr
1               5

<210>  70
<211>  10
<212>  PRT
<213>  Artificial sequence

<220>
<223>  HLA restricted peptide

<400>  70

Ala Ala Met Val Gly Ala Val Leu Thr Ala
1               5                   10

<210>  71
<211>  9
<212>  PRT
<213>  Artificial sequence

<220>
<223>  HLA restricted peptide

<400>  71

Ala Ala Asn Phe Ser Phe Arg Asn Thr
1               5

<210>  72
<211>  10
<212>  PRT
<213>  Artificial sequence

<220>
<223>  HLA restricted peptide

<400>  72

Ala Ala Asn Phe Ser Phe Arg Asn Thr Leu
1               5                   10

<210>  73
<211>  9

<212>  PRT
<213>  Artificial sequence

<220>
<223>  HLA restricted peptide

<400>  73

Ala Cys Val Ser Ser Lys Asn Leu Met
1               5


<210>  74
<211>  9
<212>  PRT
<213>  Artificial sequence

<220>
<223>  HLA restricted peptide

<400>  74

Ala Asp Ala Ser Gln Ser Ser Met His
1               5


<210>  75
<211>  10
<212>  PRT
<213>  Artificial sequence

<220>
<223>  HLA restricted peptide

<400>  75

Ala Asp Ala Ser Gln Ser Ser Met His Asn
1               5                   10


<210>  76
<211>  9
<212>  PRT
<213>  Artificial sequence

<220>
<223>  HLA restricted peptide

<400>  76

Ala Asp Val Glu Phe Cys Leu Ser Leu
1               5


<210>  77
<211>  10
<212>  PRT
<213>  Artificial sequence

<220>
<223>  HLA restricted peptide

<400>  77

Ala Asp Val Glu Phe Cys Leu Ser Leu Thr

1                   5                   10


<210>  78
<211>  10
<212>  PRT
<213>  Artificial sequence

<220>
<223>  HLA restricted peptide

<400>  78

Ala Glu Lys Cys Asp Ile Cys Thr Asp Glu
1               5                   10


<210>  79
<211>  9
<212>  PRT
<213>  Artificial sequence

<220>
<223>  HLA restricted peptide

<400>  79

Ala Phe Leu Pro Trp His Arg Leu Phe
1               5


<210>  80
<211>  10
<212>  PRT
<213>  Artificial sequence

<220>
<223>  HLA restricted peptide

<400>  80

Ala Phe Leu Pro Trp His Arg Leu Phe Leu
1               5                   10


<210>  81
<211>  9
<212>  PRT
<213>  Artificial sequence

<220>
<223>  HLA restricted peptide

<400>  81

Ala Phe Val Asp Ser Ile Phe Glu Gln
1               5


<210>  82
<211>  10
<212>  PRT
<213>  Artificial sequence

<220>

<223>  HLA restricted peptide

<400>  82

Ala Phe Val Asp Ser Ile Phe Glu Gln Trp
1               5                   10


<210>  83
<211>  9
<212>  PRT
<213>  Artificial sequence

<220>
<223>  HLA restricted peptide

<400>  83

Ala Gly His Phe Pro Arg Ala Cys Val
1               5


<210>  84
<211>  10
<212>  PRT
<213>  Artificial sequence

<220>
<223>  HLA restricted peptide

<400>  84

Ala Gly His Phe Pro Arg Ala Cys Val Ser
1               5                   10


<210>  85
<211>  9
<212>  PRT
<213>  Artificial sequence

<220>
<223>  HLA restricted peptide

<400>  85

Ala Gly Leu Val Ser Leu Leu Cys Arg
1               5


<210>  86
<211>  10
<212>  PRT
<213>  Artificial sequence

<220>
<223>  HLA restricted peptide

<400>  86

Ala Gly Leu Val Ser Leu Leu Cys Arg His
1               5                   10


<210>  87

<211>  10
<212>  PRT
<213>  Artificial sequence

<220>
<223>  HLA restricted peptide

<400>  87

Ala His Glu Ala Pro Ala Phe Leu Pro Trp
1               5                   10


<210>  88
<211>  9
<212>  PRT
<213>  Artificial sequence

<220>
<223>  HLA restricted peptide

<400>  88

Ala Lys His Thr Ile Ser Ser Asp Tyr
1               5


<210>  89
<211>  10
<212>  PRT
<213>  Artificial sequence

<220>
<223>  HLA restricted peptide

<400>  89

Ala Lys His Thr Ile Ser Ser Asp Tyr Val
1               5                   10


<210>  90
<211>  9
<212>  PRT
<213>  Artificial sequence

<220>
<223>  HLA restricted peptide

<400>  90

Ala Leu His Ile Tyr Met Asp Gly Thr
1               5


<210>  91
<211>  10
<212>  PRT
<213>  Artificial sequence

<220>
<223>  HLA restricted peptide

<400>  91

Ala Leu His Ile Tyr Met Asp Gly Thr Met
1               5                   10


<210>  92
<211>  9
<212>  PRT
<213>  Artificial sequence

<220>
<223>  HLA restricted peptide

<400>  92

Ala Leu His Ile Tyr Met Asn Gly Thr
1               5


<210>  93
<211>  10
<212>  PRT
<213>  Artificial sequence

<220>
<223>  HLA restricted peptide

<400>  93

Ala Leu His Ile Tyr Met Asn Gly Thr Met
1               5                   10


<210>  94
<211>  9
<212>  PRT
<213>  Artificial sequence

<220>
<223>  HLA restricted peptide

<400>  94

Ala Leu Leu Ala Gly Leu Val Ser Leu
1               5


<210>  95
<211>  10
<212>  PRT
<213>  Artificial sequence

<220>
<223>  HLA restricted peptide

<400>  95

Ala Leu Leu Ala Gly Leu Val Ser Leu Leu
1               5                   10


<210>  96
<211>  9
<212>  PRT
<213>  Artificial sequence

<220>
<223>  HLA restricted peptide

<400>  96

Ala Leu Leu Gly Gly Tyr Glu Ile Trp
1               5


<210>  97
<211>  10
<212>  PRT
<213>  Artificial sequence

<220>
<223>  HLA restricted peptide

<400>  97

Ala Leu Leu Gly Gly Tyr Glu Ile Trp Arg
1               5                   10


<210>  98
<211>  9
<212>  PRT
<213>  Artificial sequence

<220>
<223>  HLA restricted peptide

<400>  98

Ala Met Val Gly Ala Val Leu Thr Ala
1               5


<210>  99
<211>  10
<212>  PRT
<213>  Artificial sequence

<220>
<223>  HLA restricted peptide

<400>  99

Ala Met Val Gly Ala Val Leu Thr Ala Leu
1               5                   10


<210>  100
<211>  9
<212>  PRT
<213>  Artificial sequence

<220>
<223>  HLA restricted peptide

<400>  100

Ala Asn Ala Pro Ile Gly His Asn Arg
1               5

<210>  101
<211>  9
<212>  PRT
<213>  Artificial sequence

<220>
<223>  HLA restricted peptide

<400>  101

Ala Asn Asp Pro Ile Phe Leu Leu His
1               5


<210>  102
<211>  10
<212>  PRT
<213>  Artificial sequence

<220>
<223>  HLA restricted peptide

<400>  102

Ala Asn Asp Pro Ile Phe Leu Leu His His
1               5                   10


<210>  103
<211>  9
<212>  PRT
<213>  Artificial sequence

<220>
<223>  HLA restricted peptide

<400>  103

Ala Asn Phe Ser Phe Arg Asn Thr Leu
1               5


<210>  104
<211>  10
<212>  PRT
<213>  Artificial sequence

<220>
<223>  HLA restricted peptide

<400>  104

Ala Asn Phe Ser Phe Arg Asn Thr Leu Glu
1               5                   10


<210>  105
<211>  9
<212>  PRT
<213>  Artificial sequence

<220>
<223>  HLA restricted peptide

<400>  105

Ala Pro Ala Phe Leu Pro Trp His Arg
1               5

<210>  106
<211>  10
<212>  PRT
<213>  Artificial sequence

<220>
<223>  HLA restricted peptide

<400>  106

Ala Pro Ala Phe Leu Pro Trp His Arg Leu
1               5                   10

<210>  107
<211>  9
<212>  PRT
<213>  Artificial sequence

<220>
<223>  HLA restricted peptide

<400>  107

Ala Pro Glu Lys Asp Lys Phe Phe Ala
1               5

<210>  108
<211>  10
<212>  PRT
<213>  Artificial sequence

<220>
<223>  HLA restricted peptide

<400>  108

Ala Pro Glu Lys Asp Lys Phe Phe Ala Tyr
1               5                   10

<210>  109
<211>  9
<212>  PRT
<213>  Artificial sequence

<220>
<223>  HLA restricted peptide

<400>  109

Ala Pro Ile Gly His Asn Arg Glu Ser
1               5

<210>  110
<211>  10
<212>  PRT
<213>  Artificial sequence

<220>
<223>  HLA restricted peptide

<400>  110

Ala Pro Ile Gly His Asn Arg Glu Ser Tyr
1               5                   10


<210>  111
<211>  9
<212>  PRT
<213>  Artificial sequence

<220>
<223>  HLA restricted peptide

<400>  111

Ala Pro Leu Gly Pro Gln Phe Pro Phe
1               5


<210>  112
<211>  10
<212>  PRT
<213>  Artificial sequence

<220>
<223>  HLA restricted peptide

<400>  112

Ala Pro Leu Gly Pro Gln Phe Pro Phe Thr
1               5                   10


<210>  113
<211>  9
<212>  PRT
<213>  Artificial sequence

<220>
<223>  HLA restricted peptide

<400>  113

Ala Ser Phe Phe Ser Ser Trp Gln Ile
1               5


<210>  114
<211>  10
<212>  PRT
<213>  Artificial sequence

<220>
<223>  HLA restricted peptide

<400>  114

Ala Ser Phe Phe Ser Ser Trp Gln Ile Val
1               5                   10

<210>  115
<211>  10
<212>  PRT
<213>  Artificial sequence

<220>
<223>  HLA restricted peptide

<400>  115

Ala Ser Pro Leu Thr Gly Ile Ala Asp Ala
1               5                   10


<210>  116
<211>  9
<212>  PRT
<213>  Artificial sequence

<220>
<223>  HLA restricted peptide

<400>  116

Ala Ser Gln Ser Ser Met His Asn Ala
1               5


<210>  117
<211>  10
<212>  PRT
<213>  Artificial sequence

<220>
<223>  HLA restricted peptide

<400>  117

Ala Ser Gln Ser Ser Met His Asn Ala Leu
1               5                   10


<210>  118
<211>  9
<212>  PRT
<213>  Artificial sequence

<220>
<223>  HLA restricted peptide

<400>  118

Ala Ser Arg Ile Trp Ser Trp Leu Leu
1               5


<210>  119
<211>  10
<212>  PRT
<213>  Artificial sequence

<220>
<223>  HLA restricted peptide

<400>  119

Ala Val Leu Thr Ala Leu Leu Ala Gly Leu
1               5                   10


<210>  120
<211>  9
<212>  PRT
<213>  Artificial sequence

<220>
<223>  HLA restricted peptide

<400>  120

Ala Val Leu Tyr Cys Leu Leu Trp Ser
1               5


<210>  121
<211>  10
<212>  PRT
<213>  Artificial sequence

<220>
<223>  HLA restricted peptide

<400>  121

Ala Val Leu Tyr Cys Leu Leu Trp Ser Phe
1               5                   10


<210>  122
<211>  9
<212>  PRT
<213>  Artificial sequence

<220>
<223>  HLA restricted peptide

<400>  122

Ala Tyr Leu Thr Leu Ala Lys His Thr
1               5


<210>  123
<211>  10
<212>  PRT
<213>  Artificial sequence

<220>
<223>  HLA restricted peptide

<400>  123

Ala Tyr Leu Thr Leu Ala Lys His Thr Ile
1               5                   10


<210>  124
<211>  9
<212>  PRT

<213>  Artificial sequence

<220>
<223>  HLA restricted peptide

<400>  124

Cys Cys Pro Pro Trp Ser Gly Asp Arg
1               5


<210>  125
<211>  10
<212>  PRT
<213>  Artificial sequence

<220>
<223>  HLA restricted peptide

<400>  125

Cys Cys Pro Pro Trp Ser Gly Asp Arg Ser
1               5                   10


<210>  126
<211>  9
<212>  PRT
<213>  Artificial sequence

<220>
<223>  HLA restricted peptide

<400>  126

Cys Asp Ile Cys Thr Asp Glu Tyr Met
1               5


<210>  127
<211>  10
<212>  PRT
<213>  Artificial sequence

<220>
<223>  HLA restricted peptide

<400>  127

Cys Asp Ile Cys Thr Asp Glu Tyr Met Gly
1               5                   10


<210>  128
<211>  9
<212>  PRT
<213>  Artificial sequence

<220>
<223>  HLA restricted peptide

<400>  128

Cys Gly Asn Cys Lys Phe Gly Phe Trp
1               5

<210>  129
<211>  9
<212>  PRT
<213>  Artificial sequence

<220>
<223>  HLA restricted peptide

<400>  129

Cys Gly Gln Leu Ser Gly Arg Gly Ser
1               5


<210>  130
<211>  10
<212>  PRT
<213>  Artificial sequence

<220>
<223>  HLA restricted peptide

<400>  130

Cys Gly Gln Leu Ser Gly Arg Gly Ser Cys
1               5                   10


<210>  131
<211>  9
<212>  PRT
<213>  Artificial sequence

<220>
<223>  HLA restricted peptide

<400>  131

Cys Lys Phe Gly Phe Trp Gly Pro Asn
1               5


<210>  132
<211>  10
<212>  PRT
<213>  Artificial sequence

<220>
<223>  HLA restricted peptide

<400>  132

Cys Lys Phe Gly Phe Trp Gly Pro Asn Cys
1               5                   10


<210>  133
<211>  9
<212>  PRT
<213>  Artificial sequence

<220>
<223>  HLA restricted peptide

<400>  133

Cys Leu Leu Trp Ser Phe Gln Thr Ser
1               5

<210>  134
<211>  10
<212>  PRT
<213>  Artificial sequence

<220>
<223>  HLA restricted peptide

<400>  134

Cys Leu Leu Trp Ser Phe Gln Thr Ser Ala
1               5                   10

<210>  135
<211>  9
<212>  PRT
<213>  Artificial sequence

<220>
<223>  HLA restricted peptide

<400>  135

Cys Leu Ser Leu Thr Gln Tyr Glu Ser
1               5

<210>  136
<211>  9
<212>  PRT
<213>  Artificial sequence

<220>
<223>  HLA restricted peptide

<400>  136

Cys Asn Gly Thr Pro Glu Gly Pro Leu
1               5

<210>  137
<211>  10
<212>  PRT
<213>  Artificial sequence

<220>
<223>  HLA restricted peptide

<400>  137

Cys Asn Gly Thr Pro Glu Gly Pro Leu Arg
1               5                   10

<210>  138
<211>  9

<212>  PRT
<213>  Artificial sequence

<220>
<223>  HLA restricted peptide

<400>  138

Cys Pro Pro Trp Ser Gly Asp Arg Ser
1               5


<210>  139
<211>  10
<212>  PRT
<213>  Artificial sequence

<220>
<223>  HLA restricted peptide

<400>  139

Cys Pro Pro Trp Ser Gly Asp Arg Ser Pro
1               5                   10


<210>  140
<211>  9
<212>  PRT
<213>  Artificial sequence

<220>
<223>  HLA restricted peptide

<400>  140

Cys Gln Cys Ser Gly Asn Phe Met Gly
1               5


<210>  141
<211>  10
<212>  PRT
<213>  Artificial sequence

<220>
<223>  HLA restricted peptide

<400>  141

Cys Gln Cys Ser Gly Asn Phe Met Gly Phe
1               5                   10


<210>  142
<211>  9
<212>  PRT
<213>  Artificial sequence

<220>
<223>  HLA restricted peptide

<400>  142

Cys Gln Asn Ile Leu Leu Ser Asn Ala

1                5

<210>  143
<211>  10
<212>  PRT
<213>  Artificial sequence

<220>
<223>  HLA restricted peptide

<400>  143

Cys Gln Asn Ile Leu Leu Ser Asn Ala Pro
1               5                   10

<210>  144
<211>  9
<212>  PRT
<213>  Artificial sequence

<220>
<223>  HLA restricted peptide

<400>  144

Cys Arg His Lys Arg Lys Gln Leu Pro
1               5

<210>  145
<211>  10
<212>  PRT
<213>  Artificial sequence

<220>
<223>  HLA restricted peptide

<400>  145

Cys Arg His Lys Arg Lys Gln Leu Pro Glu
1               5                   10

<210>  146
<211>  9
<212>  PRT
<213>  Artificial sequence

<220>
<223>  HLA restricted peptide

<400>  146

Cys Ser Gly Asn Phe Met Gly Phe Asn
1               5

<210>  147
<211>  10
<212>  PRT
<213>  Artificial sequence

<220>

<223>  HLA restricted peptide

<400>  147

Cys Ser Gly Asn Phe Met Gly Phe Asn Cys
1               5                   10


<210>  148
<211>  9
<212>  PRT
<213>  Artificial sequence

<220>
<223>  HLA restricted peptide

<400>  148

Cys Ser Arg Leu Glu Glu Tyr Asn Ser
1               5


<210>  149
<211>  10
<212>  PRT
<213>  Artificial sequence

<220>
<223>  HLA restricted peptide

<400>  149

Cys Ser Arg Leu Glu Glu Tyr Asn Ser His
1               5                   10


<210>  150
<211>  9
<212>  PRT
<213>  Artificial sequence

<220>
<223>  HLA restricted peptide

<400>  150

Cys Thr Asp Glu Tyr Met Gly Gly Gln
1               5


<210>  151
<211>  10
<212>  PRT
<213>  Artificial sequence

<220>
<223>  HLA restricted peptide

<400>  151

Cys Thr Asp Glu Tyr Met Gly Gly Gln His
1               5                   10


<210>  152

<211>  9
<212>  PRT
<213>  Artificial sequence

<220>
<223>  HLA restricted peptide

<400>  152

Cys Thr Glu Arg Arg Leu Leu Val Arg
1               5


<210>  153
<211>  10
<212>  PRT
<213>  Artificial sequence

<220>
<223>  HLA restricted peptide

<400>  153

Cys Thr Glu Arg Arg Leu Leu Val Arg Arg
1               5                   10


<210>  154
<211>  10
<212>  PRT
<213>  Artificial sequence

<220>
<223>  HLA restricted peptide

<400>  154

Cys Val Ser Ser Lys Asn Leu Met Glu Lys
1               5                   10


<210>  155
<211>  9
<212>  PRT
<213>  Artificial sequence

<220>
<223>  HLA restricted peptide

<400>  155

Asp Ala Glu Lys Cys Asp Ile Cys Thr
1               5


<210>  156
<211>  10
<212>  PRT
<213>  Artificial sequence

<220>
<223>  HLA restricted peptide

<400>  156

Asp Ala Glu Lys Cys Asp Ile Cys Thr Asp
1               5                   10


<210>  157
<211>  9
<212>  PRT
<213>  Artificial sequence

<220>
<223>  HLA restricted peptide

<400>  157

Asp Ala Leu Leu Gly Gly Tyr Glu Ile
1               5


<210>  158
<211>  10
<212>  PRT
<213>  Artificial sequence

<220>
<223>  HLA restricted peptide

<400>  158

Asp Ala Leu Leu Gly Gly Tyr Glu Ile Trp
1               5                   10


<210>  159
<211>  9
<212>  PRT
<213>  Artificial sequence

<220>
<223>  HLA restricted peptide

<400>  159

Asp Ala Ser Gln Ser Ser Met His Asn
1               5


<210>  160
<211>  10
<212>  PRT
<213>  Artificial sequence

<220>
<223>  HLA restricted peptide

<400>  160

Asp Ala Ser Gln Ser Ser Met His Asn Ala
1               5                   10


<210>  161
<211>  9
<212>  PRT
<213>  Artificial sequence

<220>
<223>  HLA restricted peptide

<400>  161

Asp Asp Arg Glu Ser Trp Pro Ser Val
1               5


<210>  162
<211>  10
<212>  PRT
<213>  Artificial sequence

<220>
<223>  HLA restricted peptide

<400>  162

Asp Asp Arg Glu Ser Trp Pro Ser Val Phe
1               5                   10


<210>  163
<211>  9
<212>  PRT
<213>  Artificial sequence

<220>
<223>  HLA restricted peptide

<400>  163

Asp Glu Asn Phe Thr Ile Pro Tyr Trp
1               5


<210>  164
<211>  10
<212>  PRT
<213>  Artificial sequence

<220>
<223>  HLA restricted peptide

<400>  164

Asp Glu Asn Phe Thr Ile Pro Tyr Trp Asp
1               5                   10


<210>  165
<211>  9
<212>  PRT
<213>  Artificial sequence

<220>
<223>  HLA restricted peptide

<400>  165

Asp Glu Tyr Met Gly Gly Gln His Pro
1               5

<210>  166
<211>  10
<212>  PRT
<213>  Artificial sequence

<220>
<223>  HLA restricted peptide

<400>  166

Asp Glu Tyr Met Gly Gly Gln His Pro Thr
1               5                   10


<210>  167
<211>  9
<212>  PRT
<213>  Artificial sequence

<220>
<223>  HLA restricted peptide

<400>  167

Asp Phe Ala His Glu Ala Pro Ala Phe
1               5


<210>  168
<211>  10
<212>  PRT
<213>  Artificial sequence

<220>
<223>  HLA restricted peptide

<400>  168

Asp Phe Ala His Glu Ala Pro Ala Phe Leu
1               5                   10


<210>  169
<211>  9
<212>  PRT
<213>  Artificial sequence

<220>
<223>  HLA restricted peptide

<400>  169

Asp Phe Phe Ile Ser Ser Lys Asp Leu
1               5


<210>  170
<211>  10
<212>  PRT
<213>  Artificial sequence

<220>
<223>  HLA restricted peptide

<400>  170

Asp Gly Thr Met Ser Gln Val Gln Gly Ser
1               5                   10


<210>  171
<211>  10
<212>  PRT
<213>  Artificial sequence

<220>
<223>  HLA restricted peptide

<400>  171

Asp Ile Asp Phe Ala His Glu Ala Pro Ala
1               5                   10


<210>  172
<211>  9
<212>  PRT
<213>  Artificial sequence

<220>
<223>  HLA restricted peptide

<400>  172

Asp Ile Asn Ile Tyr Asp Leu Phe Val
1               5


<210>  173
<211>  10
<212>  PRT
<213>  Artificial sequence

<220>
<223>  HLA restricted peptide

<400>  173

Asp Ile Asn Ile Tyr Asp Leu Phe Val Trp
1               5                   10


<210>  174
<211>  9
<212>  PRT
<213>  Artificial sequence

<220>
<223>  HLA restricted peptide

<400>  174

Asp Lys Ala Ala Asn Phe Ser Phe Arg
1               5


<210>  175
<211>  10
<212>  PRT
<213>  Artificial sequence

<220>
<223>  HLA restricted peptide

<400>  175

Asp Lys Ala Ala Asn Phe Ser Phe Arg Asn
1               5                   10


<210>  176
<211>  9
<212>  PRT
<213>  Artificial sequence

<220>
<223>  HLA restricted peptide

<400>  176

Asp Lys Phe Phe Ala Tyr Leu Thr Leu
1               5


<210>  177
<211>  10
<212>  PRT
<213>  Artificial sequence

<220>
<223>  HLA restricted peptide

<400>  177

Asp Lys Phe Phe Ala Tyr Leu Thr Leu Ala
1               5                   10


<210>  178
<211>  10
<212>  PRT
<213>  Artificial sequence

<220>
<223>  HLA restricted peptide

<400>  178

Asp Lys Ser Arg Thr Pro Arg Leu Pro Ser
1               5                   10


<210>  179
<211>  9
<212>  PRT
<213>  Artificial sequence

<220>
<223>  HLA restricted peptide

<400>  179

Asp Leu Phe Val Trp Ile His Tyr Tyr
1               5

<210>  180
<211>  10
<212>  PRT
<213>  Artificial sequence

<220>
<223>  HLA restricted peptide

<400>  180

Asp Leu Phe Val Trp Ile His Tyr Tyr Val
1               5                   10


<210>  181
<211>  9
<212>  PRT
<213>  Artificial sequence

<220>
<223>  HLA restricted peptide

<400>  181

Asp Leu Gly Tyr Asp Tyr Ser Tyr Leu
1               5


<210>  182
<211>  9
<212>  PRT
<213>  Artificial sequence

<220>
<223>  HLA restricted peptide

<400>  182

Asp Leu Ser Ala Pro Glu Lys Asp Lys
1               5


<210>  183
<211>  10
<212>  PRT
<213>  Artificial sequence

<220>
<223>  HLA restricted peptide

<400>  183

Asp Leu Ser Ala Pro Glu Lys Asp Lys Phe
1               5                   10


<210>  184
<211>  9
<212>  PRT
<213>  Artificial sequence

<220>
<223>  HLA restricted peptide

<400>  184

Asp Pro Asp Ser Phe Gln Asp Tyr Ile
1               5


<210>  185
<211>  10
<212>  PRT
<213>  Artificial sequence

<220>
<223>  HLA restricted peptide

<400>  185

Asp Pro Asp Ser Phe Gln Asp Tyr Ile Lys
1               5                   10


<210>  186
<211>  9
<212>  PRT
<213>  Artificial sequence

<220>
<223>  HLA restricted peptide

<400>  186

Asp Pro Ile Phe Leu Leu His His Ala
1               5


<210>  187
<211>  10
<212>  PRT
<213>  Artificial sequence

<220>
<223>  HLA restricted peptide

<400>  187

Asp Pro Ile Phe Leu Leu His His Ala Phe
1               5                   10


<210>  188
<211>  9
<212>  PRT
<213>  Artificial sequence

<220>
<223>  HLA restricted peptide

<400>  188

Asp Arg Glu Ser Trp Pro Ser Val Phe
1               5


<210>  189
<211>  10
<212>  PRT

<213>  Artificial sequence

<220>
<223>  HLA restricted peptide

<400>  189

Asp Arg Glu Ser Trp Pro Ser Val Phe Tyr
1               5                   10


<210>  190
<211>  9
<212>  PRT
<213>  Artificial sequence

<220>
<223>  HLA restricted peptide

<400>  190

Asp Arg Ser Pro Cys Gly Gln Leu Ser
1               5


<210>  191
<211>  10
<212>  PRT
<213>  Artificial sequence

<220>
<223>  HLA restricted peptide

<400>  191

Asp Arg Ser Pro Cys Gly Gln Leu Ser Gly
1               5                   10


<210>  192
<211>  9
<212>  PRT
<213>  Artificial sequence

<220>
<223>  HLA restricted peptide

<400>  192

Asp Ser Asp Pro Asp Ser Phe Gln Asp
1               5


<210>  193
<211>  10
<212>  PRT
<213>  Artificial sequence

<220>
<223>  HLA restricted peptide

<400>  193

Asp Ser Asp Pro Asp Ser Phe Gln Asp Tyr
1               5                   10

<210>  194
<211>  9
<212>  PRT
<213>  Artificial sequence

<220>
<223>  HLA restricted peptide

<400>  194

Asp Ser Phe Gln Asp Tyr Ile Lys Ser
1               5


<210>  195
<211>  10
<212>  PRT
<213>  Artificial sequence

<220>
<223>  HLA restricted peptide

<400>  195

Asp Ser Phe Gln Asp Tyr Ile Lys Ser Tyr
1               5                   10


<210>  196
<211>  9
<212>  PRT
<213>  Artificial sequence

<220>
<223>  HLA restricted peptide

<400>  196

Asp Ser Ile Phe Glu Gln Trp Leu Arg
1               5


<210>  197
<211>  10
<212>  PRT
<213>  Artificial sequence

<220>
<223>  HLA restricted peptide

<400>  197

Asp Ser Ile Phe Glu Gln Trp Leu Arg Arg
1               5                   10


<210>  198
<211>  9
<212>  PRT
<213>  Artificial sequence

<220>
<223>  HLA restricted peptide

<400>  198

Asp Val Glu Phe Cys Leu Ser Leu Thr
1               5


<210>  199
<211>  10
<212>  PRT
<213>  Artificial sequence

<220>
<223>  HLA restricted peptide

<400>  199

Asp Val Glu Phe Cys Leu Ser Leu Thr Gln
1               5                   10


<210>  200
<211>  10
<212>  PRT
<213>  Artificial sequence

<220>
<223>  HLA restricted peptide

<400>  200

Asp Trp Arg Asp Ala Glu Lys Cys Asp Ile
1               5                   10


<210>  201
<211>  9
<212>  PRT
<213>  Artificial sequence

<220>
<223>  HLA restricted peptide

<400>  201

Asp Tyr His Ser Leu Tyr Gln Ser His
1               5


<210>  202
<211>  10
<212>  PRT
<213>  Artificial sequence

<220>
<223>  HLA restricted peptide

<400>  202

Asp Tyr His Ser Leu Tyr Gln Ser His Leu
1               5                   10


<210>  203
<211>  9

<212>  PRT
<213>  Artificial sequence

<220>
<223>  HLA restricted peptide

<400>  203

Asp Tyr Ile Lys Ser Tyr Leu Glu Gln
1               5


<210>  204
<211>  10
<212>  PRT
<213>  Artificial sequence

<220>
<223>  HLA restricted peptide

<400>  204

Asp Tyr Ile Lys Ser Tyr Leu Glu Gln Ala
1               5                   10


<210>  205
<211>  9
<212>  PRT
<213>  Artificial sequence

<220>
<223>  HLA restricted peptide

<400>  205

Asp Tyr Val Ile Pro Ile Gly Thr Tyr
1               5


<210>  206
<211>  10
<212>  PRT
<213>  Artificial sequence

<220>
<223>  HLA restricted peptide

<400>  206

Asp Tyr Val Ile Pro Ile Gly Thr Tyr Gly
1               5                   10


<210>  207
<211>  9
<212>  PRT
<213>  Artificial sequence

<220>
<223>  HLA restricted peptide

<400>  207

Glu Ala Asn Ala Pro Ile Gly His Asn

1               5


<210>  208
<211>  10
<212>  PRT
<213>  Artificial sequence

<220>
<223>  HLA restricted peptide

<400>  208

Glu Ala Asn Ala Pro Ile Gly His Asn Arg
1               5                   10


<210>  209
<211>  9
<212>  PRT
<213>  Artificial sequence

<220>
<223>  HLA restricted peptide

<400>  209

Glu Ala Pro Ala Phe Leu Pro Trp His
1               5


<210>  210
<211>  10
<212>  PRT
<213>  Artificial sequence

<220>
<223>  HLA restricted peptide

<400>  210

Glu Ala Pro Ala Phe Leu Pro Trp His Arg
1               5                   10


<210>  211
<211>  10
<212>  PRT
<213>  Artificial sequence

<220>
<223>  HLA restricted peptide

<400>  211

Glu Cys Cys Pro Pro Trp Ser Gly Asp Arg
1               5                   10


<210>  212
<211>  9
<212>  PRT
<213>  Artificial sequence

<220>

<223>  HLA restricted peptide

<400>  212

Glu Asp Tyr His Ser Leu Tyr Gln Ser
1               5

<210>  213
<211>  10
<212>  PRT
<213>  Artificial sequence

<220>
<223>  HLA restricted peptide

<400>  213

Glu Asp Tyr His Ser Leu Tyr Gln Ser His
1               5                   10

<210>  214
<211>  10
<212>  PRT
<213>  Artificial sequence

<220>
<223>  HLA restricted peptide

<400>  214

Glu Glu Lys Gln Pro Leu Leu Met Glu Lys
1               5                   10

<210>  215
<211>  9
<212>  PRT
<213>  Artificial sequence

<220>
<223>  HLA restricted peptide

<400>  215

Glu Glu Tyr Asn Ser His Gln Ser Leu
1               5

<210>  216
<211>  10
<212>  PRT
<213>  Artificial sequence

<220>
<223>  HLA restricted peptide

<400>  216

Glu Glu Tyr Asn Ser His Gln Ser Leu Cys
1               5                   10

<210>  217

<211>  9
<212>  PRT
<213>  Artificial sequence

<220>
<223>  HLA restricted peptide

<400>  217

Glu Phe Cys Leu Ser Leu Thr Gln Tyr
1               5


<210>  218
<211>  9
<212>  PRT
<213>  Artificial sequence

<220>
<223>  HLA restricted peptide

<400>  218

Glu Gly Phe Ala Ser Pro Leu Thr Gly
1               5


<210>  219
<211>  10
<212>  PRT
<213>  Artificial sequence

<220>
<223>  HLA restricted peptide

<400>  219

Glu Gly Phe Ala Ser Pro Leu Thr Gly Ile
1               5                   10


<210>  220
<211>  9
<212>  PRT
<213>  Artificial sequence

<220>
<223>  HLA restricted peptide

<400>  220

Glu Gly Pro Leu Arg Arg Asn Pro Gly
1               5


<210>  221
<211>  10
<212>  PRT
<213>  Artificial sequence

<220>
<223>  HLA restricted peptide

<400>  221

Glu Gly Pro Leu Arg Arg Asn Pro Gly Asn
1               5                   10

<210>  222
<211>  9
<212>  PRT
<213>  Artificial sequence

<220>
<223>  HLA restricted peptide

<400>  222

Glu Ile Trp Arg Asp Ile Asp Phe Ala
1               5

<210>  223
<211>  10
<212>  PRT
<213>  Artificial sequence

<220>
<223>  HLA restricted peptide

<400>  223

Glu Ile Trp Arg Asp Ile Asp Phe Ala His
1               5                   10

<210>  224
<211>  10
<212>  PRT
<213>  Artificial sequence

<220>
<223>  HLA restricted peptide

<400>  224

Glu Lys Cys Asp Ile Cys Thr Asp Glu Tyr
1               5                   10

<210>  225
<211>  9
<212>  PRT
<213>  Artificial sequence

<220>
<223>  HLA restricted peptide

<400>  225

Glu Lys Asp Lys Phe Phe Ala Tyr Leu
1               5

<210>  226
<211>  10
<212>  PRT
<213>  Artificial sequence

<220>
<223>  HLA restricted peptide

<400>  226

Glu Lys Asp Lys Phe Phe Ala Tyr Leu Thr
1               5                   10


<210>  227
<211>  9
<212>  PRT
<213>  Artificial sequence

<220>
<223>  HLA restricted peptide

<400>  227

Glu Lys Glu Cys Cys Pro Pro Trp Ser
1               5


<210>  228
<211>  9
<212>  PRT
<213>  Artificial sequence

<220>
<223>  HLA restricted peptide

<400>  228

Glu Lys Glu Asp Tyr His Ser Leu Tyr
1               5


<210>  229
<211>  9
<212>  PRT
<213>  Artificial sequence

<220>
<223>  HLA restricted peptide

<400>  229

Glu Lys Gln Pro Leu Leu Met Glu Lys
1               5


<210>  230
<211>  10
<212>  PRT
<213>  Artificial sequence

<220>
<223>  HLA restricted peptide

<400>  230

Glu Asn Phe Thr Ile Pro Tyr Trp Asp Trp
1               5                   10

<210>  231
<211>  9
<212>  PRT
<213>  Artificial sequence

<220>
<223>  HLA restricted peptide

<400>  231

Glu Gln Ala Ser Arg Ile Trp Ser Trp
1               5


<210>  232
<211>  10
<212>  PRT
<213>  Artificial sequence

<220>
<223>  HLA restricted peptide

<400>  232

Glu Gln Ala Ser Arg Ile Trp Ser Trp Leu
1               5                   10


<210>  233
<211>  9
<212>  PRT
<213>  Artificial sequence

<220>
<223>  HLA restricted peptide

<400>  233

Glu Gln Trp Leu Arg Arg His Arg Pro
1               5


<210>  234
<211>  10
<212>  PRT
<213>  Artificial sequence

<220>
<223>  HLA restricted peptide

<400>  234

Glu Gln Trp Leu Arg Arg His Arg Pro Leu
1               5                   10


<210>  235
<211>  9
<212>  PRT
<213>  Artificial sequence

<220>
<223>  HLA restricted peptide

<400>  235

Glu Arg Arg Leu Leu Val Arg Arg Asn
1               5

<210>  236
<211>  10
<212>  PRT
<213>  Artificial sequence

<220>
<223>  HLA restricted peptide

<400>  236

Glu Arg Arg Leu Leu Val Arg Arg Asn Ile
1               5                   10

<210>  237
<211>  9
<212>  PRT
<213>  Artificial sequence

<220>
<223>  HLA restricted peptide

<400>  237

Glu Ser Gly Ser Met Asp Lys Ala Ala
1               5

<210>  238
<211>  10
<212>  PRT
<213>  Artificial sequence

<220>
<223>  HLA restricted peptide

<400>  238

Glu Ser Trp Pro Ser Val Phe Tyr Asn Arg
1               5                   10

<210>  239
<211>  9
<212>  PRT
<213>  Artificial sequence

<220>
<223>  HLA restricted peptide

<400>  239

Glu Ser Tyr Met Val Pro Phe Ile Pro
1               5

<210>  240
<211>  10
<212>  PRT
<213>  Artificial sequence

<220>
<223>  HLA restricted peptide

<400>  240

Glu Ser Tyr Met Val Pro Phe Ile Pro Leu
1               5                   10


<210>  241
<211>  9
<212>  PRT
<213>  Artificial sequence

<220>
<223>  HLA restricted peptide

<400>  241

Glu Val Tyr Pro Glu Ala Asn Ala Pro
1               5


<210>  242
<211>  10
<212>  PRT
<213>  Artificial sequence

<220>
<223>  HLA restricted peptide

<400>  242

Glu Val Tyr Pro Glu Ala Asn Ala Pro Ile
1               5                   10


<210>  243
<211>  9
<212>  PRT
<213>  Artificial sequence

<220>
<223>  HLA restricted peptide

<400>  243

Glu Tyr Met Gly Gly Gln His Pro Thr
1               5


<210>  244
<211>  10
<212>  PRT
<213>  Artificial sequence

<220>
<223>  HLA restricted peptide

<400>  244

Glu Tyr Met Gly Gly Gln His Pro Thr Asn
1               5                   10

<210>  245
<211>  9
<212>  PRT
<213>  Artificial sequence

<220>
<223>  HLA restricted peptide

<400>  245

Glu Tyr Asn Ser His Gln Ser Leu Cys
1               5


<210>  246
<211>  10
<212>  PRT
<213>  Artificial sequence

<220>
<223>  HLA restricted peptide

<400>  246

Glu Tyr Asn Ser His Gln Ser Leu Cys Asn
1               5                   10


<210>  247
<211>  9
<212>  PRT
<213>  Artificial sequence

<220>
<223>  HLA restricted peptide

<400>  247

Phe Ala His Glu Ala Pro Ala Phe Leu
1               5


<210>  248
<211>  10
<212>  PRT
<213>  Artificial sequence

<220>
<223>  HLA restricted peptide

<400>  248

Phe Ala His Glu Ala Pro Ala Phe Leu Pro
1               5                   10


<210>  249
<211>  9
<212>  PRT
<213>  Artificial sequence

<220>
<223>  HLA restricted peptide

<400>  249

Phe Ala Ser Pro Leu Thr Gly Ile Ala
1               5


<210>  250
<211>  10
<212>  PRT
<213>  Artificial sequence

<220>
<223>  HLA restricted peptide

<400>  250

Phe Ala Ser Pro Leu Thr Gly Ile Ala Asp
1               5                   10


<210>  251
<211>  9
<212>  PRT
<213>  Artificial sequence

<220>
<223>  HLA restricted peptide

<400>  251

Phe Ala Tyr Leu Thr Leu Ala Lys His
1               5


<210>  252
<211>  10
<212>  PRT
<213>  Artificial sequence

<220>
<223>  HLA restricted peptide

<400>  252

Phe Ala Tyr Leu Thr Leu Ala Lys His Thr
1               5                   10


<210>  253
<211>  10
<212>  PRT
<213>  Artificial sequence

<220>
<223>  HLA restricted peptide

<400>  253

Phe Cys Leu Ser Leu Thr Gln Tyr Glu Ser
1               5                   10


<210>  254
<211>  9
<212>  PRT

<213>  Artificial sequence

<220>
<223>  HLA restricted peptide

<400>  254

Phe Glu Gln Trp Leu Arg Arg His Arg
1               5


<210>  255
<211>  9
<212>  PRT
<213>  Artificial sequence

<220>
<223>  HLA restricted peptide

<400>  255

Phe Phe Ala Tyr Leu Thr Leu Ala Lys
1               5


<210>  256
<211>  10
<212>  PRT
<213>  Artificial sequence

<220>
<223>  HLA restricted peptide

<400>  256

Phe Phe Ala Tyr Leu Thr Leu Ala Lys His
1               5                   10


<210>  257
<211>  10
<212>  PRT
<213>  Artificial sequence

<220>
<223>  HLA restricted peptide

<400>  257

Phe Phe Ile Ser Ser Lys Asp Leu Gly Tyr
1               5                   10


<210>  258
<211>  9
<212>  PRT
<213>  Artificial sequence

<220>
<223>  HLA restricted peptide

<400>  258

Phe Phe Ser Ser Trp Gln Ile Val Cys
1               5

<210>  259
<211>  10
<212>  PRT
<213>  Artificial sequence

<220>
<223>  HLA restricted peptide

<400>  259

Phe Phe Ser Ser Trp Gln Ile Val Cys Ser
1               5                   10


<210>  260
<211>  9
<212>  PRT
<213>  Artificial sequence

<220>
<223>  HLA restricted peptide

<400>  260

Phe Gly Phe Trp Gly Pro Asn Cys Thr
1               5


<210>  261
<211>  10
<212>  PRT
<213>  Artificial sequence

<220>
<223>  HLA restricted peptide

<400>  261

Phe Gly Phe Trp Gly Pro Asn Cys Thr Glu
1               5                   10


<210>  262
<211>  10
<212>  PRT
<213>  Artificial sequence

<220>
<223>  HLA restricted peptide

<400>  262

Phe Ile Pro Leu Tyr Arg Asn Gly Asp Phe
1               5                   10


<210>  263
<211>  9
<212>  PRT
<213>  Artificial sequence

<220>
<223>  HLA restricted peptide

<400>  263

Phe Ile Ser Ser Lys Asp Leu Gly Tyr
1               5


<210>  264
<211>  10
<212>  PRT
<213>  Artificial sequence

<220>
<223>  HLA restricted peptide

<400>  264

Phe Leu Leu His His Ala Phe Val Asp Ser
1               5                   10


<210>  265
<211>  9
<212>  PRT
<213>  Artificial sequence

<220>
<223>  HLA restricted peptide

<400>  265

Phe Leu Leu Arg Trp Glu Gln Glu Ile
1               5


<210>  266
<211>  9
<212>  PRT
<213>  Artificial sequence

<220>
<223>  HLA restricted peptide

<400>  266

Phe Leu Pro Trp His Arg Leu Phe Leu
1               5


<210>  267
<211>  10
<212>  PRT
<213>  Artificial sequence

<220>
<223>  HLA restricted peptide

<400>  267

Phe Leu Pro Trp His Arg Leu Phe Leu Leu
1               5                   10


<210>  268
<211>  9

<212>  PRT
<213>  Artificial sequence

<220>
<223>  HLA restricted peptide

<400>  268

Phe Met Gly Phe Asn Cys Gly Asn Cys
1               5


<210>  269
<211>  10
<212>  PRT
<213>  Artificial sequence

<220>
<223>  HLA restricted peptide

<400>  269

Phe Met Gly Phe Asn Cys Gly Asn Cys Lys
1               5                   10


<210>  270
<211>  10
<212>  PRT
<213>  Artificial sequence

<220>
<223>  HLA restricted peptide

<400>  270

Phe Asn Cys Gly Asn Cys Lys Phe Gly Phe
1               5                   10


<210>  271
<211>  9
<212>  PRT
<213>  Artificial sequence

<220>
<223>  HLA restricted peptide

<400>  271

Phe Asn Asp Ile Asn Ile Tyr Asp Leu
1               5


<210>  272
<211>  10
<212>  PRT
<213>  Artificial sequence

<220>
<223>  HLA restricted peptide

<400>  272

Phe Asn Asp Ile Asn Ile Tyr Asp Leu Phe

1                   5                   10


<210>  273
<211>  9
<212>  PRT
<213>  Artificial sequence

<220>
<223>  HLA restricted peptide

<400>  273

Phe Pro Phe Thr Gly Val Asp Asp Arg
1               5


<210>  274
<211>  10
<212>  PRT
<213>  Artificial sequence

<220>
<223>  HLA restricted peptide

<400>  274

Phe Pro Phe Thr Gly Val Asp Asp Arg Glu
1               5                   10


<210>  275
<211>  9
<212>  PRT
<213>  Artificial sequence

<220>
<223>  HLA restricted peptide

<400>  275

Phe Pro Arg Ala Cys Val Ser Ser Lys
1               5


<210>  276
<211>  10
<212>  PRT
<213>  Artificial sequence

<220>
<223>  HLA restricted peptide

<400>  276

Phe Pro Arg Ala Cys Val Ser Ser Lys Asn
1               5                   10


<210>  277
<211>  9
<212>  PRT
<213>  Artificial sequence

<220>

<223>  HLA restricted peptide

<400>  277

Phe Gln Asp Tyr Ile Lys Ser Tyr Leu
1               5


<210>  278
<211>  10
<212>  PRT
<213>  Artificial sequence

<220>
<223>  HLA restricted peptide

<400>  278

Phe Gln Asp Tyr Ile Lys Ser Tyr Leu Glu
1               5                   10


<210>  279
<211>  9
<212>  PRT
<213>  Artificial sequence

<220>
<223>  HLA restricted peptide

<400>  279

Phe Gln Thr Ser Ala Gly His Phe Pro
1               5


<210>  280
<211>  10
<212>  PRT
<213>  Artificial sequence

<220>
<223>  HLA restricted peptide

<400>  280

Phe Gln Thr Ser Ala Gly His Phe Pro Arg
1               5                   10


<210>  281
<211>  9
<212>  PRT
<213>  Artificial sequence

<220>
<223>  HLA restricted peptide

<400>  281

Phe Arg Asn Thr Leu Glu Gly Phe Ala
1               5


<210>  282

<211>  10
<212>  PRT
<213>  Artificial sequence

<220>
<223>  HLA restricted peptide

<400>  282

Phe Arg Asn Thr Leu Glu Gly Phe Ala Ser
1               5                   10


<210>  283
<211>  10
<212>  PRT
<213>  Artificial sequence

<220>
<223>  HLA restricted peptide

<400>  283

Phe Ser Phe Arg Asn Thr Leu Glu Gly Phe
1               5                   10


<210>  284
<211>  9
<212>  PRT
<213>  Artificial sequence

<220>
<223>  HLA restricted peptide

<400>  284

Phe Ser Ser Trp Gln Ile Val Cys Ser
1               5


<210>  285
<211>  10
<212>  PRT
<213>  Artificial sequence

<220>
<223>  HLA restricted peptide

<400>  285

Phe Ser Ser Trp Gln Ile Val Cys Ser Arg
1               5                   10


<210>  286
<211>  10
<212>  PRT
<213>  Artificial sequence

<220>
<223>  HLA restricted peptide

<400>  286

Phe Thr Gly Val Asp Asp Arg Glu Ser Trp
1               5                   10


<210>  287
<211>  9
<212>  PRT
<213>  Artificial sequence

<220>
<223>  HLA restricted peptide

<400>  287

Phe Thr Ile Pro Tyr Trp Asp Trp Arg
1               5


<210>  288
<211>  9
<212>  PRT
<213>  Artificial sequence

<220>
<223>  HLA restricted peptide

<400>  288

Phe Val Asp Ser Ile Phe Glu Gln Trp
1               5


<210>  289
<211>  10
<212>  PRT
<213>  Artificial sequence

<220>
<223>  HLA restricted peptide

<400>  289

Phe Val Asp Ser Ile Phe Glu Gln Trp Leu
1               5                   10


<210>  290
<211>  9
<212>  PRT
<213>  Artificial sequence

<220>
<223>  HLA restricted peptide

<400>  290

Phe Val Trp Ile His Tyr Tyr Val Ser
1               5


<210>  291
<211>  10
<212>  PRT
<213>  Artificial sequence

<220>
<223>  HLA restricted peptide

<400>  291

Phe Val Trp Ile His Tyr Tyr Val Ser Met
1               5                   10


<210>  292
<211>  9
<212>  PRT
<213>  Artificial sequence

<220>
<223>  HLA restricted peptide

<400>  292

Phe Trp Gly Pro Asn Cys Thr Glu Arg
1               5


<210>  293
<211>  10
<212>  PRT
<213>  Artificial sequence

<220>
<223>  HLA restricted peptide

<400>  293

Phe Trp Gly Pro Asn Cys Thr Glu Arg Arg
1               5                   10


<210>  294
<211>  9
<212>  PRT
<213>  Artificial sequence

<220>
<223>  HLA restricted peptide

<400>  294

Phe Tyr Asn Arg Thr Cys Gln Cys Ser
1               5


<210>  295
<211>  9
<212>  PRT
<213>  Artificial sequence

<220>
<223>  HLA restricted peptide

<400>  295

Gly Ala Ala Met Val Gly Ala Val Leu
1               5

<210>  296
<211>  10
<212>  PRT
<213>  Artificial sequence

<220>
<223>  HLA restricted peptide

<400>  296

Gly Ala Ala Met Val Gly Ala Val Leu Thr
1               5                   10


<210>  297
<211>  9
<212>  PRT
<213>  Artificial sequence

<220>
<223>  HLA restricted peptide

<400>  297

Gly Ala Val Leu Thr Ala Leu Leu Ala
1               5


<210>  298
<211>  10
<212>  PRT
<213>  Artificial sequence

<220>
<223>  HLA restricted peptide

<400>  298

Gly Ala Val Leu Thr Ala Leu Leu Ala Gly
1               5                   10


<210>  299
<211>  9
<212>  PRT
<213>  Artificial sequence

<220>
<223>  HLA restricted peptide

<400>  299

Gly Asp Glu Asn Phe Thr Ile Pro Tyr
1               5


<210>  300
<211>  10
<212>  PRT
<213>  Artificial sequence

<220>
<223>  HLA restricted peptide

<400>  300

Gly Asp Glu Asn Phe Thr Ile Pro Tyr Trp
1               5                   10


<210>  301
<211>  10
<212>  PRT
<213>  Artificial sequence

<220>
<223>  HLA restricted peptide

<400>  301

Gly Asp Phe Phe Ile Ser Ser Lys Asp Leu
1               5                   10


<210>  302
<211>  9
<212>  PRT
<213>  Artificial sequence

<220>
<223>  HLA restricted peptide

<400>  302

Gly Asp Arg Ser Pro Cys Gly Gln Leu
1               5


<210>  303
<211>  10
<212>  PRT
<213>  Artificial sequence

<220>
<223>  HLA restricted peptide

<400>  303

Gly Asp Arg Ser Pro Cys Gly Gln Leu Ser
1               5                   10


<210>  304
<211>  9
<212>  PRT
<213>  Artificial sequence

<220>
<223>  HLA restricted peptide

<400>  304

Gly Phe Ala Ser Pro Leu Thr Gly Ile
1               5


<210>  305
<211>  10
<212>  PRT
<213>  Artificial sequence

<220>
<223>  HLA restricted peptide

<400>  305

Gly Phe Ala Ser Pro Leu Thr Gly Ile Ala
1               5                   10


<210>  306
<211>  9
<212>  PRT
<213>  Artificial sequence

<220>
<223>  HLA restricted peptide

<400>  306

Gly Phe Asn Cys Gly Asn Cys Lys Phe
1               5


<210>  307
<211>  10
<212>  PRT
<213>  Artificial sequence

<220>
<223>  HLA restricted peptide

<400>  307

Gly Phe Trp Gly Pro Asn Cys Thr Glu Arg
1               5                   10


<210>  308
<211>  9
<212>  PRT
<213>  Artificial sequence

<220>
<223>  HLA restricted peptide

<400>  308

Gly Gly Gln His Pro Thr Asn Pro Asn
1               5


<210>  309
<211>  10
<212>  PRT
<213>  Artificial sequence

<220>
<223>  HLA restricted peptide

<400>  309

Gly Gly Gln His Pro Thr Asn Pro Asn Leu
1               5                   10

<210>  310
<211>  9
<212>  PRT
<213>  Artificial sequence

<220>
<223>  HLA restricted peptide

<400>  310

Gly Gly Tyr Glu Ile Trp Arg Asp Ile
1               5


<210>  311
<211>  10
<212>  PRT
<213>  Artificial sequence

<220>
<223>  HLA restricted peptide

<400>  311

Gly Gly Tyr Glu Ile Trp Arg Asp Ile Asp
1               5                   10


<210>  312
<211>  9
<212>  PRT
<213>  Artificial sequence

<220>
<223>  HLA restricted peptide

<400>  312

Gly His Phe Pro Arg Ala Cys Val Ser
1               5


<210>  313
<211>  10
<212>  PRT
<213>  Artificial sequence

<220>
<223>  HLA restricted peptide

<400>  313

Gly His Phe Pro Arg Ala Cys Val Ser Ser
1               5                   10


<210>  314
<211>  9
<212>  PRT
<213>  Artificial sequence

<220>
<223>  HLA restricted peptide

<400>  314

Gly His Asn Arg Glu Ser Tyr Met Val
1               5


<210>  315
<211>  9
<212>  PRT
<213>  Artificial sequence

<220>
<223>  HLA restricted peptide

<400>  315

Gly Ile Ala Asp Ala Ser Gln Ser Ser
1               5


<210>  316
<211>  10
<212>  PRT
<213>  Artificial sequence

<220>
<223>  HLA restricted peptide

<400>  316

Gly Ile Ala Asp Ala Ser Gln Ser Ser Met
1               5                   10


<210>  317
<211>  9
<212>  PRT
<213>  Artificial sequence

<220>
<223>  HLA restricted peptide

<400>  317

Gly Leu Val Ser Leu Leu Cys Arg His
1               5


<210>  318
<211>  10
<212>  PRT
<213>  Artificial sequence

<220>
<223>  HLA restricted peptide

<400>  318

Gly Leu Val Ser Leu Leu Cys Arg His Lys
1               5                   10


<210>  319
<211>  9
<212>  PRT

<213>  Artificial sequence

<220>
<223>  HLA restricted peptide

<400>  319

Gly Asn Phe Met Gly Phe Asn Cys Gly
1               5


<210>  320
<211>  10
<212>  PRT
<213>  Artificial sequence

<220>
<223>  HLA restricted peptide

<400>  320

Gly Asn Phe Met Gly Phe Asn Cys Gly Asn
1               5                   10


<210>  321
<211>  10
<212>  PRT
<213>  Artificial sequence

<220>
<223>  HLA restricted peptide

<400>  321

Gly Asn His Asp Lys Ser Arg Thr Pro Arg
1               5                   10


<210>  322
<211>  9
<212>  PRT
<213>  Artificial sequence

<220>
<223>  HLA restricted peptide

<400>  322

Gly Pro Leu Arg Arg Asn Pro Gly Asn
1               5


<210>  323
<211>  10
<212>  PRT
<213>  Artificial sequence

<220>
<223>  HLA restricted peptide

<400>  323

Gly Pro Leu Arg Arg Asn Pro Gly Asn His
1               5                   10

<210>  324
<211>  9
<212>  PRT
<213>  Artificial sequence

<220>
<223>  HLA restricted peptide

<400>  324

Gly Pro Asn Cys Thr Glu Arg Arg Leu
1               5


<210>  325
<211>  10
<212>  PRT
<213>  Artificial sequence

<220>
<223>  HLA restricted peptide

<400>  325

Gly Pro Asn Cys Thr Glu Arg Arg Leu Leu
1               5                   10


<210>  326
<211>  9
<212>  PRT
<213>  Artificial sequence

<220>
<223>  HLA restricted peptide

<400>  326

Gly Pro Gln Phe Pro Phe Thr Gly Val
1               5


<210>  327
<211>  10
<212>  PRT
<213>  Artificial sequence

<220>
<223>  HLA restricted peptide

<400>  327

Gly Pro Gln Phe Pro Phe Thr Gly Val Asp
1               5                   10


<210>  328
<211>  9
<212>  PRT
<213>  Artificial sequence

<220>
<223>  HLA restricted peptide

<400>  328

Gly Gln His Pro Thr Asn Pro Asn Leu
1               5


<210>  329
<211>  10
<212>  PRT
<213>  Artificial sequence

<220>
<223>  HLA restricted peptide

<400>  329

Gly Gln His Pro Thr Asn Pro Asn Leu Leu
1               5                   10


<210>  330
<211>  9
<212>  PRT
<213>  Artificial sequence

<220>
<223>  HLA restricted peptide

<400>  330

Gly Gln Leu Ser Gly Arg Gly Ser Cys
1               5


<210>  331
<211>  10
<212>  PRT
<213>  Artificial sequence

<220>
<223>  HLA restricted peptide

<400>  331

Gly Gln Leu Ser Gly Arg Gly Ser Cys Gln
1               5                   10


<210>  332
<211>  9
<212>  PRT
<213>  Artificial sequence

<220>
<223>  HLA restricted peptide

<400>  332

Gly Gln Met Lys Asn Gly Ser Thr Pro
1               5


<210>  333
<211>  10

<212>  PRT
<213>  Artificial sequence

<220>
<223>  HLA restricted peptide

<400>  333

Gly Gln Met Lys Asn Gly Ser Thr Pro Met
1               5                   10


<210>  334
<211>  9
<212>  PRT
<213>  Artificial sequence

<220>
<223>  HLA restricted peptide

<400>  334

Gly Arg Gly Ser Cys Gln Asn Ile Leu
1               5


<210>  335
<211>  10
<212>  PRT
<213>  Artificial sequence

<220>
<223>  HLA restricted peptide

<400>  335

Gly Arg Gly Ser Cys Gln Asn Ile Leu Leu
1               5                   10


<210>  336
<211>  9
<212>  PRT
<213>  Artificial sequence

<220>
<223>  HLA restricted peptide

<400>  336

Gly Ser Ala Asn Asp Pro Ile Phe Leu
1               5


<210>  337
<211>  10
<212>  PRT
<213>  Artificial sequence

<220>
<223>  HLA restricted peptide

<400>  337

Gly Ser Ala Asn Asp Pro Ile Phe Leu Leu

1                5                   10

<210>  338
<211>  9
<212>  PRT
<213>  Artificial sequence

<220>
<223>  HLA restricted peptide

<400>  338

Gly Ser Cys Gln Asn Ile Leu Leu Ser
1               5

<210>  339
<211>  10
<212>  PRT
<213>  Artificial sequence

<220>
<223>  HLA restricted peptide

<400>  339

Gly Ser Cys Gln Asn Ile Leu Leu Ser Asn
1               5                   10

<210>  340
<211>  9
<212>  PRT
<213>  Artificial sequence

<220>
<223>  HLA restricted peptide

<400>  340

Gly Ser Met Asp Lys Ala Ala Asn Phe
1               5

<210>  341
<211>  10
<212>  PRT
<213>  Artificial sequence

<220>
<223>  HLA restricted peptide

<400>  341

Gly Ser Met Asp Lys Ala Ala Asn Phe Ser
1               5                   10

<210>  342
<211>  9
<212>  PRT
<213>  Artificial sequence

<220>

<223>  HLA restricted peptide

<400>  342

Gly Ser Thr Pro Met Phe Asn Asp Ile
1               5


<210>  343
<211>  10
<212>  PRT
<213>  Artificial sequence

<220>
<223>  HLA restricted peptide

<400>  343

Gly Ser Thr Pro Met Phe Asn Asp Ile Asn
1               5                   10


<210>  344
<211>  9
<212>  PRT
<213>  Artificial sequence

<220>
<223>  HLA restricted peptide

<400>  344

Gly Thr Met Ser Gln Val Gln Gly Ser
1               5


<210>  345
<211>  10
<212>  PRT
<213>  Artificial sequence

<220>
<223>  HLA restricted peptide

<400>  345

Gly Thr Met Ser Gln Val Gln Gly Ser Ala
1               5                   10


<210>  346
<211>  9
<212>  PRT
<213>  Artificial sequence

<220>
<223>  HLA restricted peptide

<400>  346

Gly Thr Pro Glu Gly Pro Leu Arg Arg
1               5


<210>  347

<211>  10
<212>  PRT
<213>  Artificial sequence

<220>
<223>  HLA restricted peptide

<400>  347

Gly Thr Pro Glu Gly Pro Leu Arg Arg Asn
1               5                   10


<210>  348
<211>  10
<212>  PRT
<213>  Artificial sequence

<220>
<223>  HLA restricted peptide

<400>  348

Gly Thr Tyr Gly Gln Met Lys Asn Gly Ser
1               5                   10


<210>  349
<211>  10
<212>  PRT
<213>  Artificial sequence

<220>
<223>  HLA restricted peptide

<400>  349

Gly Val Asp Asp Arg Glu Ser Trp Pro Ser
1               5                   10


<210>  350
<211>  10
<212>  PRT
<213>  Artificial sequence

<220>
<223>  HLA restricted peptide

<400>  350

Gly Tyr Asp Tyr Ser Tyr Leu Gln Asp Ser
1               5                   10


<210>  351
<211>  10
<212>  PRT
<213>  Artificial sequence

<220>
<223>  HLA restricted peptide

<400>  351

Gly Tyr Glu Ile Trp Arg Asp Ile Asp Phe
1               5                   10

<210>  352
<211>  9
<212>  PRT
<213>  Artificial sequence

<220>
<223>  HLA restricted peptide

<400>  352

His Ala Phe Val Asp Ser Ile Phe Glu
1               5

<210>  353
<211>  10
<212>  PRT
<213>  Artificial sequence

<220>
<223>  HLA restricted peptide

<400>  353

His Ala Phe Val Asp Ser Ile Phe Glu Gln
1               5                   10

<210>  354
<211>  9
<212>  PRT
<213>  Artificial sequence

<220>
<223>  HLA restricted peptide

<400>  354

His Asp Lys Ser Arg Thr Pro Arg Leu
1               5

<210>  355
<211>  10
<212>  PRT
<213>  Artificial sequence

<220>
<223>  HLA restricted peptide

<400>  355

His Asp Lys Ser Arg Thr Pro Arg Leu Pro
1               5                   10

<210>  356
<211>  9
<212>  PRT
<213>  Artificial sequence

<220>
<223>  HLA restricted peptide

<400>  356

His Glu Ala Pro Ala Phe Leu Pro Trp
1               5


<210>  357
<211>  10
<212>  PRT
<213>  Artificial sequence

<220>
<223>  HLA restricted peptide

<400>  357

His Glu Ala Pro Ala Phe Leu Pro Trp His
1               5                   10


<210>  358
<211>  9
<212>  PRT
<213>  Artificial sequence

<220>
<223>  HLA restricted peptide

<400>  358

His Phe Pro Arg Ala Cys Val Ser Ser
1               5


<210>  359
<211>  10
<212>  PRT
<213>  Artificial sequence

<220>
<223>  HLA restricted peptide

<400>  359

His Phe Pro Arg Ala Cys Val Ser Ser Lys
1               5                   10


<210>  360
<211>  9
<212>  PRT
<213>  Artificial sequence

<220>
<223>  HLA restricted peptide

<400>  360

His His Ala Phe Val Asp Ser Ile Phe
1               5

<210>  361
<211>  9
<212>  PRT
<213>  Artificial sequence

<220>
<223>  HLA restricted peptide

<400>  361

His Ile Tyr Met Asp Gly Thr Met Ser
1               5


<210>  362
<211>  9
<212>  PRT
<213>  Artificial sequence

<220>
<223>  HLA restricted peptide

<400>  362

His Ile Tyr Met Asn Gly Thr Met Ser
1               5


<210>  363
<211>  10
<212>  PRT
<213>  Artificial sequence

<220>
<223>  HLA restricted peptide

<400>  363

His Lys Arg Lys Gln Leu Pro Glu Glu Lys
1               5                   10


<210>  364
<211>  9
<212>  PRT
<213>  Artificial sequence

<220>
<223>  HLA restricted peptide

<400>  364

His Asn Ala Leu His Ile Tyr Met Asn
1               5


<210>  365
<211>  10
<212>  PRT
<213>  Artificial sequence

<220>
<223>  HLA restricted peptide

<400>  365

His Asn Arg Glu Ser Tyr Met Val Pro Phe
1               5                   10


<210>  366
<211>  9
<212>  PRT
<213>  Artificial sequence

<220>
<223>  HLA restricted peptide

<400>  366

His Pro Thr Asn Pro Asn Leu Leu Ser
1               5


<210>  367
<211>  10
<212>  PRT
<213>  Artificial sequence

<220>
<223>  HLA restricted peptide

<400>  367

His Pro Thr Asn Pro Asn Leu Leu Ser Pro
1               5                   10


<210>  368
<211>  9
<212>  PRT
<213>  Artificial sequence

<220>
<223>  HLA restricted peptide

<400>  368

His Gln Ser Leu Cys Asn Gly Thr Pro
1               5


<210>  369
<211>  10
<212>  PRT
<213>  Artificial sequence

<220>
<223>  HLA restricted peptide

<400>  369

His Gln Ser Leu Cys Asn Gly Thr Pro Glu
1               5                   10


<210>  370
<211>  9
<212>  PRT
<213>  Artificial sequence

<220>
<223>  HLA restricted peptide

<400>  370

His Arg Leu Phe Leu Leu Arg Trp Glu
1               5


<210>  371
<211>  10
<212>  PRT
<213>  Artificial sequence

<220>
<223>  HLA restricted peptide

<400>  371

His Arg Leu Phe Leu Leu Arg Trp Glu Gln
1               5                   10


<210>  372
<211>  9
<212>  PRT
<213>  Artificial sequence

<220>
<223>  HLA restricted peptide

<400>  372

His Arg Pro Leu Gln Glu Val Tyr Pro
1               5


<210>  373
<211>  10
<212>  PRT
<213>  Artificial sequence

<220>
<223>  HLA restricted peptide

<400>  373

His Arg Pro Leu Gln Glu Val Tyr Pro Glu
1               5                   10


<210>  374
<211>  9
<212>  PRT
<213>  Artificial sequence

<220>
<223>  HLA restricted peptide

<400>  374

His Thr Ile Ser Ser Asp Tyr Val Ile
1               5

<210>  375
<211>  9
<212>  PRT
<213>  Artificial sequence

<220>
<223>  HLA restricted peptide

<400>  375

His Tyr Tyr Val Ser Met Asp Ala Leu
1               5


<210>  376
<211>  10
<212>  PRT
<213>  Artificial sequence

<220>
<223>  HLA restricted peptide

<400>  376

His Tyr Tyr Val Ser Met Asp Ala Leu Leu
1               5                   10


<210>  377
<211>  9
<212>  PRT
<213>  Artificial sequence

<220>
<223>  HLA restricted peptide

<400>  377

Ile Ala Asp Ala Ser Gln Ser Ser Met
1               5


<210>  378
<211>  10
<212>  PRT
<213>  Artificial sequence

<220>
<223>  HLA restricted peptide

<400>  378

Ile Ala Asp Ala Ser Gln Ser Ser Met His
1               5                   10


<210>  379
<211>  9
<212>  PRT
<213>  Artificial sequence

<220>
<223>  HLA restricted peptide

<400>  379

Ile Asp Phe Ala His Glu Ala Pro Ala
1               5


<210>  380
<211>  10
<212>  PRT
<213>  Artificial sequence

<220>
<223>  HLA restricted peptide

<400>  380

Ile Asp Phe Ala His Glu Ala Pro Ala Phe
1               5                   10


<210>  381
<211>  9
<212>  PRT
<213>  Artificial sequence

<220>
<223>  HLA restricted peptide

<400>  381

Ile Phe Asp Leu Ser Ala Pro Glu Lys
1               5


<210>  382
<211>  9
<212>  PRT
<213>  Artificial sequence

<220>
<223>  HLA restricted peptide

<400>  382

Ile Phe Glu Gln Trp Leu Arg Arg His
1               5


<210>  383
<211>  10
<212>  PRT
<213>  Artificial sequence

<220>
<223>  HLA restricted peptide

<400>  383

Ile Phe Glu Gln Trp Leu Arg Arg His Arg
1               5                   10


<210>  384
<211>  9
<212>  PRT

<213>  Artificial sequence

<220>
<223>  HLA restricted peptide

<400>  384

Ile Phe Leu Leu His His Ala Phe Val
1               5


<210>  385
<211>  9
<212>  PRT
<213>  Artificial sequence

<220>
<223>  HLA restricted peptide

<400>  385

Ile Gly His Asn Arg Glu Ser Tyr Met
1               5


<210>  386
<211>  10
<212>  PRT
<213>  Artificial sequence

<220>
<223>  HLA restricted peptide

<400>  386

Ile Gly His Asn Arg Glu Ser Tyr Met Val
1               5                   10


<210>  387
<211>  9
<212>  PRT
<213>  Artificial sequence

<220>
<223>  HLA restricted peptide

<400>  387

Ile Gly Thr Tyr Gly Gln Met Lys Asn
1               5


<210>  388
<211>  9
<212>  PRT
<213>  Artificial sequence

<220>
<223>  HLA restricted peptide

<400>  388

Ile His Tyr Tyr Val Ser Met Asp Ala
1               5

<210>  389
<211>  10
<212>  PRT
<213>  Artificial sequence

<220>
<223>  HLA restricted peptide

<400>  389

Ile His Tyr Tyr Val Ser Met Asp Ala Leu
1               5                   10


<210>  390
<211>  9
<212>  PRT
<213>  Artificial sequence

<220>
<223>  HLA restricted peptide

<400>  390

Ile Lys Ser Tyr Leu Glu Gln Ala Ser
1               5


<210>  391
<211>  9
<212>  PRT
<213>  Artificial sequence

<220>
<223>  HLA restricted peptide

<400>  391

Ile Asn Ile Tyr Asp Leu Phe Val Trp
1               5


<210>  392
<211>  10
<212>  PRT
<213>  Artificial sequence

<220>
<223>  HLA restricted peptide

<400>  392

Ile Asn Ile Tyr Asp Leu Phe Val Trp Ile
1               5                   10


<210>  393
<211>  9
<212>  PRT
<213>  Artificial sequence

<220>
<223>  HLA restricted peptide

<400>  393

Ile Pro Ile Gly Thr Tyr Gly Gln Met
1               5


<210>  394
<211>  10
<212>  PRT
<213>  Artificial sequence

<220>
<223>  HLA restricted peptide

<400>  394

Ile Pro Ile Gly Thr Tyr Gly Gln Met Lys
1               5                   10


<210>  395
<211>  9
<212>  PRT
<213>  Artificial sequence

<220>
<223>  HLA restricted peptide

<400>  395

Ile Pro Leu Tyr Arg Asn Gly Asp Phe
1               5


<210>  396
<211>  10
<212>  PRT
<213>  Artificial sequence

<220>
<223>  HLA restricted peptide

<400>  396

Ile Pro Leu Tyr Arg Asn Gly Asp Phe Phe
1               5                   10


<210>  397
<211>  9
<212>  PRT
<213>  Artificial sequence

<220>
<223>  HLA restricted peptide

<400>  397

Ile Pro Tyr Trp Asp Trp Arg Asp Ala
1               5


<210>  398
<211>  10

<212>  PRT
<213>  Artificial sequence

<220>
<223>  HLA restricted peptide

<400>  398

Ile Pro Tyr Trp Asp Trp Arg Asp Ala Glu
1               5                   10


<210>  399
<211>  9
<212>  PRT
<213>  Artificial sequence

<220>
<223>  HLA restricted peptide

<400>  399

Ile Gln Lys Leu Thr Gly Asp Glu Asn
1               5


<210>  400
<211>  10
<212>  PRT
<213>  Artificial sequence

<220>
<223>  HLA restricted peptide

<400>  400

Ile Gln Lys Leu Thr Gly Asp Glu Asn Phe
1               5                   10


<210>  401
<211>  9
<212>  PRT
<213>  Artificial sequence

<220>
<223>  HLA restricted peptide

<400>  401

Ile Ser Ser Asp Tyr Val Ile Pro Ile
1               5


<210>  402
<211>  10
<212>  PRT
<213>  Artificial sequence

<220>
<223>  HLA restricted peptide

<400>  402

Ile Ser Ser Lys Asp Leu Gly Tyr Asp Tyr

1                   5                   10


<210>  403
<211>  9
<212>  PRT
<213>  Artificial sequence

<220>
<223>  HLA restricted peptide

<400>  403

Ile Val Cys Ser Arg Leu Glu Glu Tyr
1                   5


<210>  404
<211>  10
<212>  PRT
<213>  Artificial sequence

<220>
<223>  HLA restricted peptide

<400>  404

Ile Val Cys Ser Arg Leu Glu Glu Tyr Asn
1                   5                   10


<210>  405
<211>  9
<212>  PRT
<213>  Artificial sequence

<220>
<223>  HLA restricted peptide

<400>  405

Ile Trp Ser Trp Leu Leu Gly Ala Ala
1                   5


<210>  406
<211>  10
<212>  PRT
<213>  Artificial sequence

<220>
<223>  HLA restricted peptide

<400>  406

Ile Trp Ser Trp Leu Leu Gly Ala Ala Met
1                   5                   10


<210>  407
<211>  9
<212>  PRT
<213>  Artificial sequence

<220>

<223>  HLA restricted peptide

<400>  407

Ile Tyr Asp Leu Phe Val Trp Ile His
1               5

<210>  408
<211>  10
<212>  PRT
<213>  Artificial sequence

<220>
<223>  HLA restricted peptide

<400>  408

Ile Tyr Asp Leu Phe Val Trp Ile His Tyr
1               5                   10

<210>  409
<211>  10
<212>  PRT
<213>  Artificial sequence

<220>
<223>  HLA restricted peptide

<400>  409

Ile Tyr Met Asp Gly Thr Met Ser Gln Val
1               5                   10

<210>  410
<211>  10
<212>  PRT
<213>  Artificial sequence

<220>
<223>  HLA restricted peptide

<400>  410

Ile Tyr Met Asn Gly Thr Met Ser Gln Val
1               5                   10

<210>  411
<211>  9
<212>  PRT
<213>  Artificial sequence

<220>
<223>  HLA restricted peptide

<400>  411

Lys Ala Ala Asn Phe Ser Phe Arg Asn
1               5

<210>  412

<211>  10
<212>  PRT
<213>  Artificial sequence

<220>
<223>  HLA restricted peptide

<400>  412

Lys Ala Ala Asn Phe Ser Phe Arg Asn Thr
1               5                   10


<210>  413
<211>  9
<212>  PRT
<213>  Artificial sequence

<220>
<223>  HLA restricted peptide

<400>  413

Lys Cys Asp Ile Cys Thr Asp Glu Tyr
1               5


<210>  414
<211>  10
<212>  PRT
<213>  Artificial sequence

<220>
<223>  HLA restricted peptide

<400>  414

Lys Cys Asp Ile Cys Thr Asp Glu Tyr Met
1               5                   10


<210>  415
<211>  9
<212>  PRT
<213>  Artificial sequence

<220>
<223>  HLA restricted peptide

<400>  415

Lys Asp Lys Phe Phe Ala Tyr Leu Thr
1               5


<210>  416
<211>  10
<212>  PRT
<213>  Artificial sequence

<220>
<223>  HLA restricted peptide

<400>  416

Lys Asp Lys Phe Phe Ala Tyr Leu Thr Leu
1               5                   10


<210>  417
<211>  9
<212>  PRT
<213>  Artificial sequence

<220>
<223>  HLA restricted peptide

<400>  417

Lys Asp Leu Gly Tyr Asp Tyr Ser Tyr
1               5


<210>  418
<211>  10
<212>  PRT
<213>  Artificial sequence

<220>
<223>  HLA restricted peptide

<400>  418

Lys Asp Leu Gly Tyr Asp Tyr Ser Tyr Leu
1               5                   10


<210>  419
<211>  10
<212>  PRT
<213>  Artificial sequence

<220>
<223>  HLA restricted peptide

<400>  419

Lys Glu Asp Tyr His Ser Leu Tyr Gln Ser
1               5                   10


<210>  420
<211>  9
<212>  PRT
<213>  Artificial sequence

<220>
<223>  HLA restricted peptide

<400>  420

Lys Phe Phe Ala Tyr Leu Thr Leu Ala
1               5


<210>  421
<211>  10
<212>  PRT
<213>  Artificial sequence

<220>
<223>  HLA restricted peptide

<400>  421

Lys Phe Phe Ala Tyr Leu Thr Leu Ala Lys
1               5                   10


<210>  422
<211>  9
<212>  PRT
<213>  Artificial sequence

<220>
<223>  HLA restricted peptide

<400>  422

Lys Phe Gly Phe Trp Gly Pro Asn Cys
1               5


<210>  423
<211>  10
<212>  PRT
<213>  Artificial sequence

<220>
<223>  HLA restricted peptide

<400>  423

Lys Phe Gly Phe Trp Gly Pro Asn Cys Thr
1               5                   10


<210>  424
<211>  9
<212>  PRT
<213>  Artificial sequence

<220>
<223>  HLA restricted peptide

<400>  424

Lys His Thr Ile Ser Ser Asp Tyr Val
1               5


<210>  425
<211>  10
<212>  PRT
<213>  Artificial sequence

<220>
<223>  HLA restricted peptide

<400>  425

Lys His Thr Ile Ser Ser Asp Tyr Val Ile
1               5                   10

<210>  426
<211>  9
<212>  PRT
<213>  Artificial sequence

<220>
<223>  HLA restricted peptide

<400>  426

Lys Leu Thr Gly Asp Glu Asn Phe Thr
1               5


<210>  427
<211>  10
<212>  PRT
<213>  Artificial sequence

<220>
<223>  HLA restricted peptide

<400>  427

Lys Leu Thr Gly Asp Glu Asn Phe Thr Ile
1               5                   10


<210>  428
<211>  9
<212>  PRT
<213>  Artificial sequence

<220>
<223>  HLA restricted peptide

<400>  428

Lys Asn Gly Ser Thr Pro Met Phe Asn
1               5


<210>  429
<211>  9
<212>  PRT
<213>  Artificial sequence

<220>
<223>  HLA restricted peptide

<400>  429

Lys Asn Leu Met Glu Lys Glu Cys Cys
1               5


<210>  430
<211>  9
<212>  PRT
<213>  Artificial sequence

<220>
<223>  HLA restricted peptide

<400>  430

Lys Gln Leu Pro Glu Glu Lys Gln Pro
1               5

<210>  431
<211>  10
<212>  PRT
<213>  Artificial sequence

<220>
<223>  HLA restricted peptide

<400>  431

Lys Gln Leu Pro Glu Glu Lys Gln Pro Leu
1               5                   10

<210>  432
<211>  9
<212>  PRT
<213>  Artificial sequence

<220>
<223>  HLA restricted peptide

<400>  432

Lys Gln Pro Leu Leu Met Glu Lys Glu
1               5

<210>  433
<211>  10
<212>  PRT
<213>  Artificial sequence

<220>
<223>  HLA restricted peptide

<400>  433

Lys Gln Pro Leu Leu Met Glu Lys Glu Asp
1               5                   10

<210>  434
<211>  9
<212>  PRT
<213>  Artificial sequence

<220>
<223>  HLA restricted peptide

<400>  434

Lys Arg Lys Gln Leu Pro Glu Glu Lys
1               5

<210>  435
<211>  10
<212>  PRT
<213>  Artificial sequence

<220>
<223>  HLA restricted peptide

<400>  435

Lys Arg Lys Gln Leu Pro Glu Glu Lys Gln
1               5                   10


<210>  436
<211>  9
<212>  PRT
<213>  Artificial sequence

<220>
<223>  HLA restricted peptide

<400>  436

Lys Ser Arg Thr Pro Arg Leu Pro Ser
1               5


<210>  437
<211>  10
<212>  PRT
<213>  Artificial sequence

<220>
<223>  HLA restricted peptide

<400>  437

Lys Ser Arg Thr Pro Arg Leu Pro Ser Ser
1               5                   10


<210>  438
<211>  9
<212>  PRT
<213>  Artificial sequence

<220>
<223>  HLA restricted peptide

<400>  438

Lys Ser Tyr Leu Glu Gln Ala Ser Arg
1               5


<210>  439
<211>  10
<212>  PRT
<213>  Artificial sequence

<220>
<223>  HLA restricted peptide

<400>  439

Lys Ser Tyr Leu Glu Gln Ala Ser Arg Ile
1               5                   10

<210>  440
<211>  9
<212>  PRT
<213>  Artificial sequence

<220>
<223>  HLA restricted peptide

<400>  440

Leu Ala Gly Leu Val Ser Leu Leu Cys
1               5


<210>  441
<211>  10
<212>  PRT
<213>  Artificial sequence

<220>
<223>  HLA restricted peptide

<400>  441

Leu Ala Gly Leu Val Ser Leu Leu Cys Arg
1               5                   10


<210>  442
<211>  10
<212>  PRT
<213>  Artificial sequence

<220>
<223>  HLA restricted peptide

<400>  442

Leu Ala Lys His Thr Ile Ser Ser Asp Tyr
1               5                   10


<210>  443
<211>  9
<212>  PRT
<213>  Artificial sequence

<220>
<223>  HLA restricted peptide

<400>  443

Leu Ala Val Leu Tyr Cys Leu Leu Trp
1               5


<210>  444
<211>  10
<212>  PRT
<213>  Artificial sequence

<220>
<223>  HLA restricted peptide

<400>  444

Leu Ala Val Leu Tyr Cys Leu Leu Trp Ser
1               5                   10


<210>  445
<211>  10
<212>  PRT
<213>  Artificial sequence

<220>
<223>  HLA restricted peptide

<400>  445

Leu Cys Asn Gly Thr Pro Glu Gly Pro Leu
1               5                   10


<210>  446
<211>  9
<212>  PRT
<213>  Artificial sequence

<220>
<223>  HLA restricted peptide

<400>  446

Leu Cys Arg His Lys Arg Lys Gln Leu
1               5


<210>  447
<211>  10
<212>  PRT
<213>  Artificial sequence

<220>
<223>  HLA restricted peptide

<400>  447

Leu Cys Arg His Lys Arg Lys Gln Leu Pro
1               5                   10


<210>  448
<211>  9
<212>  PRT
<213>  Artificial sequence

<220>
<223>  HLA restricted peptide

<400>  448

Leu Glu Glu Tyr Asn Ser His Gln Ser
1               5


<210>  449
<211>  10
<212>  PRT

<213>  Artificial sequence

<220>
<223>  HLA restricted peptide

<400>  449

Leu Glu Glu Tyr Asn Ser His Gln Ser Leu
1               5                   10


<210>  450
<211>  9
<212>  PRT
<213>  Artificial sequence

<220>
<223>  HLA restricted peptide

<400>  450

Leu Glu Gly Phe Ala Ser Pro Leu Thr
1               5


<210>  451
<211>  9
<212>  PRT
<213>  Artificial sequence

<220>
<223>  HLA restricted peptide

<400>  451

Leu Glu Gln Ala Ser Arg Ile Trp Ser
1               5


<210>  452
<211>  10
<212>  PRT
<213>  Artificial sequence

<220>
<223>  HLA restricted peptide

<400>  452

Leu Glu Gln Ala Ser Arg Ile Trp Ser Trp
1               5                   10


<210>  453
<211>  10
<212>  PRT
<213>  Artificial sequence

<220>
<223>  HLA restricted peptide

<400>  453

Leu Phe Leu Leu Arg Trp Glu Gln Glu Ile
1               5                   10

<210>  454
<211>  9
<212>  PRT
<213>  Artificial sequence

<220>
<223>  HLA restricted peptide

<400>  454

Leu Phe Val Trp Ile His Tyr Tyr Val
1               5


<210>  455
<211>  10
<212>  PRT
<213>  Artificial sequence

<220>
<223>  HLA restricted peptide

<400>  455

Leu Phe Val Trp Ile His Tyr Tyr Val Ser
1               5                   10


<210>  456
<211>  9
<212>  PRT
<213>  Artificial sequence

<220>
<223>  HLA restricted peptide

<400>  456

Leu Gly Ala Ala Met Val Gly Ala Val
1               5


<210>  457
<211>  10
<212>  PRT
<213>  Artificial sequence

<220>
<223>  HLA restricted peptide

<400>  457

Leu Gly Ala Ala Met Val Gly Ala Val Leu
1               5                   10


<210>  458
<211>  10
<212>  PRT
<213>  Artificial sequence

<220>
<223>  HLA restricted peptide

<400>  458

Leu Gly Gly Tyr Glu Ile Trp Arg Asp Ile
1               5                   10


<210>  459
<211>  10
<212>  PRT
<213>  Artificial sequence

<220>
<223>  HLA restricted peptide

<400>  459

Leu Gly Pro Gln Phe Pro Phe Thr Gly Val
1               5                   10


<210>  460
<211>  9
<212>  PRT
<213>  Artificial sequence

<220>
<223>  HLA restricted peptide

<400>  460

Leu Gly Tyr Asp Tyr Ser Tyr Leu Gln
1               5


<210>  461
<211>  10
<212>  PRT
<213>  Artificial sequence

<220>
<223>  HLA restricted peptide

<400>  461

Leu Gly Tyr Asp Tyr Ser Tyr Leu Gln Asp
1               5                   10


<210>  462
<211>  9
<212>  PRT
<213>  Artificial sequence

<220>
<223>  HLA restricted peptide

<400>  462

Leu His His Ala Phe Val Asp Ser Ile
1               5


<210>  463
<211>  10

<212>  PRT
<213>  Artificial sequence

<220>
<223>  HLA restricted peptide

<400>  463

Leu His His Ala Phe Val Asp Ser Ile Phe
1               5                   10


<210>  464
<211>  9
<212>  PRT
<213>  Artificial sequence

<220>
<223>  HLA restricted peptide

<400>  464

Leu His Ile Tyr Met Asp Gly Thr Met
1               5


<210>  465
<211>  10
<212>  PRT
<213>  Artificial sequence

<220>
<223>  HLA restricted peptide

<400>  465

Leu His Ile Tyr Met Asp Gly Thr Met Ser
1               5                   10


<210>  466
<211>  9
<212>  PRT
<213>  Artificial sequence

<220>
<223>  HLA restricted peptide

<400>  466

Leu His Ile Tyr Met Asn Gly Thr Met
1               5


<210>  467
<211>  10
<212>  PRT
<213>  Artificial sequence

<220>
<223>  HLA restricted peptide

<400>  467

Leu His Ile Tyr Met Asn Gly Thr Met Ser

1                   5                   10


<210>  468
<211>  9
<212>  PRT
<213>  Artificial sequence

<220>
<223>  HLA restricted peptide

<400>  468

Leu Leu Ala Gly Leu Val Ser Leu Leu
1               5


<210>  469
<211>  10
<212>  PRT
<213>  Artificial sequence

<220>
<223>  HLA restricted peptide

<400>  469

Leu Leu Ala Gly Leu Val Ser Leu Leu Cys
1               5                   10


<210>  470
<211>  9
<212>  PRT
<213>  Artificial sequence

<220>
<223>  HLA restricted peptide

<400>  470

Leu Leu Ala Val Leu Tyr Cys Leu Leu
1               5


<210>  471
<211>  10
<212>  PRT
<213>  Artificial sequence

<220>
<223>  HLA restricted peptide

<400>  471

Leu Leu Ala Val Leu Tyr Cys Leu Leu Trp
1               5                   10


<210>  472
<211>  10
<212>  PRT
<213>  Artificial sequence

<220>

<223>  HLA restricted peptide

<400>  472

Leu Leu Cys Arg His Lys Arg Lys Gln Leu
1               5                   10


<210>  473
<211>  9
<212>  PRT
<213>  Artificial sequence

<220>
<223>  HLA restricted peptide

<400>  473

Leu Leu Gly Ala Ala Met Val Gly Ala
1               5


<210>  474
<211>  10
<212>  PRT
<213>  Artificial sequence

<220>
<223>  HLA restricted peptide

<400>  474

Leu Leu Gly Ala Ala Met Val Gly Ala Val
1               5                   10


<210>  475
<211>  9
<212>  PRT
<213>  Artificial sequence

<220>
<223>  HLA restricted peptide

<400>  475

Leu Leu Gly Gly Tyr Glu Ile Trp Arg
1               5


<210>  476
<211>  9
<212>  PRT
<213>  Artificial sequence

<220>
<223>  HLA restricted peptide

<400>  476

Leu Leu His His Ala Phe Val Asp Ser
1               5


<210>  477

<211>  10
<212>  PRT
<213>  Artificial sequence

<220>
<223>  HLA restricted peptide

<400>  477

Leu Leu His His Ala Phe Val Asp Ser Ile
1               5                   10


<210>  478
<211>  9
<212>  PRT
<213>  Artificial sequence

<220>
<223>  HLA restricted peptide

<400>  478

Leu Leu Met Glu Lys Glu Asp Tyr His
1               5


<210>  479
<211>  10
<212>  PRT
<213>  Artificial sequence

<220>
<223>  HLA restricted peptide

<400>  479

Leu Leu Met Glu Lys Glu Asp Tyr His Ser
1               5                   10


<210>  480
<211>  10
<212>  PRT
<213>  Artificial sequence

<220>
<223>  HLA restricted peptide

<400>  480

Leu Leu Arg Trp Glu Gln Glu Ile Gln Lys
1               5                   10


<210>  481
<211>  9
<212>  PRT
<213>  Artificial sequence

<220>
<223>  HLA restricted peptide

<400>  481

Leu Leu Ser Pro Ala Ser Phe Phe Ser
1               5

<210>  482
<211>  10
<212>  PRT
<213>  Artificial sequence

<220>
<223>  HLA restricted peptide

<400>  482

Leu Leu Ser Pro Ala Ser Phe Phe Ser Ser
1               5                   10

<210>  483
<211>  10
<212>  PRT
<213>  Artificial sequence

<220>
<223>  HLA restricted peptide

<400>  483

Leu Leu Val Arg Arg Asn Ile Phe Asp Leu
1               5                   10

<210>  484
<211>  9
<212>  PRT
<213>  Artificial sequence

<220>
<223>  HLA restricted peptide

<400>  484

Leu Leu Trp Ser Phe Gln Thr Ser Ala
1               5

<210>  485
<211>  10
<212>  PRT
<213>  Artificial sequence

<220>
<223>  HLA restricted peptide

<400>  485

Leu Leu Trp Ser Phe Gln Thr Ser Ala Gly
1               5                   10

<210>  486
<211>  10
<212>  PRT
<213>  Artificial sequence

<220>
<223>  HLA restricted peptide

<400>  486

Leu Met Glu Lys Glu Cys Cys Pro Pro Trp
1               5                   10


<210>  487
<211>  9
<212>  PRT
<213>  Artificial sequence

<220>
<223>  HLA restricted peptide

<400>  487

Leu Met Glu Lys Glu Asp Tyr His Ser
1               5


<210>  488
<211>  10
<212>  PRT
<213>  Artificial sequence

<220>
<223>  HLA restricted peptide

<400>  488

Leu Met Glu Lys Glu Asp Tyr His Ser Leu
1               5                   10


<210>  489
<211>  9
<212>  PRT
<213>  Artificial sequence

<220>
<223>  HLA restricted peptide

<400>  489

Leu Pro Glu Glu Lys Gln Pro Leu Leu
1               5


<210>  490
<211>  10
<212>  PRT
<213>  Artificial sequence

<220>
<223>  HLA restricted peptide

<400>  490

Leu Pro Glu Glu Lys Gln Pro Leu Leu Met
1               5                   10

<210>  491
<211>  9
<212>  PRT
<213>  Artificial sequence

<220>
<223>  HLA restricted peptide

<400>  491

Leu Pro Ser Ser Ala Asp Val Glu Phe
1               5


<210>  492
<211>  10
<212>  PRT
<213>  Artificial sequence

<220>
<223>  HLA restricted peptide

<400>  492

Leu Pro Ser Ser Ala Asp Val Glu Phe Cys
1               5                   10


<210>  493
<211>  9
<212>  PRT
<213>  Artificial sequence

<220>
<223>  HLA restricted peptide

<400>  493

Leu Pro Trp His Arg Leu Phe Leu Leu
1               5


<210>  494
<211>  10
<212>  PRT
<213>  Artificial sequence

<220>
<223>  HLA restricted peptide

<400>  494

Leu Pro Trp His Arg Leu Phe Leu Leu Arg
1               5                   10


<210>  495
<211>  9
<212>  PRT
<213>  Artificial sequence

<220>
<223>  HLA restricted peptide

<400>  495

Leu Gln Asp Ser Asp Pro Asp Ser Phe
1               5

<210>  496
<211>  10
<212>  PRT
<213>  Artificial sequence

<220>
<223>  HLA restricted peptide

<400>  496

Leu Gln Asp Ser Asp Pro Asp Ser Phe Gln
1               5                   10

<210>  497
<211>  9
<212>  PRT
<213>  Artificial sequence

<220>
<223>  HLA restricted peptide

<400>  497

Leu Gln Glu Val Tyr Pro Glu Ala Asn
1               5

<210>  498
<211>  10
<212>  PRT
<213>  Artificial sequence

<220>
<223>  HLA restricted peptide

<400>  498

Leu Gln Glu Val Tyr Pro Glu Ala Asn Ala
1               5                   10

<210>  499
<211>  9
<212>  PRT
<213>  Artificial sequence

<220>
<223>  HLA restricted peptide

<400>  499

Leu Arg Arg His Arg Pro Leu Gln Glu
1               5

<210>  500
<211>  10
<212>  PRT
<213>  Artificial sequence

<220>
<223>  HLA restricted peptide

<400>  500

Leu Arg Arg His Arg Pro Leu Gln Glu Val
1               5                   10


<210>  501
<211>  9
<212>  PRT
<213>  Artificial sequence

<220>
<223>  HLA restricted peptide

<400>  501

Leu Arg Arg Asn Pro Gly Asn His Asp
1               5


<210>  502
<211>  10
<212>  PRT
<213>  Artificial sequence

<220>
<223>  HLA restricted peptide

<400>  502

Leu Arg Arg Asn Pro Gly Asn His Asp Lys
1               5                   10


<210>  503
<211>  9
<212>  PRT
<213>  Artificial sequence

<220>
<223>  HLA restricted peptide

<400>  503

Leu Arg Trp Glu Gln Glu Ile Gln Lys
1               5


<210>  504
<211>  10
<212>  PRT
<213>  Artificial sequence

<220>
<223>  HLA restricted peptide

<400>  504

Leu Arg Trp Glu Gln Glu Ile Gln Lys Leu
1               5                   10

<210>  505
<211>  9
<212>  PRT
<213>  Artificial sequence

<220>
<223>  HLA restricted peptide

<400>  505

Leu Ser Ala Pro Glu Lys Asp Lys Phe
1               5


<210>  506
<211>  10
<212>  PRT
<213>  Artificial sequence

<220>
<223>  HLA restricted peptide

<400>  506

Leu Ser Ala Pro Glu Lys Asp Lys Phe Phe
1               5                   10


<210>  507
<211>  9
<212>  PRT
<213>  Artificial sequence

<220>
<223>  HLA restricted peptide

<400>  507

Leu Ser Gly Arg Gly Ser Cys Gln Asn
1               5


<210>  508
<211>  10
<212>  PRT
<213>  Artificial sequence

<220>
<223>  HLA restricted peptide

<400>  508

Leu Ser Gly Arg Gly Ser Cys Gln Asn Ile
1               5                   10


<210>  509
<211>  10
<212>  PRT
<213>  Artificial sequence

<220>
<223>  HLA restricted peptide

<400>  509

Leu Ser Leu Thr Gln Tyr Glu Ser Gly Ser
1               5                   10


<210>  510
<211>  10
<212>  PRT
<213>  Artificial sequence

<220>
<223>  HLA restricted peptide

<400>  510

Leu Ser Asn Ala Pro Leu Gly Pro Gln Phe
1               5                   10


<210>  511
<211>  9
<212>  PRT
<213>  Artificial sequence

<220>
<223>  HLA restricted peptide

<400>  511

Leu Ser Pro Ala Ser Phe Phe Ser Ser
1               5


<210>  512
<211>  10
<212>  PRT
<213>  Artificial sequence

<220>
<223>  HLA restricted peptide

<400>  512

Leu Ser Pro Ala Ser Phe Phe Ser Ser Trp
1               5                   10


<210>  513
<211>  9
<212>  PRT
<213>  Artificial sequence

<220>
<223>  HLA restricted peptide

<400>  513

Leu Thr Ala Leu Leu Ala Gly Leu Val
1               5


<210>  514
<211>  10
<212>  PRT

<213>  Artificial sequence

<220>
<223>  HLA restricted peptide

<400>  514

Leu Thr Ala Leu Leu Ala Gly Leu Val Ser
1               5                   10


<210>  515
<211>  9
<212>  PRT
<213>  Artificial sequence

<220>
<223>  HLA restricted peptide

<400>  515

Leu Thr Gly Asp Glu Asn Phe Thr Ile
1               5


<210>  516
<211>  10
<212>  PRT
<213>  Artificial sequence

<220>
<223>  HLA restricted peptide

<400>  516

Leu Thr Gly Ile Ala Asp Ala Ser Gln Ser
1               5                   10


<210>  517
<211>  9
<212>  PRT
<213>  Artificial sequence

<220>
<223>  HLA restricted peptide

<400>  517

Leu Thr Leu Ala Lys His Thr Ile Ser
1               5


<210>  518
<211>  10
<212>  PRT
<213>  Artificial sequence

<220>
<223>  HLA restricted peptide

<400>  518

Leu Thr Leu Ala Lys His Thr Ile Ser Ser
1               5                   10

<210>  519
<211>  9
<212>  PRT
<213>  Artificial sequence

<220>
<223>  HLA restricted peptide

<400>  519

Leu Thr Gln Tyr Glu Ser Gly Ser Met
1               5


<210>  520
<211>  9
<212>  PRT
<213>  Artificial sequence

<220>
<223>  HLA restricted peptide

<400>  520

Leu Val Arg Arg Asn Ile Phe Asp Leu
1               5


<210>  521
<211>  10
<212>  PRT
<213>  Artificial sequence

<220>
<223>  HLA restricted peptide

<400>  521

Leu Val Arg Arg Asn Ile Phe Asp Leu Ser
1               5                   10


<210>  522
<211>  9
<212>  PRT
<213>  Artificial sequence

<220>
<223>  HLA restricted peptide

<400>  522

Leu Val Ser Leu Leu Cys Arg His Lys
1               5


<210>  523
<211>  10
<212>  PRT
<213>  Artificial sequence

<220>
<223>  HLA restricted peptide

<400>  523

Leu Val Ser Leu Leu Cys Arg His Lys Arg
1               5                   10


<210>  524
<211>  10
<212>  PRT
<213>  Artificial sequence

<220>
<223>  HLA restricted peptide

<400>  524

Leu Tyr Cys Leu Leu Trp Ser Phe Gln Thr
1               5                   10


<210>  525
<211>  9
<212>  PRT
<213>  Artificial sequence

<220>
<223>  HLA restricted peptide

<400>  525

Leu Tyr Arg Asn Gly Asp Phe Phe Ile
1               5


<210>  526
<211>  10
<212>  PRT
<213>  Artificial sequence

<220>
<223>  HLA restricted peptide

<400>  526

Leu Tyr Arg Asn Gly Asp Phe Phe Ile Ser
1               5                   10


<210>  527
<211>  10
<212>  PRT
<213>  Artificial sequence

<220>
<223>  HLA restricted peptide

<400>  527

Met Asp Ala Leu Leu Gly Gly Tyr Glu Ile
1               5                   10


<210>  528
<211>  9

<212>  PRT
<213>  Artificial sequence

<220>
<223>  HLA restricted peptide

<400>  528

Met Asp Lys Ala Ala Asn Phe Ser Phe
1               5


<210>  529
<211>  10
<212>  PRT
<213>  Artificial sequence

<220>
<223>  HLA restricted peptide

<400>  529

Met Asp Lys Ala Ala Asn Phe Ser Phe Arg
1               5                   10


<210>  530
<211>  9
<212>  PRT
<213>  Artificial sequence

<220>
<223>  HLA restricted peptide

<400>  530

Met Glu Lys Glu Cys Cys Pro Pro Trp
1               5


<210>  531
<211>  10
<212>  PRT
<213>  Artificial sequence

<220>
<223>  HLA restricted peptide

<400>  531

Met Glu Lys Glu Cys Cys Pro Pro Trp Ser
1               5                   10


<210>  532
<211>  9
<212>  PRT
<213>  Artificial sequence

<220>
<223>  HLA restricted peptide

<400>  532

Met Glu Lys Glu Asp Tyr His Ser Leu

1               5


<210>  533
<211>  10
<212>  PRT
<213>  Artificial sequence

<220>
<223>  HLA restricted peptide

<400>  533

Met Glu Lys Glu Asp Tyr His Ser Leu Tyr
1               5                   10


<210>  534
<211>  10
<212>  PRT
<213>  Artificial sequence

<220>
<223>  HLA restricted peptide

<400>  534

Met Phe Asn Asp Ile Asn Ile Tyr Asp Leu
1               5                   10


<210>  535
<211>  9
<212>  PRT
<213>  Artificial sequence

<220>
<223>  HLA restricted peptide

<400>  535

Met Gly Phe Asn Cys Gly Asn Cys Lys
1               5


<210>  536
<211>  10
<212>  PRT
<213>  Artificial sequence

<220>
<223>  HLA restricted peptide

<400>  536

Met Gly Phe Asn Cys Gly Asn Cys Lys Phe
1               5                   10


<210>  537
<211>  10
<212>  PRT
<213>  Artificial sequence

<220>

<223>  HLA restricted peptide

<400>  537

Met Gly Gly Gln His Pro Thr Asn Pro Asn
1               5                   10


<210>  538
<211>  9
<212>  PRT
<213>  Artificial sequence

<220>
<223>  HLA restricted peptide

<400>  538

Met His Asn Ala Leu His Ile Tyr Met
1               5


<210>  539
<211>  10
<212>  PRT
<213>  Artificial sequence

<220>
<223>  HLA restricted peptide

<400>  539

Met His Asn Ala Leu His Ile Tyr Met Asn
1               5                   10


<210>  540
<211>  9
<212>  PRT
<213>  Artificial sequence

<220>
<223>  HLA restricted peptide

<400>  540

Met Lys Asn Gly Ser Thr Pro Met Phe
1               5


<210>  541
<211>  10
<212>  PRT
<213>  Artificial sequence

<220>
<223>  HLA restricted peptide

<400>  541

Met Lys Asn Gly Ser Thr Pro Met Phe Asn
1               5                   10


<210>  542

<211>  9
<212>  PRT
<213>  Artificial sequence

<220>
<223>  HLA restricted peptide

<400>  542

Met Leu Leu Ala Val Leu Tyr Cys Leu
1               5


<210>  543
<211>  10
<212>  PRT
<213>  Artificial sequence

<220>
<223>  HLA restricted peptide

<400>  543

Met Leu Leu Ala Val Leu Tyr Cys Leu Leu
1               5                   10


<210>  544
<211>  9
<212>  PRT
<213>  Artificial sequence

<220>
<223>  HLA restricted peptide

<400>  544

Met Ser Gln Val Gln Gly Ser Ala Asn
1               5


<210>  545
<211>  9
<212>  PRT
<213>  Artificial sequence

<220>
<223>  HLA restricted peptide

<400>  545

Met Val Gly Ala Val Leu Thr Ala Leu
1               5


<210>  546
<211>  10
<212>  PRT
<213>  Artificial sequence

<220>
<223>  HLA restricted peptide

<400>  546

Met Val Gly Ala Val Leu Thr Ala Leu Leu
1               5                   10


<210>  547
<211>  9
<212>  PRT
<213>  Artificial sequence

<220>
<223>  HLA restricted peptide

<400>  547

Met Val Pro Phe Ile Pro Leu Tyr Arg
1               5


<210>  548
<211>  10
<212>  PRT
<213>  Artificial sequence

<220>
<223>  HLA restricted peptide

<400>  548

Met Val Pro Phe Ile Pro Leu Tyr Arg Asn
1               5                   10


<210>  549
<211>  9
<212>  PRT
<213>  Artificial sequence

<220>
<223>  HLA restricted peptide

<400>  549

Asn Ala Leu His Ile Tyr Met Asp Gly
1               5


<210>  550
<211>  10
<212>  PRT
<213>  Artificial sequence

<220>
<223>  HLA restricted peptide

<400>  550

Asn Ala Leu His Ile Tyr Met Asp Gly Thr
1               5                   10


<210>  551
<211>  9
<212>  PRT
<213>  Artificial sequence

<220>
<223>  HLA restricted peptide

<400>  551

Asn Ala Leu His Ile Tyr Met Asn Gly
1               5


<210>  552
<211>  10
<212>  PRT
<213>  Artificial sequence

<220>
<223>  HLA restricted peptide

<400>  552

Asn Ala Leu His Ile Tyr Met Asn Gly Thr
1               5                   10


<210>  553
<211>  9
<212>  PRT
<213>  Artificial sequence

<220>
<223>  HLA restricted peptide

<400>  553

Asn Ala Pro Ile Gly His Asn Arg Glu
1               5


<210>  554
<211>  10
<212>  PRT
<213>  Artificial sequence

<220>
<223>  HLA restricted peptide

<400>  554

Asn Ala Pro Ile Gly His Asn Arg Glu Ser
1               5                   10


<210>  555
<211>  10
<212>  PRT
<213>  Artificial sequence

<220>
<223>  HLA restricted peptide

<400>  555

Asn Ala Pro Leu Gly Pro Gln Phe Pro Phe
1               5                   10

<210> 556
<211> 9
<212> PRT
<213> Artificial sequence

<220>
<223> HLA restricted peptide

<400> 556

Asn Cys Gly Asn Cys Lys Phe Gly Phe
1               5


<210> 557
<211> 10
<212> PRT
<213> Artificial sequence

<220>
<223> HLA restricted peptide

<400> 557

Asn Cys Gly Asn Cys Lys Phe Gly Phe Trp
1               5                   10


<210> 558
<211> 10
<212> PRT
<213> Artificial sequence

<220>
<223> HLA restricted peptide

<400> 558

Asn Cys Lys Phe Gly Phe Trp Gly Pro Asn
1               5                   10


<210> 559
<211> 9
<212> PRT
<213> Artificial sequence

<220>
<223> HLA restricted peptide

<400> 559

Asn Cys Thr Glu Arg Arg Leu Leu Val
1               5


<210> 560
<211> 10
<212> PRT
<213> Artificial sequence

<220>
<223> HLA restricted peptide

<400> 560

Asn Cys Thr Glu Arg Arg Leu Leu Val Arg
1               5                   10


<210>  561
<211>  9
<212>  PRT
<213>  Artificial sequence

<220>
<223>  HLA restricted peptide

<400>  561

Asn Asp Ile Asn Ile Tyr Asp Leu Phe
1               5


<210>  562
<211>  10
<212>  PRT
<213>  Artificial sequence

<220>
<223>  HLA restricted peptide

<400>  562

Asn Asp Ile Asn Ile Tyr Asp Leu Phe Val
1               5                   10


<210>  563
<211>  10
<212>  PRT
<213>  Artificial sequence

<220>
<223>  HLA restricted peptide

<400>  563

Asn Asp Pro Ile Phe Leu Leu His His Ala
1               5                   10


<210>  564
<211>  9
<212>  PRT
<213>  Artificial sequence

<220>
<223>  HLA restricted peptide

<400>  564

Asn Phe Met Gly Phe Asn Cys Gly Asn
1               5


<210>  565
<211>  10
<212>  PRT
<213>  Artificial sequence

<220>
<223>  HLA restricted peptide

<400>  565

Asn Phe Met Gly Phe Asn Cys Gly Asn Cys
1               5                   10


<210>  566
<211>  9
<212>  PRT
<213>  Artificial sequence

<220>
<223>  HLA restricted peptide

<400>  566

Asn Phe Thr Ile Pro Tyr Trp Asp Trp
1               5


<210>  567
<211>  10
<212>  PRT
<213>  Artificial sequence

<220>
<223>  HLA restricted peptide

<400>  567

Asn Phe Thr Ile Pro Tyr Trp Asp Trp Arg
1               5                   10


<210>  568
<211>  9
<212>  PRT
<213>  Artificial sequence

<220>
<223>  HLA restricted peptide

<400>  568

Asn Gly Asp Phe Phe Ile Ser Ser Lys
1               5


<210>  569
<211>  10
<212>  PRT
<213>  Artificial sequence

<220>
<223>  HLA restricted peptide

<400>  569

Asn Gly Ser Thr Pro Met Phe Asn Asp Ile
1               5                   10

<210>  570
<211>  10
<212>  PRT
<213>  Artificial sequence

<220>
<223>  HLA restricted peptide

<400>  570

Asn Gly Thr Met Ser Gln Val Gln Gly Ser
1               5                   10


<210>  571
<211>  9
<212>  PRT
<213>  Artificial sequence

<220>
<223>  HLA restricted peptide

<400>  571

Asn Gly Thr Pro Glu Gly Pro Leu Arg
1               5


<210>  572
<211>  10
<212>  PRT
<213>  Artificial sequence

<220>
<223>  HLA restricted peptide

<400>  572

Asn Gly Thr Pro Glu Gly Pro Leu Arg Arg
1               5                   10


<210>  573
<211>  9
<212>  PRT
<213>  Artificial sequence

<220>
<223>  HLA restricted peptide

<400>  573

Asn His Asp Lys Ser Arg Thr Pro Arg
1               5


<210>  574
<211>  10
<212>  PRT
<213>  Artificial sequence

<220>
<223>  HLA restricted peptide

<400>  574

Asn His Asp Lys Ser Arg Thr Pro Arg Leu
1               5                   10


<210>  575
<211>  10
<212>  PRT
<213>  Artificial sequence

<220>
<223>  HLA restricted peptide

<400>  575

Asn Ile Phe Asp Leu Ser Ala Pro Glu Lys
1               5                   10


<210>  576
<211>  9
<212>  PRT
<213>  Artificial sequence

<220>
<223>  HLA restricted peptide

<400>  576

Asn Ile Leu Leu Ser Asn Ala Pro Leu
1               5


<210>  577
<211>  9
<212>  PRT
<213>  Artificial sequence

<220>
<223>  HLA restricted peptide

<400>  577

Asn Ile Tyr Asp Leu Phe Val Trp Ile
1               5


<210>  578
<211>  10
<212>  PRT
<213>  Artificial sequence

<220>
<223>  HLA restricted peptide

<400>  578

Asn Ile Tyr Asp Leu Phe Val Trp Ile His
1               5                   10


<210>  579
<211>  9
<212>  PRT

<213>  Artificial sequence

<220>
<223>  HLA restricted peptide

<400>  579

Asn Leu Leu Ser Pro Ala Ser Phe Phe
1               5


<210>  580
<211>  10
<212>  PRT
<213>  Artificial sequence

<220>
<223>  HLA restricted peptide

<400>  580

Asn Leu Leu Ser Pro Ala Ser Phe Phe Ser
1               5                   10


<210>  581
<211>  9
<212>  PRT
<213>  Artificial sequence

<220>
<223>  HLA restricted peptide

<400>  581

Asn Pro Gly Asn His Asp Lys Ser Arg
1               5


<210>  582
<211>  10
<212>  PRT
<213>  Artificial sequence

<220>
<223>  HLA restricted peptide

<400>  582

Asn Pro Gly Asn His Asp Lys Ser Arg Thr
1               5                   10


<210>  583
<211>  9
<212>  PRT
<213>  Artificial sequence

<220>
<223>  HLA restricted peptide

<400>  583

Asn Pro Asn Leu Leu Ser Pro Ala Ser
1               5

<210>  584
<211>  10
<212>  PRT
<213>  Artificial sequence

<220>
<223>  HLA restricted peptide

<400>  584

Asn Pro Asn Leu Leu Ser Pro Ala Ser Phe
1               5                   10


<210>  585
<211>  9
<212>  PRT
<213>  Artificial sequence

<220>
<223>  HLA restricted peptide

<400>  585

Asn Arg Glu Ser Tyr Met Val Pro Phe
1               5


<210>  586
<211>  10
<212>  PRT
<213>  Artificial sequence

<220>
<223>  HLA restricted peptide

<400>  586

Asn Arg Glu Ser Tyr Met Val Pro Phe Ile
1               5                   10


<210>  587
<211>  9
<212>  PRT
<213>  Artificial sequence

<220>
<223>  HLA restricted peptide

<400>  587

Asn Arg Thr Cys Gln Cys Ser Gly Asn
1               5


<210>  588
<211>  10
<212>  PRT
<213>  Artificial sequence

<220>
<223>  HLA restricted peptide

<400>  588

Asn Arg Thr Cys Gln Cys Ser Gly Asn Phe
1               5                   10


<210>  589
<211>  10
<212>  PRT
<213>  Artificial sequence

<220>
<223>  HLA restricted peptide

<400>  589

Asn Ser His Gln Ser Leu Cys Asn Gly Thr
1               5                   10


<210>  590
<211>  10
<212>  PRT
<213>  Artificial sequence

<220>
<223>  HLA restricted peptide

<400>  590

Asn Thr Leu Glu Gly Phe Ala Ser Pro Leu
1               5                   10


<210>  591
<211>  9
<212>  PRT
<213>  Artificial sequence

<220>
<223>  HLA restricted peptide

<400>  591

Pro Ala Phe Leu Pro Trp His Arg Leu
1               5


<210>  592
<211>  10
<212>  PRT
<213>  Artificial sequence

<220>
<223>  HLA restricted peptide

<400>  592

Pro Ala Phe Leu Pro Trp His Arg Leu Phe
1               5                   10


<210>  593
<211>  10

<212>  PRT
<213>  Artificial sequence

<220>
<223>  HLA restricted peptide

<400>  593

Pro Ala Ser Phe Phe Ser Ser Trp Gln Ile
1               5                   10


<210>  594
<211>  9
<212>  PRT
<213>  Artificial sequence

<220>
<223>  HLA restricted peptide

<400>  594

Pro Glu Glu Lys Gln Pro Leu Leu Met
1               5


<210>  595
<211>  9
<212>  PRT
<213>  Artificial sequence

<220>
<223>  HLA restricted peptide

<400>  595

Pro Glu Lys Asp Lys Phe Phe Ala Tyr
1               5


<210>  596
<211>  10
<212>  PRT
<213>  Artificial sequence

<220>
<223>  HLA restricted peptide

<400>  596

Pro Glu Lys Asp Lys Phe Phe Ala Tyr Leu
1               5                   10


<210>  597
<211>  9
<212>  PRT
<213>  Artificial sequence

<220>
<223>  HLA restricted peptide

<400>  597

Pro Ile Phe Leu Leu His His Ala Phe

1               5


<210>  598
<211>  10
<212>  PRT
<213>  Artificial sequence

<220>
<223>  HLA restricted peptide

<400>  598

Pro Ile Phe Leu Leu His His Ala Phe Val
1               5                   10


<210>  599
<211>  9
<212>  PRT
<213>  Artificial sequence

<220>
<223>  HLA restricted peptide

<400>  599

Pro Ile Gly His Asn Arg Glu Ser Tyr
1               5


<210>  600
<211>  9
<212>  PRT
<213>  Artificial sequence

<220>
<223>  HLA restricted peptide

<400>  600

Pro Ile Gly Thr Tyr Gly Gln Met Lys
1               5


<210>  601
<211>  9
<212>  PRT
<213>  Artificial sequence

<220>
<223>  HLA restricted peptide

<400>  601

Pro Leu Gly Pro Gln Phe Pro Phe Thr
1               5


<210>  602
<211>  9
<212>  PRT
<213>  Artificial sequence

<220>

<223>  HLA restricted peptide

<400>  602

Pro Leu Leu Met Glu Lys Glu Asp Tyr
1               5


<210>  603
<211>  9
<212>  PRT
<213>  Artificial sequence

<220>
<223>  HLA restricted peptide

<400>  603

Pro Leu Tyr Arg Asn Gly Asp Phe Phe
1               5


<210>  604
<211>  10
<212>  PRT
<213>  Artificial sequence

<220>
<223>  HLA restricted peptide

<400>  604

Pro Leu Tyr Arg Asn Gly Asp Phe Phe Ile
1               5                   10


<210>  605
<211>  9
<212>  PRT
<213>  Artificial sequence

<220>
<223>  HLA restricted peptide

<400>  605

Pro Met Phe Asn Asp Ile Asn Ile Tyr
1               5


<210>  606
<211>  10
<212>  PRT
<213>  Artificial sequence

<220>
<223>  HLA restricted peptide

<400>  606

Pro Asn Cys Thr Glu Arg Arg Leu Leu Val
1               5                   10


<210>  607

<211>  9
<212>  PRT
<213>  Artificial sequence

<220>
<223>  HLA restricted peptide

<400>  607

Pro Asn Leu Leu Ser Pro Ala Ser Phe
1               5


<210>  608
<211>  10
<212>  PRT
<213>  Artificial sequence

<220>
<223>  HLA restricted peptide

<400>  608

Pro Asn Leu Leu Ser Pro Ala Ser Phe Phe
1               5                   10


<210>  609
<211>  10
<212>  PRT
<213>  Artificial sequence

<220>
<223>  HLA restricted peptide

<400>  609

Pro Pro Trp Ser Gly Asp Arg Ser Pro Cys
1               5                   10


<210>  610
<211>  9
<212>  PRT
<213>  Artificial sequence

<220>
<223>  HLA restricted peptide

<400>  610

Pro Gln Phe Pro Phe Thr Gly Val Asp
1               5


<210>  611
<211>  10
<212>  PRT
<213>  Artificial sequence

<220>
<223>  HLA restricted peptide

<400>  611

Pro Gln Phe Pro Phe Thr Gly Val Asp Asp
1               5                   10


<210>  612
<211>  9
<212>  PRT
<213>  Artificial sequence

<220>
<223>  HLA restricted peptide

<400>  612

Pro Arg Ala Cys Val Ser Ser Lys Asn
1               5


<210>  613
<211>  10
<212>  PRT
<213>  Artificial sequence

<220>
<223>  HLA restricted peptide

<400>  613

Pro Arg Ala Cys Val Ser Ser Lys Asn Leu
1               5                   10


<210>  614
<211>  9
<212>  PRT
<213>  Artificial sequence

<220>
<223>  HLA restricted peptide

<400>  614

Pro Arg Leu Pro Ser Ser Ala Asp Val
1               5


<210>  615
<211>  10
<212>  PRT
<213>  Artificial sequence

<220>
<223>  HLA restricted peptide

<400>  615

Pro Arg Leu Pro Ser Ser Ala Asp Val Glu
1               5                   10


<210>  616
<211>  10
<212>  PRT
<213>  Artificial sequence

<220>
<223>  HLA restricted peptide

<400>  616

Pro Ser Ser Ala Asp Val Glu Phe Cys Leu
1               5                   10


<210>  617
<211>  9
<212>  PRT
<213>  Artificial sequence

<220>
<223>  HLA restricted peptide

<400>  617

Gln Ala Ser Arg Ile Trp Ser Trp Leu
1               5


<210>  618
<211>  10
<212>  PRT
<213>  Artificial sequence

<220>
<223>  HLA restricted peptide

<400>  618

Gln Ala Ser Arg Ile Trp Ser Trp Leu Leu
1               5                   10


<210>  619
<211>  9
<212>  PRT
<213>  Artificial sequence

<220>
<223>  HLA restricted peptide

<400>  619

Gln Cys Ser Gly Asn Phe Met Gly Phe
1               5


<210>  620
<211>  10
<212>  PRT
<213>  Artificial sequence

<220>
<223>  HLA restricted peptide

<400>  620

Gln Cys Ser Gly Asn Phe Met Gly Phe Asn
1               5                   10

<210>  621
<211>  9
<212>  PRT
<213>  Artificial sequence

<220>
<223>  HLA restricted peptide

<400>  621

Gln Asp Ser Asp Pro Asp Ser Phe Gln
1               5


<210>  622
<211>  9
<212>  PRT
<213>  Artificial sequence

<220>
<223>  HLA restricted peptide

<400>  622

Gln Asp Tyr Ile Lys Ser Tyr Leu Glu
1               5


<210>  623
<211>  9
<212>  PRT
<213>  Artificial sequence

<220>
<223>  HLA restricted peptide

<400>  623

Gln Glu Ile Gln Lys Leu Thr Gly Asp
1               5


<210>  624
<211>  10
<212>  PRT
<213>  Artificial sequence

<220>
<223>  HLA restricted peptide

<400>  624

Gln Glu Ile Gln Lys Leu Thr Gly Asp Glu
1               5                   10


<210>  625
<211>  9
<212>  PRT
<213>  Artificial sequence

<220>
<223>  HLA restricted peptide

<400>  625

Gln Glu Val Tyr Pro Glu Ala Asn Ala
1               5

<210>  626
<211>  10
<212>  PRT
<213>  Artificial sequence

<220>
<223>  HLA restricted peptide

<400>  626

Gln Glu Val Tyr Pro Glu Ala Asn Ala Pro
1               5                   10

<210>  627
<211>  10
<212>  PRT
<213>  Artificial sequence

<220>
<223>  HLA restricted peptide

<400>  627

Gln Phe Pro Phe Thr Gly Val Asp Asp Arg
1               5                   10

<210>  628
<211>  9
<212>  PRT
<213>  Artificial sequence

<220>
<223>  HLA restricted peptide

<400>  628

Gln Gly Ser Ala Asn Asp Pro Ile Phe
1               5

<210>  629
<211>  10
<212>  PRT
<213>  Artificial sequence

<220>
<223>  HLA restricted peptide

<400>  629

Gln Gly Ser Ala Asn Asp Pro Ile Phe Leu
1               5                   10

<210>  630
<211>  9
<212>  PRT
<213>  Artificial sequence

<220>
<223>  HLA restricted peptide

<400>  630

Gln His Pro Thr Asn Pro Asn Leu Leu
1               5


<210>  631
<211>  10
<212>  PRT
<213>  Artificial sequence

<220>
<223>  HLA restricted peptide

<400>  631

Gln His Pro Thr Asn Pro Asn Leu Leu Ser
1               5                   10


<210>  632
<211>  10
<212>  PRT
<213>  Artificial sequence

<220>
<223>  HLA restricted peptide

<400>  632

Gln Ile Val Cys Ser Arg Leu Glu Glu Tyr
1               5                   10


<210>  633
<211>  9
<212>  PRT
<213>  Artificial sequence

<220>
<223>  HLA restricted peptide

<400>  633

Gln Lys Leu Thr Gly Asp Glu Asn Phe
1               5


<210>  634
<211>  10
<212>  PRT
<213>  Artificial sequence

<220>
<223>  HLA restricted peptide

<400>  634

Gln Lys Leu Thr Gly Asp Glu Asn Phe Thr
1               5                   10

<210>  635
<211>  9
<212>  PRT
<213>  Artificial sequence

<220>
<223>  HLA restricted peptide

<400>  635

Gln Leu Pro Glu Glu Lys Gln Pro Leu
1               5


<210>  636
<211>  10
<212>  PRT
<213>  Artificial sequence

<220>
<223>  HLA restricted peptide

<400>  636

Gln Leu Pro Glu Glu Lys Gln Pro Leu Leu
1               5                   10


<210>  637
<211>  10
<212>  PRT
<213>  Artificial sequence

<220>
<223>  HLA restricted peptide

<400>  637

Gln Leu Ser Gly Arg Gly Ser Cys Gln Asn
1               5                   10


<210>  638
<211>  9
<212>  PRT
<213>  Artificial sequence

<220>
<223>  HLA restricted peptide

<400>  638

Gln Met Lys Asn Gly Ser Thr Pro Met
1               5


<210>  639
<211>  10
<212>  PRT
<213>  Artificial sequence

<220>
<223>  HLA restricted peptide

<400>  639

Gln Met Lys Asn Gly Ser Thr Pro Met Phe
1               5                   10


<210>  640
<211>  10
<212>  PRT
<213>  Artificial sequence

<220>
<223>  HLA restricted peptide

<400>  640

Gln Asn Ile Leu Leu Ser Asn Ala Pro Leu
1               5                   10


<210>  641
<211>  9
<212>  PRT
<213>  Artificial sequence

<220>
<223>  HLA restricted peptide

<400>  641

Gln Pro Leu Leu Met Glu Lys Glu Asp
1               5


<210>  642
<211>  10
<212>  PRT
<213>  Artificial sequence

<220>
<223>  HLA restricted peptide

<400>  642

Gln Pro Leu Leu Met Glu Lys Glu Asp Tyr
1               5                   10


<210>  643
<211>  9
<212>  PRT
<213>  Artificial sequence

<220>
<223>  HLA restricted peptide

<400>  643

Gln Ser Ser Met His Asn Ala Leu His
1               5


<210>  644
<211>  10
<212>  PRT

<213>  Artificial sequence

<220>
<223>  HLA restricted peptide

<400>  644

Gln Ser Ser Met His Asn Ala Leu His Ile
1               5                   10


<210>  645
<211>  9
<212>  PRT
<213>  Artificial sequence

<220>
<223>  HLA restricted peptide

<400>  645

Gln Thr Ser Ala Gly His Phe Pro Arg
1               5


<210>  646
<211>  10
<212>  PRT
<213>  Artificial sequence

<220>
<223>  HLA restricted peptide

<400>  646

Gln Thr Ser Ala Gly His Phe Pro Arg Ala
1               5                   10


<210>  647
<211>  10
<212>  PRT
<213>  Artificial sequence

<220>
<223>  HLA restricted peptide

<400>  647

Gln Val Gln Gly Ser Ala Asn Asp Pro Ile
1               5                   10


<210>  648
<211>  9
<212>  PRT
<213>  Artificial sequence

<220>
<223>  HLA restricted peptide

<400>  648

Gln Trp Leu Arg Arg His Arg Pro Leu
1               5

<210>  649
<211>  9
<212>  PRT
<213>  Artificial sequence

<220>
<223>  HLA restricted peptide

<400>  649

Gln Tyr Glu Ser Gly Ser Met Asp Lys
1               5


<210>  650
<211>  10
<212>  PRT
<213>  Artificial sequence

<220>
<223>  HLA restricted peptide

<400>  650

Gln Tyr Glu Ser Gly Ser Met Asp Lys Ala
1               5                   10


<210>  651
<211>  9
<212>  PRT
<213>  Artificial sequence

<220>
<223>  HLA restricted peptide

<400>  651

Arg Ala Cys Val Ser Ser Lys Asn Leu
1               5


<210>  652
<211>  10
<212>  PRT
<213>  Artificial sequence

<220>
<223>  HLA restricted peptide

<400>  652

Arg Ala Cys Val Ser Ser Lys Asn Leu Met
1               5                   10


<210>  653
<211>  9
<212>  PRT
<213>  Artificial sequence

<220>
<223>  HLA restricted peptide

<400>  653

Arg Asp Ala Glu Lys Cys Asp Ile Cys
1               5


<210>  654
<211>  10
<212>  PRT
<213>  Artificial sequence

<220>
<223>  HLA restricted peptide

<400>  654

Arg Asp Ala Glu Lys Cys Asp Ile Cys Thr
1               5                   10


<210>  655
<211>  9
<212>  PRT
<213>  Artificial sequence

<220>
<223>  HLA restricted peptide

<400>  655

Arg Asp Ile Asp Phe Ala His Glu Ala
1               5


<210>  656
<211>  9
<212>  PRT
<213>  Artificial sequence

<220>
<223>  HLA restricted peptide

<400>  656

Arg Glu Ser Trp Pro Ser Val Phe Tyr
1               5


<210>  657
<211>  10
<212>  PRT
<213>  Artificial sequence

<220>
<223>  HLA restricted peptide

<400>  657

Arg Glu Ser Trp Pro Ser Val Phe Tyr Asn
1               5                   10


<210>  658
<211>  9

<212>  PRT
<213>  Artificial sequence

<220>
<223>  HLA restricted peptide

<400>  658

Arg Glu Ser Tyr Met Val Pro Phe Ile
1               5


<210>  659
<211>  9
<212>  PRT
<213>  Artificial sequence

<220>
<223>  HLA restricted peptide

<400>  659

Arg Gly Ser Cys Gln Asn Ile Leu Leu
1               5


<210>  660
<211>  10
<212>  PRT
<213>  Artificial sequence

<220>
<223>  HLA restricted peptide

<400>  660

Arg Gly Ser Cys Gln Asn Ile Leu Leu Ser
1               5                   10


<210>  661
<211>  9
<212>  PRT
<213>  Artificial sequence

<220>
<223>  HLA restricted peptide

<400>  661

Arg His Arg Pro Leu Gln Glu Val Tyr
1               5


<210>  662
<211>  9
<212>  PRT
<213>  Artificial sequence

<220>
<223>  HLA restricted peptide

<400>  662

Arg Ile Trp Ser Trp Leu Leu Gly Ala

1               5


<210>  663
<211>  10
<212>  PRT
<213>  Artificial sequence

<220>
<223>  HLA restricted peptide

<400>  663

Arg Ile Trp Ser Trp Leu Leu Gly Ala Ala
1               5                   10


<210>  664
<211>  10
<212>  PRT
<213>  Artificial sequence

<220>
<223>  HLA restricted peptide

<400>  664

Arg Leu Glu Glu Tyr Asn Ser His Gln Ser
1               5                   10


<210>  665
<211>  9
<212>  PRT
<213>  Artificial sequence

<220>
<223>  HLA restricted peptide

<400>  665

Arg Leu Phe Leu Leu Arg Trp Glu Gln
1               5


<210>  666
<211>  10
<212>  PRT
<213>  Artificial sequence

<220>
<223>  HLA restricted peptide

<400>  666

Arg Leu Phe Leu Leu Arg Trp Glu Gln Glu
1               5                   10


<210>  667
<211>  9
<212>  PRT
<213>  Artificial sequence

<220>

<223>  HLA restricted peptide

<400>  667

Arg Leu Leu Val Arg Arg Asn Ile Phe
1               5


<210>  668
<211>  10
<212>  PRT
<213>  Artificial sequence

<220>
<223>  HLA restricted peptide

<400>  668

Arg Leu Pro Ser Ser Ala Asp Val Glu Phe
1               5                   10


<210>  669
<211>  9
<212>  PRT
<213>  Artificial sequence

<220>
<223>  HLA restricted peptide

<400>  669

Arg Asn Gly Asp Phe Phe Ile Ser Ser
1               5


<210>  670
<211>  10
<212>  PRT
<213>  Artificial sequence

<220>
<223>  HLA restricted peptide

<400>  670

Arg Asn Gly Asp Phe Phe Ile Ser Ser Lys
1               5                   10


<210>  671
<211>  9
<212>  PRT
<213>  Artificial sequence

<220>
<223>  HLA restricted peptide

<400>  671

Arg Asn Pro Gly Asn His Asp Lys Ser
1               5


<210>  672

<211>  10
<212>  PRT
<213>  Artificial sequence

<220>
<223>  HLA restricted peptide

<400>  672

Arg Asn Pro Gly Asn His Asp Lys Ser Arg
1               5                   10


<210>  673
<211>  9
<212>  PRT
<213>  Artificial sequence

<220>
<223>  HLA restricted peptide

<400>  673

Arg Asn Thr Leu Glu Gly Phe Ala Ser
1               5


<210>  674
<211>  9
<212>  PRT
<213>  Artificial sequence

<220>
<223>  HLA restricted peptide

<400>  674

Arg Pro Leu Gln Glu Val Tyr Pro Glu
1               5


<210>  675
<211>  10
<212>  PRT
<213>  Artificial sequence

<220>
<223>  HLA restricted peptide

<400>  675

Arg Pro Leu Gln Glu Val Tyr Pro Glu Ala
1               5                   10


<210>  676
<211>  9
<212>  PRT
<213>  Artificial sequence

<220>
<223>  HLA restricted peptide

<400>  676

Arg Arg His Arg Pro Leu Gln Glu Val
1               5

<210>  677
<211>  10
<212>  PRT
<213>  Artificial sequence

<220>
<223>  HLA restricted peptide

<400>  677

Arg Arg His Arg Pro Leu Gln Glu Val Tyr
1               5                   10

<210>  678
<211>  9
<212>  PRT
<213>  Artificial sequence

<220>
<223>  HLA restricted peptide

<400>  678

Arg Arg Leu Leu Val Arg Arg Asn Ile
1               5

<210>  679
<211>  10
<212>  PRT
<213>  Artificial sequence

<220>
<223>  HLA restricted peptide

<400>  679

Arg Arg Leu Leu Val Arg Arg Asn Ile Phe
1               5                   10

<210>  680
<211>  9
<212>  PRT
<213>  Artificial sequence

<220>
<223>  HLA restricted peptide

<400>  680

Arg Arg Asn Ile Phe Asp Leu Ser Ala
1               5

<210>  681
<211>  10
<212>  PRT
<213>  Artificial sequence

<220>
<223>  HLA restricted peptide

<400>  681

Arg Arg Asn Ile Phe Asp Leu Ser Ala Pro
1               5                   10


<210>  682
<211>  9
<212>  PRT
<213>  Artificial sequence

<220>
<223>  HLA restricted peptide

<400>  682

Arg Arg Asn Pro Gly Asn His Asp Lys
1               5


<210>  683
<211>  10
<212>  PRT
<213>  Artificial sequence

<220>
<223>  HLA restricted peptide

<400>  683

Arg Arg Asn Pro Gly Asn His Asp Lys Ser
1               5                   10


<210>  684
<211>  10
<212>  PRT
<213>  Artificial sequence

<220>
<223>  HLA restricted peptide

<400>  684

Arg Ser Pro Cys Gly Gln Leu Ser Gly Arg
1               5                   10


<210>  685
<211>  9
<212>  PRT
<213>  Artificial sequence

<220>
<223>  HLA restricted peptide

<400>  685

Arg Thr Cys Gln Cys Ser Gly Asn Phe
1               5

<210>  686
<211>  10
<212>  PRT
<213>  Artificial sequence

<220>
<223>  HLA restricted peptide

<400>  686

Arg Thr Cys Gln Cys Ser Gly Asn Phe Met
1               5                   10


<210>  687
<211>  9
<212>  PRT
<213>  Artificial sequence

<220>
<223>  HLA restricted peptide

<400>  687

Arg Thr Pro Arg Leu Pro Ser Ser Ala
1               5


<210>  688
<211>  9
<212>  PRT
<213>  Artificial sequence

<220>
<223>  HLA restricted peptide

<400>  688

Arg Trp Glu Gln Glu Ile Gln Lys Leu
1               5


<210>  689
<211>  10
<212>  PRT
<213>  Artificial sequence

<220>
<223>  HLA restricted peptide

<400>  689

Arg Trp Glu Gln Glu Ile Gln Lys Leu Thr
1               5                   10


<210>  690
<211>  9
<212>  PRT
<213>  Artificial sequence

<220>
<223>  HLA restricted peptide

<400>  690

Ser Ala Asp Val Glu Phe Cys Leu Ser
1               5

<210>  691
<211>  10
<212>  PRT
<213>  Artificial sequence

<220>
<223>  HLA restricted peptide

<400>  691

Ser Ala Asp Val Glu Phe Cys Leu Ser Leu
1               5                   10

<210>  692
<211>  9
<212>  PRT
<213>  Artificial sequence

<220>
<223>  HLA restricted peptide

<400>  692

Ser Ala Gly His Phe Pro Arg Ala Cys
1               5

<210>  693
<211>  10
<212>  PRT
<213>  Artificial sequence

<220>
<223>  HLA restricted peptide

<400>  693

Ser Ala Gly His Phe Pro Arg Ala Cys Val
1               5                   10

<210>  694
<211>  9
<212>  PRT
<213>  Artificial sequence

<220>
<223>  HLA restricted peptide

<400>  694

Ser Ala Asn Asp Pro Ile Phe Leu Leu
1               5

<210>  695
<211>  10
<212>  PRT
<213>  Artificial sequence

<220>
<223>  HLA restricted peptide

<400>  695

Ser Ala Asn Asp Pro Ile Phe Leu Leu His
1               5                   10


<210>  696
<211>  9
<212>  PRT
<213>  Artificial sequence

<220>
<223>  HLA restricted peptide

<400>  696

Ser Ala Pro Glu Lys Asp Lys Phe Phe
1               5


<210>  697
<211>  10
<212>  PRT
<213>  Artificial sequence

<220>
<223>  HLA restricted peptide

<400>  697

Ser Ala Pro Glu Lys Asp Lys Phe Phe Ala
1               5                   10


<210>  698
<211>  9
<212>  PRT
<213>  Artificial sequence

<220>
<223>  HLA restricted peptide

<400>  698

Ser Cys Gln Asn Ile Leu Leu Ser Asn
1               5


<210>  699
<211>  10
<212>  PRT
<213>  Artificial sequence

<220>
<223>  HLA restricted peptide

<400>  699

Ser Cys Gln Asn Ile Leu Leu Ser Asn Ala
1               5                   10

<210>  700
<211>  9
<212>  PRT
<213>  Artificial sequence

<220>
<223>  HLA restricted peptide

<400>  700

Ser Asp Pro Asp Ser Phe Gln Asp Tyr
1               5


<210>  701
<211>  10
<212>  PRT
<213>  Artificial sequence

<220>
<223>  HLA restricted peptide

<400>  701

Ser Asp Pro Asp Ser Phe Gln Asp Tyr Ile
1               5                   10


<210>  702
<211>  9
<212>  PRT
<213>  Artificial sequence

<220>
<223>  HLA restricted peptide

<400>  702

Ser Asp Tyr Val Ile Pro Ile Gly Thr
1               5


<210>  703
<211>  10
<212>  PRT
<213>  Artificial sequence

<220>
<223>  HLA restricted peptide

<400>  703

Ser Asp Tyr Val Ile Pro Ile Gly Thr Tyr
1               5                   10


<210>  704
<211>  9
<212>  PRT
<213>  Artificial sequence

<220>
<223>  HLA restricted peptide

<400>  704

Ser Phe Phe Ser Ser Trp Gln Ile Val
1               5


<210>  705
<211>  10
<212>  PRT
<213>  Artificial sequence

<220>
<223>  HLA restricted peptide

<400>  705

Ser Phe Phe Ser Ser Trp Gln Ile Val Cys
1               5                   10


<210>  706
<211>  9
<212>  PRT
<213>  Artificial sequence

<220>
<223>  HLA restricted peptide

<400>  706

Ser Phe Gln Asp Tyr Ile Lys Ser Tyr
1               5


<210>  707
<211>  10
<212>  PRT
<213>  Artificial sequence

<220>
<223>  HLA restricted peptide

<400>  707

Ser Phe Gln Asp Tyr Ile Lys Ser Tyr Leu
1               5                   10


<210>  708
<211>  9
<212>  PRT
<213>  Artificial sequence

<220>
<223>  HLA restricted peptide

<400>  708

Ser Phe Gln Thr Ser Ala Gly His Phe
1               5


<210>  709
<211>  9
<212>  PRT

<213>  Artificial sequence

<220>
<223>  HLA restricted peptide

<400>  709

Ser Phe Arg Asn Thr Leu Glu Gly Phe
1               5


<210>  710
<211>  10
<212>  PRT
<213>  Artificial sequence

<220>
<223>  HLA restricted peptide

<400>  710

Ser Phe Arg Asn Thr Leu Glu Gly Phe Ala
1               5                   10


<210>  711
<211>  10
<212>  PRT
<213>  Artificial sequence

<220>
<223>  HLA restricted peptide

<400>  711

Ser Gly Asp Arg Ser Pro Cys Gly Gln Leu
1               5                   10


<210>  712
<211>  9
<212>  PRT
<213>  Artificial sequence

<220>
<223>  HLA restricted peptide

<400>  712

Ser Gly Asn Phe Met Gly Phe Asn Cys
1               5


<210>  713
<211>  9
<212>  PRT
<213>  Artificial sequence

<220>
<223>  HLA restricted peptide

<400>  713

Ser Gly Arg Gly Ser Cys Gln Asn Ile
1               5

<210>  714
<211>  10
<212>  PRT
<213>  Artificial sequence

<220>
<223>  HLA restricted peptide

<400>  714

Ser Gly Arg Gly Ser Cys Gln Asn Ile Leu
1               5                   10


<210>  715
<211>  9
<212>  PRT
<213>  Artificial sequence

<220>
<223>  HLA restricted peptide

<400>  715

Ser Gly Ser Met Asp Lys Ala Ala Asn
1               5


<210>  716
<211>  10
<212>  PRT
<213>  Artificial sequence

<220>
<223>  HLA restricted peptide

<400>  716

Ser Gly Ser Met Asp Lys Ala Ala Asn Phe
1               5                   10


<210>  717
<211>  9
<212>  PRT
<213>  Artificial sequence

<220>
<223>  HLA restricted peptide

<400>  717

Ser His Gln Ser Leu Cys Asn Gly Thr
1               5


<210>  718
<211>  9
<212>  PRT
<213>  Artificial sequence

<220>
<223>  HLA restricted peptide

<400>  718

Ser Ile Phe Glu Gln Trp Leu Arg Arg
1               5


<210>  719
<211>  10
<212>  PRT
<213>  Artificial sequence

<220>
<223>  HLA restricted peptide

<400>  719

Ser Ile Phe Glu Gln Trp Leu Arg Arg His
1               5                   10


<210>  720
<211>  9
<212>  PRT
<213>  Artificial sequence

<220>
<223>  HLA restricted peptide

<400>  720

Ser Lys Asp Leu Gly Tyr Asp Tyr Ser
1               5


<210>  721
<211>  10
<212>  PRT
<213>  Artificial sequence

<220>
<223>  HLA restricted peptide

<400>  721

Ser Lys Asp Leu Gly Tyr Asp Tyr Ser Tyr
1               5                   10


<210>  722
<211>  9
<212>  PRT
<213>  Artificial sequence

<220>
<223>  HLA restricted peptide

<400>  722

Ser Lys Asn Leu Met Glu Lys Glu Cys
1               5


<210>  723
<211>  10

<212>  PRT
<213>  Artificial sequence

<220>
<223>  HLA restricted peptide

<400>  723

Ser Lys Asn Leu Met Glu Lys Glu Cys Cys
1               5                   10


<210>  724
<211>  9
<212>  PRT
<213>  Artificial sequence

<220>
<223>  HLA restricted peptide

<400>  724

Ser Leu Leu Cys Arg His Lys Arg Lys
1               5


<210>  725
<211>  9
<212>  PRT
<213>  Artificial sequence

<220>
<223>  HLA restricted peptide

<400>  725

Ser Leu Thr Gln Tyr Glu Ser Gly Ser
1               5


<210>  726
<211>  10
<212>  PRT
<213>  Artificial sequence

<220>
<223>  HLA restricted peptide

<400>  726

Ser Leu Thr Gln Tyr Glu Ser Gly Ser Met
1               5                   10


<210>  727
<211>  9
<212>  PRT
<213>  Artificial sequence

<220>
<223>  HLA restricted peptide

<400>  727

Ser Met Asp Ala Leu Leu Gly Gly Tyr

1                 5


<210>  728
<211>  9
<212>  PRT
<213>  Artificial sequence

<220>
<223>  HLA restricted peptide

<400>  728

Ser Met Asp Lys Ala Ala Asn Phe Ser
1               5


<210>  729
<211>  10
<212>  PRT
<213>  Artificial sequence

<220>
<223>  HLA restricted peptide

<400>  729

Ser Met Asp Lys Ala Ala Asn Phe Ser Phe
1               5                   10


<210>  730
<211>  9
<212>  PRT
<213>  Artificial sequence

<220>
<223>  HLA restricted peptide

<400>  730

Ser Met His Asn Ala Leu His Ile Tyr
1               5


<210>  731
<211>  10
<212>  PRT
<213>  Artificial sequence

<220>
<223>  HLA restricted peptide

<400>  731

Ser Met His Asn Ala Leu His Ile Tyr Met
1               5                   10


<210>  732
<211>  9
<212>  PRT
<213>  Artificial sequence

<220>

<223>  HLA restricted peptide

<400>  732

Ser Asn Ala Pro Leu Gly Pro Gln Phe
1               5

<210>  733
<211>  9
<212>  PRT
<213>  Artificial sequence

<220>
<223>  HLA restricted peptide

<400>  733

Ser Pro Ala Ser Phe Phe Ser Ser Trp
1               5

<210>  734
<211>  10
<212>  PRT
<213>  Artificial sequence

<220>
<223>  HLA restricted peptide

<400>  734

Ser Pro Ala Ser Phe Phe Ser Ser Trp Gln
1               5                   10

<210>  735
<211>  9
<212>  PRT
<213>  Artificial sequence

<220>
<223>  HLA restricted peptide

<400>  735

Ser Pro Cys Gly Gln Leu Ser Gly Arg
1               5

<210>  736
<211>  10
<212>  PRT
<213>  Artificial sequence

<220>
<223>  HLA restricted peptide

<400>  736

Ser Pro Cys Gly Gln Leu Ser Gly Arg Gly
1               5                   10

<210>  737

<211>  9
<212>  PRT
<213>  Artificial sequence

<220>
<223>  HLA restricted peptide

<400>  737

Ser Pro Leu Thr Gly Ile Ala Asp Ala
1               5


<210>  738
<211>  10
<212>  PRT
<213>  Artificial sequence

<220>
<223>  HLA restricted peptide

<400>  738

Ser Pro Leu Thr Gly Ile Ala Asp Ala Ser
1               5                   10


<210>  739
<211>  9
<212>  PRT
<213>  Artificial sequence

<220>
<223>  HLA restricted peptide

<400>  739

Ser Gln Ser Ser Met His Asn Ala Leu
1               5


<210>  740
<211>  10
<212>  PRT
<213>  Artificial sequence

<220>
<223>  HLA restricted peptide

<400>  740

Ser Gln Ser Ser Met His Asn Ala Leu His
1               5                   10


<210>  741
<211>  9
<212>  PRT
<213>  Artificial sequence

<220>
<223>  HLA restricted peptide

<400>  741

Ser Gln Val Gln Gly Ser Ala Asn Asp
1               5

<210>  742
<211>  10
<212>  PRT
<213>  Artificial sequence

<220>
<223>  HLA restricted peptide

<400>  742

Ser Gln Val Gln Gly Ser Ala Asn Asp Pro
1               5                   10

<210>  743
<211>  9
<212>  PRT
<213>  Artificial sequence

<220>
<223>  HLA restricted peptide

<400>  743

Ser Arg Ile Trp Ser Trp Leu Leu Gly
1               5

<210>  744
<211>  10
<212>  PRT
<213>  Artificial sequence

<220>
<223>  HLA restricted peptide

<400>  744

Ser Arg Ile Trp Ser Trp Leu Leu Gly Ala
1               5                   10

<210>  745
<211>  9
<212>  PRT
<213>  Artificial sequence

<220>
<223>  HLA restricted peptide

<400>  745

Ser Arg Leu Glu Glu Tyr Asn Ser His
1               5

<210>  746
<211>  10
<212>  PRT
<213>  Artificial sequence

<220>
<223>  HLA restricted peptide

<400>  746

Ser Arg Leu Glu Glu Tyr Asn Ser His Gln
1               5                   10


<210>  747
<211>  9
<212>  PRT
<213>  Artificial sequence

<220>
<223>  HLA restricted peptide

<400>  747

Ser Arg Thr Pro Arg Leu Pro Ser Ser
1               5


<210>  748
<211>  10
<212>  PRT
<213>  Artificial sequence

<220>
<223>  HLA restricted peptide

<400>  748

Ser Arg Thr Pro Arg Leu Pro Ser Ser Ala
1               5                   10


<210>  749
<211>  9
<212>  PRT
<213>  Artificial sequence

<220>
<223>  HLA restricted peptide

<400>  749

Ser Ser Ala Asp Val Glu Phe Cys Leu
1               5


<210>  750
<211>  10
<212>  PRT
<213>  Artificial sequence

<220>
<223>  HLA restricted peptide

<400>  750

Ser Ser Ala Asp Val Glu Phe Cys Leu Ser
1               5                   10

<210>  751
<211>  9
<212>  PRT
<213>  Artificial sequence

<220>
<223>  HLA restricted peptide

<400>  751

Ser Ser Asp Tyr Val Ile Pro Ile Gly
1               5


<210>  752
<211>  10
<212>  PRT
<213>  Artificial sequence

<220>
<223>  HLA restricted peptide

<400>  752

Ser Ser Asp Tyr Val Ile Pro Ile Gly Thr
1               5                   10


<210>  753
<211>  9
<212>  PRT
<213>  Artificial sequence

<220>
<223>  HLA restricted peptide

<400>  753

Ser Ser Lys Asp Leu Gly Tyr Asp Tyr
1               5


<210>  754
<211>  10
<212>  PRT
<213>  Artificial sequence

<220>
<223>  HLA restricted peptide

<400>  754

Ser Ser Lys Asp Leu Gly Tyr Asp Tyr Ser
1               5                   10


<210>  755
<211>  10
<212>  PRT
<213>  Artificial sequence

<220>
<223>  HLA restricted peptide

<400>  755

Ser Ser Lys Asn Leu Met Glu Lys Glu Cys
1               5                   10


<210>  756
<211>  9
<212>  PRT
<213>  Artificial sequence

<220>
<223>  HLA restricted peptide

<400>  756

Ser Ser Met His Asn Ala Leu His Ile
1               5


<210>  757
<211>  10
<212>  PRT
<213>  Artificial sequence

<220>
<223>  HLA restricted peptide

<400>  757

Ser Ser Met His Asn Ala Leu His Ile Tyr
1               5                   10


<210>  758
<211>  9
<212>  PRT
<213>  Artificial sequence

<220>
<223>  HLA restricted peptide

<400>  758

Ser Ser Trp Gln Ile Val Cys Ser Arg
1               5


<210>  759
<211>  10
<212>  PRT
<213>  Artificial sequence

<220>
<223>  HLA restricted peptide

<400>  759

Ser Ser Trp Gln Ile Val Cys Ser Arg Leu
1               5                   10


<210>  760
<211>  9
<212>  PRT
<213>  Artificial sequence

<220>
<223>  HLA restricted peptide

<400>  760

Ser Thr Pro Met Phe Asn Asp Ile Asn
1               5


<210>  761
<211>  10
<212>  PRT
<213>  Artificial sequence

<220>
<223>  HLA restricted peptide

<400>  761

Ser Thr Pro Met Phe Asn Asp Ile Asn Ile
1               5                   10


<210>  762
<211>  10
<212>  PRT
<213>  Artificial sequence

<220>
<223>  HLA restricted peptide

<400>  762

Ser Val Phe Tyr Asn Arg Thr Cys Gln Cys
1               5                   10


<210>  763
<211>  9
<212>  PRT
<213>  Artificial sequence

<220>
<223>  HLA restricted peptide

<400>  763

Ser Trp Leu Leu Gly Ala Ala Met Val
1               5


<210>  764
<211>  9
<212>  PRT
<213>  Artificial sequence

<220>
<223>  HLA restricted peptide

<400>  764

Ser Trp Pro Ser Val Phe Tyr Asn Arg
1               5

<210>  765
<211>  10
<212>  PRT
<213>  Artificial sequence

<220>
<223>  HLA restricted peptide

<400>  765

Ser Trp Pro Ser Val Phe Tyr Asn Arg Thr
1               5                   10


<210>  766
<211>  9
<212>  PRT
<213>  Artificial sequence

<220>
<223>  HLA restricted peptide

<400>  766

Ser Trp Gln Ile Val Cys Ser Arg Leu
1               5


<210>  767
<211>  9
<212>  PRT
<213>  Artificial sequence

<220>
<223>  HLA restricted peptide

<400>  767

Ser Tyr Leu Glu Gln Ala Ser Arg Ile
1               5


<210>  768
<211>  10
<212>  PRT
<213>  Artificial sequence

<220>
<223>  HLA restricted peptide

<400>  768

Ser Tyr Leu Glu Gln Ala Ser Arg Ile Trp
1               5                   10


<210>  769
<211>  10
<212>  PRT
<213>  Artificial sequence

<220>
<223>  HLA restricted peptide

<400>  769

Ser Tyr Leu Gln Asp Ser Asp Pro Asp Ser
1               5                   10


<210>  770
<211>  9
<212>  PRT
<213>  Artificial sequence

<220>
<223>  HLA restricted peptide

<400>  770

Ser Tyr Met Val Pro Phe Ile Pro Leu
1               5


<210>  771
<211>  10
<212>  PRT
<213>  Artificial sequence

<220>
<223>  HLA restricted peptide

<400>  771

Ser Tyr Met Val Pro Phe Ile Pro Leu Tyr
1               5                   10


<210>  772
<211>  9
<212>  PRT
<213>  Artificial sequence

<220>
<223>  HLA restricted peptide

<400>  772

Thr Ala Leu Leu Ala Gly Leu Val Ser
1               5


<210>  773
<211>  10
<212>  PRT
<213>  Artificial sequence

<220>
<223>  HLA restricted peptide

<400>  773

Thr Ala Leu Leu Ala Gly Leu Val Ser Leu
1               5                   10


<210>  774
<211>  9
<212>  PRT

<213>  Artificial sequence

<220>
<223>  HLA restricted peptide

<400>  774

Thr Cys Gln Cys Ser Gly Asn Phe Met
1               5


<210>  775
<211>  9
<212>  PRT
<213>  Artificial sequence

<220>
<223>  HLA restricted peptide

<400>  775

Thr Glu Arg Arg Leu Leu Val Arg Arg
1               5


<210>  776
<211>  10
<212>  PRT
<213>  Artificial sequence

<220>
<223>  HLA restricted peptide

<400>  776

Thr Glu Arg Arg Leu Leu Val Arg Arg Asn
1               5                   10


<210>  777
<211>  9
<212>  PRT
<213>  Artificial sequence

<220>
<223>  HLA restricted peptide

<400>  777

Thr Gly Asp Glu Asn Phe Thr Ile Pro
1               5


<210>  778
<211>  10
<212>  PRT
<213>  Artificial sequence

<220>
<223>  HLA restricted peptide

<400>  778

Thr Gly Asp Glu Asn Phe Thr Ile Pro Tyr
1               5                   10

<210>  779
<211>  9
<212>  PRT
<213>  Artificial sequence

<220>
<223>  HLA restricted peptide

<400>  779

Thr Gly Ile Ala Asp Ala Ser Gln Ser
1               5


<210>  780
<211>  10
<212>  PRT
<213>  Artificial sequence

<220>
<223>  HLA restricted peptide

<400>  780

Thr Gly Ile Ala Asp Ala Ser Gln Ser Ser
1               5                   10


<210>  781
<211>  9
<212>  PRT
<213>  Artificial sequence

<220>
<223>  HLA restricted peptide

<400>  781

Thr Gly Val Asp Asp Arg Glu Ser Trp
1               5


<210>  782
<211>  10
<212>  PRT
<213>  Artificial sequence

<220>
<223>  HLA restricted peptide

<400>  782

Thr Ile Ser Ser Asp Tyr Val Ile Pro Ile
1               5                   10


<210>  783
<211>  9
<212>  PRT
<213>  Artificial sequence

<220>
<223>  HLA restricted peptide

<400>  783

Thr Leu Ala Lys His Thr Ile Ser Ser
1               5


<210>  784
<211>  9
<212>  PRT
<213>  Artificial sequence

<220>
<223>  HLA restricted peptide

<400>  784

Thr Leu Glu Gly Phe Ala Ser Pro Leu
1               5


<210>  785
<211>  10
<212>  PRT
<213>  Artificial sequence

<220>
<223>  HLA restricted peptide

<400>  785

Thr Leu Glu Gly Phe Ala Ser Pro Leu Thr
1               5                   10


<210>  786
<211>  9
<212>  PRT
<213>  Artificial sequence

<220>
<223>  HLA restricted peptide

<400>  786

Thr Met Ser Gln Val Gln Gly Ser Ala
1               5


<210>  787
<211>  10
<212>  PRT
<213>  Artificial sequence

<220>
<223>  HLA restricted peptide

<400>  787

Thr Met Ser Gln Val Gln Gly Ser Ala Asn
1               5                   10


<210>  788
<211>  9

<212>  PRT
<213>  Artificial sequence

<220>
<223>  HLA restricted peptide

<400>  788

Thr Asn Pro Asn Leu Leu Ser Pro Ala
1               5


<210>  789
<211>  10
<212>  PRT
<213>  Artificial sequence

<220>
<223>  HLA restricted peptide

<400>  789

Thr Asn Pro Asn Leu Leu Ser Pro Ala Ser
1               5                   10


<210>  790
<211>  9
<212>  PRT
<213>  Artificial sequence

<220>
<223>  HLA restricted peptide

<400>  790

Thr Pro Glu Gly Pro Leu Arg Arg Asn
1               5


<210>  791
<211>  10
<212>  PRT
<213>  Artificial sequence

<220>
<223>  HLA restricted peptide

<400>  791

Thr Pro Glu Gly Pro Leu Arg Arg Asn Pro
1               5                   10


<210>  792
<211>  9
<212>  PRT
<213>  Artificial sequence

<220>
<223>  HLA restricted peptide

<400>  792

Thr Pro Met Phe Asn Asp Ile Asn Ile

1               5


<210>  793
<211>  10
<212>  PRT
<213>  Artificial sequence

<220>
<223>  HLA restricted peptide

<400>  793

Thr Pro Met Phe Asn Asp Ile Asn Ile Tyr
1               5                   10


<210>  794
<211>  9
<212>  PRT
<213>  Artificial sequence

<220>
<223>  HLA restricted peptide

<400>  794

Thr Pro Arg Leu Pro Ser Ser Ala Asp
1               5


<210>  795
<211>  10
<212>  PRT
<213>  Artificial sequence

<220>
<223>  HLA restricted peptide

<400>  795

Thr Pro Arg Leu Pro Ser Ser Ala Asp Val
1               5                   10


<210>  796
<211>  9
<212>  PRT
<213>  Artificial sequence

<220>
<223>  HLA restricted peptide

<400>  796

Thr Gln Tyr Glu Ser Gly Ser Met Asp
1               5


<210>  797
<211>  10
<212>  PRT
<213>  Artificial sequence

<220>

<223>  HLA restricted peptide

<400>  797

Thr Gln Tyr Glu Ser Gly Ser Met Asp Lys
1               5                   10


<210>  798
<211>  9
<212>  PRT
<213>  Artificial sequence

<220>
<223>  HLA restricted peptide

<400>  798

Thr Ser Ala Gly His Phe Pro Arg Ala
1               5


<210>  799
<211>  10
<212>  PRT
<213>  Artificial sequence

<220>
<223>  HLA restricted peptide

<400>  799

Thr Ser Ala Gly His Phe Pro Arg Ala Cys
1               5                   10


<210>  800
<211>  9
<212>  PRT
<213>  Artificial sequence

<220>
<223>  HLA restricted peptide

<400>  800

Thr Tyr Gly Gln Met Lys Asn Gly Ser
1               5


<210>  801
<211>  10
<212>  PRT
<213>  Artificial sequence

<220>
<223>  HLA restricted peptide

<400>  801

Thr Tyr Gly Gln Met Lys Asn Gly Ser Thr
1               5                   10


<210>  802

<211>  9
<212>  PRT
<213>  Artificial sequence

<220>
<223>  HLA restricted peptide

<400>  802

Val Cys Ser Arg Leu Glu Glu Tyr Asn
1               5


<210>  803
<211>  10
<212>  PRT
<213>  Artificial sequence

<220>
<223>  HLA restricted peptide

<400>  803

Val Cys Ser Arg Leu Glu Glu Tyr Asn Ser
1               5                   10


<210>  804
<211>  9
<212>  PRT
<213>  Artificial sequence

<220>
<223>  HLA restricted peptide

<400>  804

Val Asp Asp Arg Glu Ser Trp Pro Ser
1               5


<210>  805
<211>  10
<212>  PRT
<213>  Artificial sequence

<220>
<223>  HLA restricted peptide

<400>  805

Val Asp Asp Arg Glu Ser Trp Pro Ser Val
1               5                   10


<210>  806
<211>  9
<212>  PRT
<213>  Artificial sequence

<220>
<223>  HLA restricted peptide

<400>  806

Val Asp Ser Ile Phe Glu Gln Trp Leu
1               5

<210>  807
<211>  10
<212>  PRT
<213>  Artificial sequence

<220>
<223>  HLA restricted peptide

<400>  807

Val Asp Ser Ile Phe Glu Gln Trp Leu Arg
1               5                   10

<210>  808
<211>  9
<212>  PRT
<213>  Artificial sequence

<220>
<223>  HLA restricted peptide

<400>  808

Val Glu Phe Cys Leu Ser Leu Thr Gln
1               5

<210>  809
<211>  10
<212>  PRT
<213>  Artificial sequence

<220>
<223>  HLA restricted peptide

<400>  809

Val Glu Phe Cys Leu Ser Leu Thr Gln Tyr
1               5                   10

<210>  810
<211>  9
<212>  PRT
<213>  Artificial sequence

<220>
<223>  HLA restricted peptide

<400>  810

Val Phe Tyr Asn Arg Thr Cys Gln Cys
1               5

<210>  811
<211>  10
<212>  PRT
<213>  Artificial sequence

<220>
<223>  HLA restricted peptide

<400>  811

Val Phe Tyr Asn Arg Thr Cys Gln Cys Ser
1               5                   10


<210>  812
<211>  9
<212>  PRT
<213>  Artificial sequence

<220>
<223>  HLA restricted peptide

<400>  812

Val Gly Ala Val Leu Thr Ala Leu Leu
1               5


<210>  813
<211>  10
<212>  PRT
<213>  Artificial sequence

<220>
<223>  HLA restricted peptide

<400>  813

Val Gly Ala Val Leu Thr Ala Leu Leu Ala
1               5                   10


<210>  814
<211>  10
<212>  PRT
<213>  Artificial sequence

<220>
<223>  HLA restricted peptide

<400>  814

Val Ile Pro Ile Gly Thr Tyr Gly Gln Met
1               5                   10


<210>  815
<211>  9
<212>  PRT
<213>  Artificial sequence

<220>
<223>  HLA restricted peptide

<400>  815

Val Leu Thr Ala Leu Leu Ala Gly Leu
1               5

<210>  816
<211>  10
<212>  PRT
<213>  Artificial sequence

<220>
<223>  HLA restricted peptide

<400>  816

Val Leu Thr Ala Leu Leu Ala Gly Leu Val
1               5                   10


<210>  817
<211>  9
<212>  PRT
<213>  Artificial sequence

<220>
<223>  HLA restricted peptide

<400>  817

Val Leu Tyr Cys Leu Leu Trp Ser Phe
1               5


<210>  818
<211>  10
<212>  PRT
<213>  Artificial sequence

<220>
<223>  HLA restricted peptide

<400>  818

Val Leu Tyr Cys Leu Leu Trp Ser Phe Gln
1               5                   10


<210>  819
<211>  9
<212>  PRT
<213>  Artificial sequence

<220>
<223>  HLA restricted peptide

<400>  819

Val Pro Phe Ile Pro Leu Tyr Arg Asn
1               5


<210>  820
<211>  10
<212>  PRT
<213>  Artificial sequence

<220>
<223>  HLA restricted peptide

<400>  820

Val Pro Phe Ile Pro Leu Tyr Arg Asn Gly
1               5                   10


<210>  821
<211>  9
<212>  PRT
<213>  Artificial sequence

<220>
<223>  HLA restricted peptide

<400>  821

Val Gln Gly Ser Ala Asn Asp Pro Ile
1               5


<210>  822
<211>  10
<212>  PRT
<213>  Artificial sequence

<220>
<223>  HLA restricted peptide

<400>  822

Val Gln Gly Ser Ala Asn Asp Pro Ile Phe
1               5                   10


<210>  823
<211>  9
<212>  PRT
<213>  Artificial sequence

<220>
<223>  HLA restricted peptide

<400>  823

Val Arg Arg Asn Ile Phe Asp Leu Ser
1               5


<210>  824
<211>  10
<212>  PRT
<213>  Artificial sequence

<220>
<223>  HLA restricted peptide

<400>  824

Val Arg Arg Asn Ile Phe Asp Leu Ser Ala
1               5                   10


<210>  825
<211>  9
<212>  PRT
<213>  Artificial sequence

<220>
<223>  HLA restricted peptide

<400>  825

Val Ser Leu Leu Cys Arg His Lys Arg
1               5


<210>  826
<211>  10
<212>  PRT
<213>  Artificial sequence

<220>
<223>  HLA restricted peptide

<400>  826

Val Ser Leu Leu Cys Arg His Lys Arg Lys
1               5                   10


<210>  827
<211>  10
<212>  PRT
<213>  Artificial sequence

<220>
<223>  HLA restricted peptide

<400>  827

Val Ser Met Asp Ala Leu Leu Gly Gly Tyr
1               5                   10


<210>  828
<211>  9
<212>  PRT
<213>  Artificial sequence

<220>
<223>  HLA restricted peptide

<400>  828

Val Ser Ser Lys Asn Leu Met Glu Lys
1               5


<210>  829
<211>  9
<212>  PRT
<213>  Artificial sequence

<220>
<223>  HLA restricted peptide

<400>  829

Val Trp Ile His Tyr Tyr Val Ser Met
1               5

<210>  830
<211>  9
<212>  PRT
<213>  Artificial sequence

<220>
<223>  HLA restricted peptide

<400>  830

Val Tyr Pro Glu Ala Asn Ala Pro Ile
1               5


<210>  831
<211>  9
<212>  PRT
<213>  Artificial sequence

<220>
<223>  HLA restricted peptide

<400>  831

Trp Asp Trp Arg Asp Ala Glu Lys Cys
1               5


<210>  832
<211>  9
<212>  PRT
<213>  Artificial sequence

<220>
<223>  HLA restricted peptide

<400>  832

Trp Glu Gln Glu Ile Gln Lys Leu Thr
1               5


<210>  833
<211>  9
<212>  PRT
<213>  Artificial sequence

<220>
<223>  HLA restricted peptide

<400>  833

Trp Gly Pro Asn Cys Thr Glu Arg Arg
1               5


<210>  834
<211>  10
<212>  PRT
<213>  Artificial sequence

<220>
<223>  HLA restricted peptide

<400>  834

Trp Gly Pro Asn Cys Thr Glu Arg Arg Leu
1               5                   10


<210>  835
<211>  9
<212>  PRT
<213>  Artificial sequence

<220>
<223>  HLA restricted peptide

<400>  835

Trp His Arg Leu Phe Leu Leu Arg Trp
1               5


<210>  836
<211>  10
<212>  PRT
<213>  Artificial sequence

<220>
<223>  HLA restricted peptide

<400>  836

Trp Ile His Tyr Tyr Val Ser Met Asp Ala
1               5                   10


<210>  837
<211>  10
<212>  PRT
<213>  Artificial sequence

<220>
<223>  HLA restricted peptide

<400>  837

Trp Leu Leu Gly Ala Ala Met Val Gly Ala
1               5                   10


<210>  838
<211>  9
<212>  PRT
<213>  Artificial sequence

<220>
<223>  HLA restricted peptide

<400>  838

Trp Pro Ser Val Phe Tyr Asn Arg Thr
1               5


<210>  839
<211>  10
<212>  PRT

<213>  Artificial sequence

<220>
<223>  HLA restricted peptide

<400>  839

Trp Pro Ser Val Phe Tyr Asn Arg Thr Cys
1               5                   10


<210>  840
<211>  10
<212>  PRT
<213>  Artificial sequence

<220>
<223>  HLA restricted peptide

<400>  840

Trp Gln Ile Val Cys Ser Arg Leu Glu Glu
1               5                   10


<210>  841
<211>  9
<212>  PRT
<213>  Artificial sequence

<220>
<223>  HLA restricted peptide

<400>  841

Trp Arg Asp Ala Glu Lys Cys Asp Ile
1               5


<210>  842
<211>  10
<212>  PRT
<213>  Artificial sequence

<220>
<223>  HLA restricted peptide

<400>  842

Trp Arg Asp Ala Glu Lys Cys Asp Ile Cys
1               5                   10


<210>  843
<211>  9
<212>  PRT
<213>  Artificial sequence

<220>
<223>  HLA restricted peptide

<400>  843

Trp Arg Asp Ile Asp Phe Ala His Glu
1               5

<210>  844
<211>  10
<212>  PRT
<213>  Artificial sequence

<220>
<223>  HLA restricted peptide

<400>  844

Trp Arg Asp Ile Asp Phe Ala His Glu Ala
1               5                   10


<210>  845
<211>  9
<212>  PRT
<213>  Artificial sequence

<220>
<223>  HLA restricted peptide

<400>  845

Trp Ser Phe Gln Thr Ser Ala Gly His
1               5


<210>  846
<211>  10
<212>  PRT
<213>  Artificial sequence

<220>
<223>  HLA restricted peptide

<400>  846

Trp Ser Phe Gln Thr Ser Ala Gly His Phe
1               5                   10


<210>  847
<211>  9
<212>  PRT
<213>  Artificial sequence

<220>
<223>  HLA restricted peptide

<400>  847

Trp Ser Trp Leu Leu Gly Ala Ala Met
1               5


<210>  848
<211>  10
<212>  PRT
<213>  Artificial sequence

<220>
<223>  HLA restricted peptide

<400>  848

Trp Ser Trp Leu Leu Gly Ala Ala Met Val
1               5                   10


<210>  849
<211>  9
<212>  PRT
<213>  Artificial sequence

<220>
<223>  HLA restricted peptide

<400>  849

Tyr Cys Leu Leu Trp Ser Phe Gln Thr
1               5


<210>  850
<211>  10
<212>  PRT
<213>  Artificial sequence

<220>
<223>  HLA restricted peptide

<400>  850

Tyr Cys Leu Leu Trp Ser Phe Gln Thr Ser
1               5                   10


<210>  851
<211>  9
<212>  PRT
<213>  Artificial sequence

<220>
<223>  HLA restricted peptide

<400>  851

Tyr Asp Leu Phe Val Trp Ile His Tyr
1               5


<210>  852
<211>  10
<212>  PRT
<213>  Artificial sequence

<220>
<223>  HLA restricted peptide

<400>  852

Tyr Asp Leu Phe Val Trp Ile His Tyr Tyr
1               5                   10


<210>  853
<211>  9

<212>  PRT
<213>  Artificial sequence

<220>
<223>  HLA restricted peptide

<400>  853

Tyr Asp Tyr Ser Tyr Leu Gln Asp Ser
1               5


<210>  854
<211>  9
<212>  PRT
<213>  Artificial sequence

<220>
<223>  HLA restricted peptide

<400>  854

Tyr Glu Ile Trp Arg Asp Ile Asp Phe
1               5


<210>  855
<211>  10
<212>  PRT
<213>  Artificial sequence

<220>
<223>  HLA restricted peptide

<400>  855

Tyr Glu Ile Trp Arg Asp Ile Asp Phe Ala
1               5                   10


<210>  856
<211>  9
<212>  PRT
<213>  Artificial sequence

<220>
<223>  HLA restricted peptide

<400>  856

Tyr Glu Ser Gly Ser Met Asp Lys Ala
1               5


<210>  857
<211>  10
<212>  PRT
<213>  Artificial sequence

<220>
<223>  HLA restricted peptide

<400>  857

Tyr Glu Ser Gly Ser Met Asp Lys Ala Ala

1                 5                   10


<210>  858
<211>  9
<212>  PRT
<213>  Artificial sequence

<220>
<223>  HLA restricted peptide

<400>  858

Tyr Gly Gln Met Lys Asn Gly Ser Thr
1               5


<210>  859
<211>  9
<212>  PRT
<213>  Artificial sequence

<220>
<223>  HLA restricted peptide

<400>  859

Tyr His Ser Leu Tyr Gln Ser His Leu
1               5


<210>  860
<211>  9
<212>  PRT
<213>  Artificial sequence

<220>
<223>  HLA restricted peptide

<400>  860

Tyr Ile Lys Ser Tyr Leu Glu Gln Ala
1               5


<210>  861
<211>  10
<212>  PRT
<213>  Artificial sequence

<220>
<223>  HLA restricted peptide

<400>  861

Tyr Ile Lys Ser Tyr Leu Glu Gln Ala Ser
1               5                   10


<210>  862
<211>  9
<212>  PRT
<213>  Artificial sequence

<220>

<223>  HLA restricted peptide

<400>  862

Tyr Leu Glu Gln Ala Ser Arg Ile Trp
1               5


<210>  863
<211>  10
<212>  PRT
<213>  Artificial sequence

<220>
<223>  HLA restricted peptide

<400>  863

Tyr Leu Glu Gln Ala Ser Arg Ile Trp Ser
1               5                   10


<210>  864
<211>  9
<212>  PRT
<213>  Artificial sequence

<220>
<223>  HLA restricted peptide

<400>  864

Tyr Leu Gln Asp Ser Asp Pro Asp Ser
1               5


<210>  865
<211>  10
<212>  PRT
<213>  Artificial sequence

<220>
<223>  HLA restricted peptide

<400>  865

Tyr Leu Gln Asp Ser Asp Pro Asp Ser Phe
1               5                   10


<210>  866
<211>  9
<212>  PRT
<213>  Artificial sequence

<220>
<223>  HLA restricted peptide

<400>  866

Tyr Leu Thr Leu Ala Lys His Thr Ile
1               5


<210>  867

<211>  10
<212>  PRT
<213>  Artificial sequence

<220>
<223>  HLA restricted peptide

<400>  867

Tyr Leu Thr Leu Ala Lys His Thr Ile Ser
1               5                   10


<210>  868
<211>  9
<212>  PRT
<213>  Artificial sequence

<220>
<223>  HLA restricted peptide

<400>  868

Tyr Met Asp Gly Thr Met Ser Gln Val
1               5


<210>  869
<211>  9
<212>  PRT
<213>  Artificial sequence

<220>
<223>  HLA restricted peptide

<400>  869

Tyr Met Gly Gly Gln His Pro Thr Asn
1               5


<210>  870
<211>  9
<212>  PRT
<213>  Artificial sequence

<220>
<223>  HLA restricted peptide

<400>  870

Tyr Met Asn Gly Thr Met Ser Gln Val
1               5


<210>  871
<211>  9
<212>  PRT
<213>  Artificial sequence

<220>
<223>  HLA restricted peptide

<400>  871

Tyr Met Val Pro Phe Ile Pro Leu Tyr
1               5

<210>  872
<211>  10
<212>  PRT
<213>  Artificial sequence

<220>
<223>  HLA restricted peptide

<400>  872

Tyr Met Val Pro Phe Ile Pro Leu Tyr Arg
1               5                   10

<210>  873
<211>  10
<212>  PRT
<213>  Artificial sequence

<220>
<223>  HLA restricted peptide

<400>  873

Tyr Asn Arg Thr Cys Gln Cys Ser Gly Asn
1               5                   10

<210>  874
<211>  9
<212>  PRT
<213>  Artificial sequence

<220>
<223>  HLA restricted peptide

<400>  874

Tyr Asn Ser His Gln Ser Leu Cys Asn
1               5

<210>  875
<211>  9
<212>  PRT
<213>  Artificial sequence

<220>
<223>  HLA restricted peptide

<400>  875

Tyr Pro Glu Ala Asn Ala Pro Ile Gly
1               5

<210>  876
<211>  10
<212>  PRT
<213>  Artificial sequence

<220>
<223>  HLA restricted peptide

<400>  876

Tyr Pro Glu Ala Asn Ala Pro Ile Gly His
1               5                   10


<210>  877
<211>  9
<212>  PRT
<213>  Artificial sequence

<220>
<223>  HLA restricted peptide

<400>  877

Tyr Arg Asn Gly Asp Phe Phe Ile Ser
1               5


<210>  878
<211>  10
<212>  PRT
<213>  Artificial sequence

<220>
<223>  HLA restricted peptide

<400>  878

Tyr Arg Asn Gly Asp Phe Phe Ile Ser Ser
1               5                   10


<210>  879
<211>  9
<212>  PRT
<213>  Artificial sequence

<220>
<223>  HLA restricted peptide

<400>  879

Tyr Val Ile Pro Ile Gly Thr Tyr Gly
1               5


<210>  880
<211>  10
<212>  PRT
<213>  Artificial sequence

<220>
<223>  HLA restricted peptide

<400>  880

Tyr Val Ile Pro Ile Gly Thr Tyr Gly Gln
1               5                   10

<210>  881
<211>  9
<212>  PRT
<213>  Artificial sequence

<220>
<223>  HLA restricted peptide

<400>  881

Tyr Trp Asp Trp Arg Asp Ala Glu Lys
1               5


<210>  882
<211>  10
<212>  PRT
<213>  Artificial sequence

<220>
<223>  HLA restricted peptide

<400>  882

Tyr Trp Asp Trp Arg Asp Ala Glu Lys Cys
1               5                   10


<210>  883
<211>  9
<212>  PRT
<213>  Artificial sequence

<220>
<223>  HLA restricted peptide

<400>  883

Tyr Tyr Val Ser Met Asp Ala Leu Leu
1               5


<210>  884
<211>  219
<212>  PRT
<213>  Artificial sequence

<220>
<223>  MC1 Ab, VL-CL polypeptide sequence

<400>  884

Asp Val Val Met Thr Gln Ser Pro Leu Ser Leu Pro Val Thr Pro Gly
1               5                   10                  15


Glu Pro Ala Ser Ile Ser Cys Arg Ser Ser Gln Ser Leu Leu His Ser
            20                  25                  30


Ile Gly Tyr Asn Tyr Leu His Trp Phe Leu Gln Lys Pro Gly Gln Ser
        35                  40                  45


Pro Gln Leu Leu Ile Tyr Leu Gly Ser Asn Arg Ala Ser Gly Val Pro

50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Leu Gln Thr Pro Pro Thr Phe Gly Gln Gly Thr Arg Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  885
<211>  225
<212>  PRT
<213>  Artificial sequence

<220>
<223>  MC1 Ab, VH-CH polypeptide sequence

<400>  885

Gln Leu Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Val Gly Ser Pro Tyr Gly Asp Tyr Val Leu Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Ser Asn Thr Lys Val Asp Lys Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Lys Val Glu Pro Lys Ser Cys Ala Ala
    210                 215                 220

Ala
225

<210>  886
<211>  16
<212>  PRT
<213>  Artificial sequence

<220>
<223>  B2 VL-CL CDR1

<400>  886

Arg Ser Ser Gln Ser Leu Leu His Ser Ile Gly Tyr Asn Tyr Leu Asp
1               5                   10                  15

<210>  887
<211>  7
<212>  PRT

<213>  Artificial sequence

<220>
<223>  B2 VL-CL CDR2

<400>  887

Leu Gly Ser Asn Arg Ala Ser
1               5


<210>  888
<211>  9
<212>  PRT
<213>  Artificial sequence

<220>
<223>  B2 VL-CL CDR3

<400>  888

Met Gln Ala Leu Gln Thr Pro Leu Thr
1               5


<210>  889
<211>  5
<212>  PRT
<213>  Artificial sequence

<220>
<223>  B2 VH-CH CDR1

<400>  889

Ser Tyr Gly Met His
1               5


<210>  890
<211>  17
<212>  PRT
<213>  Artificial sequence

<220>
<223>  B2 VH-CH CDR2

<400>  890

Val Ile Ser Tyr Asp Gly Ser Asn Lys Tyr Tyr Thr Asp Ser Val Asn
1               5                   10                  15


Gly



<210>  891
<211>  12
<212>  PRT
<213>  Artificial sequence

<220>
<223>  B2 VH-CH CDR3

<400>  891

Gly Gly Gly Tyr Tyr Glu Thr Ser Gly Pro Asp Tyr
1               5                   10


<210>  892
<211>  219
<212>  PRT
<213>  Artificial sequence

<220>
<223>  B2 Ab, VL-CL polypeptide sequence

<400>  892

Glu Ile Val Leu Thr Gln Ser Pro Leu Ser Leu Pro Val Thr Pro Gly
1               5                   10                  15


Glu Pro Ala Ser Ile Ser Cys Arg Ser Ser Gln Ser Leu Leu His Ser
            20                  25                  30


Ile Gly Tyr Asn Tyr Leu Asp Trp Tyr Leu Gln Lys Pro Gly Gln Ser
        35                  40                  45


Pro Gln Leu Leu Ile Tyr Leu Gly Ser Asn Arg Ala Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Val Gly Val Tyr Tyr Cys Met Gln Ala
                85                  90                  95


Leu Gln Thr Pro Leu Thr Phe Gly Gly Gly Thr Lys Val Glu Ile Lys
            100                 105                 110


Arg Thr Val Ala Ala Pro Ser Val Phe Ile Phe Pro Pro Ser Asp Glu
        115                 120                 125


Gln Leu Lys Ser Gly Thr Ala Ser Val Val Cys Leu Leu Asn Asn Phe
    130                 135                 140


Tyr Pro Arg Glu Ala Lys Val Gln Trp Lys Val Asp Asn Ala Leu Gln
145                 150                 155                 160


Ser Gly Asn Ser Gln Glu Ser Val Thr Glu Gln Asp Ser Lys Asp Ser
                165                 170                 175


Thr Tyr Ser Leu Ser Ser Thr Leu Thr Leu Ser Lys Ala Asp Tyr Glu
            180                 185                 190


Lys His Lys Val Tyr Ala Cys Glu Val Thr His Gln Gly Leu Ser Ser

195                 200                 205

Pro Val Thr Lys Ser Phe Asn Arg Gly Glu Cys
    210                 215

<210>  893
<211>  227
<212>  PRT
<213>  Artificial sequence

<220>
<223>  B2 Ab, VH-CH polypeptide sequence

<400>  893

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Arg Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Thr Asp Ser Val
    50                  55                  60

Asn Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Gly Tyr Tyr Glu Thr Ser Gly Pro Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Ser Asn Thr Lys Val Asp Lys Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

Leu Ser Ser Val Val Thr Val Pro Ser Ser Ser Leu Gly Thr Gln Thr
        195                 200                 205

Tyr Ile Cys Asn Val Asn His Lys Pro Lys Val Glu Pro Lys Ser Cys
    210                 215                 220

Ala Ala Ala
225

<210>  894
<211>  16
<212>  PRT
<213>  Artificial sequence

<220>
<223>  MC1 VL-CL CDR1

<400>  894

Arg Ser Ser Gln Ser Leu Leu His Ser Ile Gly Tyr Asn Tyr Leu His
1               5                   10                  15

<210>  895
<211>  7
<212>  PRT
<213>  Artificial sequence

<220>
<223>  MC1 VL-CL CDR2

<400>  895

Leu Gly Ser Asn Arg Ala Ser
1               5

<210>  896
<211>  9
<212>  PRT
<213>  Artificial sequence

<220>
<223>  MC1 VL-CL CDR3

<400>  896

Met Gln Ala Leu Gln Thr Pro Pro Thr
1               5

<210>  897
<211>  6
<212>  PRT
<213>  Artificial sequence

<220>
<223>  MC1 VH-CH CDR1

<400>  897

Ser Ser Asn Trp Trp Ser

1               5


<210>  898
<211>  16
<212>  PRT
<213>  Artificial sequence

<220>
<223>  MC1 VH-CH CDR2

<400>  898

Glu Ile Tyr His Ser Gly Ser Thr Asn Tyr Asn Pro Ser Leu Lys Ser
1               5                   10                  15


<210>  899
<211>  10
<212>  PRT
<213>  Artificial sequence

<220>
<223>  MC1 VH-CH CDR3

<400>  899

Ser Pro Tyr Gly Asp Tyr Val Leu Asp Tyr
1               5                   10


<210>  900
<211>  15
<212>  PRT
<213>  Artificial sequence

<220>
<223>  A fusion peptide for site specific biotinylation



<220>
<221>  misc_feature
<222>  (9)..(9)
<223>  Residue undergoing biotinylation by the BirA biotin ligase enzyme

<400>  900

Leu His His Ile Leu Asp Ala Gln Lys Met Val Trp Asn His Arg
1               5                   10                  15


<210>  901
<211>  23
<212>  DNA
<213>  Artificial sequence

<220>
<223>  Single strand DNA oligonucleotide

<400>  901
agcggataac aatttcacac agg                                              23


<210>  902
<211>  24

<212>  DNA
<213>  Artificial sequence

<220>
<223>  Single strand DNA oligonucleotide

<400>  902
tttgtcgtct ttccagacgt tagt                                              24


<210>  903
<211>  336
<212>  DNA
<213>  Artificial sequence

<220>
<223>  MC1 Ab, VL coding sequence

<400>  903
gatgttgtga tgactcagtc tccactctcc ctgcccgtca cccctggaga gccggcctcc      60

atctcctgca ggtcaagtca gagcctcctg catagtattg gatacaacta tttgcattgg     120

ttcctgcaga agccagggca gtctccacaa ctcctgatct acctgggttc taatcgggcc     180

tccggggtcc ctgacaggtt cagtggcagt ggatcaggca cagattttac actgaaaatc     240

agcagagtgg aggctgagga tgttggggtt tattactgca tgcaagctct gcaaactcct     300

cccaccttcg gccaagggac acgactggag attaaa                               336


<210>  904
<211>  321
<212>  DNA
<213>  Artificial sequence

<220>
<223>  MC1 Ab, CL coding sequence

<400>  904
cgaactgtgg ctgcaccatc tgtcttcatc ttcccgccat ctgatgagca gttgaaatct      60

ggaactgcct ctgttgtgtg cctgctgaat aacttctatc ccagagaggc caaagtacag     120

tggaaggtgg ataacgccct ccaatcgggt aactcccagg agagtgtcac agagcaggac     180

agcaaggaca gcacctacag cctcagcagc accctgacgc tgagcaaagc agactacgag     240

aaacacaaag tctacgcctg cgaagtcacc catcagggcc tgagctcgcc cgtcacaaag     300

agcttcaaca ggggagagtg t                                               321


<210>  905
<211>  357
<212>  DNA
<213>  Artificial sequence

<220>
<223>  MC1 Ab, VH coding sequence

<400>  905
cagctgcagc tgcaggagtc gggcccagga ctggtgaagc cttcggggac cctgtccctc      60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgt tgggtctccc 300

tacggtgact acgtccttga ctactggggc cagggcaccc tggtcaccgtctcaagc 357

<210> 906
<211> 390
<212> DNA
<213> Artificial sequence

<220>
<223> MC1 Ab, CH coding sequence

<400> 906
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtc cacaccttcc cggctgtcct acagtcctca 180

ggactctact ccctcagcag cgtagtgacc gtgccctcca gcagcttggg cacccagacc 240

tacatctgca acgtgaatca caagcccagc aacaccaagg tggacaagaa agttgagccc 300

aaatcttgtg cggccgcaca tcatcatcac catcacgggg ccgcagaaca aaaactcatc 360

tcagaagagg atctgaatgg ggccgcataa 390

<210> 907
<211> 336
<212> DNA
<213> Artificial sequence

<220>
<223> B2 Ab, VL coding sequence

<400> 907
gaaattgtgc tgactcagtc tccactctcc ctgcccgtca cccctggaga gccggcctcc 60

atctcctgca ggtctagtca gagcctcctg catagtattg gatacaacta tttggattgg 120

tacctgcaga agccagggca gtctccacag ctcctgatct atttgggttc taatcgggcc 180

tccggggtcc ctgacaggtt cagtggcagt ggatcaggca cagattttac actgaaaatc 240

agcagagtgg aggctgagga tgttggggtt tattactgca tgcaagctct acaaactcct 300

ctcactttcg gcggagggac caaggtggag atcaaa 336

<210> 908
<211> 321
<212> DNA
<213> Artificial sequence

<220>
<223> B2 Ab, CL coding sequence

<400>  908
cgaactgtgg ctgcaccatc tgtcttcatc ttcccgccat ctgatgagca gttgaaatct      60

ggaactgcct ctgttgtgtg cctgctgaat aacttctatc ccagagaggc caaagtacag     120

tggaaggtgg ataacgccct ccaatcgggt aactcccagg agagtgtcac agagcaggac     180

agcaaggaca gcacctacag cctcagcagc accctgacgc tgagcaaagc agactacgag     240

aaacacaaag tctacgcctg cgaagtcacc catcagggcc tgagctcgcc cgtcacaaag     300

agcttcaaca ggggagagtg t                                               321


<210>  909
<211>  348
<212>  DNA
<213>  Artificial sequence

<220>
<223>  B2 Ab, VH coding sequence

<400>  909
gaggtgcagc tcgtggagtc tgggggaggc gtggtccagc ctgggaggtc cctgagactc      60

tcctgtgcag cgtctggatt caccttcagg agctatggca tgcactgggt ccggcaggct     120

ccaggcaagg ggctggagtg ggtggcagtt atatcatatg atggaagtaa taaatactat     180

acagactccg taaacggccg gttcaccatc tccagagaca attccaagaa cacgctgtat     240

ctgcaaatga acagcctgag agctgaggac acggctgtgt attactgtgc gagaggaggg     300

ggttactatg aaactagtgg ccctgactac tggggccagg gaaccctg                  348


<210>  910
<211>  390
<212>  DNA
<213>  Artificial sequence

<220>
<223>  B2 Ab, CH coding sequence

<400>  910
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtc cacaccttcc cggctgtcct acagtcctca     180

ggactctact ccctcagcag cgtagtgacc gtgccctcca gcagcttggg cacccagacc     240

tacatctgca acgtgaatca caagcccagc aacaccaagg tggacaagaa agttgagccc     300

aaatcttgtg cggccgcaca tcatcatcac catcacgggg ccgcagaaca aaaactcatc     360

tcagaagagg atctgaatgg ggccgcataa                                      390

## Description

### FIELD AND BACKGROUND OF THE INVENTION

The present invention relates to methods of treating and diagnosing melanoma and, more particularly, to antibodies capable of same.

A key advance in immunology in the past decade has been the elucidation of the antigenic basis of tumor-cell recognition and destruction. The ultimate effector cell that mediates the immune activity against tumors is the cytotoxic T cell (CTL). Protein antigens, recognized by CTLs through their clonotypic and specific T cell receptor, consist of peptide fragments which are bound within the antigen binding cleft of the major histocompatibility complex (MHC) class I molecules on the cell surface. Antigens are exposed to immune-system scrutiny by loading peptide fragments of newly synthesized cellular proteins onto MHC-class-I molecules, which are then transported to the cell surface.

As in normal cells, the surface of tumor cells contains MHC-peptide antigens that reflect their expressed 'proteome'. Tumor antigens include the gene products arising *de novo* which are unique to individual cancer cells (e.g., an epitope from a mutated β-catenin gene), the gene products derived from an aberrant expression of non-mutated genes (e.g., NY-ESO-1), the products of which are normally expressed only in testes or fetal tissues and non-mutated, and cell-lineage-specific proteins (also termed "differentiation antigens"). A similar diversity of tumor antigens is recognized by MHC class II-restricted CD4+ T cells.

Melanomas are aggressive, frequently metastatic tumors derived from either melanocytes or melanocyte related nevus cells. Even when melanoma is apparently localized to the skin, up to 30 % of patients develop systemic metastasis. Classic treatment modalities of melanoma include surgery, radiation and chemotherapy. In the past decade immunotherapy and gene therapy have emerged as new and promising methods for treating melanoma.

Shared melanoma-associated antigens (Ag) (e.g., MART-1, gp100 and Tyrosinase) expressed among a variety of melanoma patients can be recognized by cytotoxic CD8+ T lymphocytes derived from melanoma patients. T-cell lines which specifically recognize the HLA restricted antigens expressed by tumor cells were generated and used to identify the genes encoding tumor antigens and the peptide epitopes derived therefrom. The fate of a specific T cell clone is determined in a secondary lymphoid organ where it is "educated" by a professional antigen presenting cell (pAPC). Upon pAPC encounter, a constellation of events ensues, *inter alia*, co-stimulatory signaling, various interactions between membrane determinants in the immunological synapse, as well as cytokines secreted by the pAPC and the T cell, which ultimately determine whether a specific T cell clone will undergo an activation process, tolerance, or apoptosis. Activation of a T cell effectuates morphological, genetic, and biochemical changes that enhance its proliferation, migration, and effector functions. Activated CTLs survive only days to weeks after elimination of the antigen source; however a small proportion of these T cells transform and constitute subpopulations of memory T cells with distinctly different surface markers.

Current immunotherapy approaches are designed to induce and enhance T cell reactivity against tumor antigens. Intensive research on cancer peptides has culminated in clinical trials involving therapeutic vaccination of cancer patients with antigenic peptides or proteins. Such vaccinations often result in tumor-reactive CTLs in patients (see for example, A. Lev, *et al.*, 2002; C. J. Cohen, *et al.*, 2003). U.S. Pat. Appl. No. 20050158332 discloses MHC Class II restricted melanoma antigens of the tyrosinase sequence recognized by CD4+ T-lymphocytes and their therapeutic use in increasing immunogenicity by enhancing the binding of the peptide to the MHC Class II molecule. U.S. Pat. Appl. No. 20030144482 discloses MART-1 antigenic peptides capable of causing a cellular or humoral immune response in a mammal. However, vaccination alone only sporadically induces tumor regression in patients with metastatic disease. In addition, even in transgenic mice in which all T cells have been engineered to express a tumor-reactive TCR, tumors still progressively grow.

The lack of inflammatory rejection of tumors by immunized patients and TCR transgenic mice is not well understood at the cellular and molecular level. Many mechanisms could account for the failure of antigen-specific CD8+ T cells to eliminate antigen-expressing tumor cells *in vivo*. For instance, the tumor-antigen-specific T cells themselves could be functionally deficient, rendered anergic, or unable to fully differentiate in the tumor environment. In addition, the tumor environment could lack a danger signal' or other innate immune stimulation, preventing a general inflammatory reaction from evolving. Alternatively, active immune regulatory mechanisms such as CD4+CD25+ T cells might impede any endogenous immune reaction to cancer cells. Whatever the mechanism, without an inflammatory immune response, the CD8+ T cells of the adaptive immune system are rendered ineffective. As a tumor grows and metastasizes, additional systemic immune suppression could develop, and antigen-escape variants of the tumor could arise.

Several studies have demonstrated that the inability of a patient's immune system to elicit an effective immune response against a tumor is often due to poor antigen presentation [Koopman LA,et al J Exp Med 20: 961-976; Garrido F, et al., Immunol Today 18: 89-95;Rosenberg SA, Bennink JR. 1993. J Exp Med 177: 265-272]. In addition, the CD8+ T cell-mediated cytotoxic effect on targeted pathogenic cells requires at least a minimum density of antigen presentation. However, to date, the mechanisms of antigen presentation are not fully understood.

The advent of MHC-peptide tetramers has provided a new tool for studying antigen-specific T cell populations in health and disease, by monitoring tetramer-T cell binding via flow cytometry. However, to date there are limited tools available to detect, visualize, count, and study antigen (i.e., MHC-peptide complex) presentation. Antibodies that specifically recognize class I MHC-peptide complexes have been previously described, see for example U.S. Pat. Appl. No. 20050255101. However, the currently available antibodies could not explain the hyporesponsiveness of T cells to melanoma differentiation antigens. In addition, specific antibodies directed against MHC-I molecules complexed with additional melanoma differentiation antigens such as Mart-1 or Tyrosinase have not as yet been described.

There is thus a widely recognized need for, and it would be highly advantageous to have, TCR-like antibodies capable of diagnosing and treating melanoma devoid of the above limitations.

### SUMMARY OF THE INVENTION

According to an aspect of the present invention there is provided an antibody comprising an antigen recognition domain capable of binding to an MHC-I molecule being complexed with a tyrosinase peptide comprising an amino acid sequence as set forth in SEQ ID NO: 1, wherein the antibody does not bind the MHC-I in the absence of the complexed peptide, and wherein the antibody does not bind the peptide in an absence of the MHC.

According to an aspect of the present invention there is provided a pharmaceutical composition-comprising the antibody of the present invention.

According to an aspect of the present invention there is provided a method of detecting a melanoma cell, comprising contacting the cell with an antibody comprising an antigen recognition domain capable of binding to an MHC-I molecule being complexed with a tyrosinase peptide, wherein the antibody does not bind the MHC-I in the absence of the complexed peptide, and wherein the antibody does not bind the peptide in an absence of the MHC, under conditions which allow immunocomplex formation, wherein a presence of the immunocomplex or level thereof is indicative of the melanoma cell.

According to an aspect of the present invention there is provided a method of diagnosing a melanoma in a subject in need thereof, comprising contacting a cell of the subject with an antibody comprising an antigen recognition domain capable of binding to an MHC-I molecule being complexed with a tyrosinase peptide, wherein the antibody does not bind the MHC-I in the absence of the complexed peptide, and wherein the antibody does not bind the peptide in an absence of the MHC, under conditions which allow immunocomplex formation, wherein a presence of the immunocomplex or level thereof is indicative of the melanoma.

According to an aspect of the present invention there is provided a method of identifying if a subject is suitable for TCRL-based epitope directed therapy, comprising determining a level of epitope presentation on at least one cell of the subject using an antibody comprising an antigen recognition domain capable of binding to an MHC-I molecule being complexed with a peptide fragment of the antigen, wherein the antibody does not bind the MHC-I in the absence of the complexed peptide fragment of the antigen, and wherein the antibody does not bind the peptide fragment of the antigen in an absence of the MHC, wherein a level value higher than a predetermined threshold is indicative of an individual being suitable for TCRL-based epitope directed therapy.

According to an embodiment of this aspect of the present invention, a level value below a predetermined threshold is indicative of an individual not being suitable for TCRL-based epitope directed therapy.

According to an aspect of the present invention there is provided a method of identifying if a subject is suitable for CTL-based epitope directed therapy, comprising determining a level of epitope presentation on at least one cell of the subject using an antibody comprising an antigen recognition domain capable of binding to an MHC-I molecule being complexed with a peptide fragment of the antigen, wherein the antibody does not bind the MHC-I in the absence of the complexed peptide fragment of the antigen, and wherein the antibody does not bind the peptide fragment of the antigen in an absence of the MHC, wherein a level value lower than a predetermined threshold is indicative of an individual being suitable for CTL-based epitope directed therapy.

According to an aspect of the present invention there is provided a method of treating a melanoma, comprising administering to a subject in need thereof a therapeutically effective amount of an antibody comprising an antigen recognition domain capable of binding to an MHC-I molecule being complexed with a tyrosinase peptide, wherein the antibody does not bind the MHC-I in the absence of the complexed tyrosinase peptide, and wherein the antibody does not bind the tyrosinase peptide in an absence of the MHC, thereby treating the melanoma.

According to an aspect of the present invention there is provided a method of killing or ablating a cell displaying a tyrosinase peptide on a surface MHC molecule, the method comprising contacting the target cell with an antibody comprising an antigen recognition domain capable of binding to an MHC-I molecule being complexed with a tyrosinase peptide, wherein the antibody does not bind the MHC-I in the absence of the complexed tyrosinase peptide, and wherein the antibody does not bind the tyrosinase peptide in an absence of the MHC, thereby killing or ablating the cell.

According to an aspect of the present invention there is provided an antibody comprising an antigen recognition domain capable of binding to an MHC-I molecule being complexed with a MART-1 peptide comprising an amino acid sequence as set forth in SEQ ID NO: 21, wherein the antibody does not bind the MHC-I in the absence of the complexed peptide, and wherein the antibody does not bind the peptide in an absence of the MHC.

According to some embodiments of the invention, the antibody comprises an antigen recognition domain which comprise complementarity determining region (CDR) amino acid sequences as set forth in SEQ ID NOs: 59-64.

According to some embodiments of the invention, the antibody comprises complementarity determining region (CDR) amino acid sequences as set forth in SEQ ID NOs: 59-64.

According to some embodiments of the invention, the antibody binds to an MHC-I molecule being complexed with a tyrosinase peptide with a disassociation constant less than 100 nM.

According to some embodiments of the invention, the antibody comprises an antigen recognition domain which comprises complementarity determining region (CDR) amino acid sequences as set forth in SEQ ID NOs: 886-891.

According to some embodiments of the invention, the antibody comprises an antigen recognition domain which comprises complementarity determining region (CDR) amino acid sequences as set forth in SEQ ID NOs: 894-899.

According to some embodiments of the invention, the antibody comprises complementarity determining region (CDR) amino acid sequences as set forth in SEQ ID NOs: 886-891

According to some embodiments of the invention, the antibody comprises complementarity determining region (CDR) amino acid sequences as set forth in SEQ ID NOs: 894-899.

According to some embodiments of the invention, the antibody binds to an MHC-I molecule being complexed with a tyrosinase peptide with a disassociation constant less than 10 nM.

According to some embodiments of the invention, the antibody is an IgG1 antibody.

According to some embodiments of the invention, the antibody is conjugated to a therapeutic moiety.

According to some embodiments of the invention, the therapeutic moiety is selected from the group consisting of a cytotoxic moiety, a toxic moiety, a cytokine moiety and a bi-specific antibody moiety.

According to some embodiments of the invention, the toxic moiety is PE38KDEL.

According to some embodiments of the invention, the antibody is attached to a detectable moiety.

According to some embodiments of the invention, the detectable moiety is a fluorescent protein or an enzyme.

According to some embodiments of the invention, the antibody is an antibody fragment.

According to some embodiments of the invention, the antibody fragment is selected from the group consisting of an Fab fragment, an F(ab')₂ fragment and a single chain Fv fragment.

According to some embodiments of the invention, the cell is a skin cell.

According to' some embodiments of the invention, the tyrosinase peptide comprises an amino acid sequence as set forth in SEQ ID NO: 1.

According to some embodiments of the invention, the antibody comprises an antigen recognition domain capable of binding to an MHC-I molecule being complexed with a tyrosinase peptide with a disassociation constant less than 100 nM, wherein the antibody does not bind the MHC-I in the absence of the complexed peptide, and wherein the antibody does not bind the peptide in an absence of the MHC.

According to some embodiments of the invention, the antibody comprises an antigen recognition domain which comprises complementarity determining region (CDR) amino acid sequences as set forth in SEQ ID NOs: 47-52.

According to some embodiments of the invention, the antibody comprises an antigen recognition domain which comprises complementarity determining region (CDR) amino acid sequences as set forth in SEQ ID NOs: 53-58.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of' and "consisting essentially of'.

The phrase "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

The term "method" refers to manners, means, techniques and procedures for accomplishing a given task including, but not limited to, those manners, means, techniques and procedures either known to, or readily developed from known manners, means, techniques and procedures by practitioners of the biotechnology art.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice,

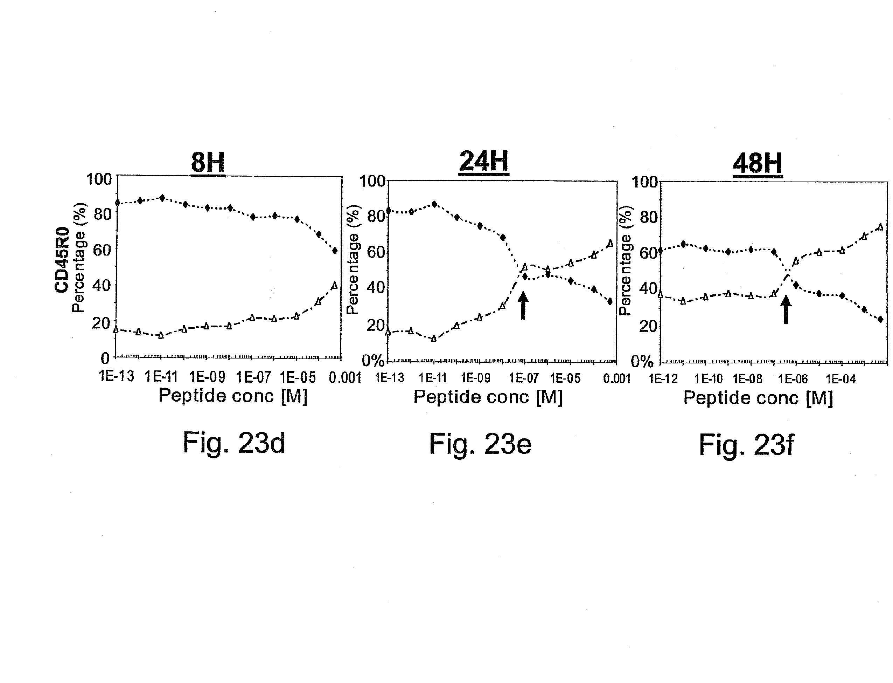

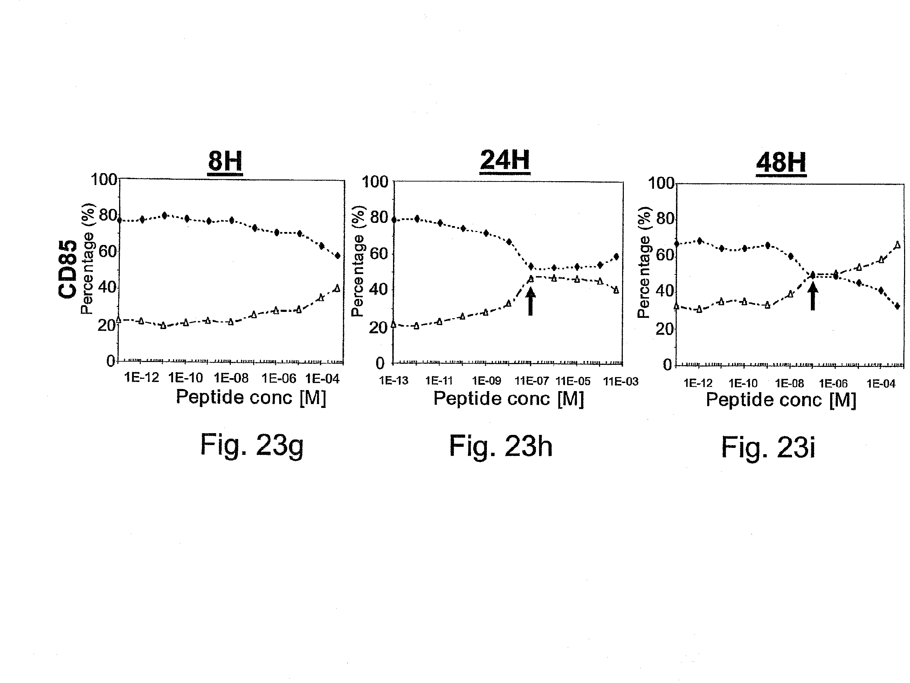

In the drawings:

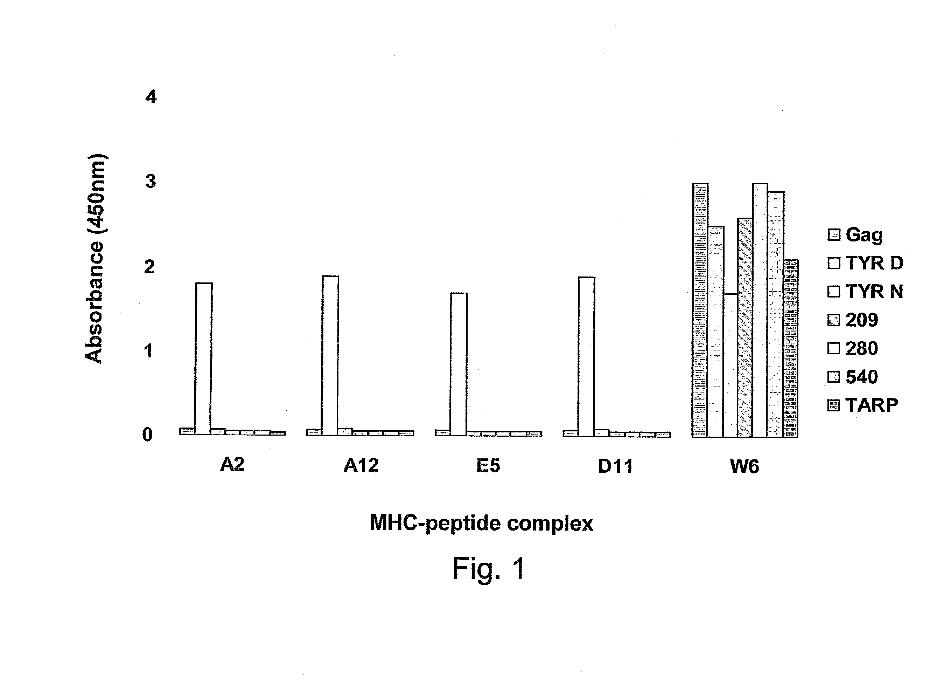

FIG. 1 is a bar graph depicting the binding of soluble purified Fabs with TCR-like specificity. Binding ELISA assay of anti-HLA-A2/Tyrosinase-specific clones with immobilized HLA-A2-peptide complexes containing Tyrosinase D 369-377 peptide (SEQ ID NO:1), and control HLA-A2-restricted peptides. Anti-HLA mAb W6/32 (W6), was used to determine the correct folding and stability of the bound complexes during the binding assay. Note the specific binding of A2, A12, E5 and D11 soluble purified Fabs to MHC class I complexes of HLA-A2- Tyrosinase D 369-377 peptide as compared to the absence of binding to MHC-class I complexes of HLA-A2 and control antigenic peptides such as Gag (SEQ ID NO:2), Tyr N (SEQ ID NO:3), 2092M (SEQ ID NO:4), 280 (SEQ ID NO:5), 540 (SEQ ID NO:6), and TARP (SEQ ID NO:7.

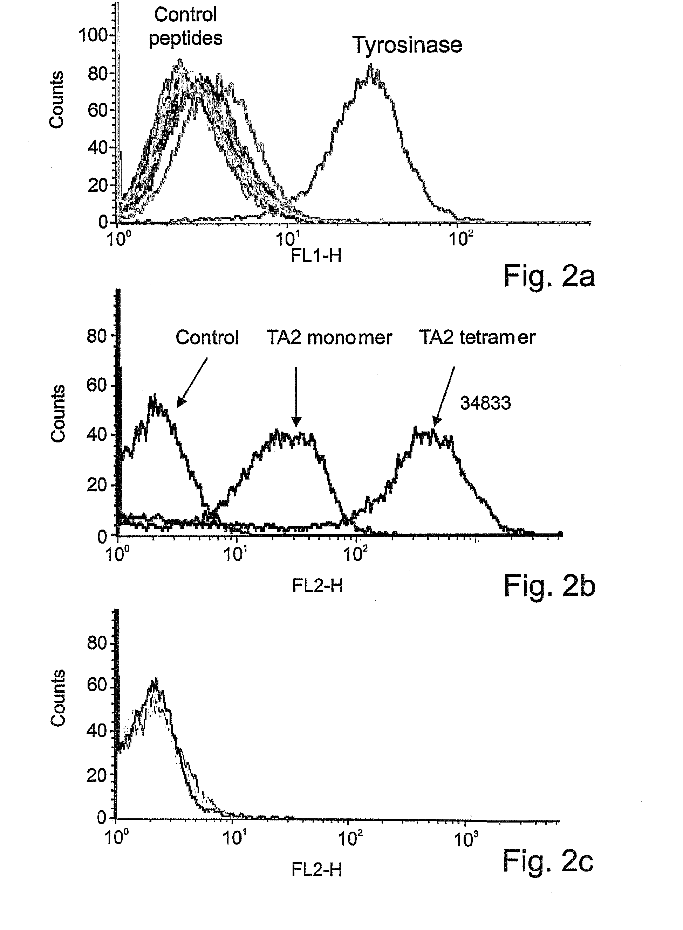

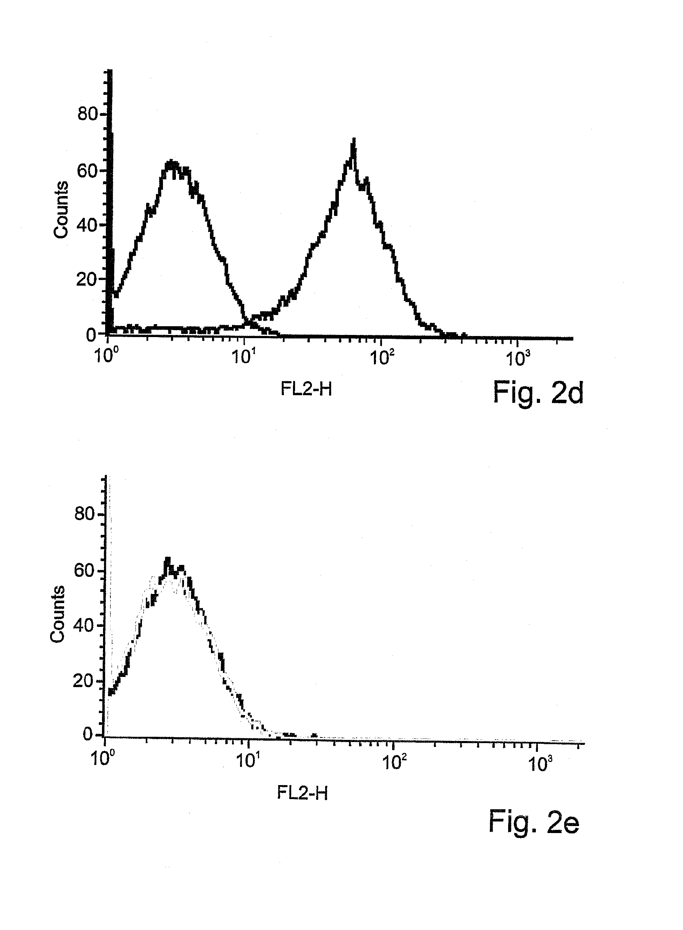

FIGs. 2a-e are flow cytometry analyses depicting the binding of HLA-A2/Tyr-specific TCR-like antibody to antigen presenting cells (APCs). Figure 2a - Flow cytometry analysis of the binding of Fab TA2 to JY APCs pulsed with Tyrosinase 369-377 peptide or the control peptides described in Figure 1 as well as 154 (SEQ ID 20), Tax (SEQ ID NO: 26), 280m (SEQ ID NO: 28), pol (SEQ ID NO: 65), 865 (SEQ ID NO: 29) and Mart (SEQ ID NO: 22) peptides were used); Figures 2b-c - Flow cytometry analyses of FabTA2 in a monomeric and tetrameric form to JY APCs pulsed with the Tyrosinase 369-377 peptide (Figure 2b) or to control peptide (209 2M peptide SEQ ID NO. 4; Figure 2c); Figures 2d-e - Flow cytometry analyses of the binding of TA2 whole IgG antibody to JY cells pulsed with Tyrosinase 369-377 peptide (Figure 2d) or control peptide (209 2M peptide; SEQ ID NO. 4) (Figure 2e).

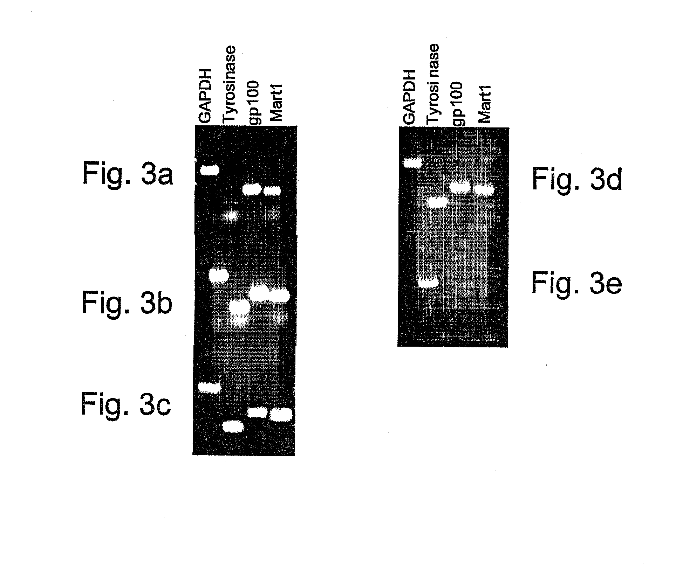

FIGs. 3a-e are RT-PCR analyses of melanoma cell lines depicting the expression of melanoma antigens. RNA was extracted from the 624.38 (Figure 3a), 501A (Figure 3b), 2224 (Figure 3c), 1352 (Figure 3d) or 1938 (Figure 3e) melanoma cell lines and RT-PCR was performed using PCR primers specific for the genes MelanA/Mart1 (SEQ ID NOs:8 and 9; lane 4), Pmel17/gp100 (SEQ ID NOs:10 and 11; lane 3), Tyrosinase (SEQ ID NOs:12 and 13; lane 2) and GAPDH (SEQ ID NOs:14 and 15; lane 1). Note that while the 624.38, 501A, 2224 and 1352 expressed all three melanoma-related genes (Tyrosinase, gp100 and Mart1, Figures 3a-d), the 1938 cell line expressed only the positive control GAPDH gene (Figure 3e).

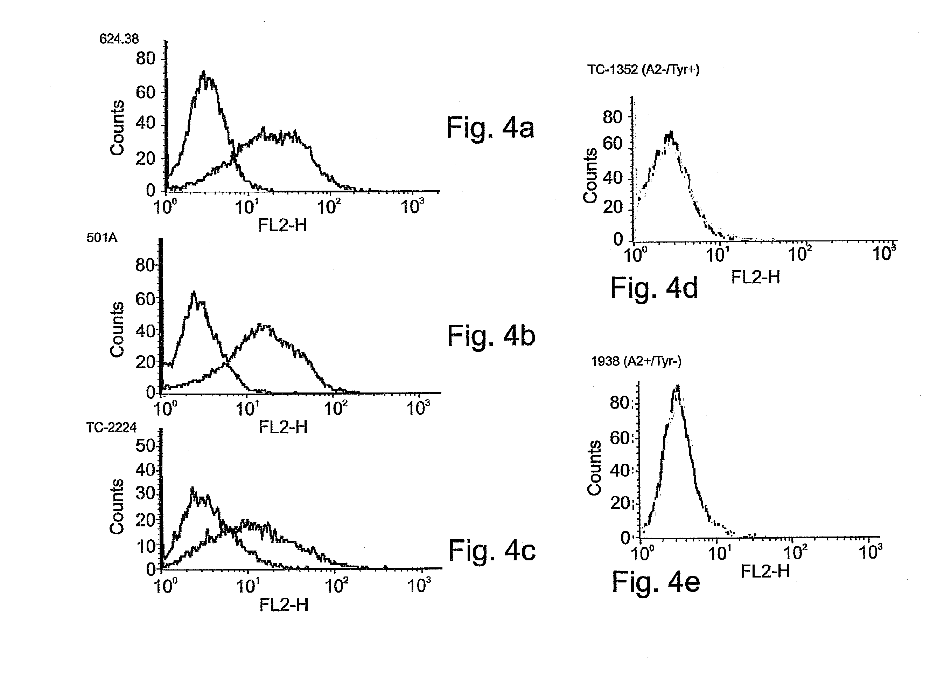

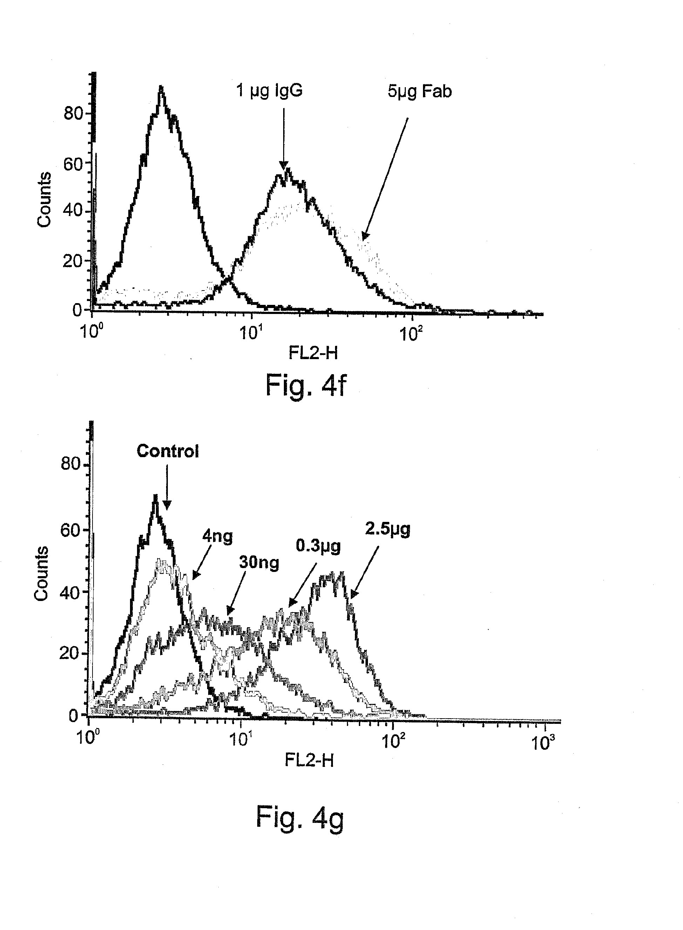

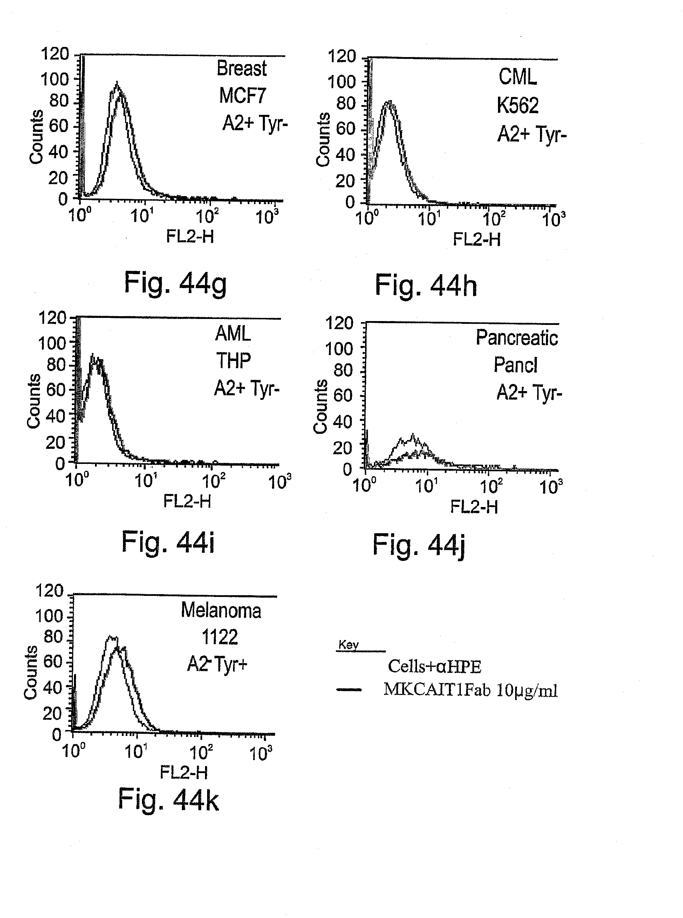

FIGs. 4a-g are flow cytometry analyses depicting the detection of HLA-A2-Tyrosinase complexes on the surface of melanoma cell by TA2 TCR-like antibody. Figures 4a-f - 624.38 (Figure 4a), 501A (Figure 4b), and TC-2224 (Figure 4c), which express both the HLA-A2 and the Tyrosinase genes, as well as HLA-A2⁻ 1352 cells (Figure 4d), and Tyr⁻ 1938 cells (Figure 4e), were reacted with TA2 Ab, followed by incubation with PE-labeled anti-human Ab. TC-2224 and TC-1352 were reacted with TA2 IgG, 624.38, 501A and 1938 were reacted with the TA2 Fab. Note the specific binding of the TA2 Ab with the melanoma cells expressing HLA-A2 and tyrosinase as compared to the negative binding of the TA2 Ab with the TC-1352 (HLA-A2⁻/Tyr⁺) or 1938 (HLA-A2⁺/Tyr⁻) cells which served as controls. Figures 4f-g are comparative flow cytometry analysis of TA2 Fab and whole IgG antibody. Figure 4f - Staining with 5 µg Fab and 1 µg IgG on Tyrosinase-pulsed JY cells. Figure 4g - Titration of the binding of TA2 whole IgG on melanoma 624.38 cells.

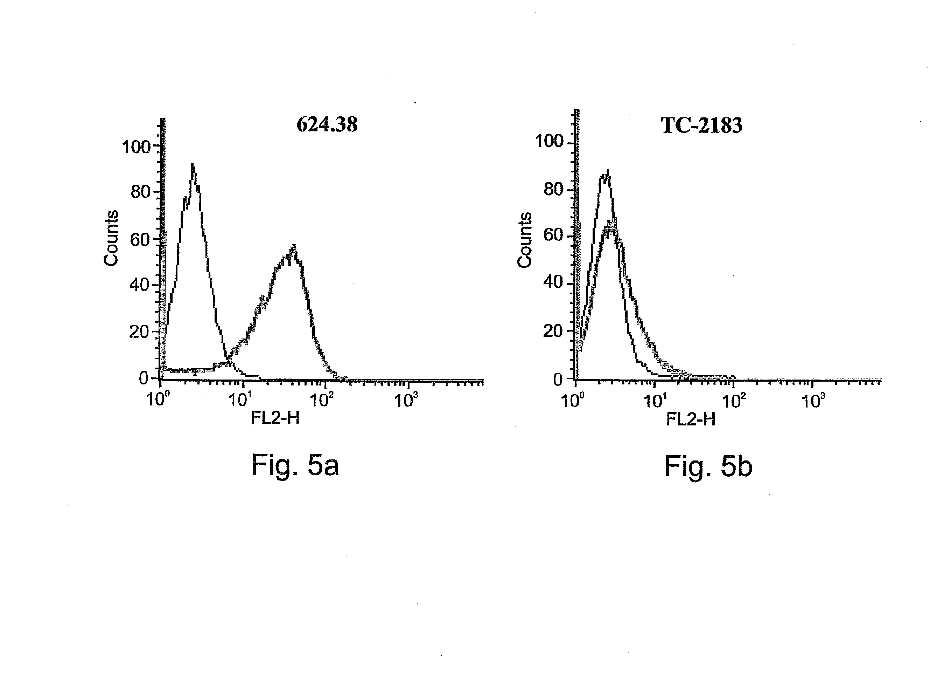

FIGs. 5a-d depict the killing of melanoma cells by Tyrosinase-specific CTLs.

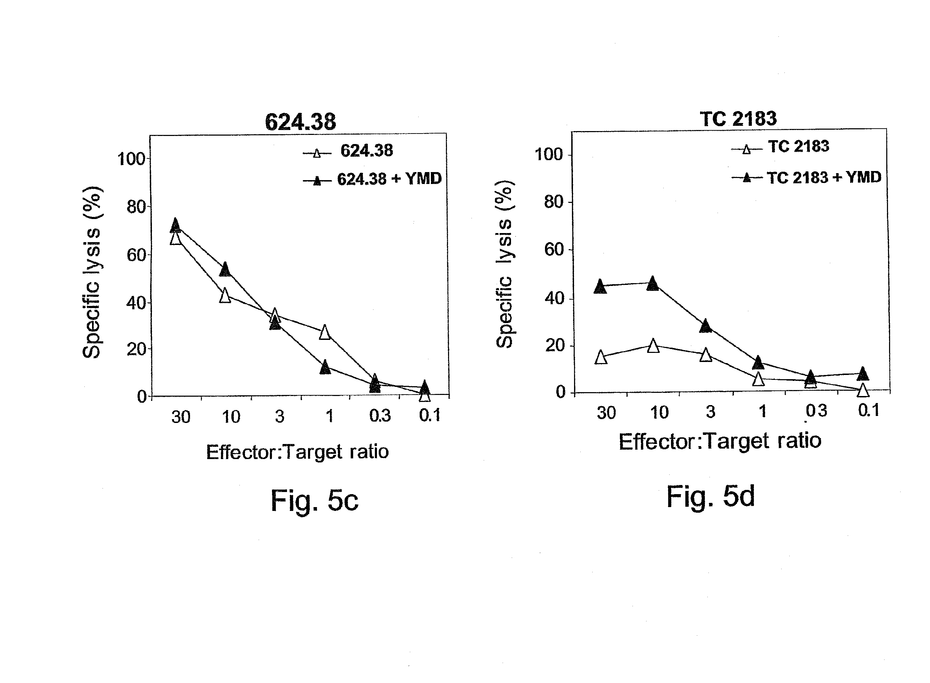

   Figures 5a-b are flow cytometry analyses of melanoma 624.38 (Figure 5a) and TC-2183 (Figure 5b) cells using the TA2 whole IgG TCR-like antibody labeled with PE-conjugated anti-human antibody, specific to the HLA-A2/tyrosianse complexes. Note the high expression level of HLA-A2/tyrosianse complexes on the surface of melanoma 624.38 cells (Figure 5a) as compared to the low expression in TC-2183 cells (Figure 5b). Figures 5c-d are graphs depicting cytotoxicity assays of 624.38 (Figure 5c) and TC-2183 (Figure 5d) melanoma cells by the TyrosinaseD-369-377-specific CTLs. Melanoma cells were either peptide-pulsed (YMD= Tyrosinase D 369- 377peptide) or un-pulsed with the TyrosinaseD-369-377 peptide and the effect of the TyrosinaseD-369-377-specific CTLs on cell killing was determined. Results are presented as the specific lysis (in percentages) as a function of the effector (CTL) to target (Melanoma cells) ratio.

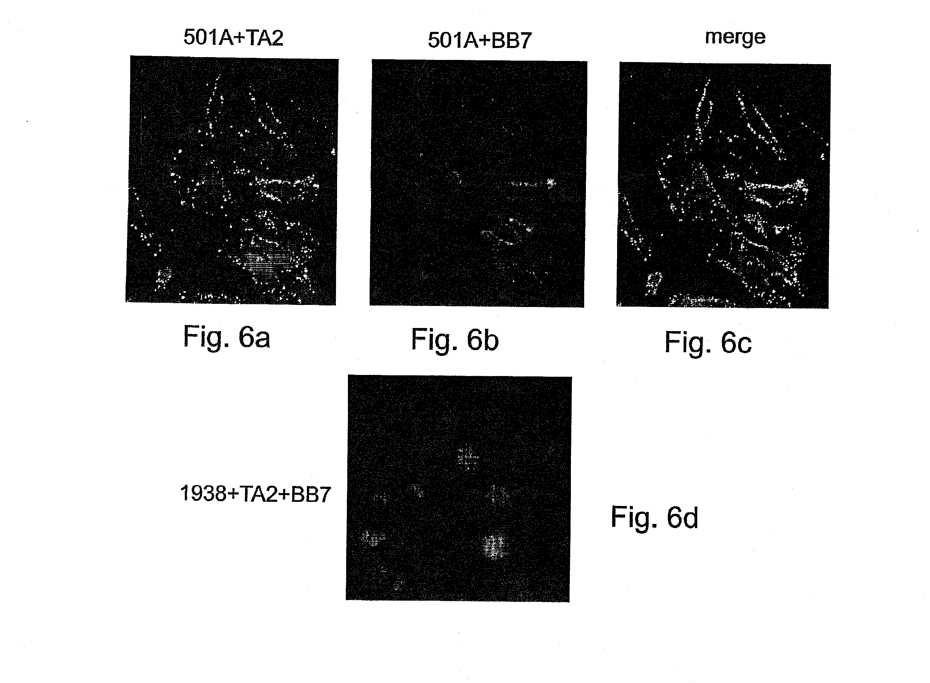

FIGs. 6a-d are confocal microscopy images of immuno-fluorescence analysis depicting the binding of HLA-A2/Tyrosinase-specific TCR-like antibody to melanoma cells. The 501A (figures 6a-c) or 1938 (Figure 6d) melanoma cells were subjected to double immuno-fluorescence analysis using labeled TA2 FAB (green) and anti-HLA-A2 MAb BB7 (red). Nuclei were stained with DRAQ5. Figure 6a - TA2 labeling; Figure 6b-BB7 labeling; Figures 6c-d -merged images of TA2 and BB7 labeling. Note the specific labeling of TA2 and BB7 on the cell membrane of the 501A melanoma cells and the absence of labeling of both antibodies on the cell membrane of 1938 cells. Figure 6d-1938 reacted with TA2 and not BB7. After incubation with TA2 or BB7, anti-human alexa 488 or anti-mouse alexa 594 antibodies were used, respectively.

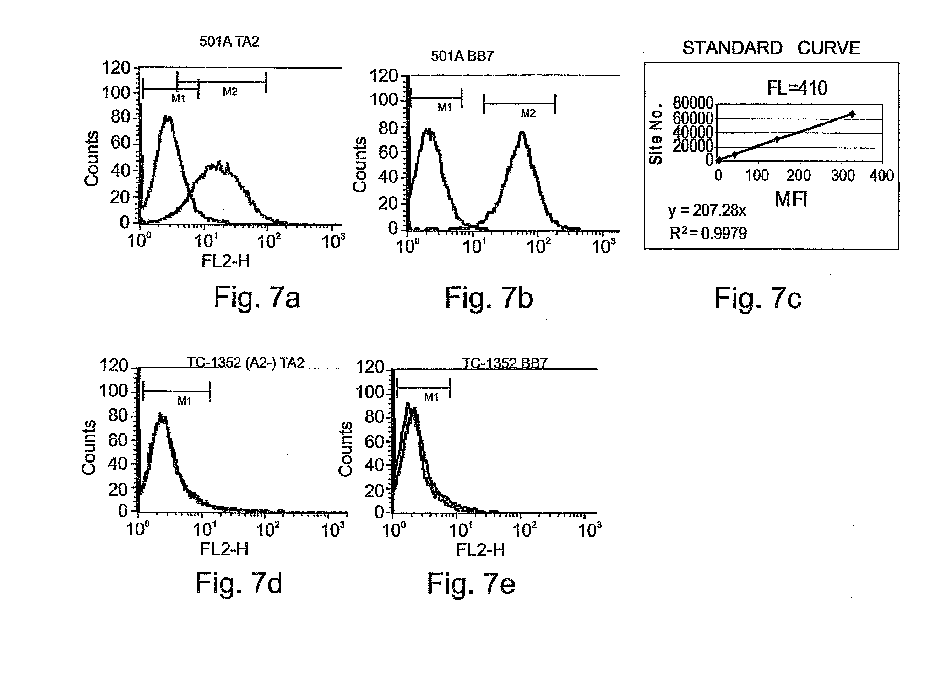

FIGs. 7a-e depict quantification of Tyrosinase-HLA-A2 complexes by TA2.
   Figures 7a, b, d and e - Flow cytometry analyses. Figure 7c - a standard curve of the flow cytometry analyses. 501A (HLA-A2⁺/Tyr⁺) (Figures 7a-b) and TC-1352 (HLA-A2⁻/Tyr⁺) (Figure 7d-e) melanoma cells were incubated with biotinylated TA2 Fab (Figures 7a and d), followed by detection with PE-labeled streptavidin. Expression of HLA-A2 complexes was monitored with PE conjugated-BB7.2 mAb (Figures 7b and e). For a standard curve, calibration beads with known numbers of PE molecules per bead were used (Figure 7c). To perform the standard curve, 1 ml of PBS 0.1% BSA was added to the beads, the beads were located in the middle of the FSC SSC dot blot and were analyzed for their fluorescence intensities in various FL2 values. MFI = mean fluorescence intensities; FL = the value of FL2 that was used; The quantification results obtained from the flow cytometry analyses are summarized in Table 2.

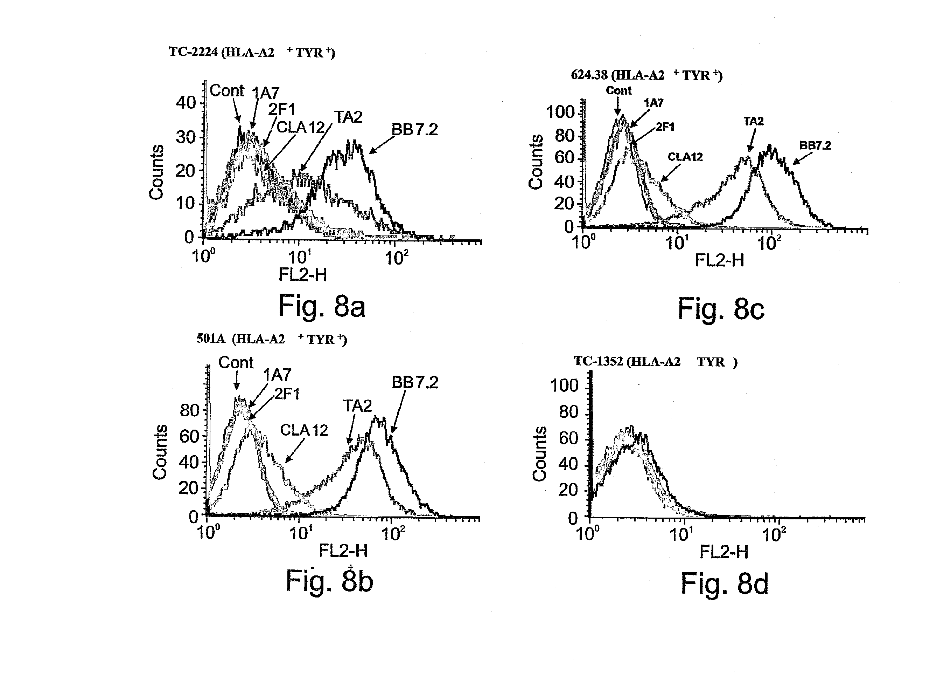

FIGs. 8a-d depict the expression hierarchy of melanoma differentiation antigens by TCR-like antibodies. Figures 8a-d are comparative flow cytometry analyses of the expression of HLA-A2-Tyr/Mart-1/gp100 complexes on the surface of TC-2224 (Figure 8a), 501A (Figure 8b), 624.38 (Figure 8c) and 1352 (Figure 8d) melanoma cells. Melanoma cells were incubated with anti-HLA-A2-Tyr TA2, anti-HLA-A2-Mart-1 CLA12, anti-HLA-A2-gp100-209 1A7, and anti-HLA-A2-gp100 280 2F1, TCR-like antibodies, all in the IgG form. HLA-A2 expression was monitored with MAb BB7.2. Gene expression of the antigens is shown. Note the relatively high representation of the TA2-positive cells in TC-2224, 501A and 634.38 cells are compared to the lower representation of 1A7, 2F1 or CLA12-positive cells.

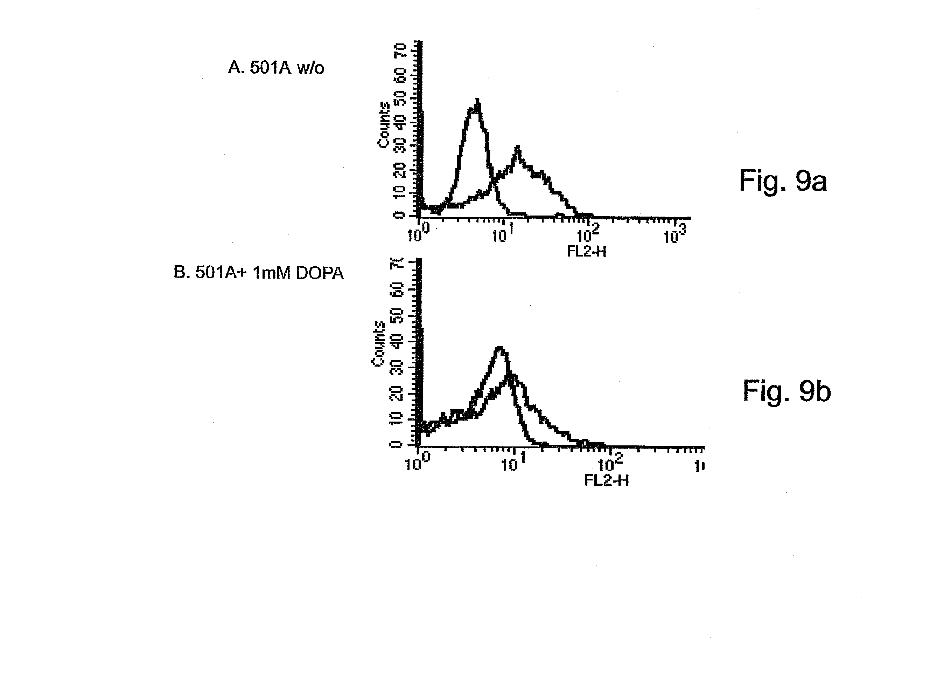

FIGs. 9a-b are flow cytometry analyses depicting the effect of Tyrosinase protein
   stability on the reactivity of TA2 TCR-like antibody with melanoma cells. 501A melanoma cells were treated (Figure 9b) or not (Figure 9a) with 1 mM DOPA for 16 hours, following
   which the cells were incubated with TA2 followed by incubation with PE-labeled anti-human Ab and the mean fluorescence intensities (MFI) were determined. Quantification of the effect of DOPA on MFI is summarized in Table 5 (Example 6 of the Examples section which follows).

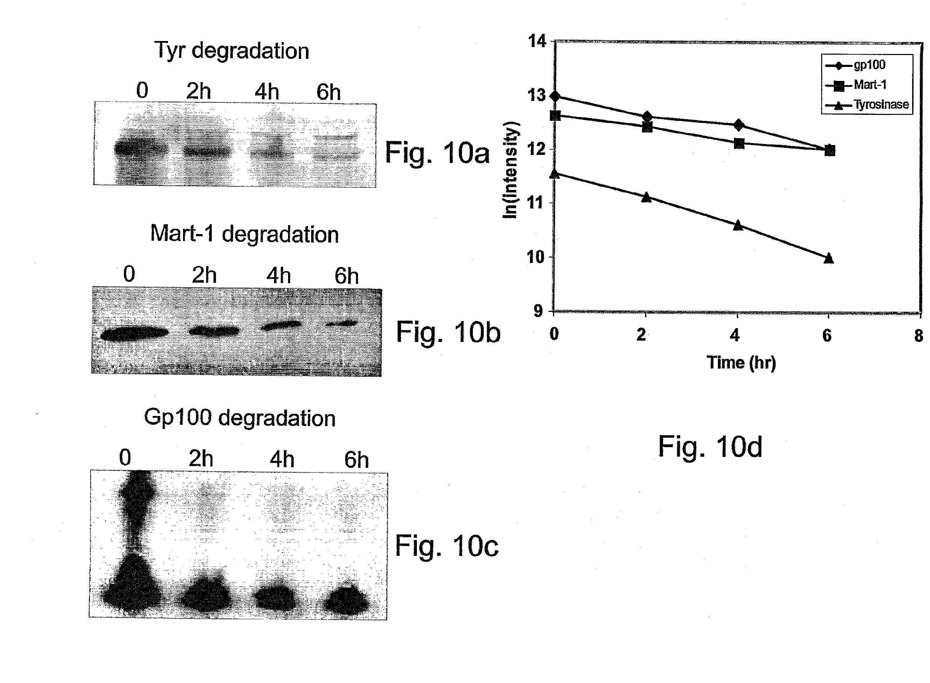

FIGs. 10a-d are Western blot analyses (Figures 10a-b) and quantification thereof (Figures 10c-d) depicting the degradation of differentiation antigens in melanoma cells. Protein synthesis in melanoma 501A cells was arrested by cyclohexamide and the stability of Tyrosinase (Figures 10a and c), Mart1 (Figures 10b and d) and gp100. The cells were lysed after 0, 2, 4 and 6 h. Equal amounts of sample were loaded on SDS-PAGE and electroblotted onto nitrocellulose. Protein was measured over time in cell extracts that were subjected to Western blot analysis using the specific monoclonal antibodies (The blots were probed with T311 mouse anti-Tyrosinase, HMB-45 mouse anti-gp100 or A103 mouse anti-Mart-1 followed by incubation with a secondary horseradish peroxidase-conjugated antibody. Note the rapid degradation of Tyrosinase (Figure 10c) as compared to that of Mart1 (Figure 10d) or gp100 (-0.152) as revealed by the line slope (-0.25 in Figure 10c vs. - 0.1 in Figure 10d). There was a hierarchy of degradation rate or protein stability with Mart-1 being the most stable with a t1/2 of ~6.5 hrs, gp100 with moderate stability (t1/2 of ~4.5 hrs) and Tyrosinase being the less stable protein of the three antigens with a t1/2 of ~2.5 hrs.

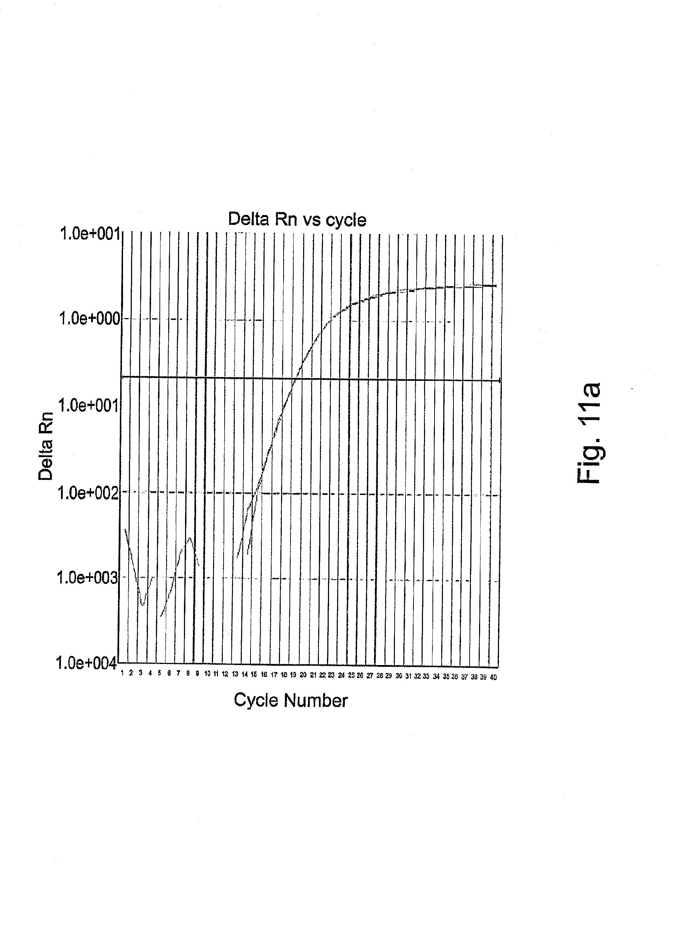

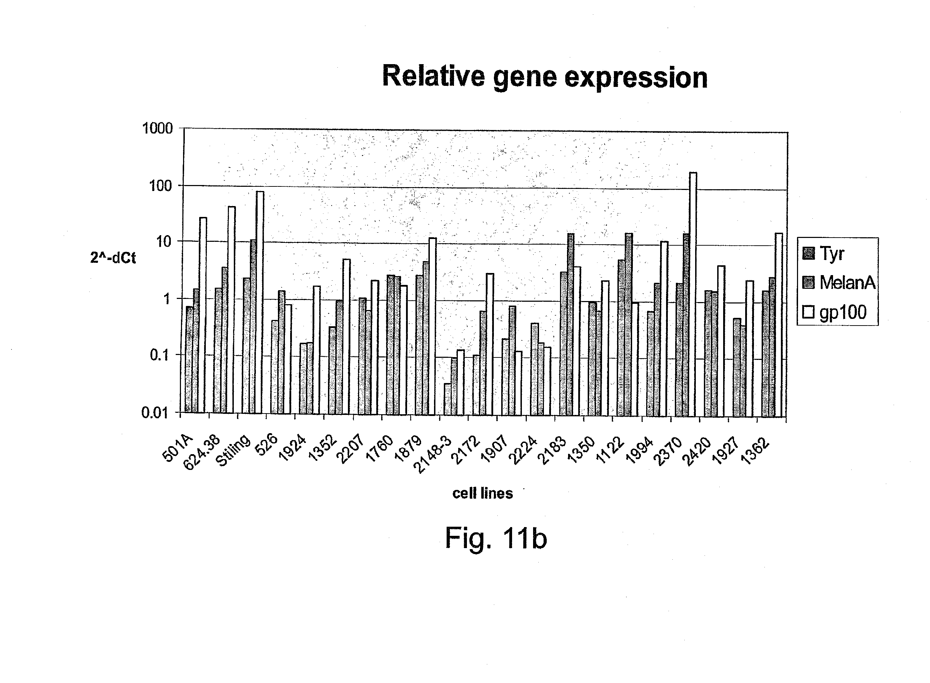

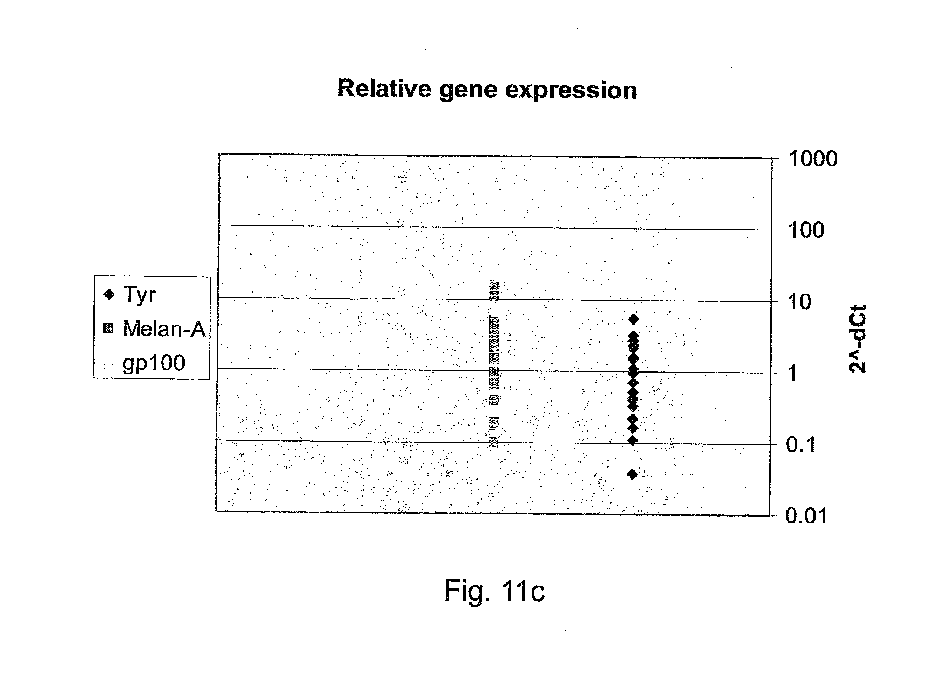

FIGs. 11a-c depict the relative expression of melanoma differentiation antigens by real-time PCR. Real-time PCR analysis was performed on RNA samples of 31 melanoma cells lines derived from patients using RT-PCR primers specific to Tyrosinase, Melan-A and gp100. Figure 11a is an example of real time PCR amplification graph; Figure 11b - A histogram depicting the relative gene expression of tyrosinase, Melan-A and gp100 expressed as 2^{-ΔCT} units for 21 out of the 31 melanoma cell lines examined. Figure 11c illustrates the relative gene expression of tyrosinase, Melan-A and gp100 expressed as 2^{-ΔCT} units for 21 out of the 31 melanoma cell lines examined.

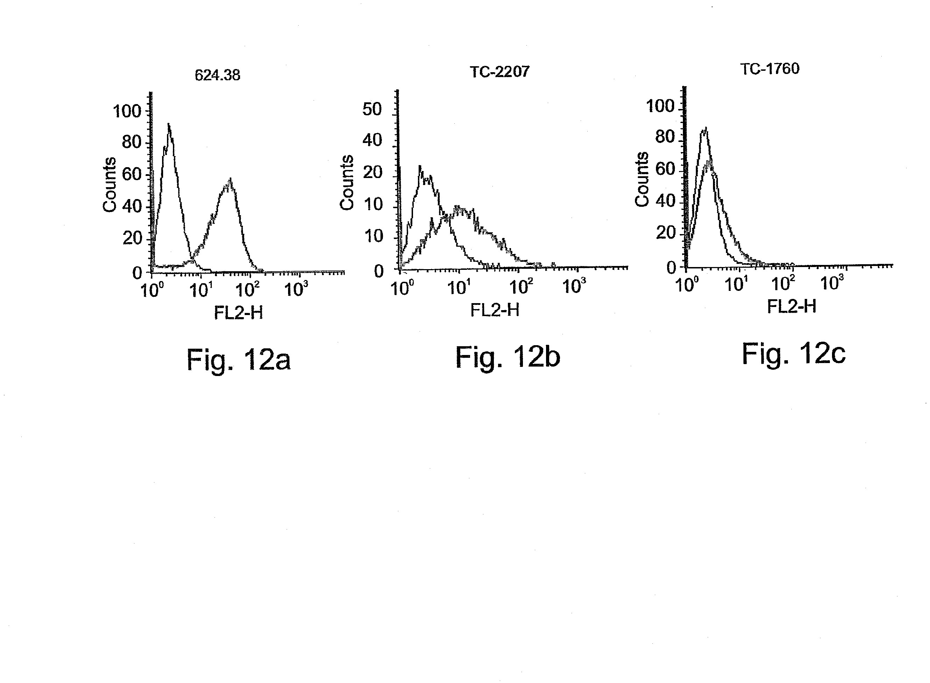

FIGs. 12a-c are flow cytometry analyses depicting the quantification of the number of HLA-A2/Tyrosinase complexes on melanoma cells. Melanoma lines 624.38 (Figure 12a), TC-2207 (Figure 12b), TC-1760 (Figure 12c) expressing high, moderate, and low levels of Tyrosinase, respectively, were incubated with biotinylated TA2 Fab followed by detection with PE-labeled streptavidin.

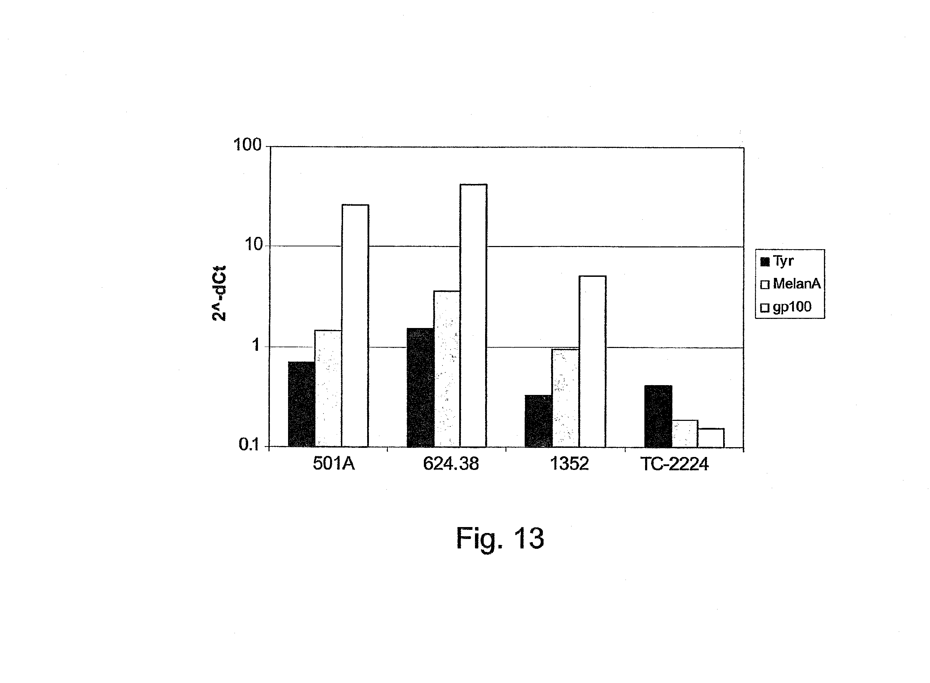

FIG. 13 is a bar graph depicting the relative gene expression of Tyrosinase, Melan-A and gp100 melanoma antigens on the 501A, 624.38, 1352 and TC-2224 melanoma cells.

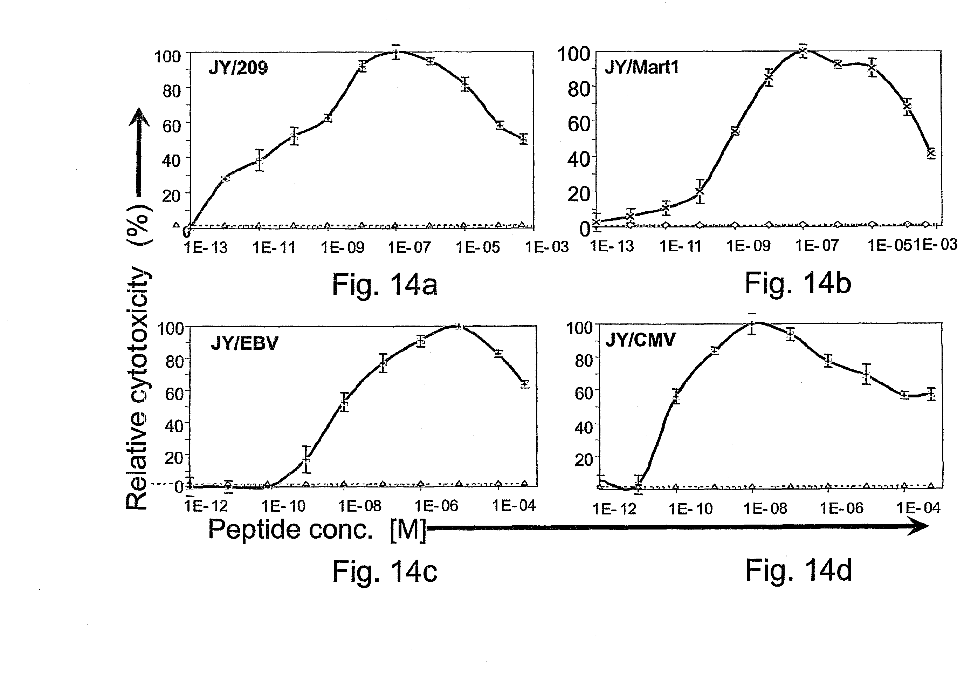

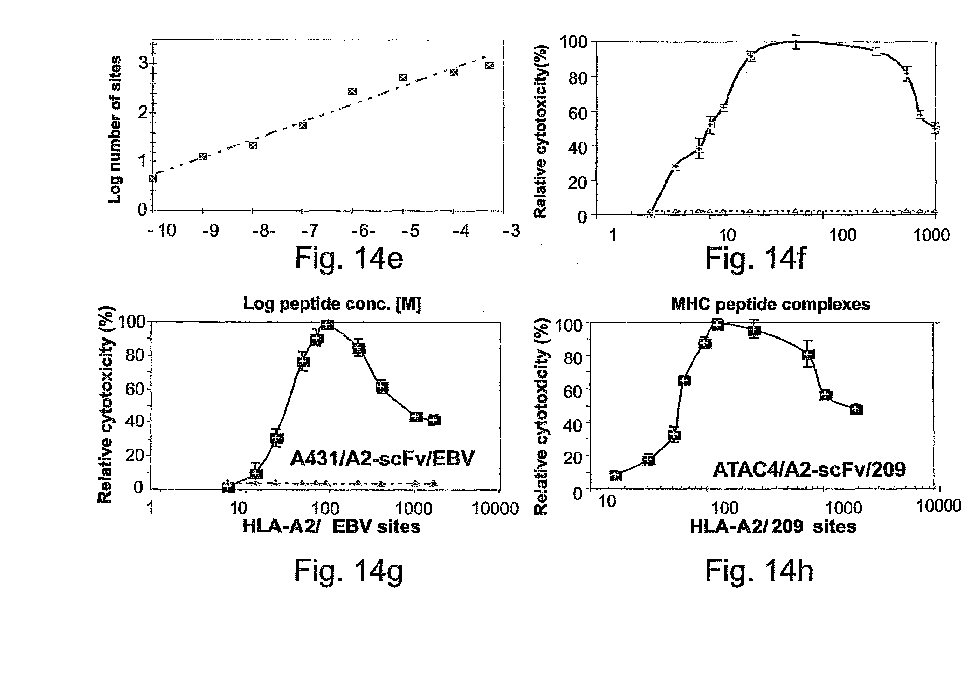

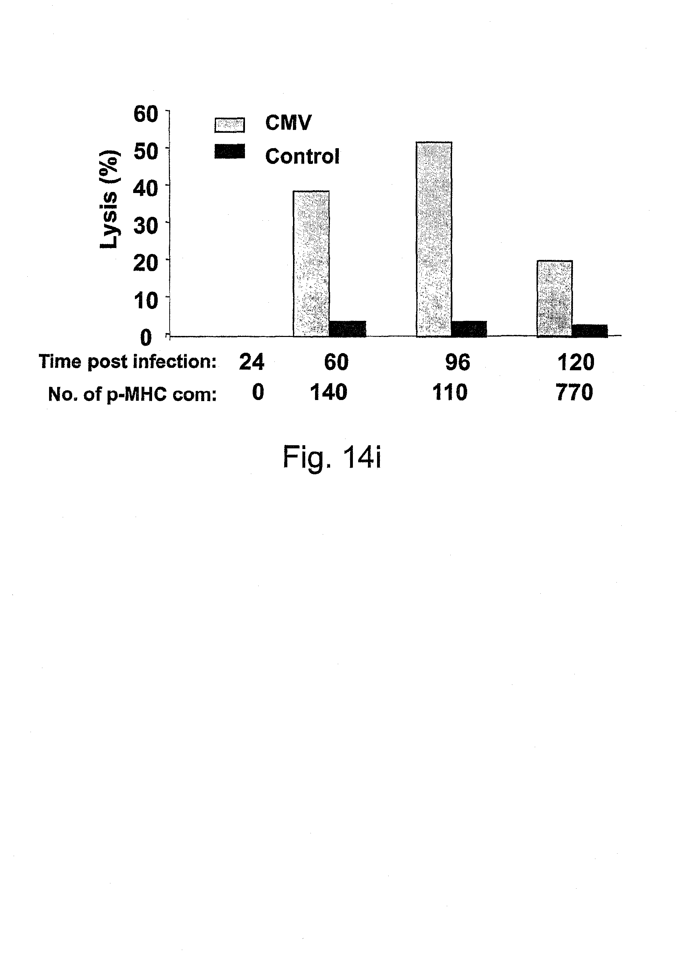

FIGs. 14a-i depict cytotoxicity assays of peptide-pulsed cells demonstrating that high antigen density induces hypo-responsiveness in memory activated CTLs. Figures 14a-d are histograms depicting the relative cytotoxicity as a function of peptide concentration. JY B-lymphoblast APCs were pulsed with increasing concentrations of the melanoma differentiation antigens gp100_{G209-217} (SEQ ID NO:4; Figure 14a) and MART1₂₇₋₃₅ (SEQ ID NO:22; Figure 14b), the EBV derived peptide BMLF-1₂₈₀₋₂₈₈ (SEQ ID NO:24; Figure 14c) and the CMV derived peptide pp65₄₉₅₋₅₀₃ (SEQ ID NO:25; Figure 14d). Peptide - pulsed JY APCs were subsequently exposed to the appropriate HLA-A2-restricted and specific CTL clones/line. In each assay pulsing with an irrelevant HLA-A2-peptide was used as a negative control. Figure 14e - JY APCs cells were pulsed with varying MART1₂₇₋₃₅ peptide (SEQ ID NO:22) concentrations ranging from 500 µM down to 10 pM. The fluorescence intensity of binding of a TCR-like antibody, CLA12, which specifically recognizes HLA-A2/MART1₂₇₋₃₅ (SEQ ID NO:22) complexes was determined by flow cytometry and directly correlated with QuantiBrite- phycoerythrin (PE) calibration beads that were used to create a calibration curve of Mean Fluorescence Intensities (MFI) for 0-10000 PE molecules for each cytometer setting. The MFI resulting from the binding of the TCR-like antibody was converted into number of PE molecules which directly correlate to the number of HLA-A2-peptide complexes detected by the TCR-like antibody. Peptide titrations correlated accurately with the fluorescent signal. Calculating MFI for each peptide concentration created a range of ~1000 to a very few HLA-peptide complexes on the surface of peptide-pulsed cells. Standard deviation of each spot is statistically significant toward its nearby spots. Figure 14f - The cytotoxicity of MART1₂₇₋₃₅-specific CTL clone acting against JY APCs pulsed at various peptide concentrations was correlated to the number of HLA-A2/ MART1₂₇₋₃₅ complexes present on the target cell surface as detected by the HLA-A2/ MART1₂₇₋₃₅-specific CLA12 TCR-like antibody. An irrelevant HLA-A2-restricted peptide was used as a negative control. Figures 14g-h - A431 (Figure 14g) and ATAC4 (Figure 14h) human epithelial carcinoma HLA-A2 negative which express EGFR and CD25, respectively, were incubated with single-chain HLA-A2/scFv fusion proteins to deposit variable amounts of HLA-A2/EBV and HLA-A2/209 peptide complexes, respectively on the surface of the target cells. Targets were exposed to the appropriate EBV and gp100₂₀₉ specific CTLs and lysis was measured. The number of HLA-A2/peptide complexes deposited on the target cell surface was determined using a PE conjugated monoclonal antibody BB7.2 which specifically recognizes HLA-A2 and PE calibration curves. Figure 14i - Human HLA-A2 positive fibroblasts were infected with CMV and cytotoxicity of CMV pp65₄₉₅₋₅₀₃-specific CTLs was determined as a function of time post infection and number of HLA-A2/pp65 complexes as determined by the TCR-like antibody H9 as described above.

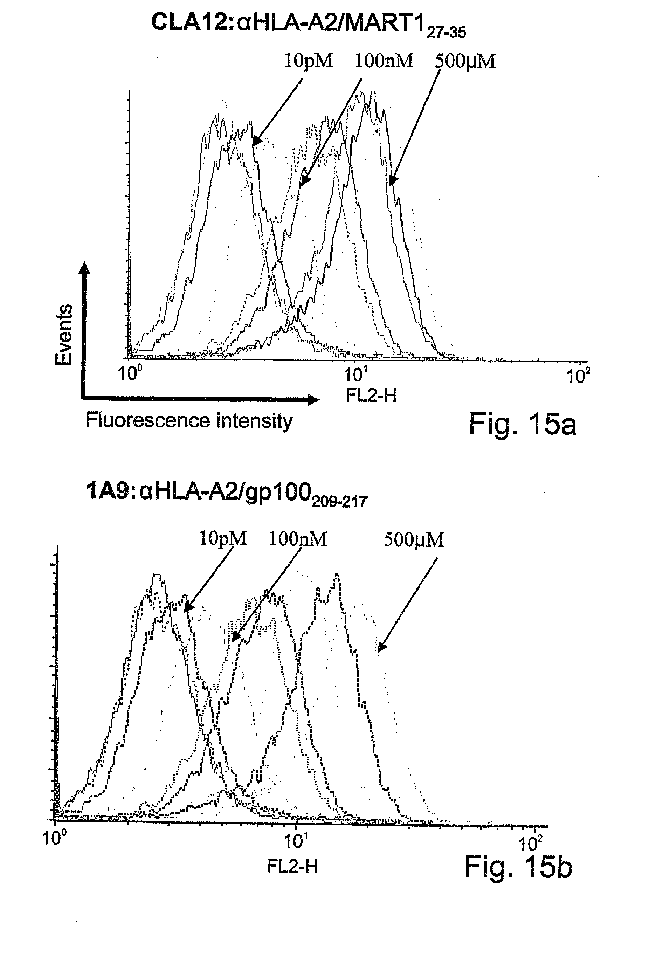

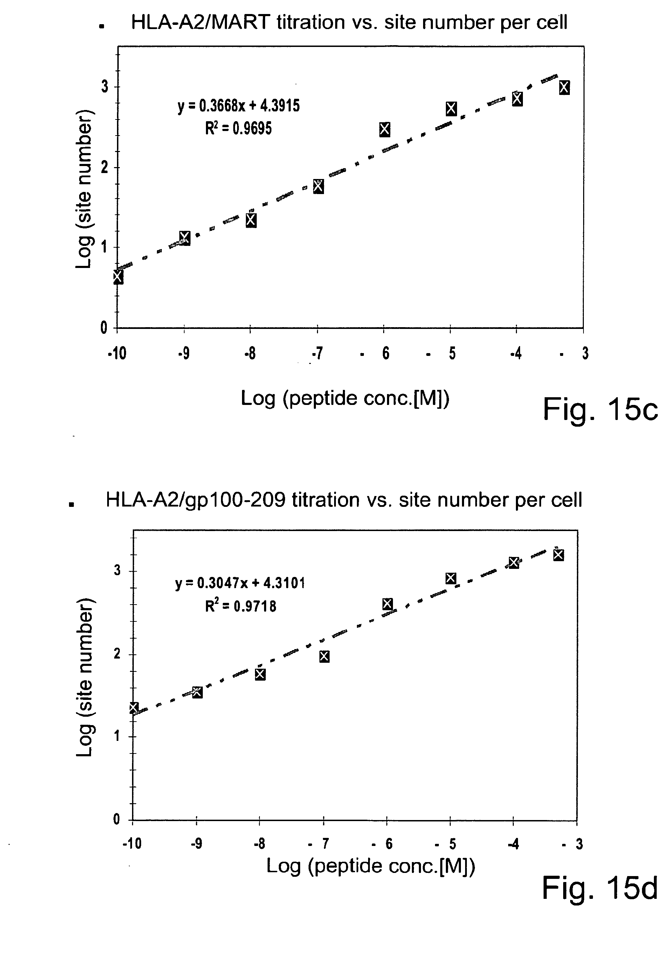

FIGs. 15a-d depict the binding of TCR-like antibodies to peptide-pulsed human pAPCs and quantitation of the number of peptide-MHC molecules on the cell surface. High affinity (~10-20 nM) TCR-like Fab antibodies CLA12 and 1A9 specific for HLA-A2 in complex with the MART-1₂₇₋₃₅ (Figure 15a) and gp100₂₀₉₋₂₁₇ (SEQ ID NO:4) (Figure 15b) HLA-A2-restricted peptides, respectively, were incubated with pAPCs pulsed with peptide concentrations ranging from 500 µM to 1 pM. PE-labeled secondary monoclonal antibodies specific to the Kappa or Lambda light chains of the TCR-like antibody were used to create a 1:1 ratio between the TCR-like Fab fragment and the PE-labeled secondary MAb. Mean fluoresence intensity (MFI) was calculated by using calibration curves of PE MFI at each cytometer settings. The MFI of each peptide loading concentration was translated into the number of specific HLA-A2-MART-1 (Figure 15c) or HLA-A2-gp100 (Figure 15d) complexes on the surface of the pAPC. This approach allowed the detection of as low as 10-20 specific peptide-MHC complexes on the surface of the target cell with high statistical significance. Peptide-MHC complexes could be detected on the surface of the pulsed pAPC with the high affinity TCR-like antibodies at peptide pulsing concentration as low as 10-100 pM.

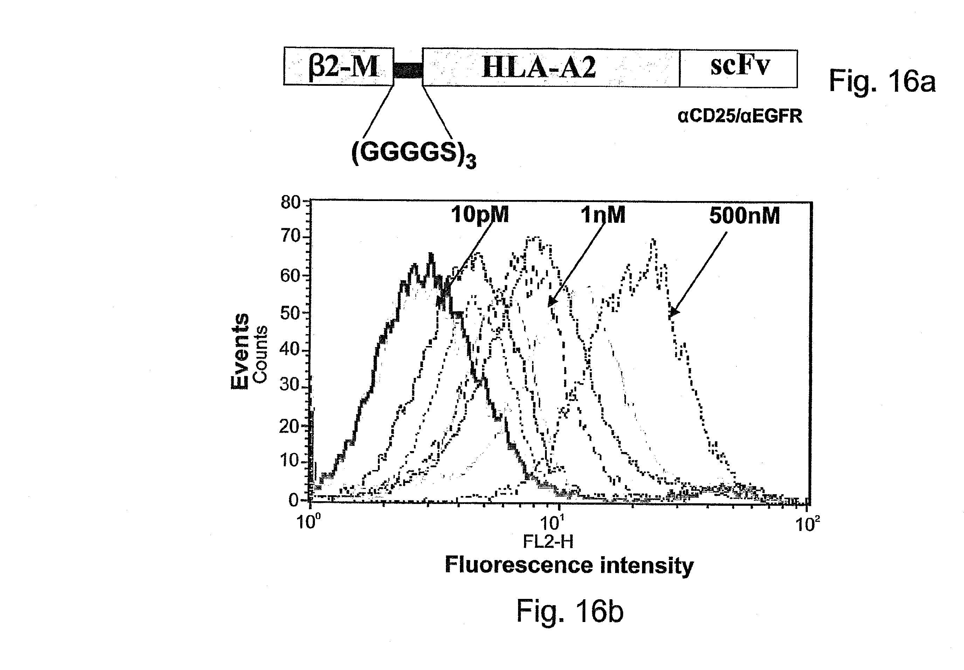

FIGs. 16a-b depict HLA-A2 deposition on the surface of HLA-A2 negative epidermoid carcinoma cells using single-chain HLA-A2-scFv fusion molecules. Figure 16a - A schematic diagram of the HLA-A2-scFv construct in which a scFv antibody fragment which recognizes EGFR or CD25 (Tac/p55) is genetically fused to the C-terminus of a single-chain HLA-A2 construct in which the β-2 microglobulin and HLA-A2 heavy chain genes are linked through a flexible peptide linker [(GGGGS)3; SEQ ID NO:23). These recombinant fusion molecules were expressed in *E coli* as intracellular inclusion bodies and refolded *in vitro* in the presence of the appropriate HLA-A2-restricted peptide (e.g., EBV derived peptide or gp100-209 peptide). Figure 16b - A431 (EGFR positive) cells (shown in the Figure) or ATAC4 (CD25 transfected A431) cells (not shown) were incubated with various concentrations of the purified scHLA-A2-scFv fusion molecules and were subsequently washed. Detection of HLA-A2 deposition was performed using PE-labeled anti-HLA-A2 monoclonal antibody BB7.2. Measuring the MFI of PE-labeled Mab BB7.2 binding and conversion of MFI values into the number of PE molecules using the QuantiBrite PE calibration beads (BD), as described above, allowed the specific determination of the number of peptide-HLA-A2 complexes deposited on the surface of target cells at each concentration of recombinant fusion molecule. Determination was performed with fusion molecule concentration ranging from 10 pM to 600 nM. This approach allowed the detection of as low as 10-20 specific peptide-MHC complexes on the surface of the target cell with high statistical significance.

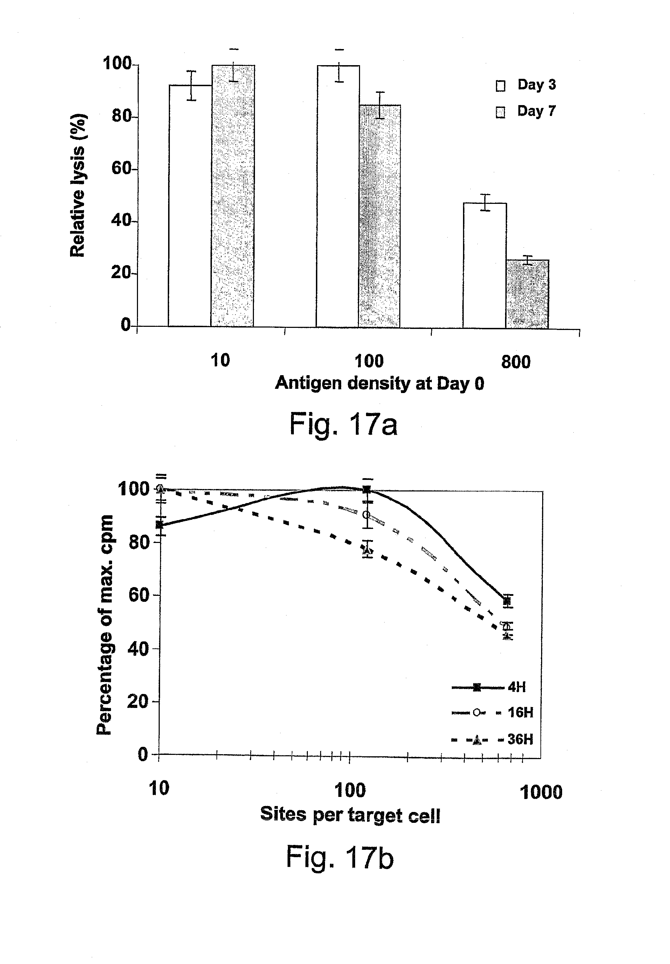

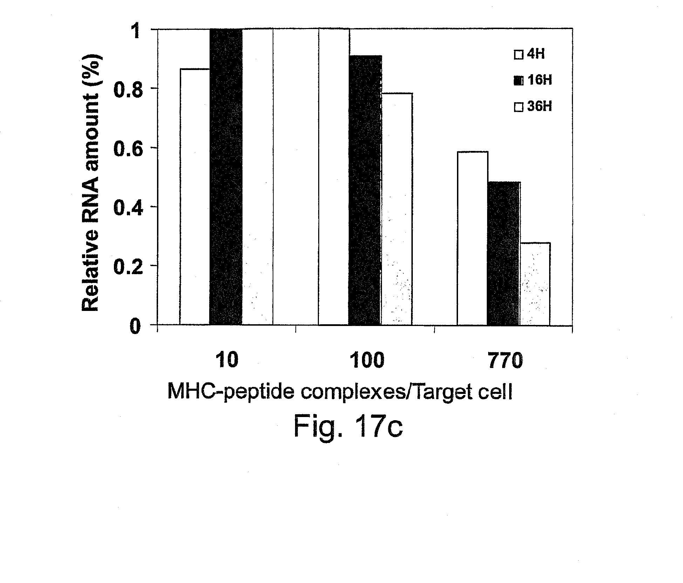

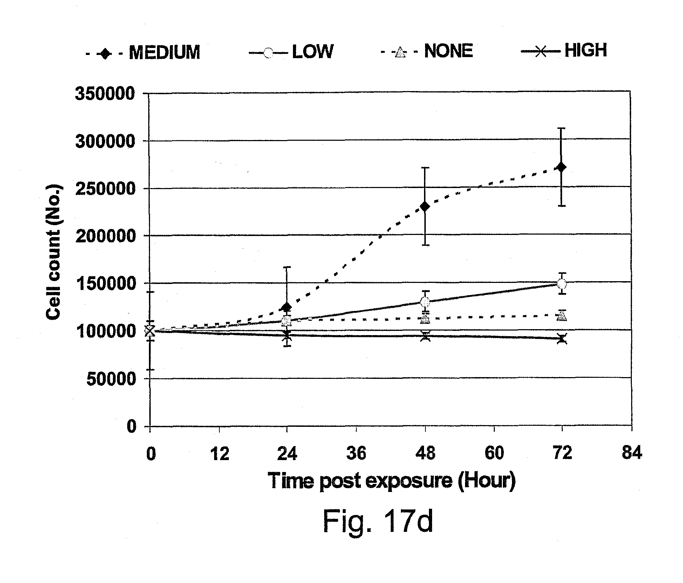

FIGs. 17a-d depict impaired proliferation after exposure of activated CTLs to high antigen (*i*.*e*., MHC-peptide complex) density. Figure 17a shows that the antigen density induced unresponsiveness is long lasting even 3 and 7 days after exposure to high antigen density. Figure 17b shows proliferation of CTLs as measured by ³H-Leucine incorporation measured 4, 16, and 36 hours after exposure of MART-1₂₇₋₃₅-specific CTL clone JKF6. The CTLs were exposed to increasing antigen densities by pulsing JY target cells. This figure shows that when CTLs are exposed to high antigen densities they reduce proliferation. Figure 17c shows a graph depicting the relative RNA amount (in percentages) as a function of MHC-peptide complex/Target cell. This figure shows the RNA content of CTLs exposed to high antigen densities is significantly low compared to low or moderate densities which indicate inhibition in gene expression and reduced metabolic function. Figure 17d shows that cell exposed to high antigen density do not proliferate after their exposure to high antigen density and that they do not die or there is no induction of apoptosis.

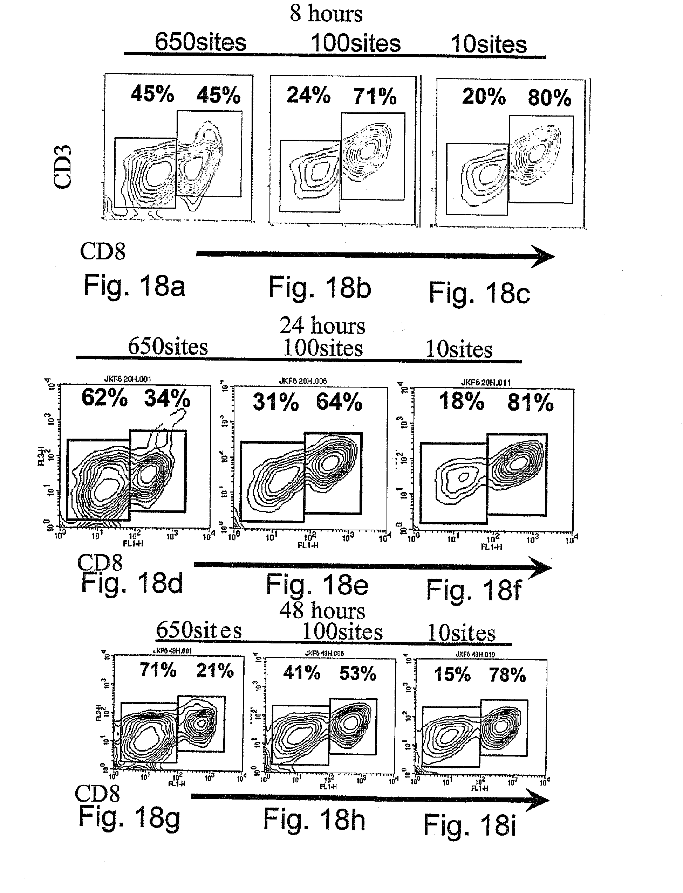

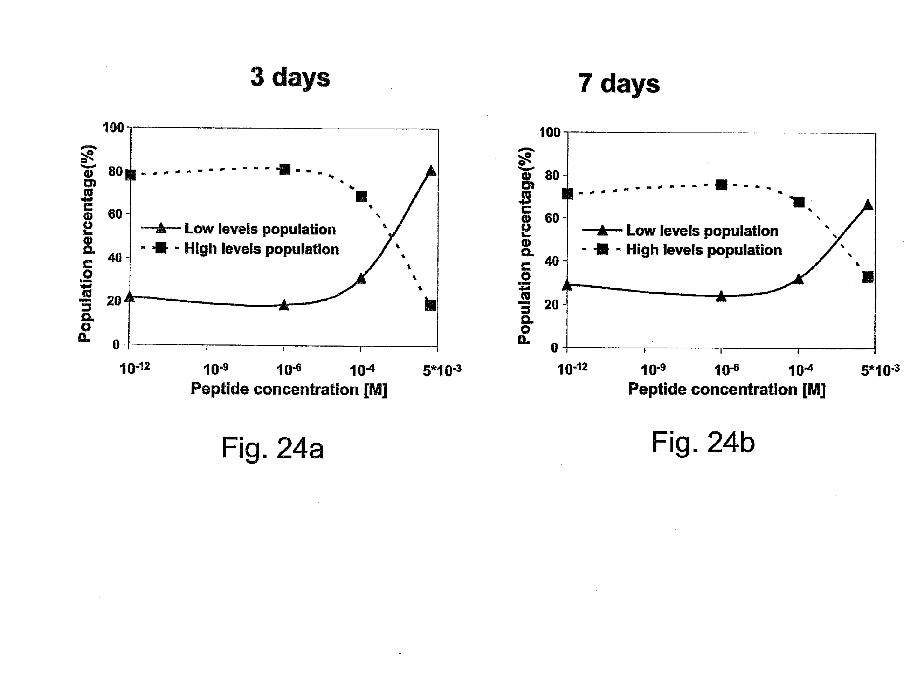

FIGs. 18a-i are FACS analyses depicting impaired expression of CD8 and CD3 after exposure of activated CTLs to high antigen density. MART-1₂₇₋₃₅-specific CTL clone JKF6 was exposed for 8 (Figures 18a-c), 24 (Figures 18d-f) or 48 (Figures 18g-i) hours to MART-1₂₇₋₃₅ peptide pulsed JY APC cells representing various antigen densities: low (10 sites; Figures 18c, f and i), intermediate-optimal (100 sites; Figures 18b, e and h) or high (650 sites; Figures 18a, d and g). Shown are the percentages of each subpopulation (CD3 or CD8). Note the impaired surface expression of CD3 or CD8 after exposure of the activated CTL to high antigen density.

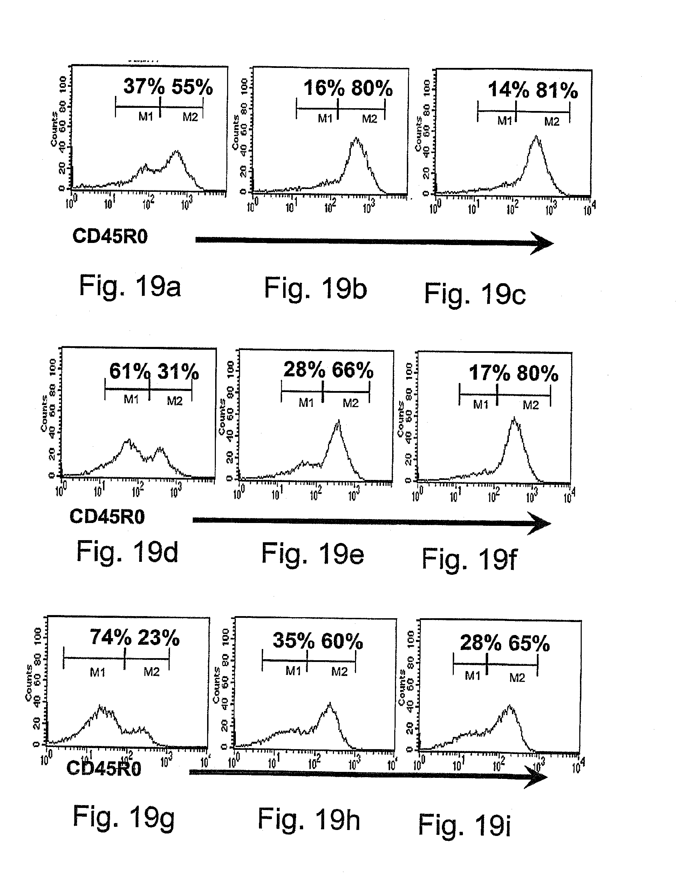

FIGs. 19a-i are FACS analyses depicting impaired expression of CD45R0 after exposure of activated CTLs to high antigen density. MART-1₂₇₋₃₅-specific CTL clone JKF6 was exposed for 8 (Figures 19a-c), 24 (Figures 19d-f) or 48 (Figures 19g-i) hours to MART-1₂₇₋₃₅ peptide pulsed JY APC cells representing various antigen densities: low (10 sites; Figures 19c, f and i), intermediate-optimal (100 sites; Figures 19b, e and h) or high (650 sites; Figures 19a, d and g). Shown are the percentages of cells expresing CD45R0. Note the impaired surface expression of CD45R0 after exposure of the activated CTL to high antigen density.

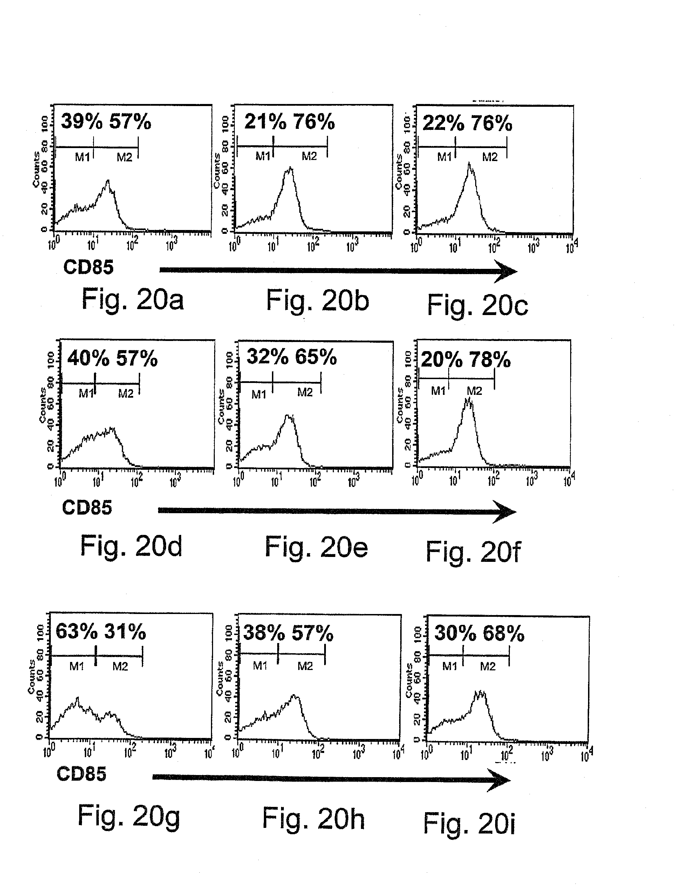

FIGs. 20a-i are FACS analyses depicting impaired expression of CD85 after exposure of activated CTLs to high antigen density. MART-1₂₇₋₃₅-specific CTL clone JKF6 was exposed for 8 (Figures 20a-c), 24 (Figures 20d-f) or 48 (Figures 20g-i) hours to MART-1₂₇₋₃₅ peptide pulsed JY APC cells representing various antigen densities: low (10 sites; Figures 20c, f and i), intermediate-optimal (100 sites; Figures 20b, e and h) or high (650 sites; Figures 20a, d and g). Shown are the percentages of CD85-expressing cells. Note the impaired surface expression of CD85 after exposure of the activated CTL to high antigen density.

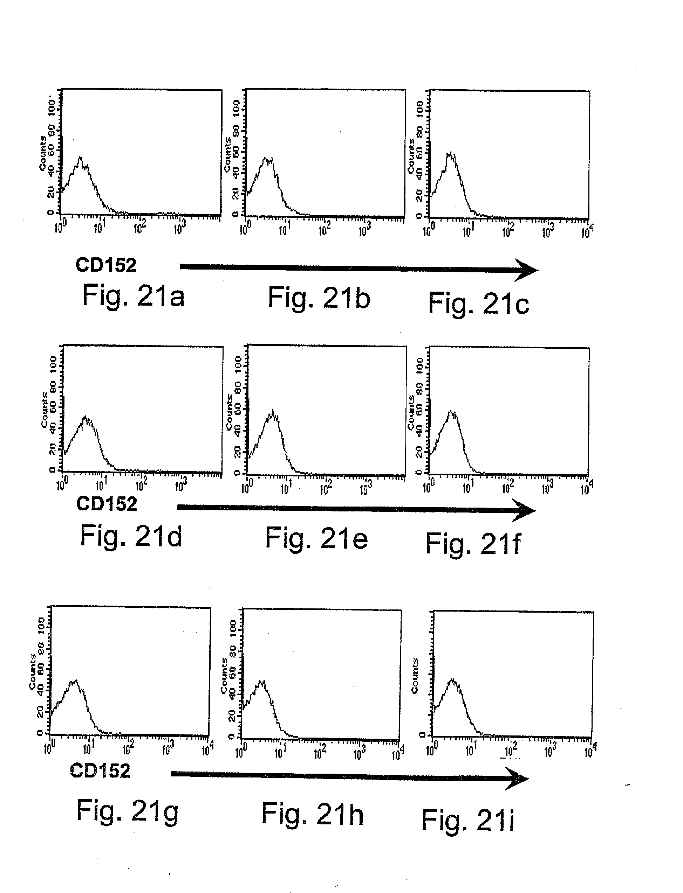

FIGs. 21a-i are FACS analyses depicting expression of CD152 after exposure of activated CTLs to high antigen density. MART-1₂₇₋₃₅-specific CTL clone JKF6 was exposed for 8 (Figures 21a-c), 24 (Figures 21d-f) or 48 (Figures 21g-i) hours to MART-1₂₇₋₃₅ peptide pulsed JY APC cells representing various antigen densities: low (10 sites; Figures 21c, f and i), intermediate-optimal (100 sites; Figures 21b, e and h) or high (650 sites; Figures 21a, d and g). Shown are the percentages of CD152-expressing cells. Note the similar expression pattern in all cells regardless of antigen density.

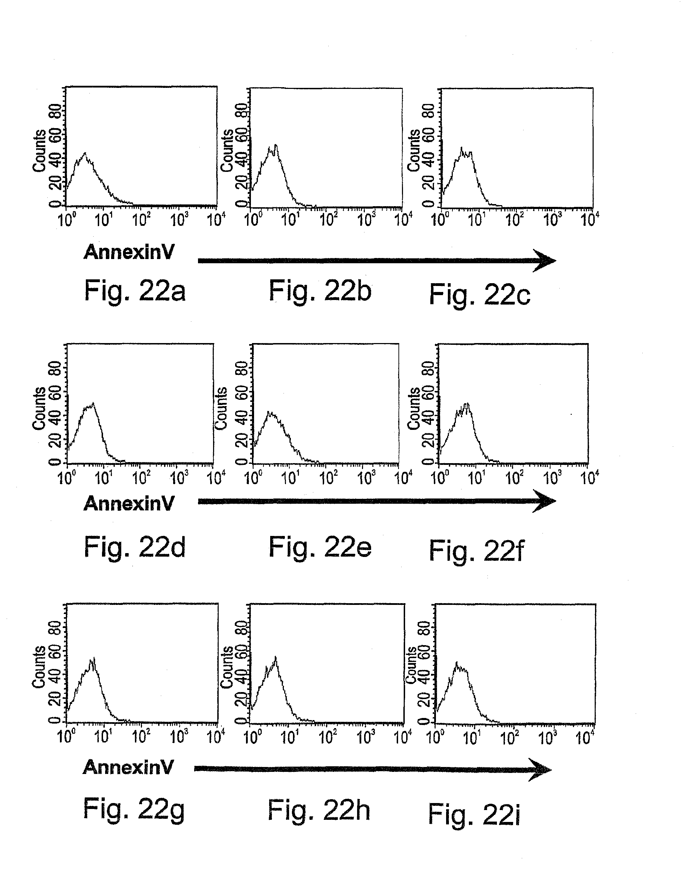

FIGs. 22a-i are FACS analyses depicting expression of Annexin V after exposure of activated CTLs to high antigen density. MART-1₂₇₋₃₅-specific CTL clone JKF6 was exposed for 8 (Figures 22a-c), 24 (Figures 22d-f) or 48 (Figures 22g-i) hours to MART-1₂₇₋₃₅ peptide pulsed JY APC cells representing various antigen densities: low (10 sites; Figures 22c, f and i), intermediate-optimal (100 sites; Figures 22b, e and h) or high (650 sites; Figures 22a, d and g). Shown are the percentages of Annexin V -expressing cells. Note the similar expression pattern in all cells regardless of antigen density.

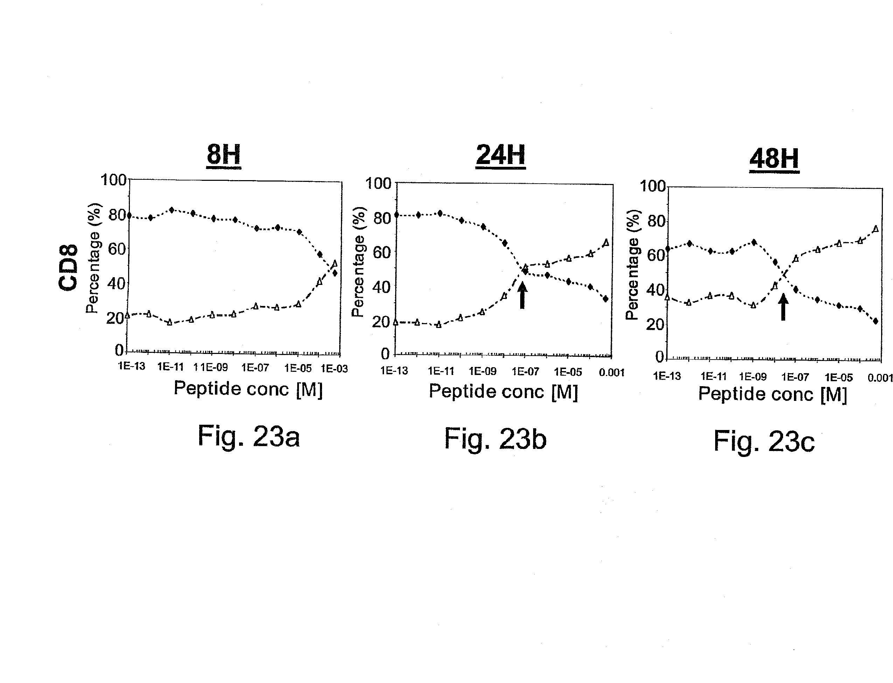

FIGs. 23a-i are histograms depicting the dynamics of the expression of key surface molecules after exposure of activated CTLs to high antigen density. CD8^{High}, CD45R0^{High},CD85^{Dim} homogenous CTLs were exposed to JY pulsed pAPCs. Two distinct subpopulations of CD8^{High}, CD45R0^{High}, CD85^{Dim} or CD8^{Low}, CD45R0^{Low}, CD85⁻phenotypes were created after exposure. The percentage of High and Low subpopulations is shown as a function of exposure time and antigen density on target cells for each of the
   surface markers: CD8 (Figures 23a-c), CD45R0 (Figures 23d-f), CD85 (Figures 23g-i). Results are shown following exposure of activated CTLs for 8 hours (Figures 23a, d and g), 24 hours (Figures 23b, e and h) and 48 hours (Figures 23c, f and i) to the JY pulsed pAPCs. High expression level population is marked with filled diamods and low expression level population is marked with empty triangles. A significant transition point marked with an arrow was observed when CTLs were exposed to peptide concentration above 1 x 10⁻⁷ M, corresponding to ~100 complexes/cell, most profoundly 48 hours post exposure.
Figure 24a-b shows the distribution of CD8 high and low subpopulation in CTLs exposed to high antigen densities 3 or 7 days post exposure. The data indicate that the phenotypic changes in these CTLs are long lasting and persist even 7 days after exposure.

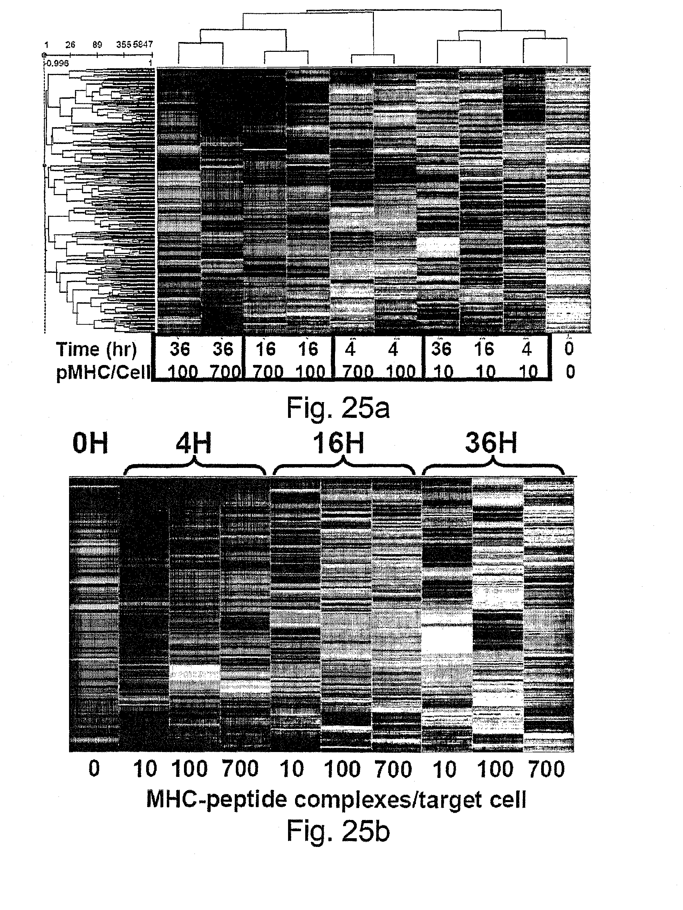

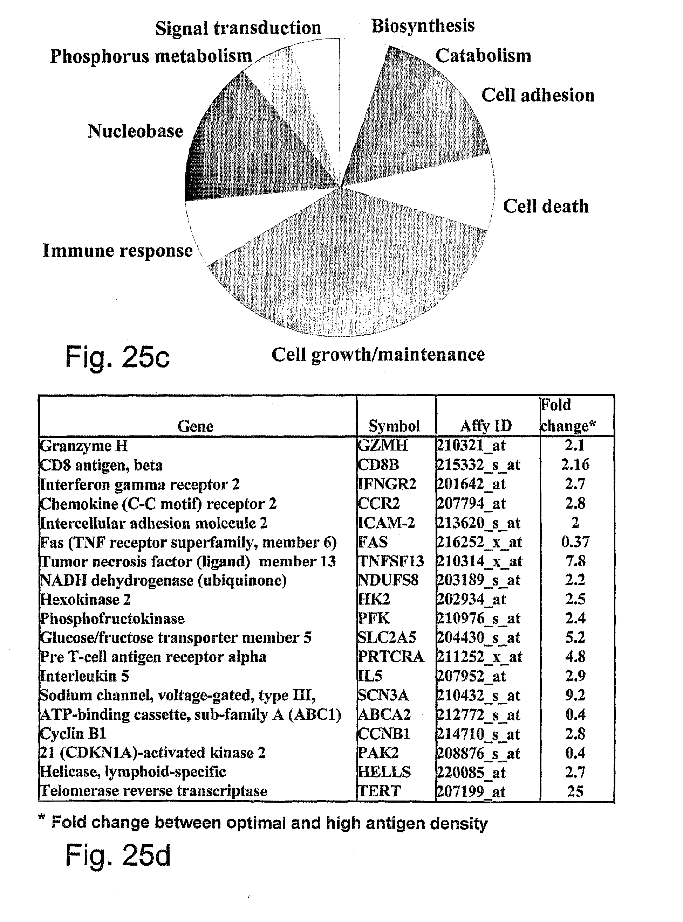

FIGs. 25a-d depict distinct gene expression signatures indicative of anergy in CTLs exposed to high density of antigen. Figure 25a - A dendogram of 5877 genes which altered at least 2 fold in at least one of the treated versus untreated CTLs. Dendogram indicates levels of similarity between samples. Green = low expression: Red = high expression. CTL samples exposed to low antigen densities were clustered together with CTLs at time 0, before exposure to antigen (right branch), indicating high similarity between the gene expression profiles of these samples. CTLs exposed to optimal (100) or high (700) antigen densities were clustered together at each time point (4, 16 or 36 hours). Dendogram distances indicate a common starting point for these samples and progressively increasing differences between CTLs exposed to optimal versus high antigen densities with a maximal effect after 36 hours. Figure 25b - Gene expression signature of 1070 probe sets which altered at least 2 fold between CTLs exposed to high or optimal antigen density, 36 hours post exposure. The clustering image demonstrates relatively high similarity between CTLs exposed to optimal or high antigen densities 4 and 16 hours post exposure. However, a profound shift toward two distinct expression profiles is demonstrated after 36 hours. This indicates separation of gene expression profile into two distinct types of CTLs; those with a gene signature of an optimal stimulation and function achieved by exposure to optimal antigen density, versus anergic CTL gene signature observed after exposure to high antigen densities. Figure 25c - A functional classification of the 1070 probe sets which were altered by at least 2 fold between high and optimal antigen density 36 hours post exposure. Classification was performed using GO classification at level 3. Figure 15d - A representative list of genes from the gene array analysis of 1070 probe sets described in Figures 25b and c. Shown are fold changes in the expression of representative genes from CTL exposed to optimal versus high antigen density. Alterations in gene expression related to cell cycle control, cell death, immune function, membrane potential, energy metabolism and signal transduction are shown. Note the genes from this analysis which exhibit major change in expression between optimal and high antigen density after 36 hours of exposure to antigen. The controls and normalizations for these DNA arrays experiments are summarized in the general methods section.

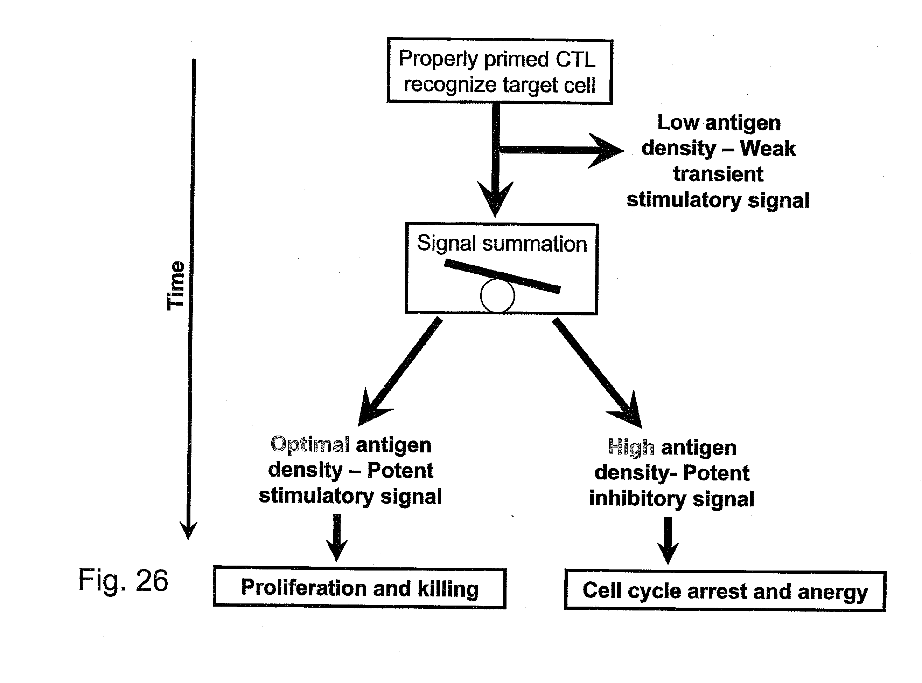

FIG. 26 is a schematic illustration depicitng a proposed model for activated CTL function as a self referential sensory organ that is highly sensitive to antigen density; the -Y model. CTLs that were properly primed can be exposed to low antigen density which is translated into a weak and transient activation state. Optimal antigen densities lead to a strong and stable activation process that result in proliferation and effective killing of target cells. However, high antigen densities on the target cell lead to cell cycle arrest and a long lasting anergic state of the CTL.

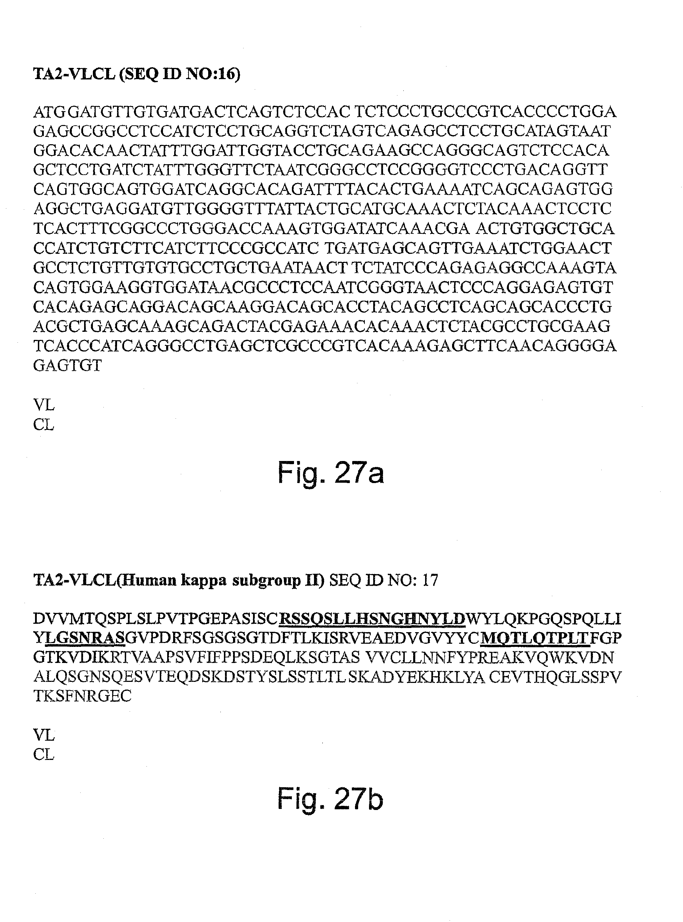

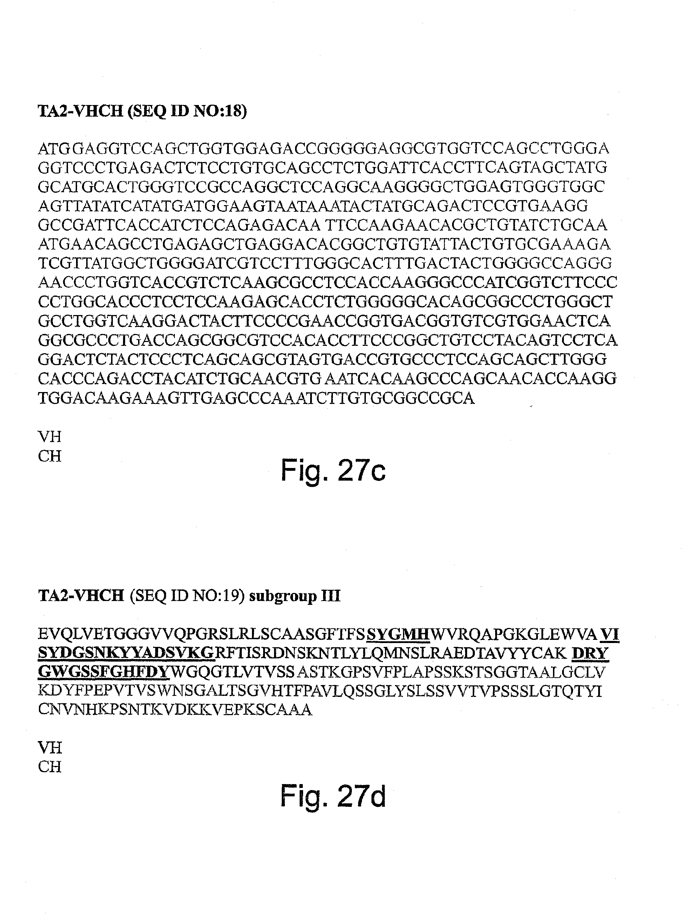

FIGs. 27a-d depict nucleic acid (Figures 27a, c) and amino acid (Figures 27b, d) sequences of TA2-VLCL (SEQ ID NOs: 16 and 17; Figures 27a-b) and TA2-VHCH (SEQ ID NOs:18 and 19; Figures 27c-d). CDR sequences are in bold and underlined.

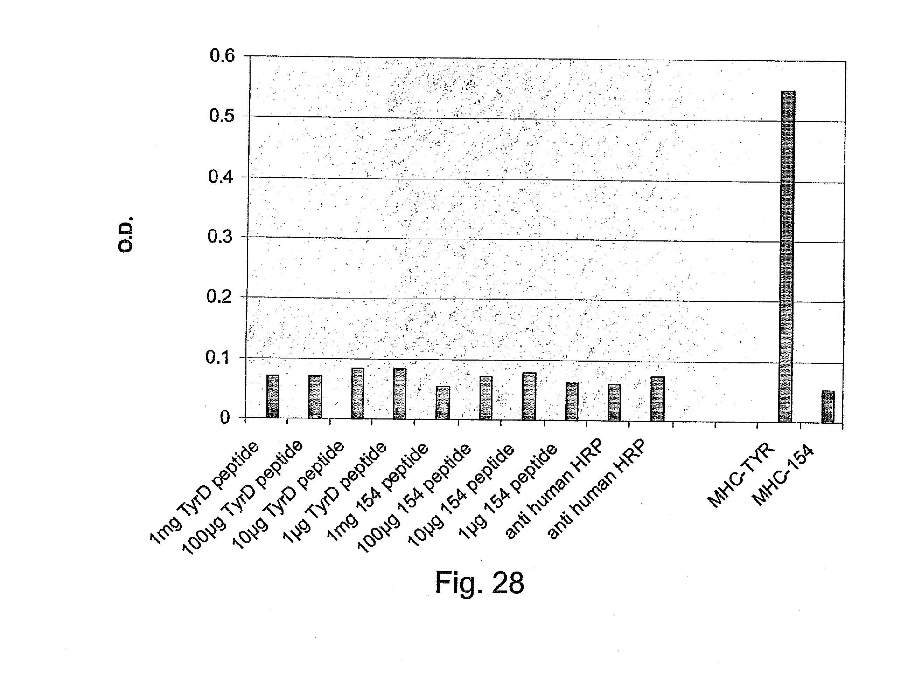

FIG. 28 is a bar graph depicting the specific recognition of binding of the TA2 TCRL antibody IgG to the Tyrosinase/HLA-2 complex. Note the specific binding of the TA2 TCRL antibody to the MHC-TYR complex (which includes the antigenic peptide of SEQ ID NO:1) as compared to the absence of binding of the TA2 TCRL to the MHC-154 complex (which includes the antigenic peptide of SEQ ID NO:20), the absence of binding of the TA2 TRCL antibody to the Tyrosinase D (TyrD; SEQ ID NO:1) peptide alone at various concentrations (1 mg, 100 µg, 10 µg or 1 µg), the absence of binding of the TA2 TCRL antibody to the 154 peptide (GP100 154 SEQ NO. 20) at various concentrations (1 mg, 100 µg, 10 µg or 1 µg) as well as the absence of binding of anti human HRP to both peptides, the left for 154 peptide and the right for Tyr D peptide).

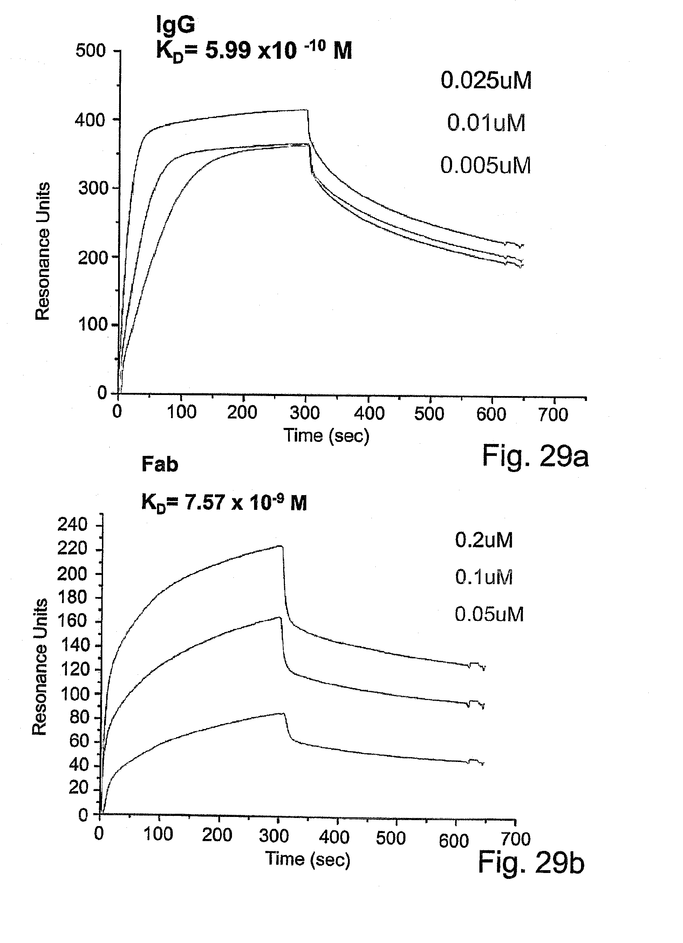

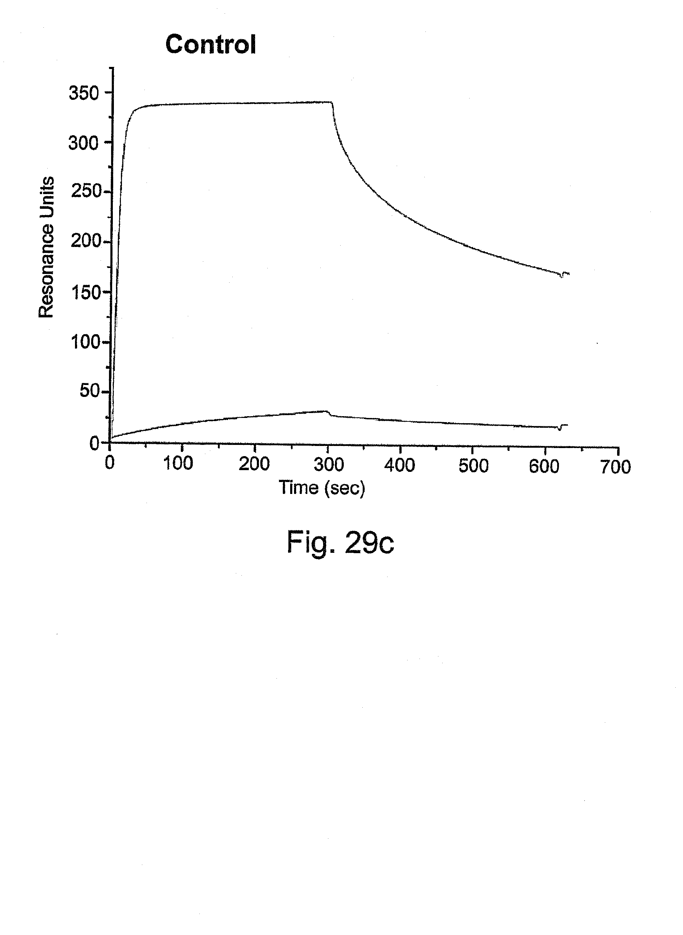

FIGs. 29a-c depict plasmon resonance real-time binding kinetics of anti-HLA-A2/Tyrosinase TA2 Fab and IgG. Streptavidin coated beads were loaded with biotinylated HLA-A2-Tyr D complexes. 0.2M, 0.1M, 0.05M TA2 Fab or 0.025M, 0.01M, 0.005M TA2 IgG antibody was attached to the complexes, followed by wash with PBS.

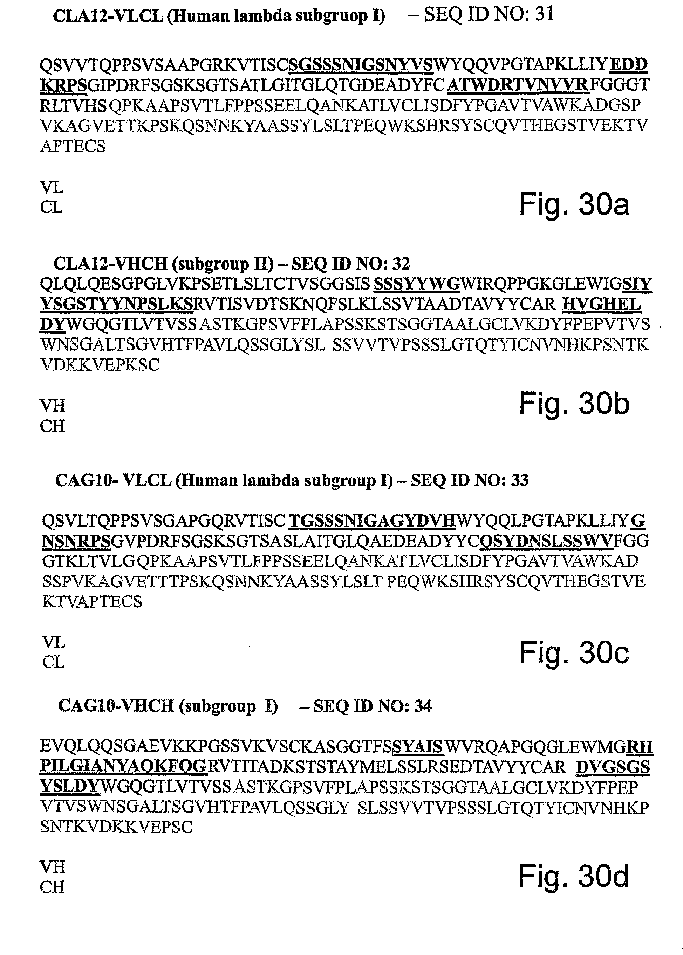

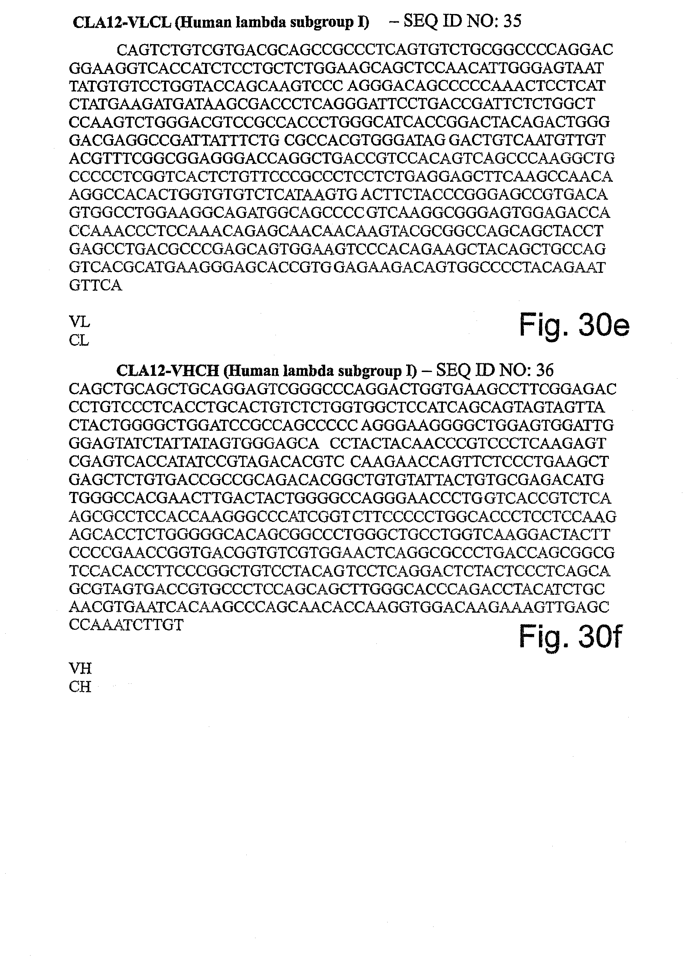

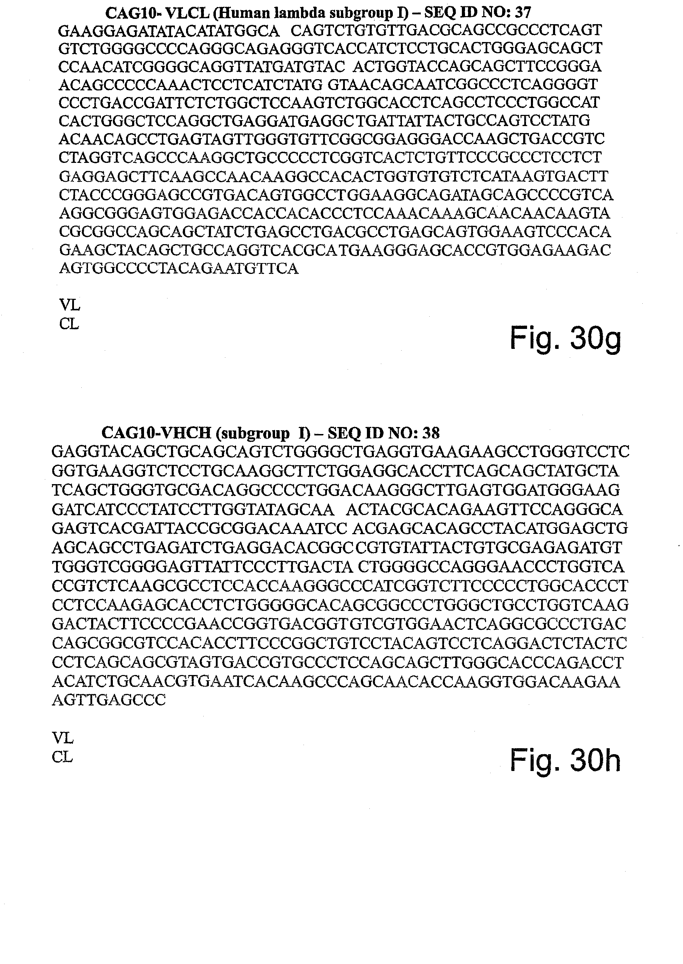

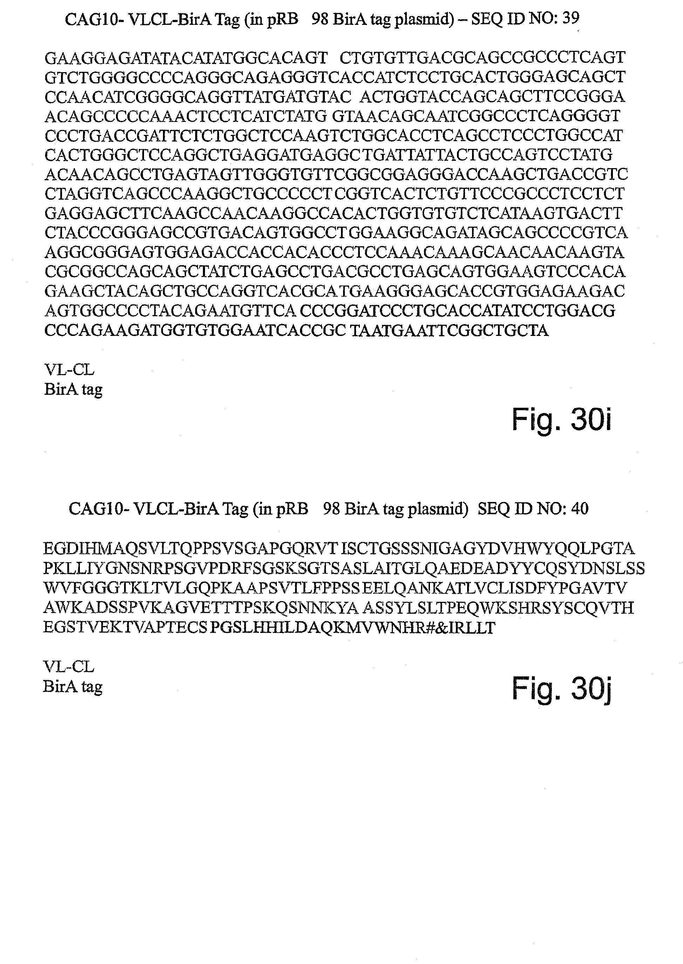

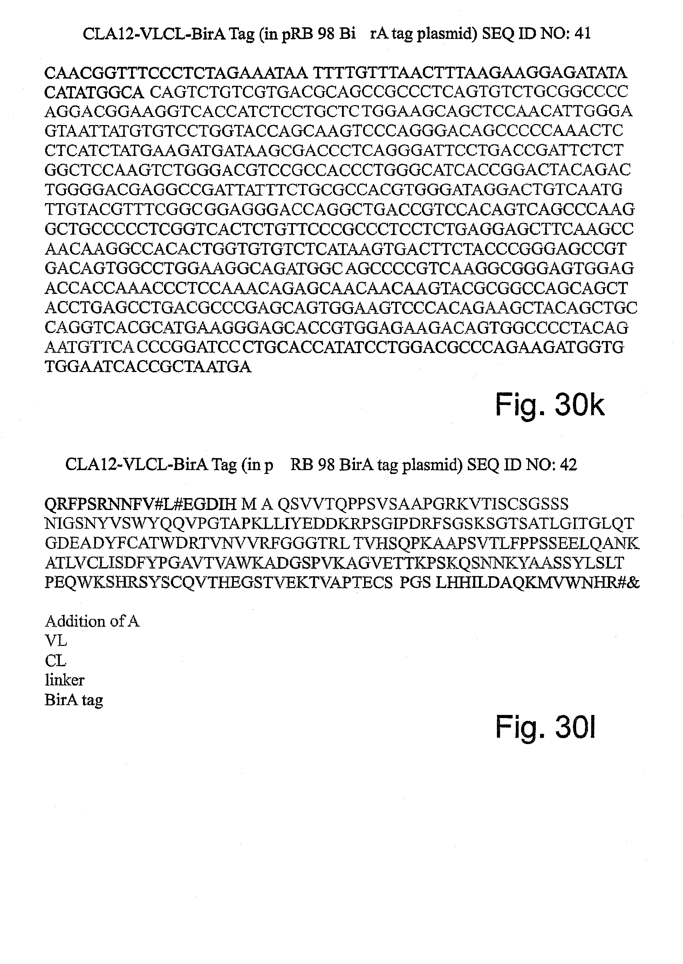

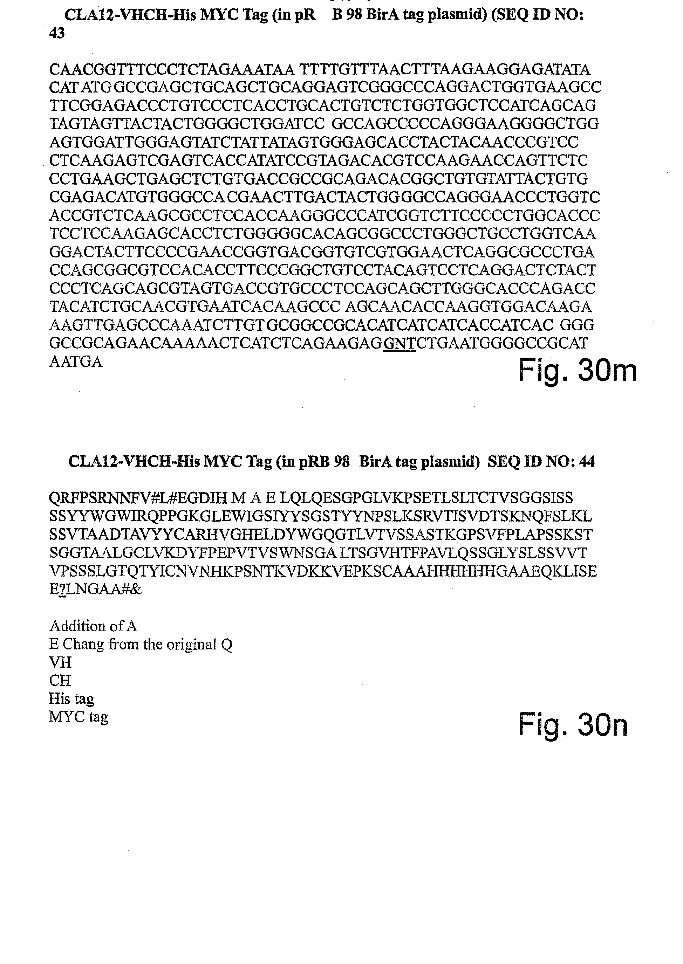

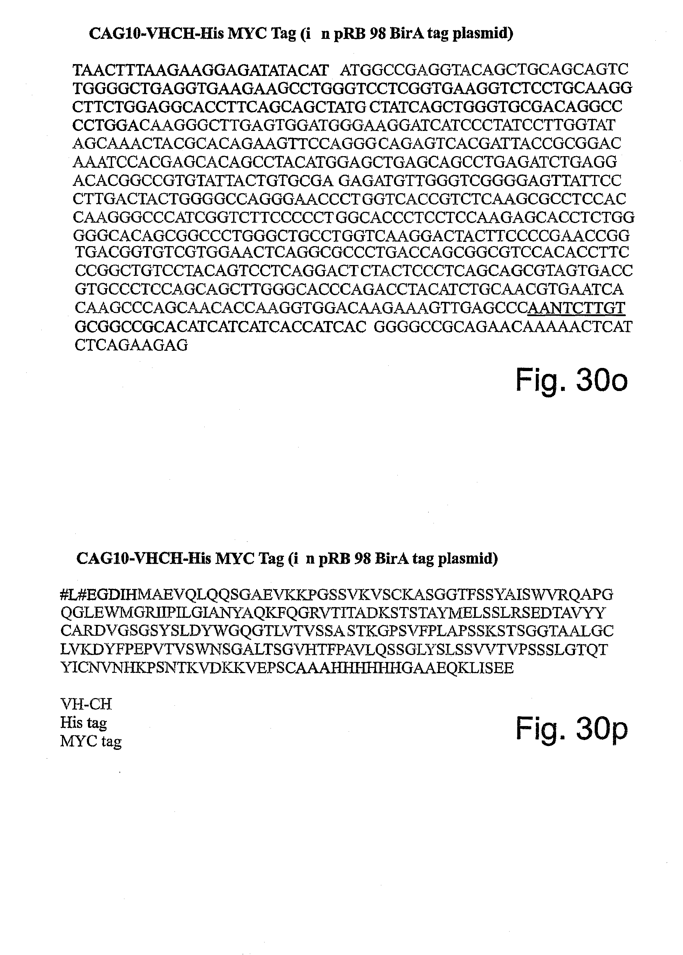

FIGs. 30a-h depict amino acid (Figures 30a-d) and nucleic acid (Figures 30e-h) sequences of CLA12-VLCL (SEQ ID NOs: 31 and 35; Figures 30a, e), CLA12-VHCH (SEQ ID NOs: 32 and 36; Figures 30b, f), CAG10-VLCL (SEQ ID NOs: 33 and 37; Figures 30c, g), CAG10 VHCH (SEQ ID NOs: 34 and 38; Figures 30d, h). CDR sequences are in bold and underlined
FIGs. 30i-p depict nucleic acid and amino acid sequences of the pRB 98 BirA tag plasmids comprising CAG10-VLCL (SEQ ID NOs: 39 and 40); CAG10-VHCH (SEQ ID NOs: 45 and 46); CLA12-VLCL (SEQ ID NO: 41 and 42) and CLA12-VHCH (SEQ ID NO: 43 and 44).

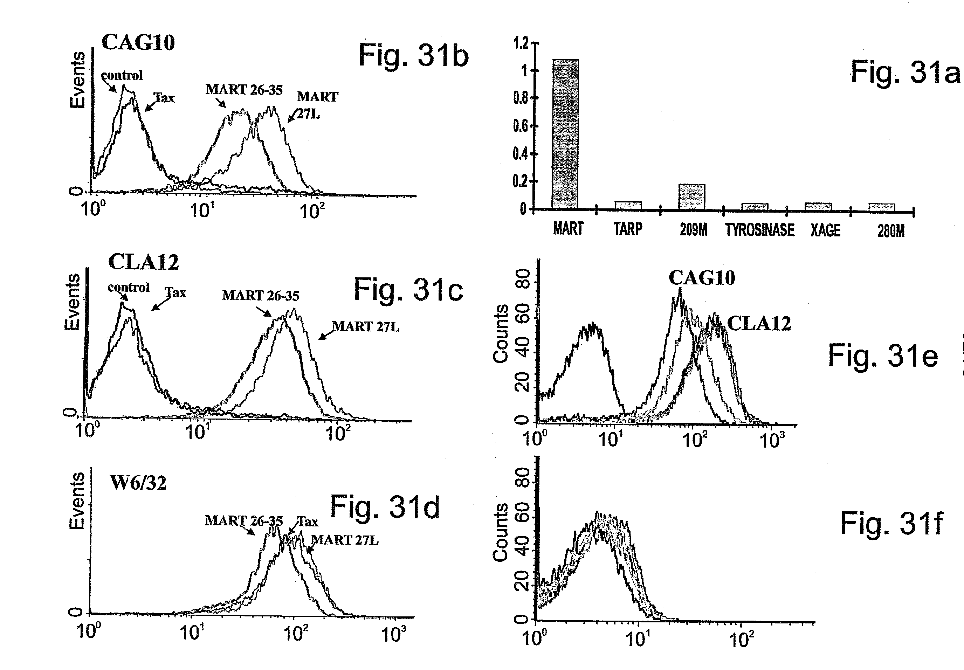

FIGs. 31a-f depict the binding of Fabs CAG10 and CLA12 to peptide-loaded antigen presenting cells. Figure 31a depicts the fine specificity of the purified Fab clones by ELISA. TCR-like Fabs as tested by ELISA for binding refolded peptide-MHC complexes. Figures 31b-c - Detection of Mart-1 peptide/HLA-A2 complexes on antigen presenting cells RMA-S-HHD. RMA-S cells were loaded with specific Mart-1 (26-35 (SEQ ID NO:21) or 27L (SEQ ID NO: 27) or control TAX peptide (SEQ ID NO:26). Complexes were probed with recombinant purified Fab CAG10 (Figure 31b) and CLA12 (Figure 31 c) and analyzed by FACS using FITC-labeled goat anti-human Fab. Figure 31d: mAb W6/32 binding demonstrates the total peptide/HLA on the APCs cell surface. Figures 31d-e: Detection by FACS of Mart-1 peptide/HLA-A2 complexes with Fab CAG10 and Fab CLA12 on antigen presenting JY cells loaded with specific (Figure 31e) or control (Figure 31f) peptide.

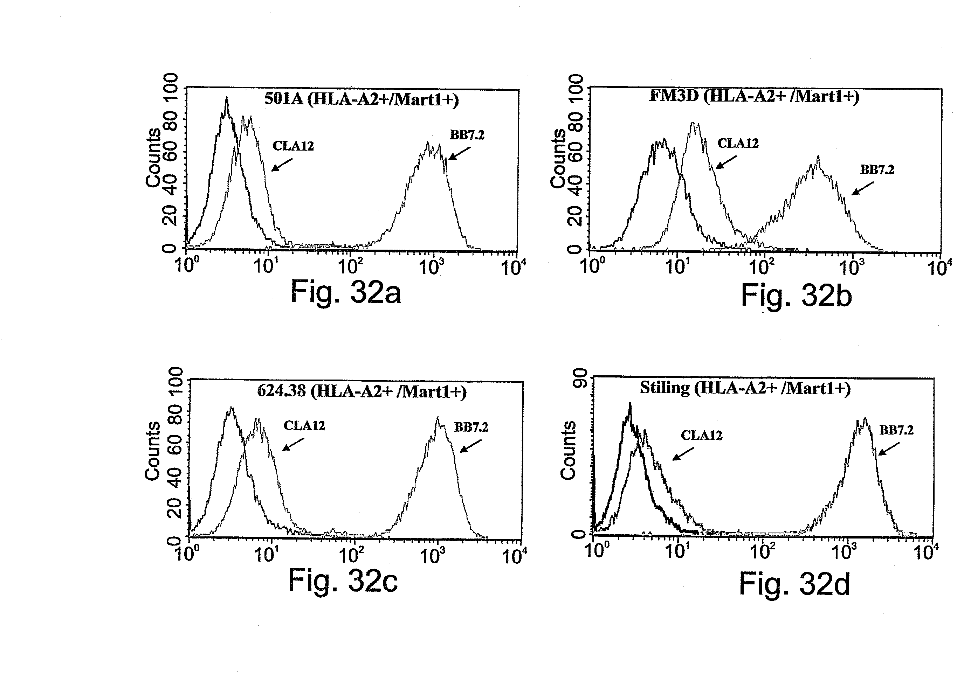

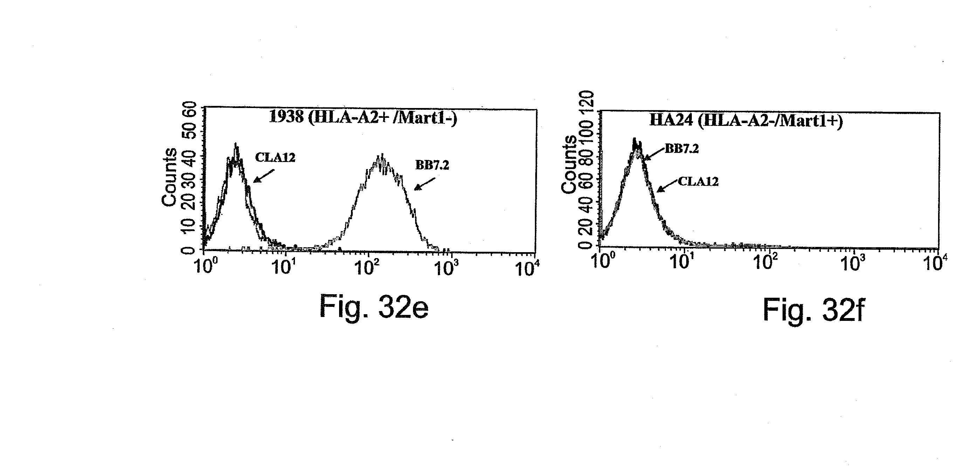

FIGs. 32a-f are FACS analyses measuring binding of Fab CLA12 to melanoma cell lines. Detection of Mart-1 peptide/HLA-A2 complexes was performed on HLA-A2+Mart-1+ melanoma cell lines 501A (Figure 32a), FM3D (Figure 32b), 624.38 (Figure 32c) and Stiling (Figure 32d) and on control cell lines: the HLA-A2+Mart-1- melanoma 1938 (Figure 32e) or the HLA-A2-Mart-1+ melanoma G-43 (Figure 32f) by Fab CLA12. Complexes were detected by FACS using recombinant purified Fab CLA12 and FITC-labeled goat anti-human Fab. mAb BB7.2 recognizes total HLA-A2 and was used to validate HLA-A2 expression by the indicated cell lines.

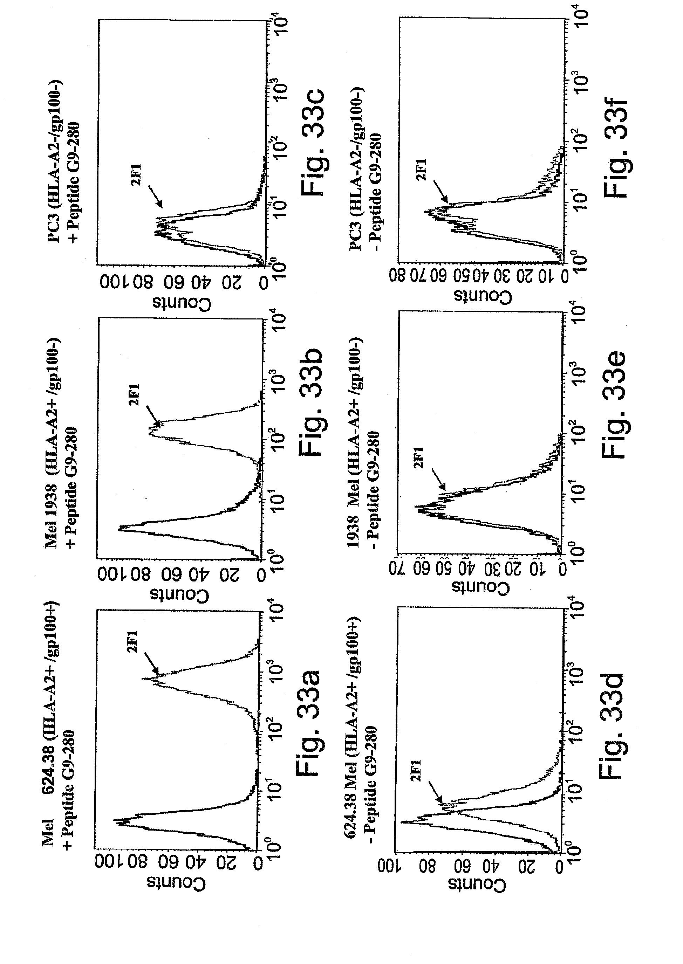

FIGs. 33a-f are FACS analyses measuring binding of Fab 2F1 to melanoma cell lines. Detection of G9-280 peptide/HLA-A2 (SEQ ID NO:5) complexes on HLA-A2+gp100+ melanoma cell lines 624.38 (Figures 33a,d) HLA-A2+gp100- 1938 (Figures 33b,e) and on the HLA-A2-gp100- melanoma PC3 (Figure 33c, f), when cells were preloaded with G9-280 (Figures 33a-c) or not (d-f).

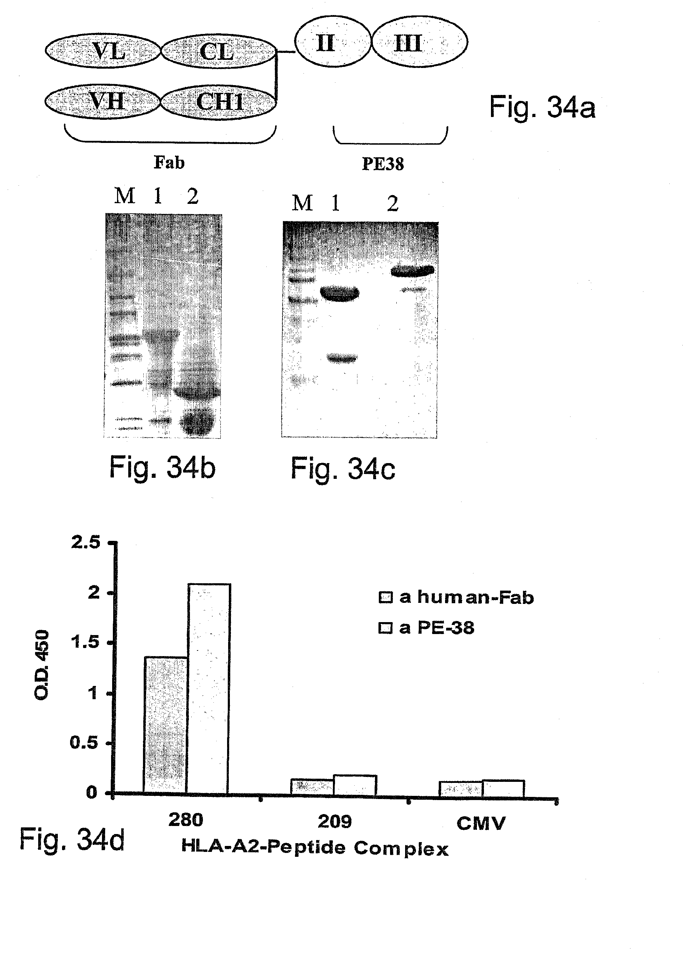

FIGs. 34a-d depict schematics and analyses of recombinant purified Fab 2F1-PE38KDEL. Figure 34a is a schematic representation of the Fab 2F1-PE38 fusion protein. The gene encoding PE38 that contains the translocation and ADP ribosylation domains of PE was fused to the C terminus of Fab 2F1 (Lκ) κ chain. Figure 34b is an SDS/gel analysis on 4-20 % gradient gels of recombinant Fab 2F1 Light chain-PE38 fusion (Lκ)-PE38 with the expected size of 63 KDa (Lanes 1) and Fab 2F1 heavy (H) chain - 25 KDa (Lane 2) from bacterial inclusion bodies. Figure 34c is an SDS/gel analysis on 4-20 % gradient gels of Fab 2F1-PE38KDEL reduced with expected bands at 63 KDa and 25 KDa (Lane 1); Fab 2F1-PE38KDEL nonreduced - 88 KDa (Lane 2). Figure 34d depicts the fine specificity of the purified 2F1 Fab PE38KDEL as analyzed by ELISA. pMHC complexes were refolded using each peptide and coated via Streptavidin on an ELISA plate. An equal concentration of TCR-like Fab-toxin was incubated for 1 h at room temperature. After extensive washing, bound clones were detected with an anti-human Fab or anti-PE mAb coupled to HRP.

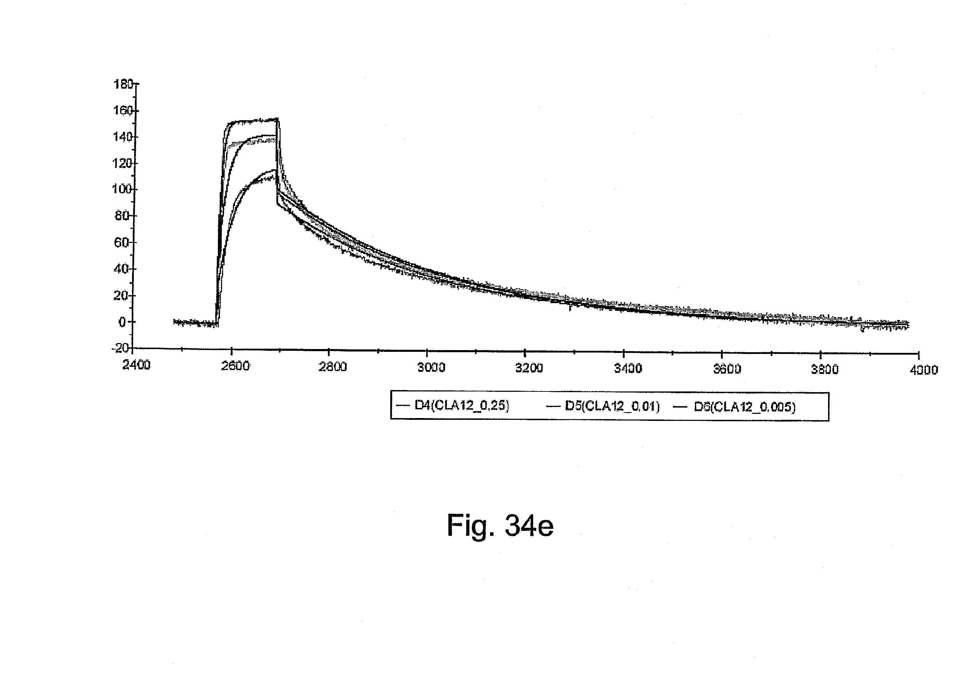

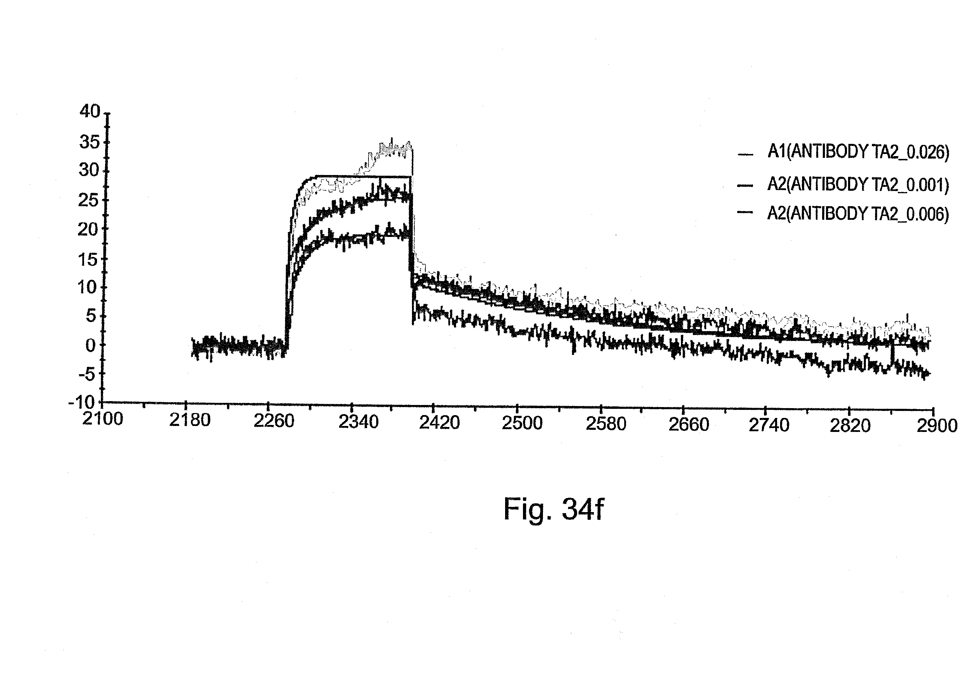

FIGs. 34e-f are biacore results showing affinity of CLA12 (Figure 34e) and TA2 (Figure 34f) to the complexed antigen.

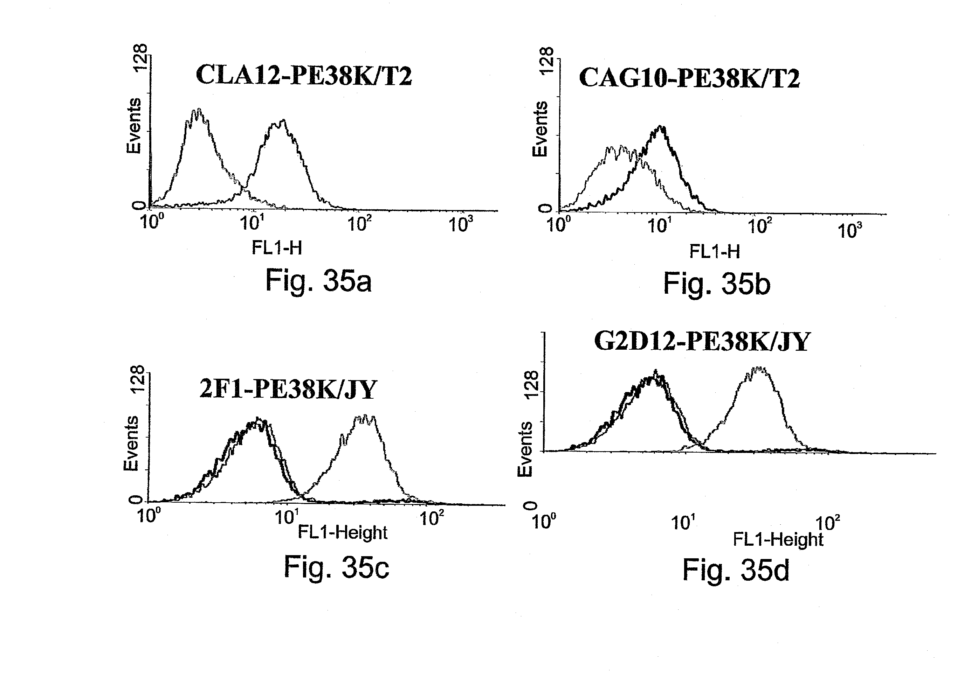

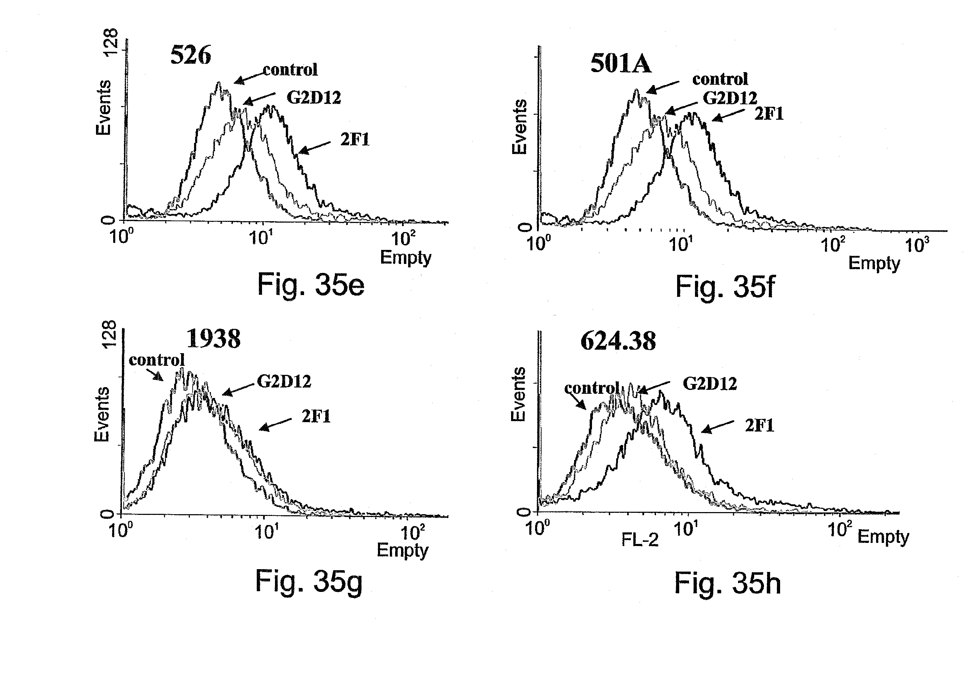

FIGs. 35a-h are FACS analyses measuring binding of Fab-PE38KDEL clones to peptide loaded APCs and melanoma cell lines. For Figures 35a-d, tap deficient A2+ T2 cells were pulsed with 50 µM MART-1 (Figures 35a-b), 50 µM G9-280 (SEQ ID NO: 5; Figure 35c), 50 µM G9-154 (SEQ ID NO: 20; Figure 35d) or no peptide (control), then stained with the designated Mart-1/HLA-A2 (Figures 35a-b), G9-280/HLA-A2 (Figure 35c) or G9-154/HLA-A2 (Figure 35d) -selected Fab-toxins and analyzed by flow cytometry. For Figures 35e-h, detection of GP100-derived peptide/HLA-A2 complexes on HLA-A2+gp100+ melanoma cell lines Me1526 (Figure 35e), Me1501A (Figure 35f) and Mel624.38 (Figure 35h) No staining was observed by Fab-toxin 2F1-PE38KDEL or G2D12-PE38KDEL on HLA-A2+gp100- melanoma 1938 (Figure 35g). Complexes were detected by flow cytometry using FITC-labeled goat anti-human Fab.

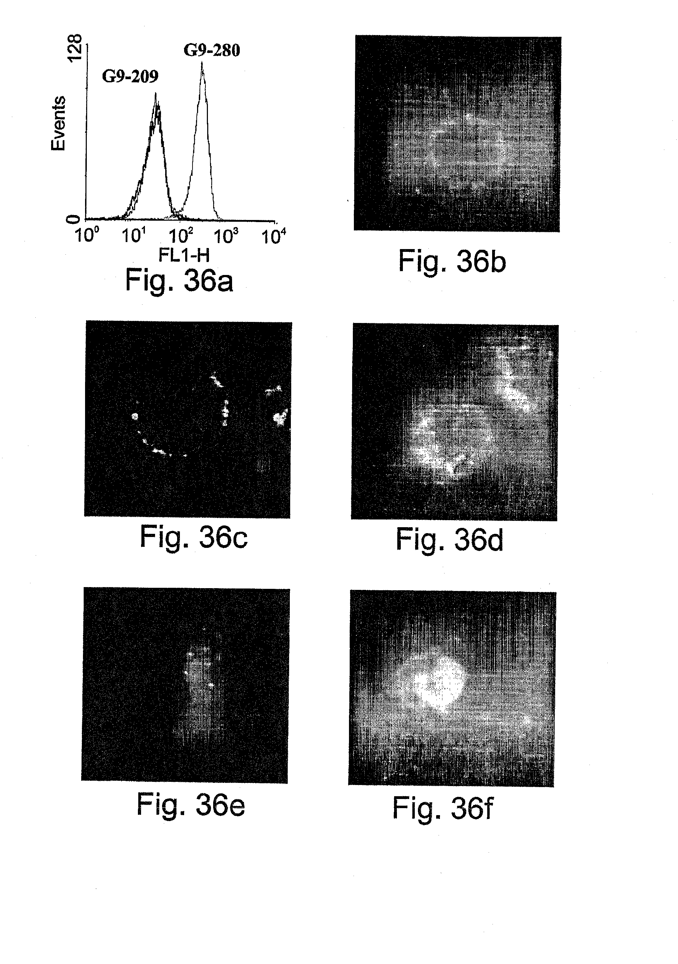

FIGs. 36a-f depict the cellular binding and internalization of 2F1-PE38-FITC. JY cells were loaded with G9-280 (SEQ ID NO: 5) or G9-209 (SEQ ID NO: 4) peptide and incubated at 4 °C in the presence of 2F1-PE38-FITC. Figure 36a - no staining was detected on JY cells loaded with G9-209. Cells were then transferred to 37 °C and monitored for the immunotoxin internalization at indicated time points 0 (Figure 36b); 15 minutes (Figure 36c); 30 minutes (Figure 36d); 1 hour (Figure 36e) and 6 hours (Figure 36f). PI was used to detect the nucleus.

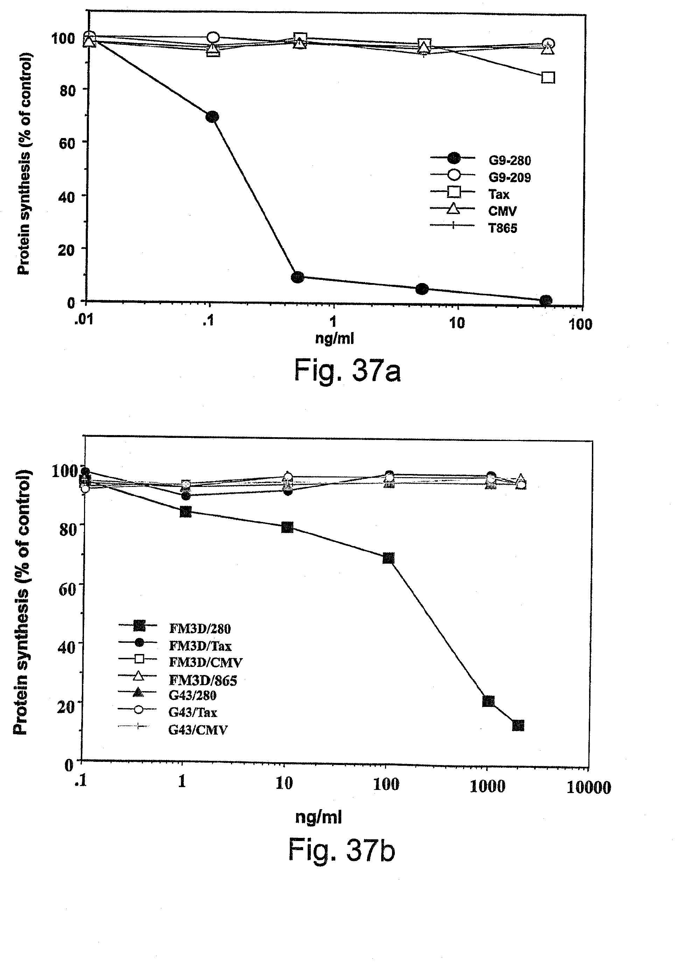

FIGs. 37a-b are graphs depicting the cytotoxic activity of 2F1-PE38-KDEL toward peptide loaded APCs. Figure 37a: Cytotoxic activity of recombinant Fab 2F1-PE38KDEL towards JY cells loaded with G9-280 peptide (SEQ ID NO: 5) or with other control HLA-A2 restricted peptides. Cells were incubated for 24 hr with recombinant Fab 2F1-PE38KDEL. [³H] leucine incorporation into cellular protein was measured. The results are expressed as a percentage of control where no immunotoxin was added. Figure 37b is the same assay as in Figure 37a was performed on HLA-A2+ FM3D cells and HLA-A2- G43 cells loaded with the specific G9-280 (SEQ ID NO: 5), or control HLA-A2 restricted peptides.

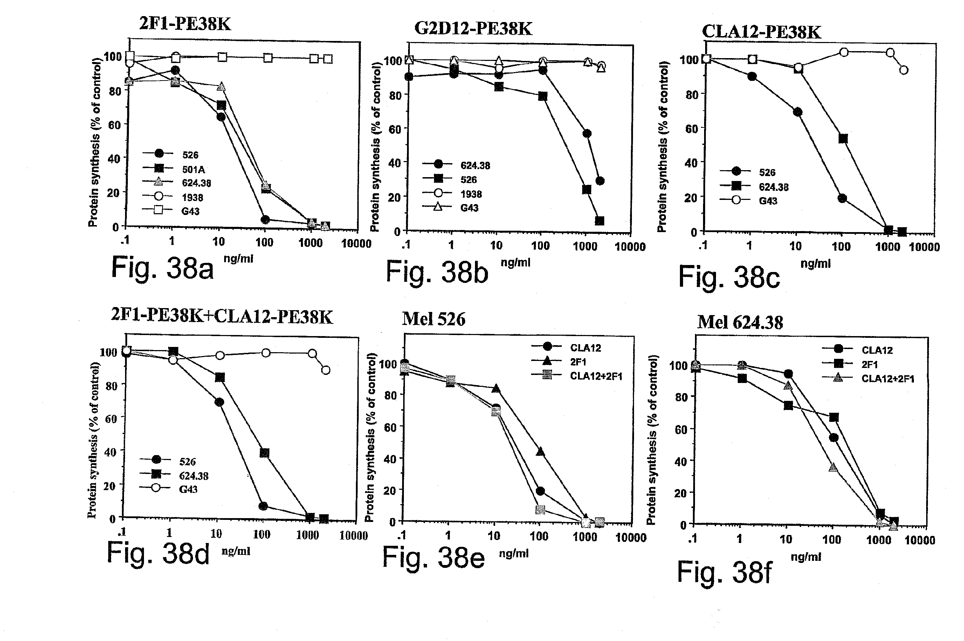

Figures 38a-f are graphs depicting the cytotoxic activity of 2F1-PE38-KDEL toward melanoma cell lines. Figures 38a-c: Cytotoxic activity of 2F1-PE38KDEL (Figure 38a), G2D12-PE38KDEL (Figure 38b) and CLA12-PE38KDEL (Figure 38c) toward melanoma cell lines Me1526, Me1624.38, Me1501A, G43, 1938 after 24 h of incubation. [H³] leucine incorporation into cellular protein was measured. The results are expressed as a percentage of control where no immunotoxin was added. Figure 39d: Cytotoxic effect of combination treatment of two immunotoxin 2F1-PE38KDEL and CLA12-PE38KDEL on melanoma cells Mel526, Mel624, G43. Figures 38e-f: Cytotoxic effect of CLA12-PE38KDEL or 2F1-PE38KDEL alone or CLA12-PE38KDEL plus 2F1-PE38KDEL on Mel526 (Figure 38e) or Mel 624.38 (Figure 38f). The cells were incubated with the immunotoxins for 24 h. [³H] leucine incorporation into cellular protein was measured. The results are expressed as a percentage of control where no immunotoxin was added.

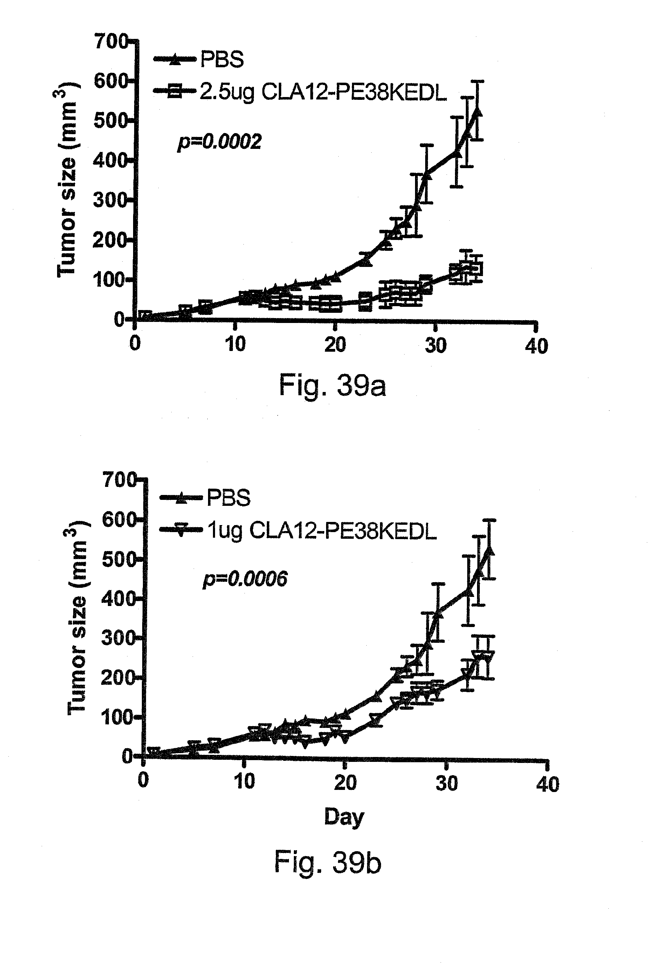

FIGs. 39a-b are graphs depicting the anti-tumor activity of CLA12-PE38KDEL on subcutanous human melanoma tumor in SCID mice. Groups of animals were injected with 10x10⁶ 526 melanoma cells on day 0. When tumor reached 55 mm³, animals were treated i.v. on day 10, 12, 14, 16 with 0.125 mg/kg (Figure 39a) and 0.05mg/kg (Figure 39b) CLA12 Fab-PE38KDEL in PBS. Control group received diluent alone. No cytotoxicity was observed at these doses. Comparison of tumor size between treated and untreated group gave P < 0.0006. Data are expressed as the mean ± SD (n=4).

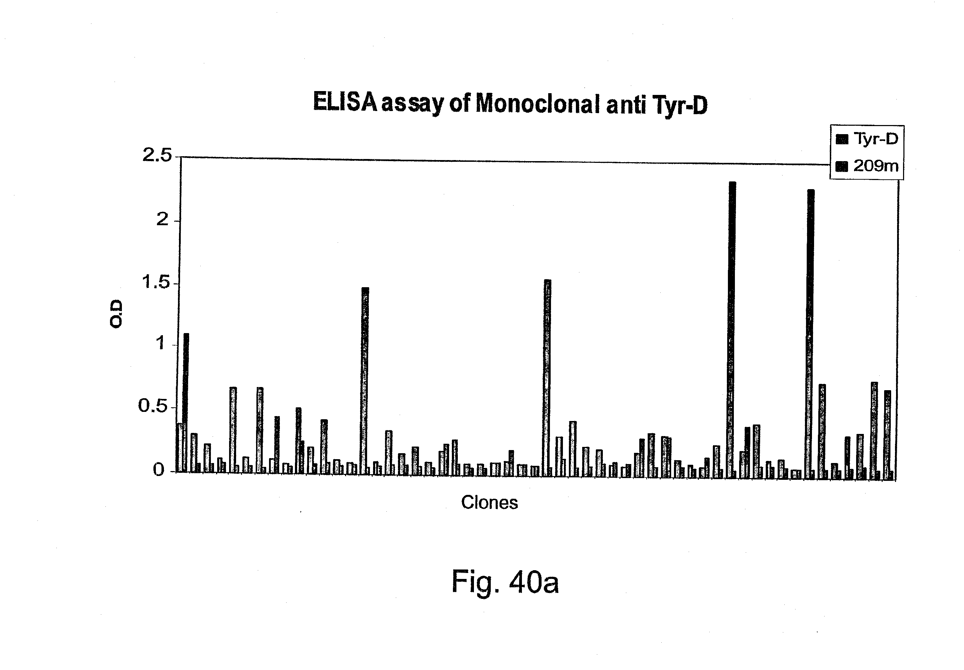

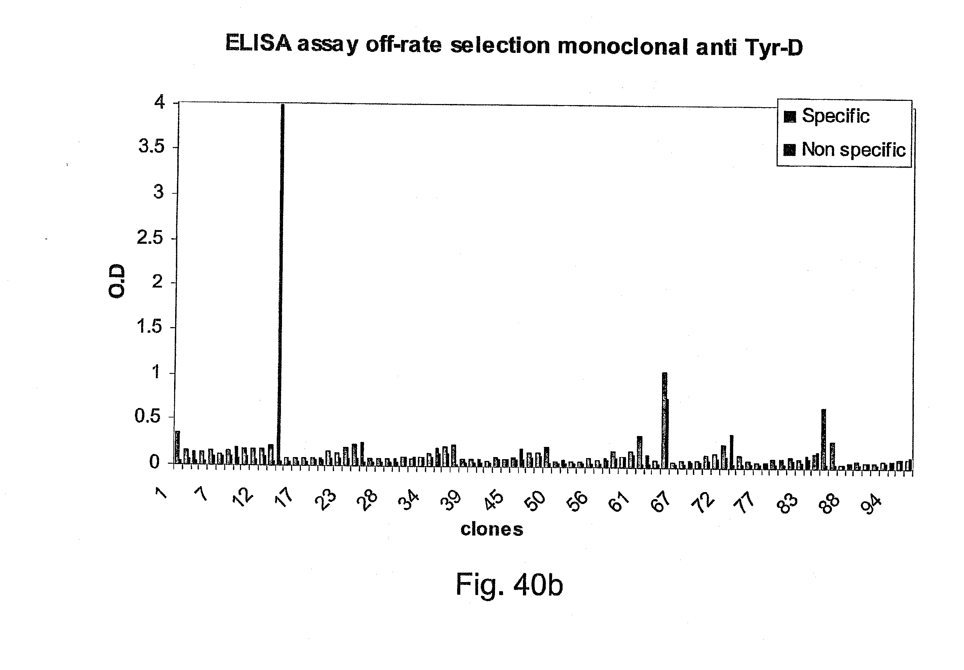

FIGs. 40a-b are bar graphs illustrating the monoclonal ELISA of Tyr/HLA-A2 TCR-like antibodies. Figure 40a: Reactivity of Fab antibody clones from the conventional screening method with recombinant purified Tyr/HLA-A2 and control complex. ScHLA-A2-peptide complexes were generated by in vitro refolding as described in materials and methods. Detection was with Peroxidase-labeled anti-human Fab. Figure 40b: Reactivity of Fab antibody clones from the Off-Rate selections with recombinant purified Tyr/HLA-A2 and control complex. ScHLA-A2-peptide complexes were generated by in vitro refolding as described in materials and methods. Detection was with Peroxidase-labeled anti-human Fab.

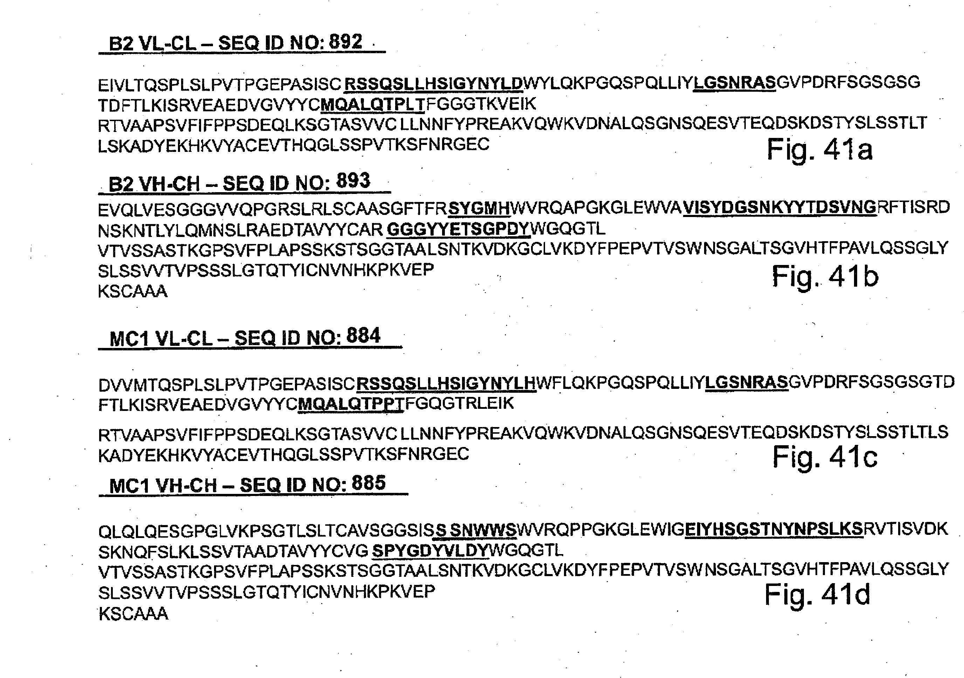

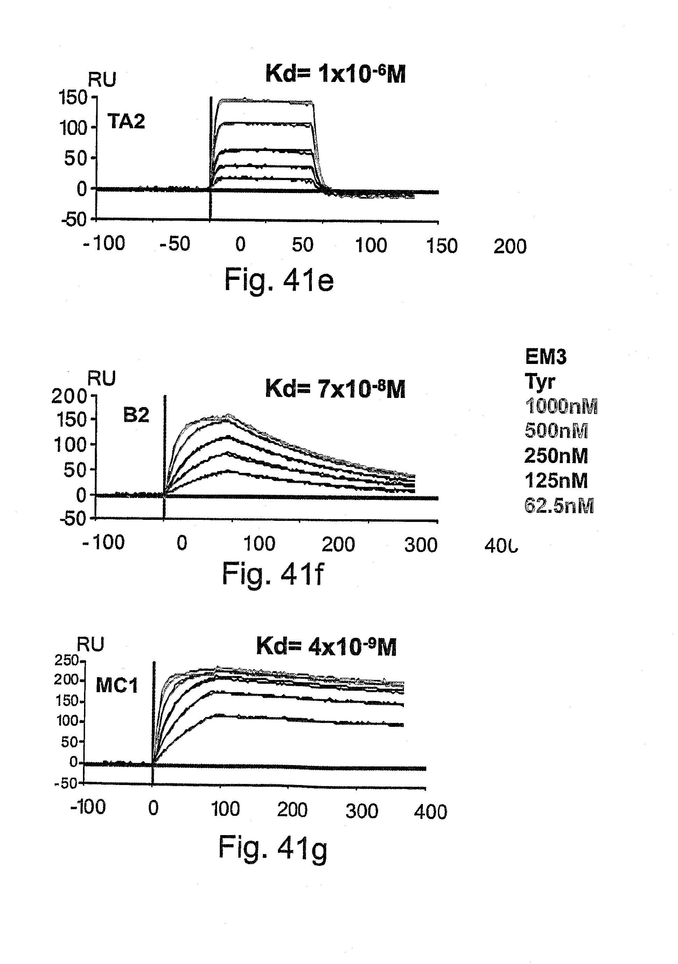

FIGs. 41a-g are sequence and SPR Affinity Determinations of the three different anti Tyr Fabs. Figure 41a-d are the deduced amino acid sequence of the HLA-A2/ *Tyr₃₆₉.* ₃₇₇ Fabs, B2, MC1 (bold letters indicating the position of the CDR's). Figure 41e-g are SPR Binding curves of the three Fabs with 5 different concentrations. The chip was covered with Fab anti Fab to which the Fab Abs were conjugated, the ligand the analyte the HLA-A2/Tyr complex. The chip was covered with Fab anti-human Fab, the three anti HLA-A2-Tyr Fabs were bound (i.e. the ligand), and then the HLA-A2-Tyr complex was bound (i.e. the analyte).

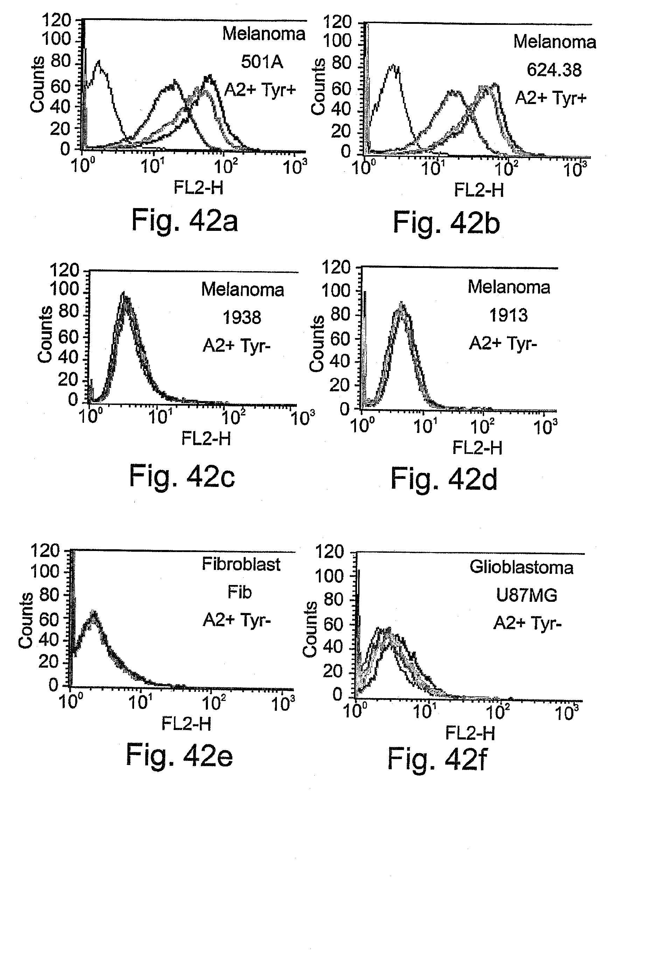

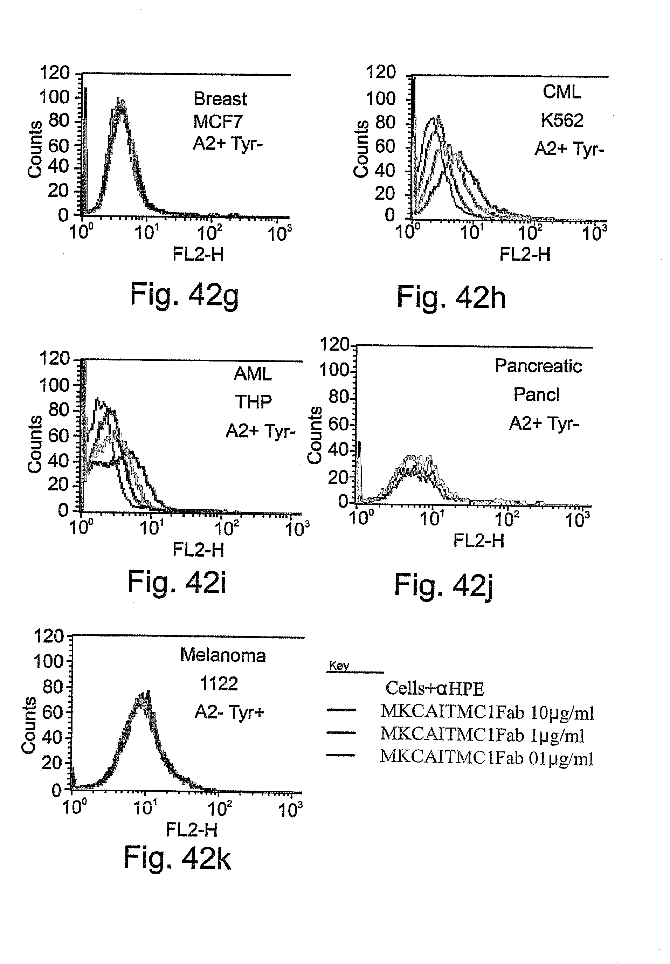

FIGs. 42a-k are graphs illustrating the results of flow cytometry analysis of the binding of MC1 Fabs to tumor cells. Detection was with PE-labeled anti-human Fab.

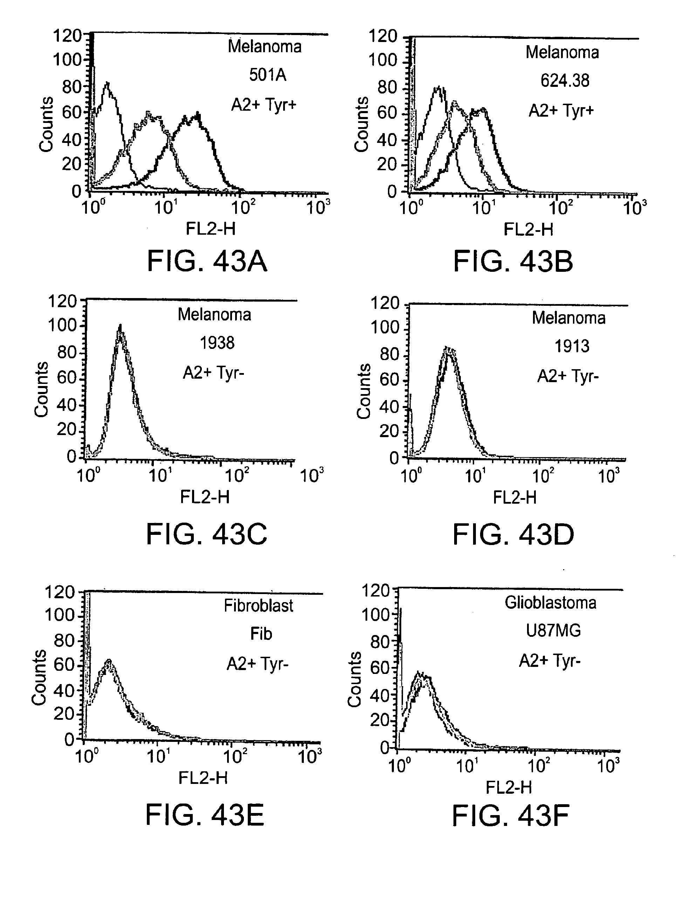

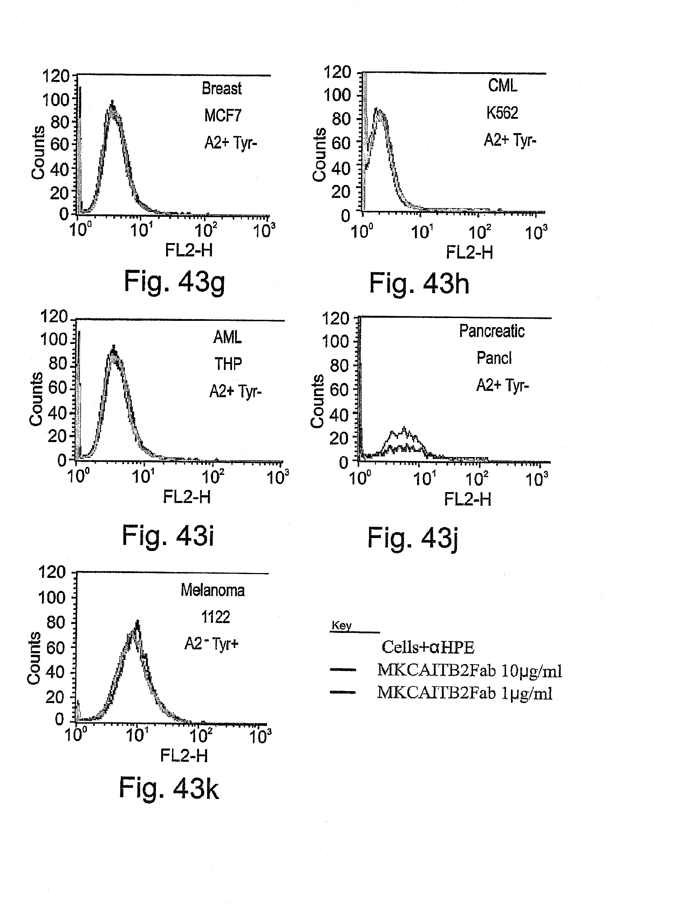

FIGs. 43a-k are graphs illustrating the results of flow cytometry analysis of the binding of B2 Fabs to tumor cells. Detection was with PE-labeled anti-human Fab.

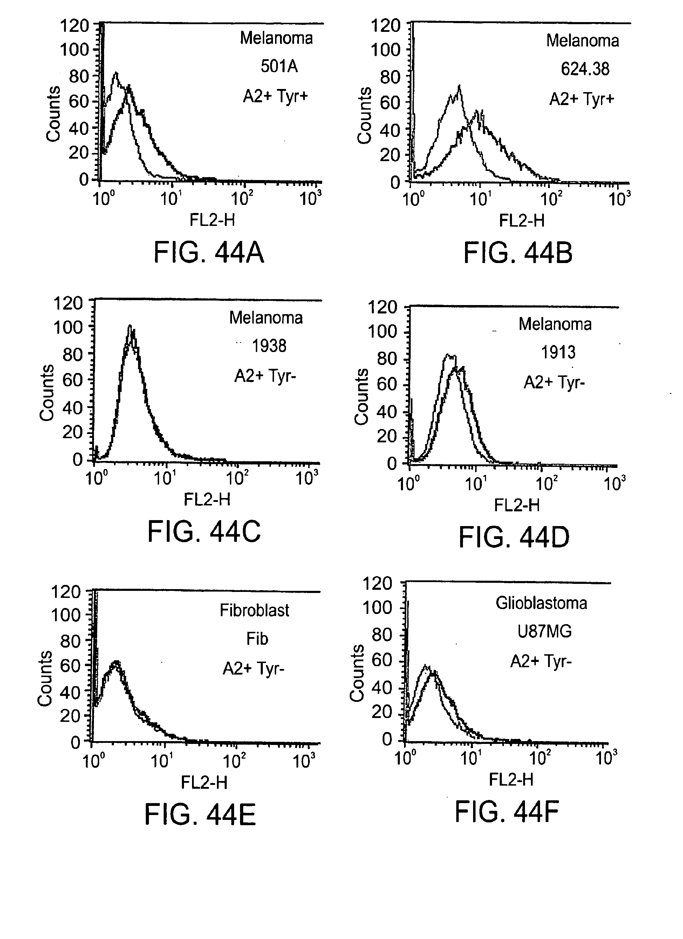

FIGs. 44a-k are graphs illustrating the results of flow cytometry analysis of the binding of TA2 Fabs to tumor cells. Detection was with PE-labeled anti-human Fab.

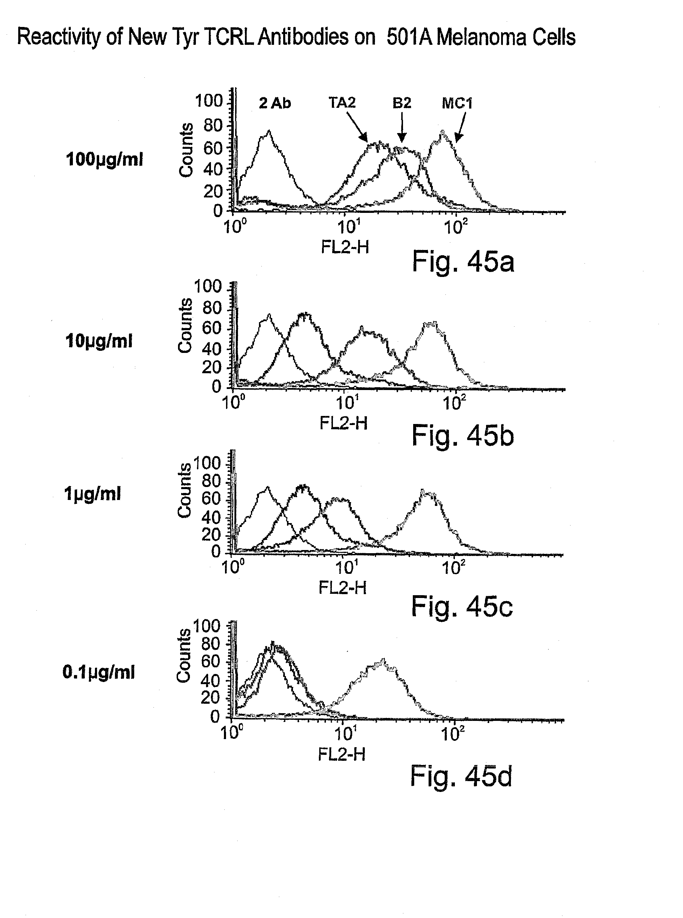

FIGs. 45a-d are graphs illustrating that the MC1, B2, TA2 Fabs recognized Tyrosinase positive and HLA-A2 positive cells (501A melanoma cells) with a very high intensity corresponding to their improved affinity. The detection limit of TA2 was 10µg/ml on positive cells while B2 had detection limit of 1µg/ml on positive cells and MC1 reacted in 0.1µg/ml on positive cells.

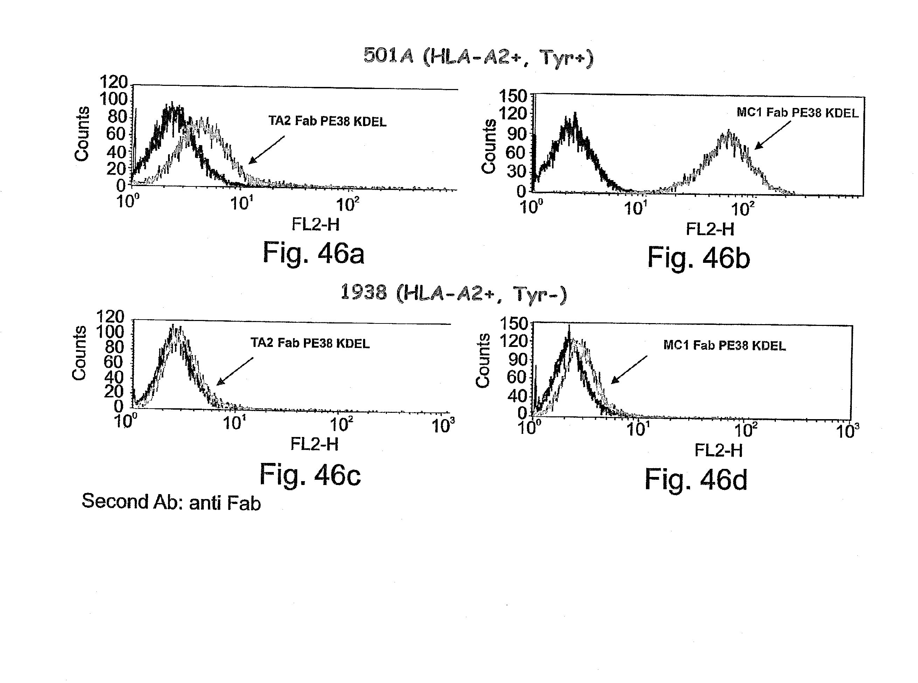

FIGs. 46a-d are graphs illustrating binding of TA2 and MC1 Fab-PE38 fusion molecules to tumor cells as measured by flow cytometry analysis. Binding of TA2 and MC1 Fab-PE38 to Tyr positive and negative tumor cells was monitored by PE-labeled anti-human Fab.

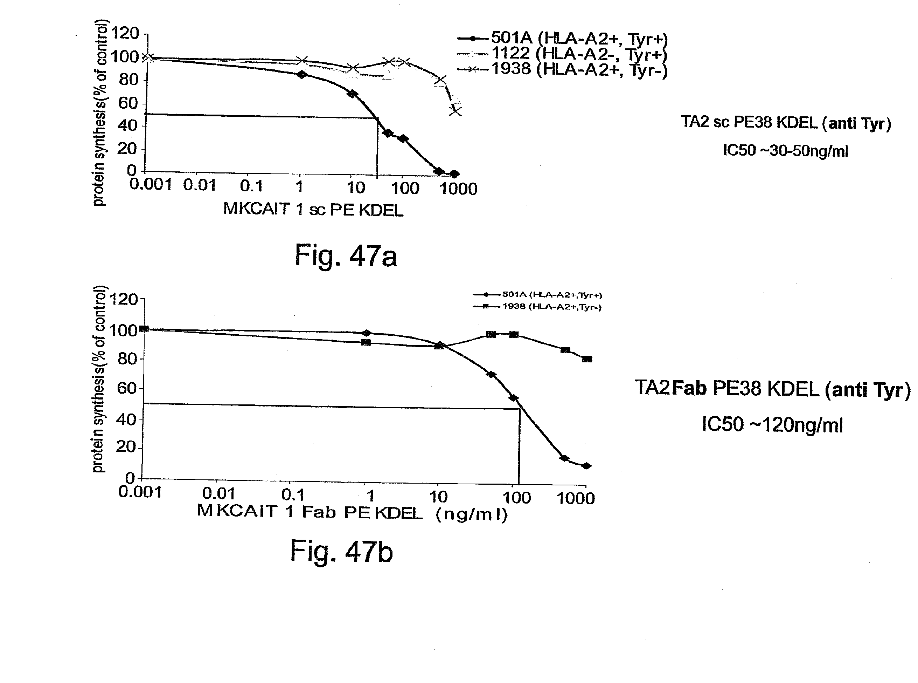

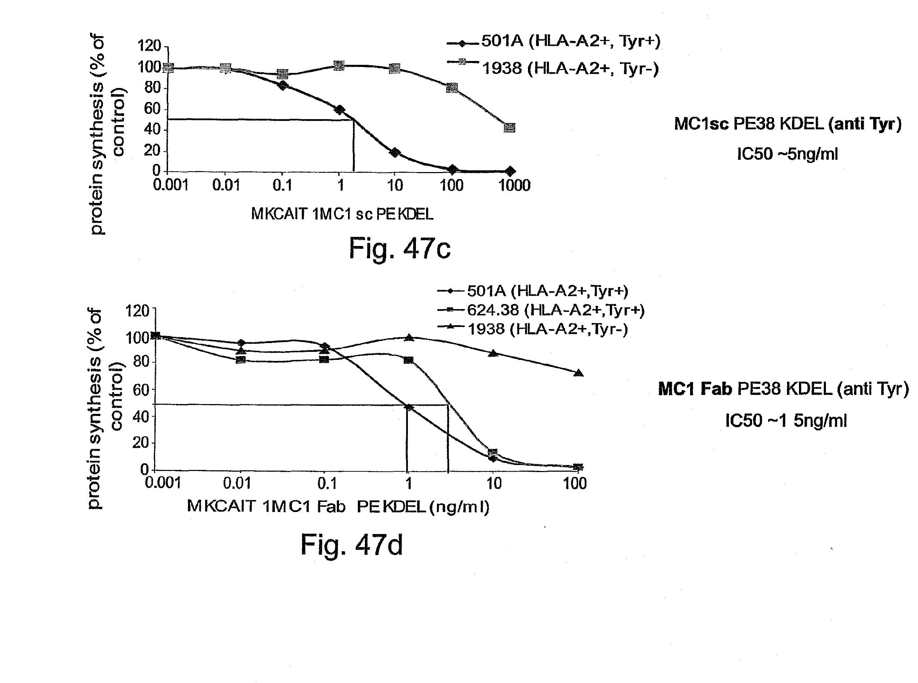

FIGs. 47a-d are graphs illustrating the cytotoxic activity of TA2 Fab/scFv-PE38 fusion and MC1 Fab/scFv-PE38KDEL fusion following analysis of inhibition of protein synthesis in tumor cells. Tyr positive and control Tyr negative tumor cells were incubated for 20 hours with increasing concentrations of TA2 Fab/scFv -PE38 (Figure 47a-b) or MC1 Fab/scFv-PE38KDEL (Figure 47c-d). Protein synthesis was determined by incorporation of 3H-Leucine into cellular proteins for 4 hours.

### DESCRIPTION OF THE PREFERRED EMBODIMENTS

The present invention is of methods of diagnosing and treating melanoma and of antibodies capable of same.

The principles and operation of the diagnostic and therapeutic methods of present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Use of antibodies that specifically recognize class I MHC-peptide complexes as therapeutic and diagnostic tools for the treatment and detection of cancer in general and melanoma specifically has already been established (e.g., U.S. Pat. Appl. No. 20050255101).

The present invention uncovers which is the optimum melanoma-associated class I MHC-peptide complex for such antibodies to target and therefore paves the way for the discovery of more potent antibodies both for the treatment and diagnosis of melanoma.

Whilst reducing the present invention to practice, the present inventors have generated phage-derived human T cell receptor (TCR)-like antibodies to the three major differentiation antigens known to be expressed on melanoma cells. The present inventors have shown, using one such antibody specific to the NMC-Tyrosinase₃₆₉₋₃₇₇ complex, that the Tyrosinase₃₆₉₋₃₇₇ epitope is highly presented on melanoma cell lines as analyzed by flow cytometry (Figures' 4a-g) and confocal microscopy (Figures 6a-d). Verification of the observation that high numbers of tyrosinase peptides are presented on HLA-A2 complexes was obtained by performing cytotoxicity tests with cytotoxic T cells (CTLs) that specifically recognize the HLA-A2- Tyrosinase₃₆₉₋₃₇₇ epitope (Figures 5a-d).

Using Fab tetramers generated around a single streptavidin-PE molecule, which are able to bind up to four different HLA-Tyr complexes, the present inventors were able to accurately determine by flow cytometry analysis the minimal number of tyrosinase complexes presented on the cell surface (Figures 7a-e). Thus, the percent of HLA-A2 peptide complexes presenting tyrosinase peptides was estimated to be as high as 20 %. The discovery that the tyrosinase epitope is so densely presented on melanoma cells promotes this epitope as an ideal target both for antibody-mediated drug delivery and for antibody-mediated diagnosis of melanoma.

The present inventors have unexpectedly found that presentation of melanoma T cell epitopes does not correlate with their gene expression profile. Accordingly HLA-A2-peptide complexes derived from Tyrosinase were found to be expressed and presented on the surface of melanoma cells at unexpectedly high numbers as compared to Gp100 or Mart-1 epitopes, as analyzed by flow cytometry (Figures 8a-c) even though the relative expression level of Tyrosinase was lower than that of Gp100 and Mart-1 (see Tables 3 and 4 herein below and Figures 11a-c). This discovery leads to the conclusion that analysis of the presentation of melanoma epitopes is a better gauge for determining if a patient is a good candidate for epitope-mediated therapy as opposed to analysis of expression of melanoma antigens.

Without being bound to theory, the present inventors suggest that the instability of the tyrosinase protein may be the cause of this unexpectedly high presentation. Furthermore, the present inventors suggest that the high presentation of HLA-A2-Tyr complexes may explain the relatively low immune response against the Tyrosinase 369-377 epitope since it is known that continual exposure of T cells to antigen maintains an unresponsive state and result in adaptive tolerance (i.e., anergy, B. Rocha, et al., 1995; L. S. Taams, et al., 1999; R. H. Schwartz, 2003; F. Ramsdell and B. J. Fowlkes, 1992). Thus, without being bound to theory the present inventors propose that anergized T cells may be wholly or partly due to the high presentation on melanoma cells.

Whilst further reducing the invention to practice, the present inventors have generated TCR-like antibody-toxin fusion proteins and have shown that such fusion molecules can undergo internalization into melanoma cells (Figures 36a-f and 46a-d) and kill them (Figures 37a-b, 38a-c and 47a-d). Moreover, the present inventors have shown that the generated antibody-toxin fusion proteins were capable of inducing regression of melanoma in vivo (Figures 39a-b) in irradiated mice implanted with human melanoma cells. These experiments provide a proof of principle that specific MHC complexes can be used in humans as target therapy for melanoma.

Thus, according to one aspect of the present invention, there is provided a method of detecting a melanoma cell. The method comprises contacting the cell with an antibody comprising an antigen recognition domain capable of binding to an MHC-1 molecule being complexed with a tyrosinase peptide, wherein the antibody does not bind the MHC-1 in an absence of the complexed peptide, and wherein the antibody does not bind the peptide in an absence of the MHC. The contacting is effected under conditions which allow immunocomplex formation, wherein a presence of the immunocomplex or level thereof is indicative of the melanoma cell.

The term "detecting", as used herein, refers to the act of detecting, perceiving; uncovering, exposing, visualizing or identifying a cell. The precise method of detecting is dependent on the detectable moiety to which the antibody is attached as further described herein below.

As used herein, the term "melanoma cell" refers to a malignant melanocyte of mammalian origin, preferably human. Typically, the melanoma cell comprises tumor associated antigens on its cell surface such as tyrosinase peptides, Mart-1 peptides and/or Gp100 peptides associated with MHC molecules.

Single cells may be used in accordance with the teachings of the present invention as well as a plurality of cells. The cells may be from any biological sample such as melanoma cell-lines, primary melanoma cultures and cellular samples, e.g. skin cell biopsies (surgical biopsies including incisional or excisional biopsy, fine needle aspirates and the like). Methods of biopsy retrieval are well known in the art.

It will be appreciated that if the skin cells are taken from a subject (i.e. biopsies), the method of the present invention may also be used to diagnose melanoma in a subject. The melanoma may be at any stage e.g. IA, IB, IIA, IIB, IIC, IIIA, IIIB, IIIC and IV.

As used herein the term "diagnosing" refers to classifying a melanoma, determining a severity of melanoma (grade or stage), monitoring melanoma progression, forecasting an outcome of the melanoma and/or prospects of recovery.

The subject may be a healthy subject (e.g., human) undergoing a routine well-being check up. Alternatively, the subject may be at risk of having melanoma (e.g., a genetically predisposed subject, a subject with medical and/or family history of cancer, a subject who has been exposed to carcinogens, occupational hazard, environmental hazard] and/or a subject who exhibits suspicious clinical signs of melanoma [e.g., a change in the appearance of a mole).

As mentioned herein above, the method comprises contacting the melanoma cell with an antibody which is able to specifically bind a tyrosinase derived peptide restricted to an MHC-1 complex.

As used herein, the phrase "major histocompatibility complex (MHC)" refers to a complex of antigens encoded by a group of linked loci, which are collectively termed H-2 in the mouse and HLA in humans. The two principal classes of the MHC antigens, class I and class II, each comprise a set of cell surface glycoproteins which play a role in determining tissue type and transplant compatibility. In transplantation reactions, cytotoxic T-cells (CTLs) respond mainly against foreign class I glycoproteins, while helper T-cells respond mainly against foreign class II glycoproteins.

Major histocompatibility complex (MHC) class I molecules are expressed on the surface of nearly all cells. These molecules function in presenting peptides which are mainly derived from endogenously synthesized proteins to CD8+ T cells via an interaction with the αβ T-cell receptor. The class I MHC molecule is a heterodimer composed of a 46-kDa heavy chain which is non-covalently associated with the 12-kDa light chain β-2 microglobulin. In humans, there are several MHC haplotypes, such as, for example, HLA-A2, HLA-A1, HLA-A3, HLA-A24, HLA-A28, HLA-A31, HLA-A33, HLA-A34, HLA-B7, HLA-B45 and HLA-Cw8, their sequences can be found at the kabbat data base, at htexttransferprotocol://immuno.bme.nwu.edu. Further information concerning MHC haaplotypes can be found in Paul, B. Fundamental Immunology Lippincott-Rven Press.

Tyrosinase peptides that bind to class I MHC molecules (also referred to herein interchangeably as HLA-restrieted tyrosinase epitopes, HLA-restricted tyrosinase epitopes and MHC-restricted tyrosinase antigens) are derived from the tyrosinase enzyme (Genebank Accession No: AH003020) and are typically 8-10 amino acids long, bind to the heavy chain α1- α2 groove via two or three anchor residues that interact with corresponding binding pockets in the MHC molecule.

Tyrosinase is a membrane- associated N-linked glycoprotein and it is the key enzyme in melanin synthesis. It is expressed in all healthy melanocytes and in nearly all melanoma tumor samples (H. Takeuchi, *et al.,* 2003; S. Reinke, *et al.,* 2005). Peptides derived from this enzyme are presented on MHC class I molecules and are recognized by autologuos cytolytic T lymphocytes in melanoma patients (T. Wolfel, *et al.,* 1994; Brichard, *et al.,* 1993).

According to one embodiment, the antibody of the present invention specifically recognizes an MHC class I complexed-tyrosinase peptide as set forth in SEQ ID NO: 1, although it should be appreciated that the present invention envisages antibodies recognizing other MHC class I complexed-tyrosinase peptides. Such tyrosinase peptides known to be restricted on MHC class I complexes and expressed on melanoma cells are described by Renkkvist et al, Cancer immunology immunotherapy 2001 50:3-15 and Novellino L, et al., March 2004 update. Cancer Immunol Immunother. 54:187-207, 2005. Additional tumor tyrosinase HLA-restricted peptides derived from tumor associated antigens (TAA) can be found at the website of the Istituto Nazionale per lo Studio e la Cura dei Tumori at hypertexttransferprotocol://worldwideweb.istitutotumori.mi.it.

As used herein the term "peptide" refers to native peptides (either proteolysis products or synthetically synthesized peptides) and further to peptidomimetics, such as peptoids and semipeptoids which are peptide analogs, which may have, for example, modifications rendering the peptides more stable while in a body, or more immunogenic. Such modifications include, but are not limited to, cyclization, N terminus modification, C terminus modification, peptide bond modification, including, but not limited to, CH₂-NH, CH₂-S, CH₂-S=O, O=C-NH, CH₂-O, CH₂-CH₂, S=C-NH, CH=CH or CF=CH, backbone modification and residue modification. Methods for preparing peptidomimetic compounds are well known in the art and are specified in Quantitative Drug Design, C.A. Ramsden Gd., Chapter 17.2, F. Choplin Pergamon Press (1992), which is incorporated by reference as if fully set forth herein. Further details in this respect are provided hereinunder.

As used herein in the specification and in the claims section below the term "amino acid" is understood to include the 20 naturally occurring amino acids; those amino acids often modified post-translationally *in vivo,* including for example hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids. Further elaboration of the possible amino acids usable according to the present invention and examples of non-natural amino acids useful in MHC-1 HLA-A2 recognizable peptide antigens are given herein under.

Based on accumulated experimental data, it is nowadays possible to predict which of the peptides of a protein will bind to MHC, class I. The HLA-A2 MHC class I has been so far characterized better than other HLA haplotypes, yet predictive and/or sporadic data is available for all other haplotypes.

With respect to HLA-A2 binding peptides, assume the following positions (P1-P9) in a 9-mer peptide:
P1-P2-P3-P4-P5-P6-P7-P8-P9

The P2 and P2 positions include the anchor residues which are the main residues participating in binding to MHC molecules. Amino acid resides engaging positions P2 and P9 are hydrophilic aliphatic non-charged natural amino (examples being Ala, Val, Leu, Ile, Gln, Thr, Ser, Cys, preferably Val and Leu) or of a non-natural hydrophilic aliphatic non-charged amino acid (examples being norleucine (Nle), norvaline (Nva), α-aminobutyric acid). Positions P1 and P3 are also known to include amino acid residues which participate or assist in binding to MHC molecules, however, these positions can include any amino acids, natural or non-natural. The other positions are engaged by amino acid residues which typically do not participate in binding, rather these amino acids are presented to the immune cells. Further details relating to the binding of peptides to MHC molecules can be found in Parker, K.C., Bednarek, M.A., Coligan, J.E., Scheme for ranking potential HLA-A2 binding peptides based on independent binding of individual peptide side-chains. J Immunol.152,163-175,1994., see Table V, in particular. Hence, scoring of HLA-A2.1 binding peptides can be performed using the HLA Peptide Binding Predictions software approachable through a worldwide web interface at hypertexttransferprotocol://worldwideweb.bimas.dcrt.nih.gov/molbio/hla_bind/index. This software is based on accumulated data and scores every possible peptide in an analyzed protein for possible binding to MHC HLA-A2.1 according to the contribution of every amino acid in the peptide. Theoretical binding scores represent calculated half-life of the HLA-A2.1-peptide complex.

Hydrophilic aliphatic natural amino acids at P2 and P9 can be substituted by synthetic amino acids, preferably Nleu, Nval and/or α-aminobutyric acid. P9 can be also substituted by aliphatic amino acids of the general formula -HN(CH₂)ₙCOOH, wherein n = 3-5, as well as by branched derivatives thereof, such as, but not limited to, 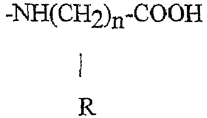 wherein R is, for example, methyl, ethyl or propyl, located at any one or more of the n carbons.

The amino terminal residue (position P1) can be substituted by positively charged aliphatic carboxylic acids, such as, but not limited to, H₂N(CH₂)ₙCOOH, wherein n = 2-4 and H₂N-C(NH)-NH(CH₂)ₙCOOH, wherein n = 2-3, as well as by hydroxy Lysine, N-methyl Lysine or ornithine (Orn). Additionally, the amino terminal residue can be substituted by enlarged aromatic residues, such as, but not limited to, H₂N-(C₆H₆)-CH₂-COOH, p-aminophenyl alanine, H₂N-F(NH)-NH-(C₆H₆)-CH₂-COOH, p-guanidinophenyl alanine or pyridinoalanine (Pal). These latter residues may form hydrogen bonding with the OH⁻ moieties of the Tyrosine residues at the MHC-1 N-terminal binding pocket, as well as to create, at the same time aromatic-aromatic interactions.

Derivatization of amino acid residues at positions P4-P8, should these residues have a side-chain, such as, OH, SH or NH₂, like Ser, Tyr, Lys, Cys or Orn, can be by alkyl, aryl, alkanoyl or aroyl, In addition, OH groups at these positions may also be derivatized by phosphorylation and/or glycosylation. These derivatizations have been shown in some cases to enhance the binding to the T cell receptor.

Longer derivatives in which the second anchor amino acid is at position P10 may include at P9 most L amino acids. In some cases shorter derivatives are also applicable, in which the C terminal acid serves as the second anchor residue.

Cyclic amino acid derivatives can engage position P4-P8, preferably positions P6 and P7. Cyclization can be obtained through amide bond formation, e.g., by incorporating Glu, Asp, Lys, Orn, di-amino butyric (Dab) acid, di-aminopropionic (Dap) acid at various positions in the chain (-CO-NH or -NH-CO bonds). Backbone to backbone cyclization can also be obtained through incorporation of modified amino acids of the formulas H-N((CH₂)ₙ-COOH)-C(R)H-COOH or H-N((CH₂)ₙ-COOH)-C(R)H-NH₂, wherein n = 1-4, and further wherein R is any natural or non-natural side chain of an amino acid.

Cyclization via formation of S-S bonds through incorporation of two Cys residues is also possible. Additional side-chain to side chain cyclization can be obtained via formation of an interaction bond of the formula -(-CH₂-)ₙ-S-CH₂-C-, wherein n = 1 or 2, which is possible, for example, through incorporation of Cys or homoCys and reaction of its free SH group with, e.g., bromoacetylated Lys, Orn, Dab or Dap.

Peptide bonds (-CO-NH-) within the peptide may be substituted by N-methylated bonds (-N(CH₃)-CO-), ester bonds (-C(R)H-C-O-O-C(R)-N-), ketomethylen bonds (-CO-CH₂-), α-aza bonds (-NH-N(R)-CO-), wherein R is any alkyl, e.g., methyl, carba bonds (-CH₂-NH-), hydroxyethylene bonds (-CH(OH)-CH₂-), thioamide bonds (-CS-NH-), olefinic double bonds (-CH=CH-), retro amide bonds (-NH-CO-), peptide derivatives (-N(R)-CH₂-CO-), wherein R is the "normal" side chain, naturally presented on the carbon atom.

These modifications can occur at any of the bonds along the peptide chain and even at several (2-3) at the same time. Preferably, but not in all cases necessary, these modifications should exclude anchor amino acids.

Natural aromatic amino acids, Trp, Tyr and Phe, may be substituted for synthetic non-natural acid such as TIC, naphthylelanine (Nol), ring-methylated derivatives of Phe, halogenated derivatives of Phe or o-methyl-Tyr.

The term "antibody" as used herein includes intact molecules as well as functional fragments thereof, such as Fab, F(ab')₂, Fv and scFv that are capable of specific binding to a human major histocompatibility complex (MHC) class I-restricted tyrosinase epitope. These functional antibody fragments are defined as follows: (i) Fab, the fragment which contains a monovalent antigen-binding fragment of an antibody molecule, can be produced by digestion of whole antibody with the enzyme papain to yield an intact light chain and a portion of one heavy chain; (ii) Fab', the fragment of an antibody molecule that can be obtained by treating whole antibody with pepsin, followed by reduction, to yield an intact light chain and a portion of the heavy chain; two Fab' fragments are obtained per antibody molecule; (iii) F(ab')₂, the fragment of the antibody that can be obtained by treating whole antibody with the enzyme pepsin without subsequent reduction; F(ab')₂ is a dimer of two Fab' fragments held together by two disulfide bonds; (iv) Fv, defined as a genetically engineered fragment containing the variable region of the light chain and the variable region of the heavy chain expressed as two chains; and (c) scFv or "single chain antibody" ("SCA"), a genetically engineered molecule containing the variable region of the light chain and the variable region of the heavy chain, linked by a suitable polypeptide linker as a genetically fused single chain molecule.

Methods of making these fragments are known in the art. (See for example, Harlow and Lane, Antibodies: A Laboratory Manual, Cold Spring Harbor Laboratory, New York, 1988, incorporated herein by reference) and are further described herein below.

An exemplary method for generating antibodies capable of specifically binding a tyrosinase peptide restricted to an MHC-I complex is described in the Examples section herein below.

Specifically, such antibodies may be generated by (i) immunizing a genetically engineered non-human mammal having cells expressing the human major histocompatibility complex (MHC) class I, with a soluble form of a MHC class I molecule being complexed with the HLA-restricted epitope; (ii) isolating mRNA molecules from antibody producing cells, such as splenocytes, of the non-human mammal; (iii) producing a phage display library displaying protein molecules encoded by the mRNA molecules; and (iv) isolating at least one phage from the phage display library, the at least one phage displaying the antibody specifically bindable (preferably with an affinity below 10 nanomolar) to the human major histocompatibility complex (MHC) class I being complexed with the HLA-restricted epitope. The genetic material of the phage isolate is then used to prepare a single chain antibody or other forms of antibodies as is further described herein below. The genetic material of the phage isolate is then used to prepare a single chain antibody or other forms of antibodies as is further described herein below and which are conjugated to identifiable or therapeutic moieties. Preferably, the non-human mammal is devoid of self MHC class I molecules. Still preferably, the soluble form of a MHC class I molecule is a single chain MHC class I polypeptide including a functional human β-2 microglobulin amino acid sequence directly or indirectly covalently linked to a functional human MHC class I heavy chain amino acid sequence.

Recombinant MHC class I and class II complexes which are soluble and which can be produced in large quantities are described in, for example, references 24, 55 and 56-68 and further in U.S. Patent Application No. 09/534,966 and PCT/IL01/00260 (published as WO 01/72768), all of which are incorporated herein by reference. Soluble MHC class I molecules are available or can be produced for any of the MHC haplotypes, such as, for example, HLA-A2, HLA-A1, HLA-A3, HLA-A24, HLA-A28, HLA-A31, HLA-A33, BLA-A34, HLA-B7, HLA-B45 and HLA-Cw8, following, for example the teachings of PCT/IL01/00260, as their sequences are known and can be found at the kabbat data base, at http://imuno.bme.nwu.edu/, the contents of the site is incorporated herein by reference. Such soluble MHC class I molecules can be loaded with suitable HLA-restricted epitopes and used for vaccination of non-human mammal having cells expressing the human major histocompatibility complex (MHC) class I as is further detailed hereinbelow.

Non-human mammal having cells expressing a human major histocompatibility complex (MHC) class I and devoid of self major histocompatibility complex (MHC) class I can be produced using (i) the sequence information provided in the kabbat data base, at http://immuno.bme.nwu.edu/, which is incorporated herein by reference and pertaining to human MHC haplotypes, such as, for example, HLA-A2, HLA-A1, HLA-A3, HLA-A24, HLA-A28, HLA-A31, HLA-A33, HLA-A34, HLA-B7, HLA-B45 and HLA-Cw8, (ii) conventional constructs preparation techniques, as described in, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); and (iii) conventional gene knock-in/knock-out techniques as set forth, for example, in United States Patents 5,487,992, 5,464,764, 5,387,742, 5,360,735, 5,347,075, 5,298,422, 5,288,846, 5,221,778, 5,175,385, 5,175,384, 5,175,383, 4,736,866; in International Publications WO 94/23049, WO93/14200 WO 94/06908 and WO 94/28123; as well as in Burke and Olson, Methods in Enzymology, 194:251-270, 1991; Capecchi, Science 244:1288-1292, 1989; Davies et al., Nucleic Acids Research, 20 (11) 2693-2698, 1992; Dickinson et al., Human Molecular Genetics, 2(8): 1299-1302, 1993; Duff and Lincoln, "Insertion of a pathogenic mutation into a yeast artificial chromosome containing the human APP gene and expression in ES cells", Research Advances in Alzheimer's Disease and Related Disorders, 1995; Huxley et al., Genomics, 9:742-750 1991; Jakobovits et al., Nature, 362:255-261, 1993; Lamb et al., Nature Genetics, 5: 22-29, 1993; Pearson and Choi, Proc. Natl. Acad. Sci. USA, 1993. 90:10578-82; Rothstein, Methods in Enzymology, 194:281-301, 1991; Schedl et al., Nature, 362: 258-261, 1993; Strauss et al., Science, 259:1.904-1907, 1993, all of which are incorporated herein by reference.

Of particular interest is the paper by Pascolo et al., published in J. Exp. Med. 185: 2043-2051, 1997, which describe the preparation of mice expressing the human HLA-A2.1, H-2Db and HHD MHC class I molecules and devoid of mice MHC class I altogether.

An exemplary antibody capable of binding to an MHC class I complexed with a tyrosinase epitope comprises complementarity determining region (CDR) amino acid sequences as set forth in SEQ ID NOs: 59-64.

According to one embodiment of this aspect of the present invention, the antibody binds to the MHC class I /tyrosine epitope complex with a dissociation constant less than 100 nM. According to another embodiment the dissociation constanst is less that 10 nM.

Such antibodies have been described in Example 12 of the Examples section herein below. Thus, according to this embodiment, the antibody may comprise complementarity determining region (CDR) amino acid sequences as set forth in SEQ ID NOs: 886-891 or complementarity determining region (CDR) amino acid sequences as set forth in SEQ ID NOs: 894-899.

It will be appreciated that although the present inventors have discovered that tyrosinase epitopes are the optimal target for melanoma detection due to their very high presentation on melanoma cells, the present invention also contemplates the use of antibodies capable of recognizing other class I MHC-peptide complexes such as class I MHC-Mart-1 complexes.

Thus, according to another aspect of the present invention, there is provided an antibody comprising an antigen recognition domain which comprises complementarity determining region (CDR) amino acid sequences as set forth in SEQ ID NOs: 47-52.

According to another aspect of the present invention, there is provided an antibody comprising an antigen recognition domain which comprises complementarity determining region (CDR) amino acid sequences as set forth in SEQ ID NOs: 53-58.

According to one embodiment the antibodies of the present invention are IgG1 antibodies. Exemplary methods for generating IgG1 antibodies are described in the general materials and experimental methods of the Examples section herein below.

As mentioned herein above, the antibodies of the present invention may be antibody fragments. Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment.

Antibody fragments can be obtained by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R., Biochern. J., 73: 119-126, 1959. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of V_{H} and V_{L} chains. This association may be noncovalent, as described in Inbar et al., Proc. Nat'l Acad. Sci. USA 69:2659-62, 1972. Alternatively, the variable chains can be linked by an intermolecular disulfide bond or cross-linked by chemicals such as glutaraldehyde. Preferably, the Fv fragments comprise V_{H} and V_{L} chains connected by a peptide linker. These single-chain antigen binding proteins (sFv) are prepared by constructing a structural gene comprising DNA sequences encoding the V_{H} and V_{L} domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by Whitlow and Filpula, Methods, 2: 97-105, 1991; Bird et al., Science 242:423-426, 1988; Pack et al., Bio/Technology 11:1271-77, 1993; and Ladner et al., U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry, Methods, 2: 106-10,1991.

Humanized forms of non-human (e.g., murine) antibodies are chimeric molecules of immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence.

The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and coworkers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

It will be appreciated that once the CDRs of an antibody are identified, using conventional genetic engineering techniques, expressible polynucleotides encoding any of the forms or fragments of antibodies described herein can be devised and modified in one of many ways in order to produce a spectrum of related-products as further described herein below.

Thus, for example polynucleotides as set forth in SEQ ID NOs: 16 and 18 may be used in nucleic acid constructs for the expression of the TA2 antibody. Polynucleotides as set forth in SEQ ID NOs: 903 -906 may be used in nucleic acid constructs for the expression of the MC1 antibody. Polynucleotides as set forth in SEQ ID NOs: 907-910 may be used in nucleic acid constructs for the expression of the B2 antibody.

Thus, polynucleotide sequences such as those described herein above are inserted into expression vectors (i.e., a nucleic acid construct) to enable expression of the recombinant antibody. The expression vector of the present invention includes additional sequences which render this vector suitable for replication and integration in prokaryotes, eukaryotes, or preferably both (e.g., shuttle vectors). Typical cloning vectors contain transcription and translation initiation sequences (e.g., promoters, enhances) and transcription and translation terminators (e.g., polyadenylation signals).

A variety of prokaryotic or eukaryotic cells can be used as host-expression systems to express the antibodies of the present invention. These include, but are not limited to, microorganism, such as bacteria transformed with a recombinant bacteriophage DNA, plasmid DNA or cosmid DNA expression vector containing the polypeptide coding sequence; yeast transformed with recombinant yeast expression vectors containing the polypeptide coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the polypeptide coding sequence.

In bacterial systems, a number of expression vectors can be advantageously selected depending upon the use intended for the antibody expressed. For example, when large quantities of antiobody are desired, vectors that direct the expression of high levels of the protein product, possibly as a fusion with a hydrophobic signal sequence, which directs the expressed product into the periplasm of the bacteria or the culture medium where the protein product is readily purified may be desired. Certain fusion protein engineered with a specific cleavage site to aid in recovery of the polypeptide may also be desirable. Such vectors adaptable to such manipulation include, but are not limited to, the pET series of *E*. coli expression vectors [Studier et al., Methods in Enzymol. 185:60-89 (1990)].

In yeast, a number of vectors containing constitutive or inducible promoters can be used, as disclosed in U.S. Pat. Application No: 5,932,447. Alternatively, vectors can be used which promote integration of foreign DNA sequences into the yeast chromosome.

In cases where plant expression vectors are used, the expression of the antibody coding sequence can be driven by a number of promoters. For example, viral promoters such as the 35S RNA and 19S RNA promoters of CaMV [Brisson et al., Nature 310:511-514 (1984)], or the coat protein promoter to TMV [Takamatsu et al., EMBO J. 6:307-311 (1987)] can be used. Alternatively, plant promoters can be used such as, for example, the small subunit of RUBISCO [Coruzzi et al., EMBO J. 3:1671-1680 (1984); and Brogli et al., Science 224:838-843 (1984)] or heat shock promoters, e.g., soybean hsp17.5-E or hsp17.3-B [Gurley et al., Mol. Cell. Biol. 6:559-565 (1986)]. These constructs can be introduced into plant cells using Ti plasmid, Ri plasmid, plant viral vectors, direct DNA transformation, microinjection, electroporation and other techniques well known to the skilled artisan. See, for example, Weissbach & Weissbach [Methods for Plant Molecular Biology, Academic Press, NY, Section VIII, pp 421-463 (1988)]. Other expression systems such as insects and mammalian host cell systems, which are well known in the art, can also be used by the present invention.

It will be appreciated that other than containing the necessary elements for the transcription and translation of the inserted coding sequence (encoding the polypeptide), the expression construct of the present invention can also include sequences engineered to optimize stability, production, purification, yield or activity of the expressed antibody.

Various methods can be used to introduce the expression vector of the present invention into the host cell system. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6):504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Transformed cells are cultured under effective conditions, which allow for the expression of high amounts of recombinant antiobody. Effective culture conditions include, but are not limited to, effective media, bioreactor, temperature, pH and oxygen conditions that permit protein production. An effective medium refers to any medium in which a cell is cultured to produce the recombinant polypeptide of the present invention. Such a medium typically includes an aqueous solution having assimilable carbon, nitrogen and phosphate sources, and appropriate salts, minerals, metals and other nutrients, such as vitamins. Cells of the present invention can be cultured in conventional fermentation bioreactors, shake flasks, test tubes, microtiter dishes and petri plates. Culturing can be carried out at a temperature, pH and oxygen content appropriate for a recombinant cell. Such culturing conditions are within the expertise of one of ordinary skill in the art.

Depending on the vector and host system used for production, resultant polypeptides of the present invention may either remain within the recombinant cell, secreted into the fermentation medium, secreted into a space between two cellular membranes, such as the periplasmic space in E. *coli;* or retained on the outer surface of a cell or viral membrane.

Following a predetermined time in culture, recovery of the recombinant antibody is effected.

The phrase "recovering the recombinant antibody" used herein refers to collecting the whole fermentation medium containing the antiobdy and need not imply additional steps of separation or purification.

Thus, antiobdies of the present invention can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization.

To facilitate recovery, the expressed coding sequence can be engineered to encode the antibody of the present invention and fused cleavable moiety. Such a fusion protein can be designed so that the antiobody can be readily isolated by affinity chromatography; e.g., by immobilization on a column specific for the cleavable moiety. Where a cleavage site is engineered between the antibody and the cleavable moiety, the antiobody can be released from the chromatographic column by treatment with an appropriate enzyme or agent that specifically cleaves the fusion protein at this site [e.g., see Booth et al., Immunol. Lett. 19:65-70 (1988); and Gardella et al., J. Biol. Chem. 265:15854-15859 (1990)].

As mentioned, the antibody of the present invention is contacted with potential melanoma cells under conditions which allow immunocomplex formation, wherein a presence of the immunocomplex or level thereof is indicative of the melanoma cell.

The contacting may be effected in vitro (i.e. in a cell line), ex vivo or in vivo.

As mentioned, the method of the present invention is effected under conditions sufficient to form an immunocomplex (e.g. a complex between the antibodies of the present invention and the tyrosinase derived peptide complexed to an MHC-I); such conditions (e.g., appropriate concentrations, buffers, temperatures, reaction times) as well as methods to optimize such conditions are known to those skilled in the art, and examples are disclosed herein.

Determining a presence or level of the immunocomplex of the present invention is dependent on the detectable moiety to which the antibody is attached.

Examples of detectable moieties that can be used in the present invention include but are not limited to radioactive isotopes, phosphorescent chemicals, chemiluminescent chemicals, fluorescent chemicals, enzymes, fluorescent polypeptides and epitope tags. The detectable moiety can be a member of a binding pair, which is identifiable via its interaction with an additional member of the binding pair, and a label which is directly visualized. In one example, the member of the binding pair is an antigen which is identified by a corresponding labeled antibody. In one example, the label is a fluorescent protein or an enzyme producing a colorimetric reaction.

Further examples of detectable moieties, include those detectable by Positron Emission Tomagraphy (PET) and Magnetic Resonance Imaging (MRI), all of which are well known to those of skill in the art.

When the detectable moiety is a polypeptide, the immunolabel (i.e. the antibody conjugated to the detectable moiety) may be produced by recombinant means or may be chemically synthesized by, for example, the stepwise addition of one or more amino acid residues in defined order using solid phase peptide synthetic techniques. Examples of polypeptide detectable moieties that can be linked to the antibodies of the present invention using recombinant DNA technology include fluorescent polypeptides, phosphorescent polypeptides, enzymes and epitope tags.

Expression vectors can be designed to fuse proteins encoded by the heterologous nucleic acid insert to fluorescent polypeptides. For example, antibodies can be expressed from an expression vector fused with a green fluorescent protein (GFP)-like polypeptide. A wide variety of vectors are commercially available that fuse proteins encoded by heterologous nucleic acids to the green fluorescent protein from Aequorea victoria ("GFP"), the yellow fluorescent protein and the red fluorescent protein and their variants (e.g., Evrogen). In these systems, the fluorescent polypeptide is entirely encoded by its amino acid sequence and can fluoresce without requirement for cofactor or substrate. Expression vectors that can be employed to fuse proteins encoded by the heterologous nucleic acid insert to epitope tags are commercially available (e.g., BD Biosciences, Clontech).

Alternatively, chemical attachment of a detectable moiety to the antibodies of the present invention can be effected using any suitable chemical linkage, direct or indirect, as via a peptide bond (when the detectable moiety is a polypeptide), or via covalent bonding to an intervening linker element, such as a linker peptide or other chemical moiety, such as an organic polymer. Such chimeric peptides may be linked via bonding at the carboxy (C) or amino (N) termini of the peptides, or via bonding to internal chemical groups such as straight, branched or cyclic side chains, internal carbon or nitrogen atoms, and the like. Such modified peptides can be easily identified and prepared by one of ordinary skill in the art, using well known methods of peptide synthesis and/or covalent linkage of peptides. Description of fluorescent labeling of antibodies is provided in details in U.S. Pat. Nos. 3,940,475, 4,289,747, and 4,376,110.

Exemplary methods for conjugating two peptide moieties are described herein below:

### SPDP conjugation:

Any SPDP conjugation method known to those skilled in the art can be used. For example, in one illustrative embodiment, a modification of the method of Cumber et al. (1985, Methods of Enzymology 112: 207-224) as described below, is used.

A peptide, such as an identifiable or therapeutic moiety, (1.7 mg/ml) is mixed with a 10-fold excess of SPDP (50 mM in ethanol) and the antibody is mixed with a 25-fold excess of SPDP in 20 mM sodium phosphate, 0.10 M NaCl pH 7.2 and each of the reactions incubated, e.g., for 3 hours at room temperature. The reactions are then dialyzed against PBS.

The peptide is reduced, e.g., with 50 mM DTT for 1 hour at room temperature. The reduced peptide is desalted by equilibration on G-25 column (up to 5 % sample/column volume) with 50 mM KH₂PO₄ pH 6.5. The reduced peptide is combined with the SPDP-antibody in a molar ratio of 1:10 antibody:peptide and incubated at 4 °C overnight to form a peptide-antibody conjugate.

### Glutaraldehyde conjugation:

Conjugation of a peptide (e.g., an identifiable or therapeutic moiety) with an antibody can be accomplished by methods known to those skilled in the art using glutaraldehyde. For example, in one illustrative embodiment, the method of conjugation by G.T. Hermanson (1996, "Antibody Modification and Conjugation, in Bioconjugate Techniques, Academic Press, San Diego) described below, is used.

The antibody and the peptide (1.1 mg/ml) are mixed at a 10-fold excess with 0.05 % glutaraldehyde in 0.1 M phosphate, 0.15 M NaCl pH 6.8, and allowed to react for 2 hours at room temperature. 0.01 M lysine can be added to block excess sites. After-the reaction, the excess glutaraldehyde is removed using a G-25 column equilibrated with PBS (10 % v/v sample/column volumes)

### Carbodiimide conjugation:

Conjugation of a peptide with an antibody can be accomplished by methods known to those skilled in the art using a dehydrating agent such as a carbodiimide. Most preferably the carbodiimide is used in the presence of 4-dimethyl aminopyridine. As is well known to those skilled in the art, carbodiimide conjugation can be used to form a covalent bond between a carboxyl group of peptide and an hydroxyl group of an antibody (resulting in the formation of an ester bond), or an amino group of an antibody (resulting in the formation of an amide bond) or a sulfhydryl group of an antibody (resulting in the formation of a thioester bond).

Likewise, carbodiimide coupling can be used to form analogous covalent bonds between a carbon group of an antibody and an hydroxyl, amino or sulfhydryl group of the peptide. See, generally, J. March, Advanced Organic Chemistry: Reaction's, Mechanism, and Structure, pp. 349-50 & 372-74 (3d ed.), 1985. By means of illustration, and not limitation, the peptide is conjugated to an antibody via a covalent bond using a carbodiimide, such as dicyclohexylcarbodiimide. See generally, the methods of conjugation by B. Neises et al. (1978, Angew Chem., Int. Ed. Engl. 17:522; A. Hassner et al. (1978, Tetrahedron Lett. 4475); E.P. Boden et al. (1986, J. Org. Chem. 50:2394) and L.J. Mathias (1979, Synthesis 561).The level of immunocomplex may be compared to a control sample from a non-diseased subject, wherein an up-regulation of immunocomplex formation is indicative of melanoma. Preferably, the subject is of the same species e.g. human, preferably matched with the same age, weight, sex etc. It will be appreciated that the control sample may also be of the same subject from a healthy tissue, prior to disease progression or following disease remission.

It will be appreciated that antibodies of the present invention may also be used to ascertain if a melanoma patient is a suitable candidate for epitope-related therapy via in vivo or ex-vivo testing. As mentioned above, the present inventors found that the expression level of a melanoma antigen (as measured by RT-PCR) did not reflect its presentation numbers on the membrane of a melanoma cell. Thus, antibodies of the present invention may be used as tools to evaluate the level (i.e. density) of a particular melanoma antigen on a cell, wherein a level higher than a predetermined threshold is indicative of an individual being suitable for TCRL epitope- directed therapy and a level lower than a predetermined threshold is indicative of an individual being suitable for CTL epitope-directed therapy. Alternatively, or additionally a level below a predetermined threshold is indicative of an individual not being suitable for TCRL epitope-directed therapy and a level above a predetermined threshold is indicative of an individual not being suitable for CTL epitope-directed therapy.

CTL epitope directed therapy aims to generate tumor-reactive CD8+ T cells in response to administration of tumor-specific peptide epitopes to bring about the destruction of the tumor cells. However, despite much effort, vaccination approaches to date can at best induce objective cancer regressions consistent with standard oncologic criteria in only a small minority of patients with solid cancers. One possible explanation of the lack of success of CTL epitope directed therapy is the phenomenom of anergy which dictates that continual exposure of T cells to antigens maintains an unresponsive state and result in adaptive tolerance (B. Rocha, et al., 1995; L. S. Taams, et al., 1999; R. H. Schwartz, 2003; F. Ramsdell and B. J. Fowlkes, 1992). The present invention seeks to overcome this obstacle by determining the level of a particular epitope by examining their levels using the antibodies of the present invention.

Further information on CTL epitope directed therapy can be obtained in the following review articles - Acres B, et al., Curr Opin Drug Discov Devel. 2007 Mar;10(2):185-92. Finn OJ. Immunol Res. 2006;36(1-3):73-82. Chen W, McCluskey J. Adv Cancer Res. 2006;95:203-47. Klebanoff CA, Gattinoni L, Restifo NP. Immunol Rev. 2006 Jun;211:214-24. Slingluff CL Jr, Engelhard VH, Ferrone S. Clin Cancer Res. 2006 Apr 1;12(7 Pt 2):2342s-2345s. Mocellin S, Lise M, Nitti D. Tumor immunology. Adv Exp Med Biol. 2007;593:147-56. Gattinoni L, Powell DJ Jr, Rosenberg SA, Restifo NP. Nat Rev Immunol. 2006 May;6(5):383-93. Rosenberg SA. N Engl J Med. 2004 Apr 1;350(14):1461-3. Rosenberg SA. Nature. 2001 May 17;411(6835):380-4, each of which is incorporated herein by reference in its entirety.

The predetermined thresholds may be estimated as shown in the Example section herein below. An exemplary threshold above which it may be determined that a patient is suitable for TCRL epitope-directed therapy (or unsuitable for CTL epitope directed therapy) is above about 500, more preferably above about 800, more preferably above about 1000, more preferably above about 1200 and even more preferably above about 1500 MHC-peptide complexes per cell that present a single epitope. An exemplary threshold below which it may be determined that a patient is not suitable for TCRL epitope-directed therapy (or suitable for CTL epitope directed therapy) is below about 400, more preferably below about 300, more preferably below about 200, and even more preferably below about 100 MHC-peptide complexes per cell that present a single epitope.

It will be appreciated that antibodies of the present invention targeted against a variety of peptide antigens including, but not limited to gp100, Mart-1 and Tyrosinase may also be used to identify the optimal candidate target for epitope-directed therapy on an individual level.

Furthermore, the antibodies of the present invention can be used to monitor via in vivo or ex vivo testing, the therapeutic impact of epitope directed therapy to identify whether a tumoral process evolved and became refractory via mechanisms relying on or independent of loss of antigen expression.

It will be appreciated that the antibodies of the present invention may also be used to kill or ablate cells (e.g., a non-cancerous cell, e.g., a normal, benign or hyperplastic cell, or a cancerous cell, e.g., a malignant cell, e.g., a cell found in a solid tumor, a soft tissue tumor, or a metastatic lesion (e.g., a cell found in renal, urothelial, colonic, rectal, pulmonary, breast or hepatic, cancers and/or metastasis). Methods of the invention include the steps of contacting the cell with an anti-(MHC-peptide complex) ligand, e.g., an anti-(MHC-peptide complex) antibody described herein, in an amount sufficient to ablate or kill the cell.

This method can be used on cells in culture, e.g. *in vitro* or *ex vivo.* For example, cancerous or metastatic cells (e.g., renal, urothelial, colon, rectal, lung, breast, ovarian, prostatic, or liver cancerous or metastatic cells) can be cultured *in vitro* in culture medium and the contacting step can be effected by adding the anti-(MHC-peptide complex) ligand to the culture medium. The method can be performed on cells (e.g., cancerous or metastatic cells) present in a subject, as part of an *in vivo* (e.g., therapeutic or prophylactic) protocol as described herein below.

Since the present inventors discovered that tyrosinase peptides are highly presented on melanoma cells compared to other peptide antigens, it may be concluded that tyrosinase peptides may serve as attractive targets for TCRL epitope-directed therapy.

Thus, according to another aspect of the present invention there is provided a method of treating a melanoma. The method comprises administering to a subject in need thereof a therapeutically effective amount of the tyrosinase peptide directed antibodies of the present invention.

According to one embodiment of this aspect of the present invention, the antibody is an IgG1 antibody. Using IgG antibodies, tumor cell lysis can be achieved by antibody-dependent cell-mediated cytotoxicity (ADCC) or complement-dependant cytotoxicity (CDC) mechanisms (J. Golay, *et al.*, 2004; H. Mellstedt, 2003; Modjtahedi, *et al.*, 2003; N. Prang, *et al.,* 2005).

According to another embodiment of this aspect of the present invention, the antibody is conjugated to a therapeutic moiety.

The therapeutic moiety can be, for example, a cytotoxic moiety, a toxic moiety, a cytokine moiety and a second antibody moiety comprising a different specificity to the antibodies of the present invention.

In a similar fashion to an immunolabel, an immunotoxin (i.e. a therapeutic moiety attached to an antibody of the present invention) may be generated by recombinant or non-recombinant means. Thus, the present invention envisages a first and second polynucleotide encoding the antibody of the present invention and the therapeutic moiety, respectively, ligated in frame, so as to encode an immunotoxin. The following table provides examples of sequences of therapeutic moieties.

**Table 1**

| ***Therapeutic Moiety*** | ***Amino Acid sequence** (**Genebank Accession No.**)* | ***Nucleic Acid sequence (Genebank Accession No.)*** |
|---|---|---|
| Pseudomonas exotoxin | AAB25018 | S53109 |
| Diphtheria toxin | E00489 | E00489 |
| interleukin 2 | CAA00227 | A02159 |
| CD3 | P07766 | X03884 |
| CD16 | AAK54251 | AF372455 |
| interleukin 4 | P20096 | ICRT4 |
| HLA-A2 | P01892 | K02883 |
| interleukin 10 | P22301 | M57627 |
| Ricin A toxin | 225988 | A23903 |

Exemplary methods of conjugating the antibodies of the present invention to peptide therapeutic agents are described herein above.

The antibodies of the present invention may be provided per se or may be administered as a pharmaceutical composition.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the antibodies of the present invention accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intravenous, inrtaperitoneal, intranasal, or intraocular injections.

Alternately, one may administer the pharmaceutical composition in a local rather than systemic manner, for example, via injection of the pharmaceutical composition directly into a tissue region of a patient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran, Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients (antibodies) effective to prevent, alleviate or ameliorate symptoms of a disorder (e.g., melanom) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide plasma or brain levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the' disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as if further detailed above.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### GENERAL MATERIALS AND EXPERIMENTAL METHODS FOR

### EXAMPLES 1-9

***Production of biotinylated single-chain MHC*/*peptide complexes -*** Single-chain MHC (scMHC)/peptide complexes were produced by *in vitro* refolding of inclusion bodies produced in *Escherichia coli* upon IPTG induction, as described (Denkberg, *et al.*, 2000). Briefly, a scMHC, which contains the β₂-microglobulin and the extracellular domains of the HLA-A2 gene connected to each other by a flexible linker, was engineered to contain the BirA recognition sequence for site-specific biotinylation at the C terminus (scMHC-BirA). *In vitro* refolding was performed in the presence of peptides, as described (Denkberg, *et al.*, 2000). Correctly folded MHC/peptide complexes were isolated and purified by anion exchange Q-Sepharose chromatography (Pharmacia, Peapack, NJ), followed by site-specific biotinylation using the BirA enzyme (Avidity, Denver, CO).

***Selection of phage Abs on biotinylated complexes -*** Selection of phage Abs on biotinylated complexes was performed, as described [Denkberg G, et al., Proc Natl Acad Sci U S A. 2002 Jul 9;99(14):9421-6. Epub 2002 Jul 1]. Briefly, a large human Fab library containing 3.7 x 10¹⁰ different Fab clones was used for the selection. Phages were first preincubated with streptavidin-coated paramagnetic beads (200 µl; Dynal, Oslo, Norway) to deplete the streptavidin binders. The remaining phages were subsequently used for panning with decreasing amounts of biotinylated scMHC-peptide complexes. The streptavidin-depleted library was incubated in solution with soluble biotinylated scHLA-A2/Tyrosinase complexes (500 nM for the first round, and 100 nM for the following rounds) for 30 minutes at room temperature (RT). Streptavidin-coated magnetic beads (200 µl for the first round of selection, and 100 µl for the following rounds) were added to the mixture and incubated for 10-15 minutes at RT. The beads were washed extensively 12 times with PBS/Tween 0.1 %, and additional two washes were with PBS. Bound phages were eluted with triethylamine (100 mM, 5 minutes at RT), followed by neutralization with Tris-HCl (1 M, pH 7.4), and used to infect *E. coli* TG1 cells (OD = 0.5) for 30 minutes at 37 °C. The diversity of the selected Abs was determined by DNA fingerprinting using a restriction endonuclease (*Bst*NI), which is a frequent cutter of Ab V gene sequences.

***Expression and purification of soluble recombinant Fab Abs -*** Fab Abs were expressed and purified, as described recently [Denkberg G, et al., Proc Natl Acad Sci U S A. 2002 Jul 9,99(14):9421-6. Epub 2002 Jul 1]. TG1 or BL21 cells were grown to OD₆₀₀= 0.8-1.0 and induced to express the recombinant Fab Ab by the addition of IPTG for 3-4 hours at 30 °C. Periplasmic content was released using the B-PER solution (Pierce, Rockford, IL), which was applied onto a prewashed TALON column (Clontech, Palo Alto, CA). Bound Fabs were eluted using 0.5 ml of 100 mM imidazole in PBS. The eluted Fabs were dialyzed twice against PBS (overnight, 4 °C) to remove residual imidazole. Specificity of the produced Fabs was verified by ELISA analysis, as described [Denkberg G, et al., Proc Natl Acad Sci U S A. 2002 Jul 9;99(14):9421-6. Epub 2002 Jul 1].

***Flow cytometry -*** EBV-transformed B-lymphoblast JY cells were washed with serum-free RPMI medium and incubated overnight with medium containing 10 µM Tyrosinase 369-377 peptide [YMDGTMSQV (SEQ ID NO:1) or control peptides: Gag (SEQ ID NO:2), Tyr N (SEQ ID N0:3), 2092M (SEQ ID N0:4), 280 (SEQ ID NO:5), 540 (SEQ ID NO:6), TARP (SEQ ID NO:7), 154 (SEQ ID NO: 20), Tax (SEQ ID NO: 26), 280m (SEQ ID NO: 28), pol (SEQ ID NO: 65) 865 (SEQ ID NO: 29) and Mart (SEQ ID NO: 22)). Cells (10 ⁶) were incubated I hour at 4 °C with 1-2 µg specific antibody, followed by incubation for 45 minutes at 4 °C with PE-Iabeled anti-human antibody. Cells were finally washed and analyzed by a FACStar flow cytometer (BD Biosciences, San Jose, CA). Melanoma cells were examined for endogenous antigen expression by staining with 2-5 µg of the specific antibodies.

***cDNA production and quantitative real-time PCR analysis -*** mRNA was isolated from melanoma cell lines with oligo (dT) magnetic beads using Dynabeads mRNA DIRECT Kit (Dynal) according to manufacture's instructions. mRNA was converted into cDNA using cloned -AMV reverse transcriptase (invitrogen) according to manufacture's instructions. Real time PCR was performed using Assays-on Demand Gene Expression Assays (Applied Biosystems). Assay IDs Hs00165976_ml, Hs00173854_ml, Hs00194133_ml were used for tyrosinase, gp100 and Mart-1 expression assays, respectively. For all real time PCR reactions, total volume of 20 µl contained 15 ng of mRNA converted to cDNA and 10 µl TaqMan Universal PCR Mastermix (Applied Biosystems). Primers and FAM-labeled probes were added to each reaction at the final concentration of 0.9 µM and 0.25 µM, respectively. A "no template" control that contained all the above reagents was also included to detect the presence of contaminating DNA. 5 ng of Glyceraldehyde-3-phosphate dehydrogenase *(GAPDH)* mRNA converted to cDNA was used as an internal control gene for mRNA expression and for analyzing relative expression (Assay ID Hs99999905_ml). Amplification and fluorescence detection was conducted in an ABI/Prism 7700 sequence detector (Applied Biosystems, USA) with a program of 50 °C for 2 minutes, 95 °C for 10 minutes, 40 cycles of 95 °C for 15 seconds and 60 °C for one minute.

The amount of target genes was determined from the comparative C_{T} method. The target genes were normalized to GAPDH and were expressed as ΔC_{T} (C_{T}- threshold cycle of target gene minus C_{T} of GAPDH).

***Production of Fab tetramers -*** The light and heavy chains of the Fab were PCR amplified and cloned separately into pRB vector. The C terminus of the, Fab light chain was fused to the BirA tag for site specific biotinylation. Each of the vectors was transformed into *E. coli* BL21 cells and expressed upon IPTG induction as inclusion bodies. The inclusion bodies, containing light or heavy chains of the Fab' were isolated, solubilized, refolded with each other and purified by ion exchange chromatography. The recombinant Fab was biotinylated and tetramers were generated by adding fluorescently-labeled streptavidin (Reinke, et al., 2005).

***Construction of whole IgG antibody -*** The heavy and light Fab genes were cloned for expression as human IgG1 kappa antibody into mammalian backbone of the eukaryotic expression vector pCMV/myc/ER. For the heavy chain, the multiple cloning site, the myc epitope tag, and the ER retention signal of pCMV/myc/ER were replaced by a cloning site containing recognition sites for *Bss*HI and *Nhe*I followed by the human IgG1 constant heavy chain region cDNA isolated by RT-PCR from human lymphocyte total RNA. A similar construct was generated for the light chain. Each shuttle expression vector carries a different antibiotics resistance gene. Expression was facilitated by co-transfection of the two constructs into human embryonic kidney HEK293 cell, by using FuGENE 6 Transfection Reagent (Roche). After co-transfection, cells were grown on selective media. Antibody-producing cells were adapted to growth in 0.5 % serum followed by purification using proteinA affinity chromatography.

***Immunofluorescence:*** Cells were fixed for 10 minutes at RT with 0.1 % formaldehyde, rinsed with 0.1 % BSA-PBS, incubated for 1 hour at 4 °C with primary antibody, followed by incubation with the fluorescence-labeled secondary antibody goat anti-human Alexa Fluor 488 or goat anti-mouse Alexa Fluor 594 (Molecular Probes). For staining of nuclear DNA DRAQ5 (Alexis biochemicals) was used. A BioRad MRC1024 confocal microscope was employed for analysis.

***Determination of protein stability:*** 501A melanoma cells were incubated with Cyclohexamide (100 µg/ml, Sigma) to inhibit protein synthesis. The cells were lysed after 0, 2, 4 and 6 hours in PBS containing 10 % NP-40, 1 % DOC, 2 mM EDTA, 10 mM Tris pH = 7, 1 mM PMSF and protease inhibitor cocktail. The lysate was passed through needle and was incubated 10 minutes on ice. Cells were centrifuged at 14 000 r.p.m. for 5 minutes at 4 °C and the supernatant was collected. Equal amounts of sample were loaded on SDS-PAGE and electroblotted onto nitrocellulose. The blots were probed with T311 mouse anti-Tyrosinase, HMB-45 mouse anti-gp100 or A103 mouse anti-Mart-1 followed by incubation with a secondary horseradish peroxidase-conjugated antibody and detection by chemiluminescence. For negative control, Panc-1 cells, which do not express tyrosinase, gp100 and mart-1, were used. The resulting bands were quantified with multi gauge v2.2 software. The degradation rate is expressed as half-life (*t*_{1/2}), the time for degradation of 50 % of the sample. The degradation rate of each protein was evaluated by three to eight independent determinations of *t*_{1/2}. The data are expressed as mean ± SD.

***DNA microarray*:** The gene expression profile of JKF6 memory T cell clone as a function of time post exposure to various antigen densities on target cells was analyzed using Affymetrix human gene DNA array chip. Each Time-Antigen spot was composed of 3 independent biological assays. CTLs were incubated with pulsed JY APCs loaded with a peptide concentration that corresponds to antigen density of 10,100 and 800 sites per cell. CTLs were incubated for 4,16 or 36 hours with peptide-pulsed JY target cells which subsequently after incubation were depleted using CD19 easySep^{©} (Stem cell®) magnetic beads. JY Depleted cultures contained 97.3% or more CD8+ T cells with no detectable contamination of CD 19 positive JY target cells.

All experiments were performed using Affymetrix Hu133A 2.0 oligonucleotide arrays, as described at (url1). Total RNA from each sample was used to prepare biotinylated target RNA (ur12). Briefly, 5 µg of mRNA pooled from 3 independent biological assays was used to generate first-strand cDNA by using a T7-linked oligo(dT) primer. After second-strand synthesis, in vitro transcription was performed with biotinylated UTP and CTP (Affymetrix), resulting in approximately 300-fold amplification of mRNA. The target cDNA generated from each sample was processed as per manufacturer's recommendation using an Affymetrix GeneChip Instrument System (url2). Briefly, spike controls were added to 15 µg fragmented cRNA before overnight hybridisation. Arrays were then washed and stained with streptavidin-phycoerythrin, before being scanned on an Affymetrix GeneChip scanner. A complete description of these procedures is available at (url2). Additionally, quality and amount of starting RNA was confirmed using an agarose gel. After scanning, array images were assessed by eye to confirm scanner alignment and the absence of significant bubbles or scratches on the chip surface. 3'/5' ratios for GAPDH and beta-actin were confirmed to be within acceptable limits (0.97-0.96 and 1.14-1.2), and BioB spike controls were found to be present on all chips, with BioC, BioD and CreX also present in increasing intensity. When scaled to a target intensity of 150 (using Affymetrix MAS 5.0 array analysis software), scaling factors for all arrays were within acceptable limits (1.355-1.655), as were background, Q values and mean intensities. Details of quality control measures can be found at www.ncbi.nlm.nih.gov/geo/ or at http://eng.sheba.co.il/genomics.

***Data analysis:*** The probe sets contained in the Affymetrix Human Hu133A2 oligonucleotide array or oligonucleotide arrays were filtered using Mas 5 algorithm. Treated and control samples were compared. The comparison generated a list of "active genes" representing probe sets changed by at least 2 fold (as calculated from the MAS 5 Log Ratio values)(LR>=1 or LR<=-1) and detected as "Increased" or as "Decrease" (I or D, *p*-value 0.0025) or Marginal Increased" or as Marginal Decrease (MI or MD, *p*-value 0.003) in all treated sample as compared to the control samples (CTLs at time 0) in at least one time point. This list excluded up- regulated genes in all treated samples with signals lower than 20 or detected as absent, and down- regulated gene with base line signals lower than 20 and detected as absent in the control samples. For further filtering we used the probe sets changed by at least 2 fold (between signals) between the 2 treated samples at 36 hrs: 700 sites per target -36hrs (P22) and 100 sites per target -36hrs (P21). Hierarchical clustering was performed using Spotfire DecisionSite for Functional Genomics (Somerville,MA).

Genes were classified into functional groups using the GO annotation tool (G. Dennis, Jr., *et al.,*2003)*.* Over- representation calculations were done using Ease (D. A. Hosack, *et al.,* 2003) Functional classifications with an "Ease score" lower than 0.05 were marked as over represented.

### EXAMPLE 1

### ISOLATION OF FAB TCRL ANTIRODIES CAPABLE OF SPECIFIC BINDING TO

### MHC-TYRD₃₆₉₋₃₇₇ COMPLEX

### Experimental Results

***Generation of MHC-Tyrosinase**₃₆₉₋₃₇₇ **complex -*** Previous studies performed by the present inventors have shown the generation of recombinant antibodies with peptide-specific, HLA-A2-restricted specificity to tumor and viral T cell epitopes using large antibody phage libraries. These molecules are termed TCR-like antibodies. To generate such antibodies with a specificity to the HLA-A2/Tyrosinase₃₆₉₋₃₇₇ complex, recombinant peptide-HLA-A2 complexes were generated that present the Tyrosinase peptide (SEQ ID NO: 1) using a single chain MHC construct. In this construct, the extracellular domains of HLA-A2 were connected into a single chain molecule with β₂ microglubulin using a 15-amino acid flexible linker. The complexes were bacterially produced in *E. Coli* BL21 cells as intracellular inclusion bodies and refolded with Tyrosinase 369-377 peptide by redox-shuffling buffering system. Correctly folded complexes were purified by ion exchange chromatography on Q-Sepharose column, followed by biotinylation of the complexes by BirA ligase.

***Isolation of TCR-like antibodies specific to the MHC-Tyrosinase₃₆₉₋₃₇₇ complex** -* To isolate TCR-like antibodies, a large naive Fab antibody phage display library [de Haard HJ, J Biol Chem. 1999 Jun 25;274(26):18218-30] containing 3.7 x 10¹⁰ different Fab clones was screened with the produced complexes. Following four rounds of selection, a two-fold enrichment in phage output was observed. Specific clones were detected by an ELISA assay in which binding was tested with specific and non-specific complexes. Soluble Fab fragments were produced from the phage clones, using *E. Coli* BL21 cells for expression and metal affinity chromatography for purification. The specificity of the soluble Fab was determined by ELISA on biotinylated MHC-peptide complexes that were immobilized to BSA-biotin-streptavidin-coated wells (Figure 1). To determine the correct folding of the bound complexes and their stability during the binding assay, anti-HLA mAb W6/32 was used [Barnstable CJ, Cell. 1978 May;14(1):9-20]. This Ab recognizes HLA complexes only when folded correctly and when containing a peptide. Figure 1 shows the reactivity of several clones (A2, A12, E5, D11) in an ELISA assay with purified HLA-A2-Tyr complexes as well as with control HLA-A2 complexes displaying other HLA-A2-restricted peptides. The soluble Fabs reacted specifically with the MHC class I complex containing the Tyr 369-377 peptide (SEQ ID NO:1) but not with the other 6 control peptides (SEQ ID NOs:2-7).

***Determination of the CDR sequences of the Fab TCRL capable of binding to MHC-Tyrosinase D369-377 complex -*** To determine a DNA pattern of the selected clones, a DNA fingerprint assay was performed, by PCR amplification followed by *Bst*NI restriction reaction. The results showed only one pattern indicating one positive anti-Tyrosinase 369-377 clone isolated from the library (data not shown). The final clone used further in this study was designated TA2 (from phage Fab A2 of Figure 1). The DNA and deduced amino acid sequences of TA2 VH+CH1 and VL+CL (heavy chain + light chain) are presented in Figures 27a-d and are set forth by SEQ ID NOs: 16-19. The CDR sequences of the VL are set forth by amino acids 24-39, 55-61 and 94-102 of SEQ ID NO: 17. The CDR sequences of the VH are set forth by amino acids 94-102, 50-66 and 99-112 of SEQ ID NO: 19. The nucleic acid sequences encoding the VL CDR sequences are set forth by nucleic acids 70-117, 152-183 and 280-306 of SEQ ID NO: 16; the nucleic acid sequences encoding the VH CDR sequences are set forth by nucleic acids 91-105, 147-198 and 295-336 of SEQ ID NO:18.

***The soluble TA2 Fab antibody is capable of binding to MHC-Tyrosinase complexes expressed on antigen presenting cells (APCs) -*** To test the ability of the anti-HLA-A2/Tyr TA2 Fab to bind the target in its native form as expressed on antigen presenting cells, EBV-tranformed B-lymphoblasts HLA-A2⁺ JY cells were loaded with Tyrosinase 369-377 YMDGTMSQV peptide (SEQ ID NO: 1) or control peptides (SEQ ID NOs: 2-7). Peptide-loaded cells were incubated with the soluble purified Fab, followed by incubation with FITC labeled anti-human antibody. Figure 2a shows specific binding of Fab TA2 to cells loaded with the Tyrosinase peptide (SEQ ID NO: 1) but not to cells loaded with the control peptides (SEQ ID NOs: 2-7, 20, 22, 26, 28, 29 and 65).

***The soluble TA2 Fab antibody is sensitive to a point mutation in the Tyrosinase369-377 peptide* -** One of the peptides used in this assay was the unmodified Tyrosinase 369-377 peptide containing Asparagine (N) instead of Aspartic acid (D) at position 371 (YMNGTMSQV; SEQ ID NO:3). As shown in Figure 2a, low level reactivity of the antibody with this peptide was observed, compared with the native peptide (SEQ ID NO:1) presented on the surface of cells, which contains the amino-acid Asp at position 371. This emphasizes the fine specificity of the TA2 Fab to HLA-A2-Tyrosinase 369-377 complex.

Altogether, these results demonstrate the specific binding of the TA2 Fab antibody to the HLA-A2-Tyrosinase 369-377 complex.

### EXAMPLE 2

### GENERATION OF TA2 IgG ANTIBODY

Since Fab fragments isolated from phage libraries are monovalent, the reactivity and sensitivity of the Fab can be improved by increasing its avidity. This was achieved by using two strategies: (i) generating Fab tetramers as was shown previously for other TCR-like antibodies (Cohen, et al., 2003) and (ii) transforming a TCR-like Fab fragment into a whole bi-valent IgG molecule.

### Experimental Results

***Generation of TA2 Fab tetramers -*** To generate Fab tetramers, the light and heavy chain encoding sequences of the TA2 Fab were PCR amplified and cloned separately into an pET-based expression vector. The C terminus of the TA2 Fab light chain was fused to the BirA tag (SEQ ID NO: 66)) for site specific biotinylation. Each of the vectors were transformed into *E*. *coli* BL21 cells and expressed as inclusion bodies which were further refolded together and purified by ion exchange chromatography. The purified recombinant Fab was biotinylated and tetramers were generated by adding fluorescently-labeled streptavidin. Binding of the TA2 Fab tetramer was examined by Flow cytometry with JY-pulsed cells. As shown in Figure 2b, the staining intensity was significantly improved when cells were incubated with the TA2 Fab tetramer compared to the staining with TA2 Fab monomer. The Fab tetramers maintained their binding specificity (Figure 2c).

***Generation of TA2 IgG -*** For the second approach, the antibody Fab domains were implanted onto an IgG1 antibody scaffold. The heavy and light genes encoding the human TA2 Fab were cloned into a human IgG1 kappa antibody shuffling vector based on the backbone of the eukaryotic expression vector pCMV/myc/ER, as described in the General Materials and Experimental Methods section. Each shuttle expression vector carries a different selective gene and thus expression of TCR-like whole IgG molecules was facilitated by co-transfection of the two constructs into HEK293 cells. After co-transfection of HEK293 cells with the two heavy and light chain gene-containing plasmids, cells were grown on selective media. To determine the presence of specific human IgG in culture supernatants, flow cytometry analysis were performed using JY APCs which were loaded with Tyrosinase 369-377 peptide or control peptide and incubated with culture supernatants originating from a single colony. 12 of 14 clones tested were peptide specific and bound only MHC-Tyrosinase 369-377 complexes, and not the control complexes (data not shown). The Tyrosinase-HLA-A2-specific TA2-IgG antibody was purified from HEK293 cells and purified using protein A affinity chromatography. SDS-PAGE analysis of the purified protein revealed homogenous, pure IgG with the expected M.W. of ~ 200 kDa (data not shown).

***The TA2 IgG maintains the specificity of the TA2 Fab antibody -*** In order to examine the specificity of the purified TA2-IgG, JY cells loaded with specific or control peptide, were incubated with the Ab, followed by incubation with PE-labeled anti-human Ab. Flow cytometry analysis demonstrated specific binding to peptide-loaded cells (Figure 2d) as well as maintenance of specificity as shown by lack of binding of the IgG antibody to cells loaded with control peptides (Figure 2e).

Overall these data demonstrate the fine unique specificity of the HLA-A2-Tyrosinase-specific Fab or whole IgG Abs. These results demonstrate the feasibility of transforming phage-derived Fab antibodies into whole IgGs without loss of specificity but with improved avidity and binding reactivity.

***The sensitivity of ligand recognition of the TA2 IgG is improved -*** To demonstrate that ligand recognition sensitivity is improved by using a whole IgG Ab, titration experiments were performed in which peptide-loaded JY cells were incubated with a broad range of Fab or whole Ab concentrations. Specific detection of Tyrosinase-MHC complexes was achieved with 60- fold lower IgG Ab concentration, compared with the detection achieved with the TA2 Fab (data not shown).

***Generation of TCR-like antibodies to gp100 (209, 280 and 154) and MelanA (Mart1) -*** TCR-like antibodies to the other 2 major differentiation antigens gp100 and MelanA (Mart1) were also generated using the same strategy described above. For gp100 TCR-like antibodies directed to the 209, 280, and 154 T cells epitopes were characterized and reported previously [Galit Denkberg et al., Proc Natl Acad Sci U S A. 2002 July 9; 99(14): 9421-9426]. Mart1 TCR-like antibodies to the T cell epitope 26-35 (SEQ ID NO:21) were generated and characterized as described above (data not shown). All TCR-like Fab antibodies to gp100, Mart1, and Tyrosinase were transformed into Fab tetramers or whole IgG molecules as described above.

Altogether, these results demonstrate the generation of IgG antibodies from Fabs directed against MHC-class I complexes with melanoma specific epitopic peptides derived from gp100, Mart-1 or Tyrosinase.

### EXAMPLE 3

### PRESENTATION OF MHC CLASS I- TYROSINASE COMPLEXES ON MELANOMA CELLS

### Experimental Results

***The melanoma lines 624.38, 501A**, **TC-2224 and TC-1352, but not 1938 express all three melanoma differentiation antigens -*** To study expression of melanoma differentiation-derived HLA-A2-peptide complexes, 5 lines derived from melanoma patients were used. To determine gene expression of the differentiation antigens, mRNA was isolated from the melanoma cell lines and RT-PCR analysis was performed using specific PCR primers for MelanA/Mart1 (SEQ ID NOs:8 and 9), Pme117/gp100 (SEQ ID NOs:10 and 11), Tyrosinase (SEQ ID NOs:12 and 13) and GAPDH (control, SEQ ID NOs:14 and 15). As show in Figures 3a-e, the amplification results show that the melanoma cell lines 624.38, 501A, TC-2224 and TC-1352 express all three melanoma differentiation antigens [*i.e*., MelanA (Mart-1), gp100 and Tyrosinase. No expression of the three differentiation antigens was detected in the cell line 193 8.

***Large numbers of HLA-A2*/*Tyrosinase₃₆₉₋₃₇₇complexes are present on the surface of melanoma cells -*** To explore whether the HLA-A2/Tyrosinase TCR-like TA2 Ab is capable of binding endogenously derived MHC-Tyrosinase complexes on the surface of tumor cells, flow cytometry analysis was performed on cell lines derived from melanoma patients. Cells were incubated with TA2 anti-HLA-A2-Tyrosinase 369-377 Ab followed by incubation with PE-labeled anti-human antibody. HLA expression on these cell lines was determined by flow cytometry using monoclonal antibodies W6/32, for total HLA expression, and BB7.2 for HLA-A2 expression (Data not shown). As shown in Figure 4a-c, the TA2 antibody recognized Tyrosinase and HLA-A2 positive (624.38, 501A with TA2 Fab and TC-2224 with TA2 IgG) cells with a very high intensity, which implies that large numbers of HLA-A2-Tyrosinase complexes are presented on the surface of the melanoma cells. No reactivity was detected with Tyrosinase-negative (1938 with TA2 Fab) or HLA-A2-negative (TC-1352 with TA2 IgG) cells (Figures 4d and e). Further evidence for the high reactivity of TA2 with melanoma cells can be seen in experiments in which the reactivity of the TA2 Fab and whole IgG were compared in titration experiments. As is shown in Figure 4g, the whole TA2 IgG molecule could be titrated down to 4 ng yet still demonstrate a clear reactivity with the melanoma cells. As shown in Figure 4f, the amount of whole IgG required to achieve a comparable intensity to that observed with the Fab fragment was 5-fold lower (1 µg of IgG vs. 5 µg of Fab). To achieve such a degree of binding it is know that a density of several thousands of sites is required based on experimental data and titrations further presented herein below. It will be appreciated that such low amount of antibody which can specifically stain cells can be achieved only if sufficient complexes are present on the surface of the cells. In addition, it should be mentioned that such an intense reactivity for TCR-like antibodies was not observed before in studies performed by the present inventors or by other groups (Cohen, et al. 2003; Denkberg, et al., 2003; Lev, et al., 2002).

***Immunofluorescence detection of HLA-A2*/*Tyrosinase complexes on the surface of melanoma cells -*** To further study the high HLA-A2-Tyrosinase presentation on melanoma cells, the present inventors attempted to visualize these complexes on the surface of melanoma cells by confocal microscopy. The 501A and 1938 melanoma cells were reacted with the TA2 anti-HLA-A2-Tyr antibody (FAB) as well as with anti-HLA-A2 BB7.2 mAb, and examined by confocal microscopy. As shown in Figures 6a-d, the 501A melanoma cells were stained very intensely for HLA-A2 expression using the anti-HLA-A2 BB7.2 mAb (Figure 6b) as well as for Tyr⁺/HLA-A2⁺ using the TA2 Ab (Figure 6a) indicating the large number of Tyrosinase-derived complexes expressed on the surface of these cells. Interestingly, the specific HLA-A2-Tyr complexes are organized in unique clusters on the surface of the melanoma cells (Figures 6a and c). In comparison, no significant staining was shown on 1938 melanoma cells.

***Direct quantization of the number of HLA-A2*/*Tyrosinase₃₆₉₋₃₇₇ complexes on the surface of melanoma cells -*** The specific detection of Tyrosinase-HLA-A2 complexes with the TA2 Ab enabled determination of the number of HLA-A2-Tyrosinase complexes displayed on the surface of melanoma cells. To this end, Fab tetramers (C. J. Cohen, *et al.* 2003) which are generated around a single streptavidin-PE molecule, and which are able to bind up to four different HLA-Tyr complexes were used. Using this strategy, the minimal number of complexes presented on the cell surface was determined. The level of fluorescence intensity on melanoma cells stained with TA2 Fab tetramers was compared with the fluorescence intensities of calibration beads with known numbers of PE molecules per bead (QuantiBRITE PE beads), thus providing a means of quantifying PE-stained cells using a flow cytometer. The number of HLA-A2 complexes expressed on the surface of the melanoma lines tested was measured by staining with PE-labeled anti-HLA-A2.1 mAb BB7.2 and was determined to be in the range of 10,000-20,000 molecules/cell. As shown in Figures 7a-e, the minimal number of HLA-A2-Tyrosinase complexes or HLA-A2 molecules displayed on the Tyr⁺/HLA-A2⁺ 501A melanoma cells could be calculated from the measured MFI obtained using TA2 (18.33) and BB7 (56.37) antibodies, respectively. Thus, the number of HLA-A2-Tyrosinase complexes on 501A cells was found to be approximately 3,780 complexes/cell, which is about 20 % of the total HLA-A2-Tyrosinase complexes (11,668) on the surface of 501A cells (Table 2, hereinbelow). In addition, as is further shown in Figures 12a-c and Table 2 hereinbelow, in other melanoma cells the number of HLA-A2 complexes estimated with mAb BB7.2-PE conjugated was about ~12,000-20,000 (in the melanoma cells that exhibit high HLA-A2 tyrosinase presentation, the number of HLA-A2 is 12000-20000. Cells that exhibit low HLA-A2-Tyr complexes express ~5000 HLA-A2 complexes) while the number of HLA-A2/Tyr complexes displayed on high expressing cells such as 624.38 Tyr⁺/HLA-A2⁺ melanoma cell line was determined as ~3500 (average number of ~10 experiments), which is ~20 % of the total number of HLA-A2 complexes. The cell lines 501A and TC-2224, which were also stained with high reactivity, revealed similar results (Table 2 and data not shown). The cell line TC-2207, which was stained at a moderate level, displayed a minimal number of 800-1100 HLA-A2-Tyr complexes (Table 2). Other cell lines, such as TC-1760, which exhibit low reactivity with the TA2 Ab, displayed 100-300 HLA-A2-Tyr complexes (Figure 12c and Table 2, hereinbelow).

**Table 2**

| ***Determination of the number of HLA-A2-Tyrosinase complexes on melanoma cells*** | | | | |
|---|---|---|---|---|
| | ***501A*** | ***624.38*** | ***TC-2207*** | ***TC-1760*** |
| HLA-A2 (BB7.2) | 11,668 | 10,900 | 11,680 | 5,480 |
| HLA-A2/Tyr (TA2) | 3,780 | 4,100 | 1,120 | 120 |

Table 2: Quantization of the number of HLA-A2-Tyrosinase complexes on melanoma cells. 501A, 624.38, TC-2207, TC-1760 melanoma cells were interacted with TA2 Fab tetramers generated around a single streptavidin-PE molecule and the number of HLA-A2-Tyrosinase complexes was determined using a PE calibration curve as shown for example in Figures 7a-e.

Altogether, these results demonstrate, for the first time the determination of the minimal number of MHG-peptide complexes on cells using TCR-L antibodies. In addition, these results demonstrate the high representation of MHC-tyrosinase complexes on melanoma cells (up to 40 % of all HLA-A2-peptide complexes).

### EXAMPLE 4

### MHC-I/TYROSINASE MEDIATED CYTOTOXIC EFFECT OF MELANOMA CELLS

To further verify the observation made by the TA2 TCR-like antibody that high numbers of HLA-A2-Tyr complexes are expressed on the surface of melanoma cells, cytotoxicity experiments were performed with CTLs that specifically recognize the HLA-A2-Tyr₃₆₉₋₃₇₇ epitope. To this end, two melanoma cell lines were used: 624.38, which express high levels of the HLA-A2-Tyr complexes on the surface, as shown by flow cytometry via the reactivity of the TA2 antibody (Figure 5a), and TC-2183, which express low levels of these complexes (Figure 5b). For cytotoxicity assays, chromium-labeled target melanoma cells were incubated overnight in the presence or absence of 10 µM Tyrosinase peptide 369-377 and subsequently exposed to increasing Effector (anti-HLA-A2-Tyr CTLs) to Target (tyrosinase-MHC complex presented on melanoma cell) (E:T) ratios. As shown in Figures 5c-d, the addition of the Tyrosinase peptide significantly increased by 2.5-fold specific lysis of TC-2183 melanoma target cells, which express low numbers of HLA-A2/Tyrosinase complexes according to the FACS analysis (as shown in Figure 5b), compared to target cells exposed to CTL lysis without prior pulsing with Tyrosinase peptide. Thus, the pulsing with peptide increased the number of complexes on the surface and resulted in enhanced killing. In contrast, the addition of the tyrosinase peptide to 624.38 melanoma target cells, which express high levels of HLA-A2-Tyr complexes (as shown in Figure 5a), did not significantly affect the degree of lysis accomplished with the anti-HLA-A2-Tyr CTLs. These results suggest that the 624.38 target cells express endogenously-derived high levels of the HLA-A2-Tyr complexes and in contrast to TC-2183 cells, which express low levels, additional pulsed peptide does not contribute to increased killing. This data further suggests that the native endogenously-derived expression of the HLA-A2/Tyr epitope on the surface of melanoma target cells is high.

### EXAMPLE 5

### PRESENTATION OF MHC CLASS I- MELANOMA ANTIGEN COMPLEXES ON MELANOMA CELLS

### Experimental Results

***Expression hierarchy of T cell epitopes derived from melanoma differentiation antigens as revealed by reactivity of TCR-like antibodies -*** In order to examine the expression level of HLA-A2 in complex with the peptides Tyrosinase 369-377, Mart-1 27-35, gp100 209-217 and gp100 280-288, FACS analysis was performed on melanoma cell lines. For this purpose, the present inventors used whole IgG antibodies of the CLA12 antibody which recognizes the Mart1 derived epitope 27-35 (SEQ ID NO:22), the 1A7 and 2F1 antibodies which recognize the gp100-derived epitopes 209 (SEQ ID NO:4) and 280 (SEQ ID NO:5), respectively, and anti-Tyrosinase Ab TA2, all in context of HLA-A2 (A. Lev, *et al.,* 2002). For the construction of the whole Ab, the heavy and light Fab genes were cloned for expression as human IgG1 antibody into mammalian backbone of eukaryotic expression vectors, and expression was facilitated by co-transfection of the two vectors, as described under the General Materials and Experimental Methods section, hereinabove. As shown in Figures 8a-d, flow cytometry analyses of the staining of three melanoma cells lines (TC-2224, 624.38 and 501A) with the various TCR-like whole IgGs revealed a clear distinctive expression hierarchy. All three lines were intensely stained with BB7.2, indicating high level of expression of the HLA-A2 molecules on the surface (Figures 8a-c). The reactivity with the anti HLA-A2/Tyrosianse TA2 antibody was significantly high confirming the results shown in Figures 4a-h. The expression of HLA-A2/Mart1 complexes (as detected using the CLA12 Ab) was low to modest depending on the cell line used and the expression of HLA-A2/gp100 complexes (as detected using the 1A7 and 2F1) was very low (Figures 8a-c). No detection of complexes by these HLA-A2-restricted TCR-like antibodies was observed on control melanoma cells which were HLA-A2 negative (TC-1352; Figure 8d). These results suggest a clear expression hierarchy of the three major antigens with the number of HLA-A2-Tyrosinase complexes presented on melanoma cells being significantly higher than the number of HLA-A2-Mart1 or HLA-A2-gp100 complexes. Thus, a large number of HLA-A2/Tyrosinase complexes are presented on the surface of melanoma cells with no direct correlation to the expression of other major antigens such as gp100.

***The expression level of Tyrosinase mRNA in melanoma cells is significantly lower than that of Gp100 or Mart-1-*** To further investigate the high level of presentation of the HLA-A2/Tyrosinase complexes on the surface of melanoma cells the relative expression of the three major melanoma differentiation antigens, *i.e.*, Tyrosinase, gp100 and Mart-1, was examined. This was performed to exclude the possibility that the high numbers of HLA-A2-Tyr complexes are due to a significant over-expression of the Tyrosinase mRNA in melanoma cells compared to the other antigens.

To this end, cDNA was produced from 5 melanoma cell lines, of them the 501A, 624.38, TC-2224, TC-1352 cell lines which express tyrosinase mRNA (as shown in Figures 3a-e), the 1938 cell line which expresses HLA-A2 but not the tyrosinase mRNA and the TC-1352 melanoma cell line expresses tyrosinase mRNA but not HLA-A2. The cDNA was subjected to real-time PCR analysis using the glyceraldehyde-3-phosphate-dehydrogenase (GAPDH) as a control gene for verification of cDNA production and for normalizing the relative expression of the three target genes. The relative expression of the target genes was determined as 2^{-ΔCT} ΔC_{T} is defined as the threshold cycle of the target gene minus threshold cycle of the control gene, GAPDH. As shown in Figure 13 and Table 3, hereinbelow, in the 501A, 624.38 and TC-1452 melanoma cell lines the relative expression level of Mart-1 was about 2.5 higher than that of tyrosinase. Moreover, in these cells, the relative expression level of gp100 was about 15-37 higher than that of tyorosinase. Thus, the expression level of Tyrosinase mRNA is the lowest in the expression hierarchy of the three genes with gp100 being the highest in 3 out of 4 lines. In TC-2224 cells Tyrosinase was somewhat higher in expression levels compared to the two other genes.

**Table 3**

| ***Relative gene expression by real-time PCR*** | | | | | |
|---|---|---|---|---|---|
| ***Cell line*** | ***ΔCt Tyr*** | ***ΔCt Mart-1*** | ***ΔCt gp100*** | ***Relative expression of Mart-1 vs. Tyrosinase*** | ***Relative expression of gp100*** vs. ***Tyrosinase*** |
| ***501A*** | 0.504 | -0.524 | -4.731 | 2.03 | 37.66 |
| ***624.38*** | -0.596 | -1.843 | -5.405 | 2.37 | 28.03 |
| ***TC-2224*** | 1.302 | 2.419 | 2.717 | 0.46 | 0.37 |
| ***TC-1352*** | 1.596 | 0.092 | -2.357 | 2.83 | 15.48 |
| ***1938*** | - | - | - | - | - |

***The expression level of tyrosinase mRNA is significantly lower than other melanoma antigens in multiple melanoma cell lines** -* To further substantiate the results obtained for the three melanoma cell lines (501A, 624.38 and TC-2224), the relative expression level of the melanoma antigens was determined in 31 melanoma cell lines. Briefly, cDNA was produced from 31 melanoma cell lines, followed by real-time PCR analysis. 21 of 31 (67 %) melanoma cells were found to express all three melanoma differentiation antigens. As is shown in Figures 11a-c, there is a strict correlation between Melan-A (Mart-1) and Tyrosinase expression level (r-Pearson Correlation Coefficients (rP) = 0.8 with confidence level of 95 %) in all 21 melanoma cell lines with an average of 2.6-fold higher transcript number for Melan-A compared to Tyrosinase. These results are in agreement with published data [Giese,T., et al., J. Invest Dermatol. 124, 633-637 (2005)]. As is further shown in Figures 11a-c, the expression level of gp100 was higher than Mart-1 or Tyrosinase, but no correlation was observed.

***No correlation between the high representation of tyrosinase*/*****HLA-A2 complexes on melanoma cells and tyrosinase mRNA expression** -* Table 4, hereinbelow, summarizes the results of FACS analysis performed on 21 melanoma cell lines, which express the three differentiation antigens, as determined by real-time PCR. 18 of 21 (85 %) melanoma cells express HLA-A2. 12 of 18 (66 %) melanoma cells, which express Tyrosinase and HLA-A2, were recognized by the TA2 Ab. 50 % of these cell lines were recognized with very high reactivity, which implies the high presentation number of HLA-A2-Tyr complexes on the surface of these cells. In contrast, only 6 of 18 (33 %) were recognized by the anti-Mart-1 or anti-gp100 antibodies. The low reactivity of these antibodies with melanoma cells implies that low numbers of Mart-1 and gp100 complexes are presented on these cells. The relative expression level of melanoma antigens by real-time PCR is presented in Table 4 herein below. Presentation levels were revealed by reactivity with TCRLs.

All TCRL antibodies for gp100, Mart1 and Tyrosinase possess a similar binding affinity as demonstrated by real-time SPR (Surface Plasmon Resonance) binding studies (See Tables 7 and 8 herein below).

**Table 4**

| ***Expression and presentation of melanoma antigens on melanoma cell lines*** | | | | | | | | |
|---|---|---|---|---|---|---|---|---|
| | | ***mRNA*** | | | ***Presentation*** | | | |
| | ***Cell line*** | ***Tyrosinase*** | ***Mart1*** | ***gp100*** | ***Tyrosinase*** | ***Mart1*** | ***gp100*** | A2 |
| 1 | 501A | + | ++ | +++ | +++ | ++ | + | +++ |
| 2 | 624.38 | ++ | ++ | +++ | +++ | ++ | + | +++ |
| 3 | 1938 | - | - | - | - | - | - | +++ |
| 4 | stiling | -++ | +++ | +++ | +++ | ++ | + | +++ |
| 5 | 526 | + | ++ | + | - | - | + | +++ |
| 6 | SW | - | - | + | - | - | - | +++ |
| 7 | 1924 | + | + | ++ | +++ | + | + | +++ |
| 8 | 1352 | + | + | +++ | - | - | - | - |
| 9 | 2207 | ++ | + | ++ | +++ | + | + | +++ |
| 10 | 1760 | ++ | ++ | +++ | +++ | - | - | +++ |
| 11 | 1879 | ++ | +++ | +++ | ++ | - | - | +++ |
| 12 | 2081 | - | - | + | - | ++ | - | +++ |
| 13 | 2148-3 | + | + | + | + | - | - | +++ |
| 14 | 2119 | - | - | + | - | - | - | +++ |
| 15 | 2436 | + | + | + | + | - | - | +++ |
| 16 | 1913 | - | - | + | - | - | - | ++ |
| 17 | 2172 | + | + | ++ | + | - | - | + |
| 18 | 1961 | + | + | + | - | - | - | +++ |
| 19 | 2028-1 | - | - | - | - | - | - | +++ |
| 20 | 1907 | + | + | + | + | - | - | ++ |
| 21 | 2224 | + | + | + | +++ | - | - | +++ |
| 22 | 2183 | + | + | + | + | - | - | +++ |
| 23 | 2319 | + | - | + | - | - | - | +++ |
| 24 | 1350 | + | + | ++ | - | - | - | - |
| 25 | 1122 | +++ | +++ | + | - | - | - | - |
| 26 | 1994 | + | ++ | +++ | + | - | - | + |
| 27 | 2370 | ++ | ++ | +++ | - | - | - | - |
| 28 | 2420 | ++ | ++ | +++ | +++ | + | - | +++ |
| 29 | 1927 | + | + | ++ | + | - | - | +++ |
| 30 | 1362 | ++ | ++ | +++ | - | - | - | - |
| 31 | 1851 | - | + | - | - | + | - | +++ |

The results of the real-time PCR analysis negate the possibility that the high amount of HLA-A2-Tyr complexes is the result of overexpression of the Tyrosinase protein in melanoma cell lines. Tyrosinase was expressed at a lower level than Melan-A and gp100 in most of the cell lines examined, thus there is no correlation between the relative gene expression and the presentation of the specific HLA-A2 complexes.

Altogether, the presentation of tumor epitopes with such a high magnitude has not been described until now. This information is particularly important when targets for immunotherapy are considered.

### EXAMPLE 6

### STABILIZATION OF TYROSINASE PROTEIN INDUCES A DECREASE IN THE TYROSINASE-MHC-I COMPLEXES PRESENTED ON MELANOMA CELLS

Following verification that the high presentation of HLA-A2-Tyr complexes on the surface of melanoma cells is not due to overexpression of the Tyrosinase gene, the effect of Tyrosinase protein stability on complex presentation was examined. Since the melanoma cells exhibit an amelanotic phenotype, the present inventors have hypothesized that the Tyrosinase protein is inactive, thus large amounts of the protein are being degraded by the proteasome, leading to high presentation of MHC-tyrosinase complexes on the cell surface. It was shown elsewhere [R. Halaban, et al., J. Biol. Chem. 276(15), 11933 (2001)] that DOPA can induce conformational changes favorable for the exit of Tyrosinase from the endoplasmic reticulum (ER) to the Golgi apparatus and restore melanin synthesis thus inducing activity of inactive Tyrosinase. To test the effect of DOPA on presentation of HLA-A2/Tyrosinase complexes, in order to eliminate endogenously derived complexes prior to treatment with DOPA, the peptides presented on MHC were eluted using citrate-phosphate buffer as previously described (Storkus WJ, et al., J Immunother. 1993 Aug;14(2):94-103]. The cells were then incubated for 20 hours with 1 mM DOPA, and the presentation of the Tyrosinase/MHC complexes was determined by flow cytometry using the TA2 antibody.

### Experimental Results

***Stabilization of Tyrosinase protein induces decrease in the number of HLA-A2*/*Tyrosinase₃₆₉₋₃₇₇ complexes on the surface of melanoma cells -*** Flow cytometry analysis of DOPA-treated melanoma cells was performed by incubating these cells with the TA2 Ab followed by incubation with PE-labeled anti-human Ab. As shown in Figures 9a-b for a representative experiment, the presentation of HLA-A2-Tyr complexes was significantly decreased following incubation with DOPA. Quantization of the results is presented in Table 5, hereinbelow.

**Table 5**

| ***Quantization of HLA-A2-Tyr complexes on the surface of melanoma cells after DOPA treatment*** | | |
|---|---|---|
| | ***MFI signal*** | ***MFI background*** |
| 501A | 17.61 | 4.54 |
| 501A+1mM DOPA | 9.81 | 6.11 |

As shown in Table 5, hereinabove, there was a 50-75 % decrease in the MFI of DOPA treated vs. untreated cells suggesting a significant decrease in the number of HLA-A2/Tyrosinase complexes following DOPA treatment. Thus, without being bound by any theory, these results demonstrate that the high presentation of HLA-A2-Tyr complexes results from the instability of the Tyrosinase protein in melanoma cells. Thus, stabilization of the Tyrosinase protein results in a significant decrease in the number of HLA-A2/Tyrosinase complexes on the surface of the cell.

***Determination of protein stability and its correlation to presentation of melanoma-derived differentiation antigens** -* To further investigate the relationship between protein stability and antigen presentation, the rate of protein degradation was determined in melanoma cells. To this end, the 501A melanoma cells which express HLA-A2 and the three melanoma antigens as determined by real-time PCR (Table 4, hereinabove) and reactivity with the TCR-like antibodies (Figure 8b) were used. To determine the half-life of the proteins (gp100, tyrosinase and Mart-1), protein degradation was arrested by treating the cells with Cyclohexamide followed by cell harvesting at various time points. Protein degradation was monitored by running SDS-PAGE of cell extracts at fixed protein amount followed by Western blotting and detection of the amount of each antigen by using Peroxidase-labeled antibodies specific for gp100, Mart1, and Tyrosinase whole proteins. Antibody reactivity on blots was quantified by scanning densitometry using a densitometry computer program. As shown in Figures 10a-d, there was a hierarchy of degradation rate or protein stability with Mart-1 being the most stable with a t1/2 of ~6.5 hours, gp100 with moderate stability (t1/2 of ~4.5 hours) and Tyrosinase being the less stable protein of the three antigens with a t1/2 of ~2.5 hours (the half life was calculated as ln(2)/k, -k is the slope of the graph ). These results indicate the possible relationships between protein stability and the presentation hierarchy of the three melanoma antigen as observed with the TCR-like antibodies. It is possible that rapid degradation of the antigen results in a more efficient intracellular processing and presentation as observed for Tyrosinase which exhibits a relative short half-life compared to the other antigens but extremely high level presentation on the cell surface.

### EXAMPLE 7

### HIGH ANTIGEN DENSITY INDUCES HYPO-RESPONSIVENESS IN MEMORY ACTIVATED CTLS

To study the role of antigen density on the response of activated memory CTLs, the present inventors used activated human HLA-A2-restricetd CD8+ CTL clones and lines with viral or tumor antigen specificity, which originated from tumor infiltrating lymphocytes (TILs) (J. Zhou, et al., 2004) or peripheral blood. These CTLs were established as effector memory CD8+T cells by virtue of their characteristic phenotype, *i.e*., potent cytotoxic activity, cytokine secretions - IL-2, IFNγ, TNFα - but lack of IL-10 or IL-4. They exhibit surface expression of the memory phenotype; *i.e.*, CD3⁺ ,CD8⁺ ,TCRαβ⁺ ,CD45RO⁺, CD45RA⁻ ,CD62L⁻ ,CCR*T* ,CD56⁻ ,CD85⁻ ,CD69^{low}, and CD25^{low}.

The memory CTLs were exposed to increasing antigen (MHC-peptide complexes) densities using three experimental systems: (i) peptide-loaded pAPCs; (ii) deposition of HLA-A2-peptide complexes on cells lacking endogenous expression of HLA-A2 through the use of an antibody-HLA-A2 fusion molecule (A. Lev, et al. 2004) and (iii) the use of viral-infected HLA-A2 target cells.

### Experimental Results

### (i) Peptide-loaded pAPCs

***High MHC-peptide complexes presented on cells induce hypo-responsiveness of CTLs -*** As shown in Figures 14a-d, the lytic activity induced by the CTL clones increased in a dose dependent manner from low to intermediate concentrations of epitopic peptide. However, further increase in peptide concentration significantly impaired the lytic activity of the CTLs, up to 60 % decrease from optimal lytic activity (Figures 14a-d). These results were observed with 4 different human HLA-A2-restricted activated CD8+ T cell clones which recognize the melanoma differentiation antigens gp100-derived T cell epitope 209-217 (Figure 14a) or 280-288 (data not shown) and MART-1 derived epitope 27-35 (Figure 14b). Moreover, lytic activity was also tested in CTL lines which recognize viral-derived T cell epitopes. Figures 14c and d depict the results with two CTL lines originated from peripheral blood, which recognize the EBV BMLF-1 derived peptide 280-288 (Figure 14c) and the CMV pp65 derived peptide 495-503 (Figure 14d). The killing assays presented depict 5-hour cytotoxicity experiments but similar results were observed with 2 and 16-hours assays indicating that CTL function is stably impaired. Overall these results show that the optimal peptide concentration, which is required to induce maximum killing, is approximately in the range of 1 x 10⁻⁷ M. The binding affinities of these peptides to HLA-A2 were similar and in the high range of the HLA Peptide Binding Predictions algorithm (hypertexttransferprotocol://bimas.dcrt.nih.gov/molbio/hla_bind/). Furthermore, according to peptide titration data, the affinities of the CTL for the two antigens are similar (data not shown).

These results suggest that high antigen densities induce hypo-responsiveness in properly activated T cell lines and clones.

***Quantification of the number of HLA-A2-peptide complexes required to achieve optimal lysis by CTLs -*** In order to directly quantify the specific antigen densities which mediate optimal cytotoxic activity or induce hypo-responsiveness of CTL activity, aliquots of TCR-like antibodies which were previously developed by the present inventors (C. J. Cohen, et al., 2003; G. Denkberg, et al., 2003) were utilized. TCR-like antibodies bind to specific HLA-A2/peptide complexes with a peptide-specific, MHC-restricted manner but in contrast to the low affinity of TCRs these antibodies exhibit a high affinity binding in the nM range (C. J. Cohen, et al., 2003; G. Denkberg, et al., 2003). Titration of binding of such a TCR-like antibody is shown in Figures15a-d. Fluorescently (PE)-labeled secondary monoclonal antibody and PE-calibration curves were used to enumerate the number of peptide-MHC complexes for each peptide pulsing concentration as detected by the TCR-like antibodies (Figure 14e). As shown in Figure 14f, for MART1₂₇₋₃₅ HLA-A2-restricted CTL clone JKF6, these calibration curves enabled quantification of the number of HLA-A2-peptide complexes required to achieve initiation, optimal, and inhibition of lysis by the appropriate CTL. Similar results were observed with other CTL specificities shown in Figures 14a, c and d. Such direct quantification strategy reveals in all cases that very few complexes are required to initiate killing (10 complexes yielded > 20-50 % of cytotoxicity). An average of 80-120 specific HLA-peptide complexes on the surface of each target cell, termed as the optimal antigen density, induced maximal CTL mediated killing, however higher levels of antigen densities in the range of 500-700 HLA-A2-peptide complexes significantly reduced the CTLs lytic activity.

### (ii) Deposition of HLA-A2-peptide complexes on cells lacking endogenous expression of HLA-A2 (e.g., non-lymphoid cancerous epidermal cells) through the use of an antibody-HLA-A2 fusion molecule

In this approach, targeted deposition of HLA-A2-peptide complexes on target cells devoid of HLA-A2 expression is facilitated through a recently developed genetic fusion that was generated by the present inventors (A. Lev, *et al.,* 2004). In this fusion molecule, a cell targeting scFv antibody fragment is genetically fused to a single-chain HLA-A2 molecule (Figure 16a). These fusion proteins enabled the present inventors to coat HLA-A2 negative cell lines with different densities of HLA-A2 molecules bearing a specific peptide (Figure 16b). HLA-A2 negative A431 epidermoid carcinoma cells expressing the Epidermal growth factor receptor (EGFR) were coated with an αEGFR scFv HLA-A2 fusion which carries the HLA-A2-restricetd EBV BMLF-1 derived peptide 280-288 (SEQ ID NO:24) (Figure 14g) or ATAC4 cells (A431 stably transfected with CD25/Tac) were coated with an αTac scFv HLA-A2 fusion which carries the melanoma antigen gp100-derived epitope 209-217 (SEQ ID NO:4) (Figure 14h). These fusion molecules were effectively used to titrate and segregate low, optimal and high antigen density cell colonies. Subsequently, HLA-A2 restricted lysis of these cohorts by the appropriate activated CD8+ CTLs was observed as shown in Figure 14g and 14h. Quantification of the number of αTac scFv HLA-A2 209 and αEGFR scFv HLA-A2-EBV fusion molecules bound to the target HLA-A2 negative carcinoma cells at various concentrations was measured with PE-labeled HLA-A2-specific MAb BB7.2 and PE calibration curves.

Lytic activity for deposited HLA-A2-peptide complexes was similar to that of peptide-loaded pAPCs (Figures 14a-d), namely the CTLs killing was initiated at 10-20 complexes, exhibited a peak of optimal lytic activity at intermediate HLA-peptide densities of ~100 complexes/cell, and was significantly inhibited by 40-60 % at high antigen densities of > 500 complexes/cell. These results were also observed with 3 other T cell clones and lines (data not shown). The pattern of inhibition of CTL lytic activity at high antigen densities was similar whether peptide-loaded pAPCs or HLA-A2 deposition on target cells have been used.

### (iii) Use of viral-infected HLA-A2 target cells -

In this experimental approach normal human HLA-A2+ fibroblasts were infected with CMV and the cytotoxic activity of CMV- pp65-derived epitope 495-503 (SEQ ID NO:25)-specific CTL line was determined. The number of pp65-derived HLA-A2/pp65₄₉₅₋₅₀₃ complexes on the surface of the CMV-infected cells was determined using the TCR-like antibody H9 as described above. As shown in Figure 14i, optimal CTL cytotoxic activity towards virus-infected cells was observed 96 hours post infection when the number of HLA-A2/pp65₄₉₅₋₅₀₃ complexes was ~100 sites per cell. When the density of pp65-derived HLA-A2-peptide complex was ~700 sites, CTL hypo-responsiveness was observed in a commensurate manner to the two other experimental systems. In comparison with peptide pulsing or MHC deposition, this experimental model reflects the innate physiological processes in which intracellular viral derived peptides are translocated in the MHC complex to the cell surface and the density of such endogenously-derived viral peptide-MHC complexes is increased as a function of time after infection.

Interestingly the phenomenon of antigen density-induced non-responsiveness is persistent. As shown in Figure 17a, CTLs exposed initially to low or intermediate antigen density maintained their proper cytotoxic activity when they re-encounter antigens at optimal density after a week. However, the CTLs that were exposed initially to high antigen densities remained anergenic. They exhibited 75 % reduction in lytic activity compared to CTLs exposed to low or intermediate antigen densities. In addition, as shown in Figure 17b and 17c significant inhibition in CTL proliferation and reduction in total RNA content was observed when exposed to high density (500-700 complexes) but not to low or intermediate densities of antigen.

### EXAMPLE 8

### IMPAIRED PROLIFERATION AND CYTOKlNE SECRETION AFTER EXPOSURE OF ACTIVATED CTLS TO HIGH ANTIGEN DENSITY

To further investigate the molecular mechanisms that control antigen-induced CTL hypo-responsiveness two major parameters were examined in T cell biology; secretion of cytokines and expression of surface molecules associated with CTL function.

### Experimental Results

As shown in Table 6, hereinbelow, significant changes were observed over time in the secretion pattern of the Th1 cytokines IL-5, IL-2, and IFNγ but not TNFα.

**Table 6**

| ***Effect of antigen density on Th1 cytokines*** | | | | |
|---|---|---|---|---|
| ***Condition*** | ***IL-2*** | ***IL-5*** | ***IFNγ*** | ***TNFα*** |
| 4h-High Ag Density | 3856 | 79 | 748 | 179.3 |
| 4h-Optimal Ag Density | 5100 | 122 | 696 | 215 |
| 4h-Low Ag Density | 431 | 58 | 195 | 24 |
| 16h-High Ag Density | 2032 | 202 | 1182 | 523 |
| 16h-Optimal Ag Density | 3593 | 355 | 2037 | 473.9 |
| 16h-Low Ag Density | 211 | 158 | 338 | 52 |
| 36h-High Ag Density | 826 | 393 | 1152 | 175 |
| 36h - Optimal Ag Density | 1386 | 642 | 1492 | 156 |
| 36h - Low Ag Density | 91 | 171 | 270 | 6 |

Table 6: The effect of antigen (MHC-peptide complexes) presented on cells on the secretion of Th1 cytokines. TNFα, IL-5, IL-2, and IFNγ secretion from MART-1₂₇₋₃₅-specific CTL clone JKF6 exposed to various antigen densities as measured by cytometric bead assay (BD). Ag - antigen; h - hours after exposure; high - 700 complexes; optimal- 100 complexes; low - 10 complexes

As shown in Table 6, hereinabove, the profile of cytokine release correlated with the killing pattern observed as a function of antigen density. Thus, CTLs exposed to optimal densities of peptide-MHC (~100 complexes) secret a certain level of cytokines. However, once the threshold of peptide-MHC density has reached its upper limit, cytokine secretion decreased significantly, corresponding to a reduction in T cell function, to wit, anergy.

***Impaired expression of key functional molecules, after exposure of activated CTLs to high antigen density** -* The expression of key surface molecules that are associated with effector CTL function and immunological synapse formation as a function of time and antigen density were studies. Exposure to increasing densities of antigen reveals significant and correlative alterations in surface molecules expression (Figures 18-22). Major differences were observed in the expression patterns of CD8 and CD3 (Figures 18a-i), CD45RO (Figures 19a-i), and CD85 (Figures 20a-i) as well as down regulation of 70 % in TCRαβ. Moreover, as shown in Figures 18-20, expression of CD8+ CD45RO and CD85 was palpably altered, CD8 and CD45RO surface expression decreased concordance with increasing antigen density on the target cell and time of exposure. Expression of CD85 (LIR1) was also altered upon CTLs exposure to increasing densities of antigen. In contrast CD152 (Figures 21a-i) and staining with AnnexinV (Figures 22a-i) indicative of apoptosis was not altered.

Figures 23a-i summarize the alterations in numerous experiments for the expression pattern of the three key surface markers as a function of time after exposure to increasing concentrations of peptide (antigen density). The percentage of CD8^{high}/CD8^{low}, CD45RO^{high}/CD45RO^{low}, and CD85^{Dim}/CD85⁻ CTLs subpopulations is shown. Most pronounced are the results observed 48 hours after encounter with high antigen densities in which there is a clear shift or conversion point between the "high" and "low" phenotype that occurs at peptide concentration of~1 x 10⁻⁷ M which correlates to antigen density of ~ 100 peptide-MHC complexes per cell. The results obtained demonstrate that high antigen densities engender changes in the expression pattern of key molecules that are required for proper T cell function and synaptic formation/interaction. These results demonstrate that three critically important parameters in CTL function namely, proper cytotoxic activity, secretion of Th1 cytokines, and expression of key surface molecules intercept at an optimal antigen density of 100 complexes per target cell and that above a threshold of ~250 complexes per target they become hyporesponsive with concurrent down regulation of respective parameters.

### EXAMPLE 9

### DISTINCT GENE EXPRESSION SIGNATURE INDICATIVE OF ANERGY IN CTLS EXPOSED TO HIGH DENSITY OF ANTIGEN

The fact that maximal alterations in expression of surface markers and cytokine secretion occurred after a relative long period of time (maximal effect after 48 hours) suggested alteration in gene expression. Therefore, the present inventors have performed a comparative genome-wide microarray analysis (Affymetrix) of genes expressed by CTLs exposed to low, optimal, and high antigen density as a function of time.

### Experimental Results

Comparison between treated and non treated samples identified a list of 5877 probe sets changed by at least 2 fold in one or more of the samples compared with time 0 (Figure 25a). Comparing CTL samples exposed to high versus optimal antigen densities for 36 hours identified 1070 probe sets that are differentially expressed by at least two folds between these two samples (Figure 25b). Classification of these 1070 genes revealed four major categories of genes for which significant alternations in gene expression were observed between optimal and high density: energy metabolism, membrane potential, cell signaling and apoptosis versus cell cycle control (Figure 25c). A representative list of genes from the gene array analysis of 1070 probe sets described in Figures 25b and c is shown along with the fold changes in the expression of representative genes from CTL exposed to optimal versus high antigen density (Figure 25d).

The gene analysis results of CTLs exposed to high antigen density revealed that energy metabolism was marked by inhibition of glycolysis indicative by reduction of key glycolysis enzymes such as hexokinase and phosphofructokinase. Changes in membrane potential were reflected by alteration in ion channels expression. Severe depolarization of membrane potential and decreased calcium compliance might result from these alterations. Further impairments were observed in T cell signaling in general including alterations genes of the RAS, MAPK, and IP3 pathways as well as alterations in TCR complex signaling. Gene expression profiles related to cell cycle control and apoptosis indicate a signature of cell cycle arrest with no marked shift toward apoptosis.

Overall, the gene chip analysis presents a signature of an anergic T cell with ablated energy metabolism, altered membrane potential, impaired signaling, and cell cycle arrest. Without being bound by any theory, these data explains the severely impaired response of memory activated CTLs to high antigen density.

### Analysis and Discussion

In previous studies, the present inventors have demonstrated the production of recombinant TCR-like antibodies, which can specifically recognize HLA-A2 in complex with peptides derived from MAA (melanoma associated antigens) or other tumor and viral proteins. The importance of TCR-like antibodies for research purposes was demonstrated by obtaining precise information about the antigen presentation of MHC-class I complexes on cancer cells, as well as viral-infected cells (C. J. Cohen*, et al.,* 2003; G. Denkberg, *et al.*, 2003; A*.* Lev, *et al.,* 2002). These molecules also enable the quantification of the specific MHC class I complexes on the surface of cells (C. J. Gohen, *et al.,* 2003). In addition, the TCR-like antibodies are important for purposes of research since they provide valuable information about potential targets for immunotherapy. These antibodies can also actively participate in immunotherapy as targeting molecules, considering their high affinity and specificity. By generating a whole IgG antibody, tumor cell lysis can be achieved by antibody-dependent cell-mediated cytotoxicity (ADCC) or complement-dependant cytotoxicity (CDC) mechanisms (J. Golay, *et al.,* 2004; H. Mellstedt, 2003; Modjtahedi, *et al.*, 2003; N. Prang, *et al.,* 2005).

In the present study, a TCR-like antibody against the Tyrosinase epitope 369-377 was isolated. Tyrosinase is a membrane-associated N-linked glycoprotein and it is the key enzyme in melanin synthesis. It is expressed in all healthy melanocytes and in nearly all melanoma tumor samples (H. Takeuchi*, et al.,* 2003; S. Reinke, *et al.,* 2005). Peptides derived from this enzyme are presented on MHC class I molecules and are recognized by autologuos cytolytic T lymphocytes in melanoma patients (T. Wolfel, *et al.,* 1994; . Brichard*, et al.*, 1993). The Tyrosinase HLA-A2-associated epitope 369-377, YM*D*GTMSQV, is generated by posttranslational conversion of the sequence YM*N*GTMSQV. Only YM*D*GTMSQV and not YM*N*GTMSQV is presented by HLA-A*0201 on cells expressing full-length tyrosinase (J. C. Skipper*, et al.,* 1996; C. A. Mosse, *et al.,* 1998). Following protein synthesis, the Tyrosinase is folded in the ER. Correctly folded Tyrosinase is transported via the trans Golgi network to melanosomes (K. Jimbow, *et al.,* 2000). The proposed model for Tyrosinase epitope presentation process includes glycosylation on the N₃₇₃ residue during synthesis of the full-length protein in the ER, followed by reverse translocation of the enzyme to the cytosol, deglycosylation accompanied by deamination (thus conversion of N₃₇₃ to D), degradation by the proteasome, and TAP mediated transport of the resulting peptide fragments into the ER for HLA-A2 binding (V. H. Engelhard., 2002). Loss of pigmentation is frequently observed in human melanoma cells. In these amelanotic melanoma cell lines, tyrosinase failed to reach the melanosomes, and retained in the ER. The aberrant accumulation of Tyrosinase in the ER of melanoma cells results from tumor induced metabolic changes. The acidification of the ER-Golgi boundary of melanoma cells which is hostile to Tyrosinase maturation is the cause of the amelanotic phenotype (R. Halaban, *et al.*, 2002). It has been shown that the substrates DOPA and tyrosine can induce conformational change favorable for the exit of Tyrosinase from the ER to the Golgi and restore melanin synthesis (R. Halaban, *et al.,* 2001). Here the present inventors show that the TCR-like antibody, specific to the Tyrosinase epitope 369-377 presented on melanoma cells, is capable of recognizing melanoma cell lines with a high reactivity, which impliesthat very high amounts of the Tyrosinase epitope are presented on the surface of melanoma cells. In addition, and without being bound by any theory, the present inventors propose that the inactive Tyrosinase, which results in the abundant amelnotic phenotype, is the cause of the very high presentation of tyrosinase-HLA-A2 complexes on the surface of melanoma cells. In addition, the use of DOPA, which causes active Tyrosinase and melanin synthesis, prevents the high presentation of tyrosinase-HLA-A2 complexes. Moreover, the present inventors have found that among the three antigens analyzed, Tyrosinase which is highly presented on the cell surface is the fastest in degradation thus its relative low stability may contribute to the high level of presentation that was observed. However, protein stability may be only one reason for this finding. Other possibilities may include efficiency of processing that relates to the particular composition of the antigen.

Mart-1 27-35 is a very common immunogenic epitope for HLA-A2-restricted melanoma-specific TIL. It is known as an immunodominant epitope and CD8⁺ CTLs specific for this epitope are frequently found in melanoma patients (Y. Kawakami, *et al.,* 1994). In contrast, the generation of a Tyrosinase-specific response in melanoma patients is a relatively infrequent event. In several studies, Tyrosinase was hardly detectable by the TILs used (Y. Kawakami, *et al.*, 2000). In addition, flow cytometric analysis of PBMCs stained with tetramers showed that Tyrosinase peptide 369-377-specific CD8+ T cells were hardly detectable in peripheral blood of melanoma patients. However, significant numbers of such cells were detected after short-term stimulation of CD8+ lymphocytes with Tyrosinase peptide (D. Valmori, *et al.*, 1999). The results presented here can explain the low immunogenicity of the Tyrosinase epitope. Continual exposure of T cells to antigen maintains an unresponsive state and result in adaptive tolerance (B. Rocha, et al., 1995; L. S. Taams, et al., 1999; R. H. Schwartz, 2003; F. Ramsdell and B. J. Fowlkes, 1992). Without being bound by any theory, the relatively low immune response against Tyrosinase 369-377 epitope can be a result of the high presentation of HLA-A2-Tyr complexes. It may be assumed that HLA-A2-Tyr 369-377 complexes are not presented on healthy melanocytes membrane, because in these cells the Tyrosinase protein is stable and melanin synthesis is accomplished. Thus, the tolerated T cells are a result of the high presentation on melanoma cells and not a result of self presentation on healthy melanocytes.

Since the level of epitope presentation impacts the effectiveness of different immunotherapies differently this information can be used diagnostically to select which type (antibody- or CTL-based) of immunotherapy can or should be used against a particular antigen or for a particular patient. For example a CTL-based immunotherapy could be contraindicated for a patient whose tumor expressed the targeted epitope at the high levels observed here for the tyrosinase 369-377 epitope in many cell lines. Conversely a low level of expression of the targeted epitope can indicate that a CTL-based immunotherapy would be more advantageous than an antibody based one.

The information presented here describes the unique presentation hierarchy of melanoma differentiation tumor antigens. According to these results, Tyrosinase 369-377 is presented with thousands of copies on melanoma cell lines. Presentation of a tumor antigen with such magnitude was not described until now. This information is particularly important when targets for immunotherapy are considered.

The most important question with respect to immunotherapeutic and diagnostic applications of TCR-like antibodies relates to the low density and turnover of the specific epitope on the target cell surface. With regard to the density and targeted killing of cells the present inventors have previously showed in a murine model, that to achieve efficient killing with a TCR-like immunotoxin molecule a density of several hundreds to a thousand MHC-peptide complexes is required for selective elimination of APCs (W. J. Storkus, *et al.*, 1993). The present data suggest that the concept that T cell epitopes are expressed at low numbers on target tumor cells is not universal and there are probably many exceptions represented by antigens such as Tyrosianse. These targets may be ideal for antibody-mediated drug delivery, as well as tumor cell lysis achieved by antibody-dependent cell-mediated cytotoxicity (ADCC) or complement-dependant cytotoxicity (CDC) mechanisms. Exploring these highly expressed T cell epitopes may shed new light on the biology of antigen processing and presentation as well as the process of tolerance. For the clinical aspects such epitopes that are tumor associated and are expressed at high levels open new opportunities for therapeutic interventions particularly in cases where T cell responses fail because of high level expression and tolerance or anergy of antigen-specific CD8+ T cells. In such cases TCR-like antibodies can replace cellular immunity and constitute an attractive therapeutic modality.

Current literature describes two major mechanisms for vetting T cells, thymus selection and priming by pAPCs in lymph nodes (T. R. Mempel, et al., 2004; S. C. Jameson, et al., 1994). The data presented here suggests a third new control point that occurs at the peripheral *in situ* tissue level. Activated memory CD8+ cytotoxic T cells possess properties of a self-referential sensory organ that is highly sensitive to antigen density. Killing is initiated at very low antigen density and reaches optimal killing activity at around 100 MHC-peptide complexes, however, once a certain upper threshold of about 250 MHC-peptide complexes is being detected on the surface of the target cell significant inhibition of CTL activity was observed which induces hyporesponsiveness or anergy of activated CD8+ T cell lines or clones. This hyporesponsive response is characterized by a significant long term decrease in cytotoxic activity, inhibition of proliferation, and a substantially remodeled anergic T cell.

This work uncovers control mechanism that functions as a negative regulator of CTL cytotoxic activity. Although conservation of this phenomena is yet to be studied *in vivo,* it is however, well appreciated that self antigens are in general richly expressed, thus one may hypothesize that this novel modality may serve to protect localized tissue from an errant auto reactive attack, by essentially revoking its license to kill.

This induced anergy might explain as least in part ― peripheral tolerance, a safe guard mechanism that prevents auto reactivity when T cells sense high antigens densities which signal a dangerous auto reactive circumstance. The results presented here may correlate with the recent finding that agonist/endogenous peptide-MHC heterodimers can regulate T cell activation and sensitivity in CD4+ lymphocytes (M. Krogsgaard, *et al*.,2005). According to these findings which predict that also CD8+ T cells use self-peptide-MHC complexes in their response, initial killing at very low antigen densities (1-20 complexes) involves agonist/endogenous heterodimer complexes. Optimal killing is achieved at ~100 complexes when most of agonist/endogenous heterodimers are shifted into agonist homodimers when the number of agonist peptide-MHC complexes on the surface increase. However, the new safe guard control mechanism on CTL function, described herein, starts to operate when the number of agonist homodimers or larger MHC-peptide structures are generated due to high antigen density of >250 complexes (see schematic model in Figure 26).

Most profound in this machinery are anergic state gene expression signatures at high antigen densities compared with a full activation signature at the optimal density. Impairment in T cell function is due to major alterations in TCR signaling and function, membrane permeability, and alterations in energy metabolism and cell cycle control. Glycolysis was largely ablated which indicate that energy metabolism may play a critical role in the control of function of T cells. It was recently suggested that T cell metabolic needs are governed by eternal signals (transcriptional and translations responses as well as co-stimulation) (C. J. Fox, et al., 2005). Once T cells do not receive these signals they fail to increase their energy metabolism to meet the hyperbioenergetic demands of cell growth and are either deleted or rendered unresponsive to mitogenic signals. T cells exposed to high antigen densities are not subject to enhanced apoptosis but rather cell cycle arrest. Thus, these results further strengthen the anergic model for effector memory CTLs rather than deletion through antigen-induced cell death or apoptosis.

Taken in the context of the multitude of signaling and gene alteration events that occur in the high antigen density induced anergenic T cells, it is hoped that this work will further elucidate on the underlying mechanism of T cell biology, with ramifications extending into understanding more about the control of immune function and regulation such as peripheral tolerance, control of viral infections, anti tumor immune responses, hypersensitivity, and autoimmunity.

In light of the unexpectedly high density of Tyrosinase 369-377 MHC class I (A2+) complexes in Tyrosinase antigen+ cells, TCRL specific for this epitope-MHC combination can be used to diagnose melanoma.

Taking into account the lack of correlation between antigen expression or gene expression and the density of MHC-peptide complexes, TCRL can be used as a more reliable reagent / procedure (in vivo or ex vivo) to measure target epitope presentation by tumor cells, in support of identifying patients that are candidates for epitope directed therapy. Alternatively, excluding patients that fail to express epitope-MHC (as detected by TCRL) with or without whole protein or gene expression, from being treated by epitope directed therapies.

### EXAMPLE 10

### CYTOTOXICITY OF TOXIN-COMPLEXED ANTIBODIES OF THE PRESENT INVENTION

### Materials and Methods

***Peptides and** cell **lines:*** The HLA-A2-restricted peptides used for specificity studies are gp100₁₅₄₋₁₆₂: KTWGQYWQV (SEQ ID NO: 20); gp100₂₀₉₋₂₁₇: IMDQVPFSV (G9-209 - SEQ ID NO: 4); gp100₂₈₀₋₂₈₈: LLLTVLTVL (G9-280 - SEQ ID NO: 5); HTLV-1_{TAX11-19}: LLFGYPVYV (TAX- SEQ ID NO: 26); CMV P65₄₉₅₋₅₀₃: NLVPMVATV (SEQ ID NO: 25); TARP₂₉₋₃₇: FLRNFSLML (SEQ ID NO: 7) ; XAGE-1 (SEQ ID NO: 30);Mart-1₂₆₋₃₅ EAAGIGILTV (Mart-1 26-35 SEQ ID NO: 21); Mart-1_{27L} ELAGIGILTV (Mart-1 27L ― SEQ ID NO: 27) ; hTERT₈₆₅₋₈₇₃: RLVDDFLLV (SEQ ID NO:29).

Cell lines used in this study: B cell line RMAS-HHD, which is transfected with a single-chain β₂m-HLA-A2 gene, the EBV-transformed HLA-A2⁺ JY cells, melanoma cell lines: HLA-A2+gp100+Mart-1+ :Me1624.38, Me1526,Me1501A, FM3D, Stiling. HLA-A2+gp100-Mart-1-: Me11938 HLA-A2-gp100+Mart-1+: HA24, G-43; HLA-A2-gp100-Mart-1-: PC3.

*Selection **and characterization of recombinant** Fabs **with specificity** for* ***Mart-1*/*HLA-A2:*** The generation and characterization of a panel of Fabs specific for peptide/HLA-A2 were previously described in detail [Lev et al., Cancer Res 62, 3184-94 (2002)]. Phage Abs were selected for binding to single-chain MHC-peptide complexes [Denkberg G et al., Eur J Immunol 30, 3522-32 (2000)] using a large human Fab library containing 3.7 x 10¹⁰ different Fab clones [Lev et al., Cancer Res 62, 3184-94 (2002)]. The binding specificity of the phage clones selected were tested against soluble Mart-1/HLA-A2 complexes in ELISA assays. Mart-1/HLA-A2-specific Fab Abs were expressed and purified as previously described [Lev et al., Cancer Res 62, 3184-94 (2002)]. The eluted Fabs were dialyzed twice against PBS (overnight, 4°C) to remove residual imidazole.

The DNA and deduced amino acid sequences of CLA12 VH+CH1 and VL+CL (heavy chain + light chain) are presented in Figures 30a-b and Figures 30e-f and are set forth by SEQ ID NOs:31-32 and 35-36. The CDR sequences of the VL are set forth by amino acids 23-35, 51-57 and 90-100 of SEQ ID NO:31; The CDR sequences of the VH are set forth by amino acids 31-37, 52-67 and 100-107 of SEQ ID NO:32. The nucleic acid sequences encoding the VL CDR sequences are set forth by nucleic acids 67-105, 161-171 and 268-300 of SEQ ID NO:35; the nucleic acid sequences encoding the VH CDR sequences are set forth by nucleic acids 91-111, 154-201 and 298-321 of SEQ ID NO:36.

The DNA and deduced amino acid sequences of CAG10 VH+CH1 and VL+CL (heavy chain + light chain) are presented in Figures 30c-d and Figures 30g-h and are set forth by SEQ ID NOs:33-34 and 37-38. The CDR sequences of the VL are set forth by amino acids 23-36, 52-58 and 91-101 of SEQ ID NO:33; The CDR sequences of the VH are set forth by amino acids 31-35, 50-66 and 99-109 of SEQ ID NO:34. The nucleic acid sequences encoding the VL CDR sequences are set forth by nucleic acids 67-108, 154-174 and 271-303 of SEQ ID NO:37; the nucleic acid sequences encoding the VH CDR sequences are set forth by nucleic acids 91-105, 148-198 and 295-328 of SEQ ID NO:38.

***Construction, expression, and production of melanoma specific Fab-PE38KDEL** and **Fab-PE38KDEL:*** The light chains and the heavy chain containing the variable and constant region 1 (V_{L}C_{L} or V_{H}C_{H}) of Fabs 2F1, G2D12, CAG10 and CLA12, were cloned separately by PCR The V_{L}C_{L} PCR fragments were cloned into the expression vector pULI9 for the construction of Fab-PE38KED [Brinkmann et al, J Immunol 150, 2774-82 (1993)]. The resulting plasmids encode the Fab V_{L}C_{L} fused to a gene encoding PE38KDEL. The V_{H}C_{H} of the Fabs were cloned into the same expression vector after the toxin gene was removed. The expression vectors for the Fab-PE38KDEL fusion proteins are driven by the T7 promoter. These constructs were expressed in *Escherichia coli* BL21 λDE3 and accumulated in insoluble intracellular inclusion bodies. The recombinant Fab-PE38KDEL and Fab Fab-PE38KDEL were produced from inclusion bodies by established protocols of solubilization and refolding [Lev et al., Cancer Res 62, 3184-94 (2002)]. Fab-PE38KDEL fusions were purified by ion-exchange chromatography on Q-Sepharose and Mono-Q.

*ELISA **with purified** Fab Abs or **Fab-PE38KDEL immunotoxin:*** The binding specificities of individual soluble Fabs and recombinant Fab-PE38KDEL immunotoxin were determined by ELISA using biotinylated scMHC-peptide complexes. pMHC complexes were refolded using each peptide and coated via streptavidin on an ELISA plate (Falcon). After extensive washing, plates were blocked with PBS/2% skim milk and incubated with various concentrations of soluble purified Fab or Fab-PE38KDEL for 1h at room temperature. Bound clones were detected with an anti-human Fab mAb coupled to HRP or HRP-conjugated anti-PE (for Fab-immunotoxin). Detection was performed using tetramethylbenzidine reagent (Sigma-Aldrich, St. Louis, MO).

*Measurement of* ***melanoma-specific p*/*****HLA-A2-specific** Fabs or Fabs-**PE38KDEL immunotoxin binding** to* ***cell surface peptide*/*MHC complexes:*** RMA-S·HHD or JY cells (10⁶) were pulsed overnight with 50 µM peptide at 26 °C or 37 °C, respectively. RMAS-HHD cells were subsequently incubated at 37 °C for 2―3 h to stabilize cell surface expression of MHC-peptide complexes. The cells were then washed in FACS assay medium (PBS, 2 % BSA, and 0.09 % sodium azide), and incubated for 1 h at 4 °C with 20 µg/ml Fabs or Fab-toxin and FITC-labeled goat anti-human IgG (Fab-specific; The Jackson Laboratory, Bar Harbor, ME). Cells were washed 3 times with PBS and analyzed by FACSCalibur (BD Biosciences, Mountain View, CA). Melanoma cells were trypsinized and stained with the Fab or Fab-toxin as described above. The level of total HLA-A2 expression was detected using the mouse anti-human HLA-A2 (clone BB7.2).

*Fab-toxin **affinity measurement:*** Fab-toxin was labeled with [¹²⁵I] using Bolton-Hunter reagent. ¹²⁵I-labeled Fab-toxin (3-5 x 10⁵ cpm/10⁶ cells) was incubated with JY cells that were loaded with specific or irrelevant peptide and with increasing concentrations of unlabeled Fab-toxin for 1 h at RT. The cells were washed extensively with PBS, and the bound radioactivity was measured by a gamma counter. The apparent binding affinity of the recombinant immunotoxin was determined as the concentration of competitor (soluble purified Fab-toxin) required for 50 % inhibition of ¹²⁵I-labeled Fab-toxin binding to the cells. Nonspecific binding was determined by adding a 20- to 40-fold excess of unlabeled Fab-toxin, and by measuring radioactive on cells loaded with irrelevant peptide.

***Internalization assay:*** JY cells were loaded with specific or control peptide, washed and incubated with 20-30 µg/ml FITC labeled Fab-toxin for 1 h on ice. Cells were then washed and resuspended in RPMI containing 10 % FCS. Half of the cells were kept on ice while the other half was incubated at 37 °C. At indicated time points, a sample was removed, washed, and fixed in Tris/glycerol/polyvinyl alcohol mounting solution. Specimens were examined with a Zeiss confocal laser fluorescence inverted microscope (LSM 410, Carl Zeiss, Oberkochen, Germany) using simultaneous lasers with excitation wavelength 488 nm.

***Cytotoxicity assays on JY APCs and melanoma cell lines:*** JY cells were incubated overnight with 0.1 mM specific peptide or control peptides at 37 °C. Peptide-loaded cells were then washed twice with medium and incubated for 24 h with increasing concentrations of recombinant Fab-PE38KDEL. For melanoma killing assay, 5*10⁴ cells were plated in each well of a flat bottom 96 well plate for 36 h. Graduate amounts of Fab-toxin were then added for an additional 24 h. Protein synthesis inhibition is measured by incorporation of [³H] leucine into cell proteins. IC₅₀ is the concentration of immunotoxin which causes 50 % inhibition of protein synthesis.

***Antitumor activity** (in vivo antitumor assay):* The antitumor activity of Fab-PE38KDEL fusion was determined in SCID mice bearing human cancer cells. Me1526 cells (10 x 10⁶) were injected s.c. into irradiated NOD-SCID β2M deficient mice on day 0. Tumors (about 0.05 cm³ in size) developed in animals by day 10 after tumor implantation. Starting on day 10, animals were treated with i.v. injections of CLA12 Fab-PE38KDEL diluted in 0.2 ml of PBS. Therapy was given once every other day on days 10, 12, and 14; treatment groups consisted of 4 animals. Tumors were measured with a caliper every other day, and the volume of the tumor was calculated by using the following formula: tumor volume (cm³) = length x (width)² x 0.4.

***Statistical Analysis:*** Tumor sizes in animal experiments are expressed as mean ± SD. For comparison between the two experimental groups, Mann-Whitney test was used. P < 0.05 is considered statistically significant.

### Experimental Results

***Isolation** and **characterization** of recombinant antibodies **with T-cell receptor-like specificity** to **melanoma differentiation** antigens **gp100** and* ***MelanA*/*Mart-1*:** Mart1 TCR-like antibodies to the T cell epitope 26-35 (SEQ ID NO:21) were generated and characterized as described in Example 2 herein above and transformed into Fab tetramers and whole IgG molecules as described above.

Specifically, the HLA-A2/Mart-1 complexes were exposed to a large naive repertoire of 3.7x10¹⁰ human recombinant Fab fragments displayed on the surface of phage. A 1000 to-2500-fold enrichment in phage titer was observed after three rounds of panning on the Mart-1-derived peptide-HLA-A2 complex (data not shown). After an initial screen for specificity, two Fab phage clones, CAG10 and CLA12 Abs were selected and produced in a soluble form in *E*. *coli* BL21 cells, then purified by IMAC as described [Lev A. et al., Cancer Res 62, 3184-94, 2002]. SDS-PAGE analysis revealed a homogenous and pure population of Fabs with the expected molecular size (data not shown). The binding specificity of these purified Fab fragments was determined by ELISA with biotinylated MHC-peptide complexes immobilized to wells through BSA-biotin-streptavidin. The correct folding and stability of the bound complexes was determined by their reactivity with the conformational-specific monoclonal antibody W6/32, which only binds correctly folded peptide-containing HLA complexes (data not shown). As shown in Figure 31A, these soluble Fabs show a very specific recognition pattern in ELISA. For example, Fab CLA12 binds only to the Mart-1/HLA-A2 complex but not to complexes displaying other HLA-A2-restriced MHC-peptides (Figure 31A). Further specificity studies were performed on antigen-presenting cells (APCs) that display HLA-A2/Mart-1 (Figures 31B-F). Two APC systems were utilized. The first consists of murine TAP2-deficient RMA-S cells transfected with the human HLA-A2 gene in a single-chain form (HLA-A2.1/Db-β2m single chain) (RMA-S-HHD cells). Mart-1-derived or control peptides were loaded on the RMA-S-HHD cells and the ability of the selected Fab antibodies to bind to peptide-loaded cells was monitored by FACS. Peptide-induced MHC stabilization of the TAP2 mutant RMA-S-HHD cells was determined by analyzing the reactivity of anti-HLA-A2 MAb W6/32. As shown in Figures 31B and 31C, Fabs CAG10 and CLA12 bound in a peptide-specific manner to RMAS-HHD cells that were loaded with the Mart-1 26-35 peptide (as set forth in SEQ ID NO: 21) but not control HTLV-1-derived HLA-A2-restricted peptide (Tax 11-19 peptide) as set forth in SEQ ID NO: 26. The Fabs bound RMAS-HHD cells that were loaded with the anchor modified Mart- 1-derived peptide 27L (SEQ ID NO: 27), where the alanine in position 2 is replaced by a leucine residue, giving stronger binding to HLA-A2 [Rivoltini, L. et al. Cancer Res 59, 301-6 (1999); Valmori, D. et al. J Immunol 160, 1750-8 (1998)]. The Mart-1 26-35, 27L-35, and the Tax peptide were all presented at the same level on the surface of the pulsed RMAS-HHD as demonstrated by the binding of MAb W6/32 to these cells (Figure 31D).

The second type of APCs tested were EBV-transformed B lymphoblast JY cells, which express HLA-A2, and were incubated with the Mart-1-derived or control peptides. These cells are TAP+, and consequently, displaying the exogenous peptide is facilitated by peptide exchange. Using this strategy, a mixture of exogenously and endogenously-derived peptides presented on HLA-A2 are displayed on the cell surface. As shown in Figures 31E and 31F, CAG10 and CLA12 bound JY cells pulsed with the Mart-1 26-35 and modified 27L peptides (Figure 31E), but not JY cells pulsed with 5 control HLA-A2 restricted peptides as indicated (Figure 31F). These results show that Fab CAG10 and CLA12 antibodies exhibit TCR-like fine specificity and can recognize only native HLA-A2 complexes bearing appropriate peptide in situ on the surface of cells. Similar studies were performed previously with Fab-2F1 and Fab-G2D12 which, respectively, target the gp100-derived HLA-A2-restricted epitopes G9-280 (SEQ ID NO: 5) and G9-154 (SEQ ID NO: 6) [Denkberg G et al., Proc Natl Acad Sci U S A 99, 9421-6 (2002)].

***Binding** of **Mart-1 and gp100-specific TCR-like** Fab antibodies to melanoma **cells:*** To test whether the melanoma-specific TCR-like Fab antibodies can bind naturally processed HLA-A2-peptide complexes on the surface of tumor cells, the present inventors performed flow cytometry studies on HLA-A2+ melanoma tumor cell lines (Figures 32A-F and Figures 33A-F). In these cells, the specific peptide/HLA-A2 complex is only represented as a minor fraction out of the total surface HLA-A2 complexes. Mart-1-specific Fab antibody CLA12 reacted with the HLA-A2+/Mart-1+ melanoma lines 501A, 624.38, FM3D, and Stilling (Figures 32A-D), but not with HLA-A2+/Mart-1- melanoma 1938 or with melanoma HA24 cells which is HLA-A2-/Mart-1+ (Figures 32E-F). The expression level of HLA-A2 was monitored by MAb BB7.2. These results indicate that the TCR-like antibody CLA12 can detect the native HLA-A2/Mart-1 epitope on the surface of melanoma cells. Similar studies were performed with TCR-like Fab antibodies which are specific for the gp100-derived epitopes in complex with HLA-A2 [Denkberg G et al., Proc Natl Acad Sci U S A 99, 9421-6 (2002)]. For example, Fab 2F1 directed to the HLA-A2/gp100-G9-280 epitope binds HLA-A2 positive melanoma 624.38 (gp100+) and melanoma 1938 (gp100-) cells pulsed with the G9-280 peptide (SEQ ID NO: 5), but not to G9-280-pulsed PC3 HLA-A2 negative cells (Figures 33A-C). When binding to these cells was tested without pulsing, Fab 2F1 binds to HLA-A2+/gp100+ 624.38 melanoma cells but not to HLA-A2+/gp100- 1938 melanoma nor to HLA-A2-/gp100- PC3 cells (Figure3 33D-F). These results further indicate the capabilities of these unique antibodies to bind in a peptide-dependent, MHC-restricted, manner to target cells which express naturally processed endogenously-derived peptide-MHC complexes.

*Construction and **purification** of **TCR-like antibody-toxin** fusion **proteins:*** To explore the tumor targeting capabilities of the gp100 and MelanA/Mart-1 TCR-like antibodies, fusion molecules were generated in which the Fabs were fused to a form of *Pseudomonas* Exotoxin A (PE38KDEL) [Allured, V. S., et al., Proc Natl Acad Sci U S A 83, 1320-4 (1986)]. This truncated form of PE contains the translocation and ADP-ribosylation domains of whole PE but lacks the cell-binding domain, which is replaced by the Fab fragment fused at the N-terminus of the truncated toxin. In addition, the 5 C-terminal amino acids REDLK of the native PE were replaced with KDEL, which increases cytotoxicity of PE [Seetharam et al., J Biol Chem 266, 17376-81 (1991)] due to efficient binding to the ER retention receptor [Kreitman et al, Biochem J 307 (Pt 1), 29-37 (1995)] where it is translocated to the cytosol and inhibits protein synthesis. The truncated PE38KDEL gene was fused at its N-terminus to the C-terminus of each Fab light chain (CL) as shown schematically in Figure 34A. The Fab-PE38KDEL fusions were expressed in *E. coli* BL21 (λDE3) cells and upon induction with IPTG, large amounts of recombinant protein accumulated as intracellular inclusion bodies. The inclusion bodies were analyzed by SDS-PAGE and those corresponding to the Light chain fused to PE and the Heavy chain each contained more than 90 % recombinant protein (Figure 34B). Using established renaturation protocols Fab-PE38KDEL was refolded from solubilised inclusion bodies in a redox-shuffling refolding buffer and purified by anion-exchange chromatography on Q-Sepharose and Mono-Q columns. Highly purified Fab-PE38KDEL fusion protein with the expected size was obtained as analyzed by SDS-PAGE under reducing and non reducing conditions (Figure 34C lanes 1 and 2, respectively). The yield of the refolded Fab-PE38KDEL fusions was ~ 4 %, thus, 4 mg of highly pure protein could be routinely obtained from the refolding of 100 mg of protein derived from inclusion bodies containing 80-90 % of recombinant protein. This yield is similar to previously reported scFv-immunotoxins that were well-expressed and were produced using a similar expression and refolding system [Denkberg et al., J Immunol 171, 2197-207 (2003)].

The binding specificity of the soluble purified Fab-PE38KDEL fusion proteins was determined by ELISA on biotinylated MHC-peptide complexes immobilized to wells through BSA-biotin-streptavidin to ensure correct folding of the complexes.

As shown in Figure 34D, Fab 2F1-PE38KDEL reacts specifically with the immobilized HLA-A2/gp100-G-280 complexes and not with control HLA-A2-peptide complexes. Detection of binding, as shown, was with anti-human Fab as well with anti-PE38 antibodies to detect the toxin portion of the fusion molecule. Similar results were observed with the other TCR-like fusions recognizing specifically the gp100 G9-154 epitope and the Mart-1 26-35 HLA-A2-peptide complex (not shown).

***Binding of the Fab-PE38KDEL fusions** to **APCs** and **melanoma cells displaying** the **gp100** and **Mart-1-derived** epitopes.* As shown in Figures 32A-F and 33A-F, TCR-like Fabs can bind in an MHC-peptide-dependent manner to peptide-loaded APCs as well as to endogenous peptide/MHC complexes on the surface of melanoma tumor cells. To demonstrate that similar binding can occur with the Fab-PE38KDEL fusion proteins, flow cytometry studies were performed on APCs and melanoma cells. As shown in Figures 35A-D, the Fab-PE38KDEL fusions bound to APCs (T2 or JY cells) pulsed with the appropriate peptide, but did not bind to control pulsed cells. Binding was also tested by flow cytometry with melanoma cells. As shown in Figures 35E-H, the Fab-PE38KDEL fusion proteins bound to HLA-A2+ and gp100/Mart-1+ melanoma cells 526, 501A, and 624.38, but not to 1938 melanoma cells which are HLA-A2+ but gp100/Mart-1-. These results demonstrate the ability of the TCR-like Fab-PE38KDEL fusion molecules to bind the authentic endogenously-derived MHC-peptide complex when at a limited density on the surface of the tumor cells.

Affinities of CLA12 and TA2 to the complexed target was determined by Biacore analysis as shown in Figures 34 E-F (for CLA12 and TA2, respectively) and Tables 7 and 8, herein below (for CLA12 and TA2, respectively).

**Table 7**

| ***Biacore analysis for CLA12*** | | | | | | | | | | |
|---|---|---|---|---|---|---|---|---|---|---|
| | ***Ka (1*/*Ms)*** | ***Kd(1*/*s)*** | ***Rmax (RU)*** | ***R1(RU)*** | ***Conc of analyte*** | ***KA(1*/*M)*** | ***KD(M)*** | ***Req (RU) (1*/*s)*** | ***Kobs*** | ***Chi2*** |
| | 5.18e6 | 2.86e⁻³ | 104 | | | 1.81e⁹ | 5.53e⁻¹⁰ | | | 16.6 |
| D4(1A7-0.025) | | | | 51.7 | 25n | | | 102 | 0.132 | |
| D5(1A7-0.01) | | | | 44.1 | 10n | | | 98.6 | 0.0546 | |
| D6(1A7-0.005) | | | | 25.9 | 5n | | | 93.6 | 0.0287 | |

**Table 8**

| ***Biacore analysis for TA2*** | | | | | | | | | | |
|---|---|---|---|---|---|---|---|---|---|---|
| | ***Ka (1*/*Ms)*** | ***Kd(1*/*s)*** | ***Rmax (RU)*** | ***R1(RU)*** | ***Conc of analyte*** | ***KA(1*/*M)*** | ***KD(M)*** | ***Req (RU)*** | ***Kobs (1*/*s)*** | ***Chi2*** |
| | 5.79e6 | 4.73e⁻³ | 13.6 | | | 1.22e⁹ | 8.18e⁻¹⁰ | | | 8.39 |
| A1(Antibody TA2-0.025) | | | | 16.6 | 25n | | | 13.2 | 0.149 | |
| A2(Antibody TA2-0.01) | | | | 6.98 | 10n | | | 12.6 | 0.0626 | |
| A3(Antibody TA2-0.005) | | | | 14.6 | 5n | | | 11.7 | 0.0337 | |

*Internalization of **TCR-like** antibodies:* An open question regarding the targeting and drug delivery capabilities of TCR-like antibodies is whether these molecules can induce internalization of the MHC-peptide complex. To determine if the TCR-like antibodies internalize, the Fab 2F1-PE38KDEL fusion protein was labeled with FITC and its binding and internalization was tested on JY APCs pulsed with the appropriate gp100-derived G9-280 peptide (SEQ ID NO: 5). As shown in Figure 36A, the FITC-labeled Fab 2F1-PE38KDEL molecule binds specifically to G9-280 peptide-pulsed JY cells but not to cells pulsed with a control peptide G9-209. Internalization was monitored by confocal microscopy (Figures 36B-F). When cells were incubated with FITC-labeled Fab 2F1-PE38KDEL at 4°C membranous binding was observed. Similar membrane binding was observed at 37°C at time 0 immediately after incubation with labeled fusion molecule (Figures 36B, 0 min), and no fluorescence was observed on the negative control (JY cells + peptide G9-209, not shown). At 37°C, internalization of 2F1-PE38KDEL-FITC became visible in JY cells loaded with peptide G9-280 (SEQ ID NO: 5) (Figures 36C-F). After 15 minutes the majority of stain intensity was mainly on the surface of the cell (Figure 36C) and dense areas of fluorescence were detected, which may indicate processes of aggregation or capping of MHC-peptide complexes. After 30 minutes, cells displayed internalizing molecules in small vesicles (Figure 36D). After 1 hour of incubation, the cells internalized more than 50 % of the FITC-labeled antibody, which was detected in larger vesicles (Figure 36E). After 6 hours, intensive staining was observed around the nucleus in the ER-Golgi compartment (Figure 36F). Cells on ice demonstrated mainly or only membrane staining even after 3 hours of incubation (not shown).

These results show that active internalization is induced in cells after binding of TCR-like antibodies to the cell surface and therefore such molecules can deliver toxic agents or drugs into target cells.

***Cytotoxic activity of TCR-like Fab-PE38KDEL fusion toward APCs:*** To determine the ability of the Fab-PE38KDEL to deliver toxin and eliminate cells that express the appropriate peptide/MHC complex, peptide-loaded APCs were used. RMAS-HHD or JY cells were loaded with the gp100-derived epitope G9-280 as well as with other control HLA-A2-restricted peptides. FACS analysis with anti-HLA-A2 antibody revealed similar expression patterns of HLA-A2 molecules with G9-280 (SEQ ID NO: 5), and other control peptide-loaded cells (not shown). The ability of the Fab- PE38KDEL to inhibit protein synthesis was used as a measure of its cytotoxic effect.

As shown in Figure 37A, cytotoxicity by 2F1 Fab-PE38KDEL was observed only on JY cells loaded with the G9-280 peptide (SEQ ID NO: 5) with an IC₅₀ of ~0.5 ng/ml. No cytotoxic activity was observed on JY cells that were loaded with other control HLA-A2-restricted peptides or cells that were not loaded with peptide. Similar results were observed when RMAS-HHD cells were loaded with the G9-280 (SEQ ID NO: 5) and control peptides. The sensitivity of JY cells to immunotoxin is mainly due to the high number of HLA-A2 molecules on the surface of the cells, measured in previous studies of the present inventors to be 1.5-2x10⁵ molecules/cell [Cohen et al., J Immunol 170, 4349-61 (2003)] and the high efficiency of peptide loading. To demonstrate cytotoxic activity towards other cells with limited HLA-A2 expression, melanoma cells FM3D were used, which express 20-fold fewer sites on their surface (1x10⁴ molecules/cell). As controls melanoma G-43 cells were used which are HLA-A2 negative. Both melanomas were pulsed with the G9-280 (SEQ ID NO: 5) and control peptides and exposed to the 2F1 Fab-PE38KDEL fusion molecule. As shown in Figure 37B, 2F1 Fab-PE38KDEL induced killing in FM3D cells pulsed with the G9- 280 peptide (SEQ ID NO: 5), but not with other HLA-A2-restricted control peptides nor with G-43 cells pulsed with G9-280 or control peptides. The IC₅₀ for G9-280-pulsed FM3D was ~200 ng/ml reflecting the lower number of HLA-A2/G9-280 sites compared to G9-280-pulsed JY cells. Thus, a direct correlation between Fab-PE38KDEL cytotoxic activity and the number of target sites per cell was observed. Similar results were observed with the Mart-1-specific CLA12 Fab-PE38KDEL fusion proteins (data not shown). These results demonstrate that Fab-PE38KDEL fusion proteins are very specific agents that, like a T cell receptor, can recognize particular peptide/MHC complexes. Such specificity characteristics are necessary for each new therapeutic candidate antibody that can target drugs or toxins to a defined population of cells that express a particular peptide/MHC class I complexes.

*Cytotoxic **activity of TCR-like Fab-PE38KDEL fusion** molecules toward **melanoma cells expressing endogenous gp100 and Mart-1.*** To evaluate the activity of TCR-like Fab-PE38KDEL fusion molecules to cells which express the natural endogenous target antigen melanoma cells were used that express gp100 and Mart-1 and are HLA-A2 positive. These cells and controls were exposed to increasing concentrations of TCR-like-Fab-toxin. As shown in Figures 38A-C, TCR-like Fab-PE38KDEL fusion proteins exhibited cytotoxic activity on antigen+, HLA-A2+ melanoma cells (526, 501A, and 624.38) but not on HLA-A2-, antigen+ G-43 cells or on HLA-A2+, antigen- 1938 cells. The IC₅₀ of the immunotoxin molecules to antigen and HLA-A2+ cells was 20-100 ng/ml depending on the cell type and the target antigen. 2F1 Fab-PE38KDEL and CLA12 Fab-PE38KDEL were most potent in inducing specific cell killing with an IC₅₀ of 20-30 ng/ml on 526 melanoma cells (Figures 38A and 38C). The present inventors further investigated whether the combined use of two immunotoxins against different epitopes results in increased cell cytotoxicity. Therefore, target melanoma cells were incubated with a mixture of 2F1 Fab-PE38KDEL and CLA12 Fab-PE38KDEL at increasing concentrations as indicated (Figure 38D). As shown, the specificity was not altered and the cytotoxic activity was slightly improved. Detailed analysis on individual target cells, melanomas 526 (Figure 38E) and 624.38 (Figure 38F), revealed that the IC₅₀ of 2F1 Fab-PE38KDEL and CLA12 Fab-PE38KDEL on 526 melanoma cells was 70 and 30 ng/ml, respectively, however their combined effect yielded an IC₅₀ of 20 ng/ml. A more significant combined effect was observed on 634.38 target cells in which 2F1 and CLA12 Fab-PE38KDEL fusion proteins exhibited an IC₅₀ of 200 and 150 ng/ml, respectively, however the combination of them yielded cytotoxic activity with an IC₅₀ of 50 ng/ml. These results indicate the capability of TCR-like antibodies fused to PE38KDEL to induce efficient and specific cytotoxic activity on melanoma target cells that express natural endogenous differentiation antigens gp100 and Mart-1. The use of cocktails of fusion molecules targeting more than one antigen may have a slight beneficial effect on the overall cytotoxic activity as compared to the use of a single agent and likely reflects increased peptide-MHC target density for toxin delivery.

***In vivo anti-tumor activity of TCR-like Fab-PE38KDEL-KDEL fusion proteins:*** Based on the *in vitro* cytotoxic activity of CLA12 Fab-PE38KDEL, the present inventors sought to evaluate its *in vivo* activity. Therefore, the ability of TCR-like Fab-PE38KDEL was assessed to induce regression or inhibition of human melanoma in irradiated NOD SCID β2M-deficient mice. Mice had been inoculated with 10⁷ Mel-526 tumor cells and were randomly assigned to treatment groups, with 4 mice in each group. Mice were administrated with four i.v. doses each of CLA12-PE38KDEL (0.05-0.125 mg/kg), or PBS diluent control. Treatment began on day 11 post inoculation when the tumor size reached approximately 55 mm² and was administrated at 48 h intervals for 8 days. Tumor volumes were recorded for 34 days. By day 34, tumors in mice receiving PBS diluent grew to a size averaging 530 mm³. Treatment with 0.05 mg/kg CLA12-PE38KDEL delayed tumor development compared with control and by day 34 tumor size reached 259 mm³ (Figures 39A-B). The effect on tumor was dose-dependent; since treatment with 0.125 mg/kg CLA12-PE38KDEL delayed tumor development to a greater extent, reaching 25% (135 mm³) of the average size of the tumors in the control group on day 34. Inhibition of tumor growth was statistically significant for any dose of toxin used with P value of less than 0.0006. It can therefore be concluded that CLA12-PE38KDEL is effective in slowing the growth of HLA-A2+Mart-1+ tumors at non-toxic doses.

### Discussion

This example teaches the isolation of 2 novel human recombinant Fab antibodies Fabs-CLA12 and Fab-CAG10. These Fabs recognize peptide derived from the melanoma differentiation antigen Mart-1 (Mart-1₂₆₋₃₇) in the context of HLA-A2.

Studies in mice [Reiter et al, Proc Natl Acad Sci U S A 94, 4631-6 (1997)] demonstrated that a recombinant antibody which recognizes mouse H2-K^{k} class I molecules complexed with the K^{k}-restricted influenza virus-derived peptide of hemagglutinin (peptide Ha₂₅₅₋₂₆₂) can be used very efficiently to deliver a cytotoxic drug or toxin to APCs that express the suitable peptide/MHC complex. The present inventors sought to apply this immunotherapy approach to human diseases such as cancer.

An extended panel of melanoma-specific antibodies with T cell receptor-like specificity were used for targeting toxin to cells that express specific peptide/MHC complex *in vitro* and *in vivo.* 4 different human antibody-toxin hybrid molecules (immunotoxins) were generated, in which a human Fab antibody is fused to the PE derivative, PE38KDEL.

All 4 recombinant immunotoxins that were analyzed had high binding specificity to APCs and melanoma cells expressing the specific peptide/HLA-A2 complex. The immunotoxin 2F1-PE38KEDL has a high rate of internalization (within 15 min) and accumulation in the cytosol of the cell (at 6 h) where it exerts its function. When tested *in vitro*, Fab-PE38KDEL immunotoxins kill APCs and melanoma cells in a peptide-dependent MHC-restricted manner. Immunotoxin G2D12-PE38KDEL was not as potent in killing melanoma cell lines compared to 2F1-PE38KDEL, CLA12-PE38KDEL and CAG10-PE38KDEL, and this order correlates with lower expression of p154/HLA-A2 complexes on the surface of the melanoma cell line. Furthermore, CLA12-PE38KDEL has specific antitumor activity in a mouse xenograft model for human melanoma. It is therefore able to penetrate solid tumor and substantially delay tumor growth in mice at doses that do not produce animal toxicity.

Having constructed immunotoxin against several melanoma epitopes, the present inventors aimed at increasing the probability of eradicating tumor cell variants. As tumor cells tend to mutate, they may lack the target antigen entirely or express it at levels too low for effective immunotoxin-mediated killing. Such mutant cells could be eradicated with cocktails of two or more immunotoxins recognizing different target antigens and this has been demonstrated by targeting lymphoma with a cocktail of anti-CD19 and anti-CD22 ricin-conjugated immunotoxins [Ghetie et al., Blood 80, 2315-20 (1992)]. In the present *in vitro* study, a significantly higher cytotoxic effect was not seen when using more than one immunotoxin. This may probably be due to relatively homogenous expression of the target antigen in melanoma cell lines. However, in patients carrying a large tumor mass, mutations are abundant and using a cocktail of two or more immunotoxins may show a beneficial effect.

The only immunotoxin currently being evaluated for treatment of metastatic melanoma is comprised of ricin A chain conjugated to a murine monoclonal antibody directed against high molecular weight melanoma antigens. This immunotoxin induces toxicities such as myalgia, arthralgia, hypoalbuminemia, fatigue, elevations in liver function tests, and increased peripheral edema. In addition, the use of murine antibody may induce the development of anti-immunotoxin antibodies which will result in decreased efficacy and limit repetitive dosing with an immunotoxin [Gonzalez, R. et al. Mol Biother 3, 192-6 (1991); Oratz, R. et al. J Biol Response Mod 9, 345-54 (1990); Selvaggi, K. et al. J Immunother 13,201-7 (1993)].

Thus the present inventors propose the evaluation of a different class of toxin target for melanoma, the use of a human antibody which is less immunogenic and of smaller size which should penetrate tumor more easily and be processed more efficiently.

The data presented here are a proof of principal that specific MHC complexes can be used in humans as target therapy for melanoma. Moreover, the delivery of TCR-L-PE38KDEL toxin could serve as a first line therapy, to debulk tumor mass and prevent further rigorous growth and is a general approach that can be readily extended to known immunodominant peptides for other HLA [ Krogsgaard, M. et al. J Exp Med 191, 1395-412 (2000); Chames, P., Proc Natl Acad Sci U S A 97, 7969-74 (2000)] and different cancer types.

### EXAMPLE 11

### IDENTIFICATION OF TYROSINASE PEPTIDES

The present inventors ranked potential 8-mer, 9-mer, or 10-mer tyrosinase peptides based on a predicted half-time of dissociation to HLA class I molecules using bioinformatics and molecular tools found on www-bimas.cit.nih.gov/molbio/hla_bind/. Tyrosinase peptides were selected from both the unmodified protein (SEQ ID NO: 67) and unmodified protein (SEQ ID NO: 68) as set forth in Table 139, all of which can be used according to the teachings of the present invention.

### HLA PEPTIDE MOTIF SEARCH RESULTS

**Table 9**

| **User Parameters and Scoring Information** | |
|---|---|
| method selected to limit number of results | cutoff score |
| cutoff score selected | 1 |
| HLA molecule type selected | A1 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 20 |

**Table 10**

| **User Parameters and Scoring Information** | |
|---|---|
| method selected to limit number of results | cutoff score |
| cutoff score selected | 1 |
| HLA molecule type selected | A1 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 26 |

**Table 11**

| **User Parameters and Scoring Information** | |
|---|---|
| method selected to limit number of results | cutoff score |
| cutoff score selected | 1 |
| HLA molecule type selected | A_0201 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 53 |

**Table 12**

| **User Parameters and Scoring Information** | |
|---|---|
| method selected to limit number of results | cutoff score |
| cutoff score selected | 1 |
| HLA molecule type selected | A_0201 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 53 |

**Table 13**

| **User Parameters and Scoring Information** | |
|---|---|
| method selected to limit number of results | cutoff score |
| cutoff score selected | 1 |
| HLA molecule type selected | A_0205 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 34 |

**Table 14**

| **User Parameters and Scoring Information** | |
|---|---|
| method selected to limit number of results | cutoff score |
| cutoff score selected | 1 |
| HLA melecule type selected | A_0205 |
| | |
| oing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 34 |

**Table 15**

| **User Parameters and Scoring Information** | |
|---|---|
| method selected to limit number of results | cutoff score |
| cutoff score selected | 1 |
| HLA molecule type selected | A24 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 87 |

**Table 16**

| **User Parameters and Scoring Information** | |
|---|---|
| method selected to limit number of results | cutoff score |
| cutoff score selected | 1 |
| HLA molecule type selected | A24 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 100 |

**Table 17**

| **User Parameters and Scoring Information** | |
|---|---|
| method selected to limit number of results | cutoff score |
| cutoff score selected | 1 |
| HLA molecule type selected | A3 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 1 521 |
| number of top-scoring subsequences reported back in scoring output | table 25 |

**Table 18**

| **User Parameters and Scoring Information** | |
|---|---|
| method selected to limit number of results | cutoff score |
| cutoff score selected | 1 |
| HLA molecule type selected | A3 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 25 |

**Table 19**

| **User Parameters and Scoring Information** | |
|---|---|
| method selected to limit number of results | cutoff score |
| cutoff score selected | 1 |
| HLA molecule type selected | A68.1 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 39 |

**Table 20**

| **User Parameters and Scoring Information** | |
|---|---|
| method selected to limit number of results | cutoff score |
| cutoff score selected | 1 |
| HLA molecule type selected | A68.1 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 44 |

**Table 21**

| **User Parameters and Scoring Information** | |
|---|---|
| method selected to limit number of results | cutoff score |
| cutoff score selected | 1 |
| HLA molecule type selected | A_1101 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 1 |

**Table 22**

| **User Parameters and Scoring Information** | |
|---|---|
| method selected to limit number of results | cutoff score |
| cutoff score selected | 1 |
| HLA molecule type selected | A_1101 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 3 |

**Table 23**

| **User Parameters and Scoring Information** | |
|---|---|
| method selected to limit number of results | cutoff score |
| cutoff score selected | 1 |
| HLA molecule type selected | A_3101 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 10 |

**Table 24**

| **User Parameters and Scoring Information** | |
|---|---|
| method selected to limit number of results | cutoff score |
| cutoff score selected | 1 |
| HLA molecule type selected | A_3101 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 10 |

**Table 25**

| **User Parameters and Scoring Information** | |
|---|---|
| method selected to limit number of results | cutoff score |
| cutoff score selected | 1 |
| HLA molecule type selected | A_3302 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 32 |

**Table 26**

| **User Parameters and Scoring Information** | |
|---|---|
| method selected to limit number of results | cutoff score |
| cutoff score selected | 1 |
| HLA molecule type selected | A_3302 |
| selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 30 |

**Table 27**

| **User Parameters and Scoring Information** | |
|---|---|
| method selected to limit number of results | cutoff score |
| cutoff score selected | 1 |
| HLA molecule type selected | B14 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 62 |

**Table 28**

| **User Parameters and Scoring Information** | |
|---|---|
| method selected to limit number of results | cutoff score |
| cutoff score selected | 1 |
| HLA molecule type selected | B14 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number oftop-scoring subsequences reported back in scoring output table | 62 |

**Table 29**

| **User Parameters and Scoring Information** | |
|---|---|
| method selected to limit number of results | cutoff score |
| cutoff score selected | 1 |
| HLA molecule type selected | B40 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 52 |

**Table 30**

| **User Parameters and Scoring Information** | |
|---|---|
| method selected to limit number of results | cutoff score |
| cutoff score selected | 1 |
| HLA molecule type selected | B40 |
| length selected for subsequences to be scored | 10 |
| echoing mode selected for input sequence | Y |

| echoing format | numbered lines |
|---|---|
| length of user's input peptide sequence | 529 |
| number of_subsequence scores calculated | 520 |
| number of top-scoring subsequences reported back in scoring output table | 48 |

**Table 31**

| **User Parameters and Scoring Information** | |
|---|---|
| method selected to limit number of results | cutoff score |
| cutoff score selected | 1 |
| HLA molecule type selected | B60 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 47 |

**Table 32**

| **User Parameters and Scoring Information** | |
|---|---|
| method selected to limit number of results | cutoff score |
| cutoff score selected | 1 |
| HLA molecule type selected | B60 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 43 |

**Table 33**

| **User Parameters and Scoring Information** | |
|---|---|
| method selected to limit number of results | cutoff score |
| cutoff score selected | 1 |
| HLA molecule type selected | I B62 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 53 |

**Table 34**

| **User Parameters and Scoring Information** | |
|---|---|
| method selected to limit number of results | cutoff score |
| cutoff score selected | 1 |
| HLA molecule type selected | B62 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 54 |

**Table 35**

| **User Parameters and Scoring Information** | |
|---|---|
| method selected to limit number of results | cutoff score |
| cutoff score selected | 1 |
| HLA molecule type selected | B7 |
| length selected for subsequences to be scored | 9 |
| echoing mode selected for input sequence | Y |

| echoing format | numbered lines |
|---|---|
| length of user's input peptide sequence | 529 |
| - number of subsequence scores calculated | 521 |

| **User Parameters and Scoring Information** | |
|---|---|
| number of top-scoring subsequences reported back in scoring output table | 61 |

**Table 36**

| **User Parameters and Scoring Information** | |
|---|---|
| method selected to limit number of results | cutoffscore |
| cutoffscore selected | 1 |
| HLA molecule type selected | B7 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 59 |

**Table 37**

| **User Parameters and Scoring Information** | |
|---|---|
| method selected to limit number of results | cutoff score |

| cutoff score selected | 1 |
|---|---|
| HLA molecule type selected | B8 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 10 |

**Table 38**

| **User Parameters and Scoring Information** | |
|---|---|
| method selected to limit number of results | cutoff score |
| cutoff score selected | 1 |
| HLA molecule type selected | B8 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 7 |

**Table 39**

| **User Parameters and Scoring Information** | |
|---|---|
| method selected to limit number of results | cutoff score |
| score selected | 1 |
| HLA molecule type selected | B_2702 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 72 |

**Table 40**

| **User Parameters and Scoring Information** | |
|---|---|
| method selected to limit number of results | cutoff score |
| cutoff score selected | 1 |
| HLA molecule type selected | B_2702 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 J |
| number of top-scoring subsequences reported back in scoring output table | 73 |

**Table 41**

| **User Parameters and Scoring Information** | |
|---|---|
| method selected to limit number of results | cutoff score |
| cutoff score selected | 1 |
| HLA molecule type selected | B 2705 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 269 |

**Table 42**

| **User Parameters and Scoring Information** | |
|---|---|
| method selected to limit number of results | cutoff score |
| score selected | 1 |
| HLA molecule type selected | B_2705 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 272 |

**Table 43**

| **User Parameters and Scoring Information** | |
|---|---|
| method selected to limit number of results | cutoff score |
| cutoff score selected | 1 |
| HLA molecule type selected | B_3501 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 84 |

**Table 44**

| **User Parameters and Scoring Information** | |
|---|---|
| method selected to limit number of results | cutoff score |
| cutoff score selected | 1 |
| HLA molecule type selected | B_3501 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 96 |

**Table 45**

| **User Parameters and Scoring Information** | |
|---|---|
| method selected to limit number of results | cutoff score |
| cutoff score selected | 1 |
| HLA molecule type selected | B_3701 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 119 |

**Table 46**

| **User Parameters and Scoring Information** | |
|---|---|
| method selected to limit number of results | cutoff score |
| cutoff score selected | 1 |
| HLA molecule type selected | B_3701 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 109 |

**Table 47**

| **User Parameters and Scoring Information** | |
|---|---|
| method selected to limit number of results | cutoff score |
| cutoff score selected | 1 |
| HLA molecule type selected | B_3801 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 ! |
| number of top-scoring subsequences reported back in scoring output table | 71 |

**Table 48**

| **User Parameters and Scoring Information** | |
|---|---|
| method selected to limit number of results | cutoff score |
| cutoff score selected | 1 |
| HLA molecule type selected | B_3801 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 70 |

**Table 49**

| **User Parameters and Scoring Information** | |
|---|---|
| method selected to limit number of results | cutoff score |
| cutoff score selected | 1 |
| HLA molecule type selected | B_3901 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 91 |

**Table 50**

| **User Parameters and Scoring Information** | |
|---|---|
| method selected to limit number of results | cutoff score |
| cutoff score selected | 1 |
| HLA molecule type selected | B_3901 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 91 |

**Table 51**

| **User Parameters and Scoring Information** | |
|---|---|
| method selected to limit number of results | cutoff score |
| cutoff score selected | 1 |
| HLA molecule type selected | B_3902 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 66 |

**Table 52**

| **User Parameters and Scoring Information** | |
|---|---|
| method selected to limit number of results | cutoff score |
| cutoff score selected | 1 |
| HLA molecule type selected | B_3902 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 70 |

**Table 53**

| **User Parameters and Scoring Information** | |
|---|---|
| method selected to limit number of results | cutoff score |
| cutoff score selected | 1 |
| HLA molecule type selected | B_4403 |
| length selected for subsequences to be scored | 9 |
| echoing mode selected for input sequence | Y |

| echoing fonnat | numbered lines |
|---|---|
| length of user's input peptide sequence | 529 |
| number of subsequence scores calculated | 521 |
| number of top-scoring subsequences reported back in scoring output table | 50 |

**Table 54**

| **User Parameters and Scoring Information** | |
|---|---|
| method selected to limit number of results | cutoff score |
| cutoff score selected | 1 |
| HLA molecule type selected | B 4403 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 57 |

**Table 55**

| **User Parameters and Scoring Information** | |
|---|---|
| method selected to limit number of results | cutoff score |
| cutoff score selected | 1 |
| HLA molecule type selected | B_5101 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 122 |

**Table 56**

| **User Parameters and Scoring Information** | |
|---|---|
| method selected to limit number of results | cutoff score |
| cutoff score selected | 1 |
| HLA molecule type selected | B_5101 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 123 |

**Table 57**

| **User Parameters and Scoring Information** | |
|---|---|
| method selected to limit number of results | cutoff score |
| cutoff score selected | 1 |
| HLA molecule type selected | B_5102 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 127 |

**Table 58**

| **User Parameters and Scoring Information** | |
|---|---|
| method selected to limit number of results | cutoff score |
| cutoff score selected | 1 |
| HLA molecule type selected | B_5102 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 130 |

**Table 59**

| **User Parameters and Scoring Information** | |
|---|---|
| method selected to limit number of results | cutoff score |
| cutoff score selected | 1 |
| HLA molecule type selected | B 5103 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output | 79 |

**Table 60**

| **User Parameters and Scoring Information** | |
|---|---|
| method selected to limit number of results | cutoff score |
| cutoff score selected | 1 |
| HLA molecule type selected | B_5103 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 69 |

**Table 61**

| **User Parameters and Scoring Information** | |
|---|---|
| method selected to limit number of results | cutoff score |
| cutoff score selected | 1 |
| HLA molecule type selected | B_5201 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 91 |

**Table 62**

| **User Parameters and Scoring Information** | |
|---|---|
| method selected to limit number of results | cutoff score |
| cutoff score selected | 1 |
| HLA molecule type selected | B_5201 |
| selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 98 |

**Table 63**

| **User Parameters and Scoring Information** | |
|---|---|
| method selected to limit number of results | cutoff score |
| cutoff score selected | 1 |
| HLA molecule type selected | B_5801 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 50 |

**Table 64**

| **User Parameters and Scoring Information** | |
|---|---|
| method selected to limit number of results | cutoff score |
| cutoff score selected | 1 |
| HLA molecule type selected | B_5801 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 58 |

**Table 65**

| **User Parameters and Scoring Information** | |
|---|---|
| method selected to limit number of results | cutoff score |
| cutoff score selected | 1 |
| HLA molecule type selected | Cw_0301 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 104 |

**Table 66**

| **User Parameters and Scoring Information** | |
|---|---|
| method selected to limit number of results | cutoff score |
| cutoff score selected | 1 |
| HLA molecule type selected | |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 99 |

**Table 67**

| **User Parameters and Scoring Information** | |
|---|---|
| method selected to limit number of results | cutoff score |
| cutoff score selected | 1 |
| HLA molecule type selected | Cw_0401 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 111 |

**Table 68**

| **User Parameters and Scoring Information** | |
|---|---|
| method selected to limit number of results | cutoff score |
| cutoff score selected | 1 |
| HLA molecule type selected | Cw_0401 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 127 |

**Table 69**

| **User Parameters and Scoring Information** | |
|---|---|
| method selected to limit number of results | cutoff score |
| cutoff score selected | 1 |
| HLA molecule type selected | Cw_0602 |
| length selected for subsequences to be scored | 9 |
| echoing mode selected for input sequence | Y |

| echoing format | numbered lines |
|---|---|
| **User Parameters and Scoring Information** | |
| length of user's input peptide sequence | 529 |
| number of subsequence scores calculated | 521 |
| number of top-scoring subsequences reported back in scoring output table | 116 |

**Table 70**

| **User Parameters and Scoring Information** | |
|---|---|
| method selected to limit number of results | cutoff score |
| cutoff score selected | 1 |
| HL,A molecule type selected | Cw_0602 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 113 |

**Table 71**

| **User Parameters and Scoring Information** | |
|---|---|
| method selected to limit number of results | cutoff score |
| cutoff score selected | 1 |
| HLA molecule type selected | Cw_0702 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 88 |

**Table 72**

| **User Parameters and Scoring Information** | |
|---|---|
| method selected to limit number of results | I cutoff score |
| cutoff score selected | 1 |
| HLA molecule type selected | Cw_0702 |
| length selected for subsequences to be scored | 10 |
| echoing mode selected for input sequence | Y |

| echoing format 11 | numbered lines |
|---|---|
| length of user's input peptide sequence | 529 |
| number of subsequence scores calculated, | 520 |
| number of top-scoring subsequences reported back in scoring output table | 85 |

**Table 73**

| **User Parameters and Scoring Information** | |
|---|---|
| method selected to limit number of results | cutoff score |
| cutoff score selected | 1 |
| HLA molecule type selected | A1 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 20 |

**Table 74**

| **User Parameters and Scoring Information** | |
|---|---|
| method selected to limit number of results | cutoff score |
| cutoff score selected | 1 |
| HLA molecule type selected | A1 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 26 |

**Table 75**

| **User Parameters and Scoring Information** | |
|---|---|
| method selected to limit number of results | cutoff score |
| cutoff score selected | 1 |
| HLA molecule type selected | A_0201 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 53 |

**Table 76**

| **User Parameters and Scoring Information** | |
|---|---|
| method selected to limit number of results | cutoff score |
| cutoff score selected | 1 |
| type selected | A_0201 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 53 |

**Table 77**

| **User Parameters and Scoring Information** | |
|---|---|
| method selected to limit number of results | cutoff score |
| cutoff score selected | 1 |
| HLA molecule type selected | A 0205 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 34 |

**Table 78**

| **User Parameters and Scoring Information** | |
|---|---|
| method selected to limit number of results | cutoff score |
| cutoff score selected | 1 |
| HLA molecule type selected | A_0205 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 34 |

**Table 79**

| **User Parameters and Scoring Information** | |
|---|---|
| method selected to limit number of results | cutoff score |
| cutoff score selected | 1 |
| HLA molecule type selected | A24 |
| length selected for subsequences to be scored | 9 |
| echoing mode selected for input sequence | Y |

| echoing format | number lines |
|---|---|
| length of user's input peptide sequence | 529 |
| number of subsequence scores calculated | 521 |
| number of top-scoring subsequences reported back in scoring output table | 87 |

**Table 80**

| **User Parameters and Scoring Information** | |
|---|---|
| method selected to limit number of results | cutoff score |
| cutoff score selected | 1 |
| HLA molecule type selected | A24 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 100 |

**Table 81**

| **User Parameters and Scoring Information** | |
|---|---|
| method selected to limit number of results | cutoff score |
| cutoff score selected | 1 |
| HLA molecule type selected | A3 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 25 |

**Table 82**

| **User Parameters and Scoring Information** | |
|---|---|
| method selected to limit number of results | cutoff score |
| cutoff score selected | 1 |
| HLA molecule type selected | A3 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 25 |

**Table 83**

| **User Parameters and Scoring Information** | |
|---|---|
| method selected to limit number of results | cutoff score |
| cutoff score selected | 1 |
| HLA molecule type selected | A68.1 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 39 |

**Table 84**

| **User Parameters and Scoring Information** | |
|---|---|
| method selected to limit number of results | cutoff score |
| cutoff score selected | 1 |
| HLA molecule type selected | A68.1 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 44 |

**Table 85**

| **User Parameters and Scoring Information** | |
|---|---|
| method selected to limit number of results | cutoff score |
| cutoff score selected | 1 |
| HLA molecule type selected | A_1101 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 1 |

**Table 86**

| **User Parameters and Scoring Information** | |
|---|---|
| method selected to limit number of results | cutoff score |
| cutoff score selected | 1 |
| HLA molecule type selected | A_1101 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 3 |

**Table 87**

| **User Parameters and Scoring Information** | |
|---|---|
| method selected to limit number of results | cutoff score |
| cutoff score selected | 1 |
| HLA molecule type selected | A_3101 |
| length selected for subsequences to be scored | 9 |
| echoing mode selected for input sequence | Y |

| - echoing format | numbered lines |
|---|---|
| length of user's input peptide sequence | 529 |
| number of subsequence scores calculated | 521 |
| number of top-scoring subsequences reported back in scoring output table | 10 |

**Table 88**

| **User Parameters and Scoring Information** | |
|---|---|
| method selected to limit number of results | cutoff score |
| cutoff score selected | 1 |
| HLA molecule type selected | A_3101 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 10 |

**Table 89**

| **User Parameters and Scoring Information** | |
|---|---|
| method selected to limit number of results | cutoff score |
| cutoff score selected | 1 |
| HLA molecule type selected | A_3302 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 32 |

**Table 90**

| **User Parameters and Scoring Information** | |
|---|---|
| method selected to limit number of results | cutoff score |
| cutoff score selected | 1 |
| HLA molecule type selected | A_3302 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 30 |

**Table 91**

| **User Parameters and Scoring Information** | |
|---|---|
| method selected to limit number of results | cutoff score |
| cutoff score selected | 1 |
| HLA molecule type selected B14 | B14 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 62 |

**Table 92**

| U**ser Parameters and Scoring Information** | |
|---|---|
| method selected to limit number of results | cutoff score |
| cutoff score selected | 1 |
| HLA molecule type selected | B14 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 62 |

**Table 93**

| **User Parameters and Scoring Information** | |
|---|---|
| method selected to limit number of results | cutoff score |
| cutoff score selected | 1 |
| HLA molecule type selected | B40 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 52 |

**Table 94**

| **User Parameters and Scoring Information** | |
|---|---|
| method selected to limit number of results | cutoff score |
| cutoff score selected | 1 |
| HLA molecule type selected | B40 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 48 |

**Table 95**

| **User Parameters and Scoring Information** | |
|---|---|
| method selected to limit number of results | cutoff score |
| cutoff score selected | 1 |
| HLA molecule type selected | B60 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 47 |

**Table 96**

| **User Parameters and Scoring Information** | |
|---|---|
| method selected to limit number of results | cutoff score |
| cutoff score selected | 1 |
| HLA molecule type selected | B60 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 43 |

**Table 97**

| **User Parameters and Scoring Information** | |
|---|---|
| method selected to limit number of results | cutoff score |
| cutoff score selected | 1 |
| HLA molecule type selected | B61 |
| length selected for subsequences to be scored | 9 |
| echoing mode selected for input sequence | Y |

| echoing format | numbered lines |
|---|---|
| length of_user's input peptide sequence | 529 |
| number of subsequence scores calculated | 521 |
| number of top-scoring subsequences reported back in *scoring* output table | 32 |

**Table 98**

| **User Parameters and Scoring Information** | |
|---|---|
| method selected to limit number of results | cutoff score |
| cutoff score selected | 1 |
| HLA molecule type selected | B61 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 31 |

**Table 99**

| **User Parameters and Scoring Information** | |
|---|---|
| method selected to limit number of results | cutoff score |
| cutoff score selected | 1 |
| HLA molecule type selected | B62 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| unmber of top-scoring subsequences reported back in scoring output table | 53 |

**Table 100**

| **User Parameters and Scoring Information** | |
|---|---|
| method selected to limit number of results | cutoff score |
| cuto*ff* score selected | 1 |
| HLA molecule type selected | B62 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 5 |

**Table 101**

| **User Parameters and Scoring Information** | |
|---|---|
| method selected to limit number of results | cutoff score |
| cutoff score selected | 1 |
| HLA molecule type selected | B7 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 61 |

**Table 102**

| **User Parameters and Scoring Information** | |
|---|---|
| method selected to limit number of results | cutoff score |
| cutoff score selected | 1 |
| HLA molecule type selected | B7 |
| length selected for subsequences to be scored | 10 |
| echoing mode selected for input sequence | Y |

| echoing format numbered | lines |
|---|---|
| length of user's input peptide sequence | 529 |
| number of subsequence scores calculated | 520 |
| number of top-scoring subsequences reported back in scoring output table | 59 |

**Table 103**

| **User Parameters and Scoring Information** | |
|---|---|
| method selected to limit number of results | cutoff score |
| cutoff score selected | 1 |
| HLA molecule type selected | B8 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 10 |

**Table 104**

| **User Parameters and Scoring Information** | |
|---|---|
| method selected to limit number of results | cutoff score |
| cutoff score selected | 1 |
| HLA molecule type selected | B8 |
| length selected for subsequences to be scored | 10 |
| echoing mode selected for input sequence | Y |

| echoing format | numbered lines! |
|---|---|
| length of user's input peptide sequence | 529 |
| number of subsequence scores calculated | 520 |
| number of top-scoring subsequences reported back in scoring output table | 7 |

**Table 105**

| **User Parameters and Scoring Information** | |
|---|---|
| method selected to limit number of results | cutoff score |
| cutoff score selected | 1 |
| HLA molecule type selected | B_2702 |
| length selected for subsequences to be scored | 9 |
| echoing mode selected for input sequence | Y |

| echoing format | numbered lines |
|---|---|
| length of user's input peptide sequence | 529 |
| numbers of subsequence scores calculated | 521 |
| number of top-scoring subsequences reported back in scoring output table | 72 |

**Table 106**

| **User Parameters and Scoring Information** | |
|---|---|
| method selected to limit number of results | cutoff score |
| cutoff score selected | 1 |
| HLA molecule type selected | B_2702 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoringsubsequences reported back in scoring output table | 73 |

**Table 107**

| **User Parameters and Scoring Information** | |
|---|---|
| method selected to limit number of results | cutoff score |
| cutoff score selected | 1 |
| HLA molecule type selected | B_2705 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 268 |

**Table 108**

| **User Parameters and Scoring Information** | |
|---|---|
| method selected to limit number of results | cutoff score |
| cutoff score selected 1 | 1 |
| HLA molecule type selected | B_2705 |
| length selected for subsequences to be scored 10 | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 272 |

**Table 109**

| **User Parameters and Scoring Information** | |
|---|---|
| method selected to limit number of results | cutoff score |
| cutoff score selected | 1 |
| HLA molecule type selected | B_3501 |
| length selected for subsequences to be scored | 9 |
| echoing mode selected for input sequence | Y |

| ₋echoing format | numbered lines |
|---|---|
| length of user's input peptide sequence | 529 |
| number of subsequence scores calculated | 521 |
| number of top-scoring subsequences reported back in scoring output table | 48 |

**Table 110**

| **User Parameters and Scoring Information** | |
|---|---|
| method selected to limit number of results | cutoff score |
| cutoff score selected | 1 |
| HLA molecule type selected | B_3501 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 96 |

**Table 111**

| **User Parameters and Scoring Information** | |
|---|---|
| method selected to limit number of results | cutoff score |
| cutoff score selected | 1 |
| HLA molecule type selected | B_3701 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 119 |

**Table 112**

| **User Parameters and Scoring Information** | |
|---|---|
| method selected to limit number of results | cutoff score |
| cutoff score selected | 1 |
| HLA molecule type selected | B_3701 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 109 |

**Table 113**

| **User Parameters and Scoring Information** | |
|---|---|
| method selected to limit number of results | cutoff score |
| cutoff score selected 1 | 1 |
| HLA molecule type selected | B_3801 |
| length selected for subsequences to be scored | 9 |
| echoing mode selected for input sequence | Y |

| echoing format_{.} | numbered lines |
|---|---|
| length of user's input peptide sequence | 529 |
| number of subsequence scores calculated | 521 |
| number of top-scoring subsequences reported back in scoring output table | 72 |

**Table 114**

| **User Parameters and Scoring Information** | |
|---|---|
| method selected to limit number of results | cutoff score |
| cutoff score selected | 1 |
| HLA molecule type selected | B_3801 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 69 |

**Table 115**

| **User Parameters and Scoring Information** | |
|---|---|
| method selected to limit number of results | cutoff score |
| cutoff score selected | 1 |
| HLA molecule type selected | B_3901 |
| length selected for subsequences to be scored | 9 |
| echoing mode selected for input sequence | Y |

| choing format | numbered lines |
|---|---|
| length of user's input peptide sequence | 529 |
| number of subsequence scores calculated | 521 |
| number of top-scoring subsequences reported back in scoring output table | 91 |

**Table 116**

| **User Parameters and Scoring** | |
|---|---|
| method selected to limit number of results | cutoff score |
| cutoff score selected | 1 |
| HLA molecule type selected | B_3901 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 89 |

**Table 117**

| **User Parameters and Scoring Information** | |
|---|---|
| method selected to limit number of results | cutoff score |
| cutoff score selected | 1 |
| HLA molecule type selected | B_3902 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 66 |

**Table 118**

| **User Parameters and Scoring Information** | |
|---|---|
| method selected to limit number of results | cutoff score |

| cutoff score selected | 1 |
|---|---|
| HLA molecule type selected | B_3902 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring: subsequences reported back in scoring output table | 70 |

**Table 119**

| **User Parameters and Scoring Information** | |
|---|---|
| method selected to limit number of results | cutoff score |
| cutoff score selected | 1 |
| HLA molecule type selected | B_4403 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 50 |

**Table 120**

| **User Parameters and Scoring Information** | |
|---|---|
| method selected to limit number of results | cutoff score |
| cutoff score selected | 1 |
| HLA molecule type selected | B 4403 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 57 |

**Table 121**

| **User Parameters and Scoring Information** | |
|---|---|
| method selected to limit number of results | cutoff score |
| cutoff score selected | 1 |
| HLA molecule type selected | B_5101 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 122 |

**Table 122**

| **User Parameters and Scoring Information** | |
|---|---|
| method selected to limit number of results | cutoff score |
| cutoff score selected | 1 |
| HLA molecule type selected | B_5101 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 123 |

**Table 123**

| **User Parameters and Scoring Information** | |
|---|---|
| method selected to limit number of results | cutoff score |
| cutoff score selected | 1 |
| HLA molecule type selected | B_5102 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 127 |

**Table 124**

| **User Parameters and Scoring Information** | |
|---|---|
| method selected to limit number of results | cutoff score |
| cutoff score selected | 1 |
| to HLA molecule type selected | B_5102 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 130 |

**Table 125**

| **User Parameters and Scoring Information** | |
|---|---|
| method selected to limit number of results | cutoff score |
| cutoff score selected | 1 |
| HLA molecule type selected | B 5103 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 79 |

**Table 126**

| **User Paramaters and Scoring Information** | |
|---|---|
| method selected to limit number of results | cutoff score |
| cutoff score selected | |
| HLA molecule type selected | B_5103 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 69 |

**Table 127**

| **User Parameters and Scoring Information** | |
|---|---|
| method selected to limit number of results | cutoff score |
| cutoff score selected | 1 |
| HLA molecule type selected | B_5201 |
| length selected for subsequences to be scored | 9 |
| echoing mode selected for input sequence | Y |

| echoing format | numbered lines. |
|---|---|
| length of user's input peptide sequence | 529 |
| number of subsequence scores calculated | 521 |
| number of top-scoring subsequences reported back in scoring output table | 91 |

**Table 128**

| **User Parameters and Scoring Information** | |
|---|---|
| method selected to limit number of results | cutoff score |
| cutoff score selected | 1 |
| HLA molecule type selected | B_5201 |
| length selected for subsequences to be scored | 10 |
| echoing mode selected for input sequence | Y |

| echoing format | numbered lines |
|---|---|
| length of user's input peptide sequence | 529 |

| **User Parameters and Scoring Information** | |
|---|---|
| number of subsequence scores calculated 520 | 520 |
| number of top-scoring subsequences reported back in scoring output table | 98 |

**Table 129**

| **User Parameters and Scoring Information** | |
|---|---|
| method selected to limit number of results | cutoff score |
| cutoff score selected | 1 |
| HLA molecule type selected | B_5801 |
| length selected for subsequences to be scored | 9 |
| echoing mode selected for input sequence | Y |

| echoing format | numbered lines |
|---|---|
| | 529 |
| number of subsequence scores calculated | 521 |
| number of top-scoring subsequences reported back in scoring output table | 50 |

**Table 130**

| **User Parameters and Scoring Information** | |
|---|---|
| method selected to limit number of results | cutoff score |
| cutoff score selected | 1 |
| HLA molecule type selected | B_5801 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 58 |

**Table 131**

| **User Parameters and Scoring Information** | |
|---|---|
| method selected to limit number of results | cutoff score |
| cutoff score selected | 1 |
| HLA molecule type selected | Cw_0301 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 104 |

**Table 132**

| **User Parameters and Scoring Information** | |
|---|---|
| method selected to limit number of results | cutoff score |
| cutoff score selected | 1 |
| HLA molecule type selected | Cw_0301 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 99 |

**Table 133**

| **User Parameters and Scoring Information** | |
|---|---|
| method selected to limit number of results | cutoff score |
| cutoff score selected | 1 |
| HLA molecule type selected | Cw_0401 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 112 |

**Table 134**

| **User Parameters and Scoring Information** | |
|---|---|
| method selected to limit number of results | cutoff score |
| cutoff score selected | 1 |
| HLA molecule type selected | Cw_0401 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 127 |

**Table 135**

| **User Parameters and Scoring Information** | |
|---|---|
| method selected to limit number of results | cutoff score |
| cutoff score selected | 1 |
| HLA molecule type selected | Cw_0602 |
| length selected for subsequences to be scored | 9 |
| echoing mode selected for input sequence | Y |

| echoing format | numbered lines |
|---|---|
| length of user's input peptide sequence | 529 |
| number of subsequence scores calculated | 521 |
| number of top-scoring subsequences reported back in scoring output table | 116 |

**Table 136**

| **User Parameters and Scoring Information** | |
|---|---|
| method selected to limit number of results | cutoff score |
| cutoff score selected | 1 |
| HLA molecule type selected | Cw_0602 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 113 |

**Table 137**

| **User Parameters and Scoring Information** | |
|---|---|
| method selected to limit number of results | cutoff score |
| cutoff score selected | 1 |
| HLA molecule type selected | Cw_0702 |
| length selected for subsequences to be scored | 9 |
| echoing mode selected for input sequence | I Y |

| echoing format | numbered lines |
|---|---|
| length of user's input peptide sequence | 529 |
| number of subsequence scores calculated | 521 |
| number of top-scoring subsequences reported back in scoring output table | 88 |

**Table 138**

| | |
|---|---|
| **method selected to limit number of results** | cutoff score |
| cutoff score selected | 1 |
| HLA molecule type selected | Cw_0702 |
| length selected for subsequences to be scored | 10 |
| echoing mode selected for input sequence | Y |

| echoing format | numbered lines |
|---|---|
| length of user's input peptide sequence | 529 |
| number of subsequence scores calculated | 520 |
| number of top-scoring subsequences reported back in scoring output table | 85 |

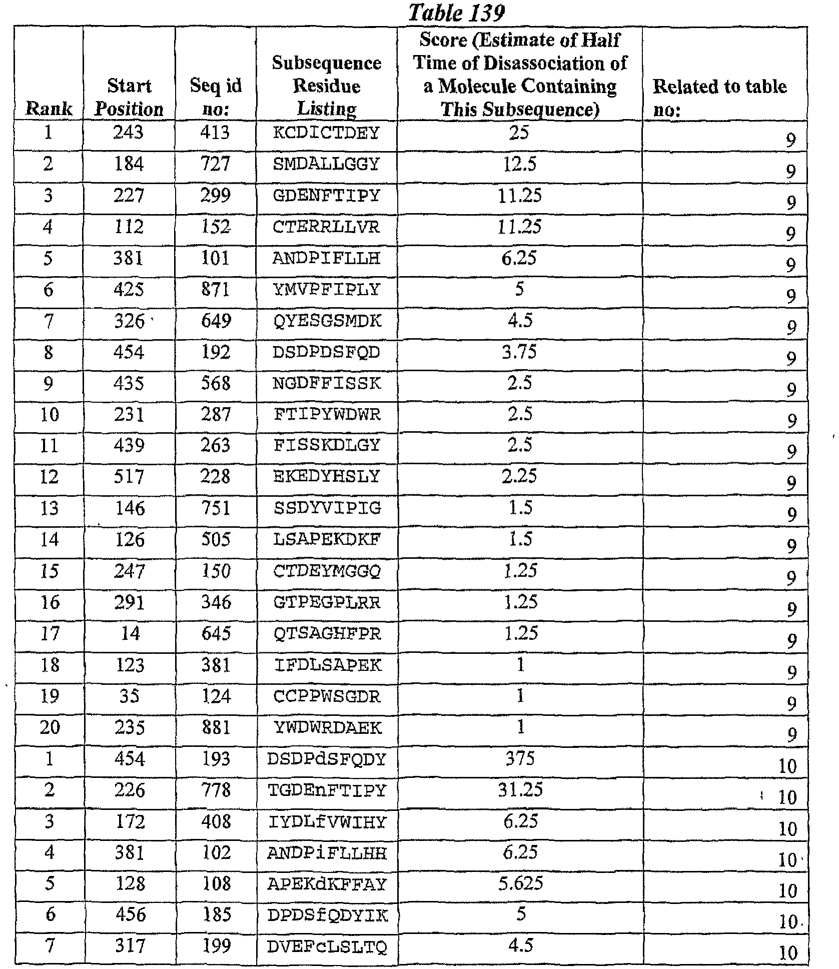

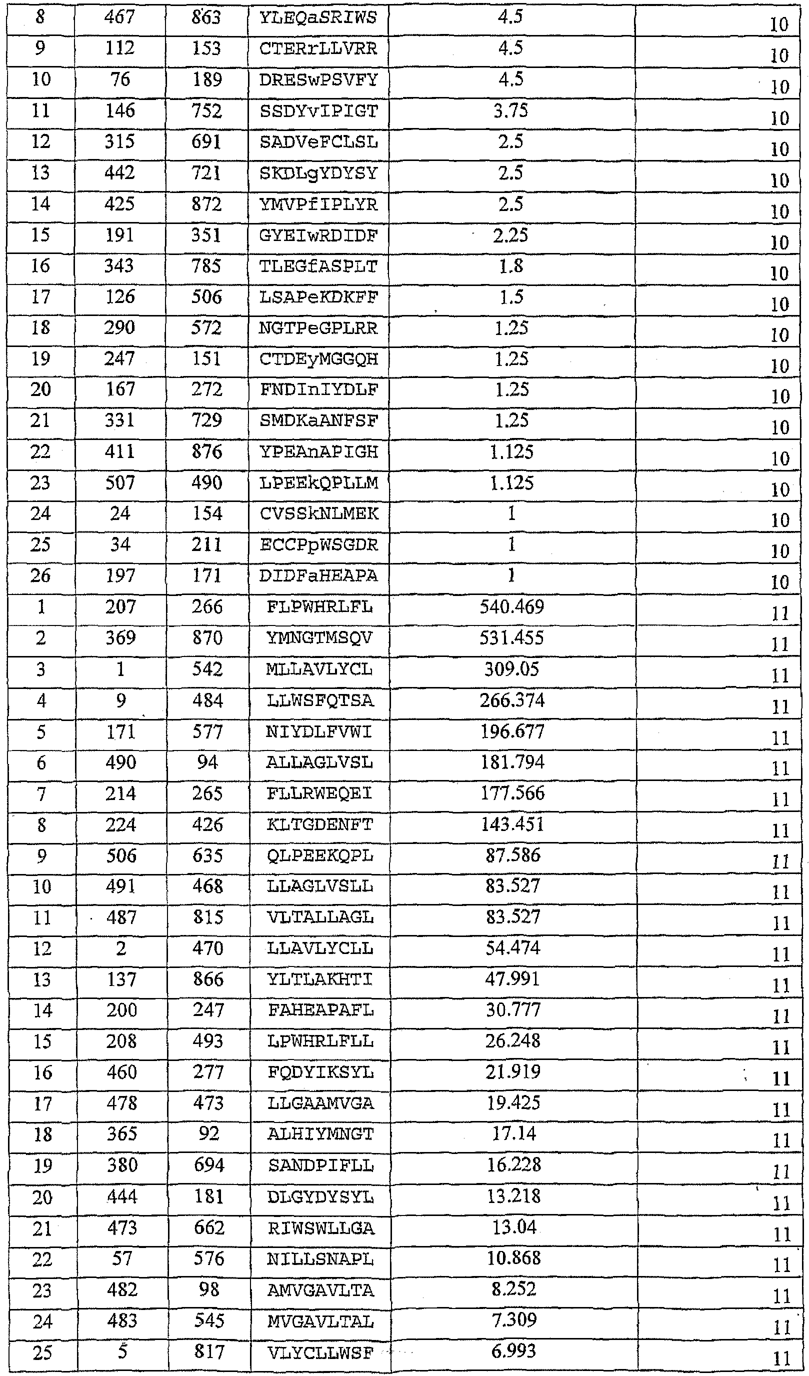

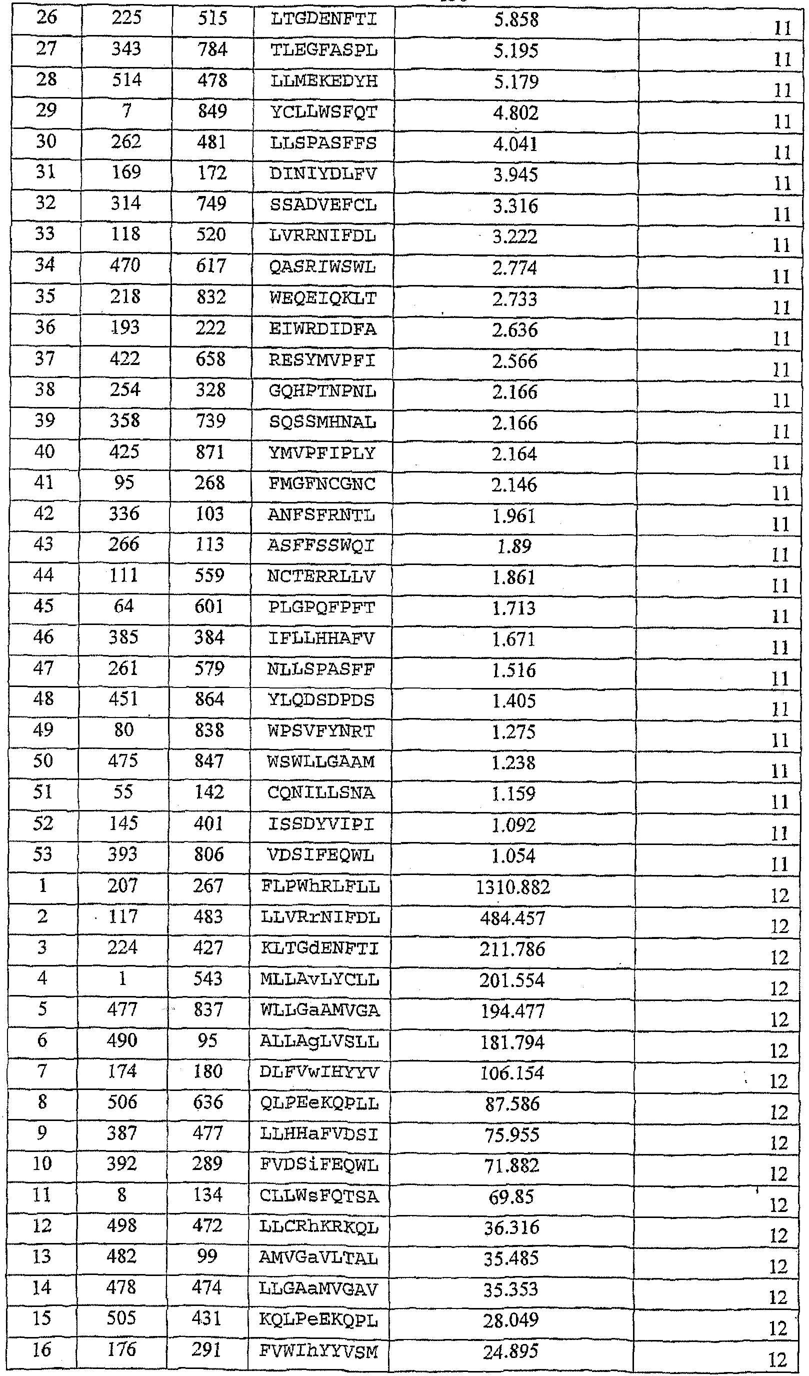

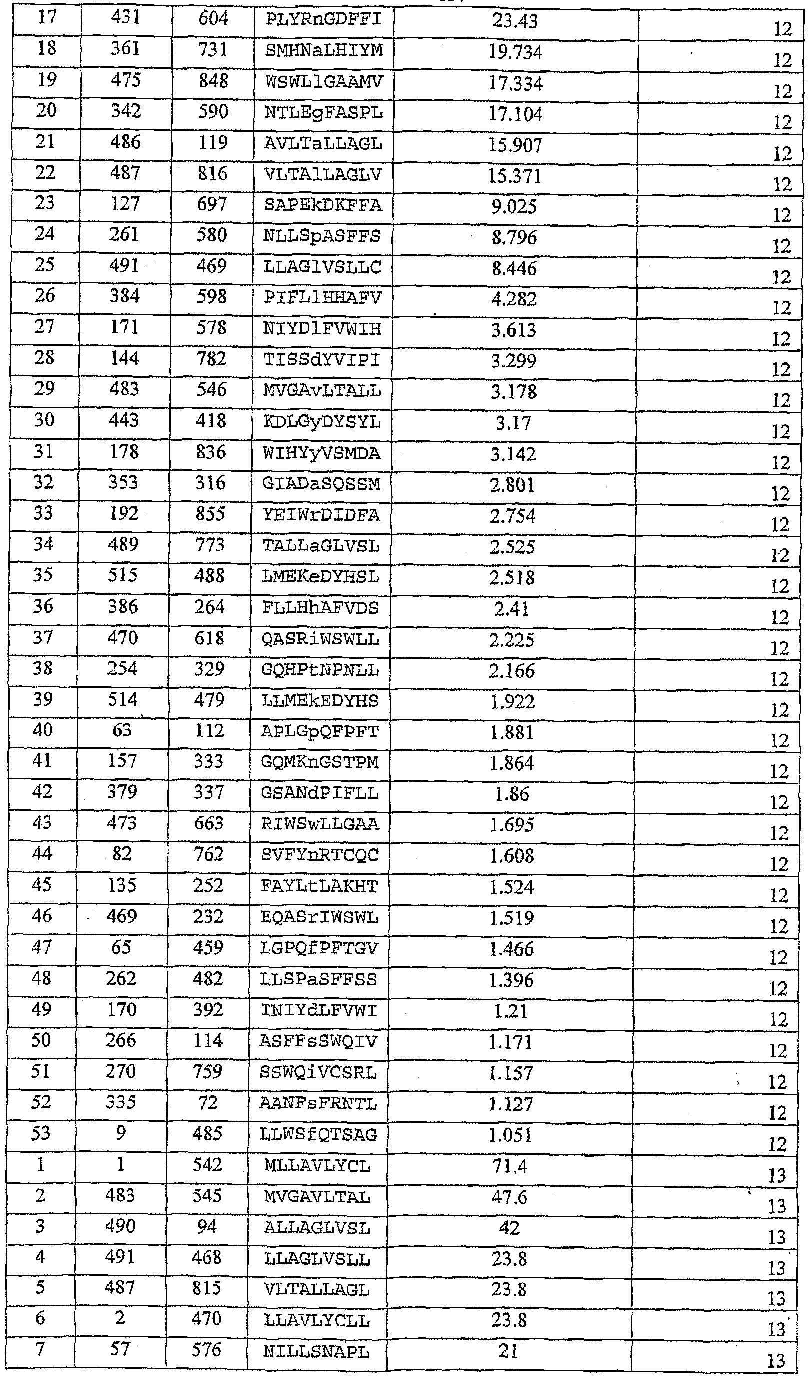

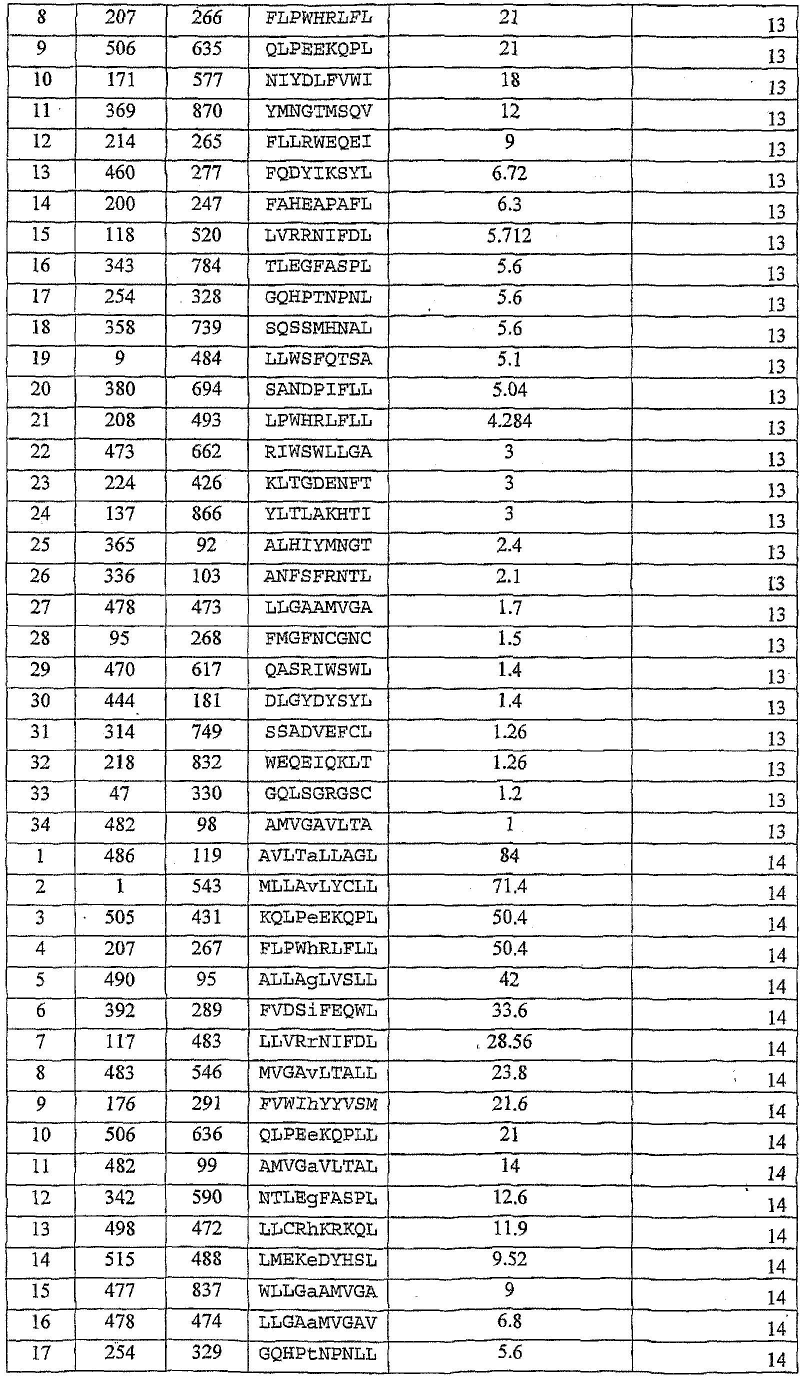

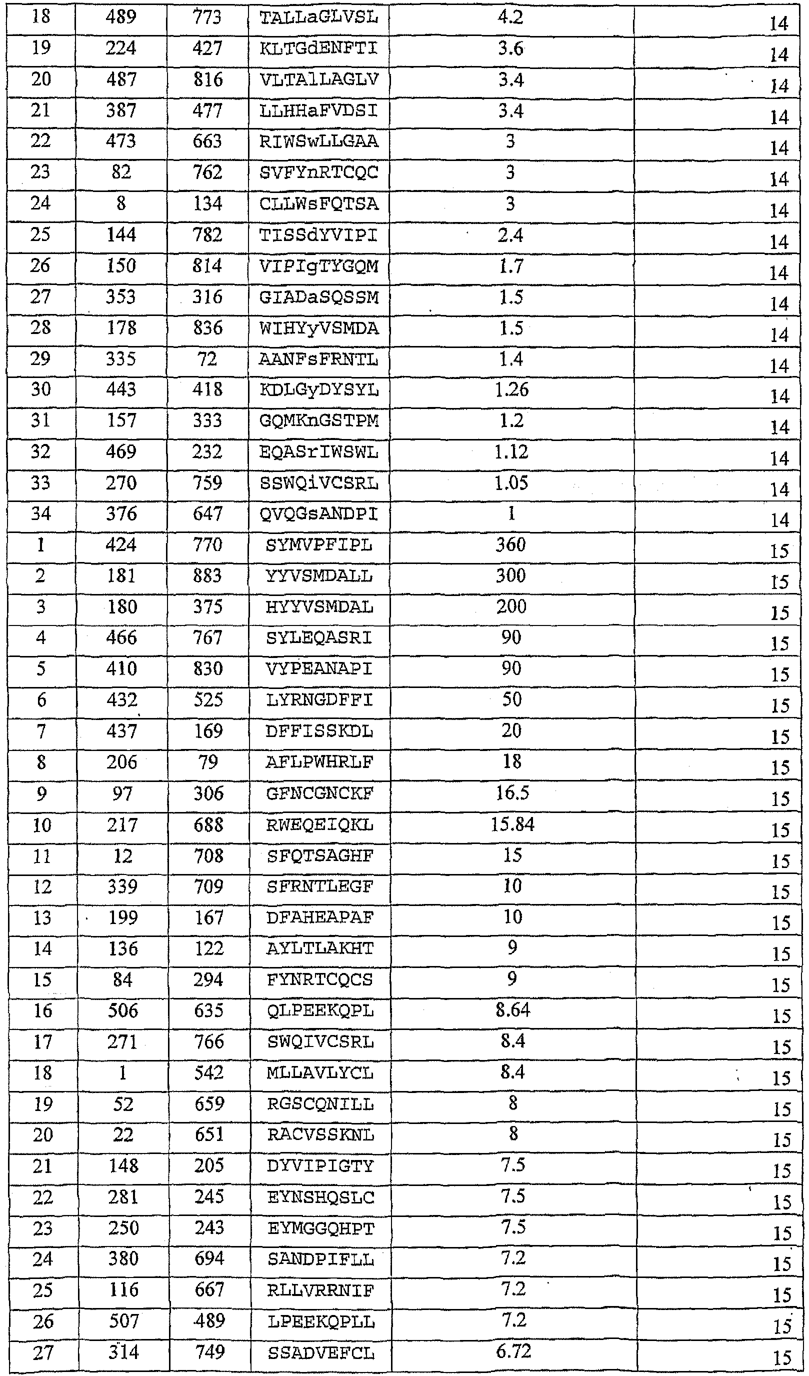

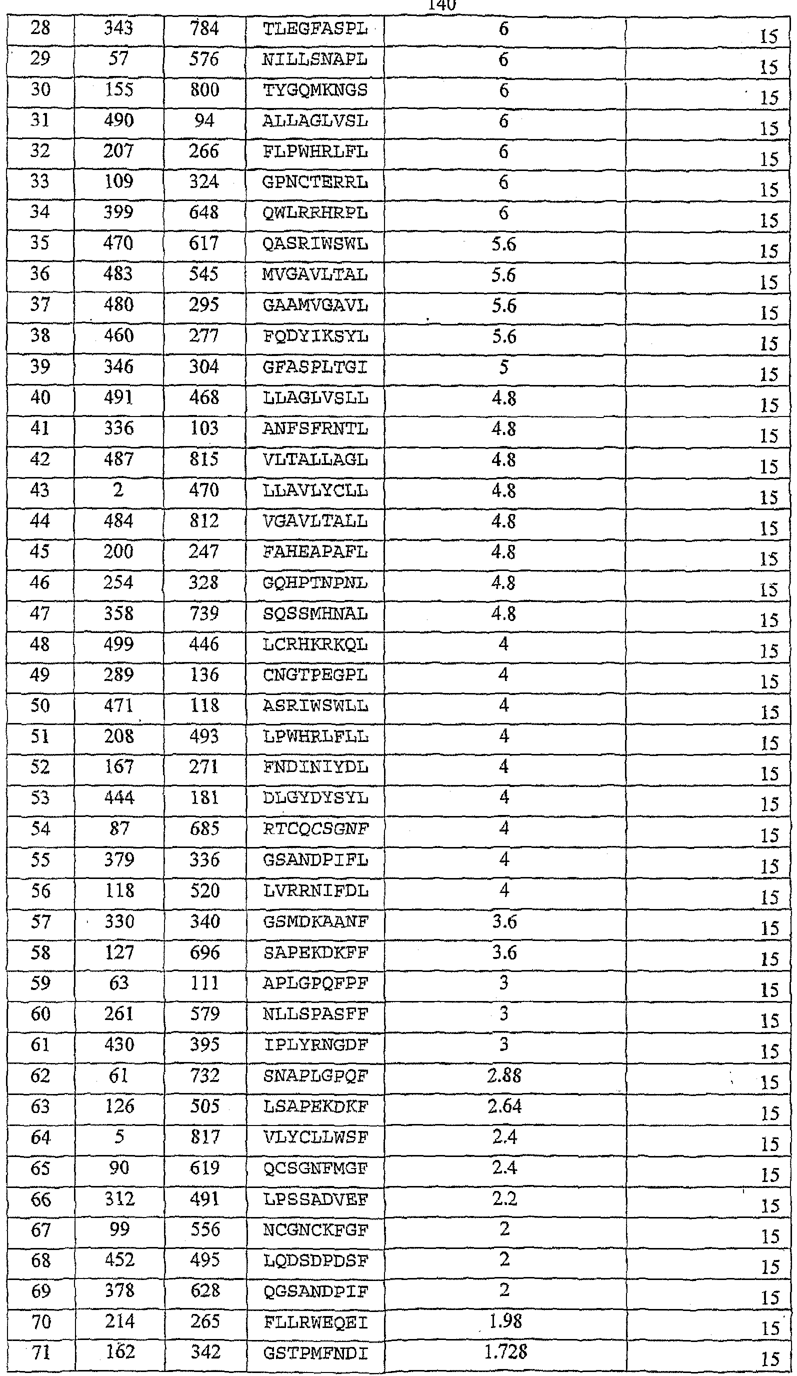

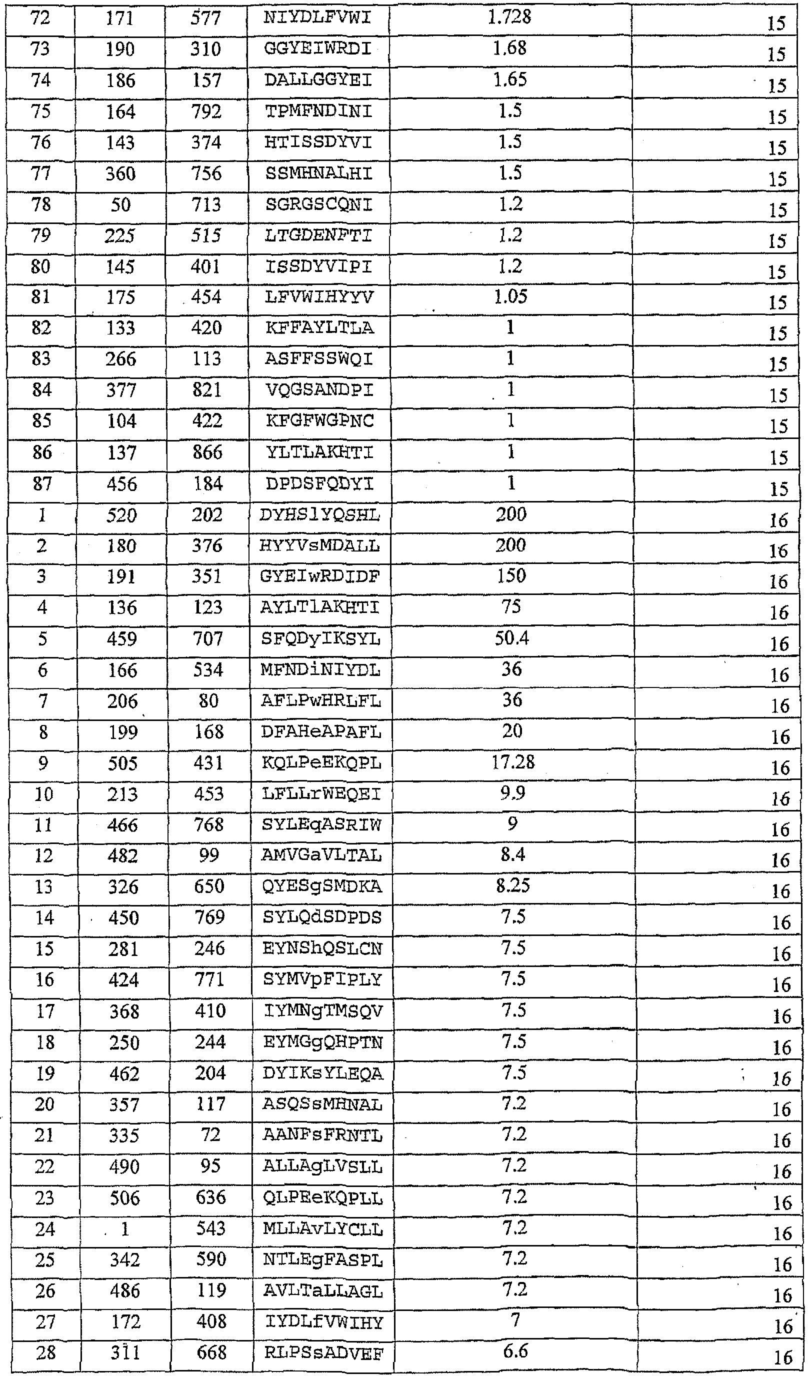

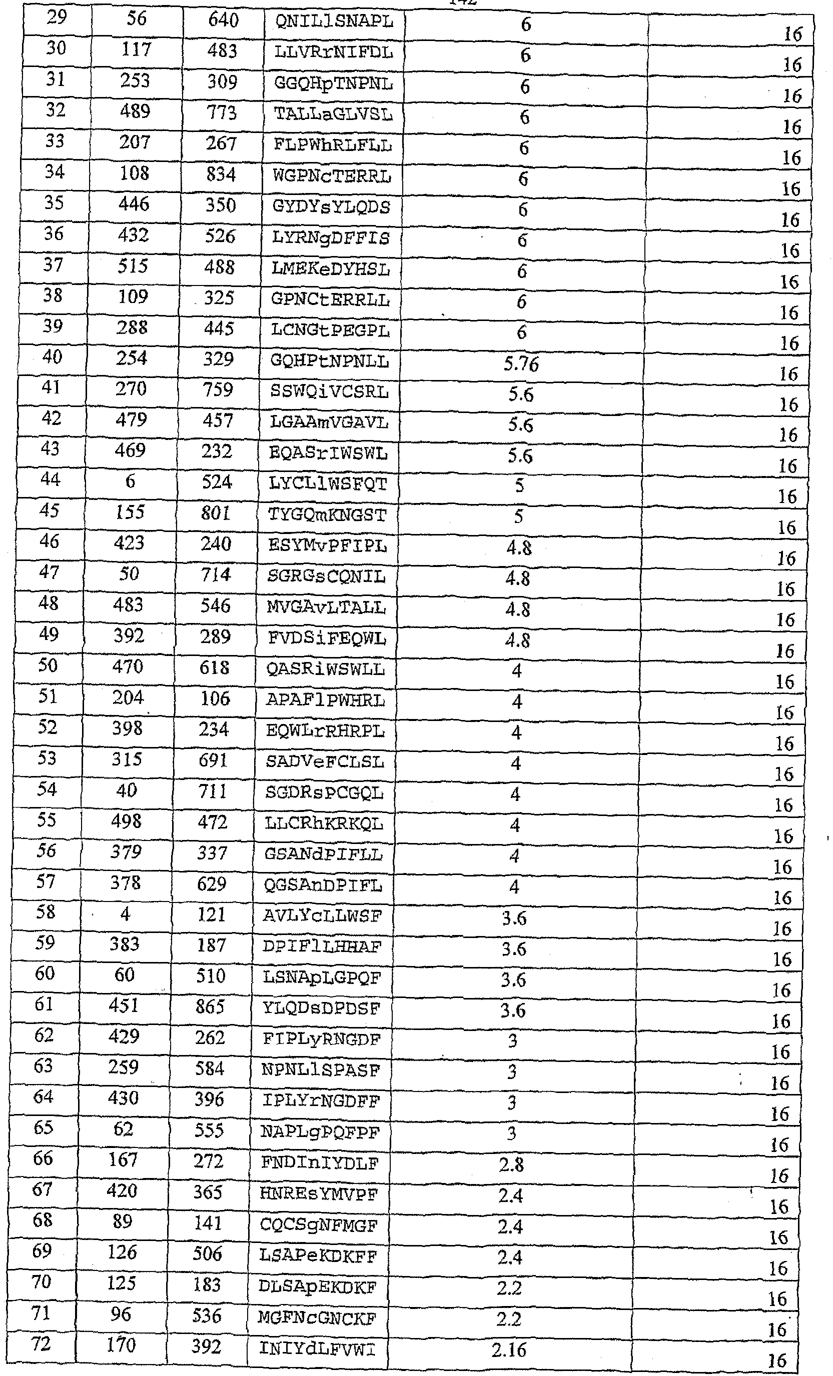

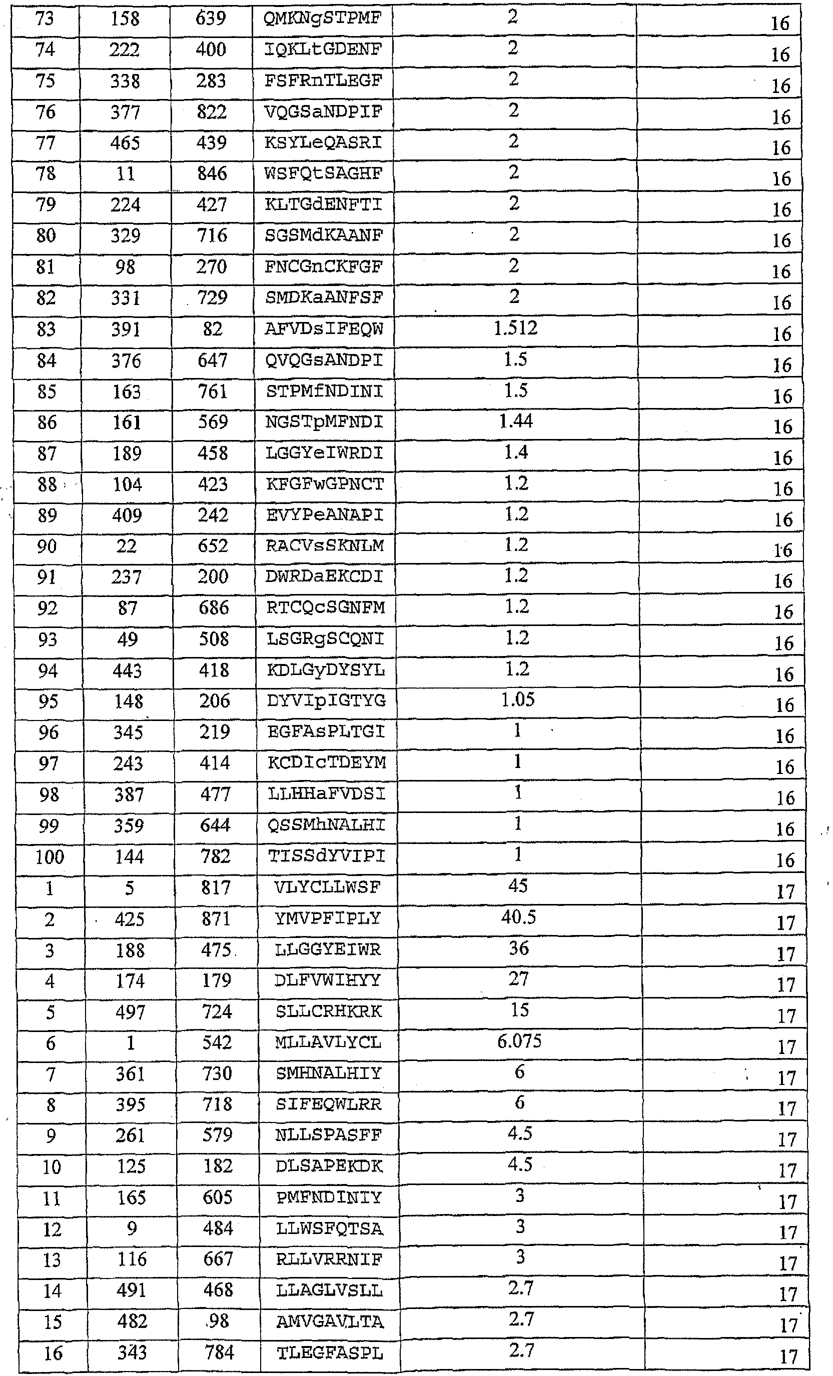

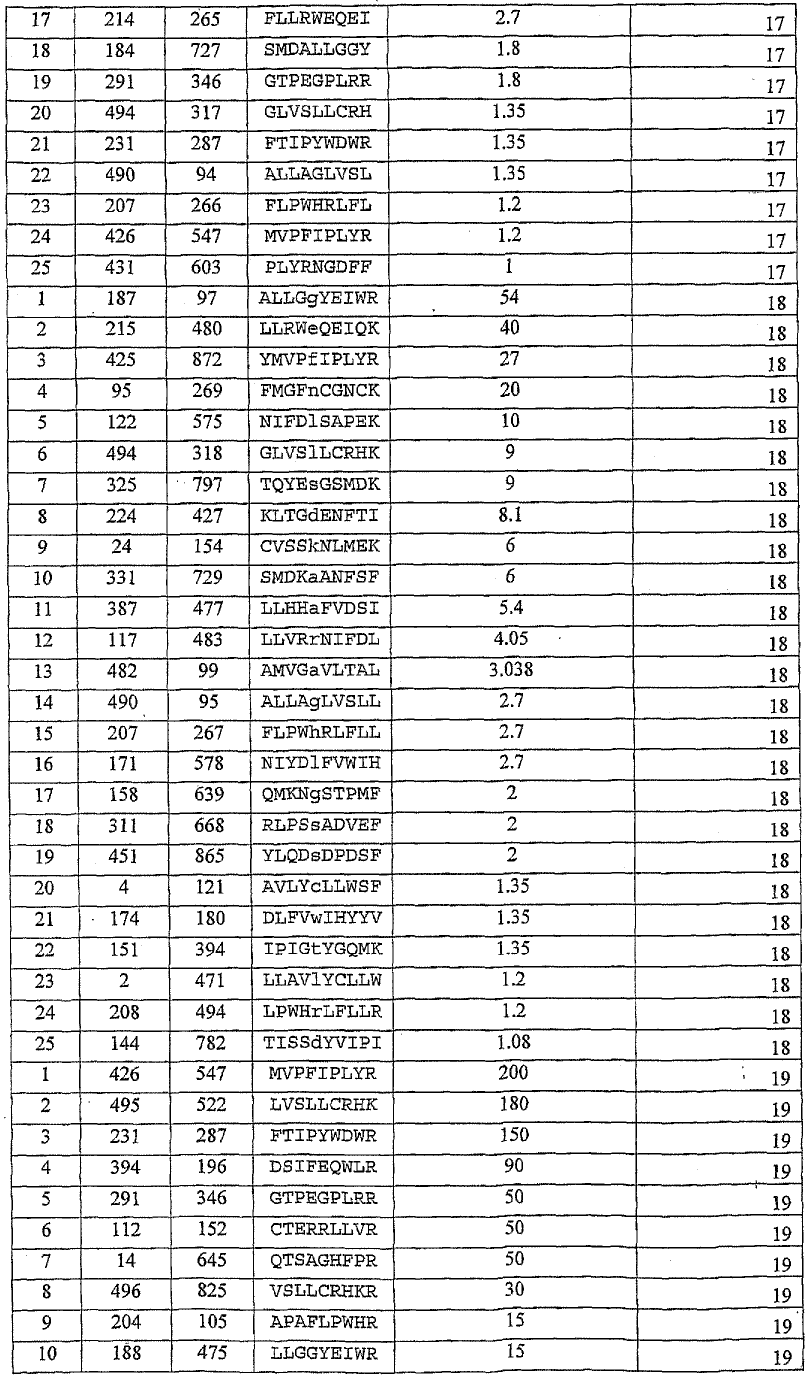

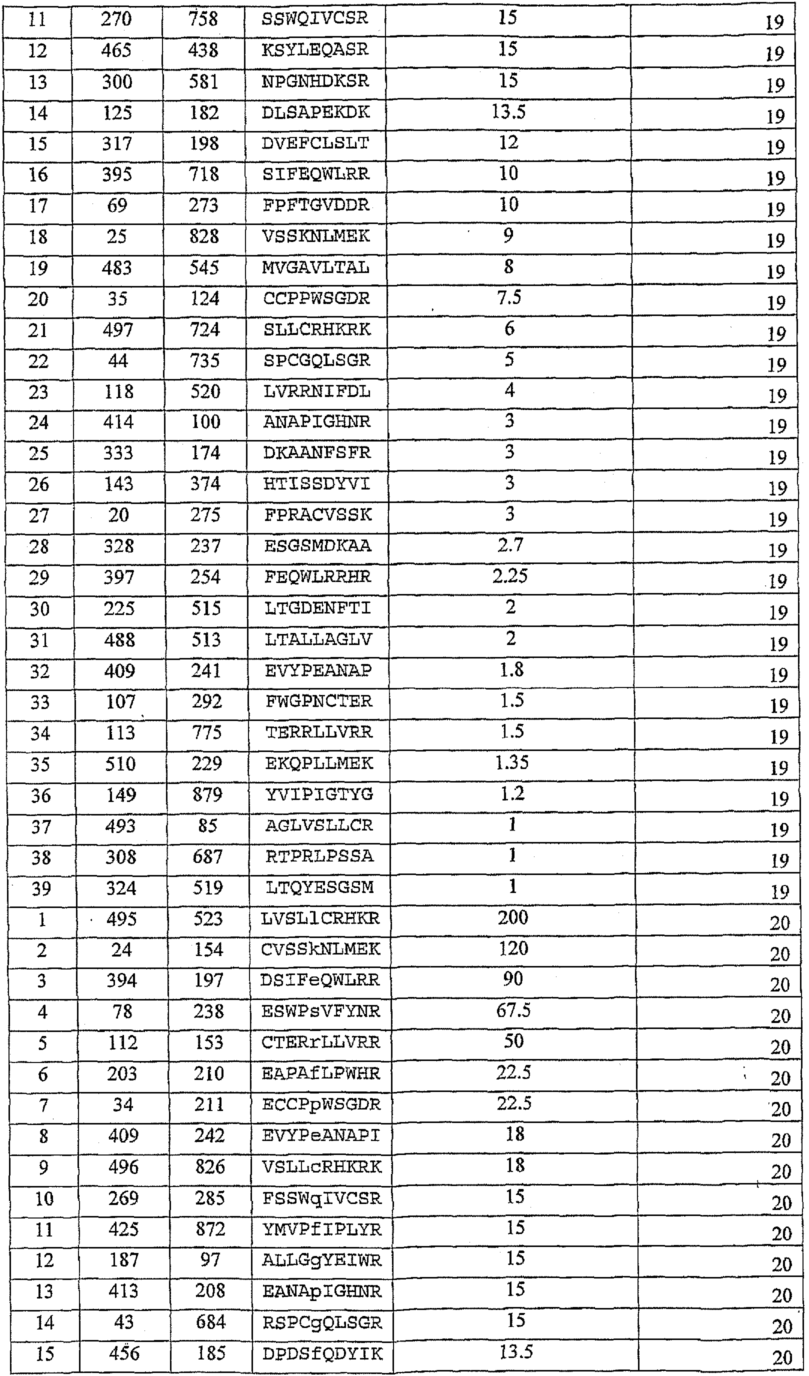

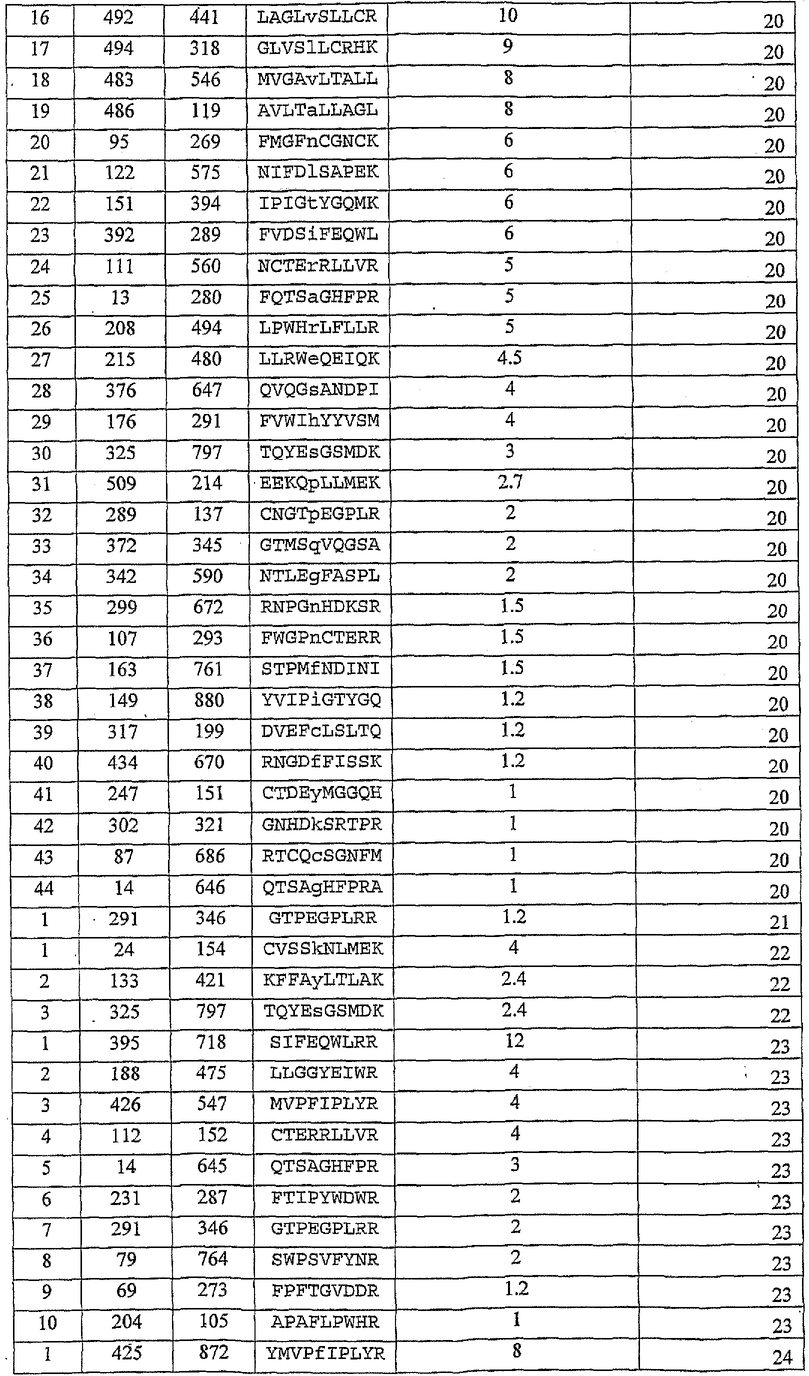

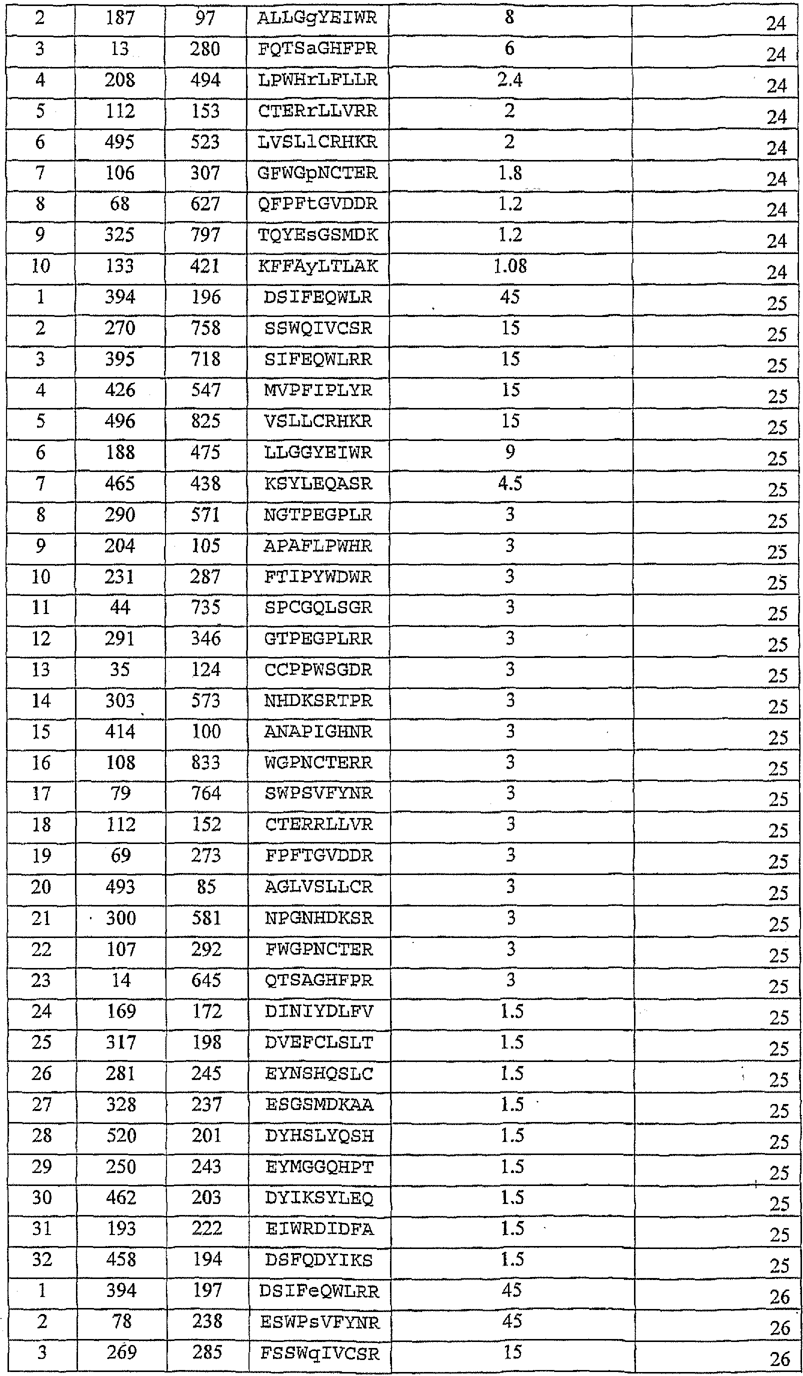

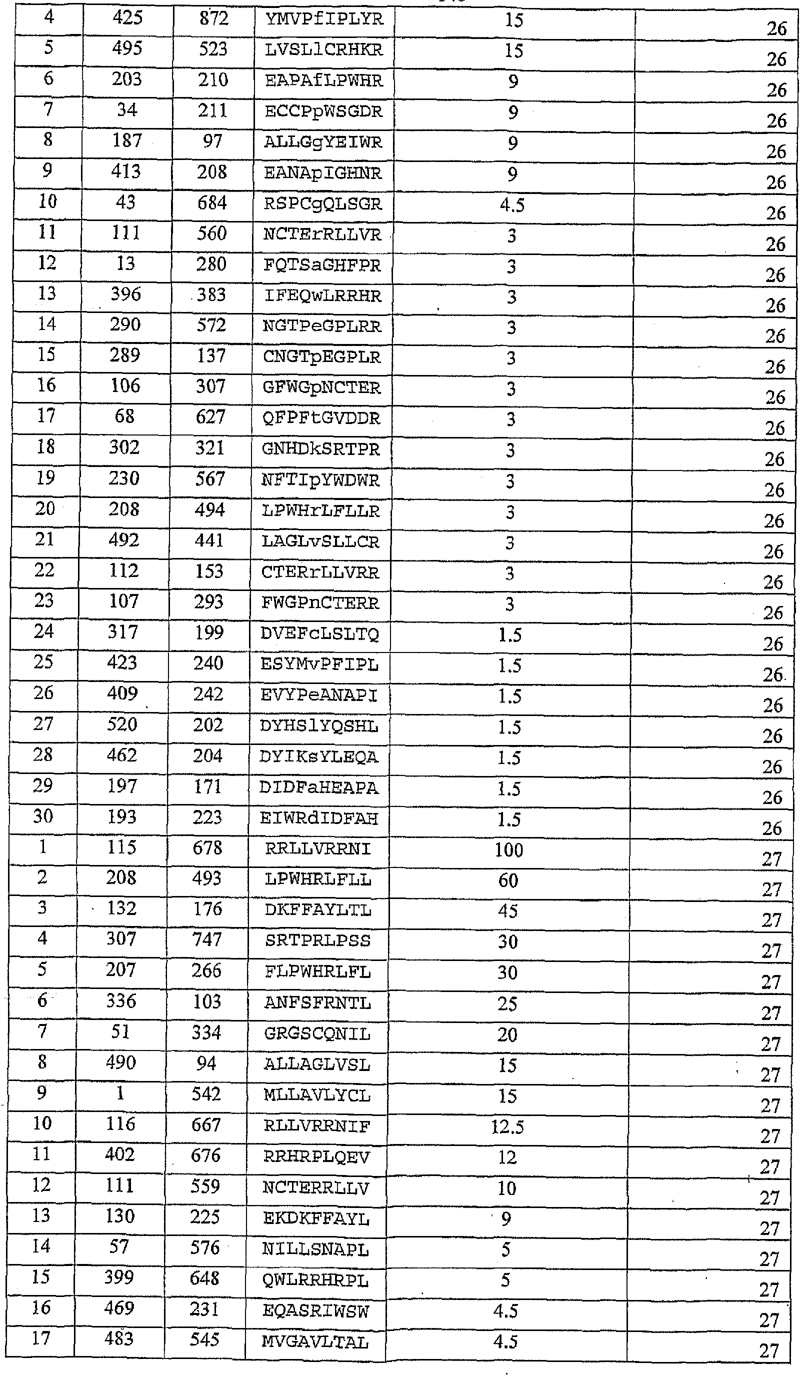

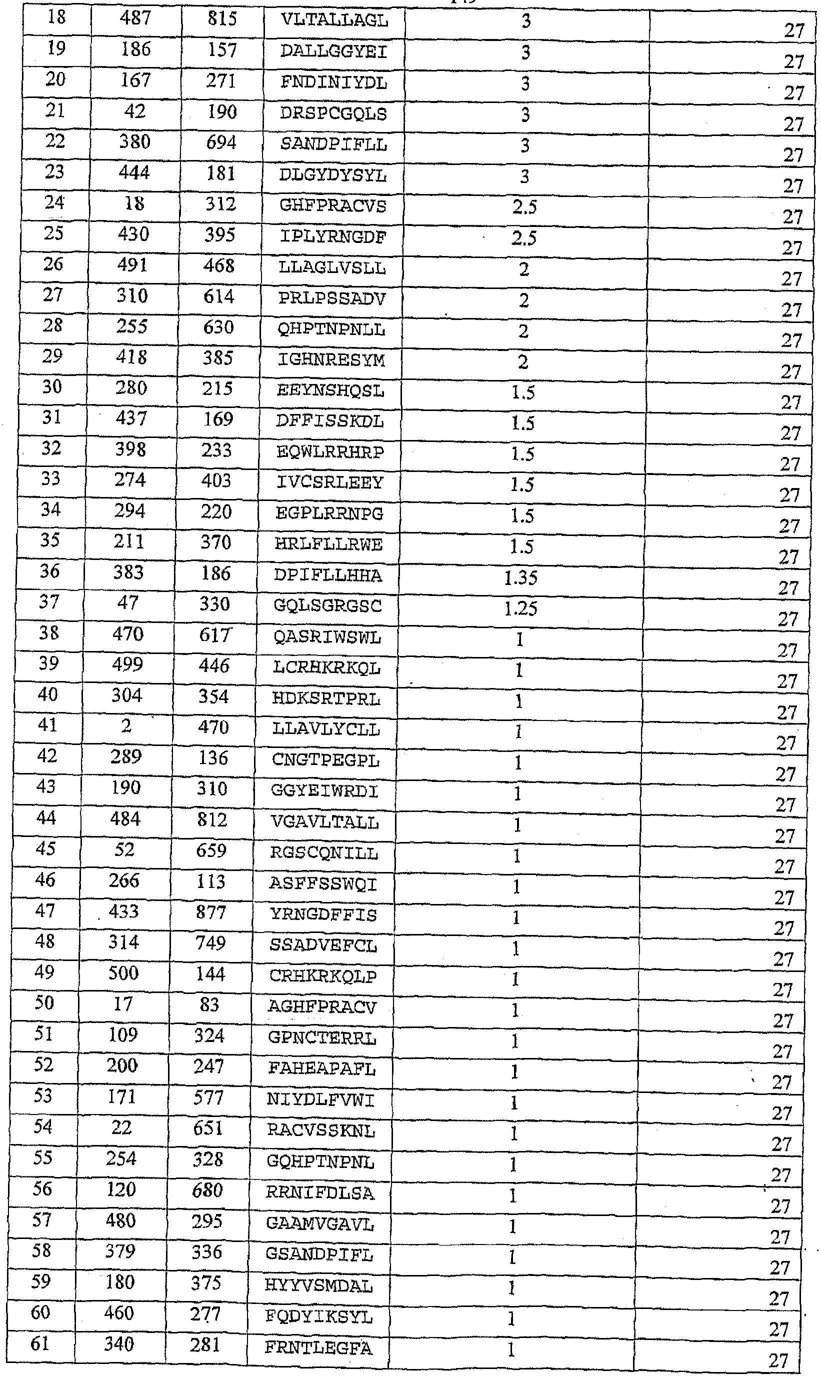

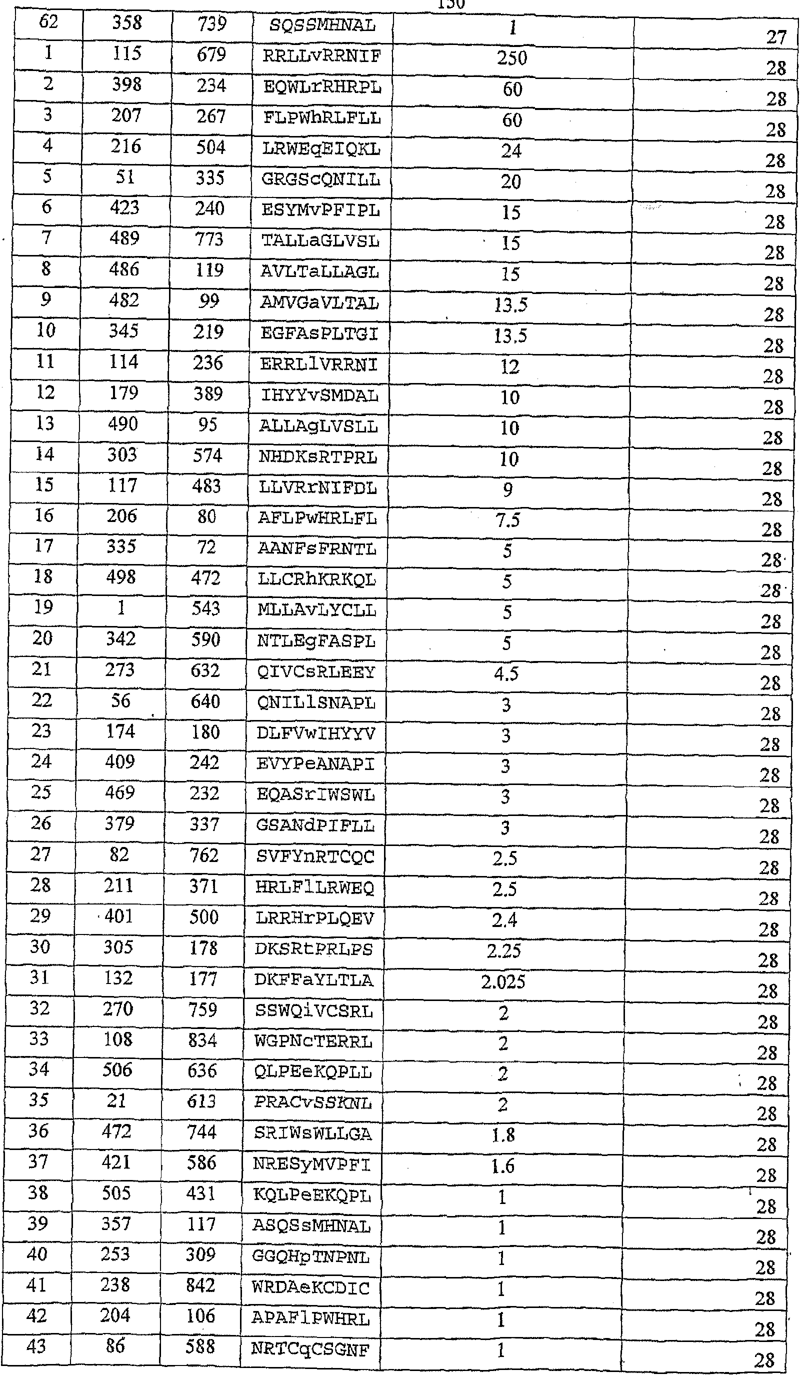

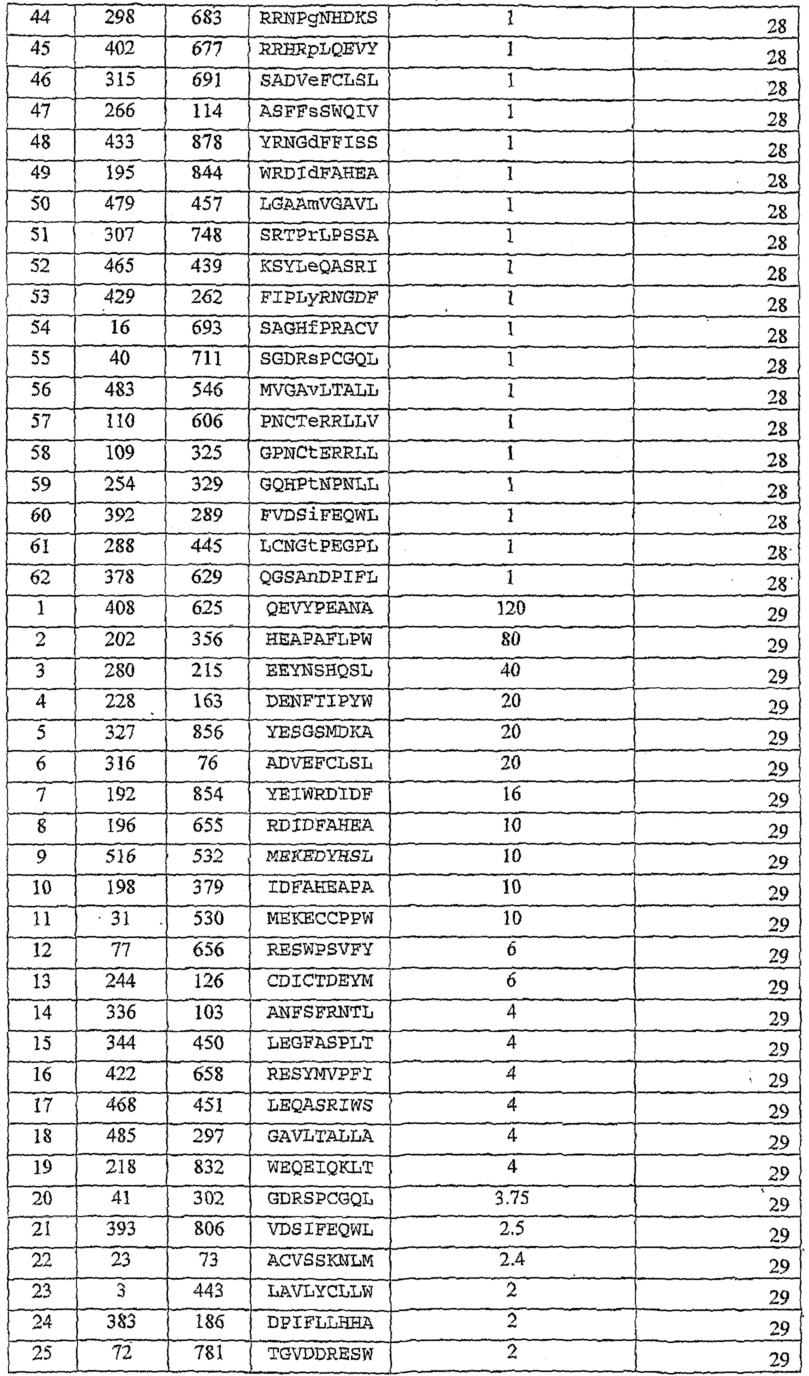

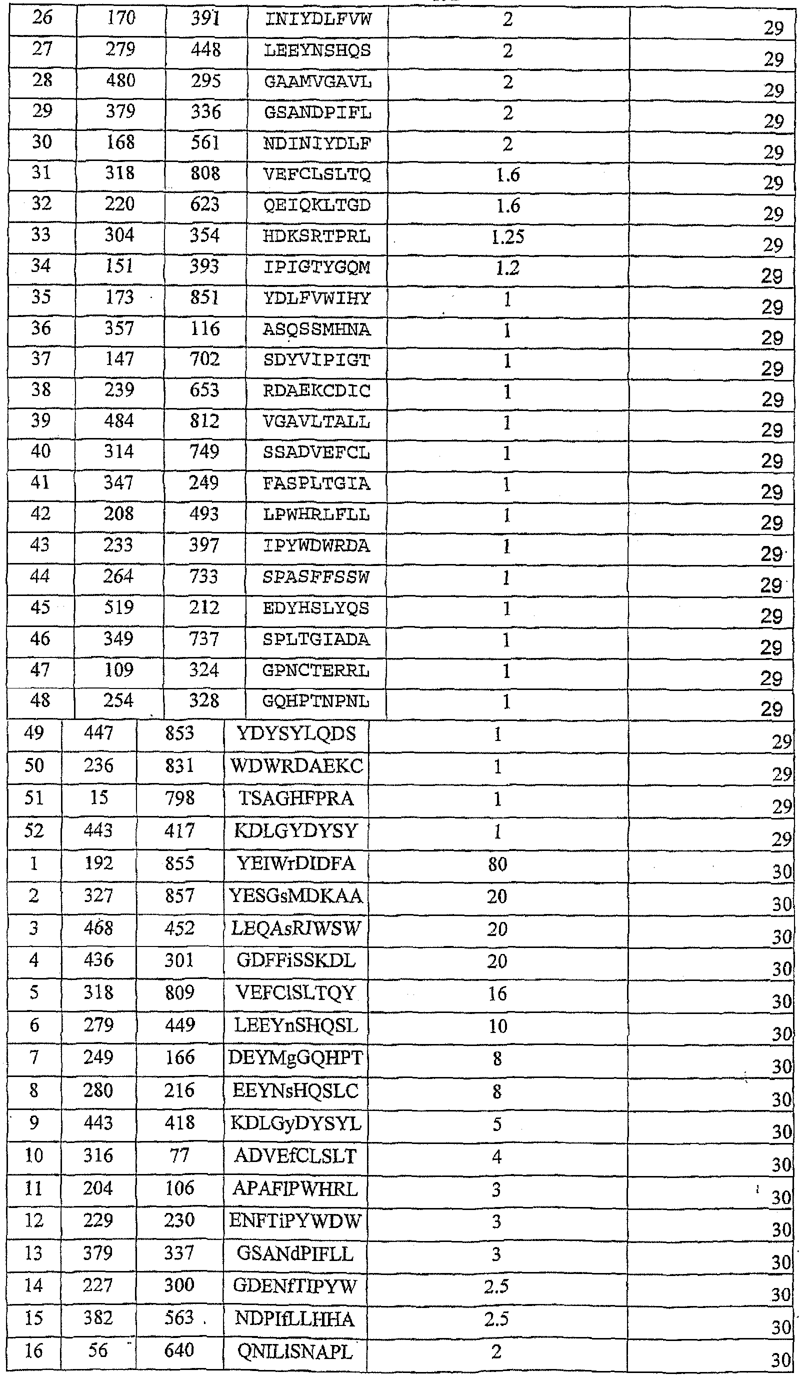

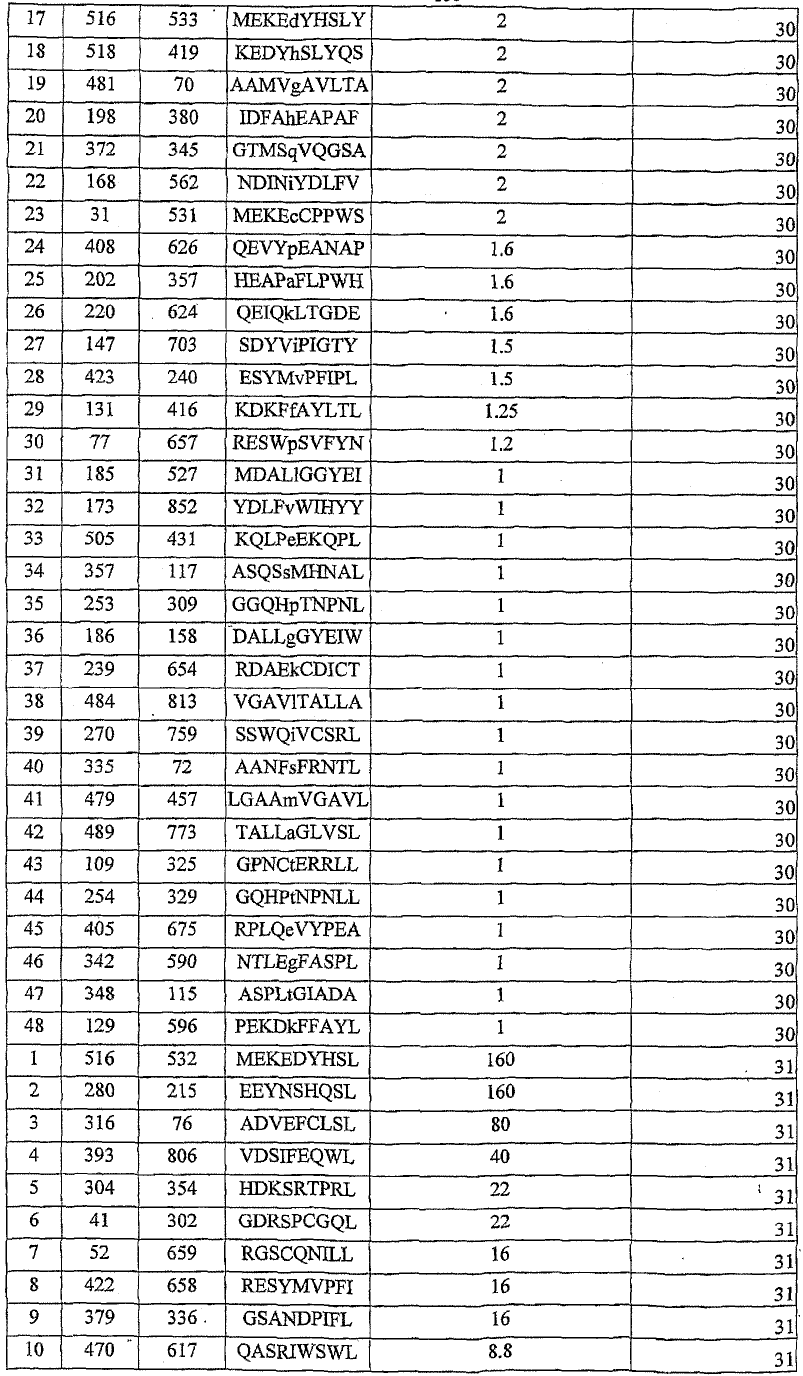

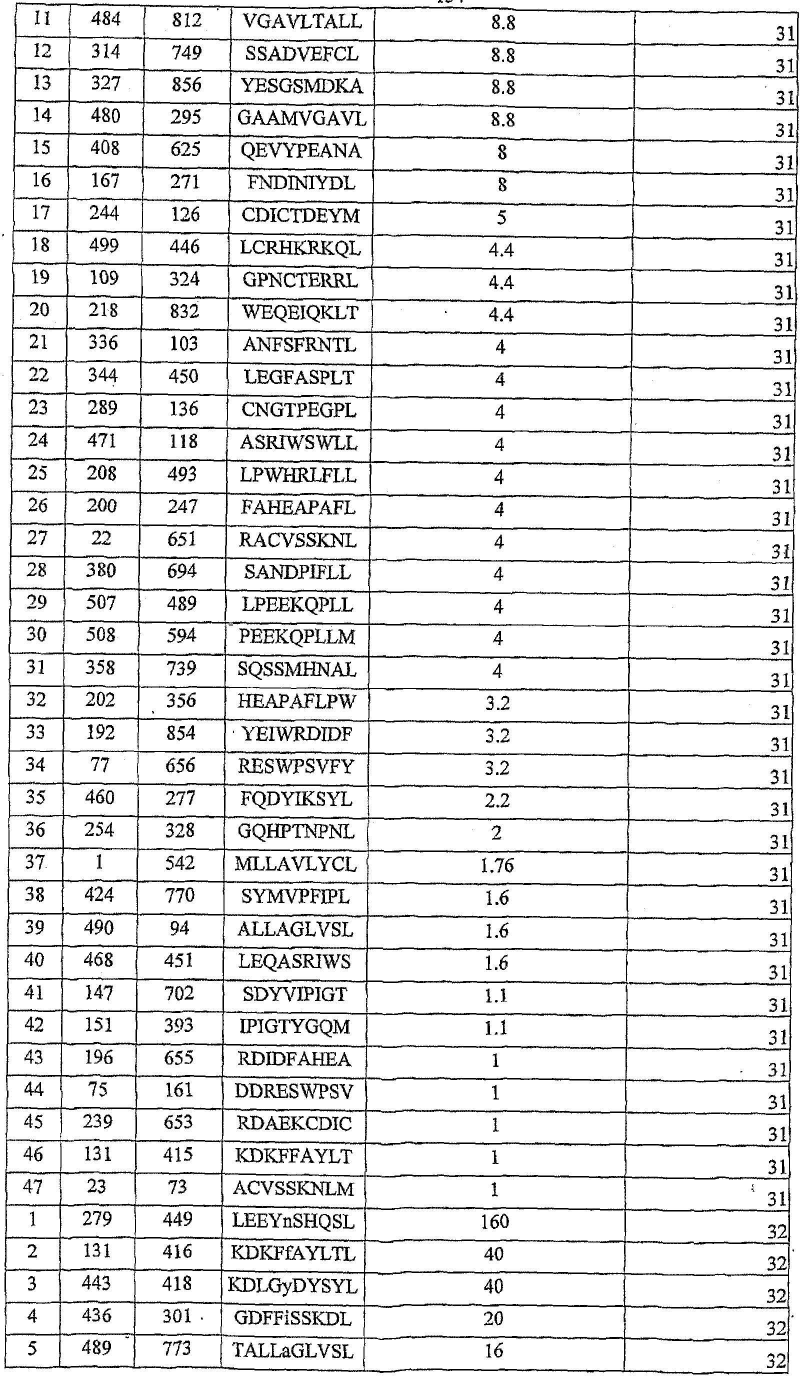

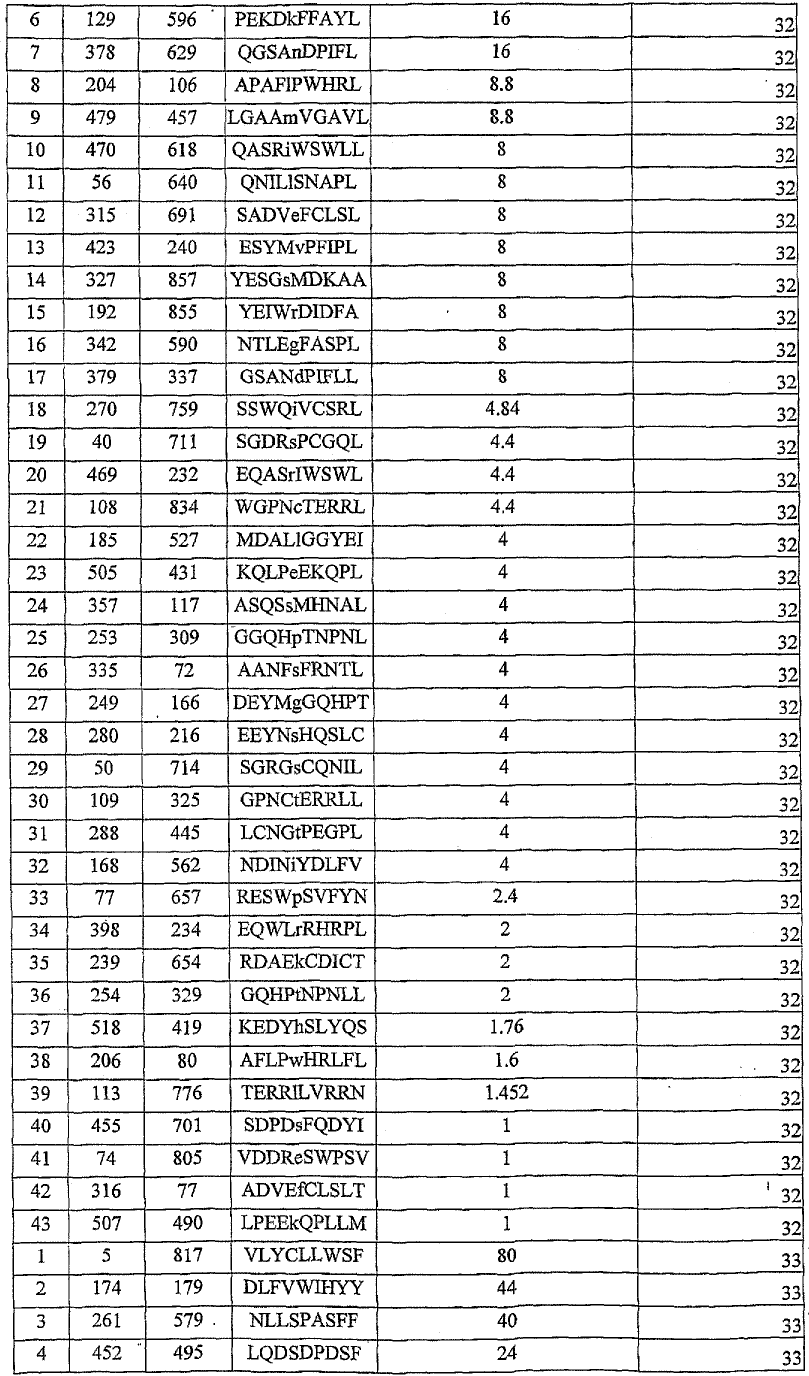

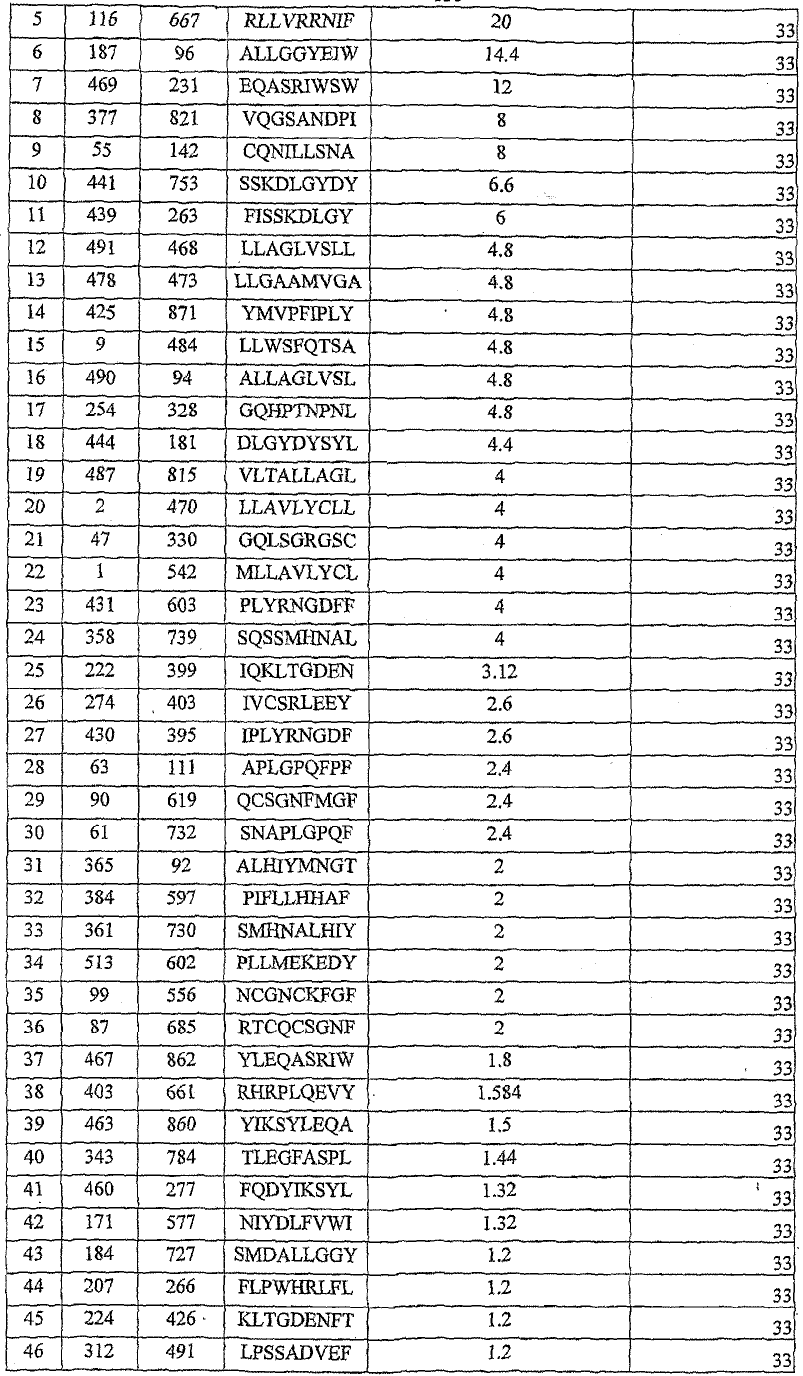

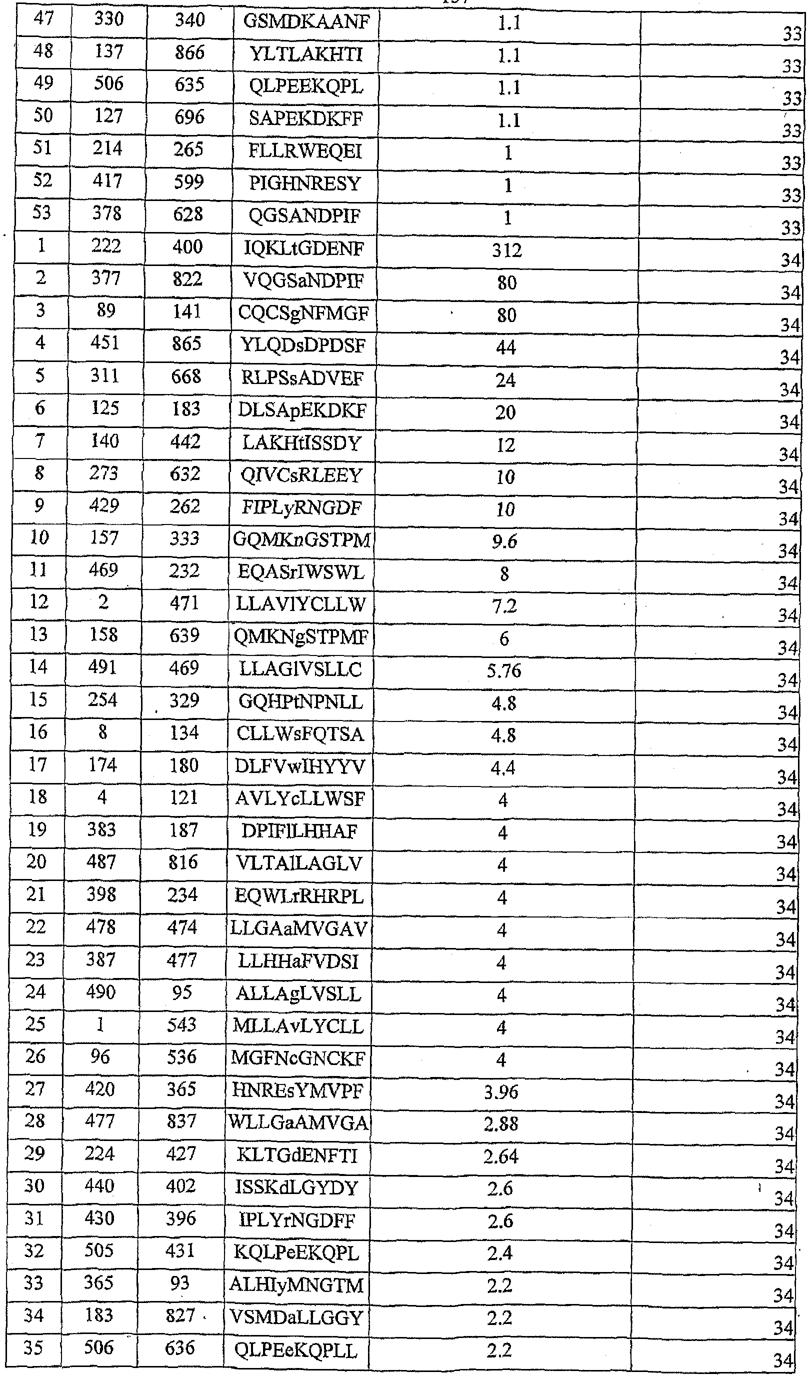

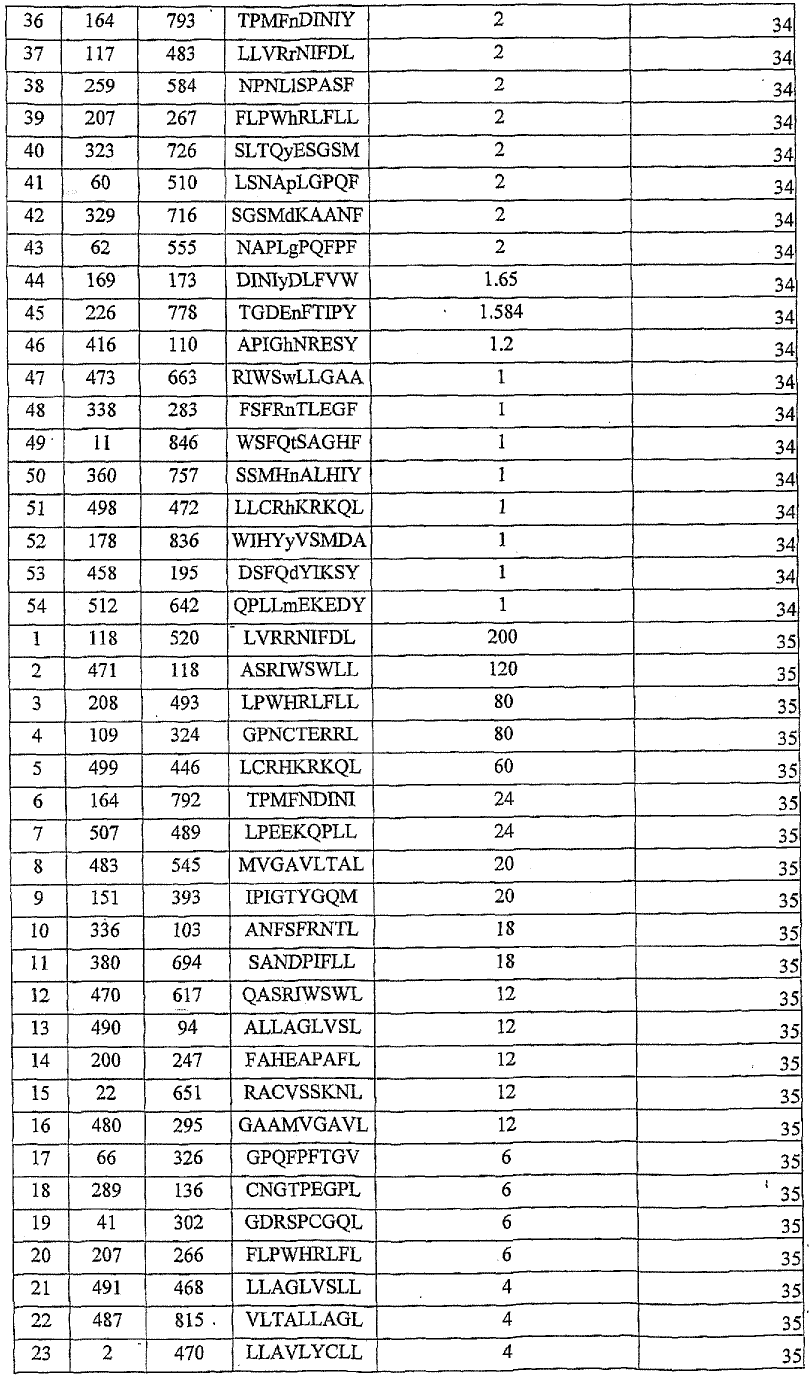

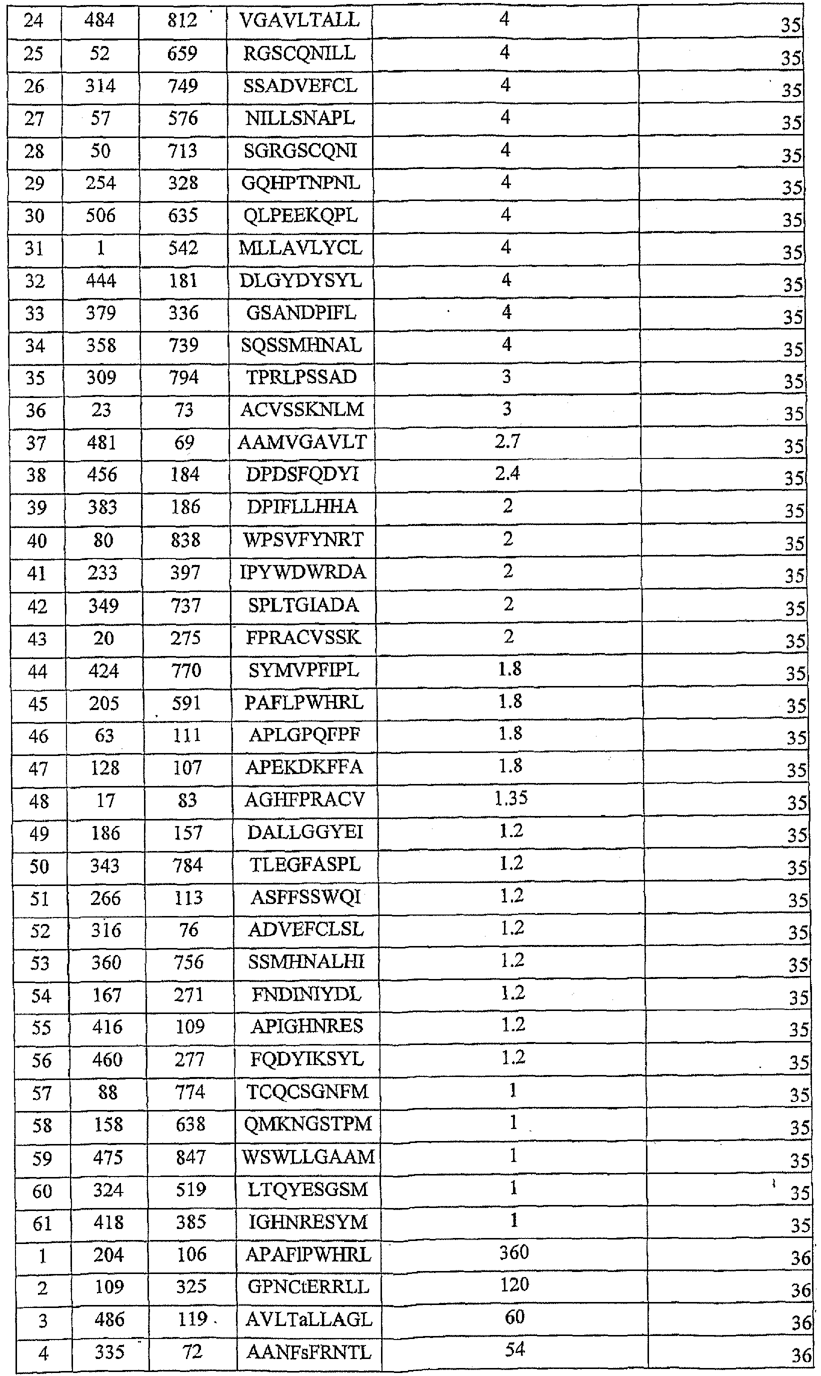

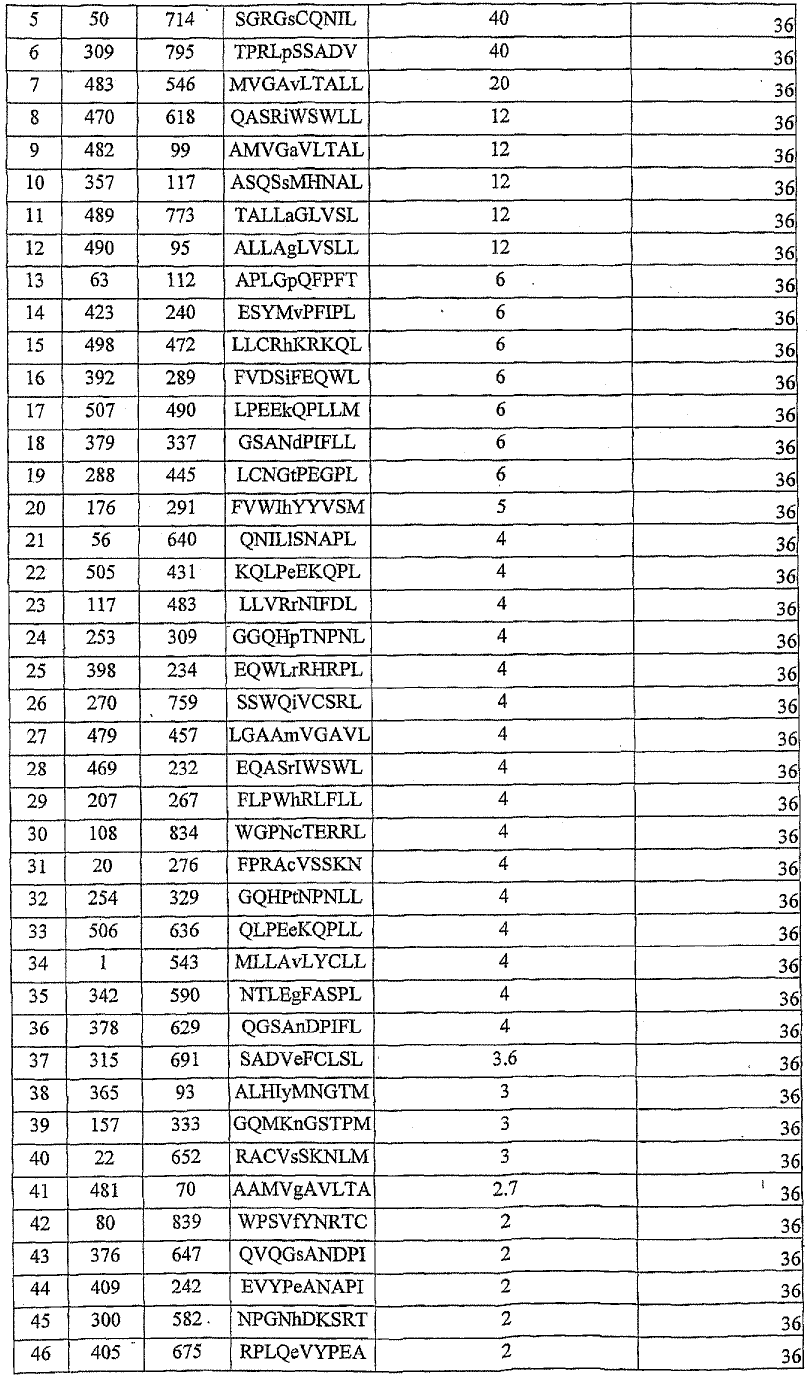

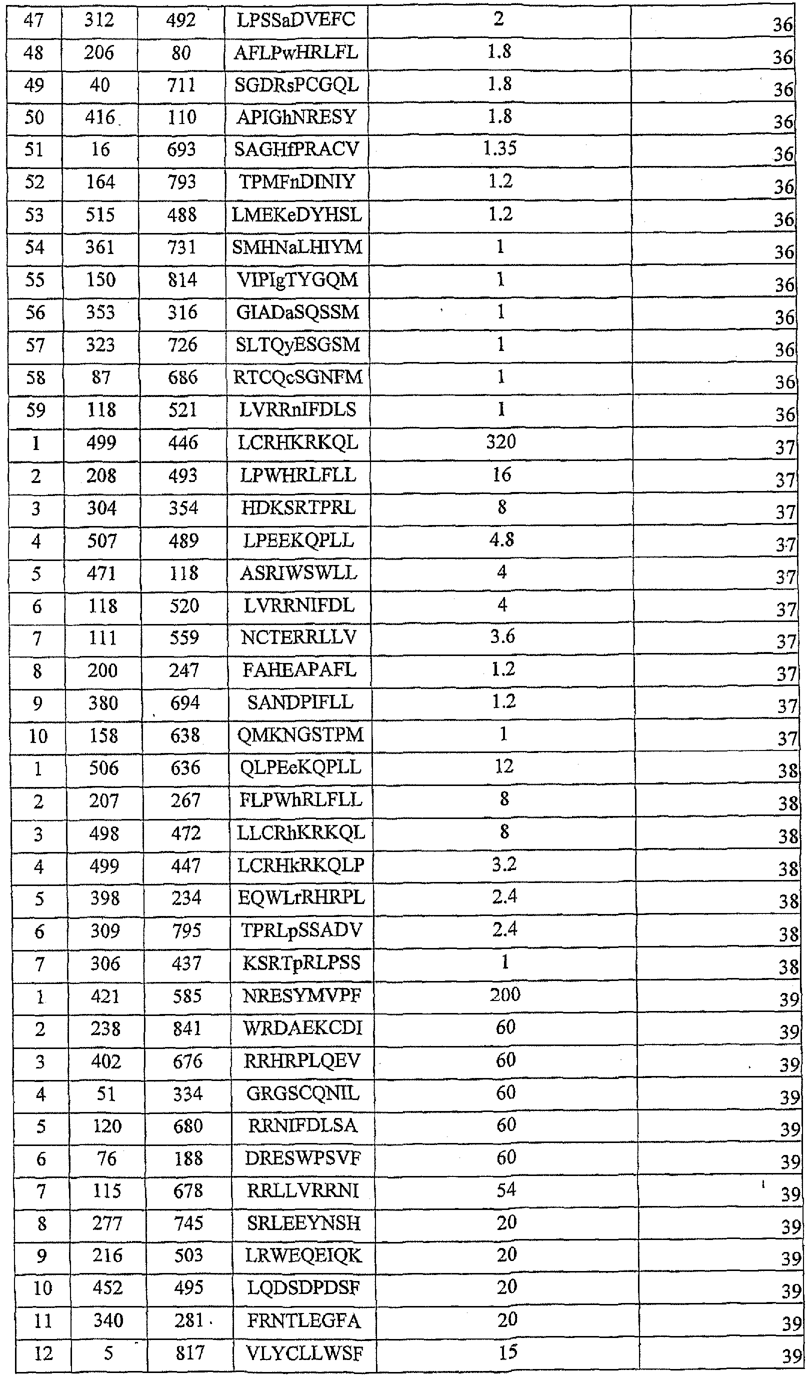

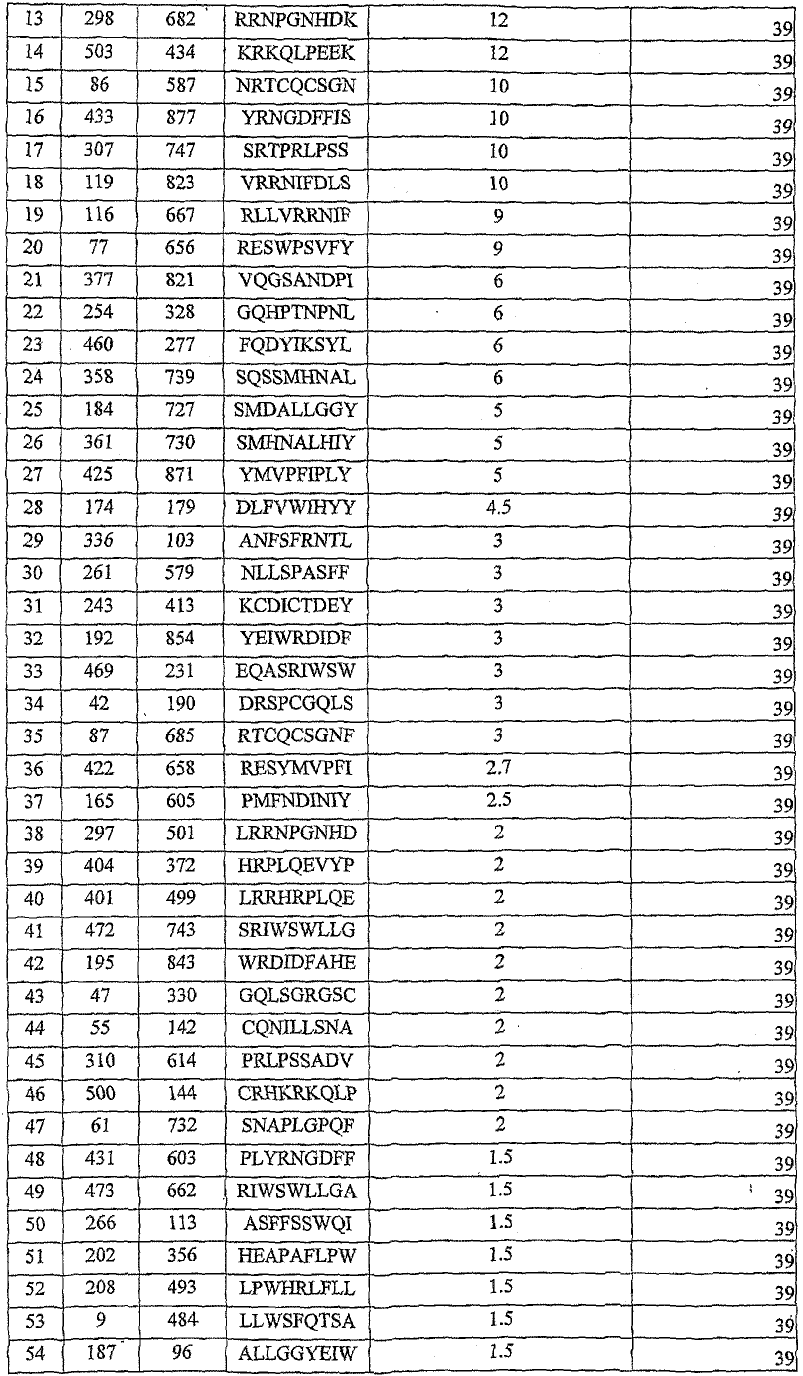

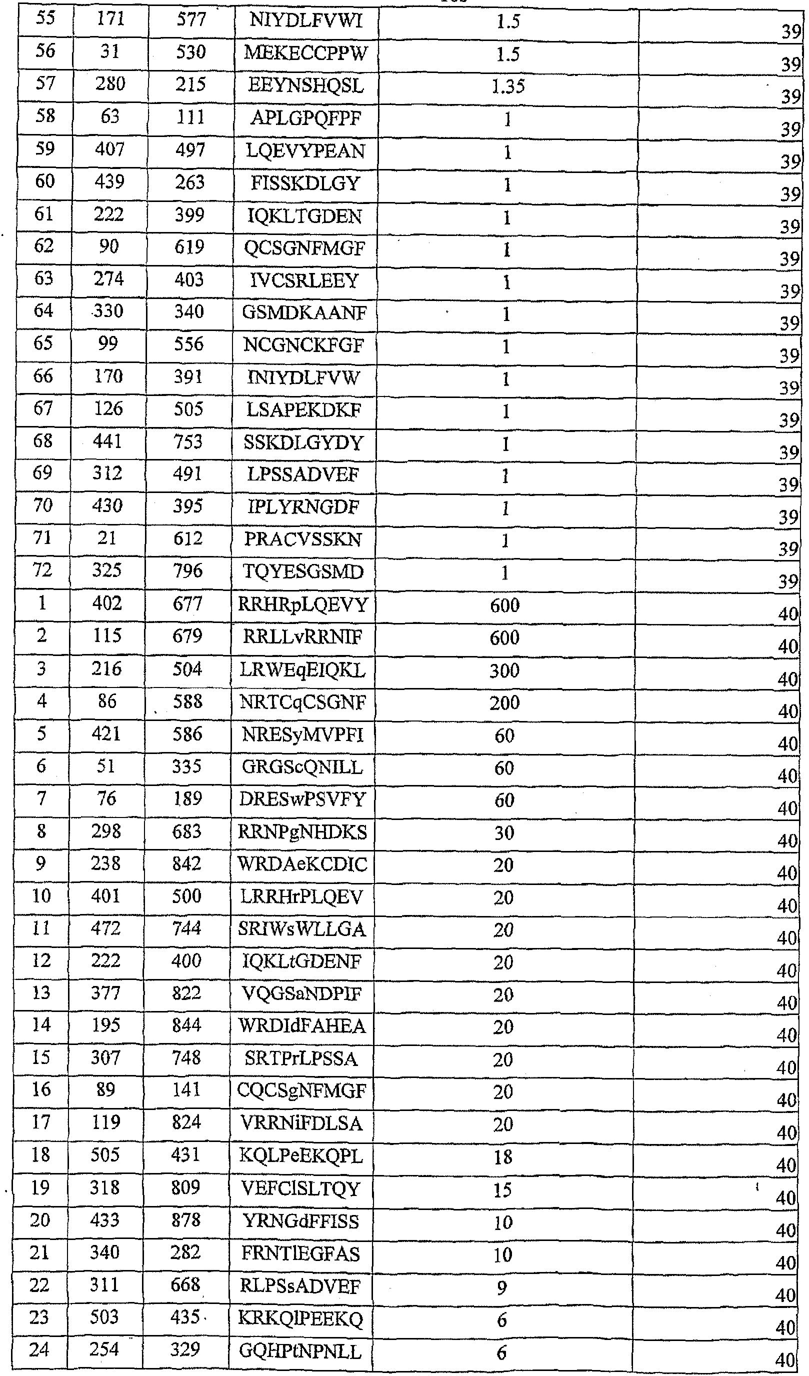

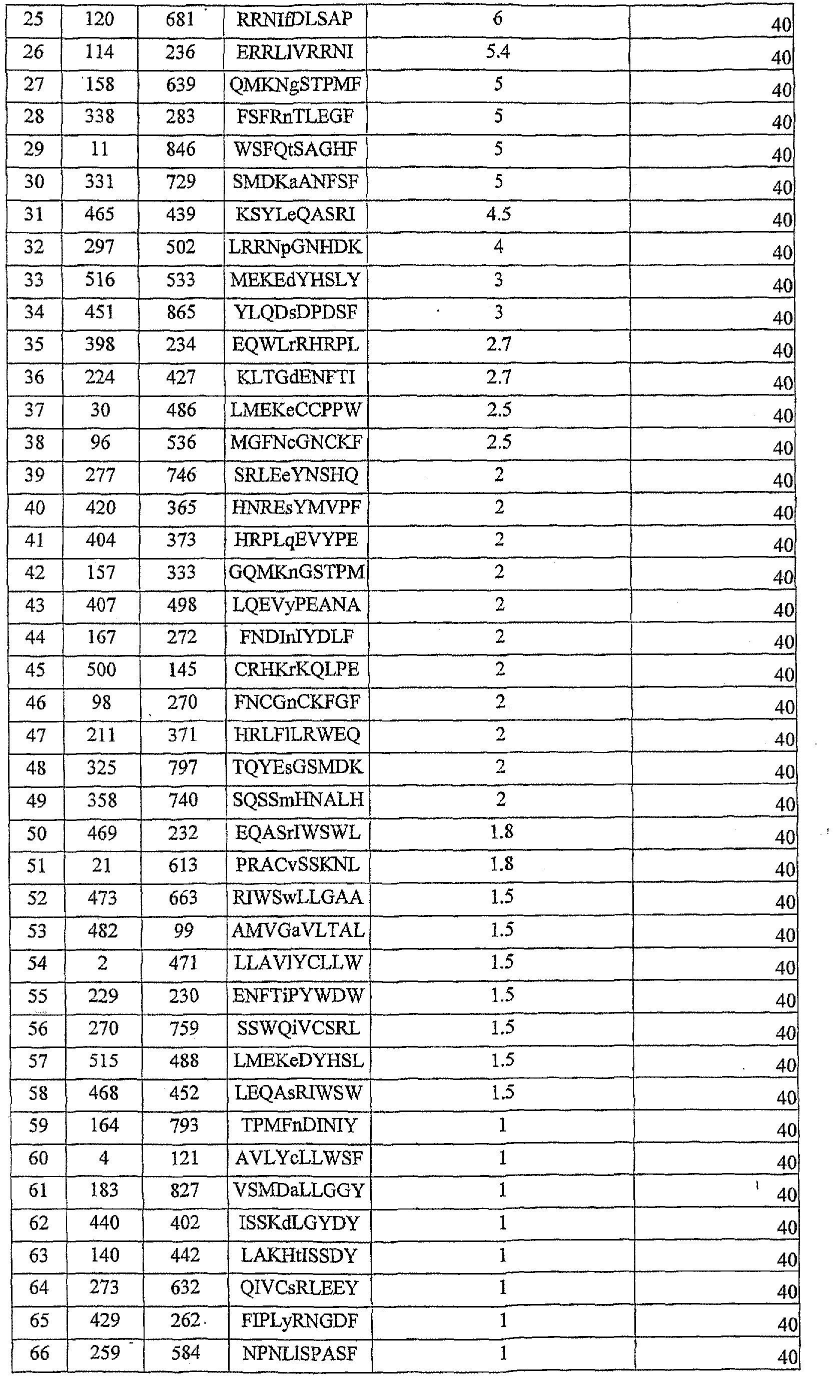

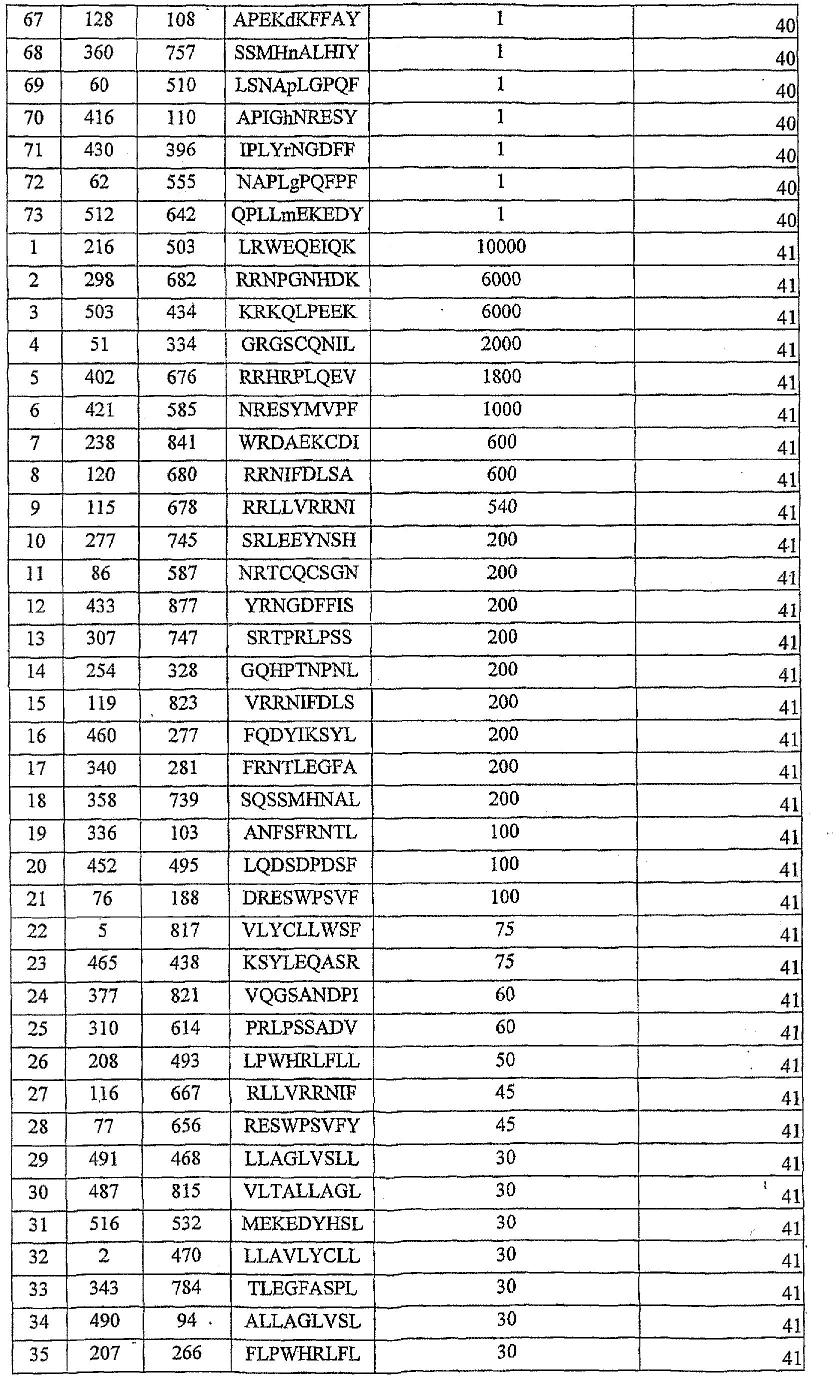

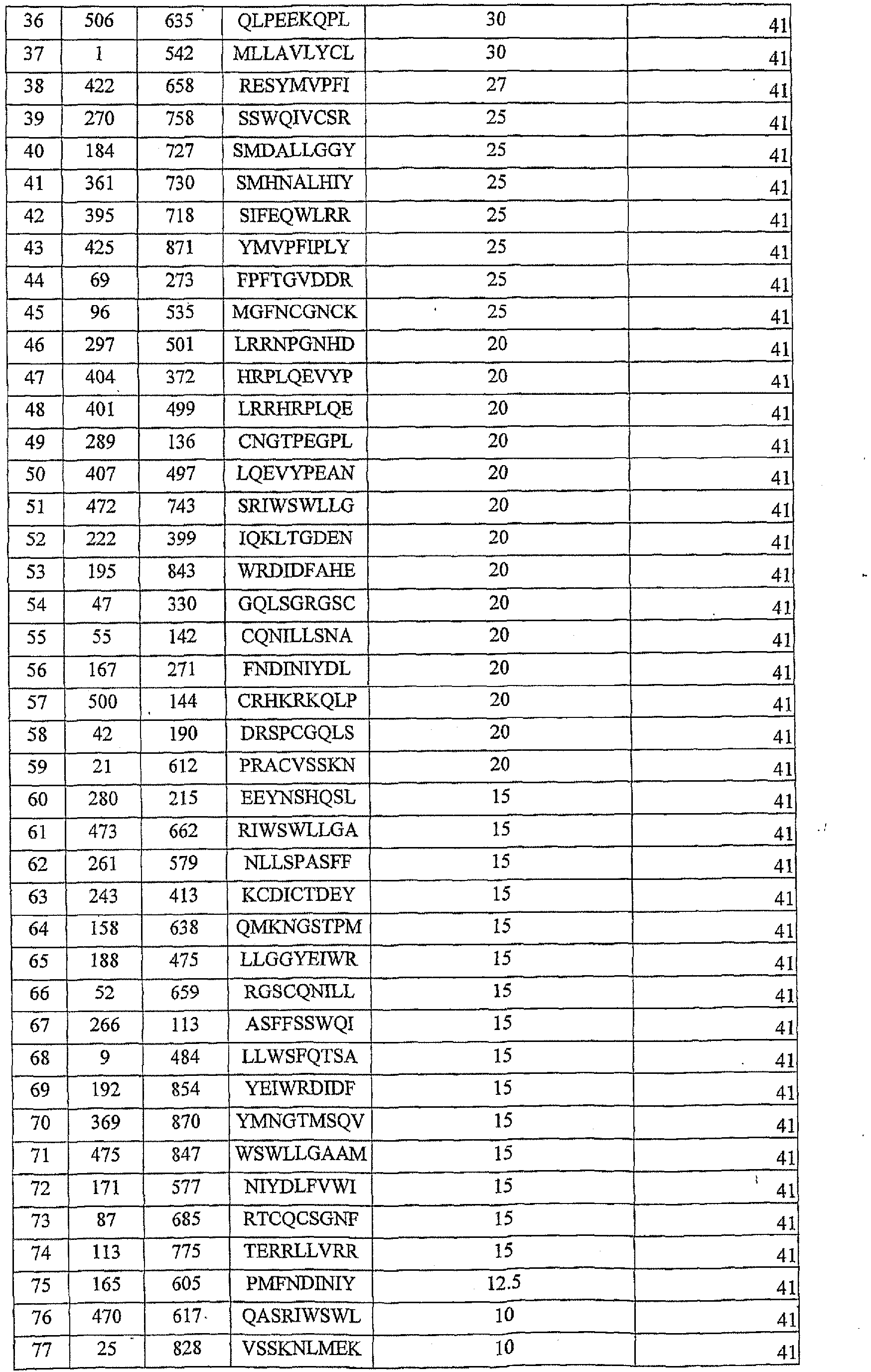

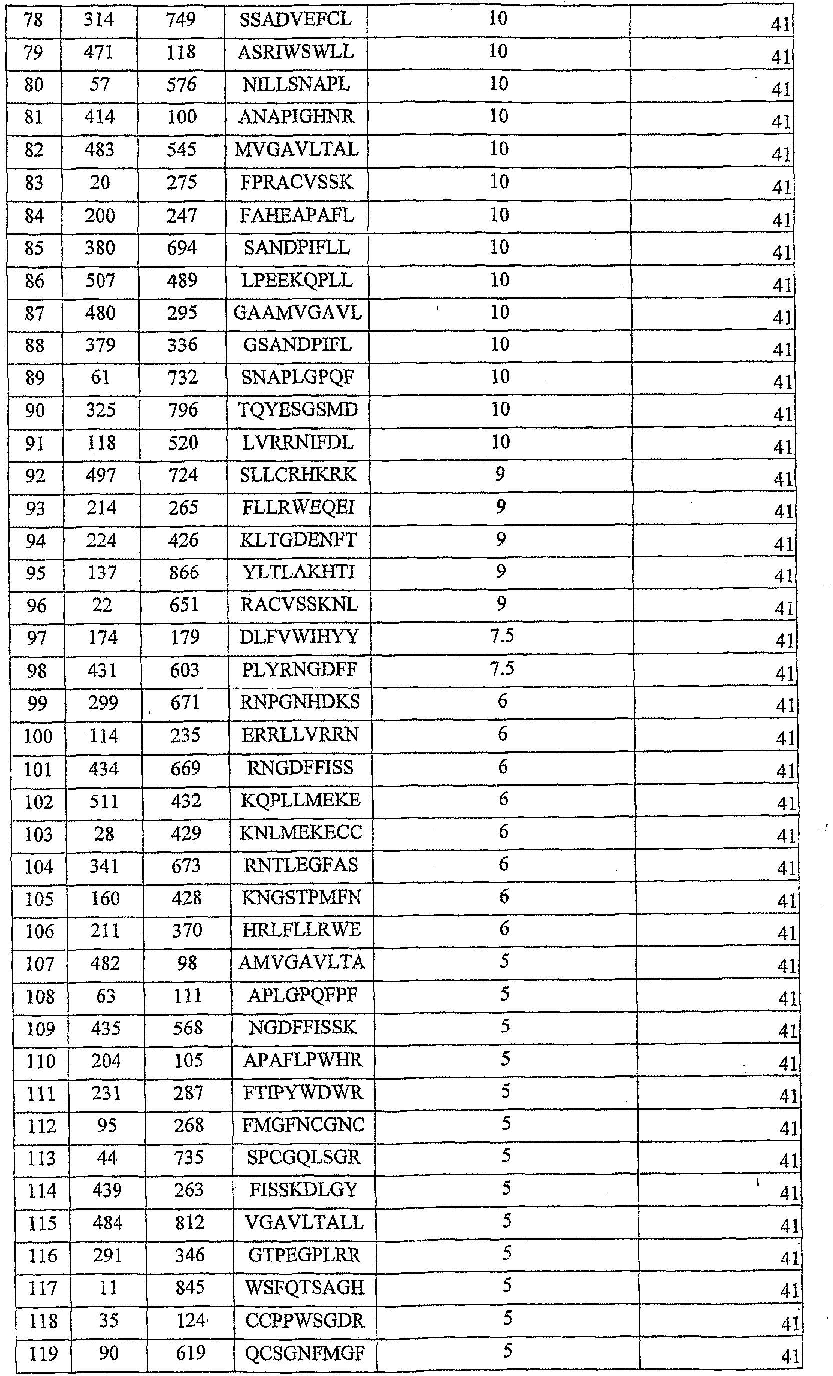

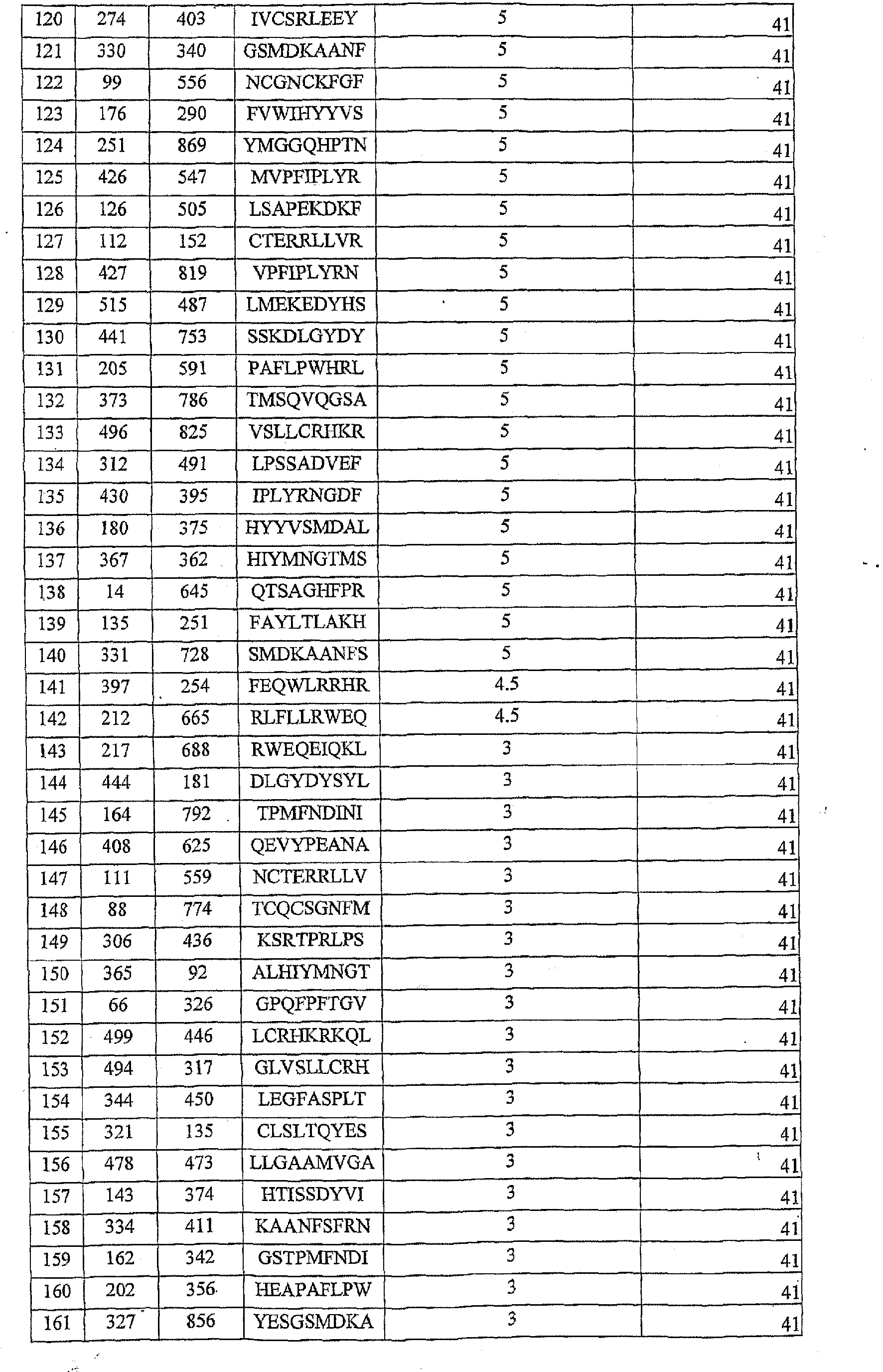

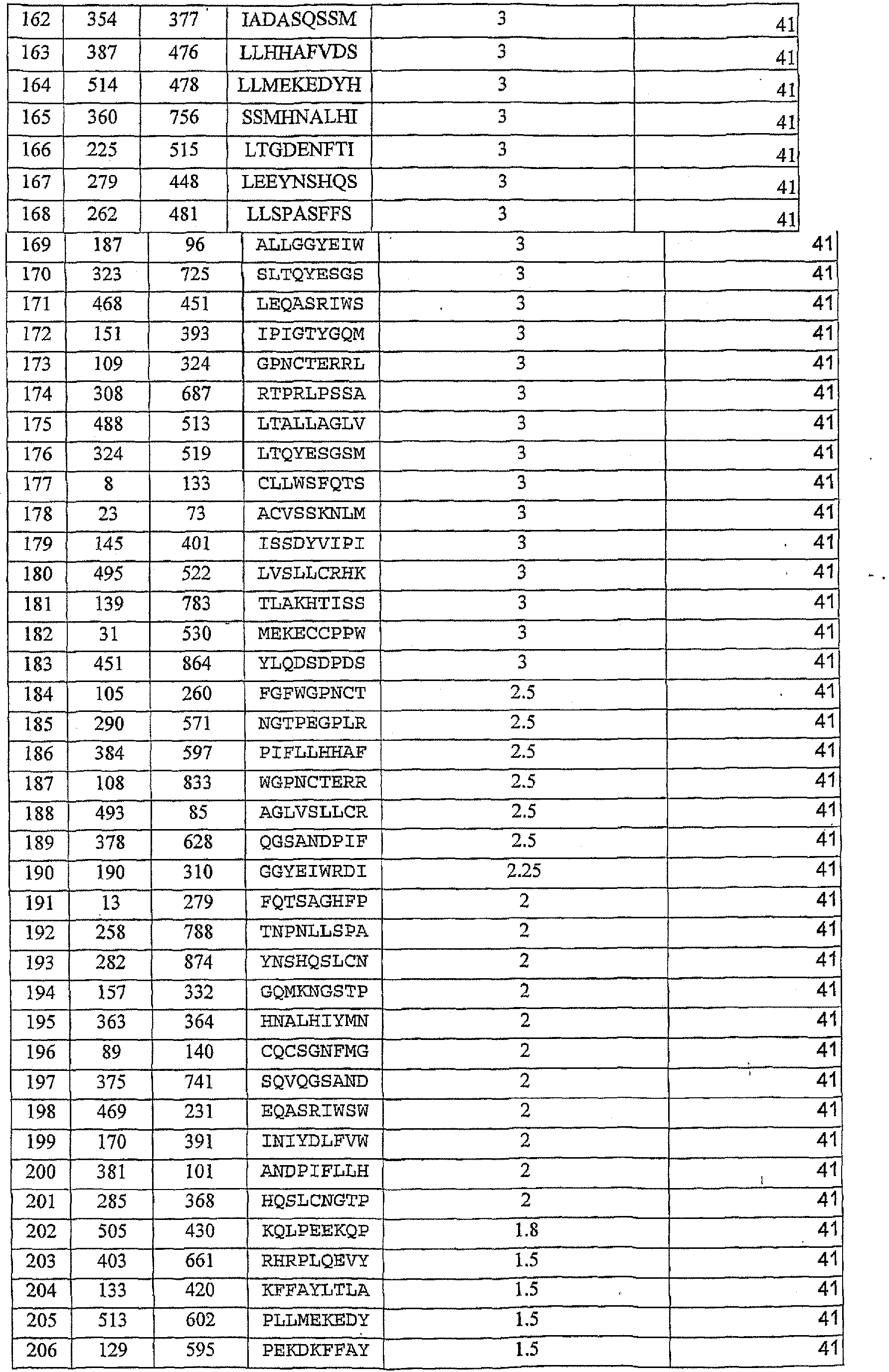

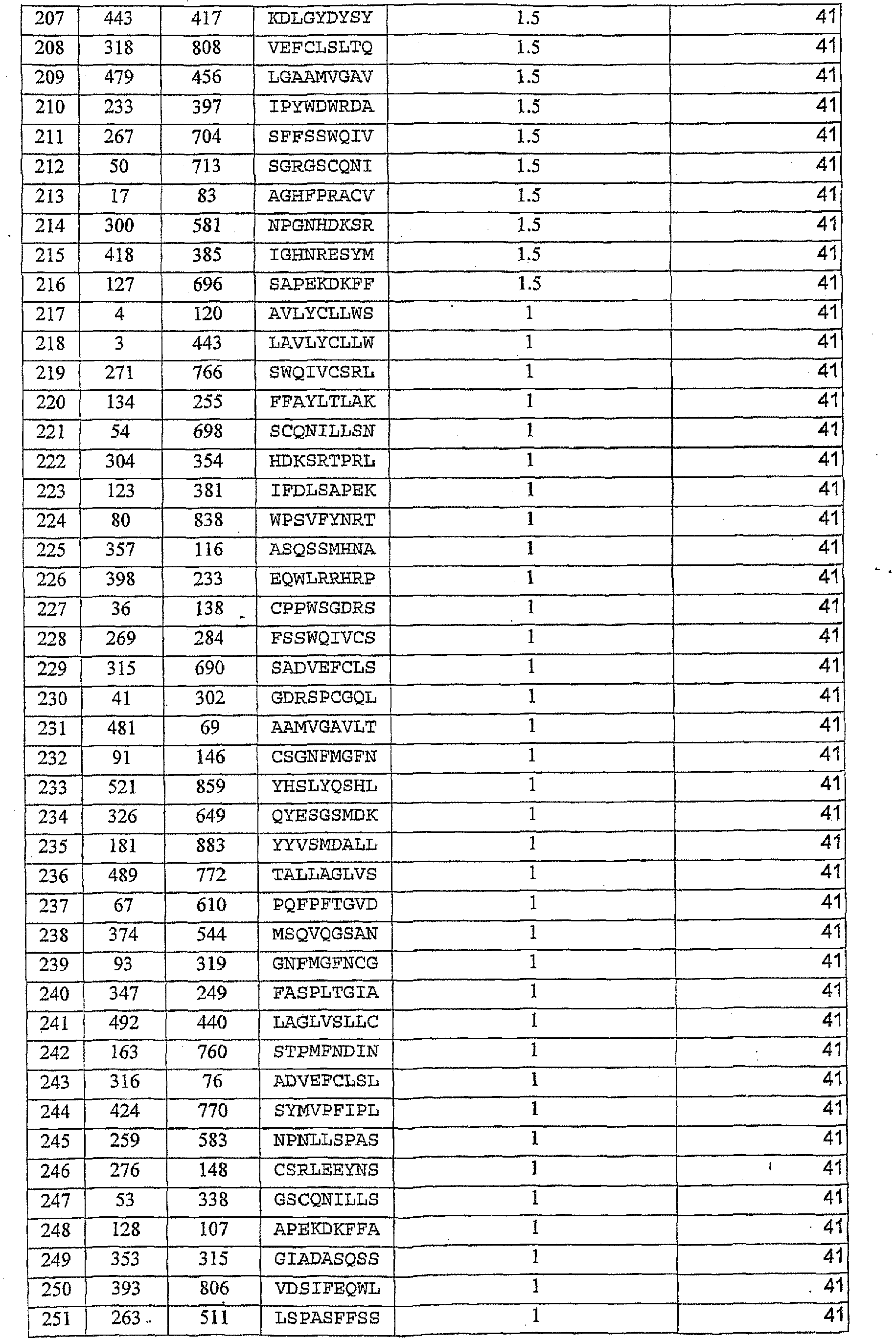

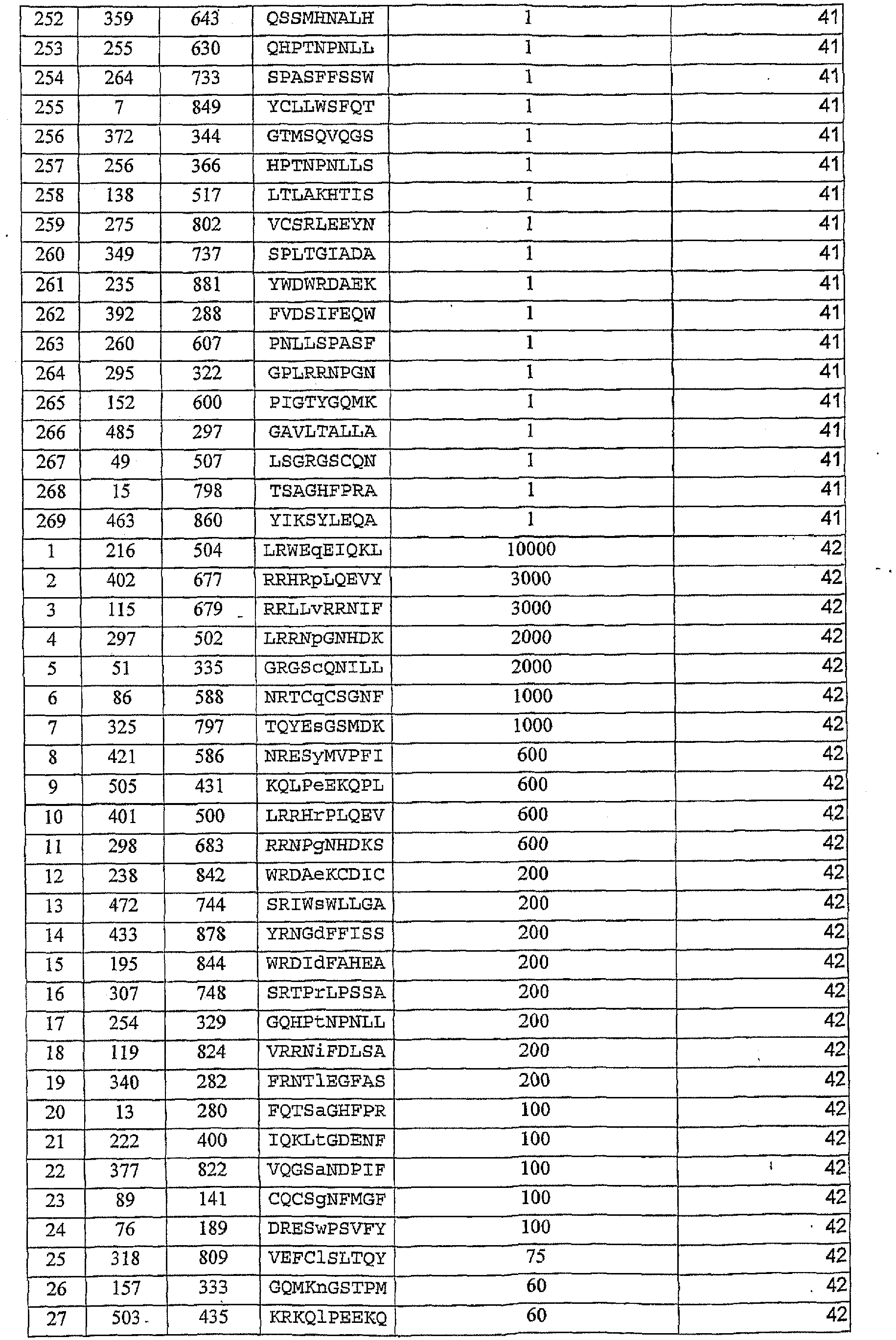

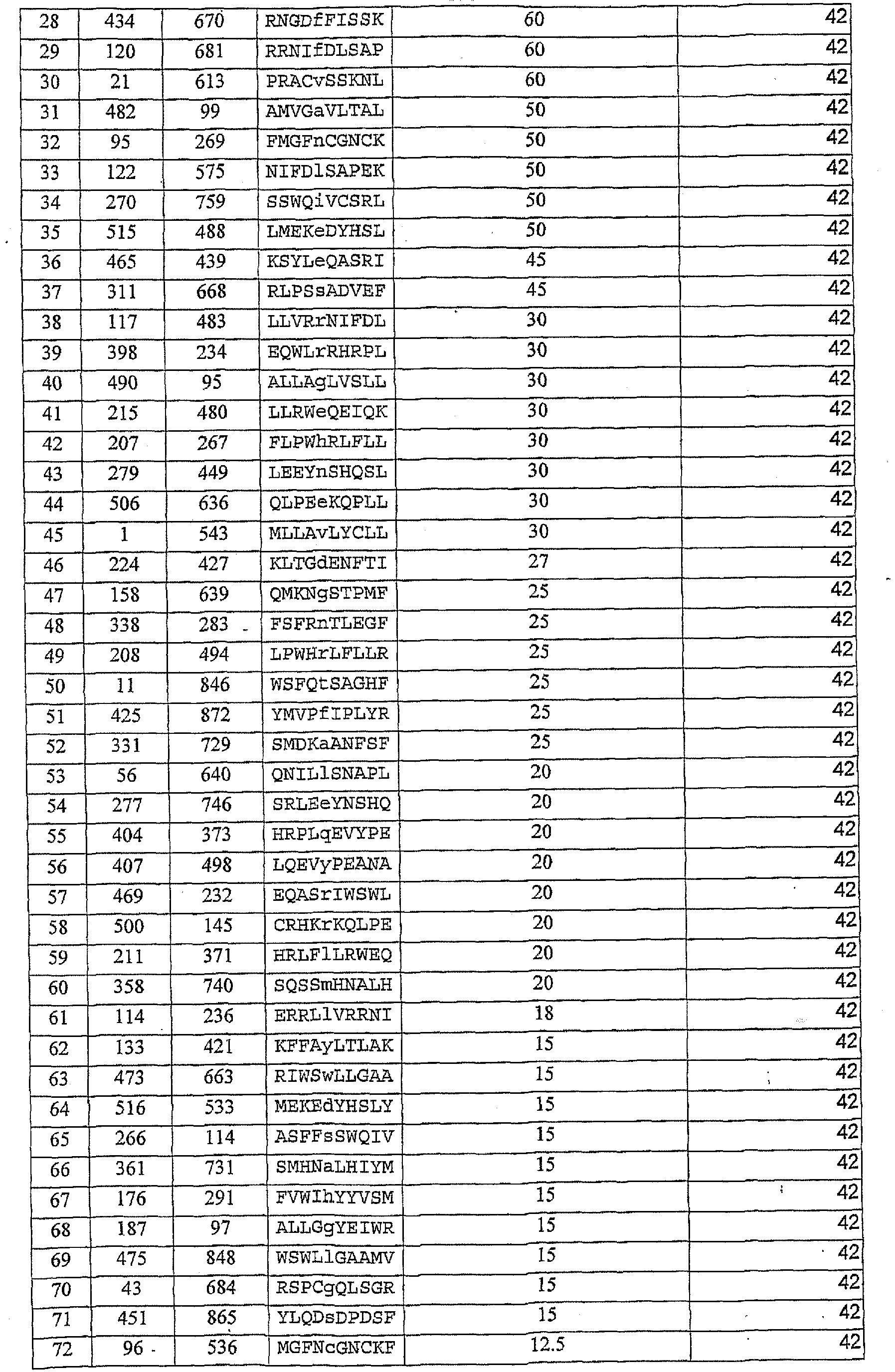

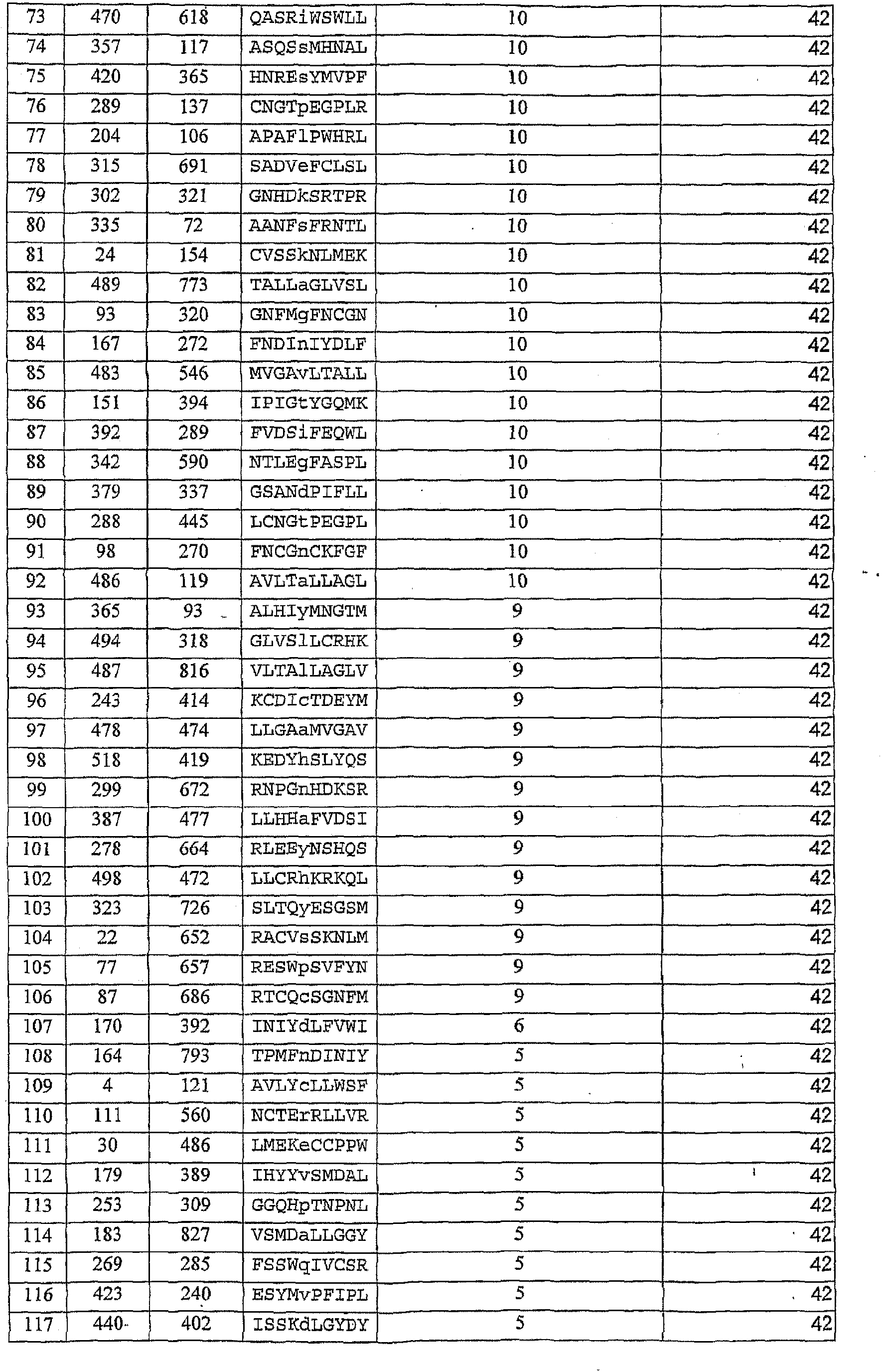

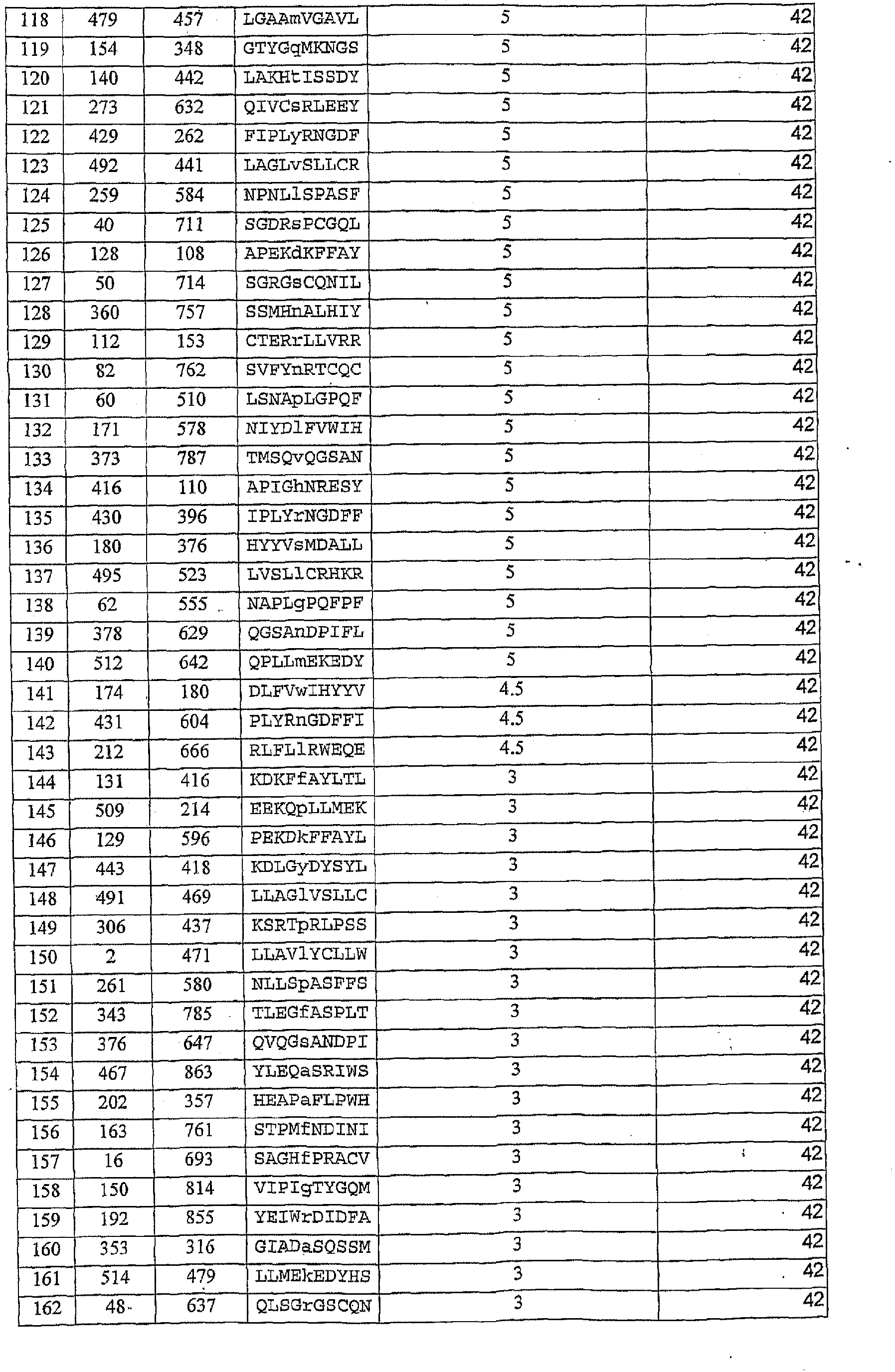

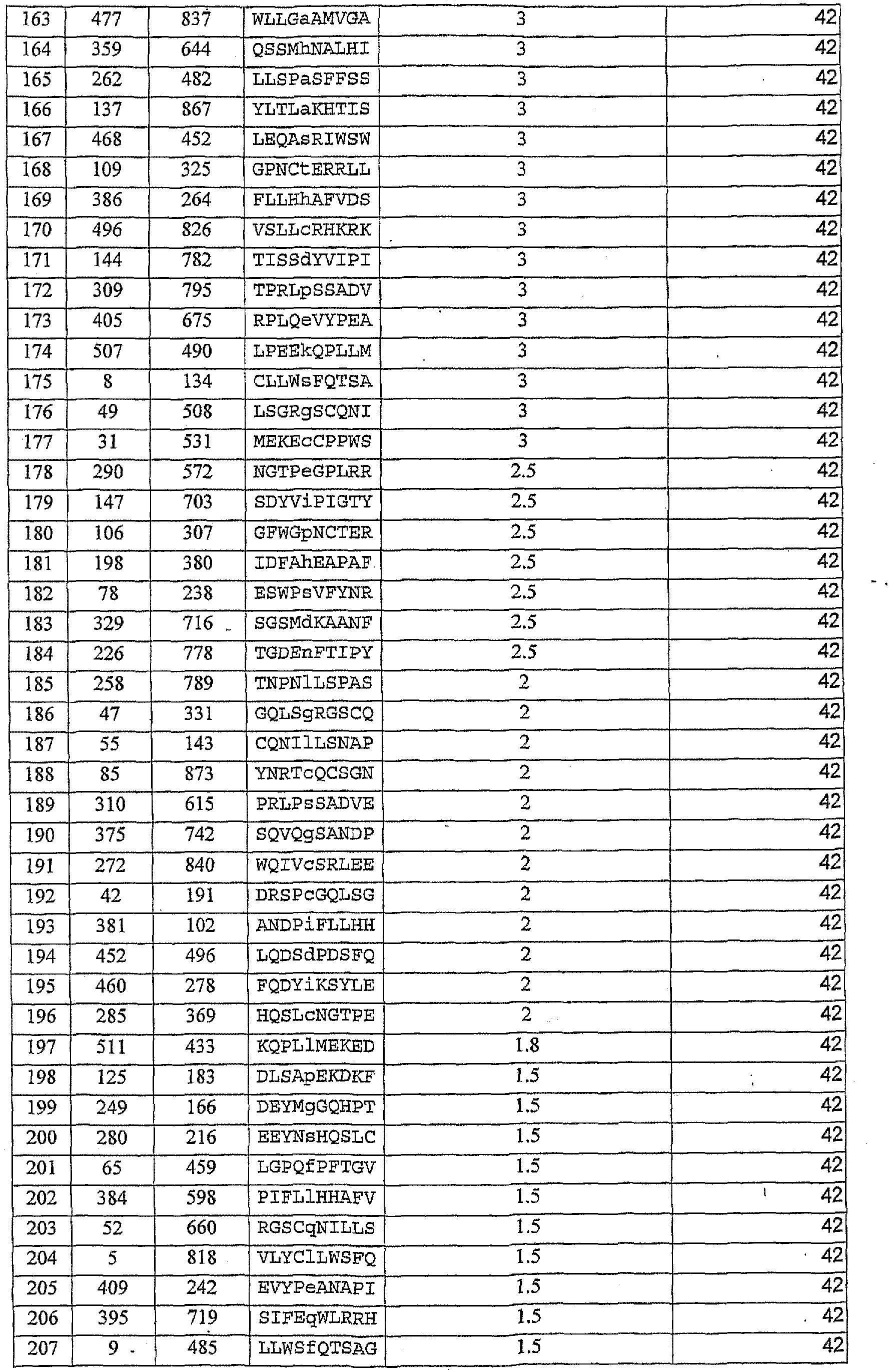

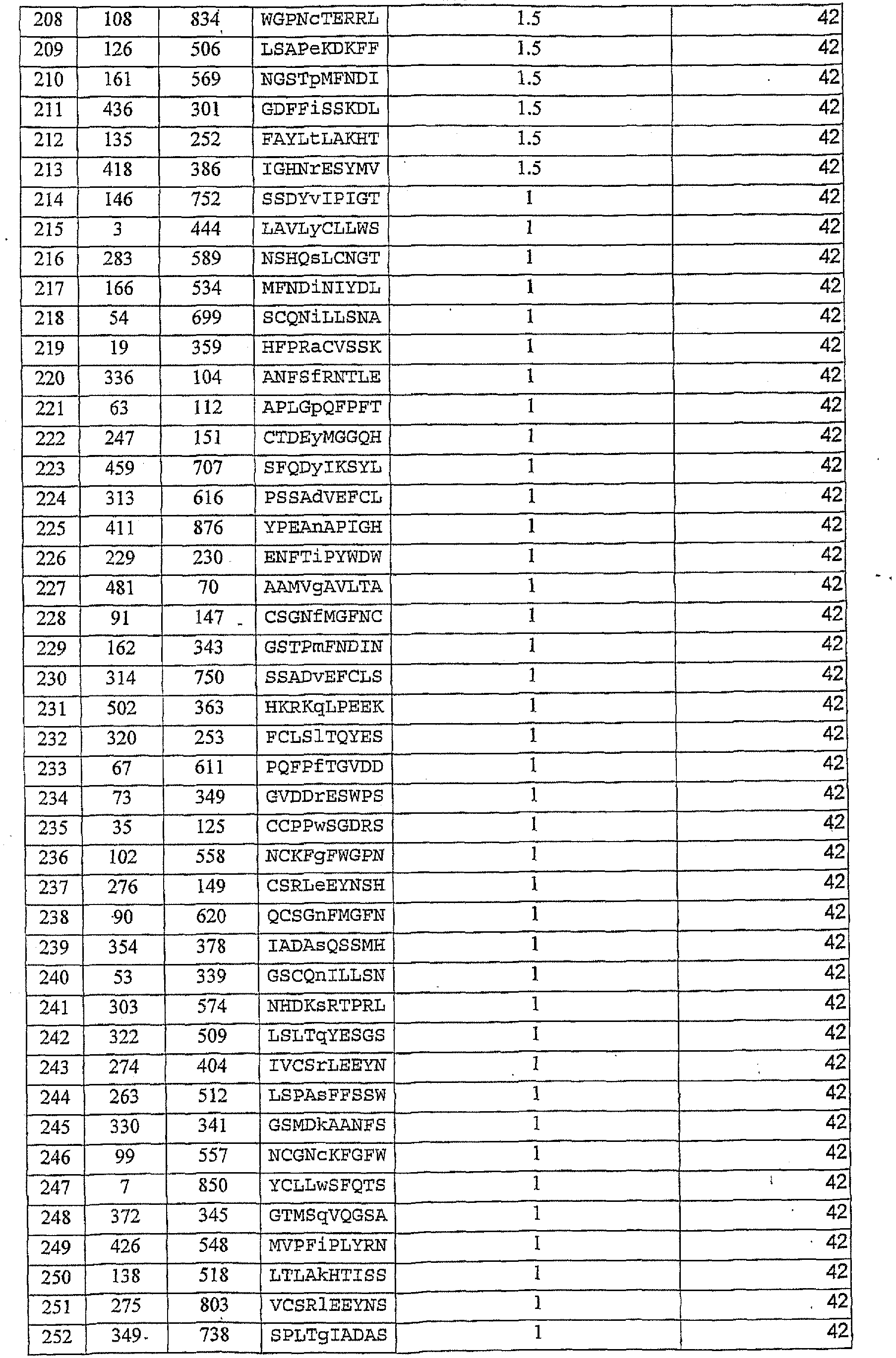

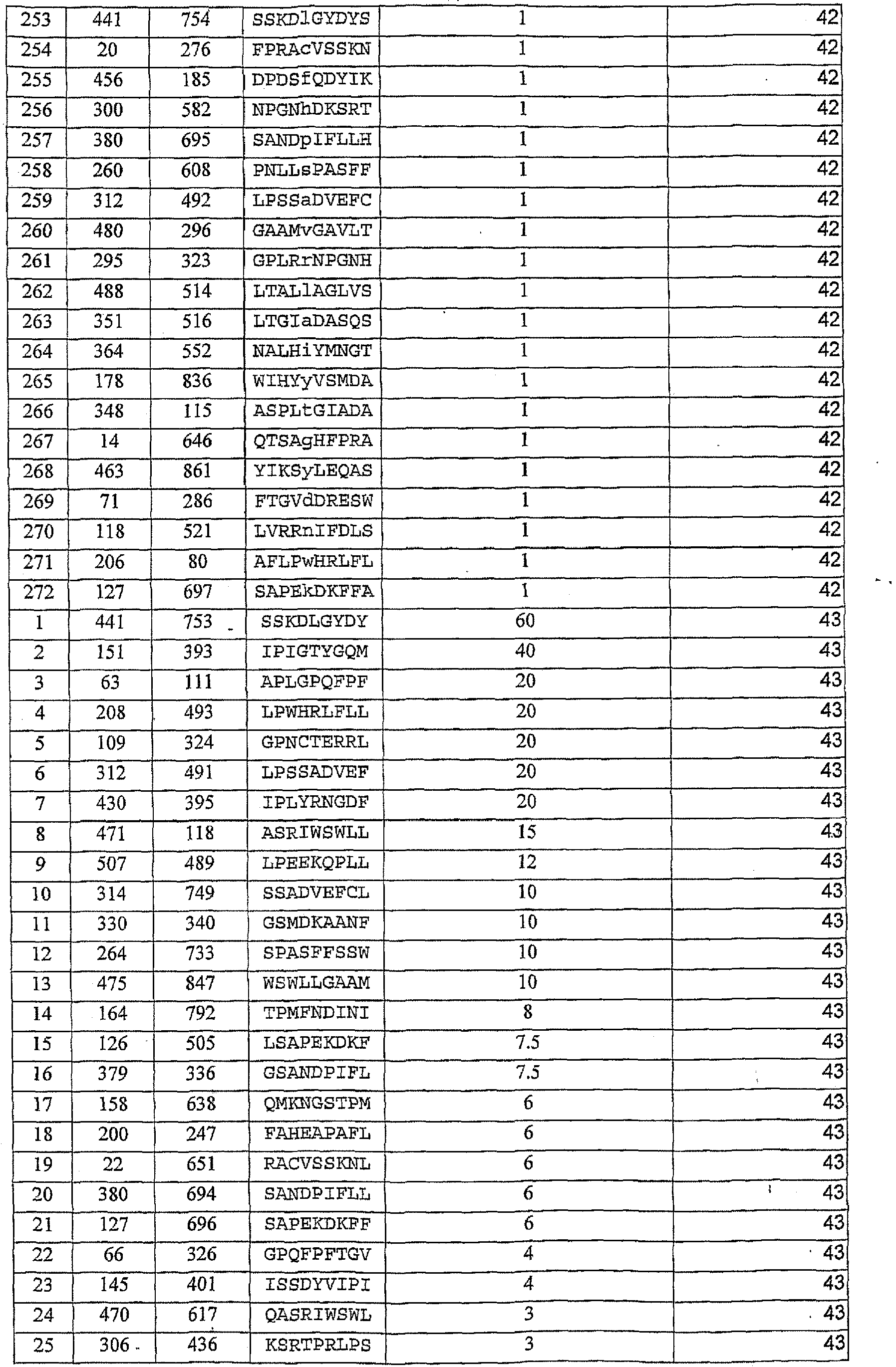

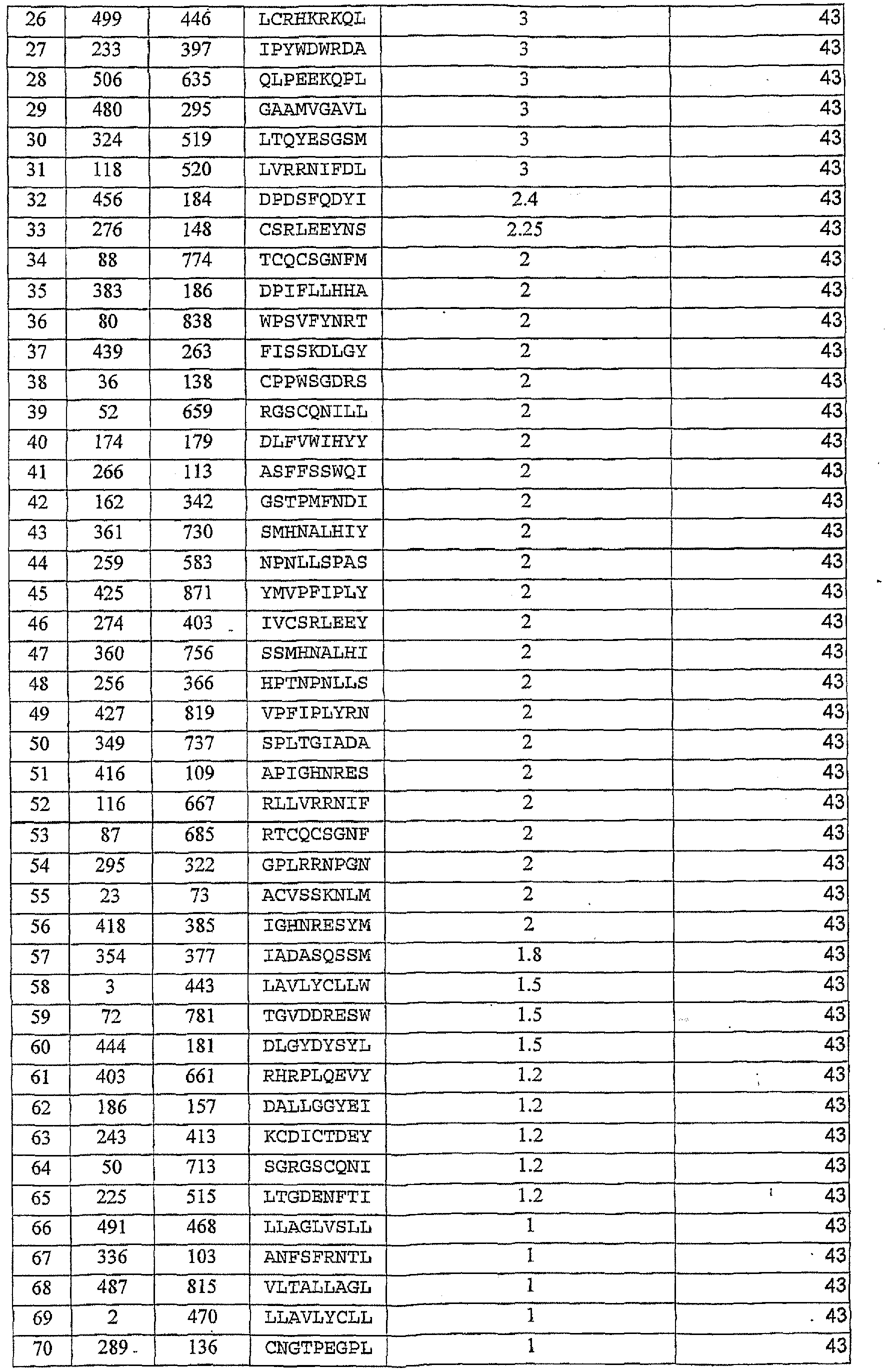

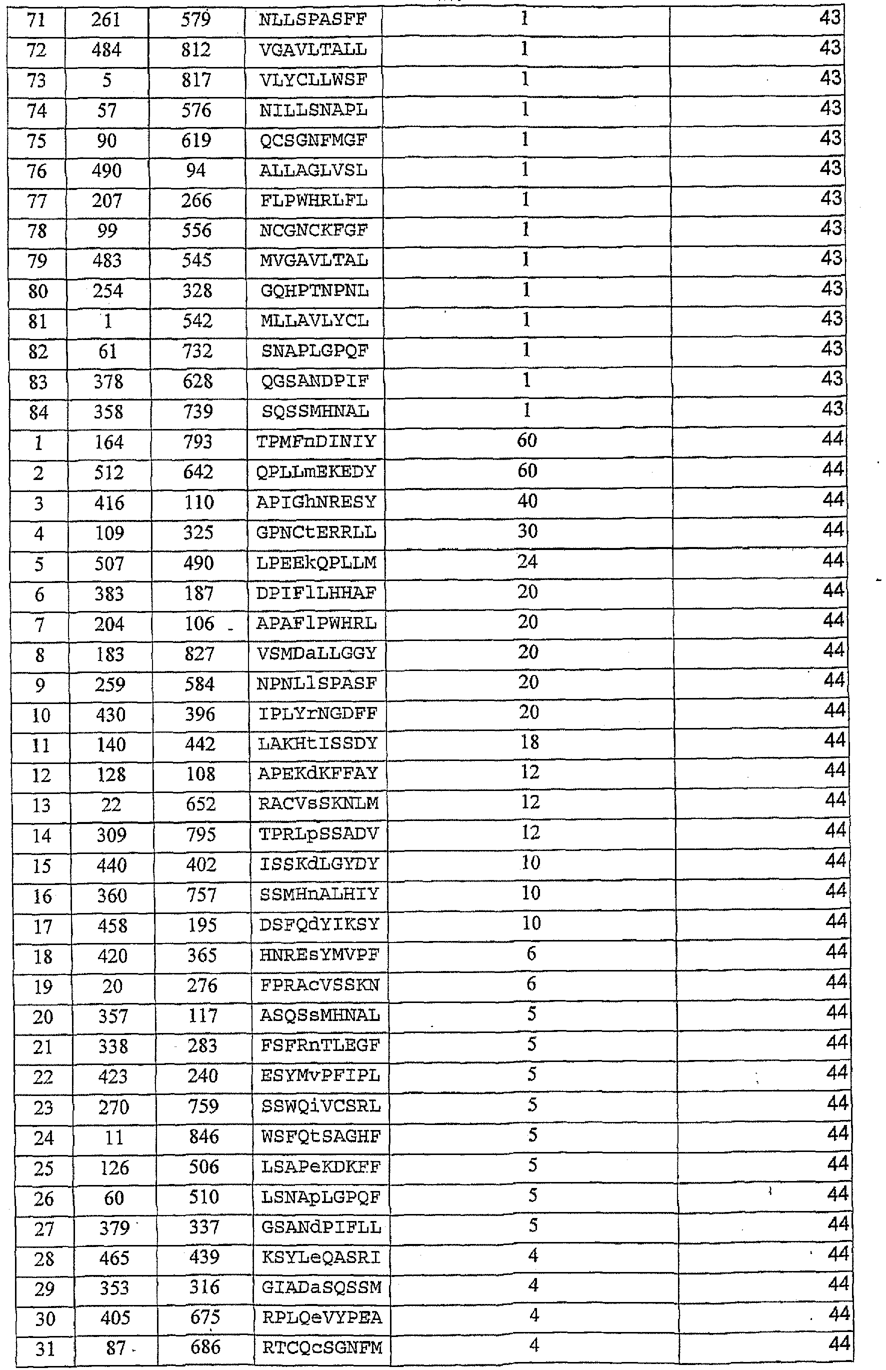

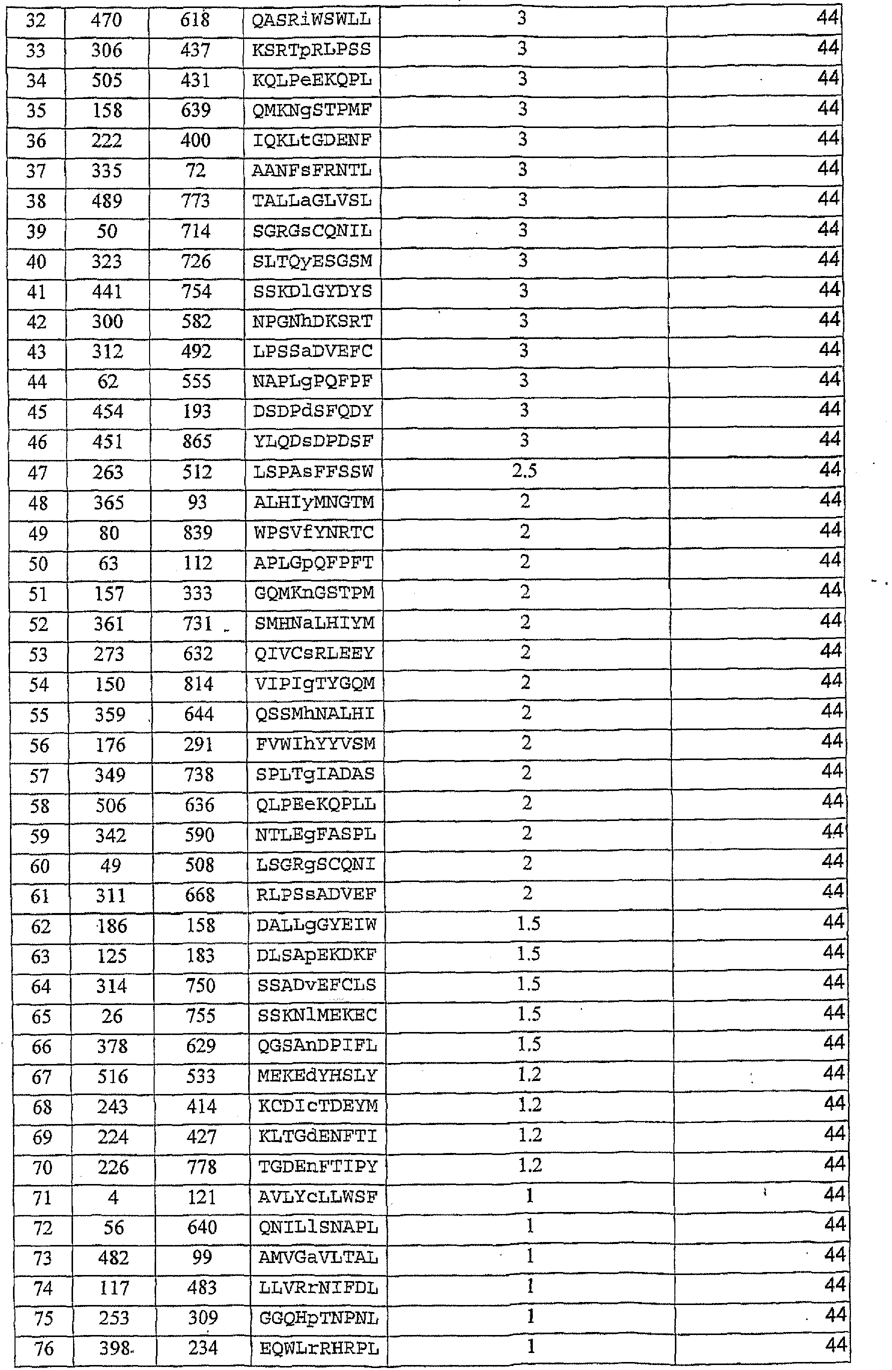

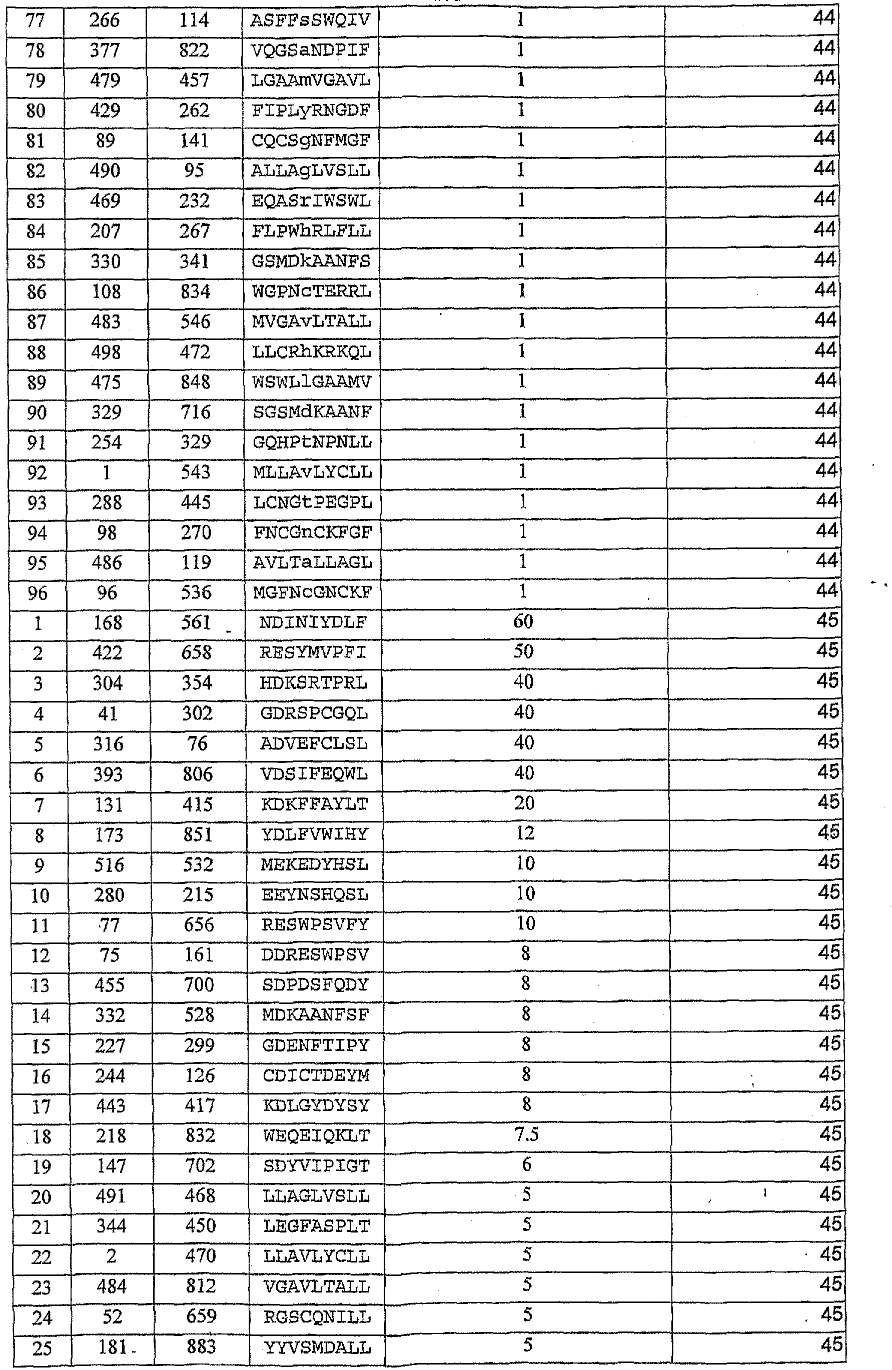

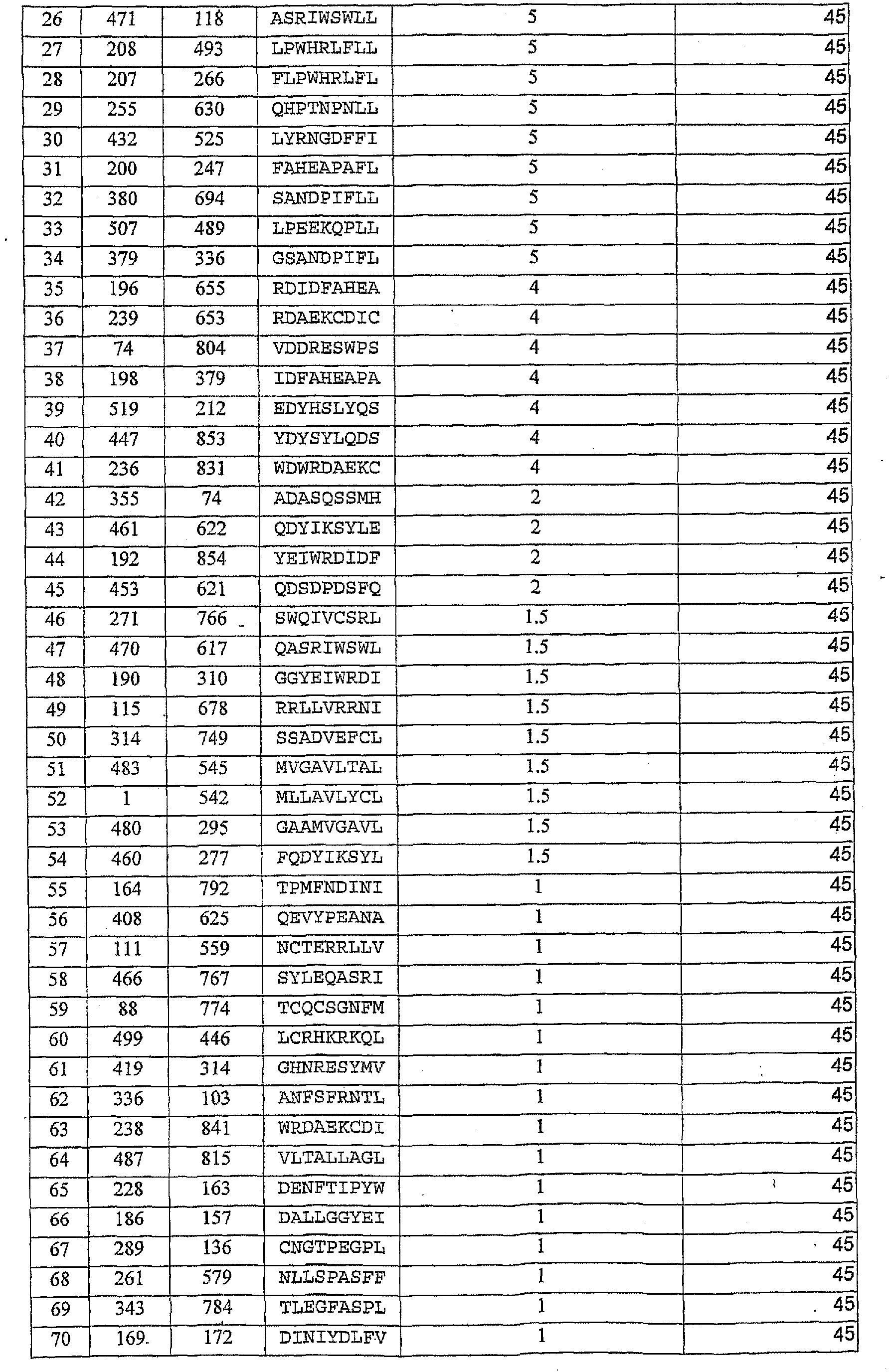

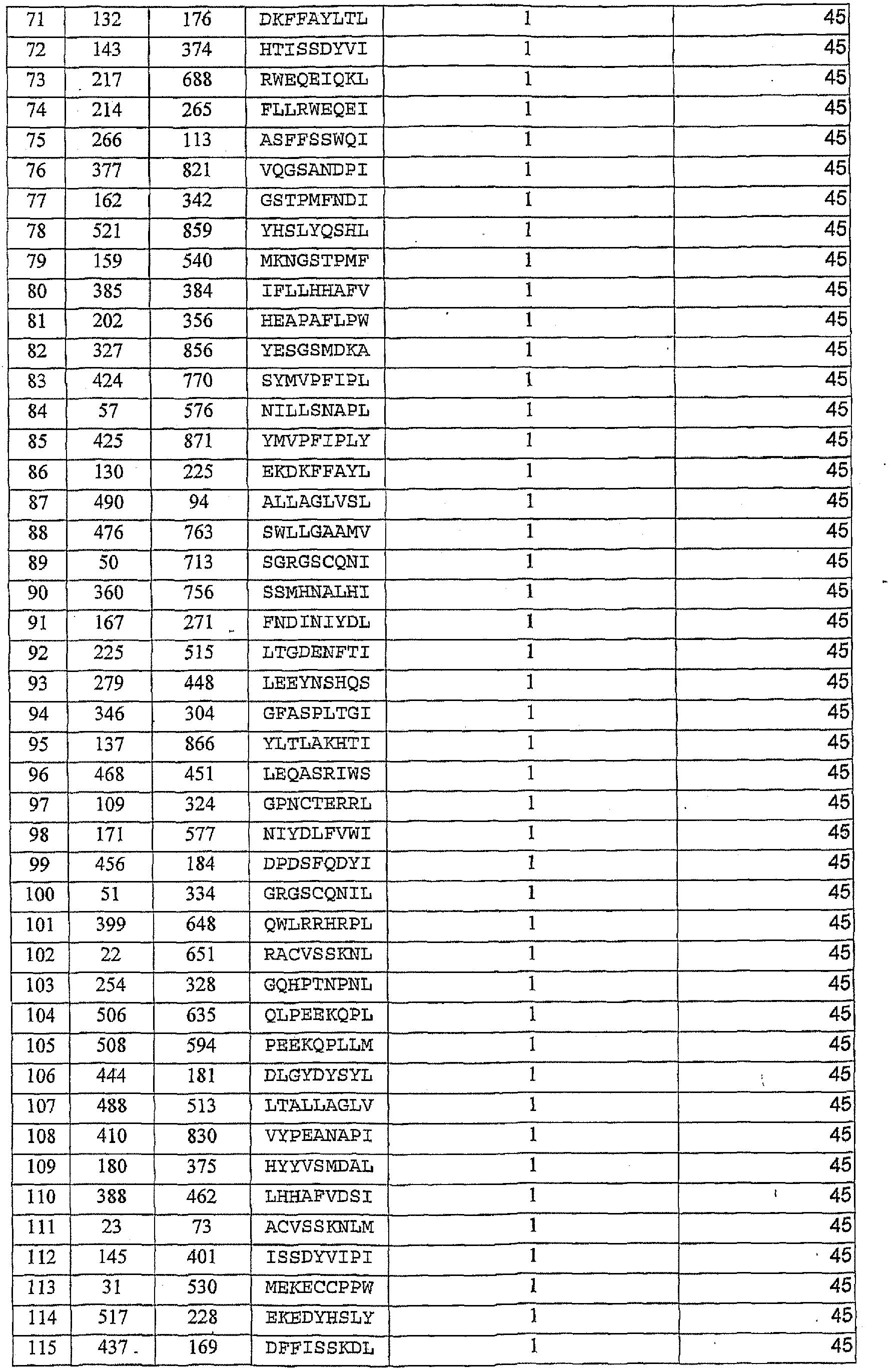

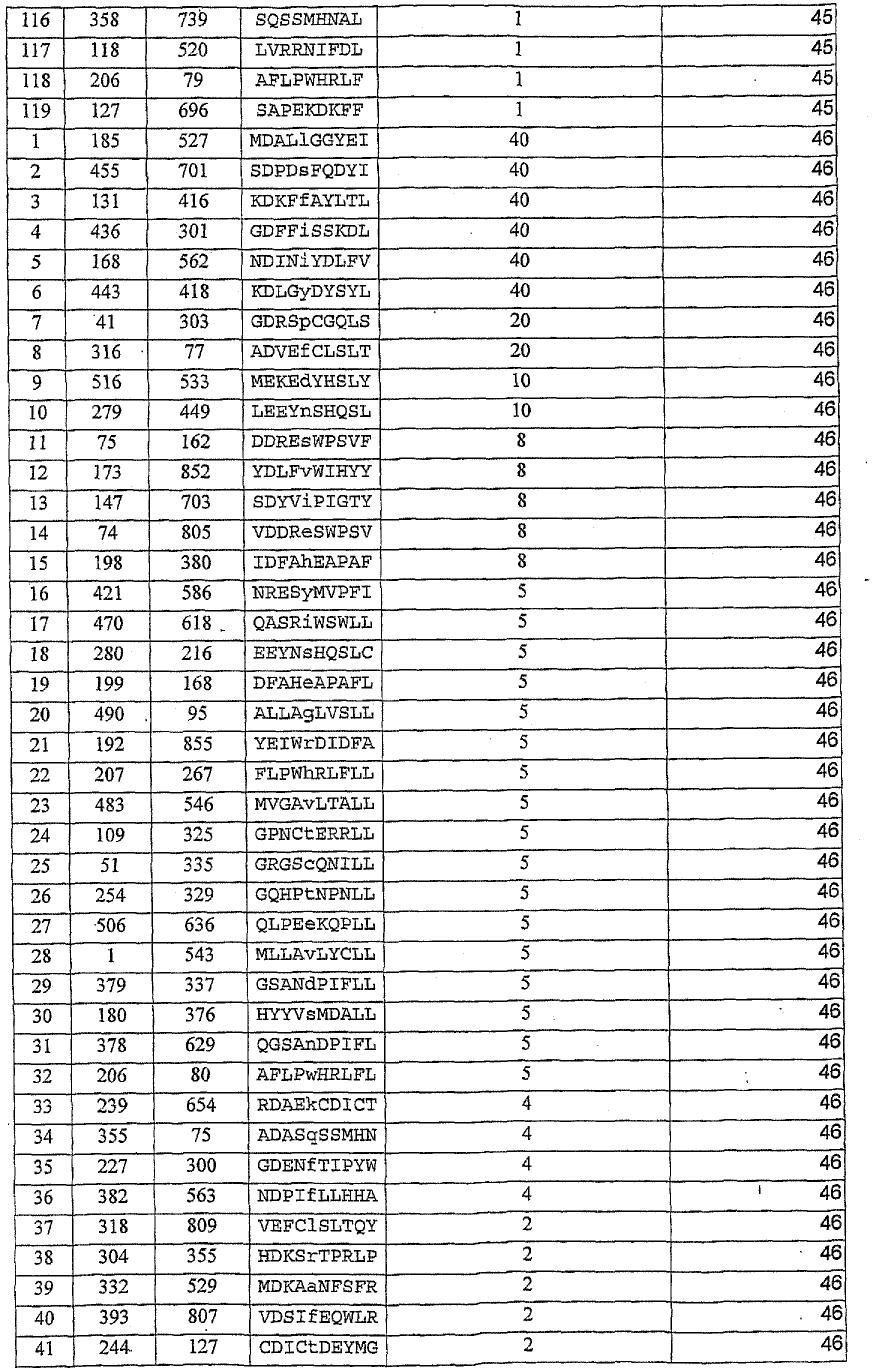

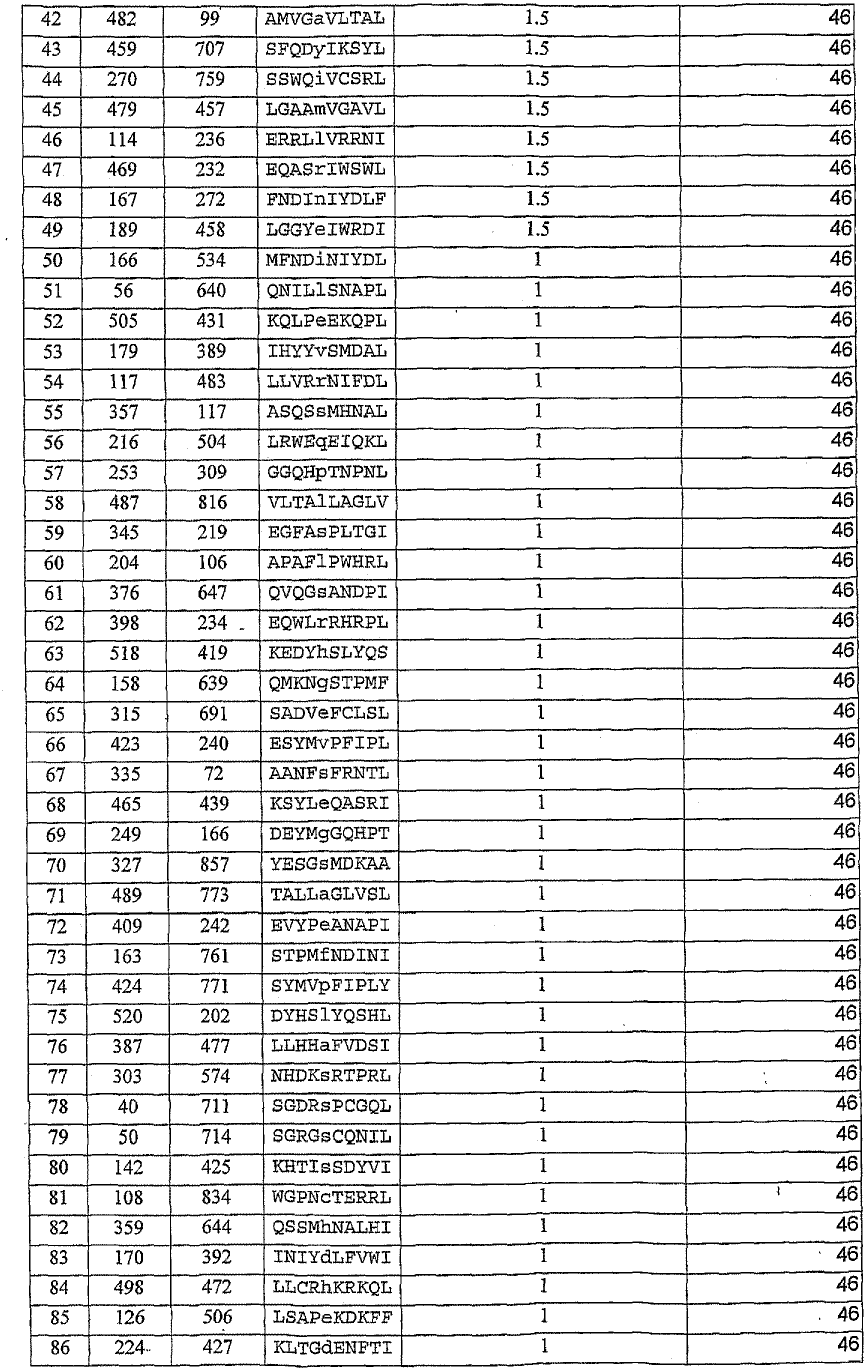

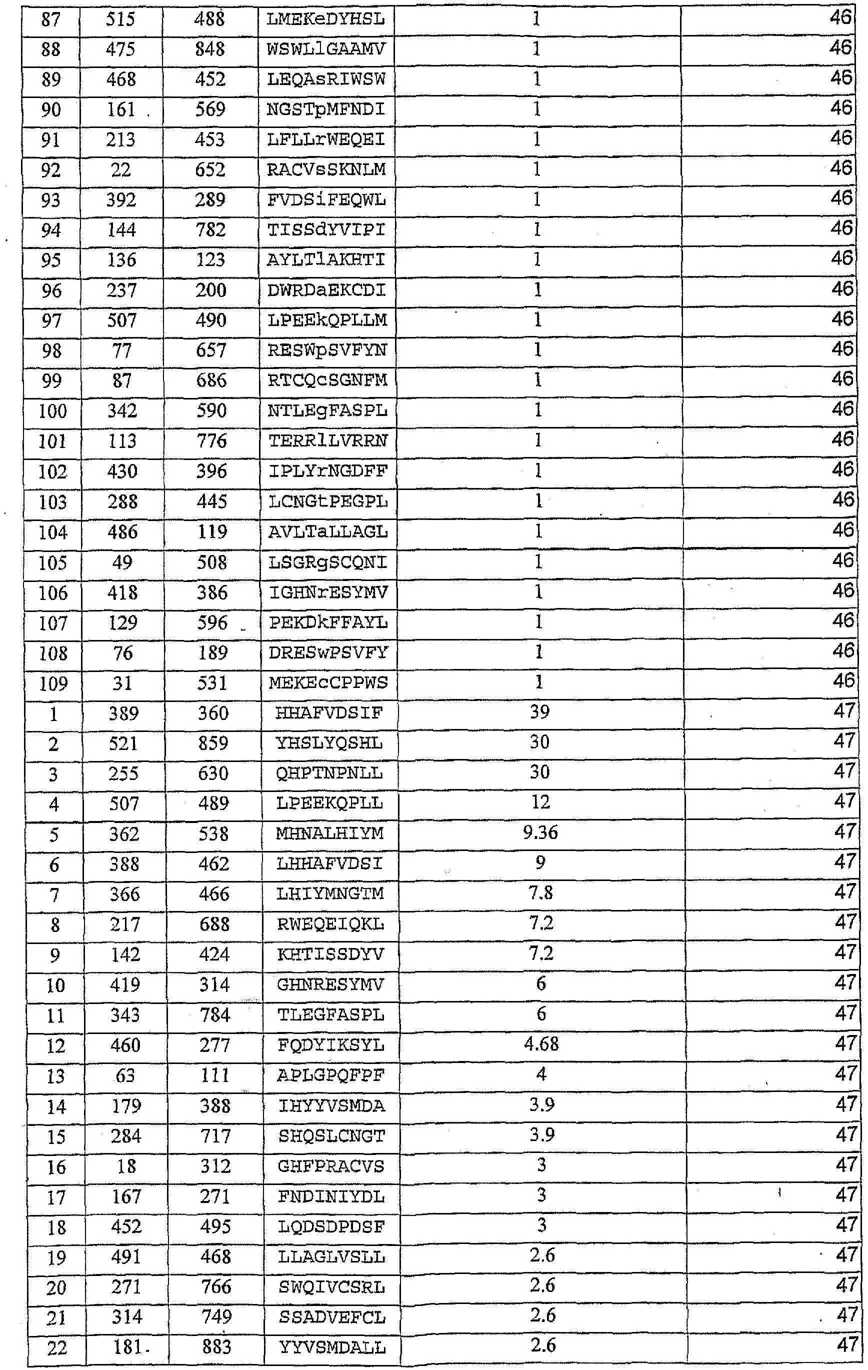

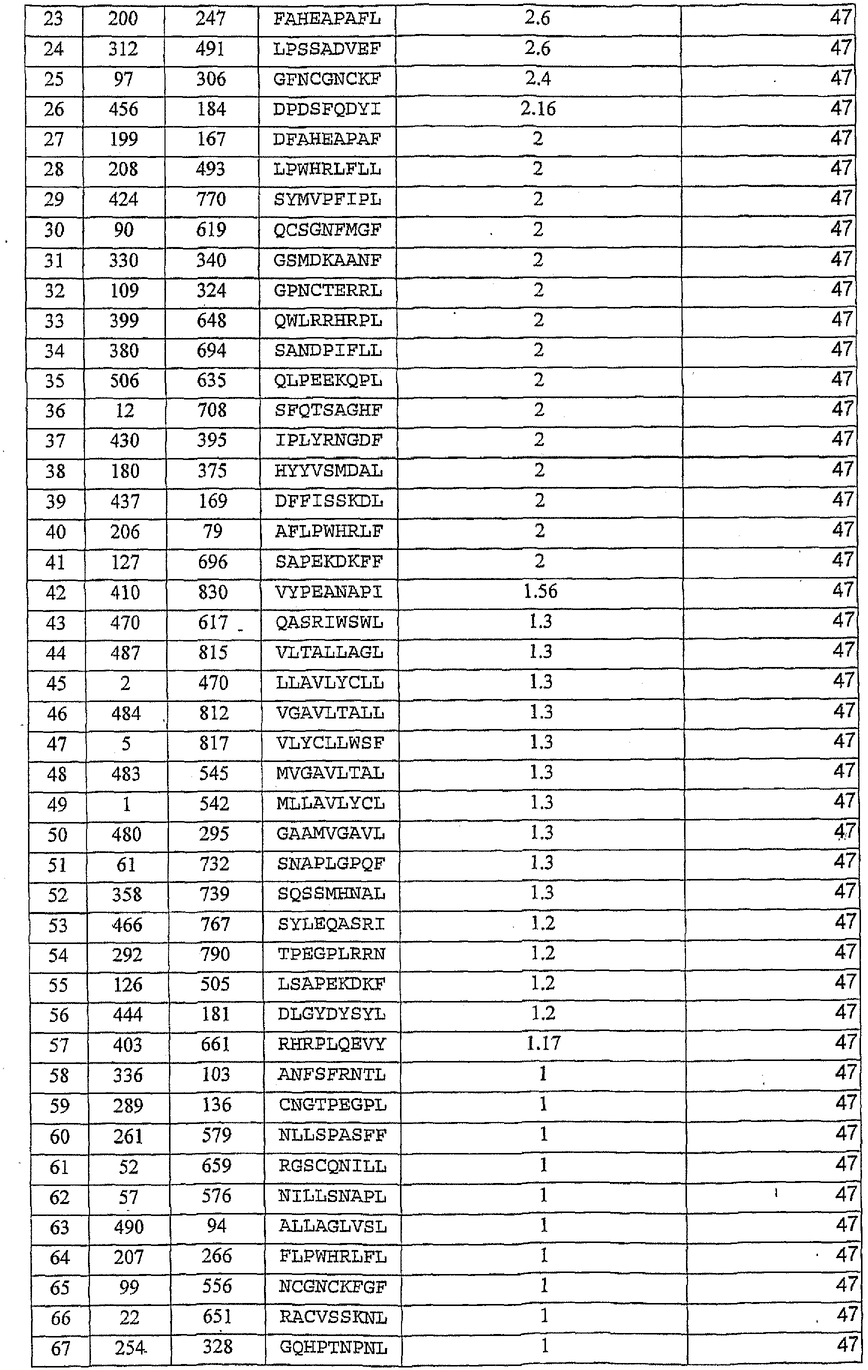

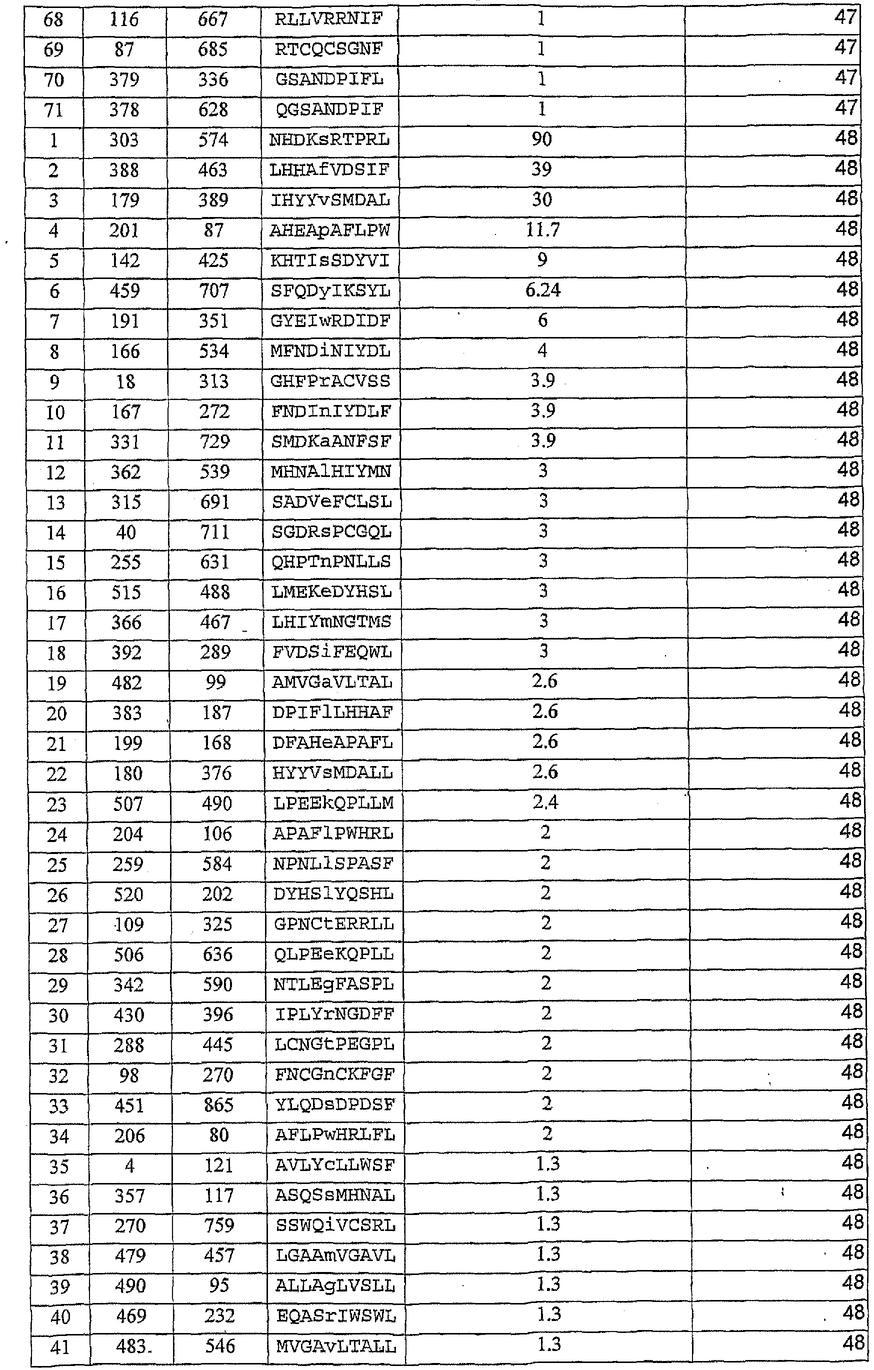

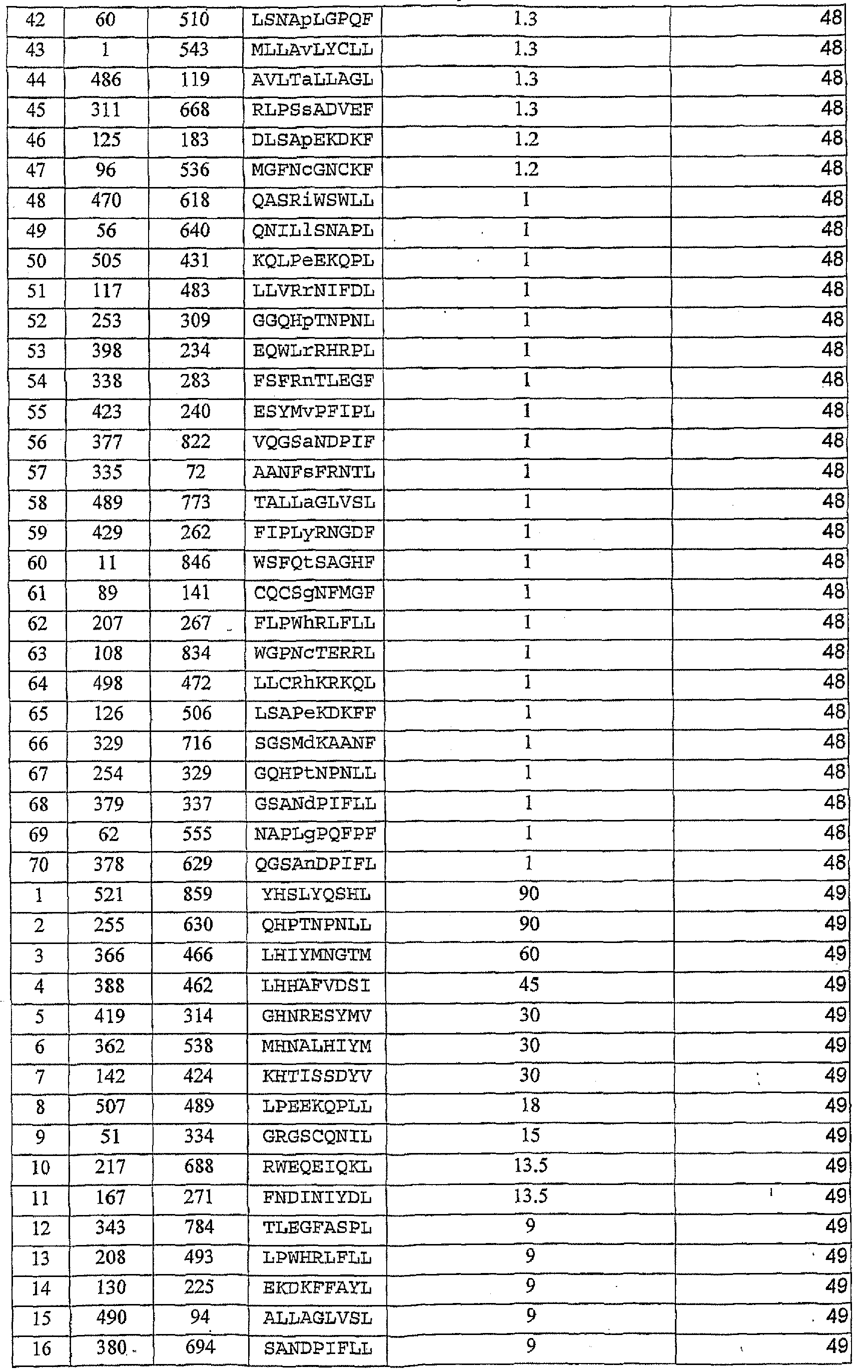

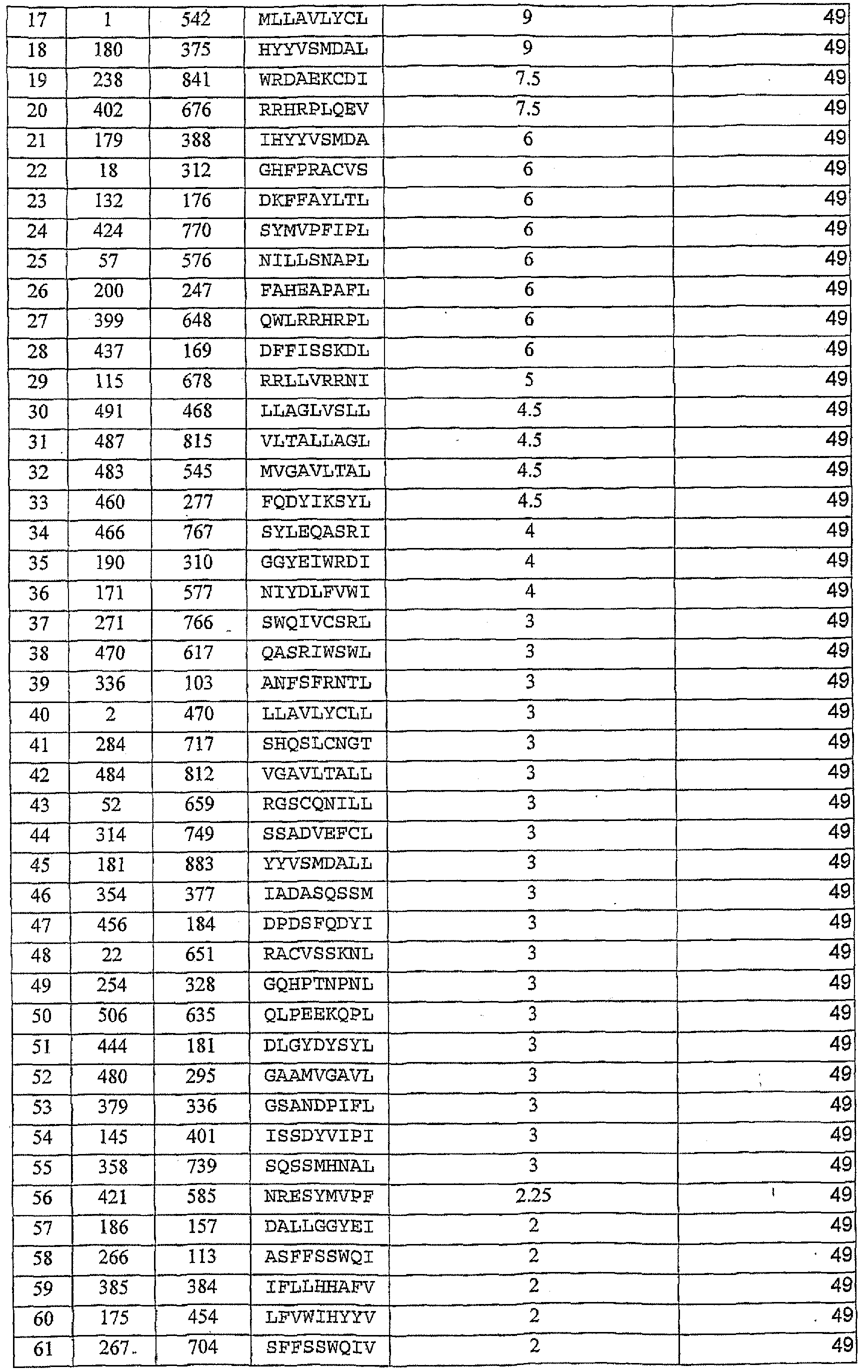

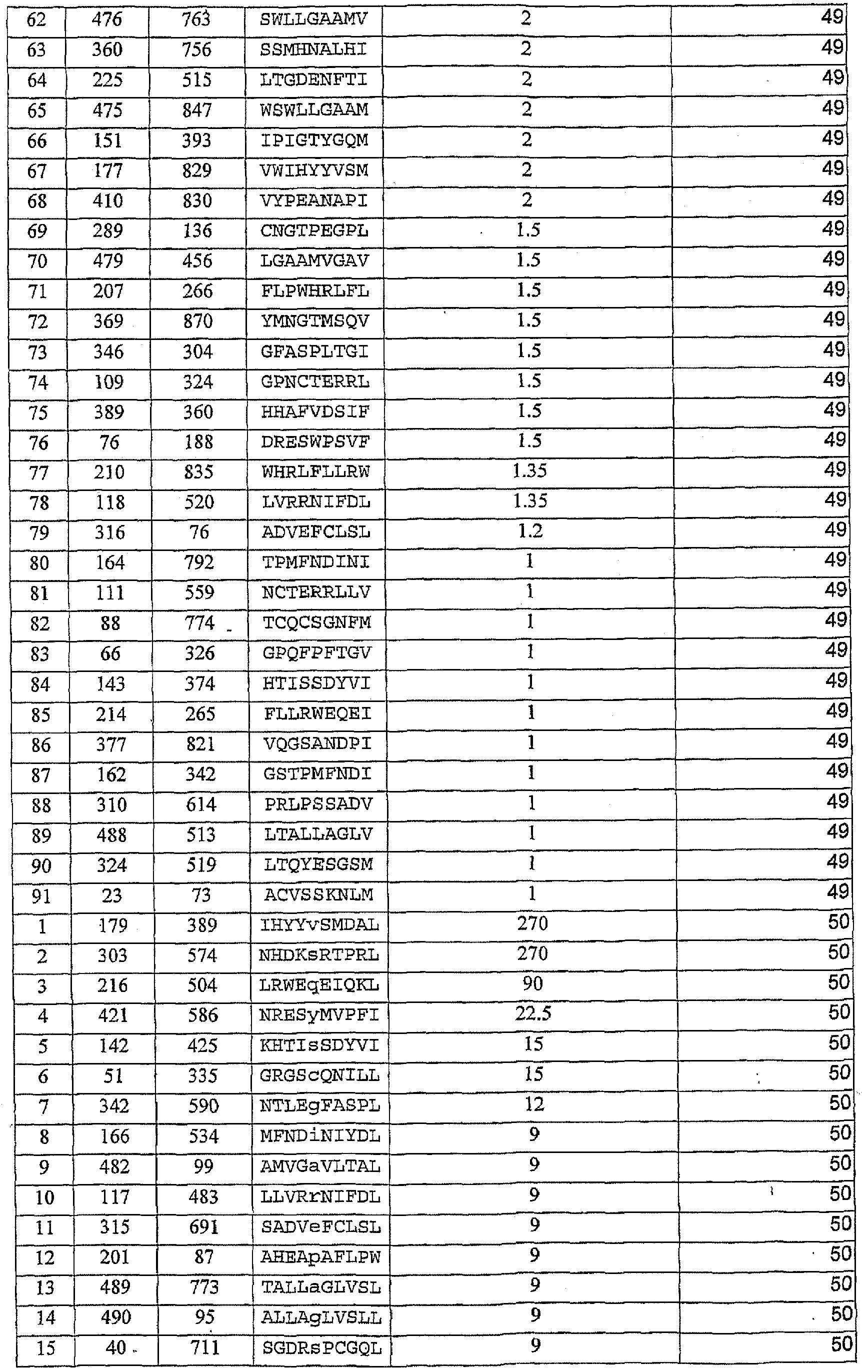

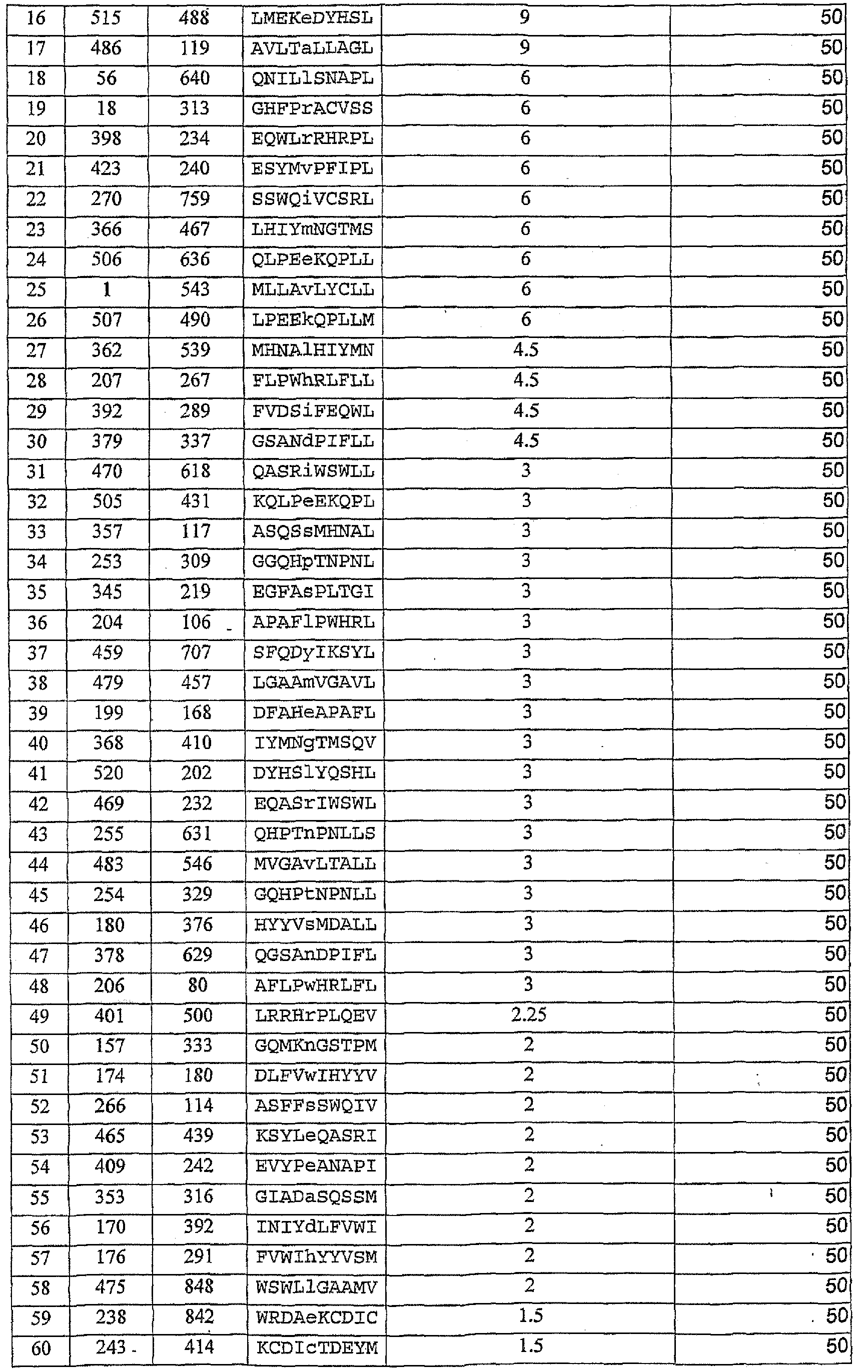

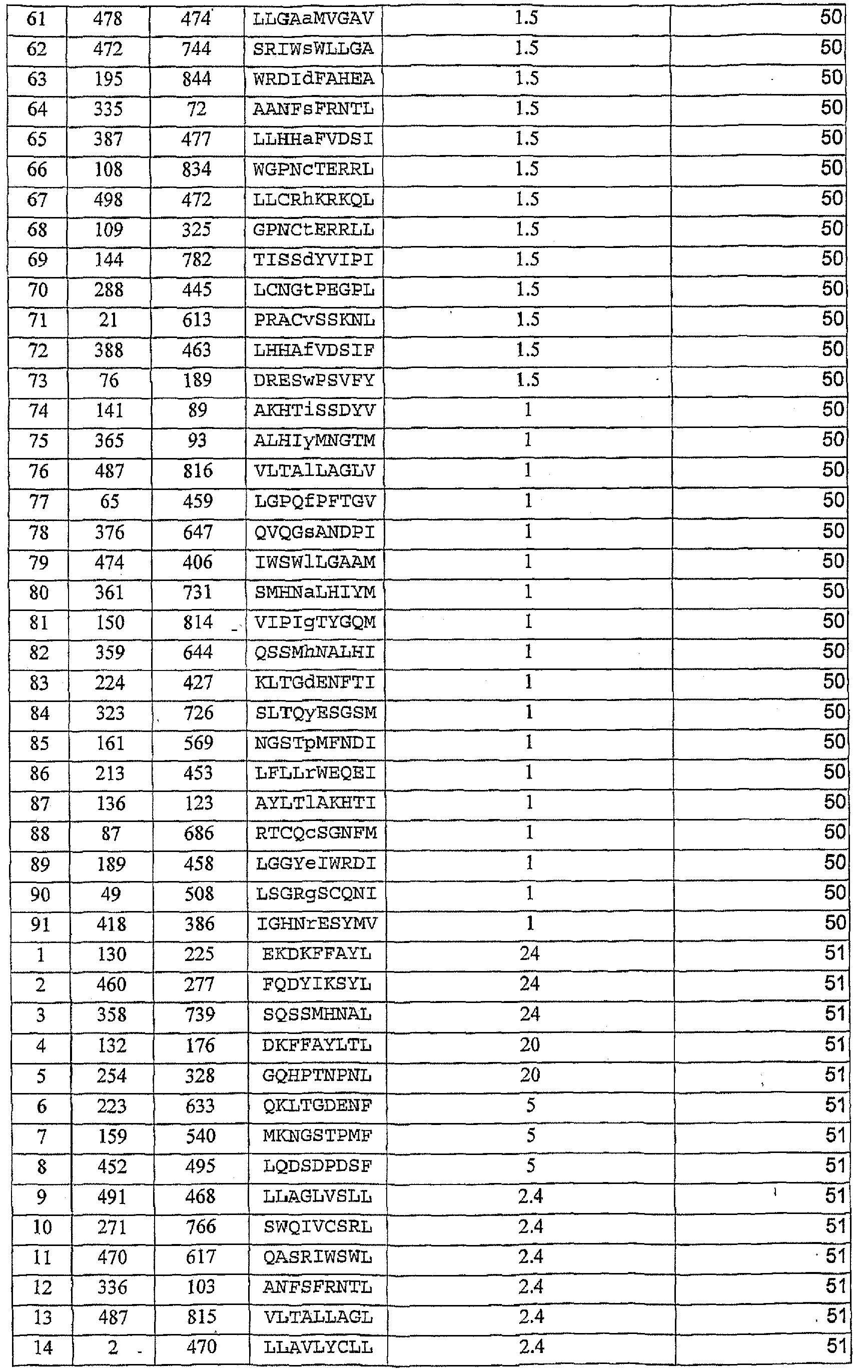

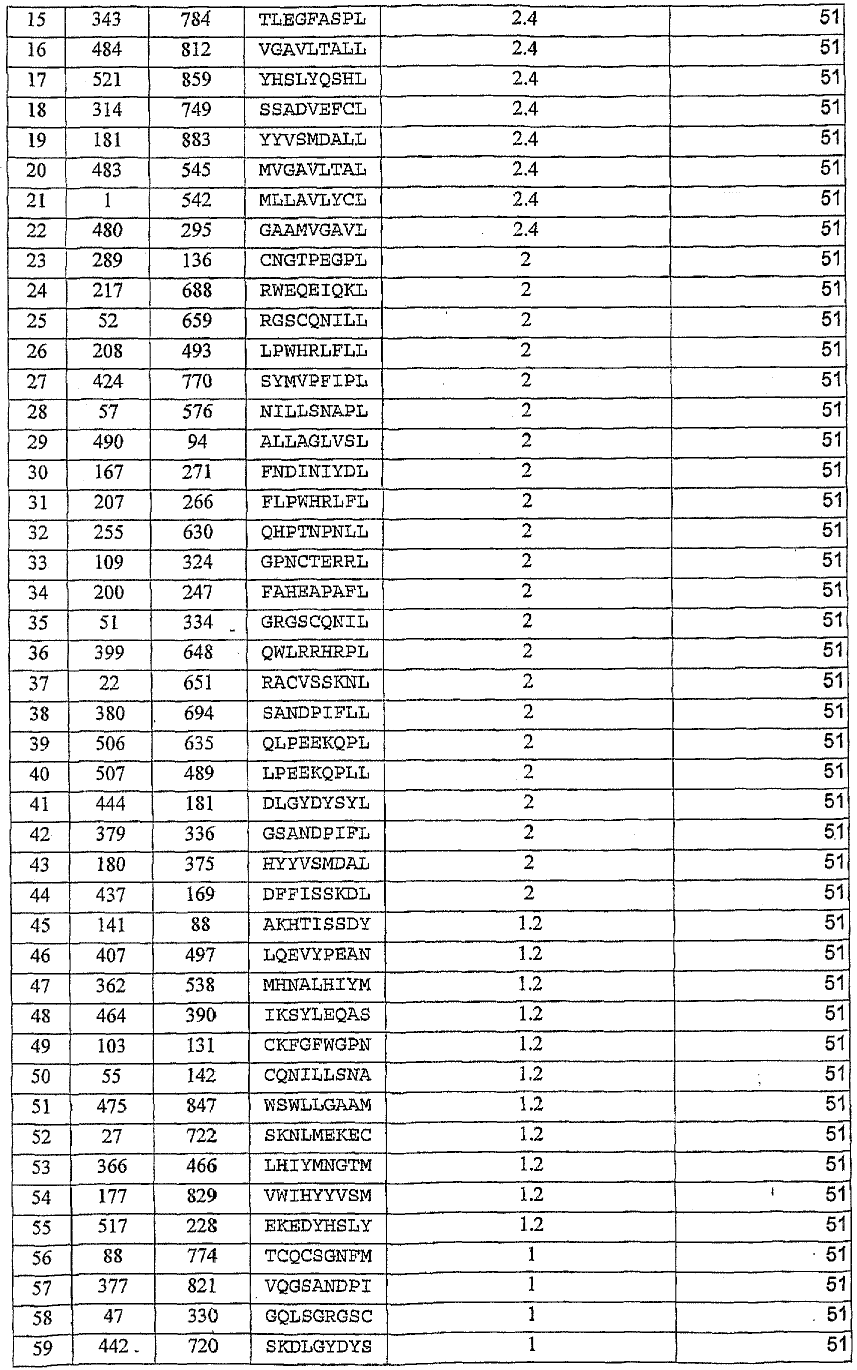

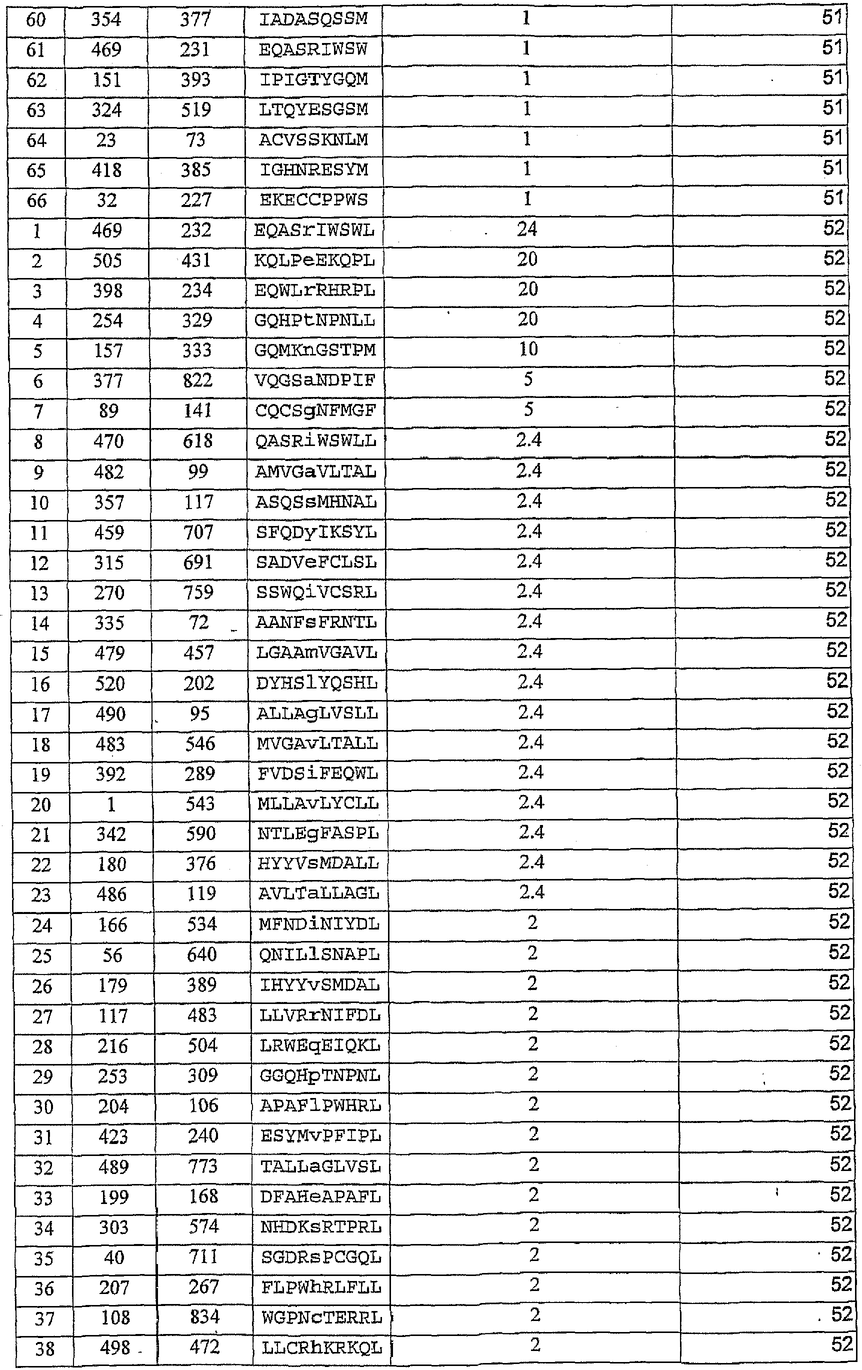

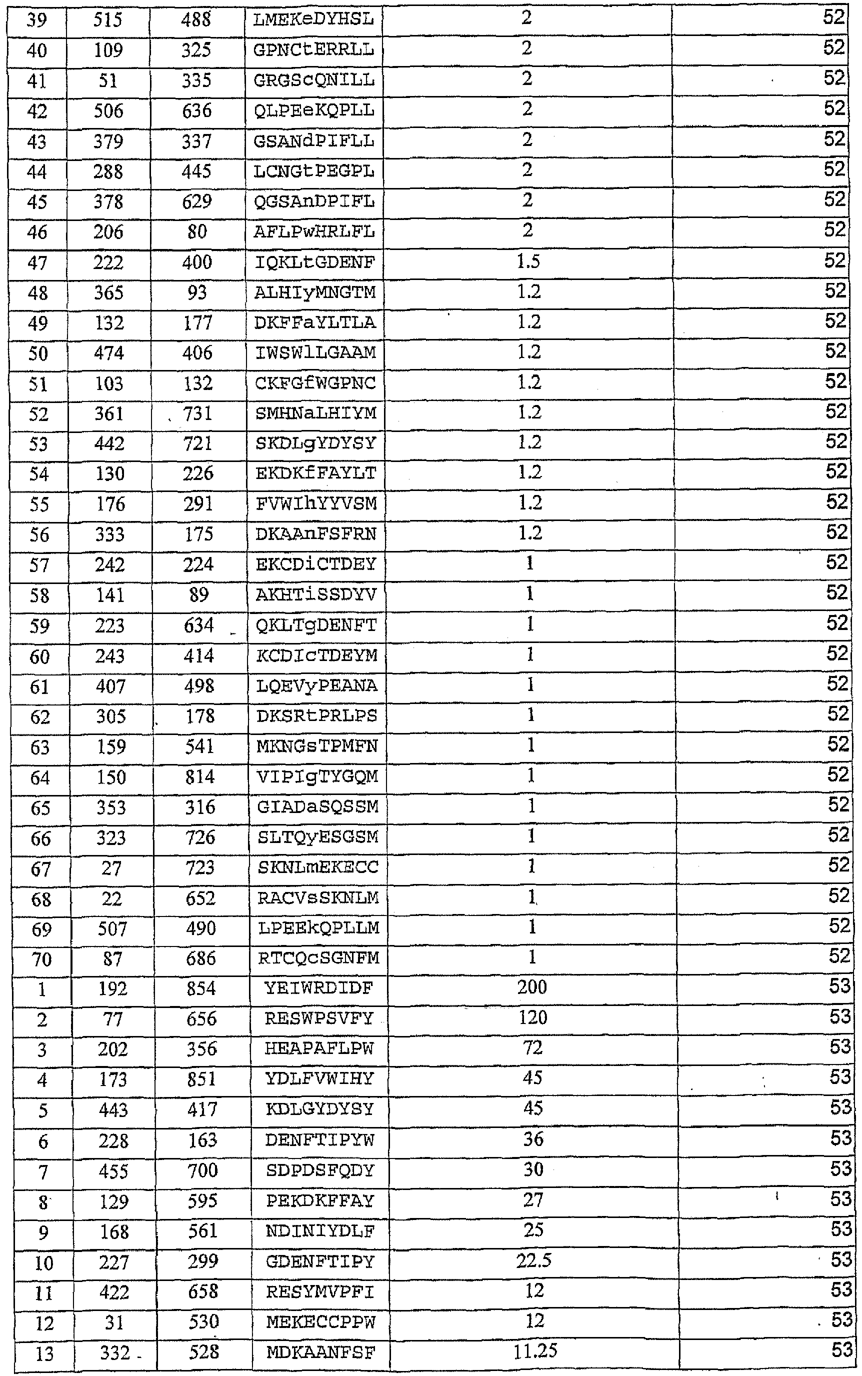

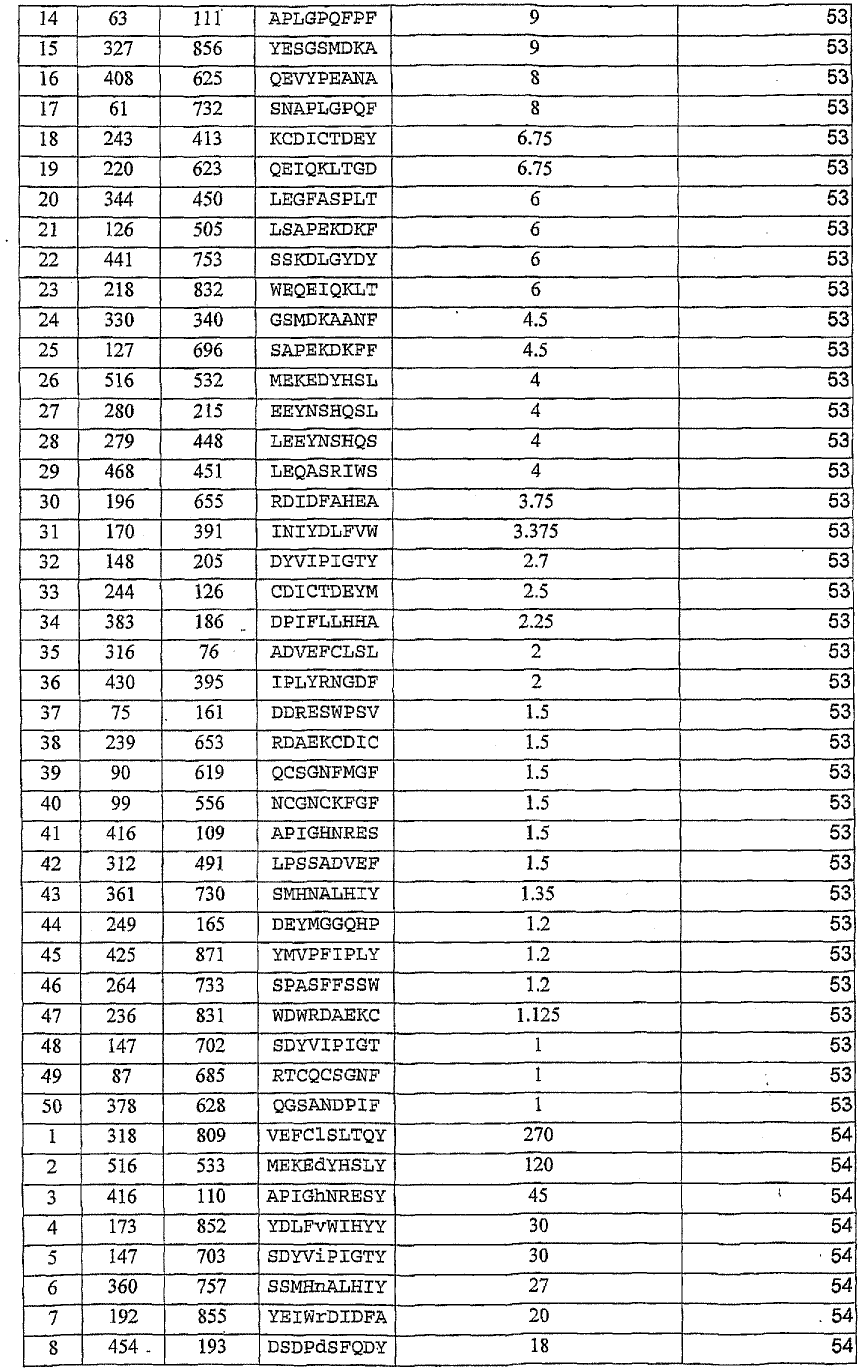

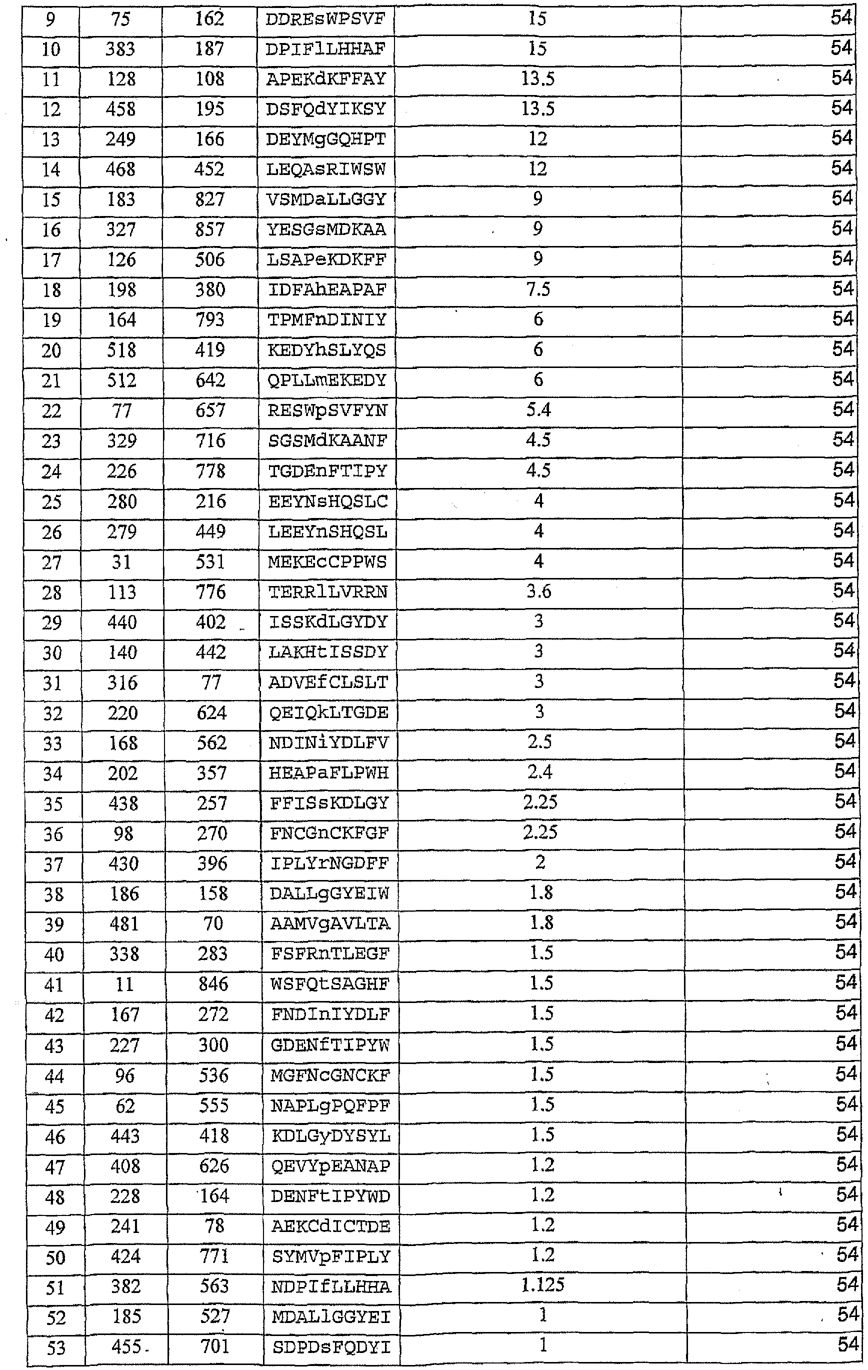

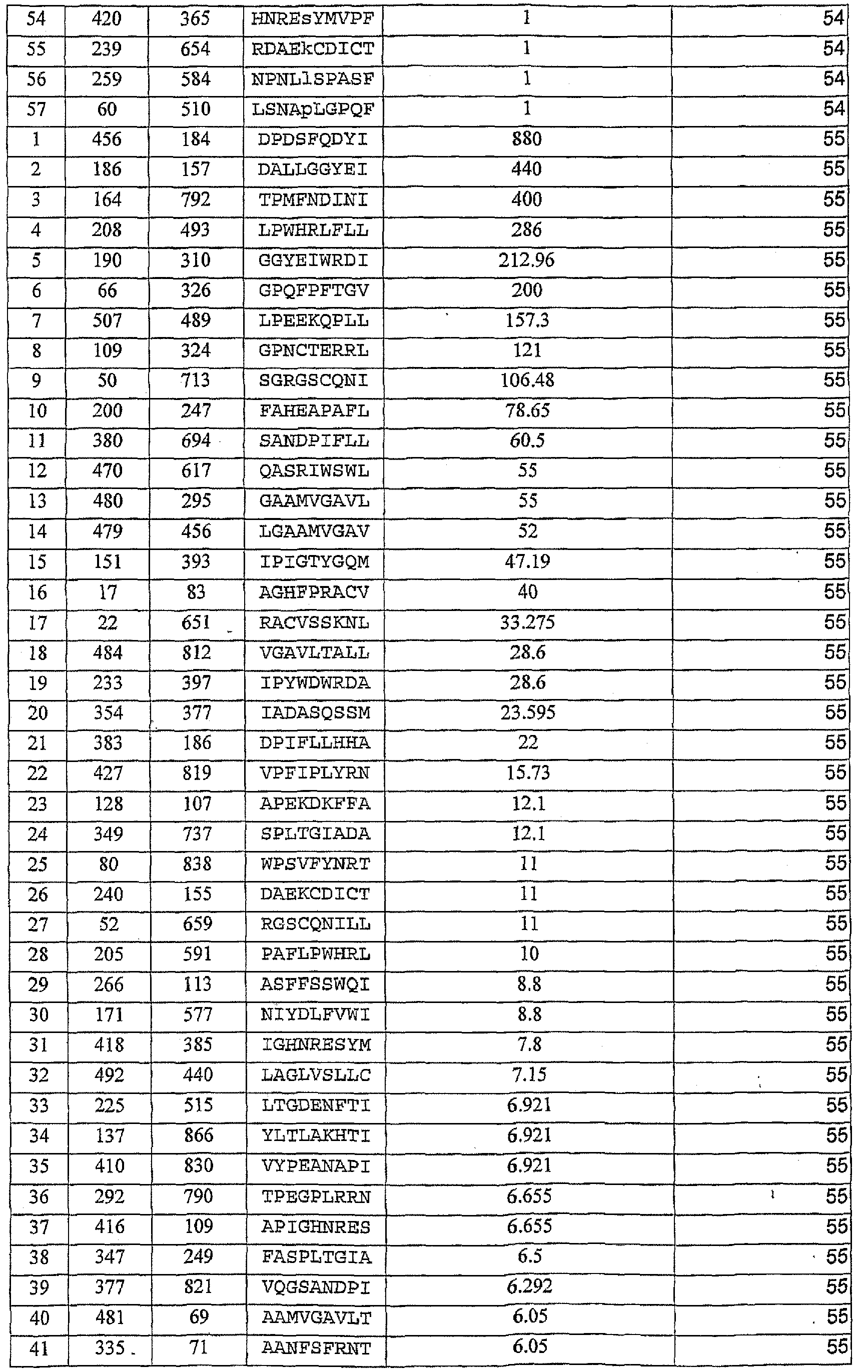

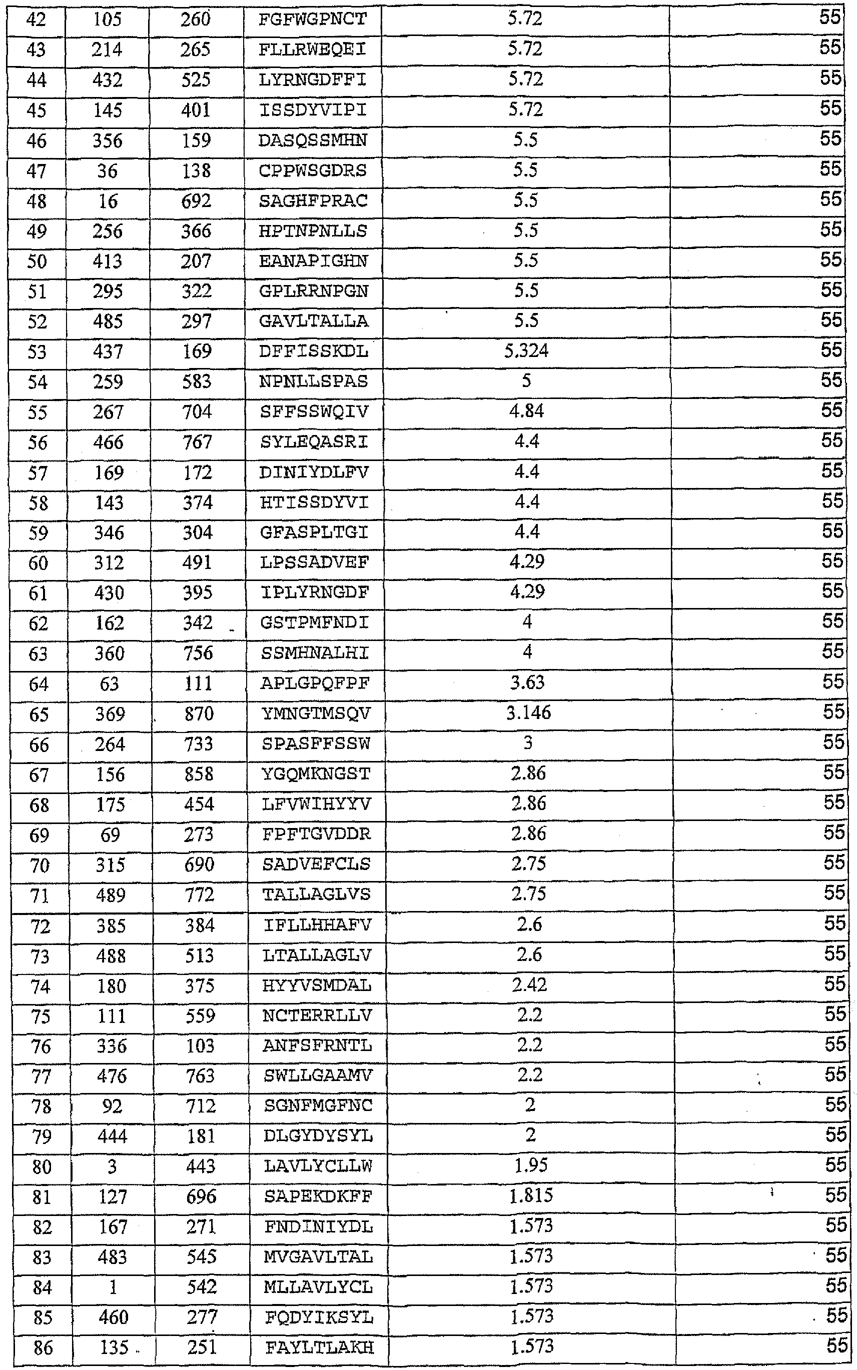

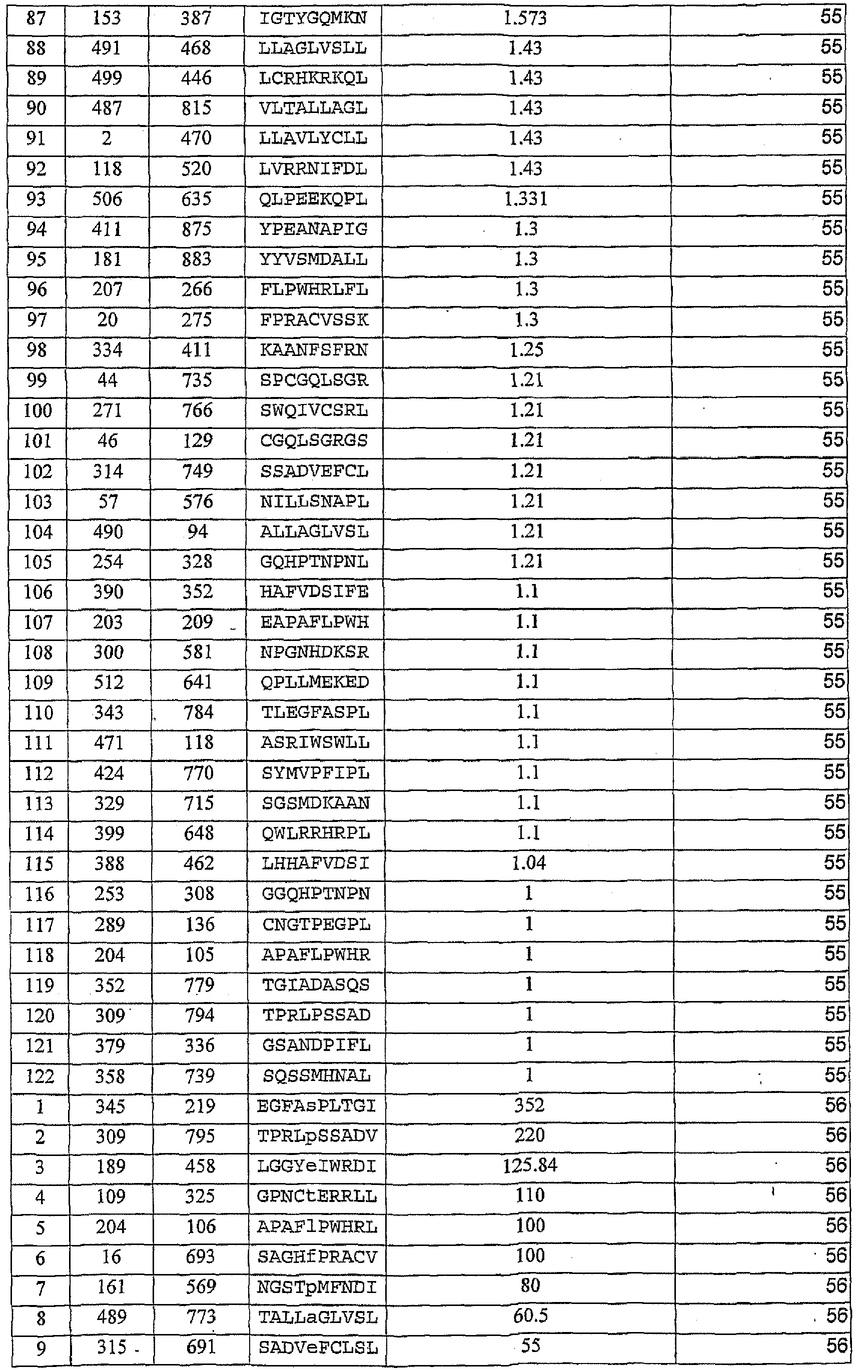

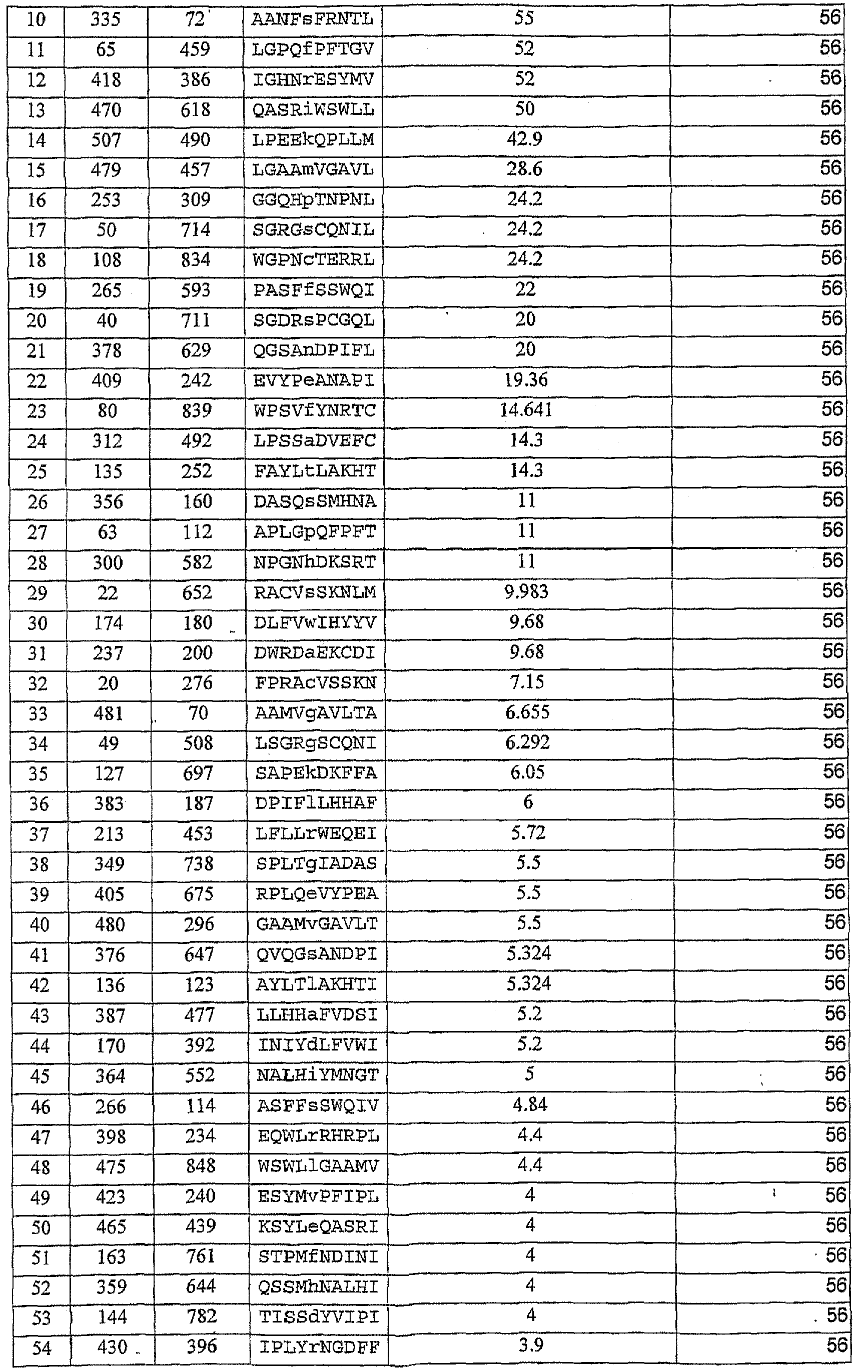

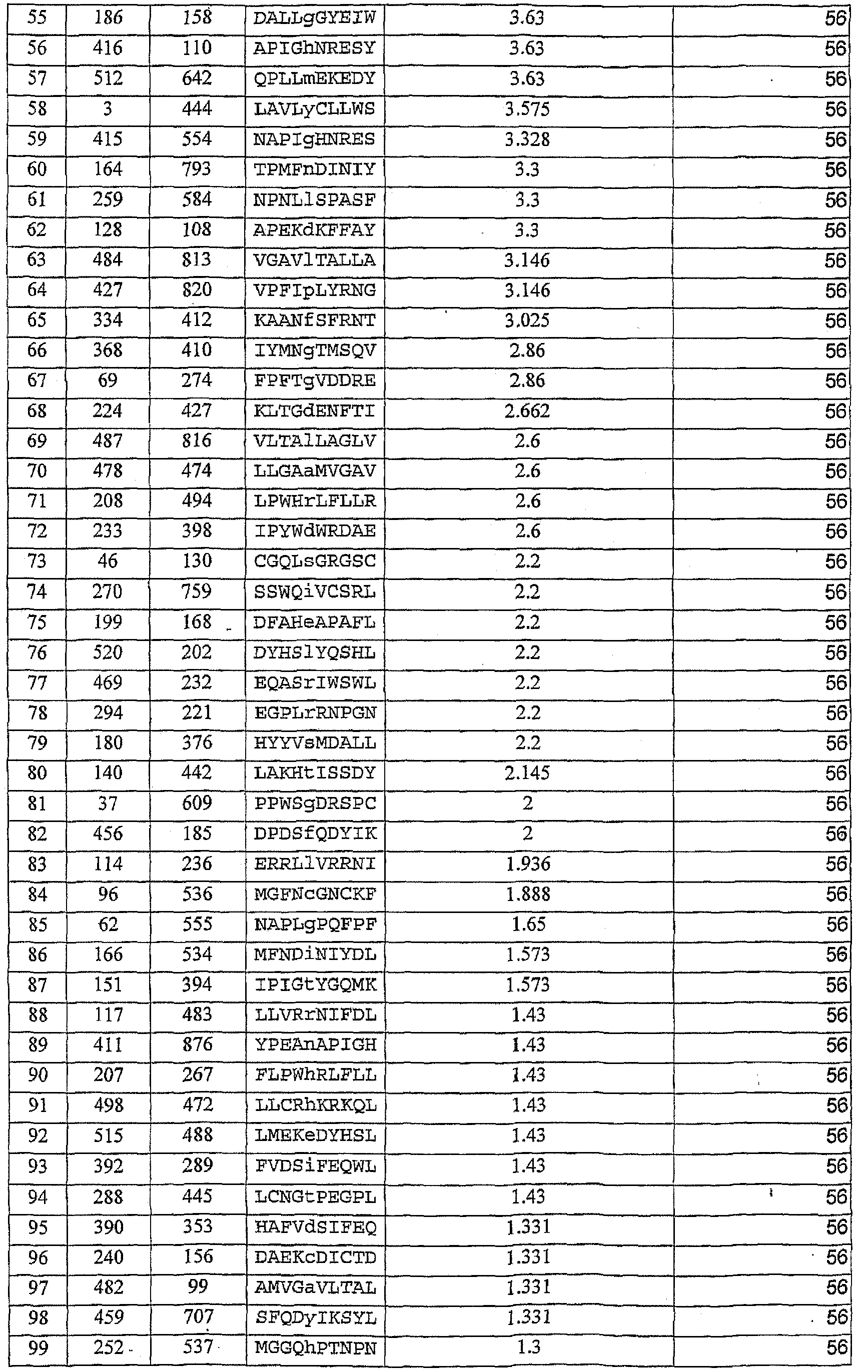

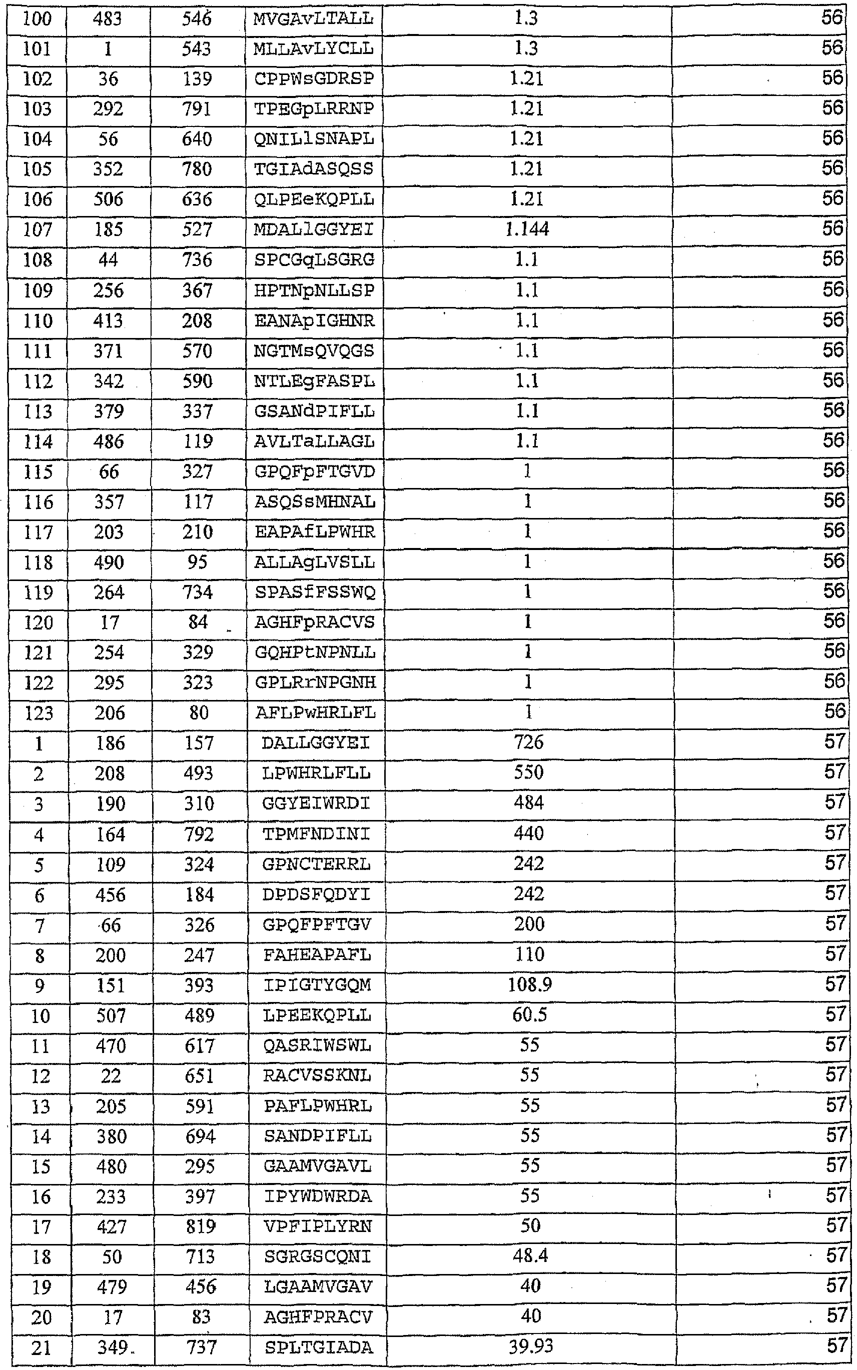

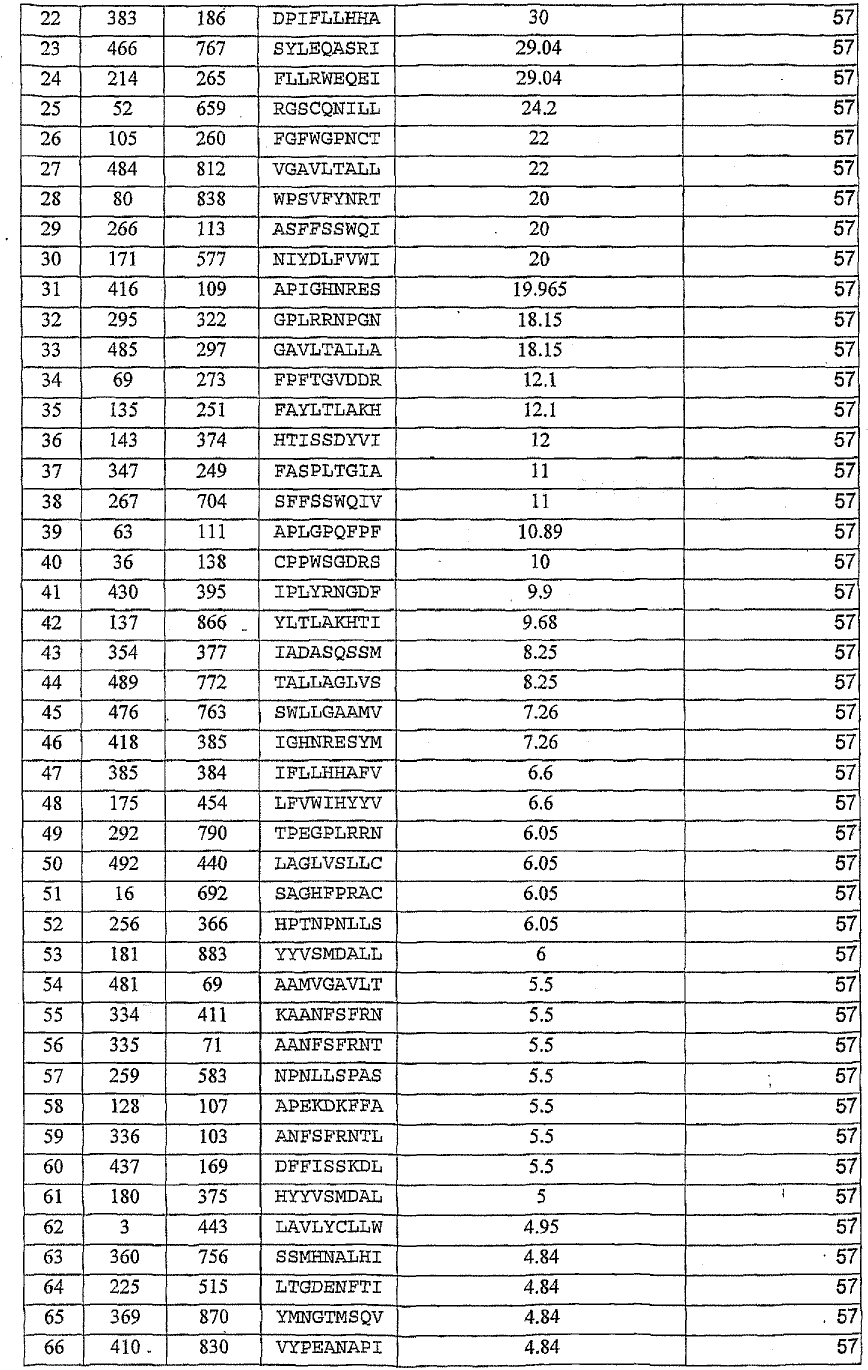

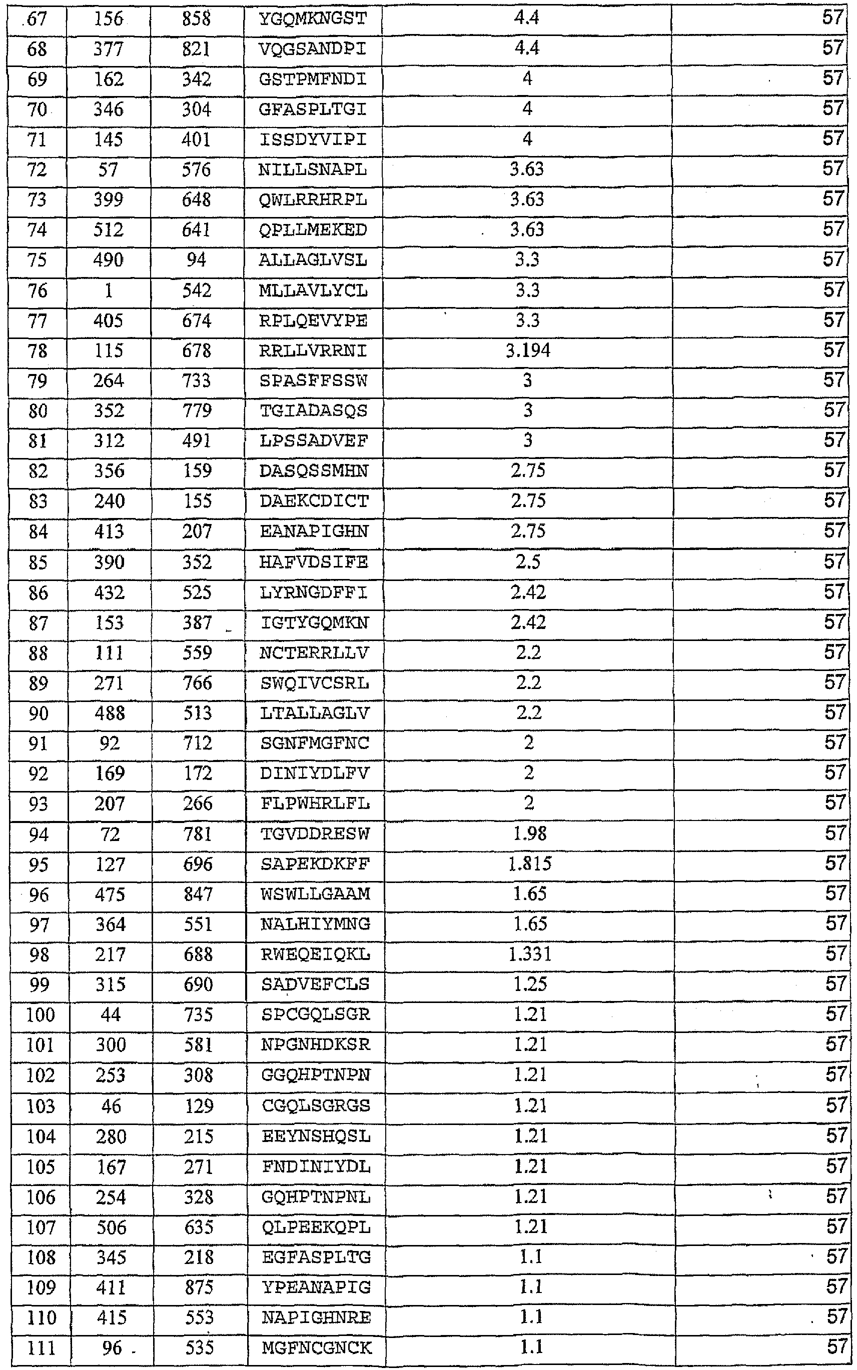

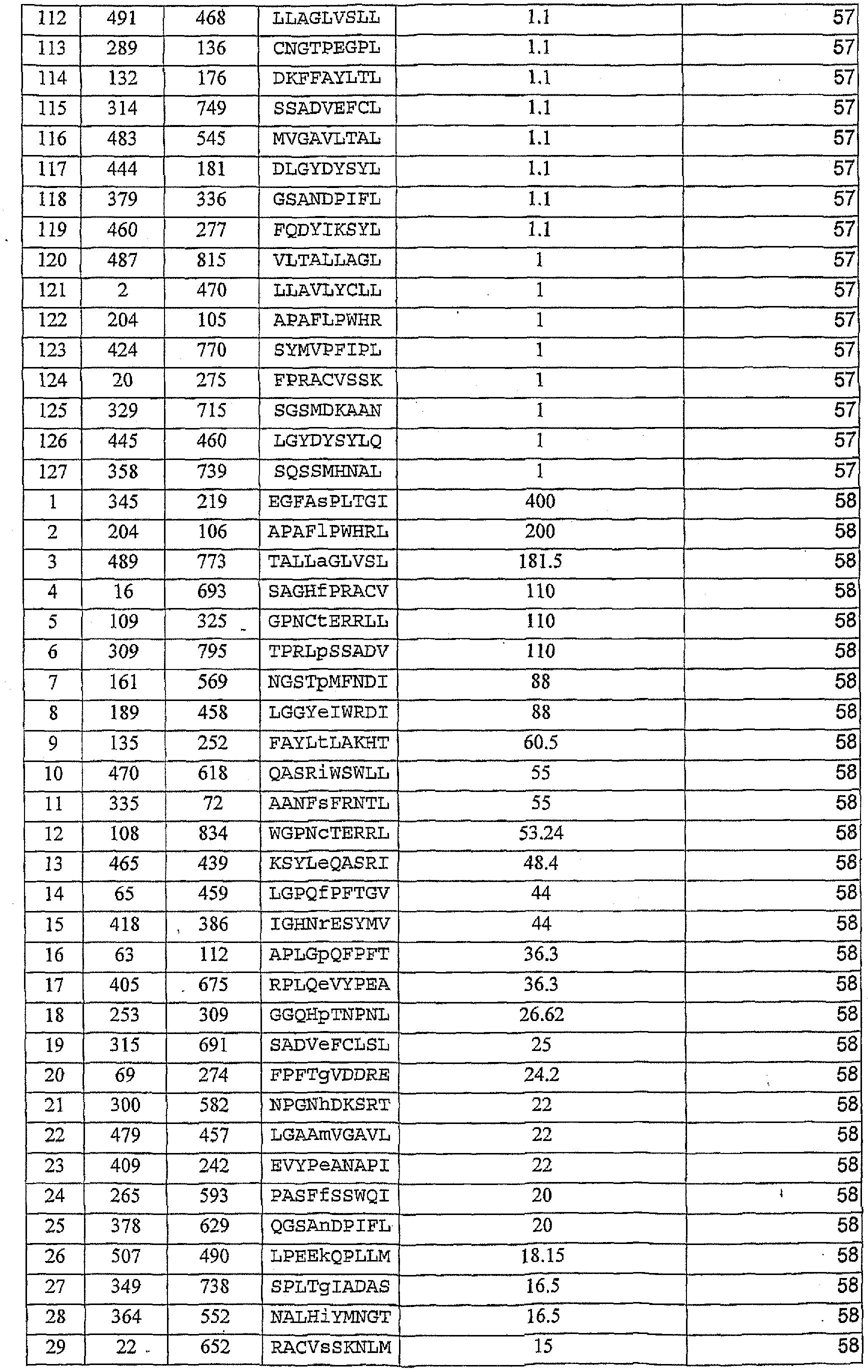

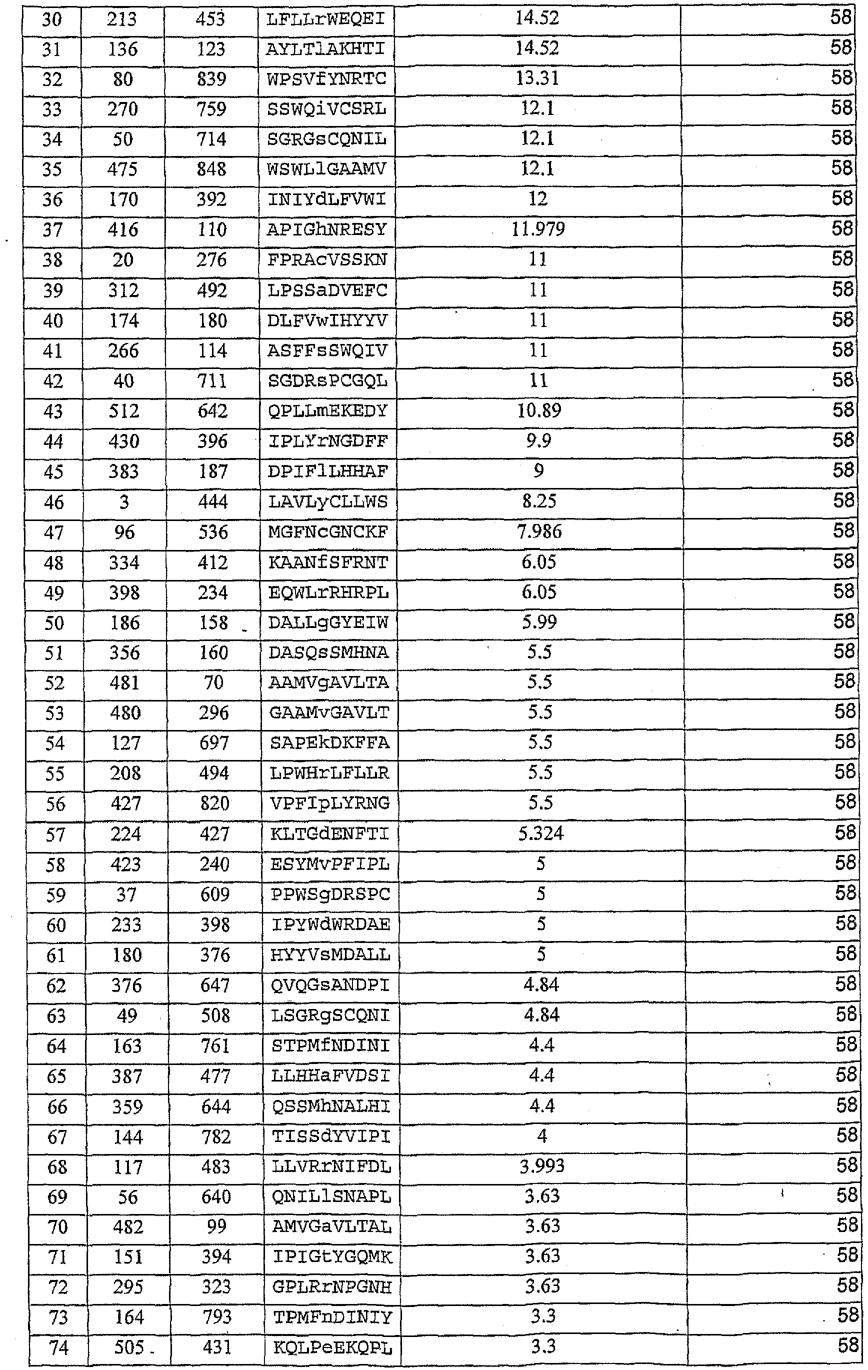

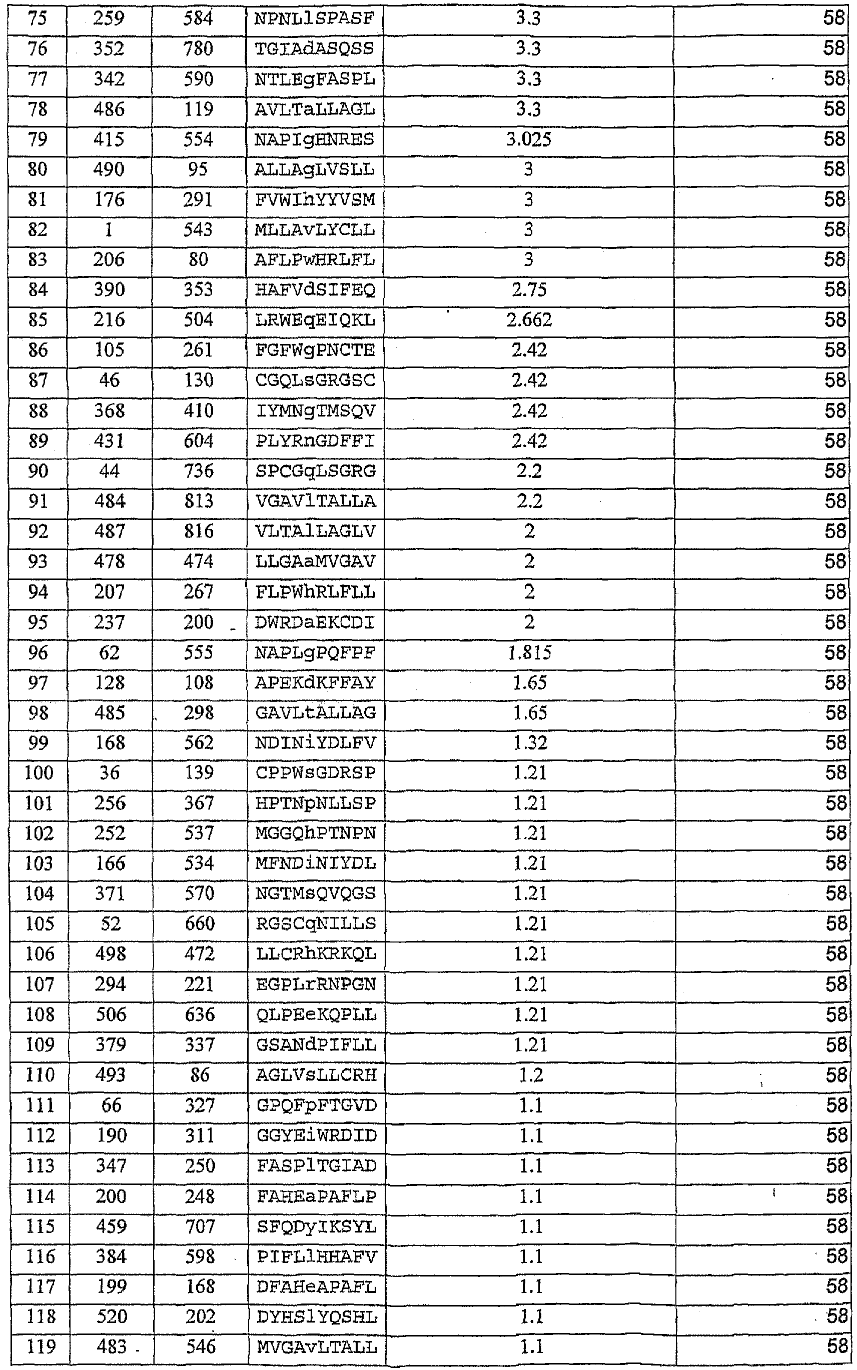

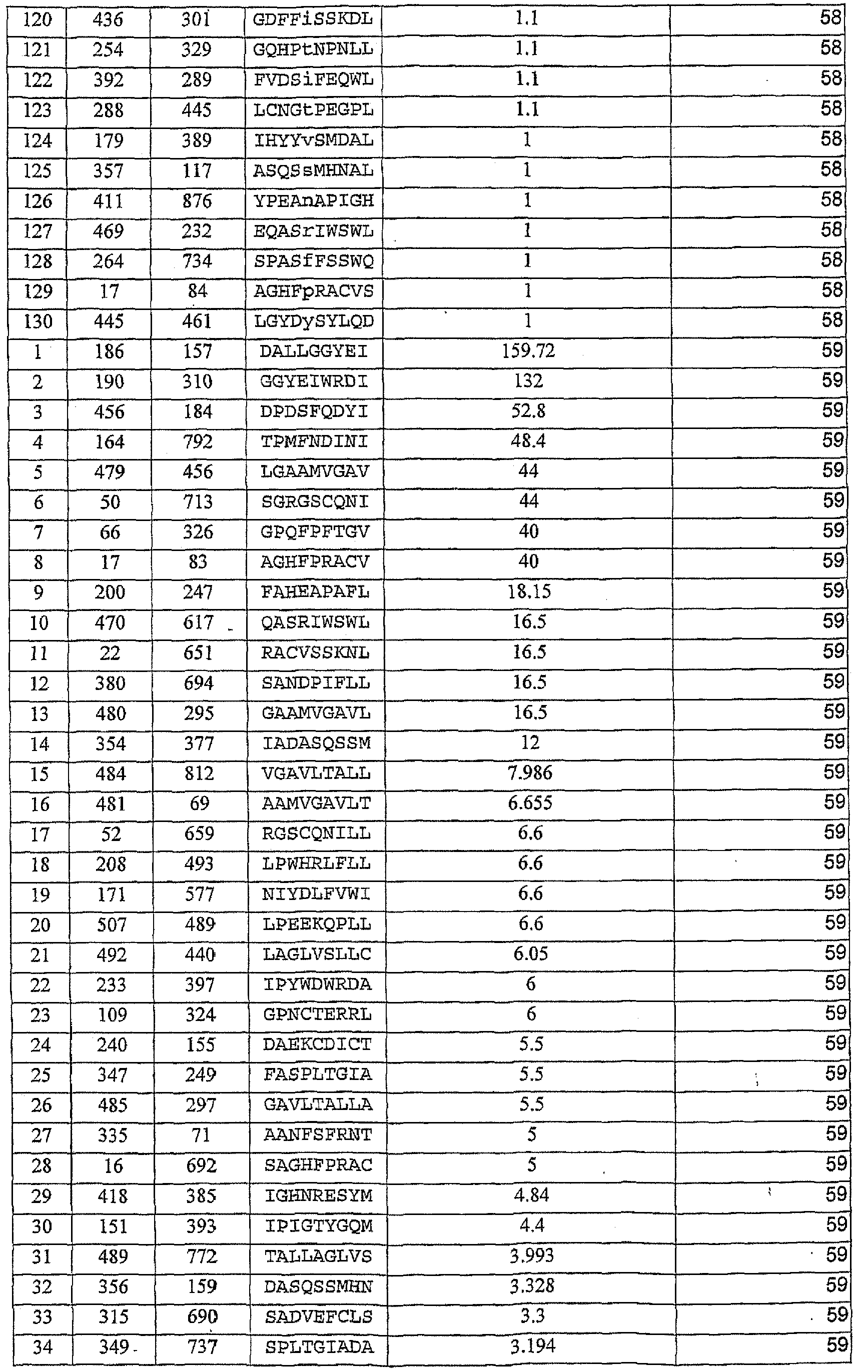

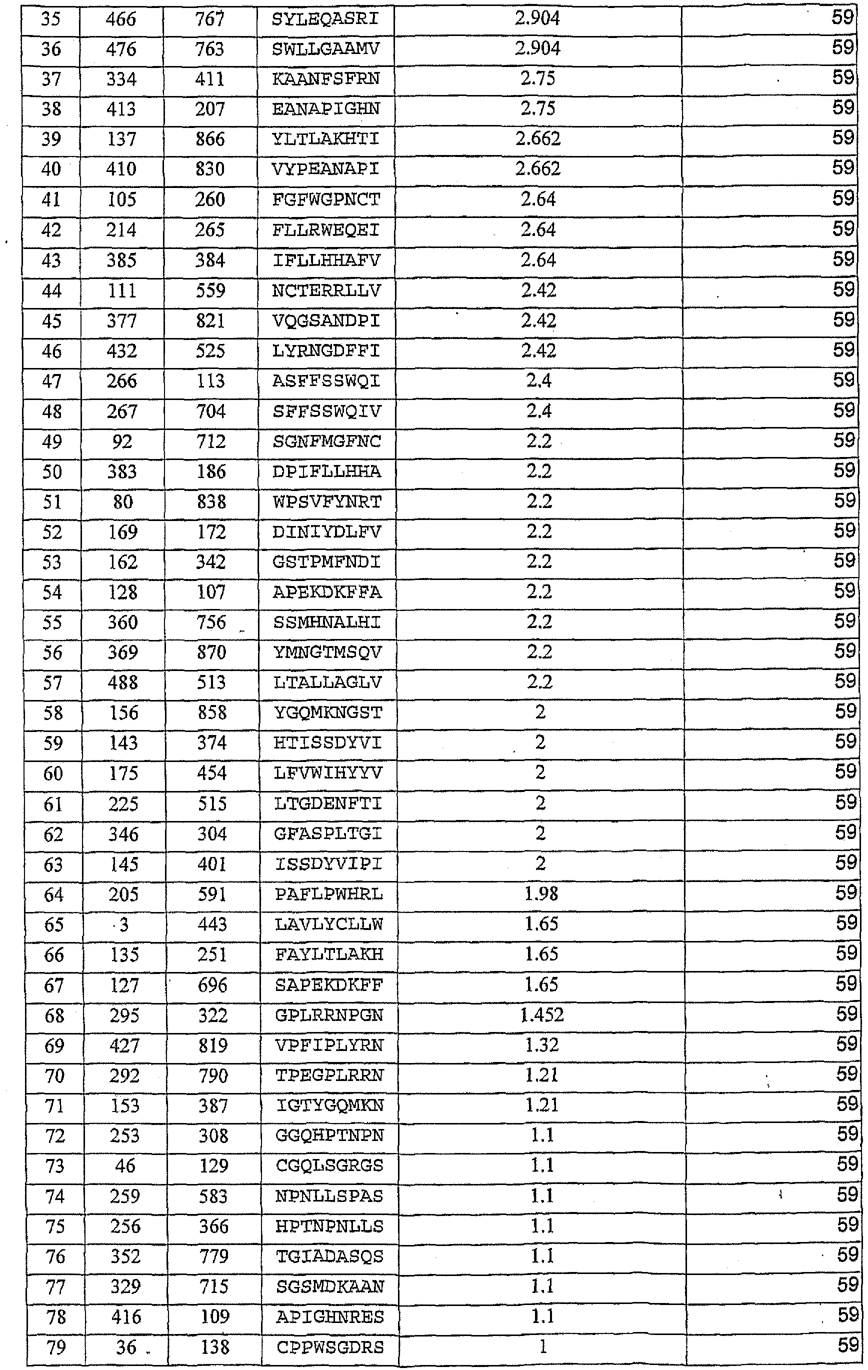

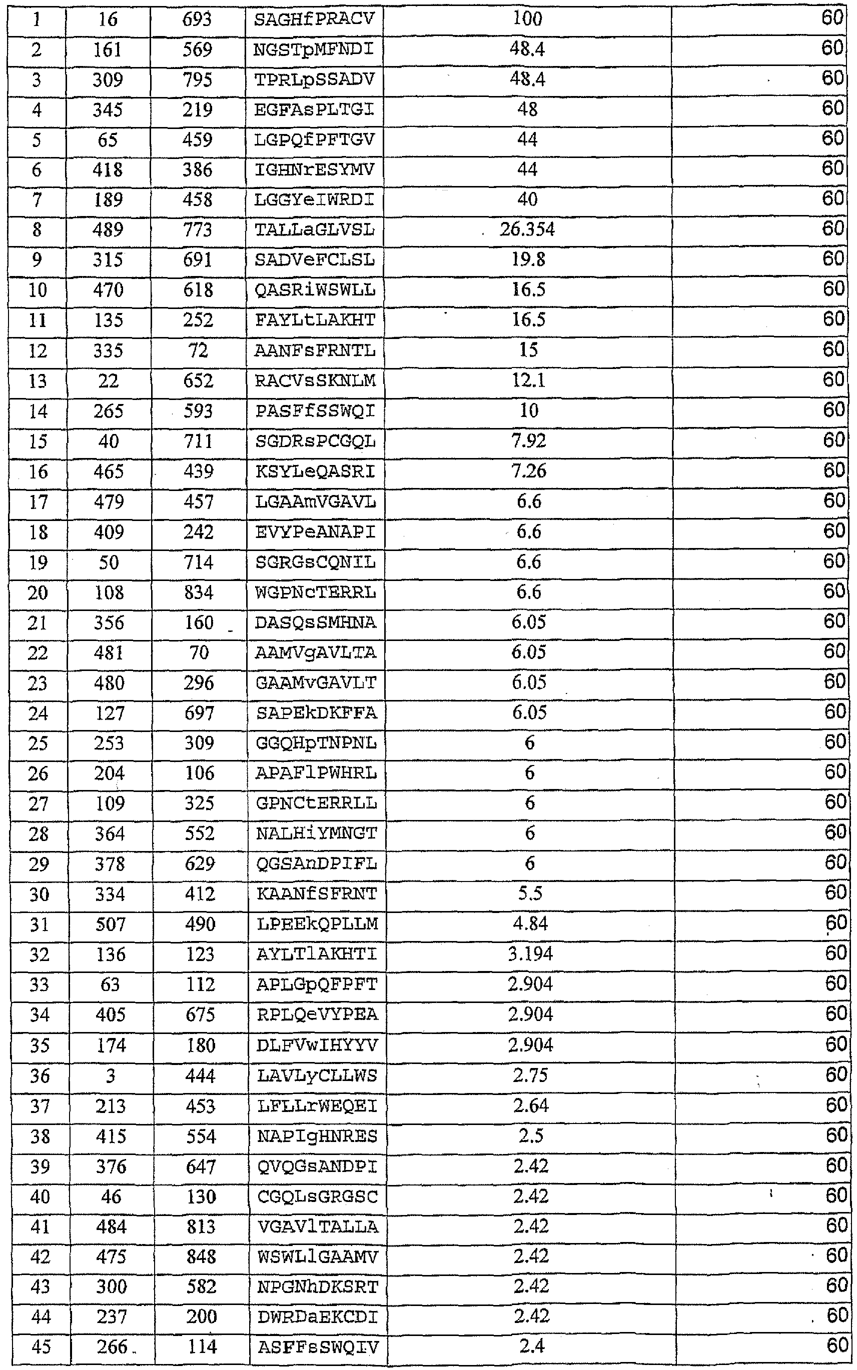

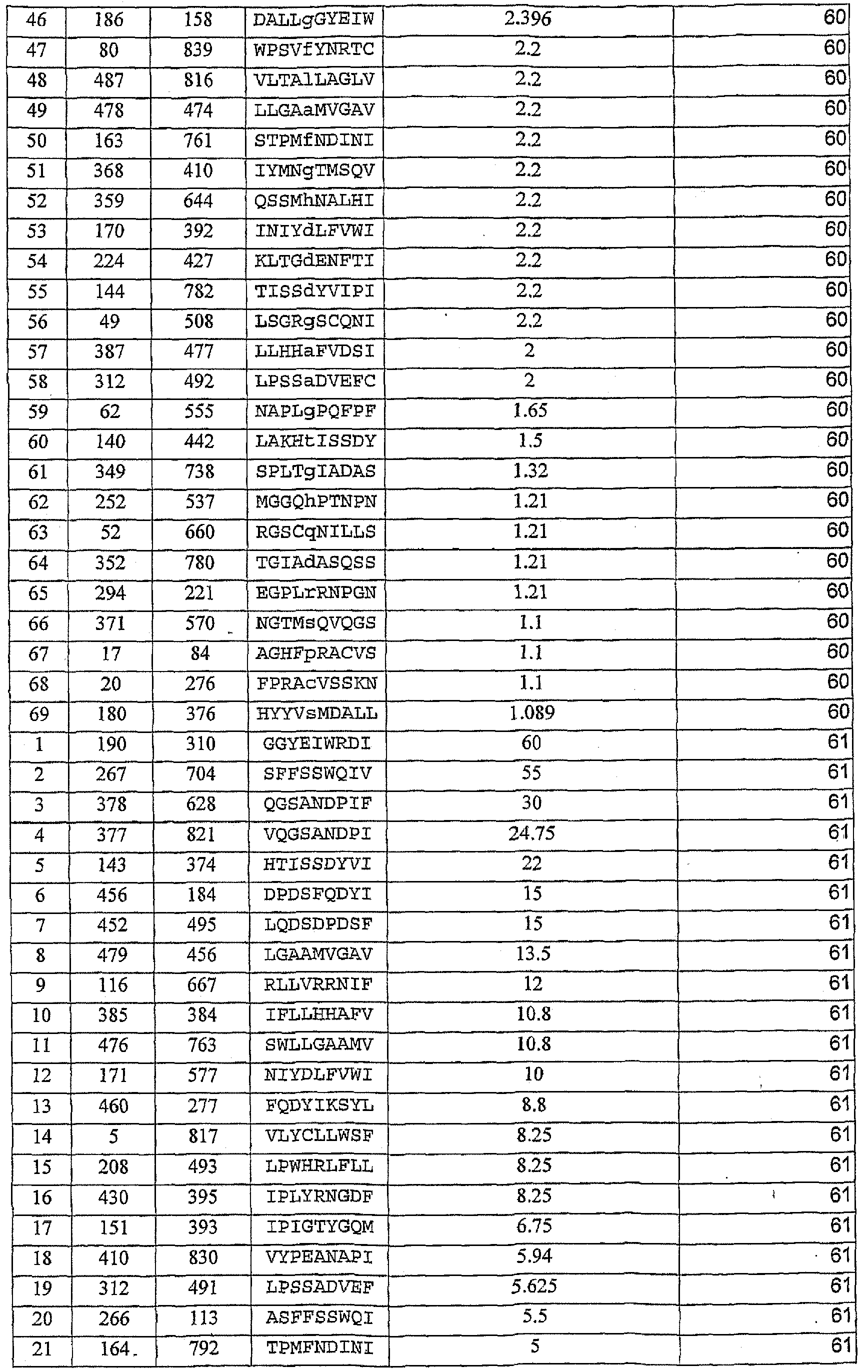

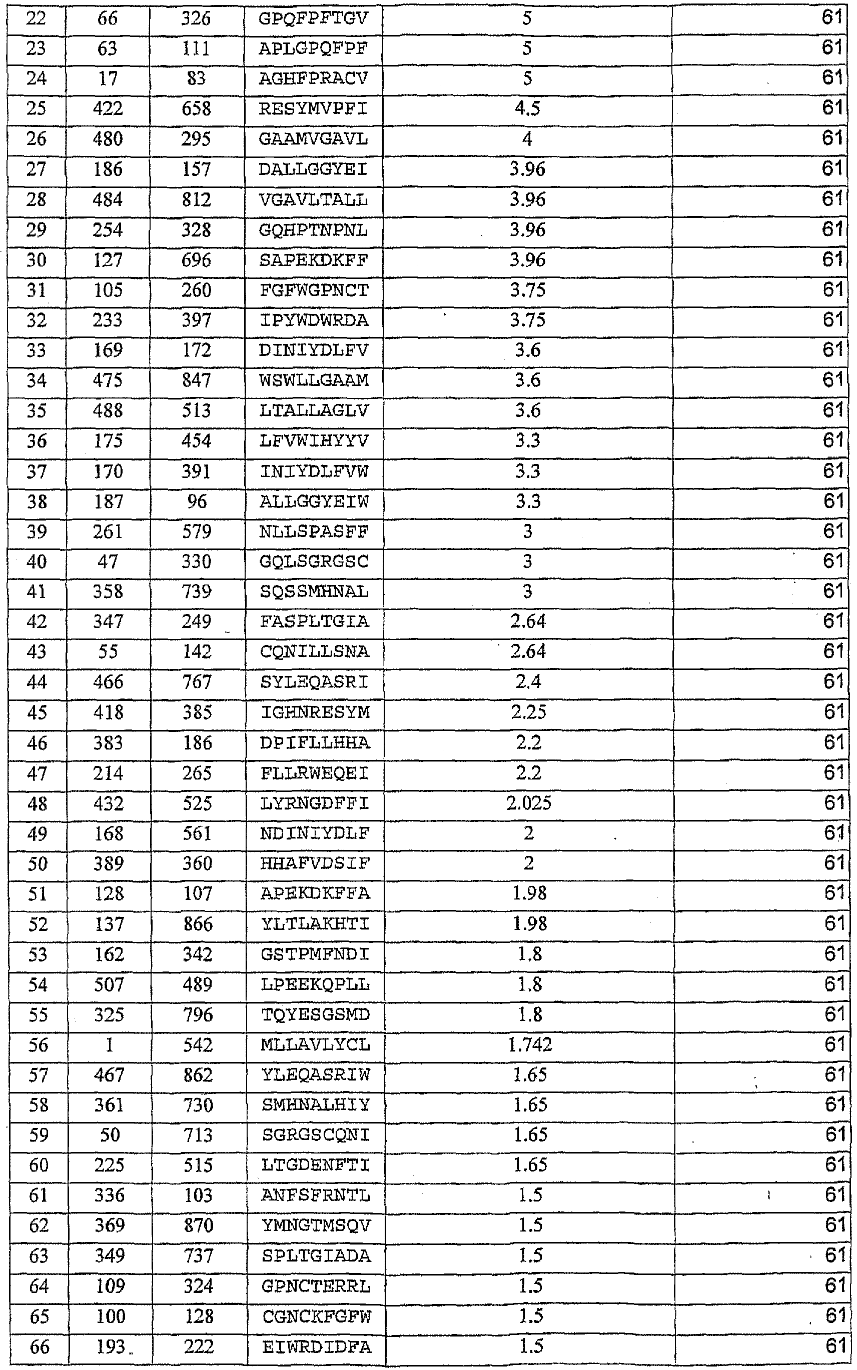

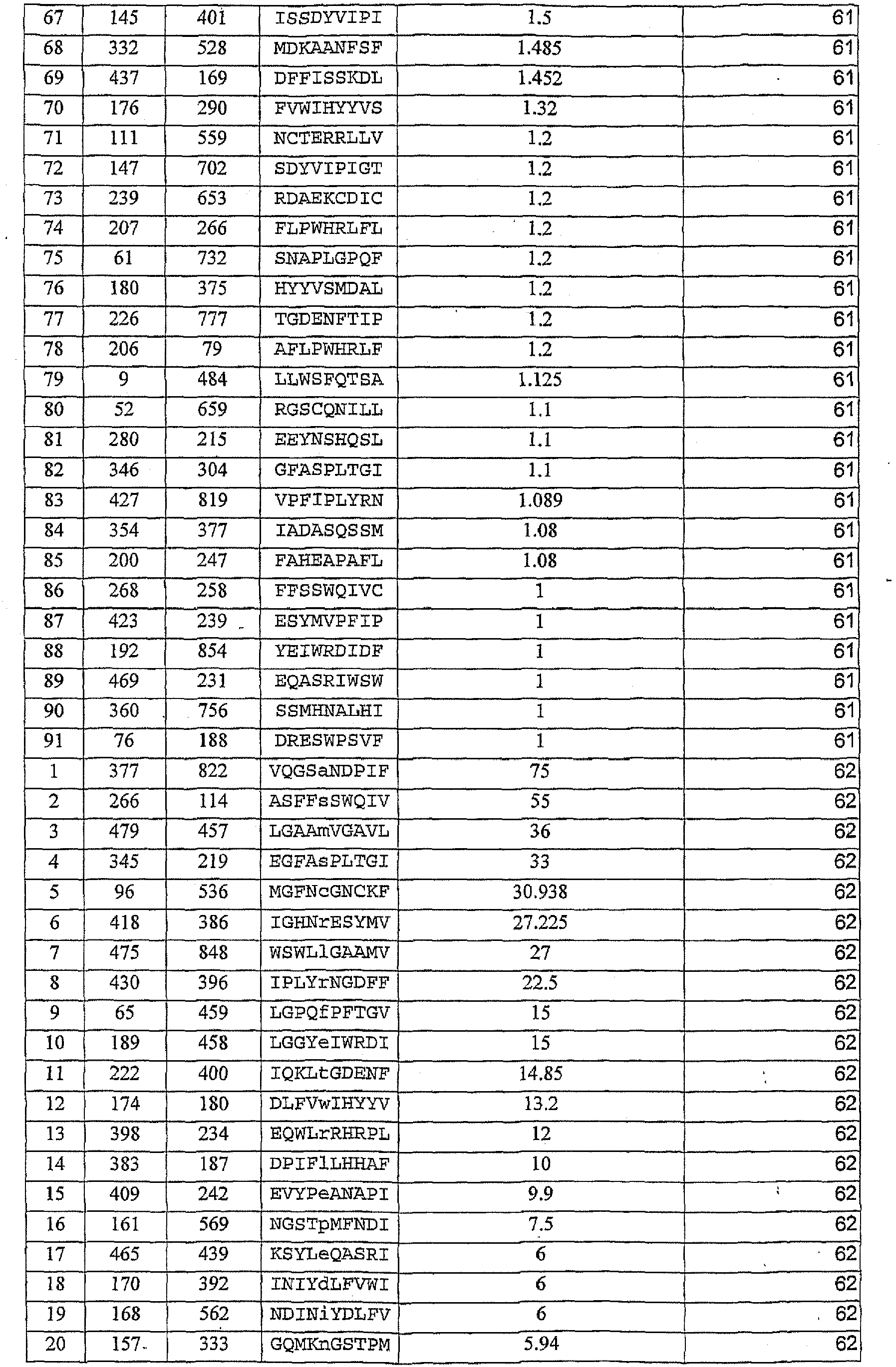

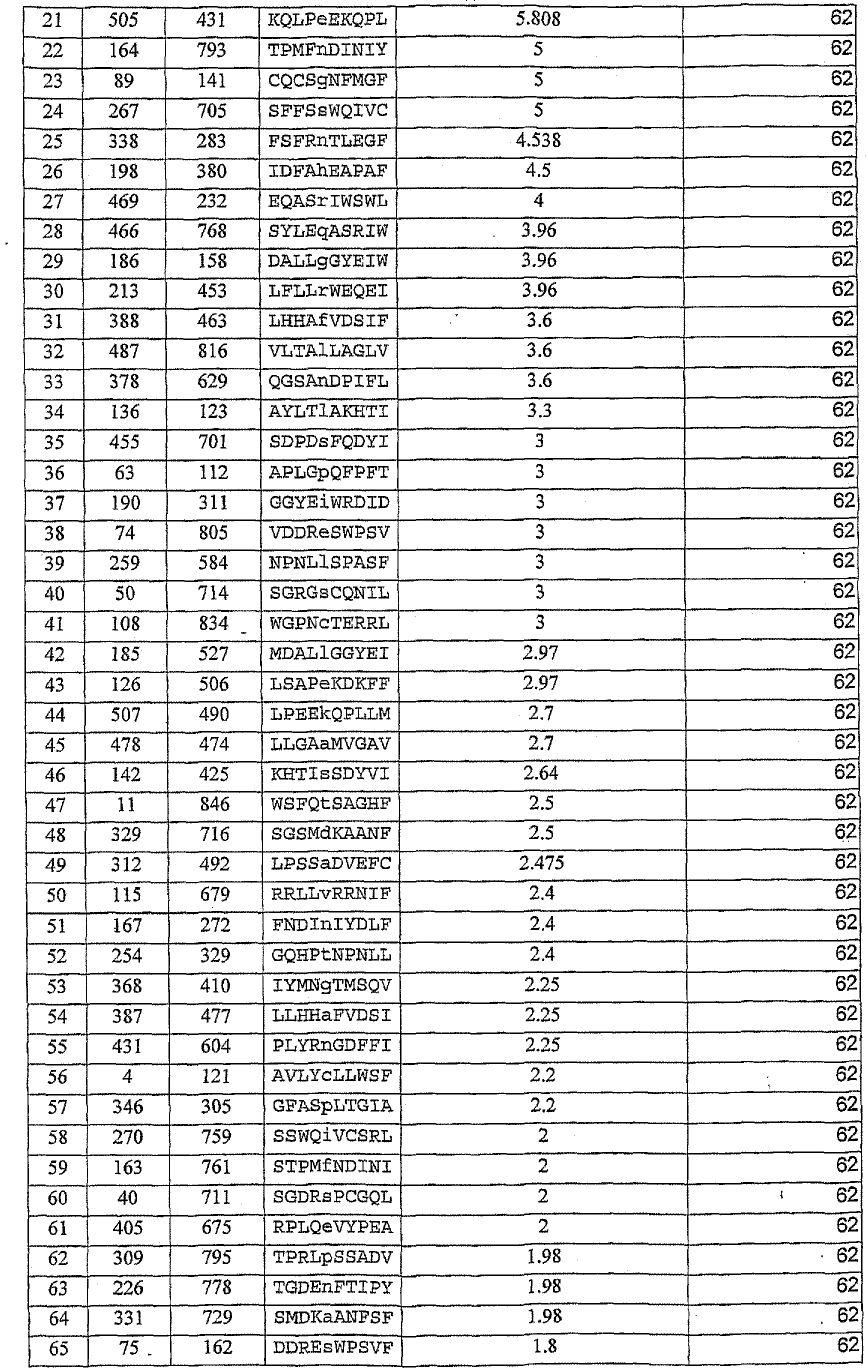

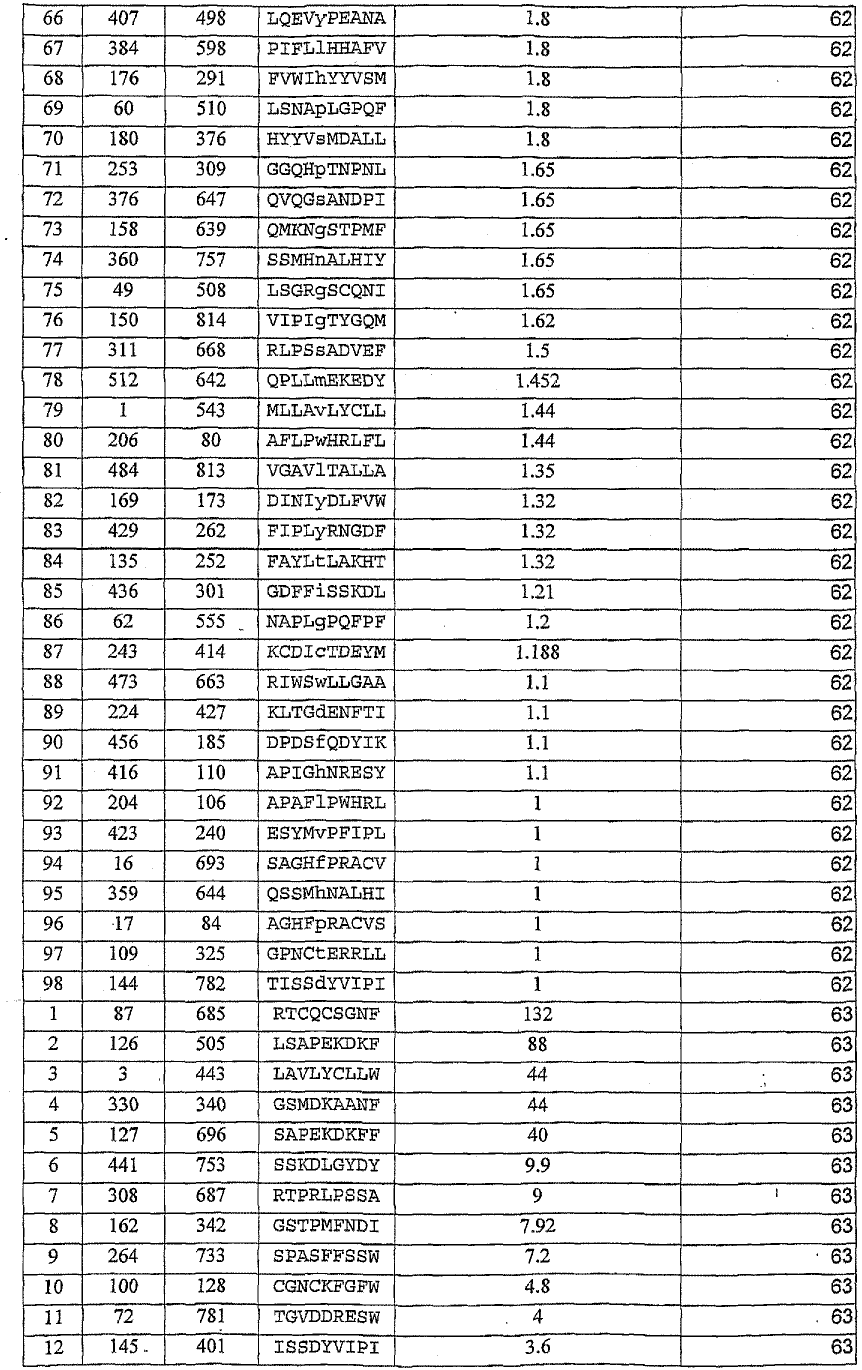

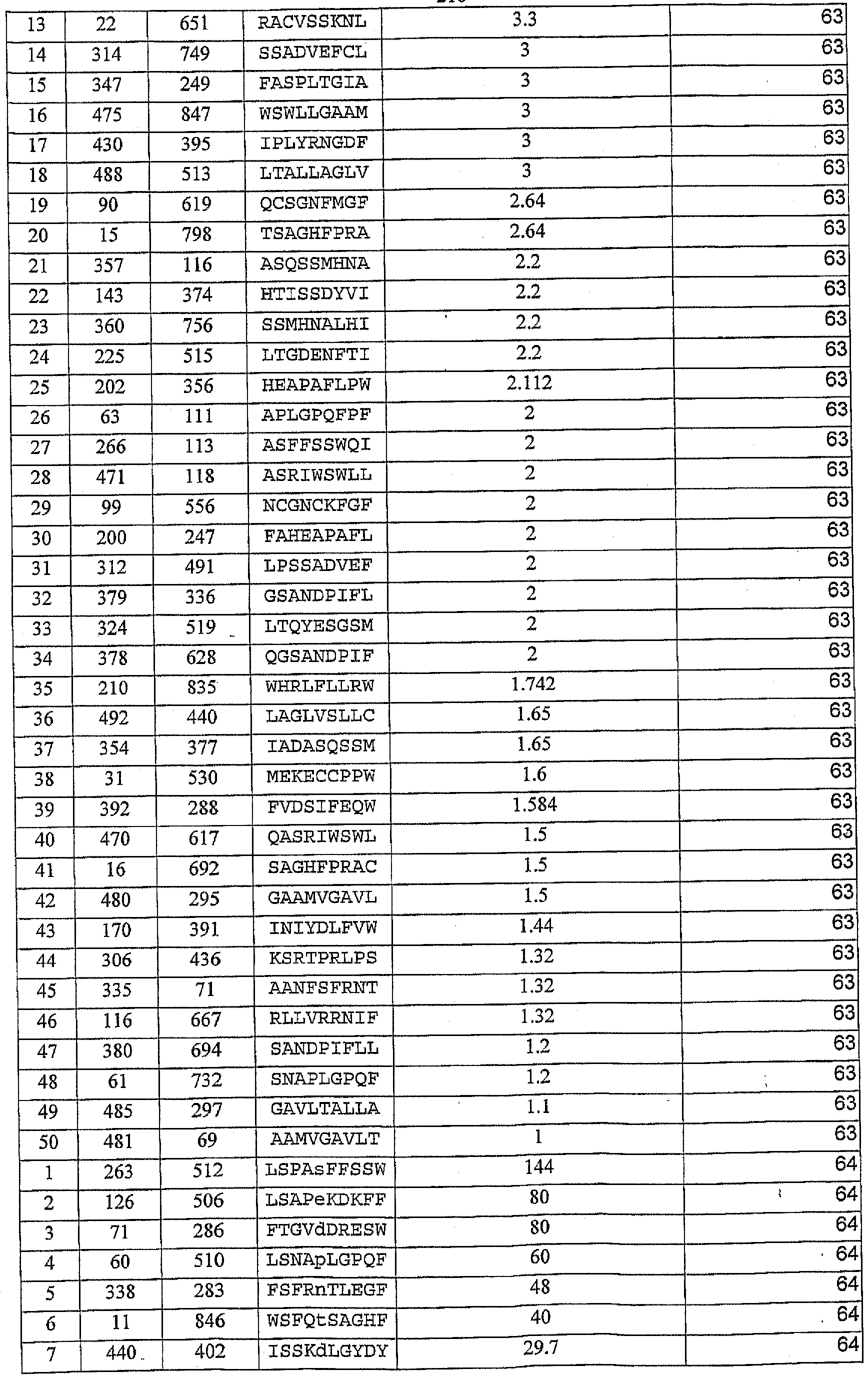

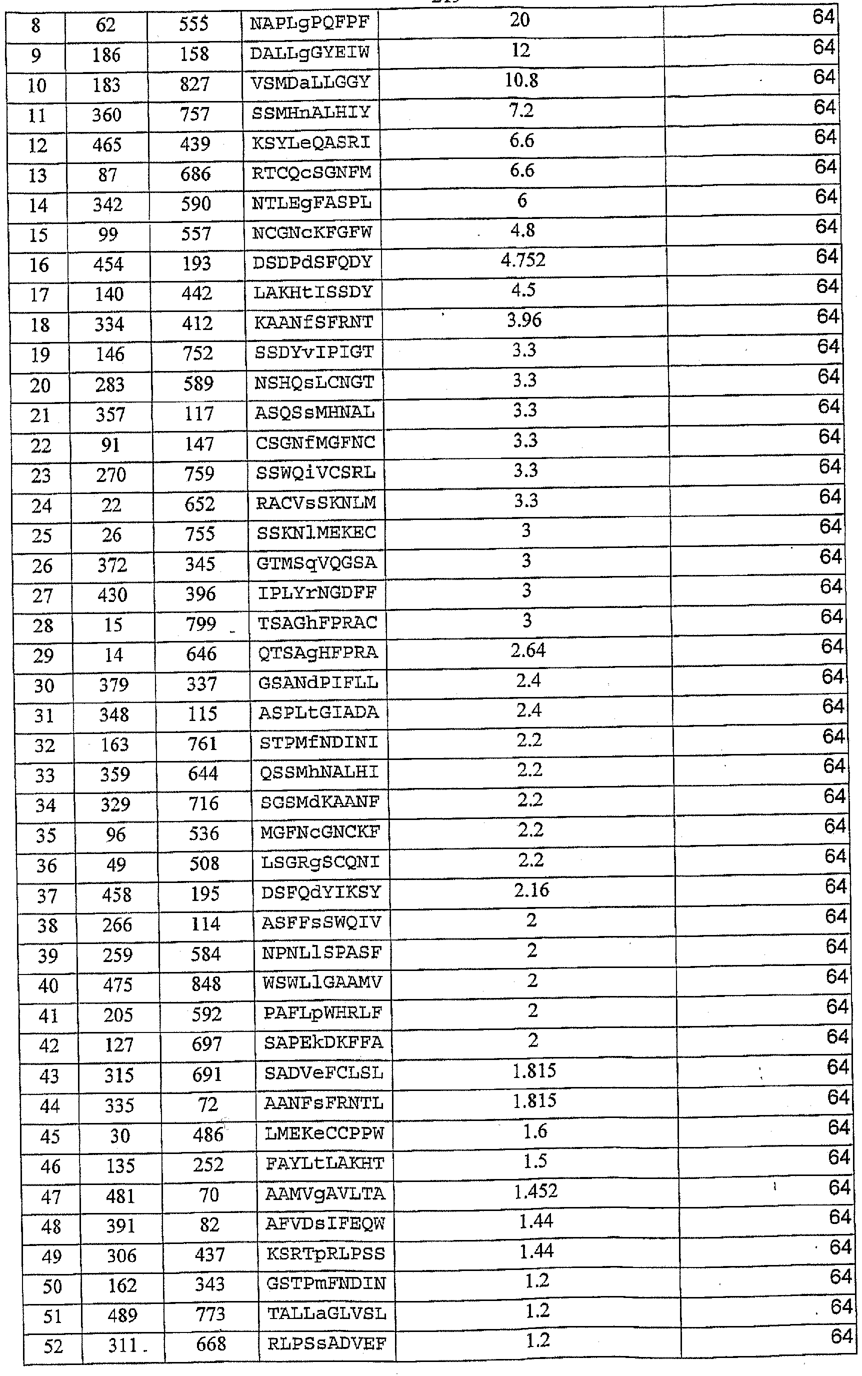

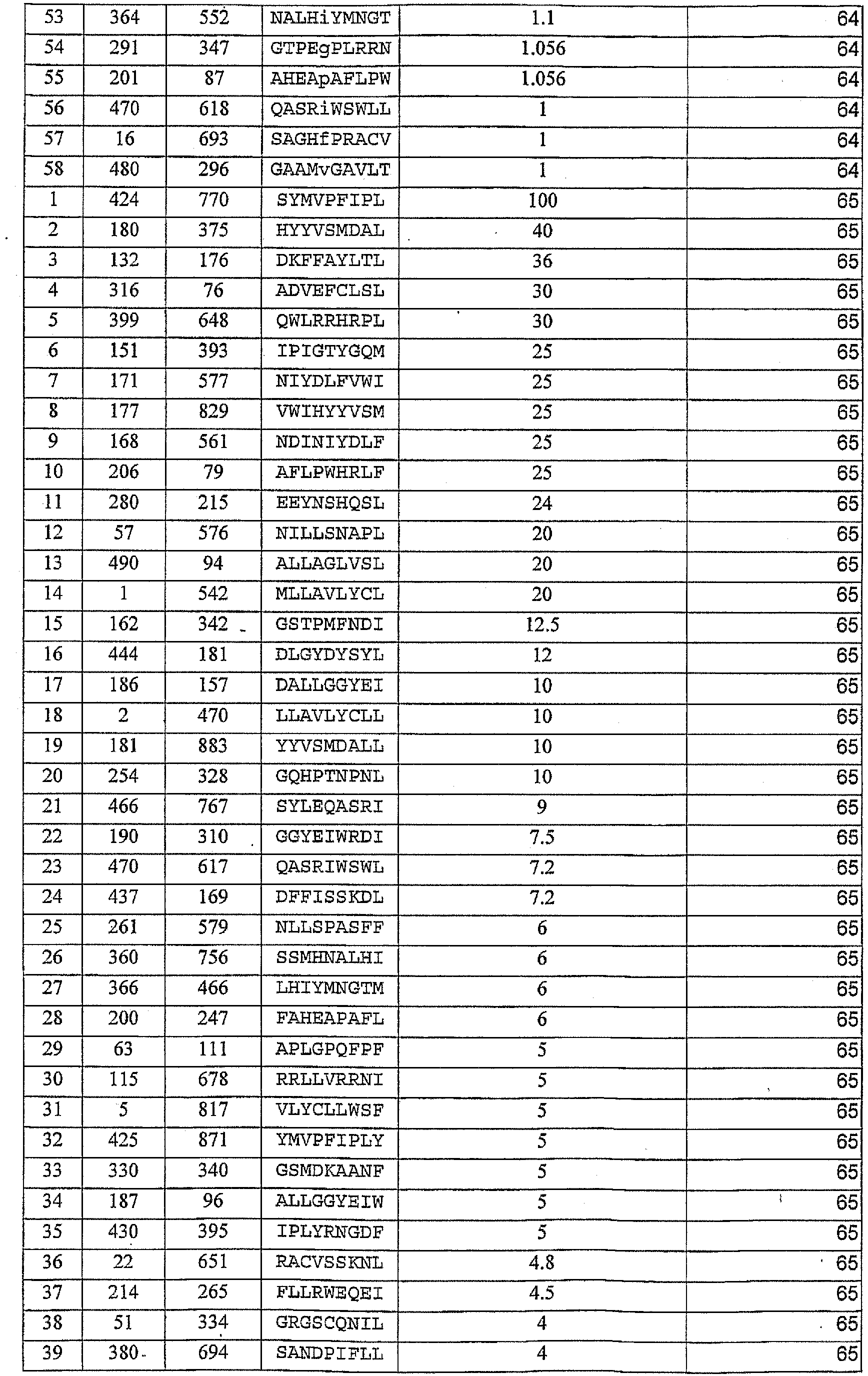

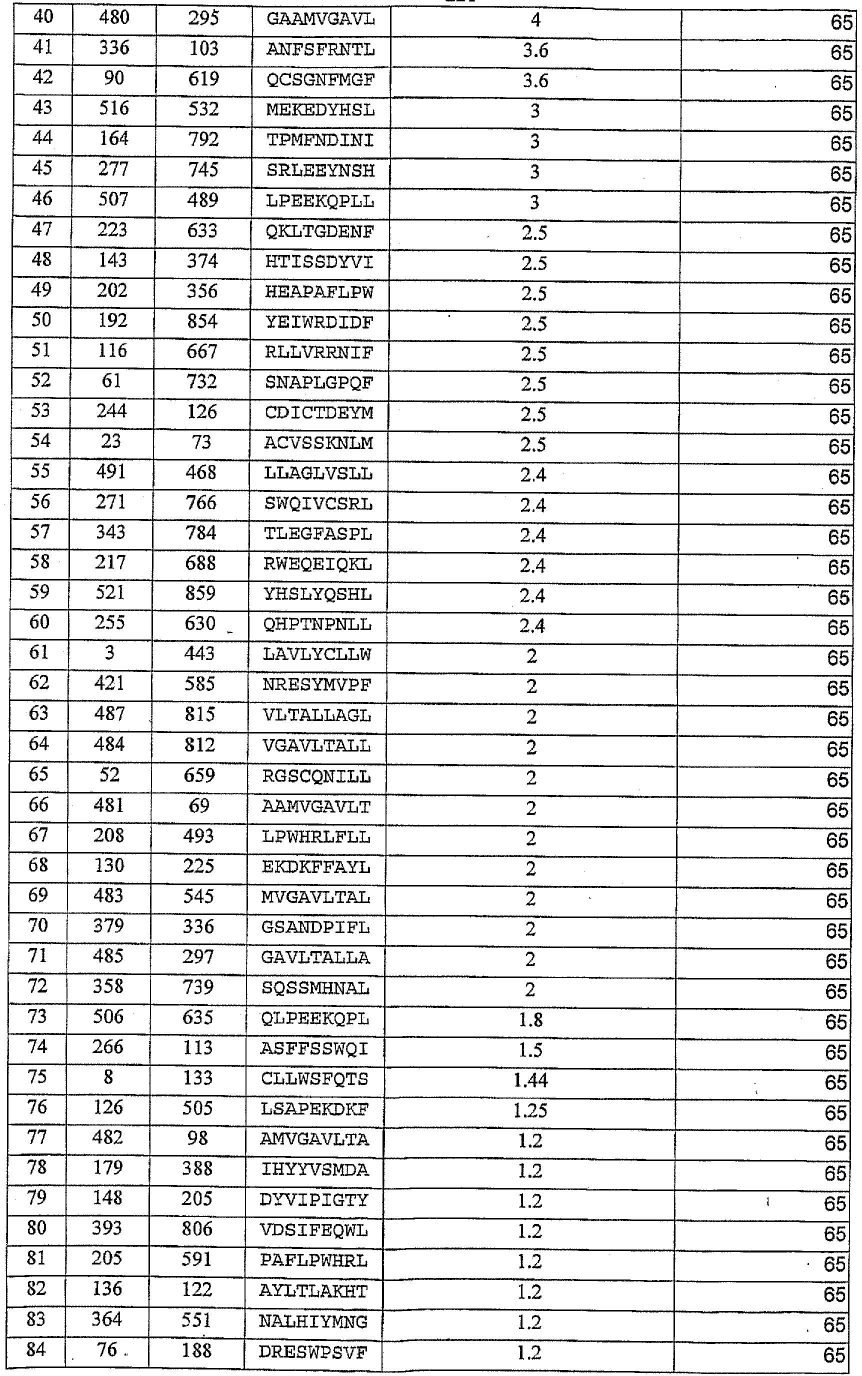

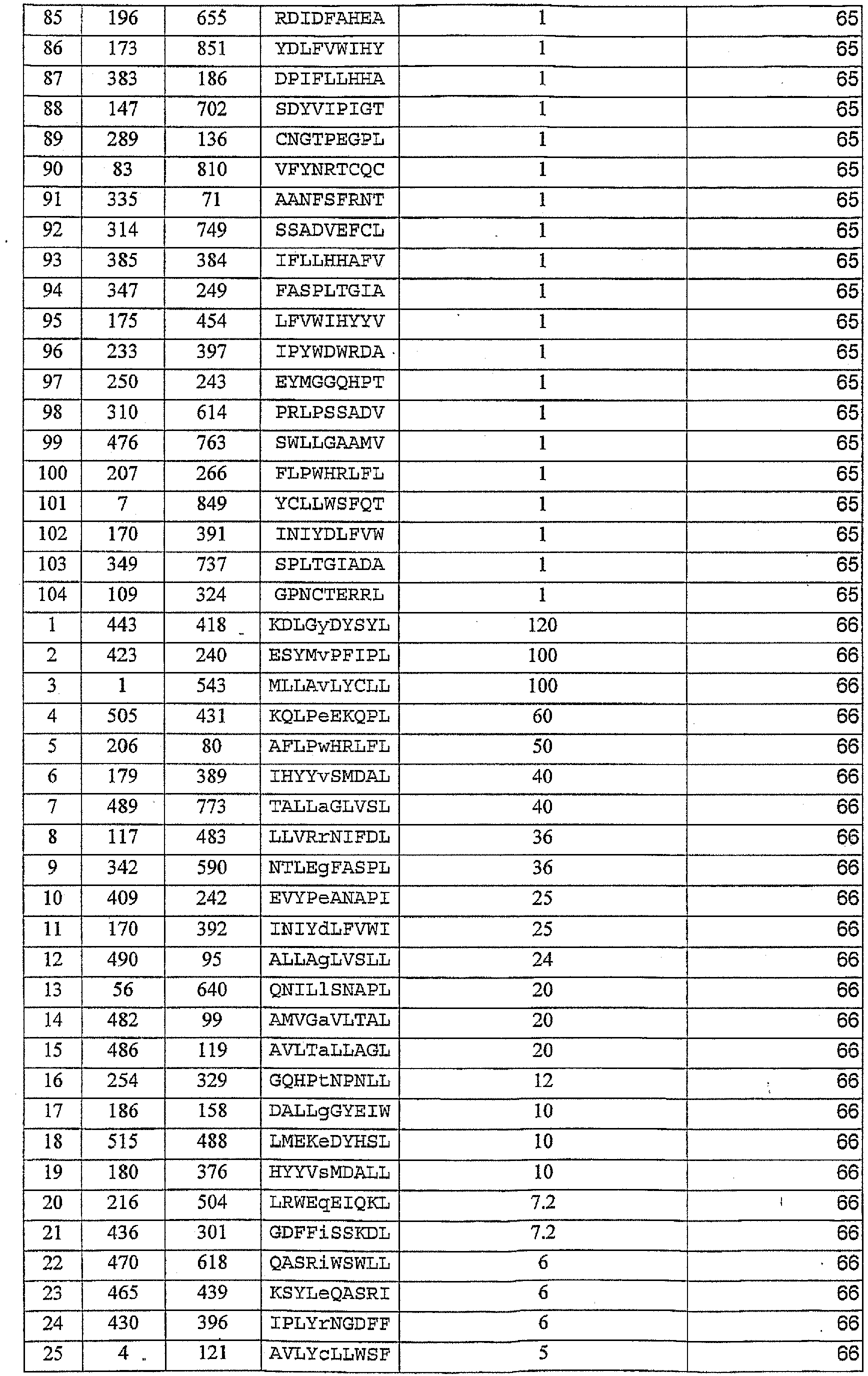

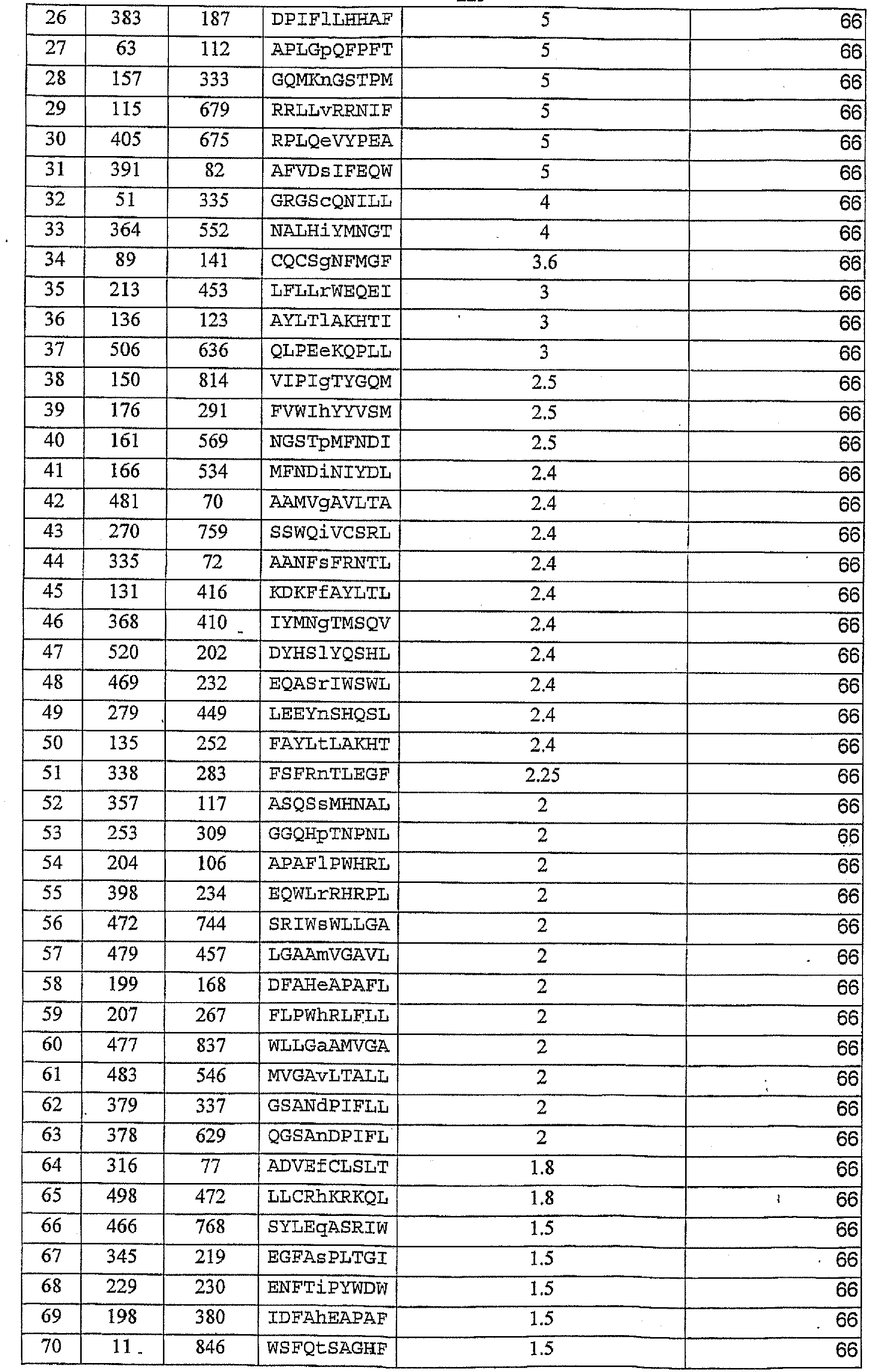

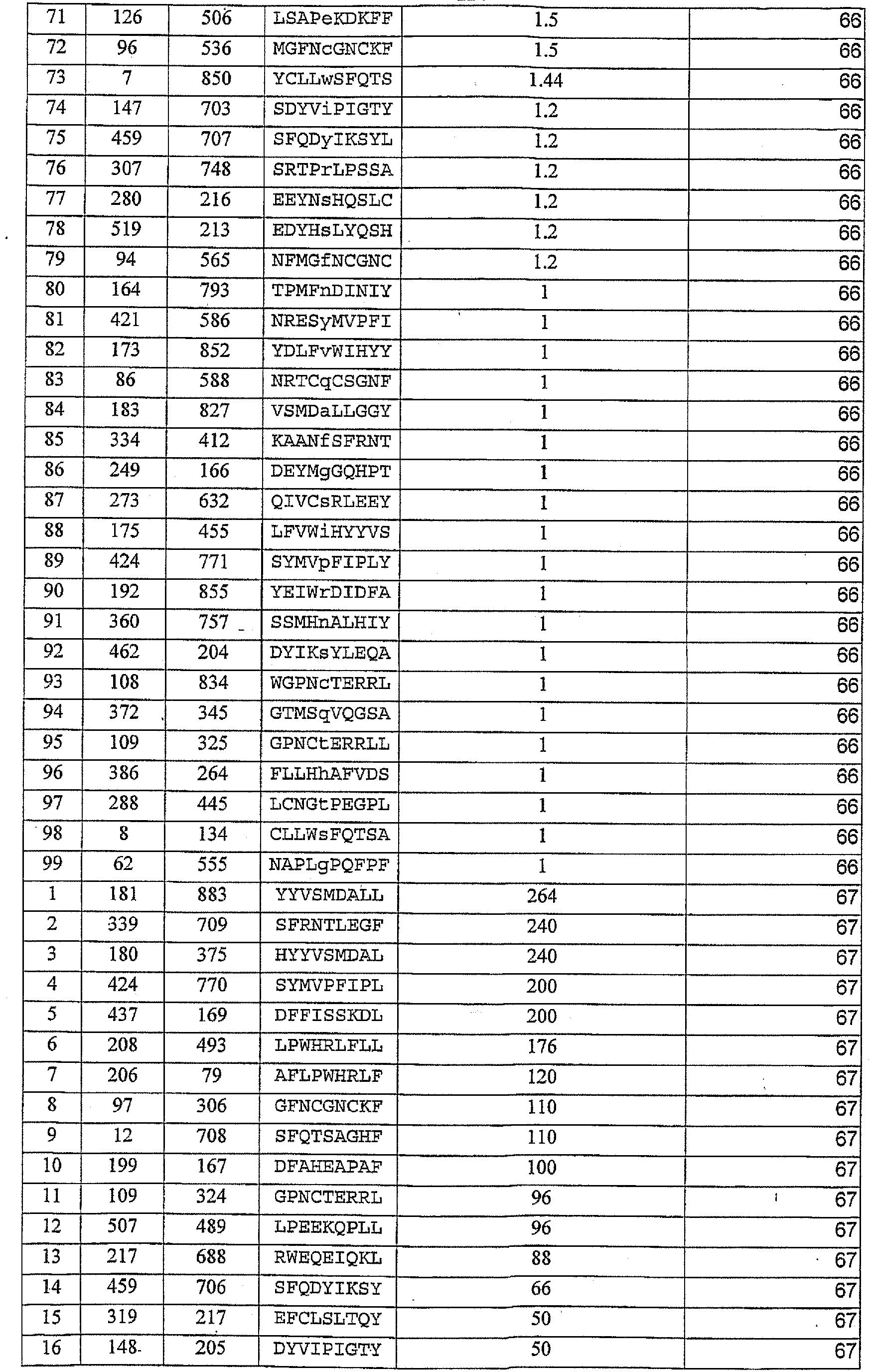

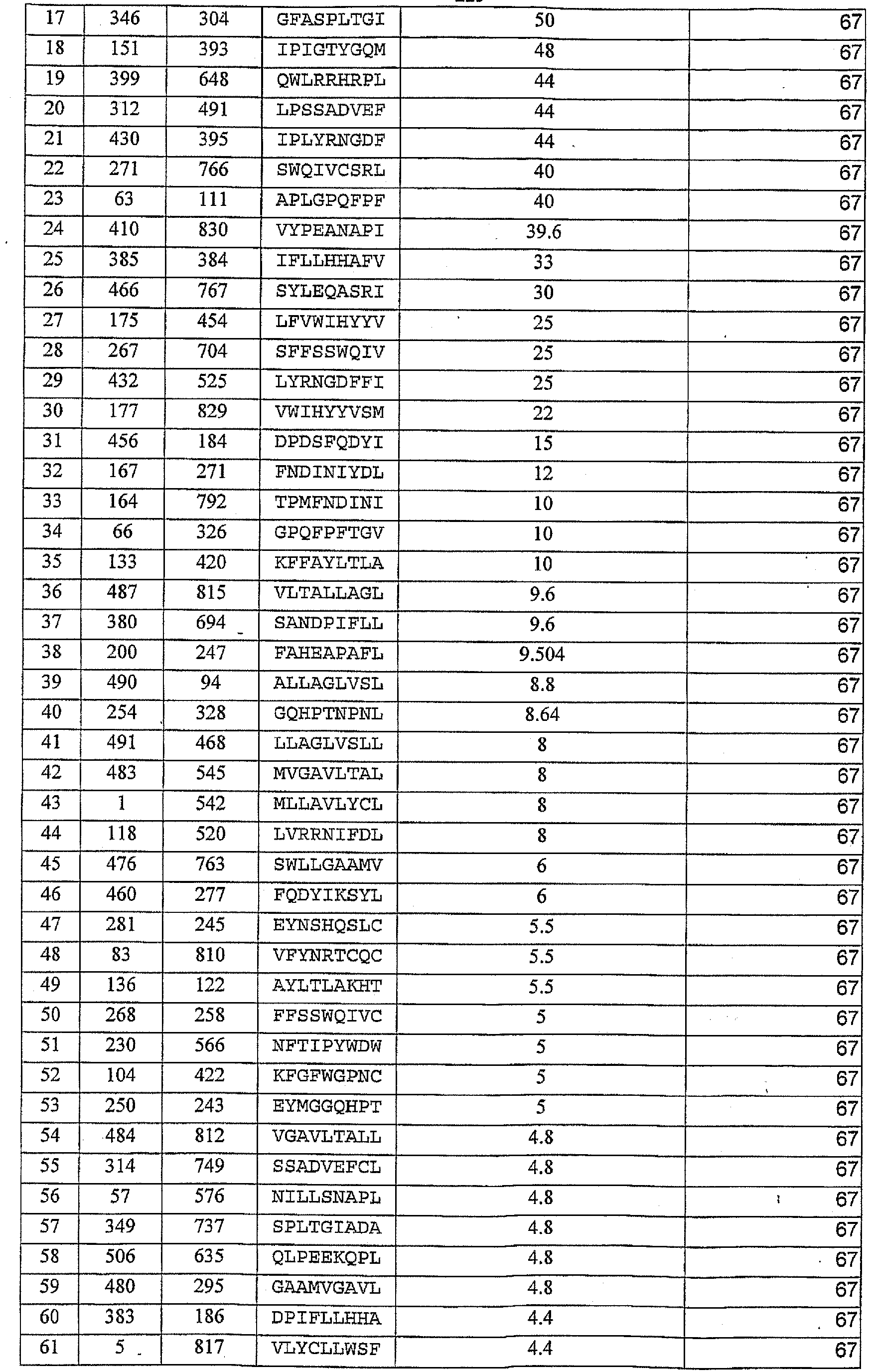

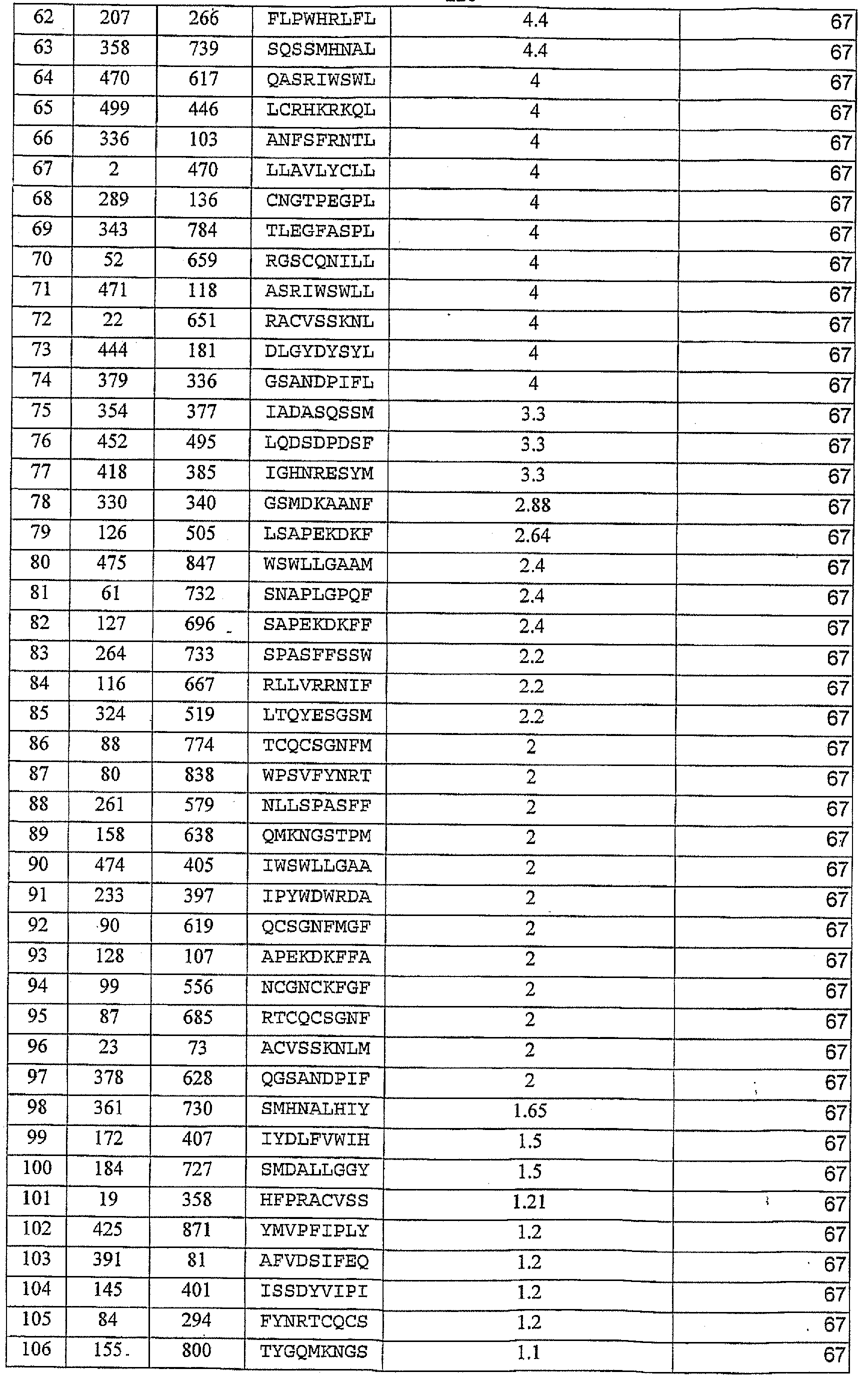

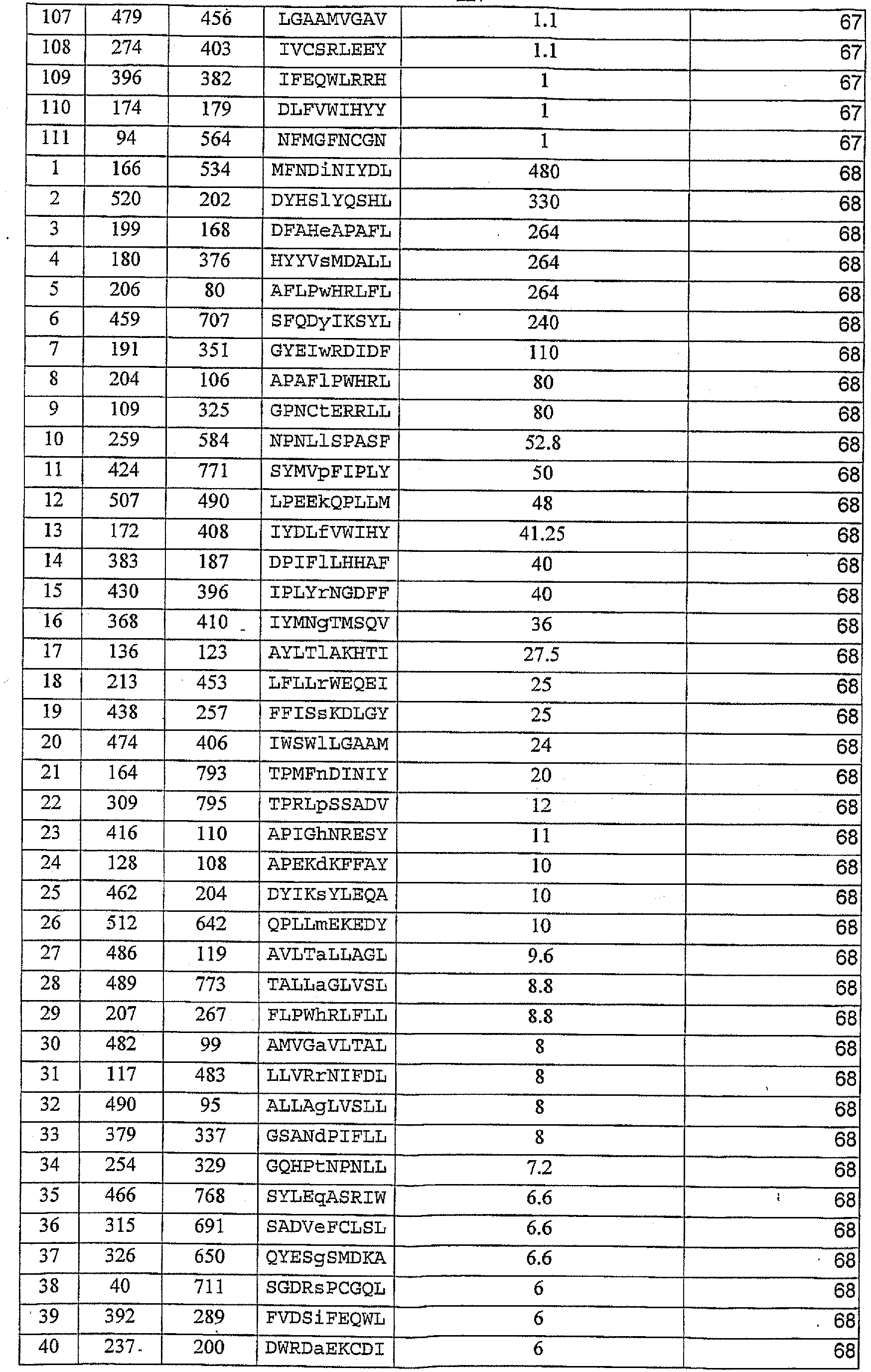

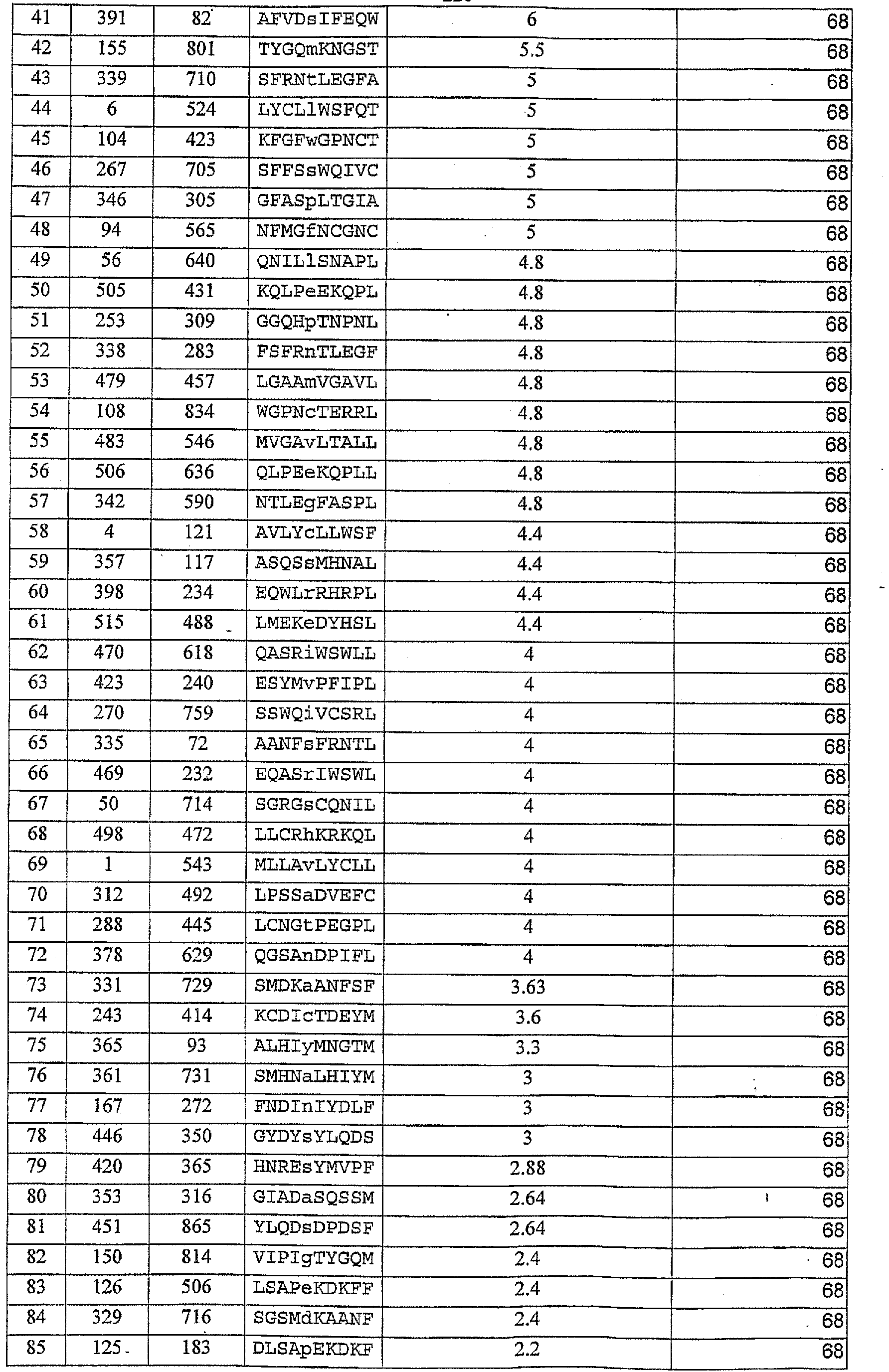

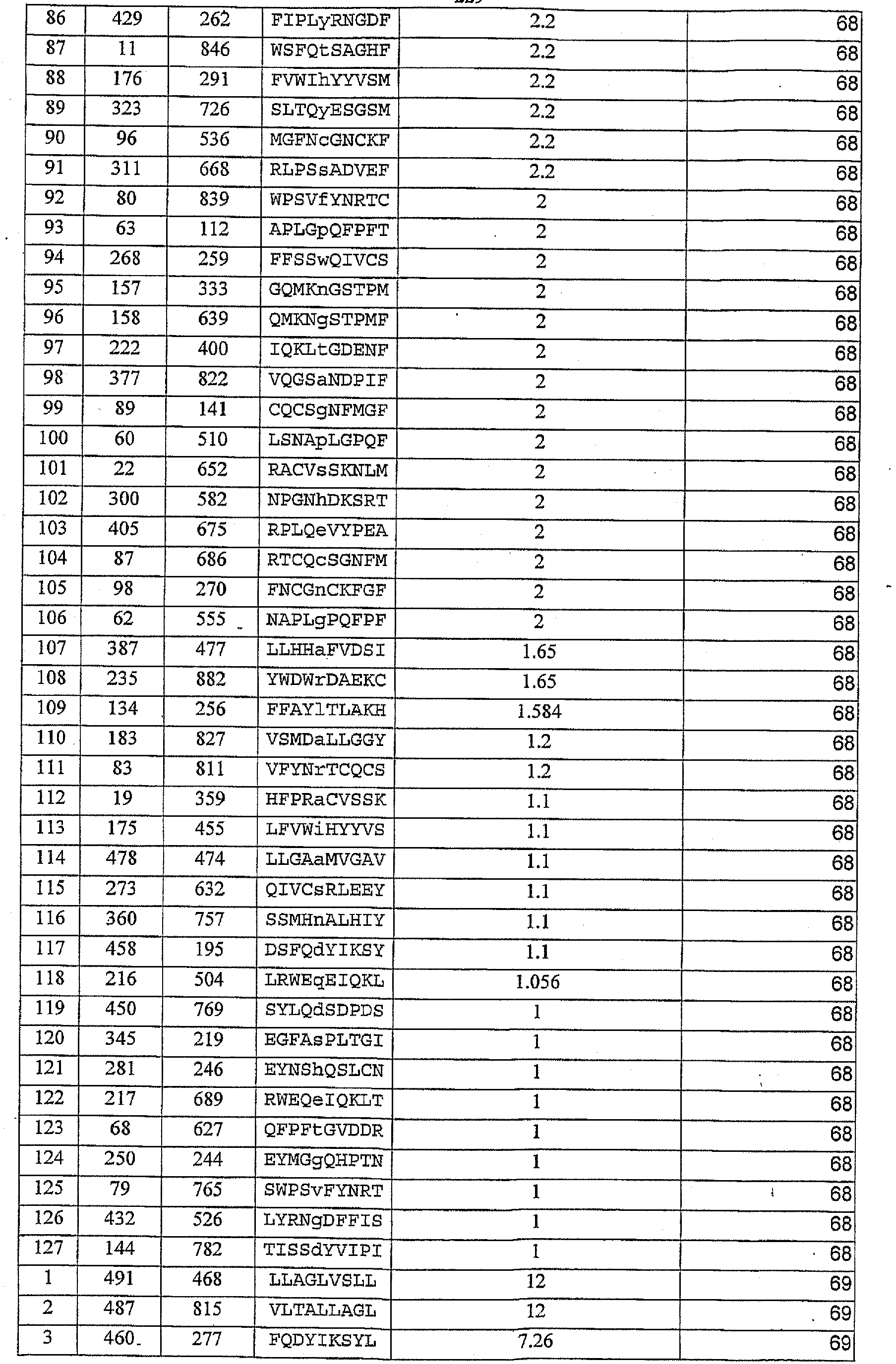

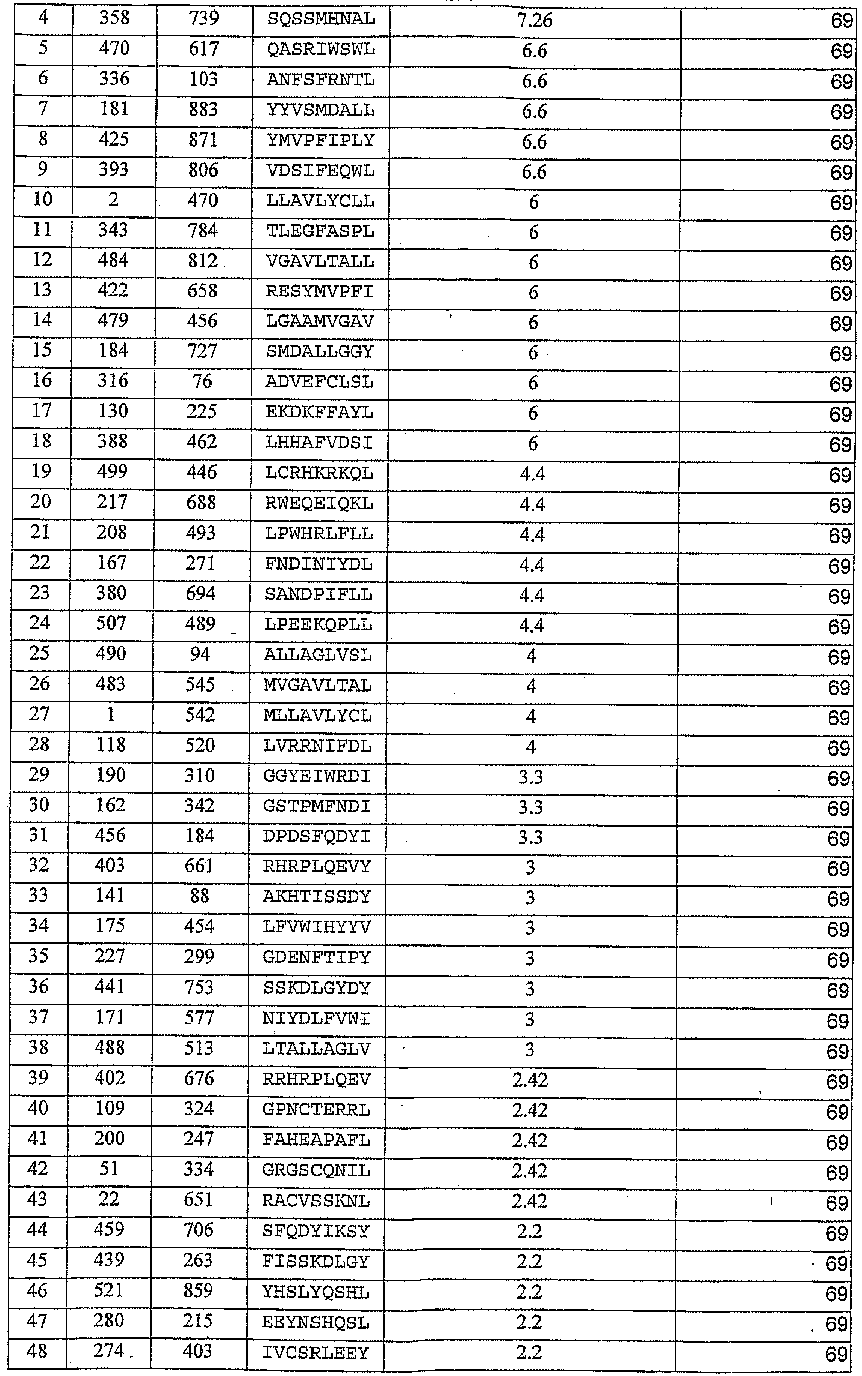

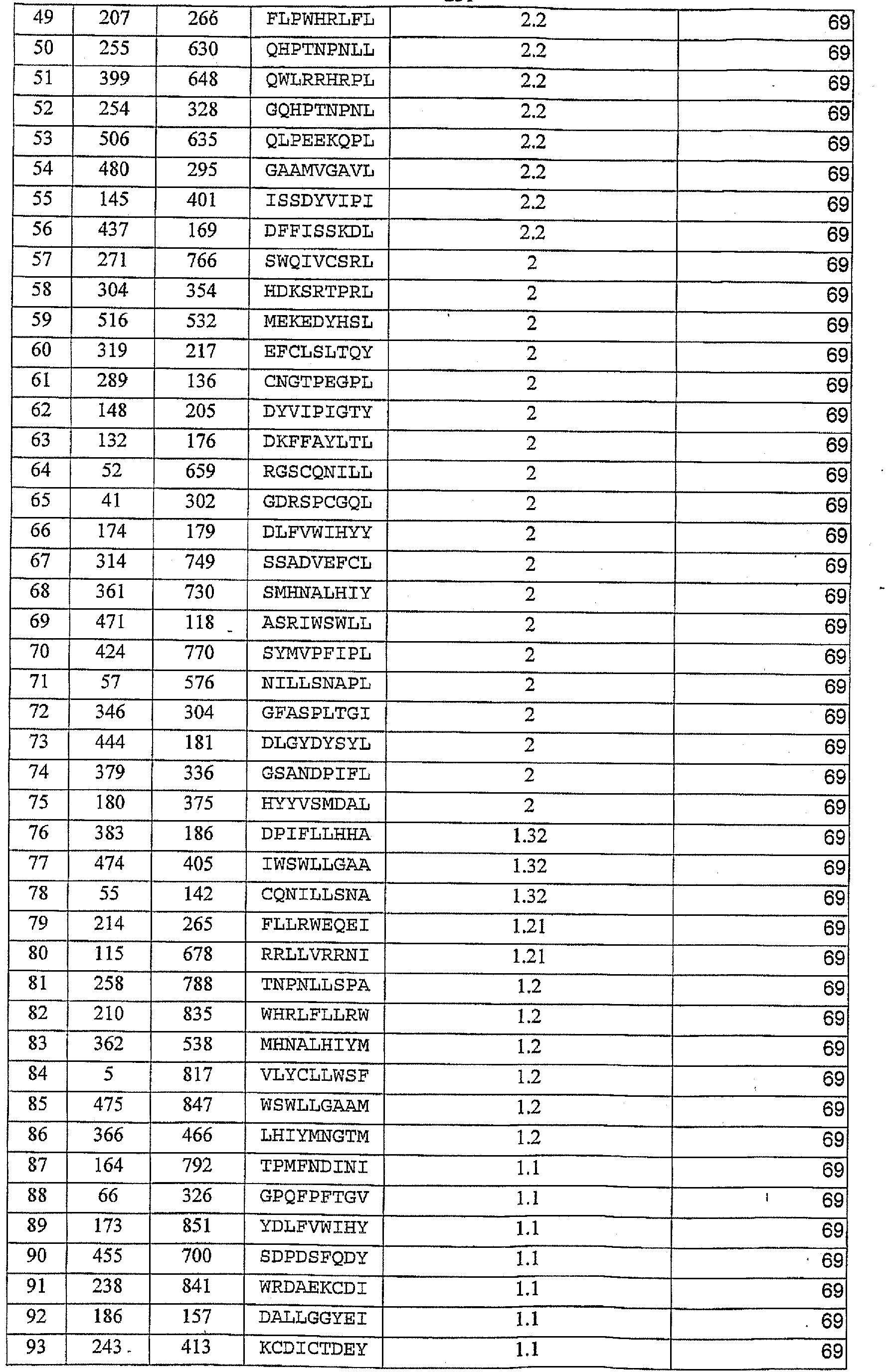

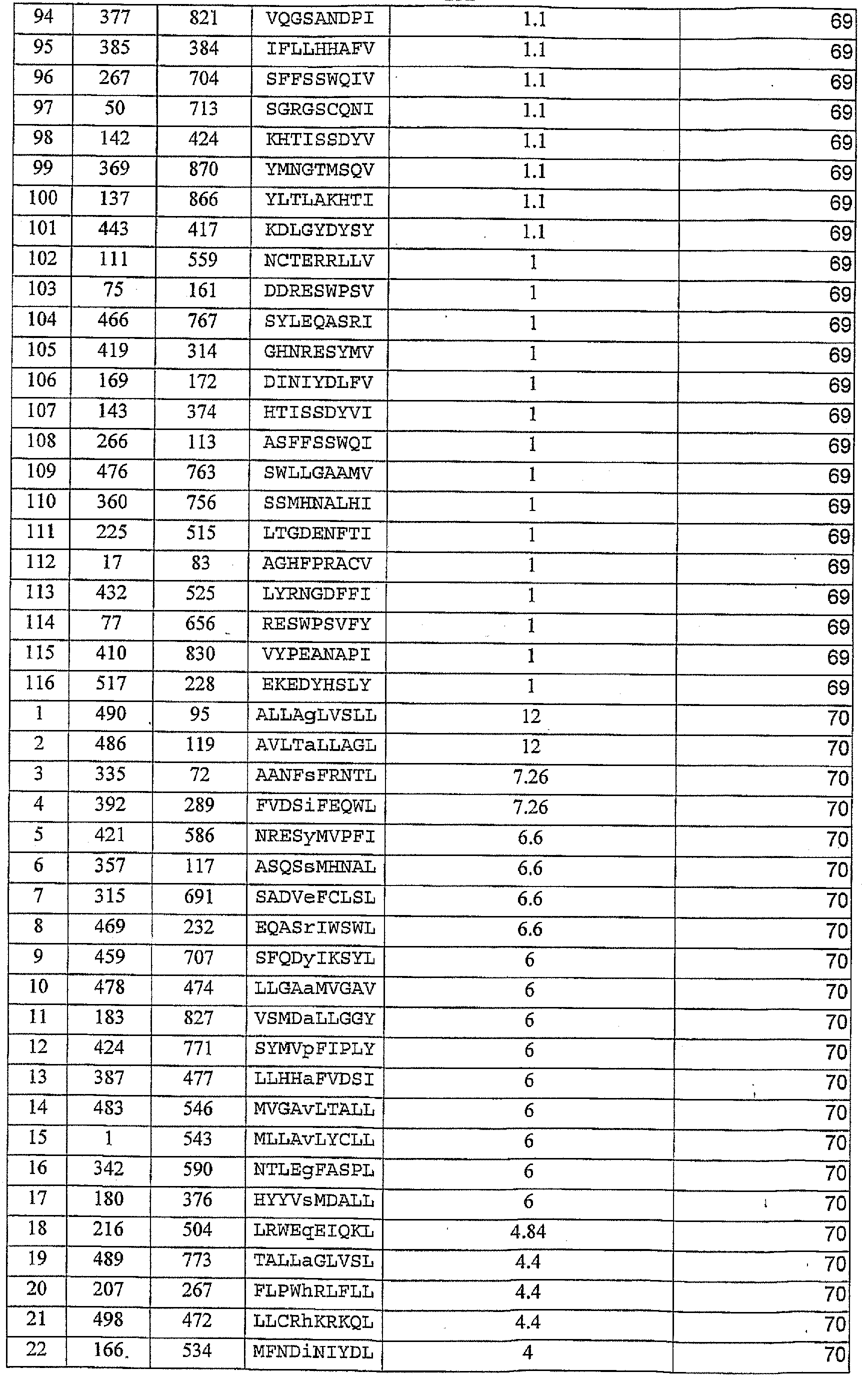

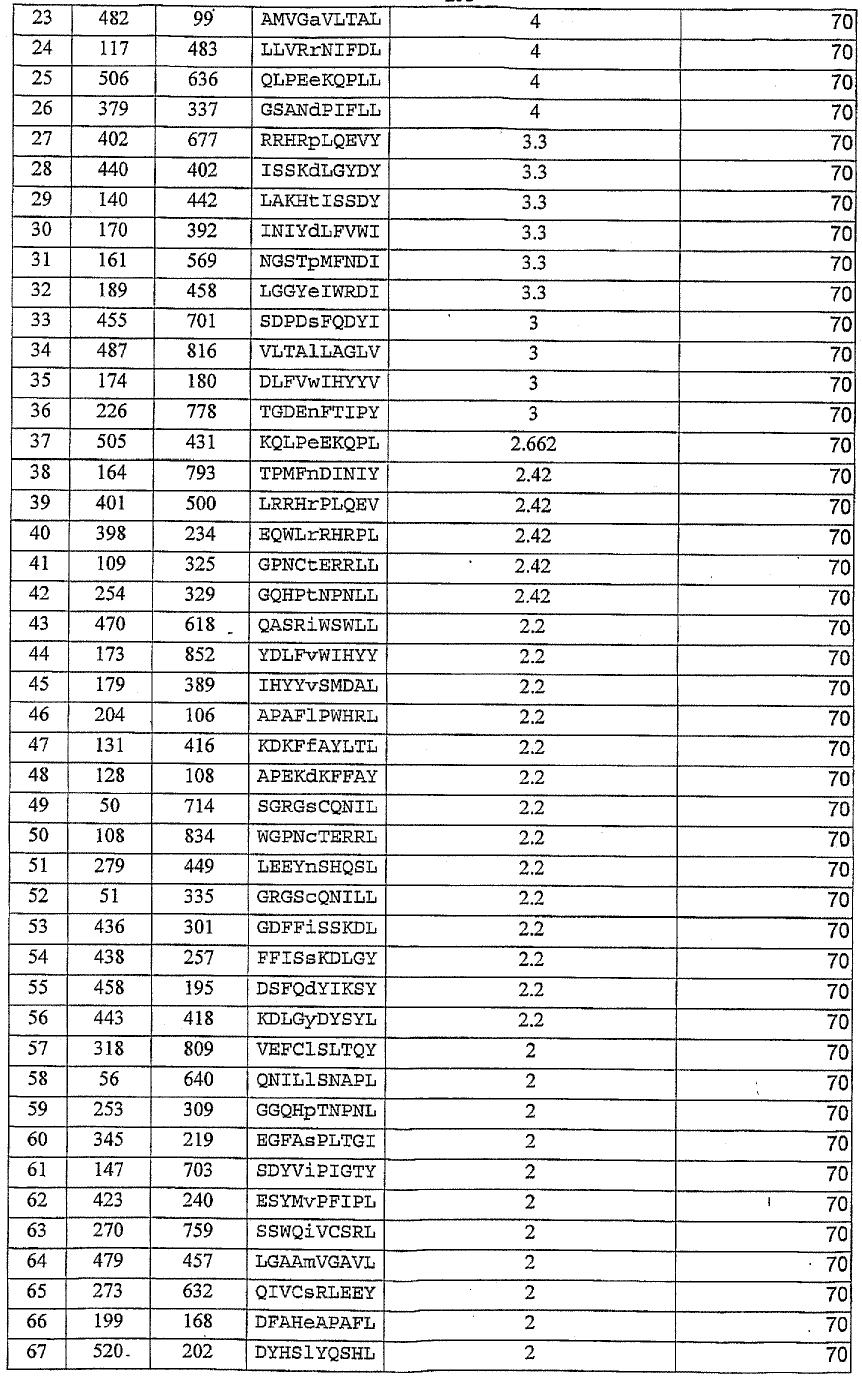

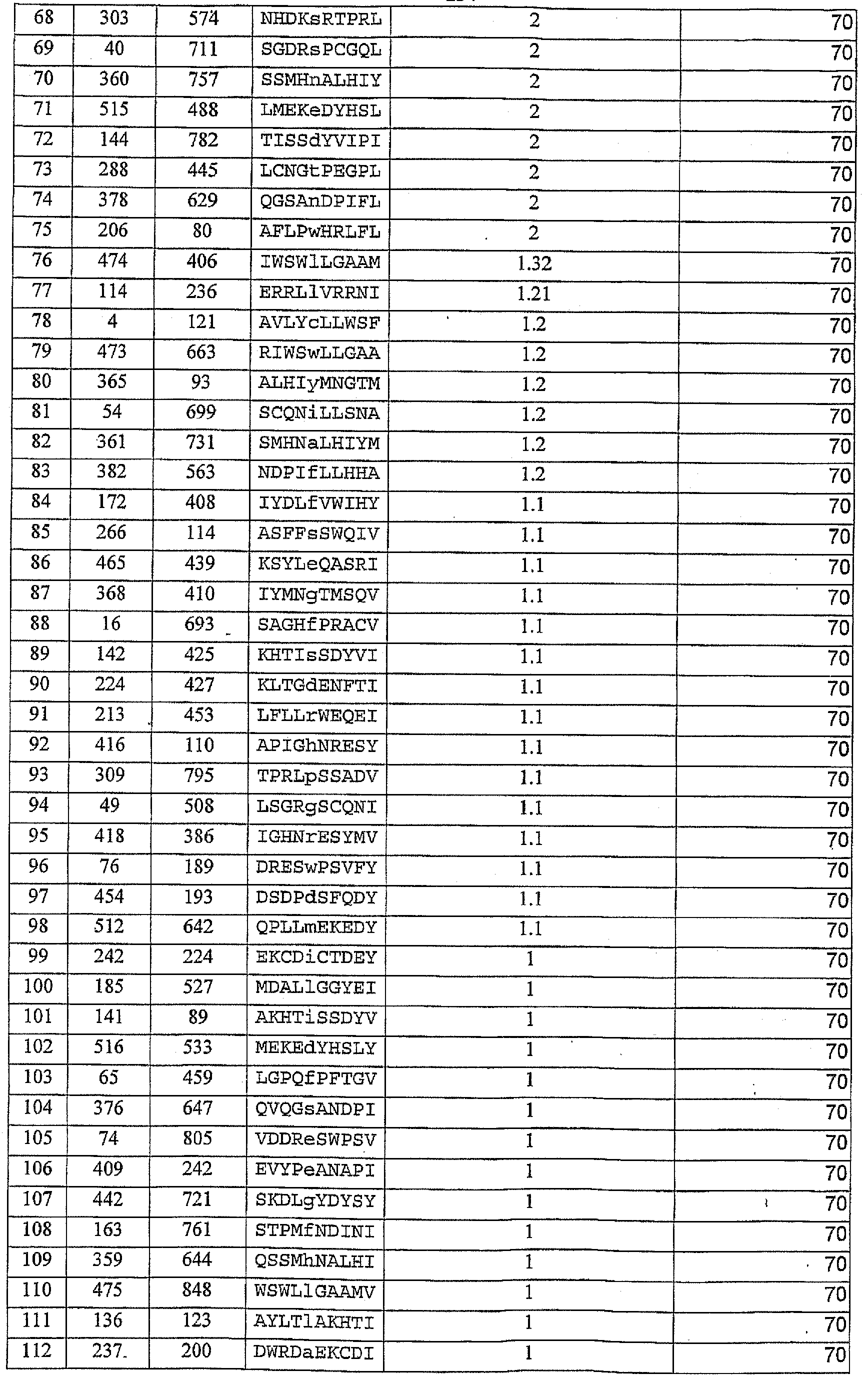

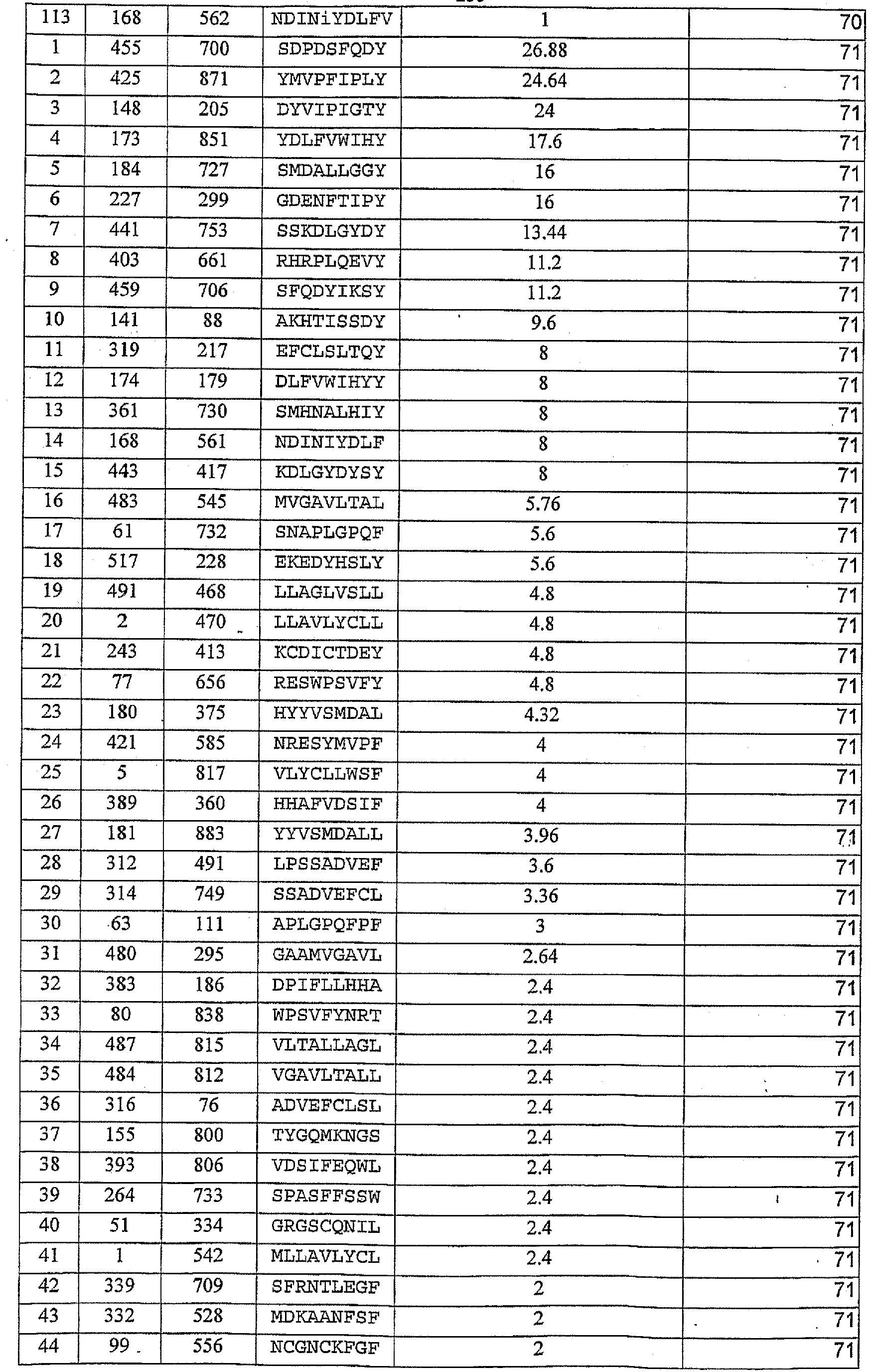

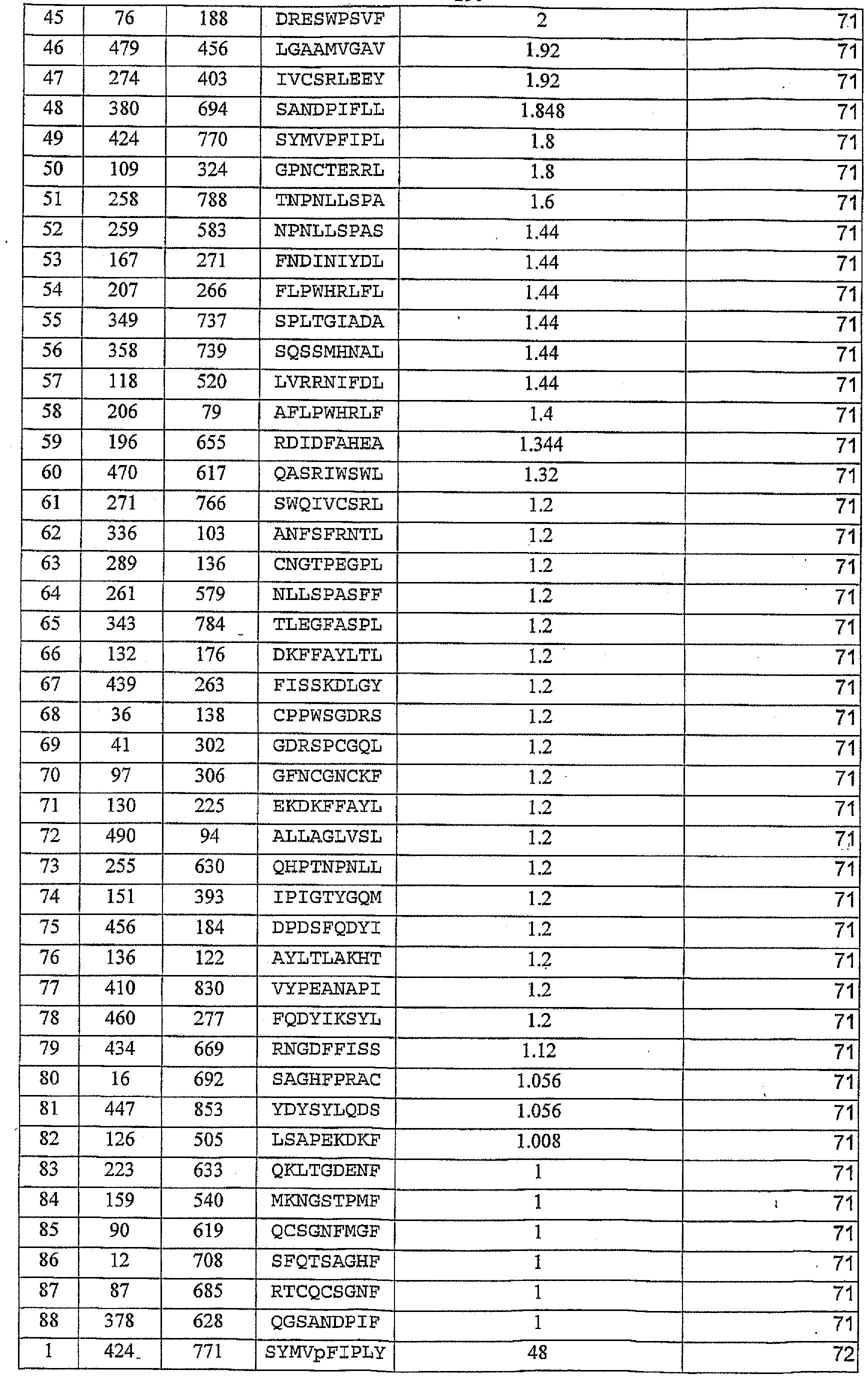

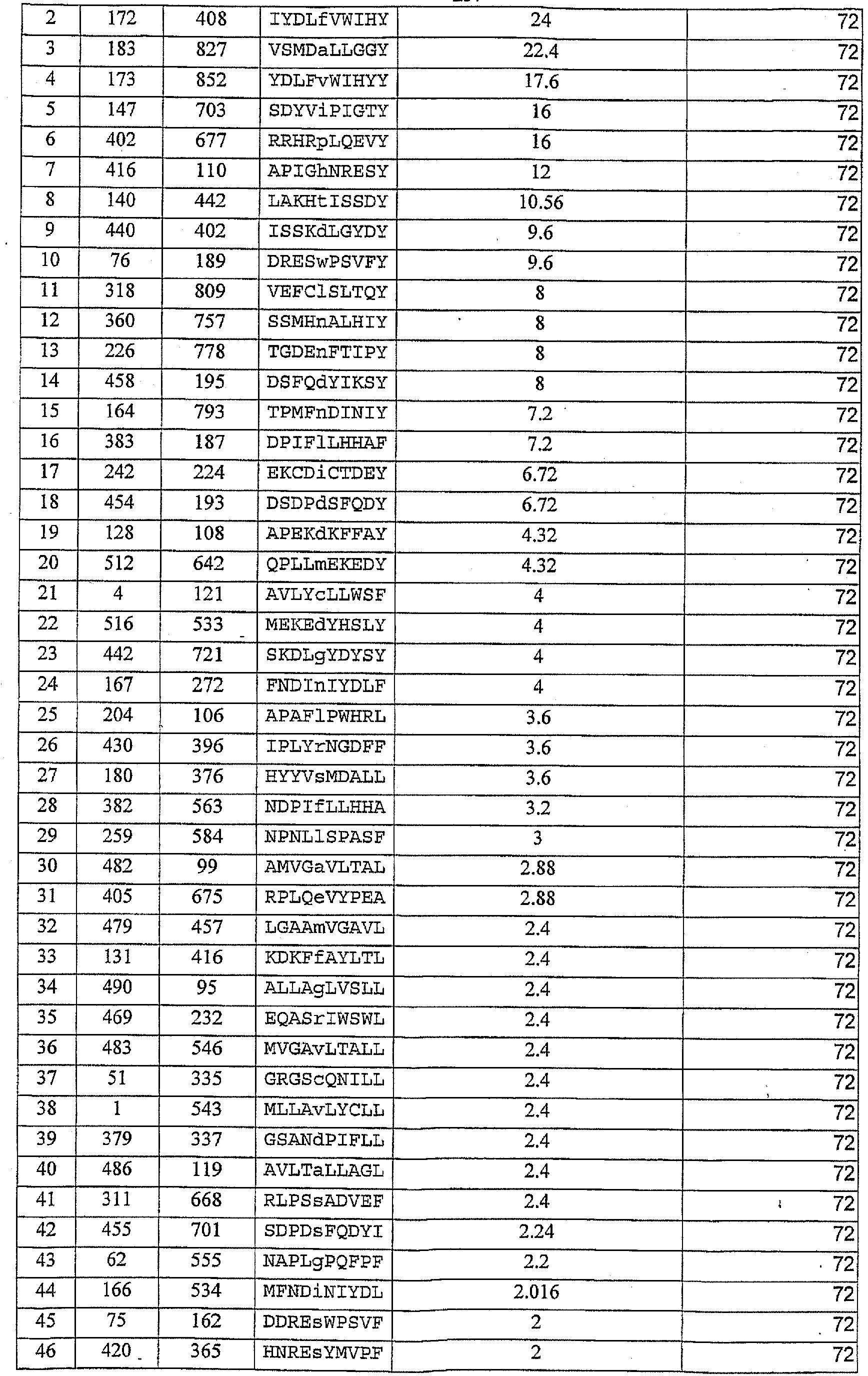

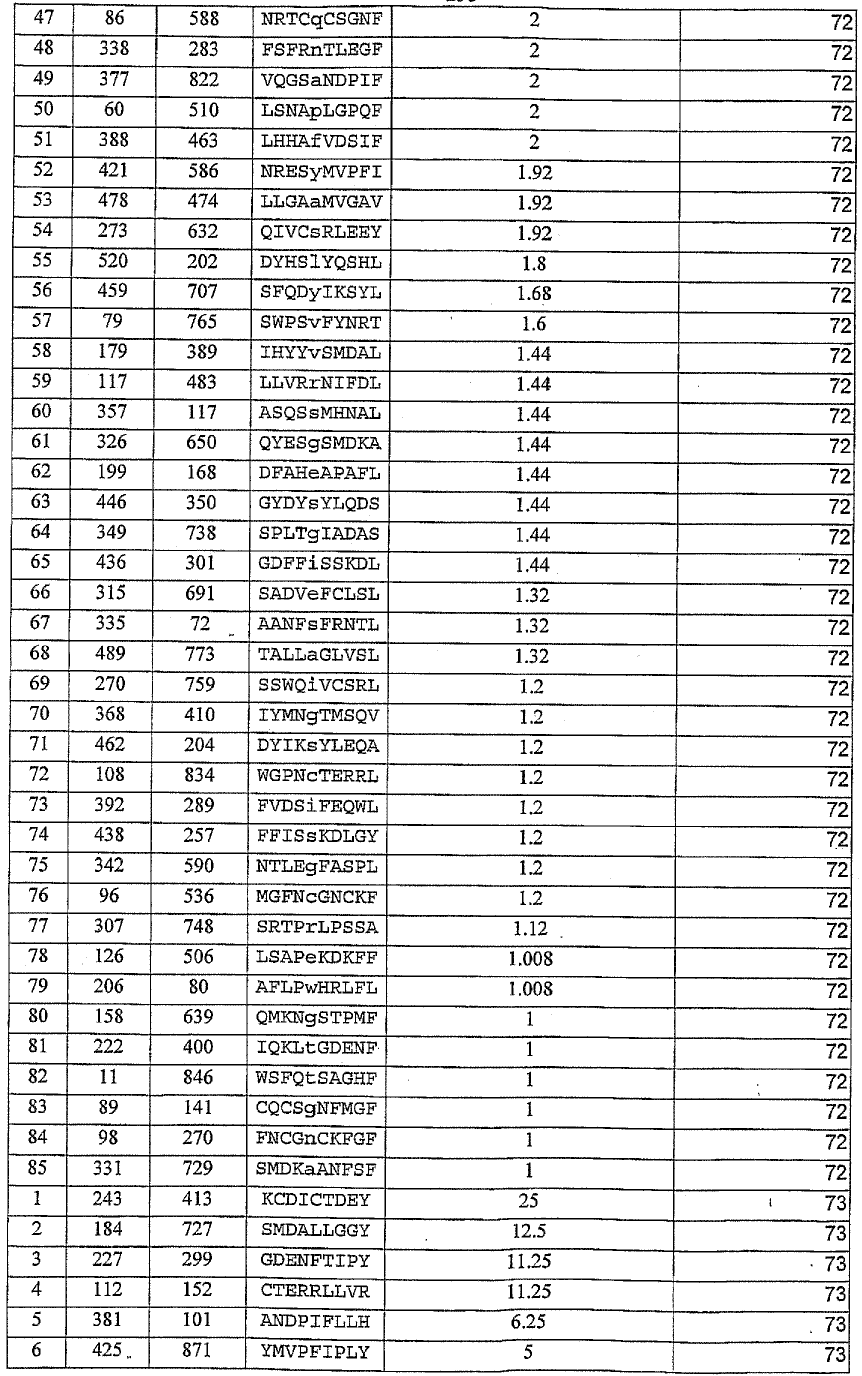

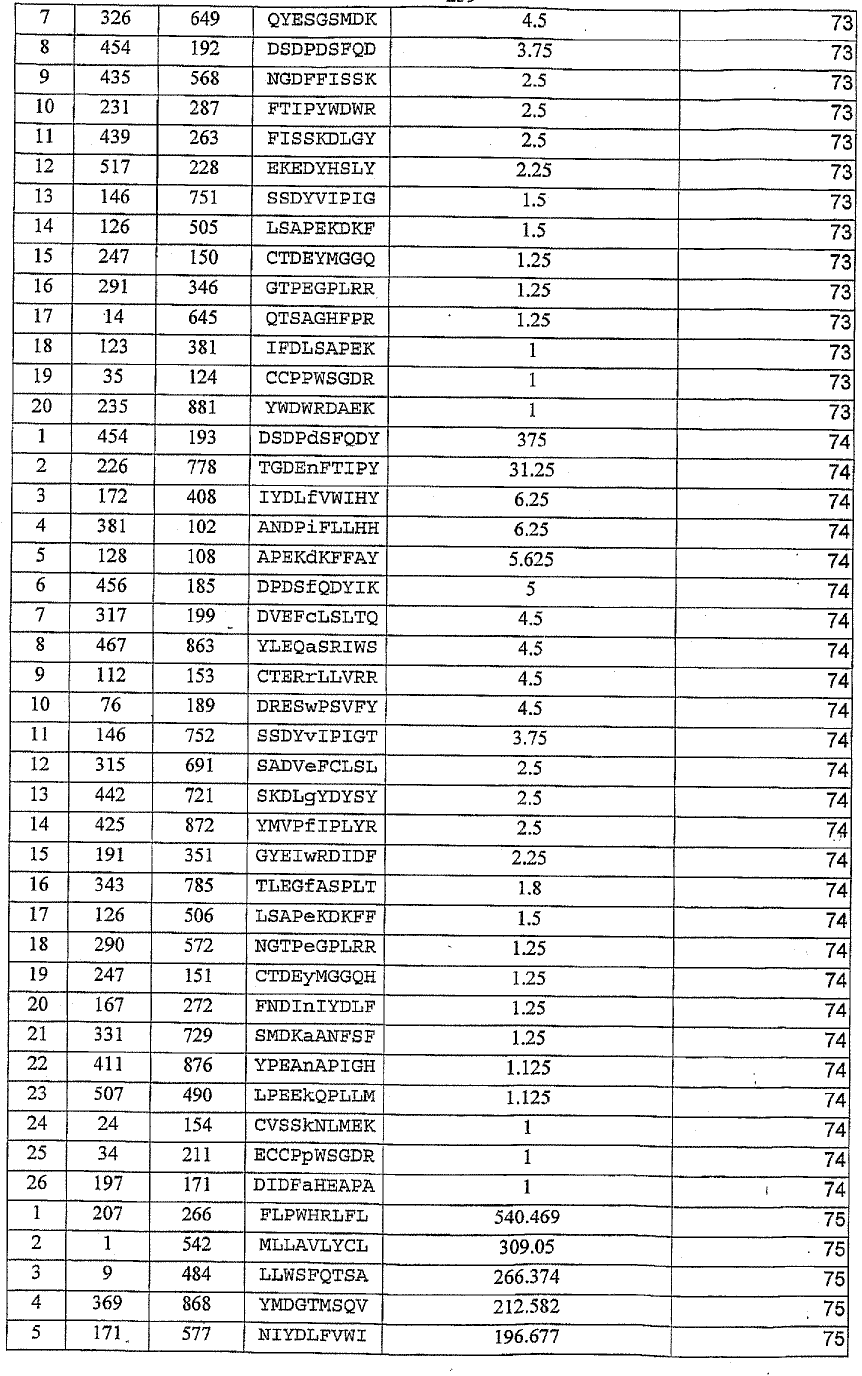

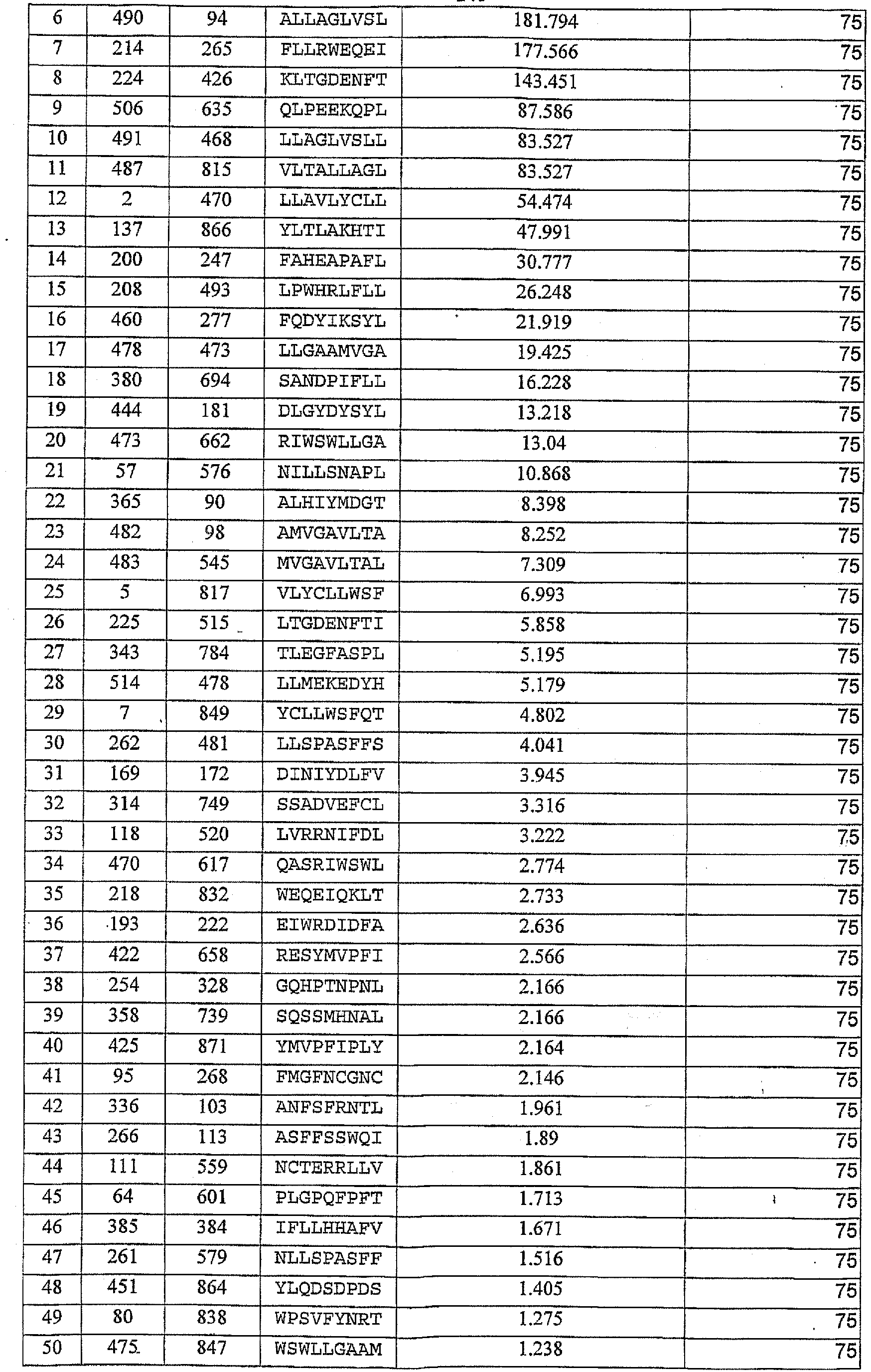

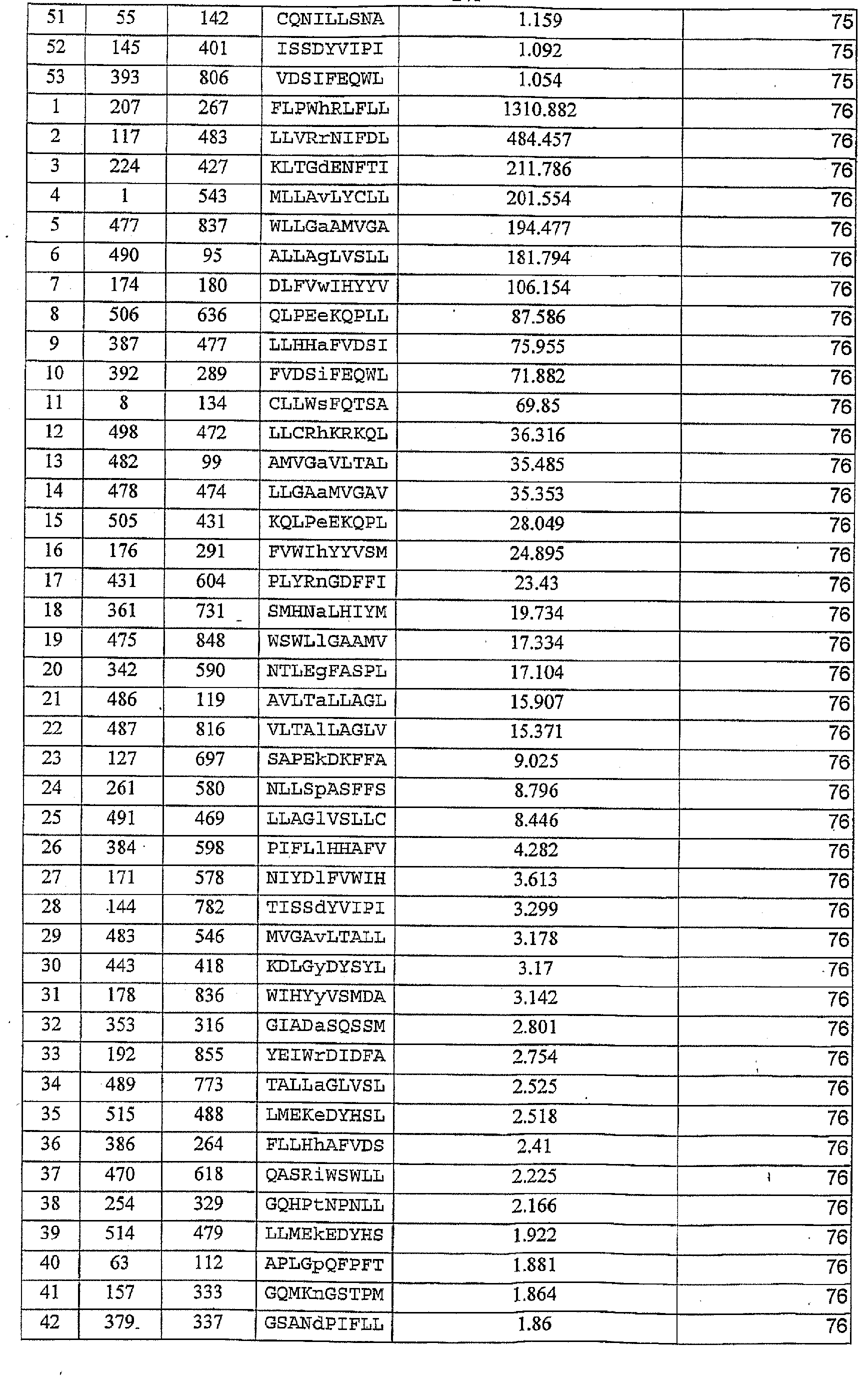

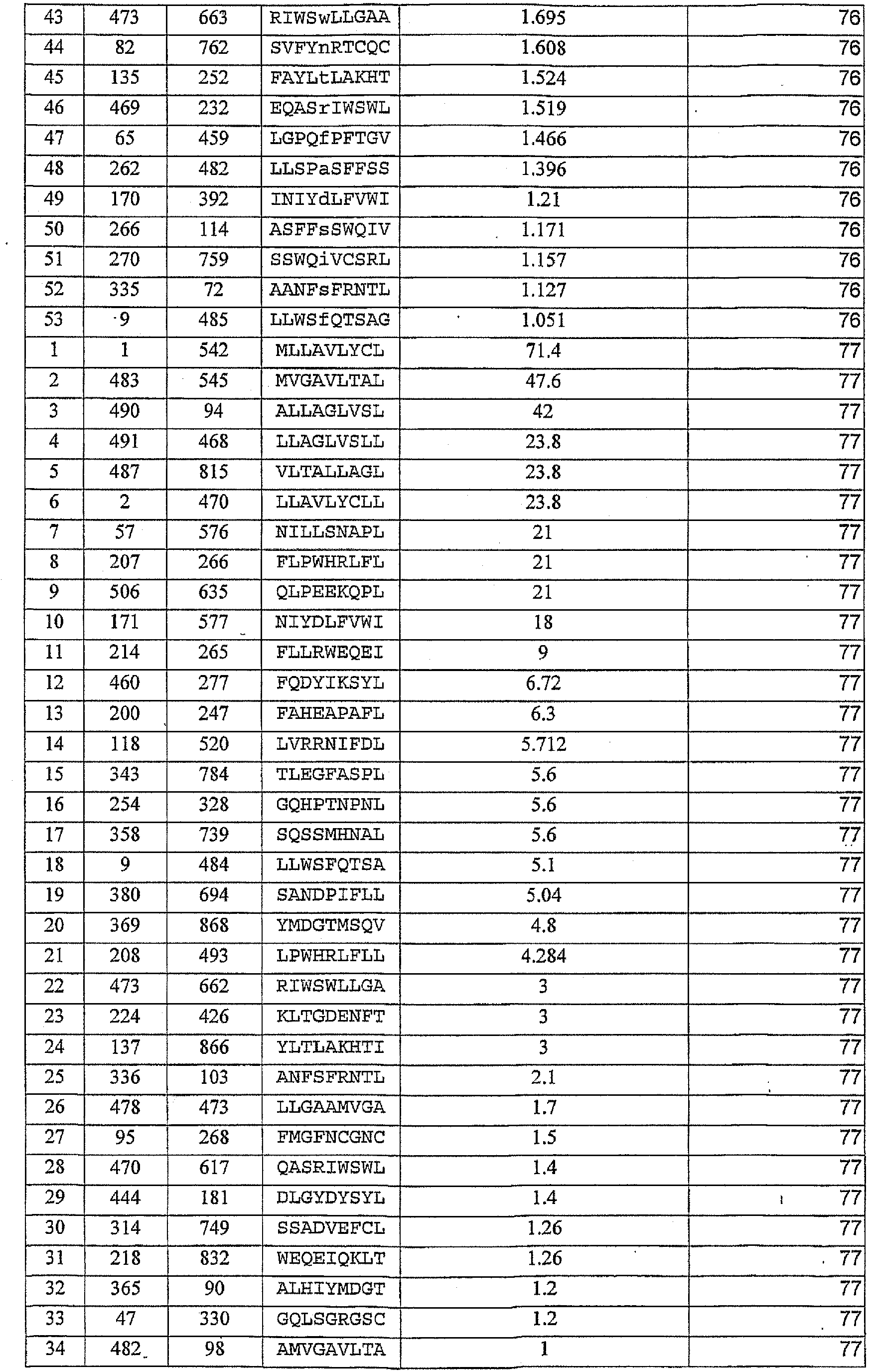

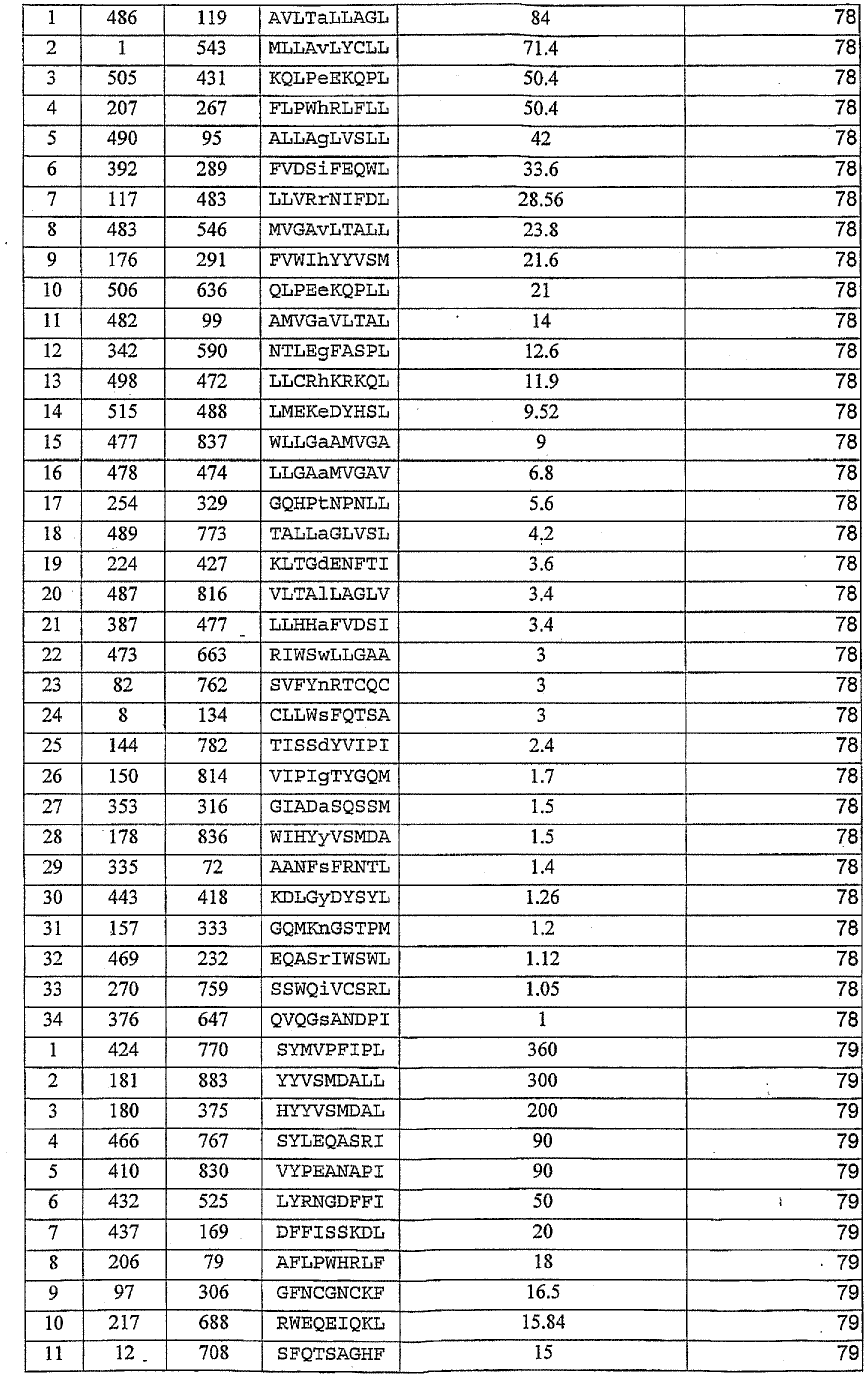

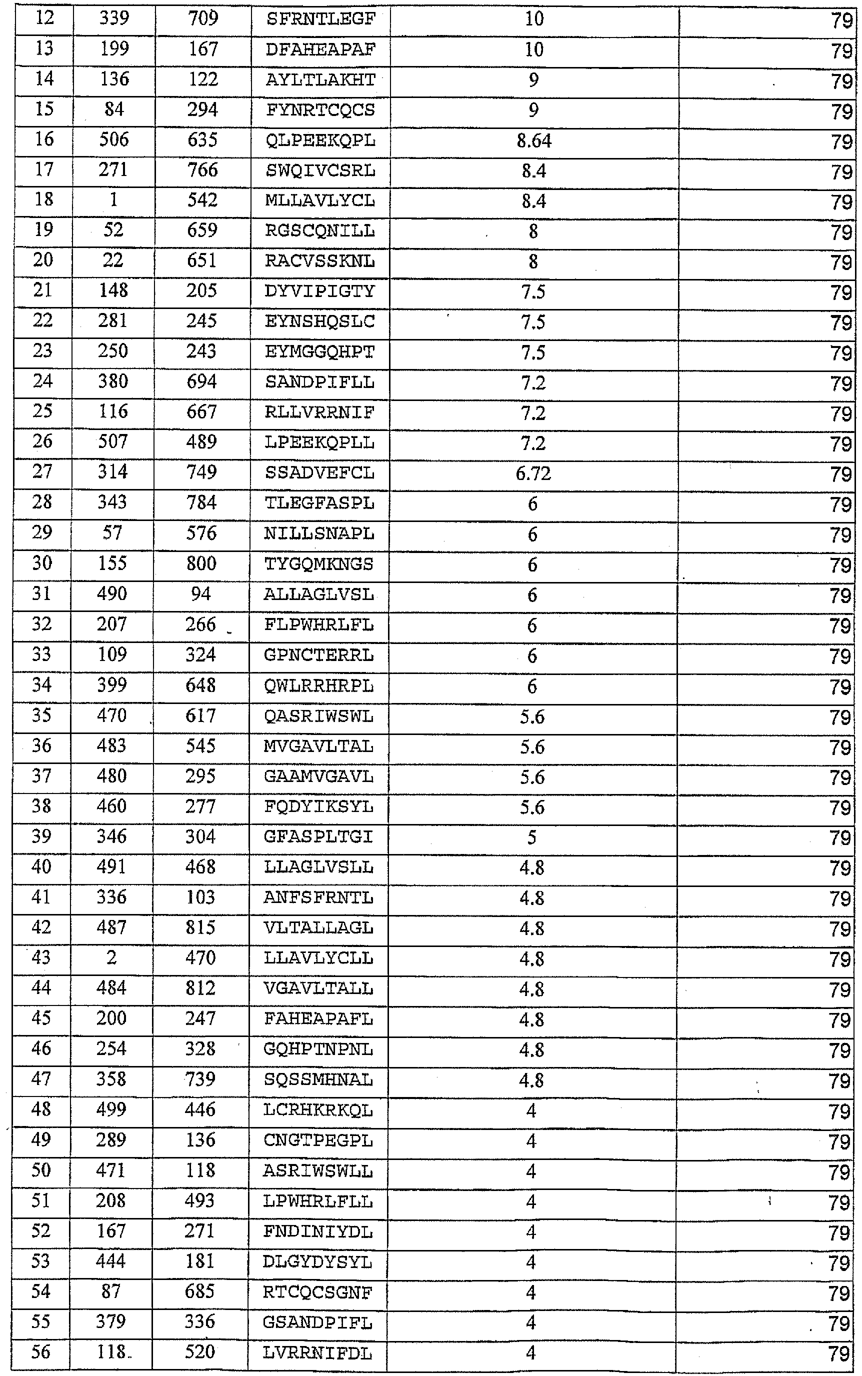

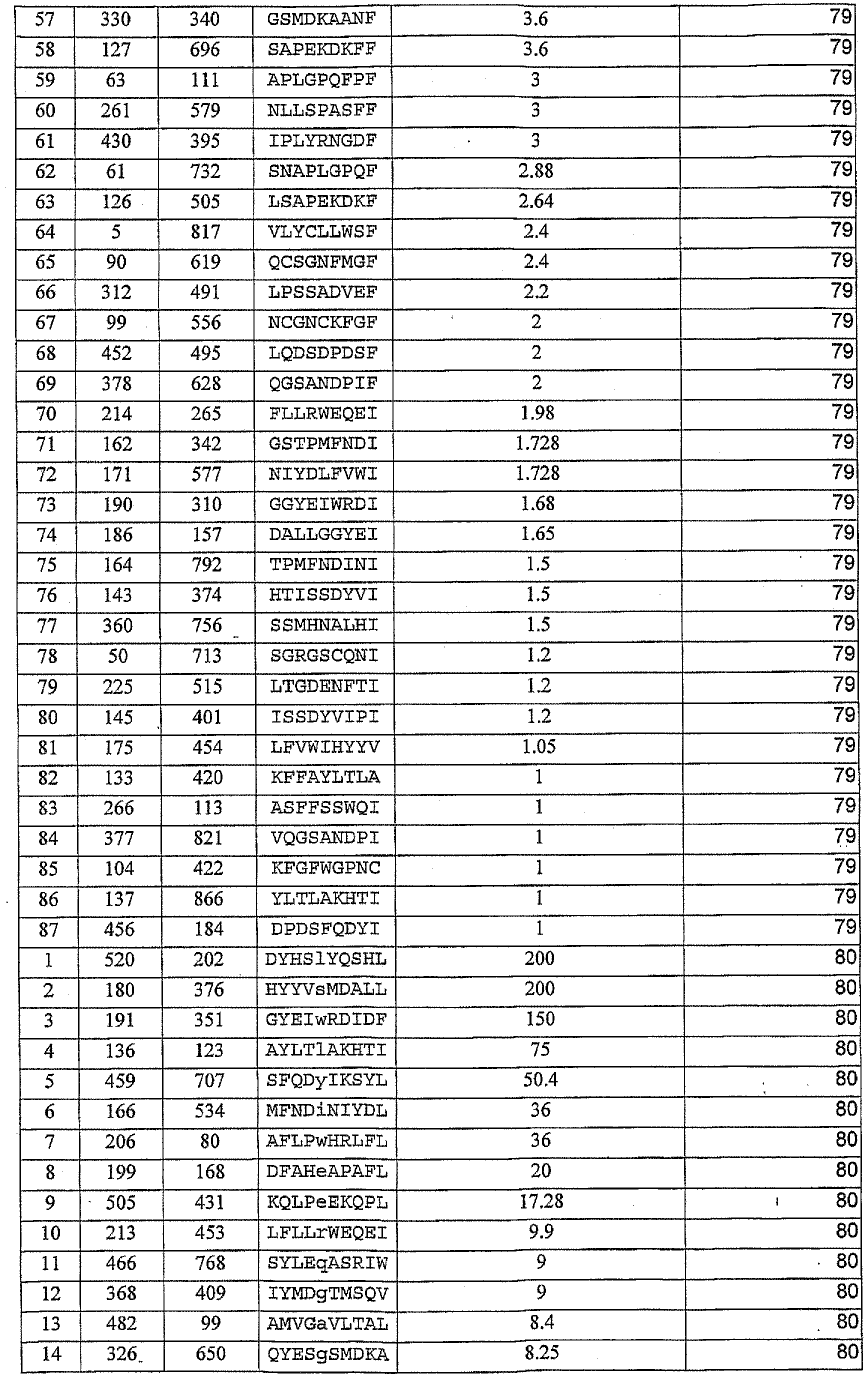

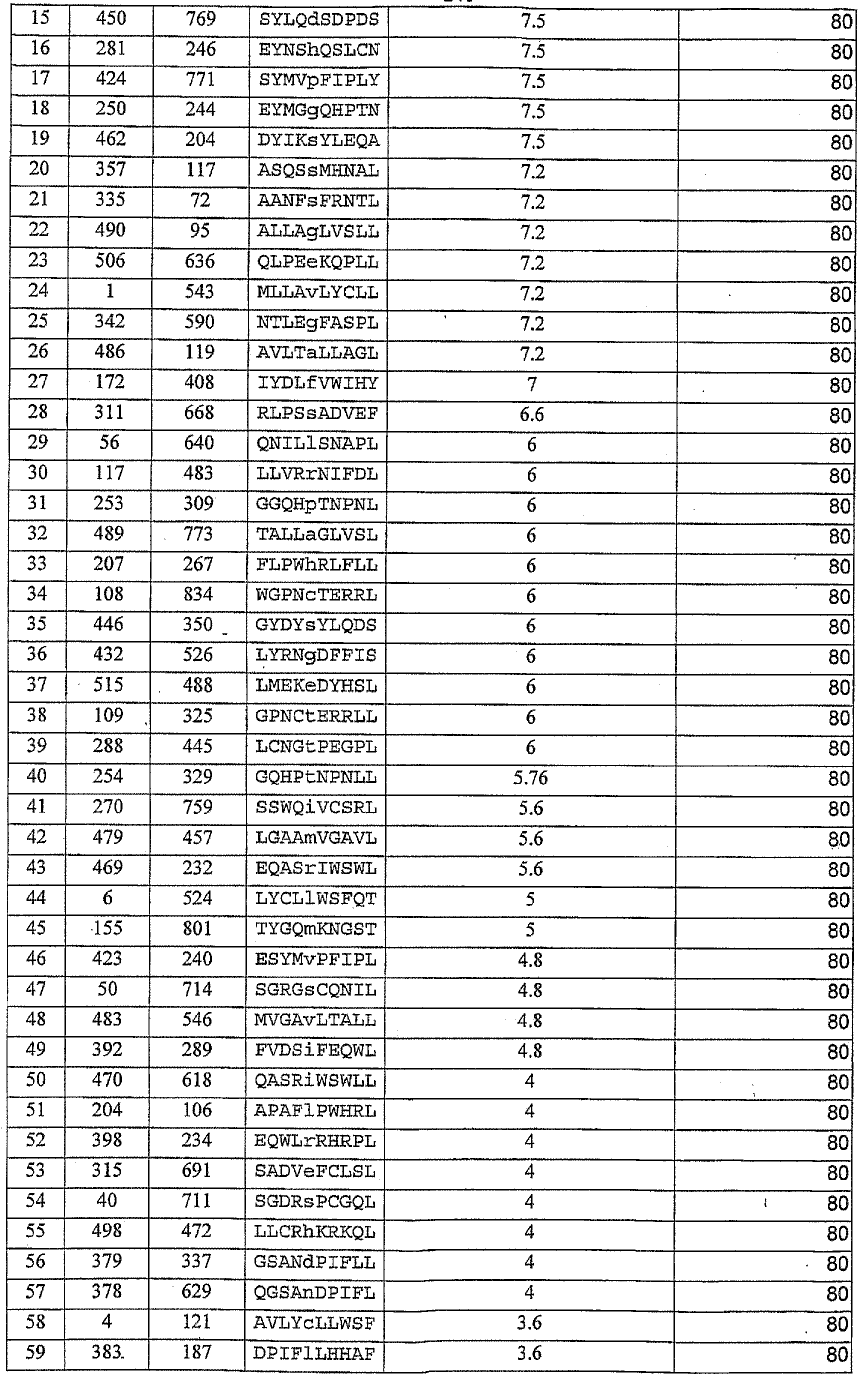

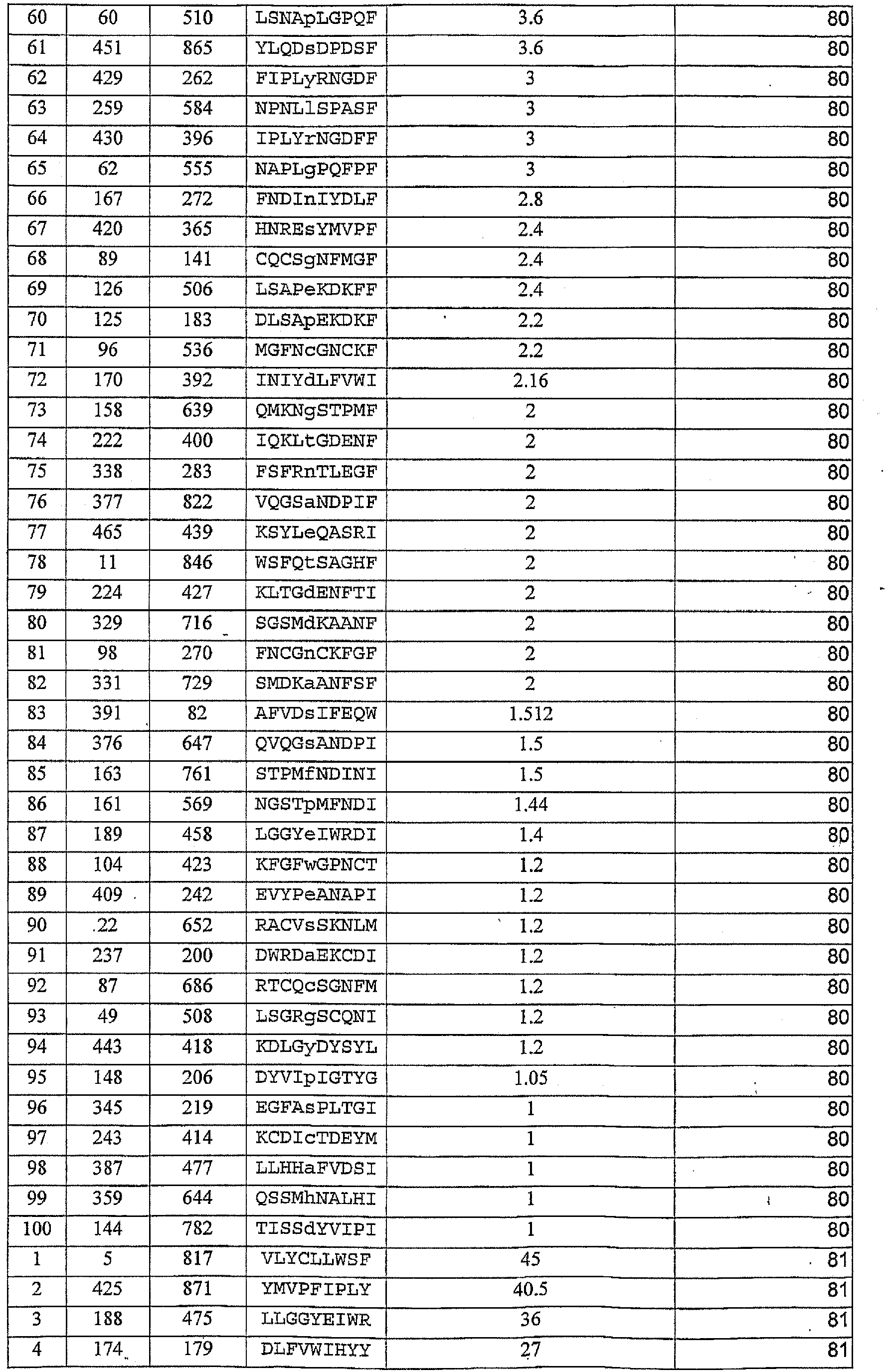

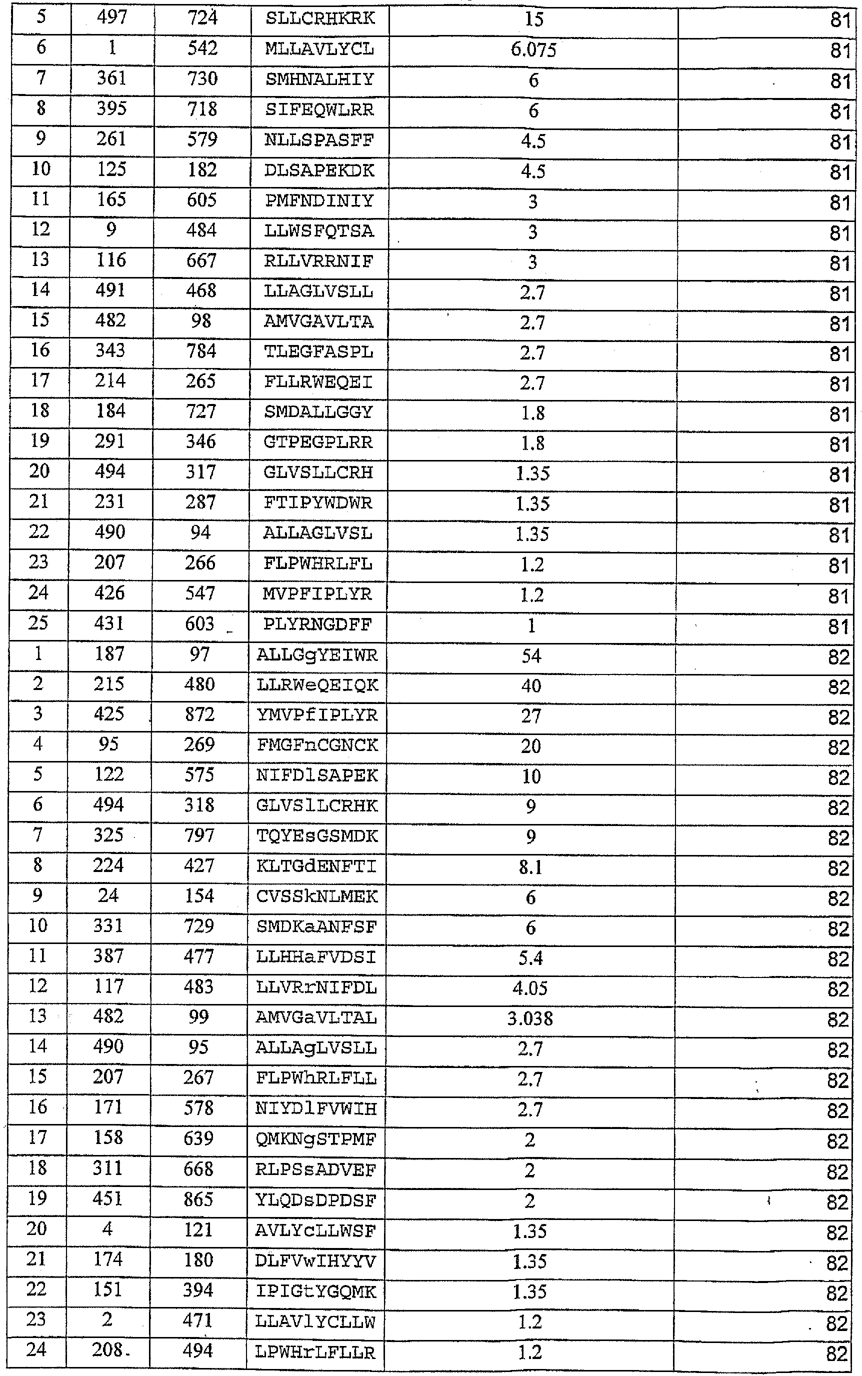

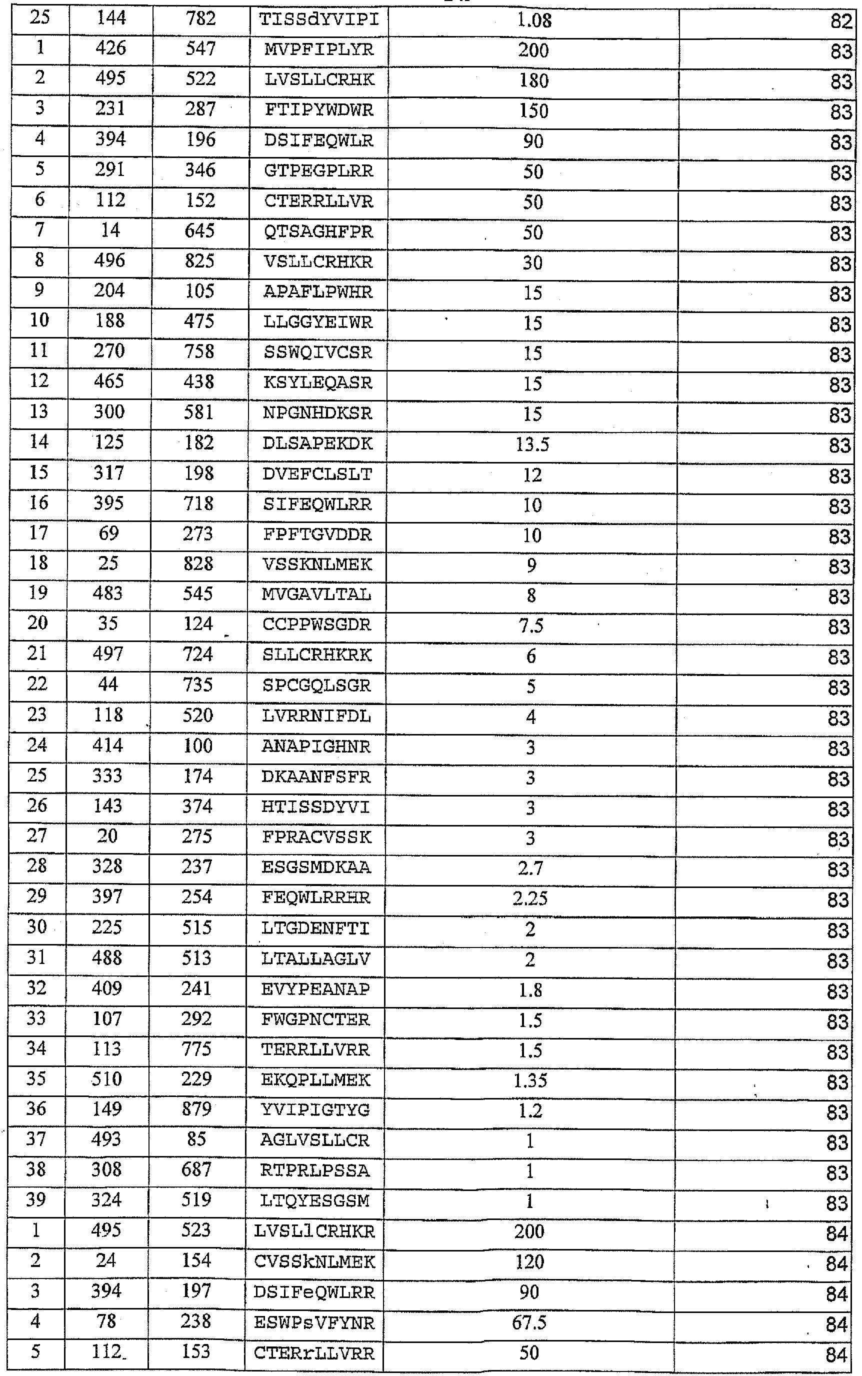

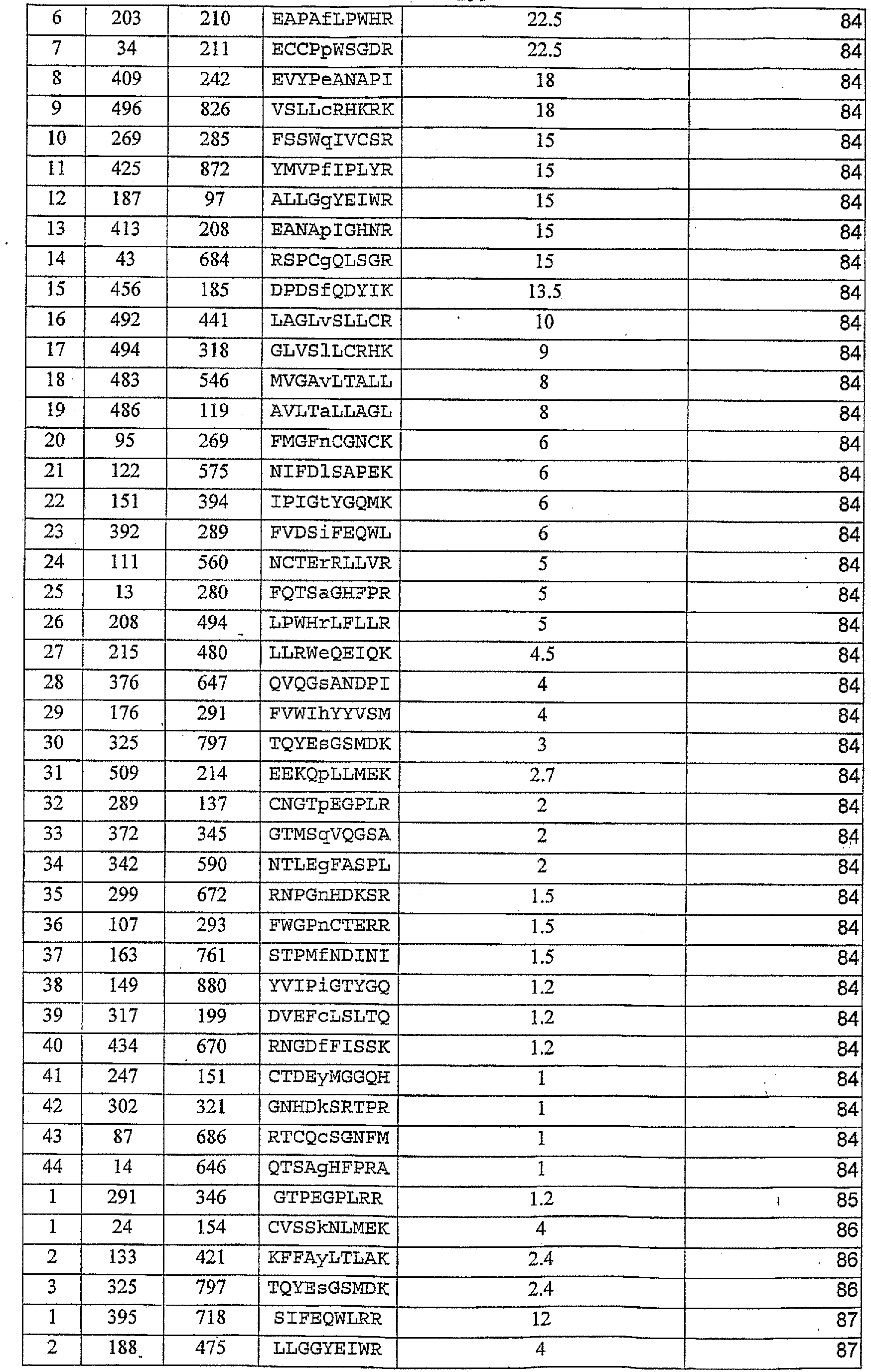

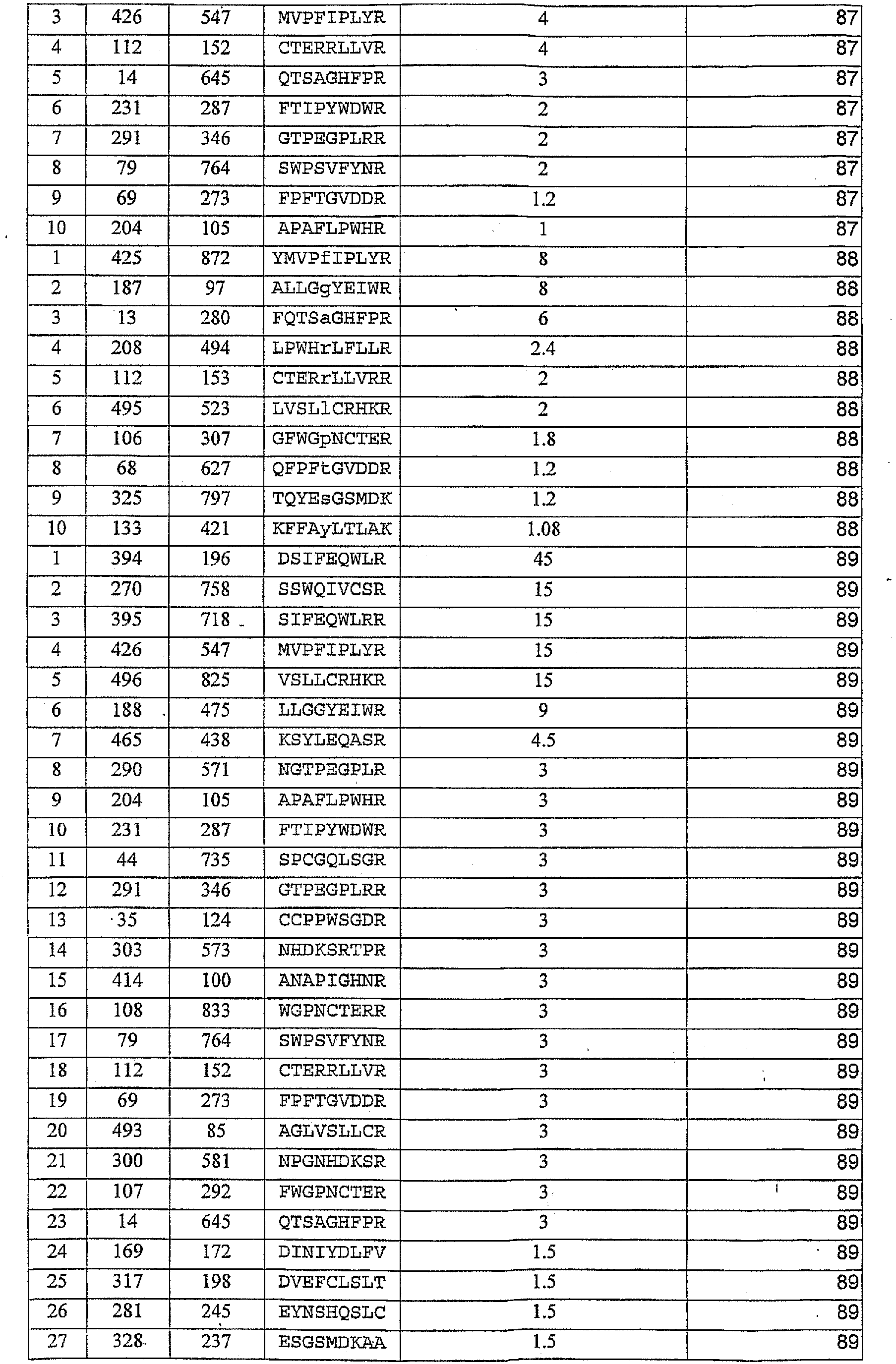

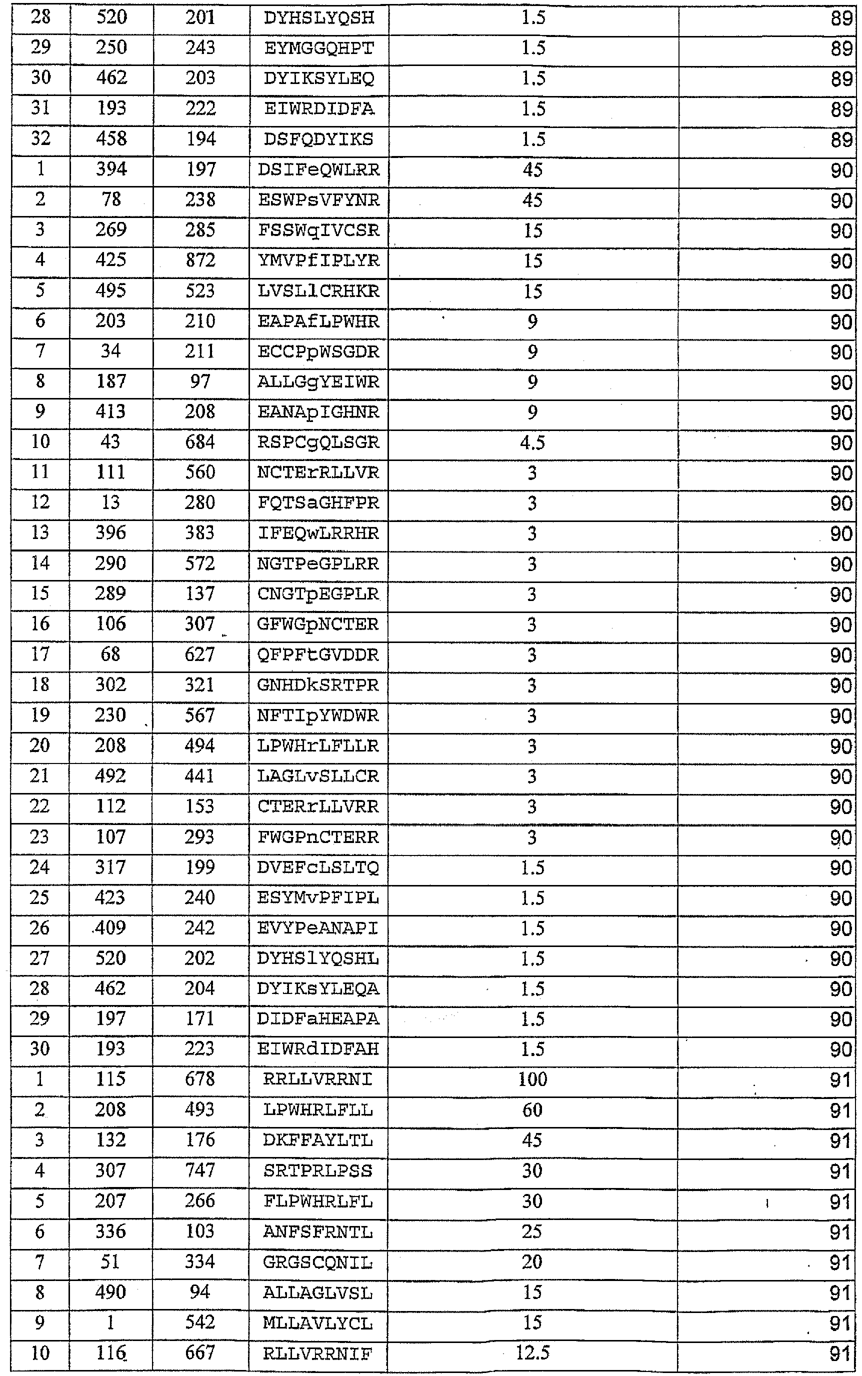

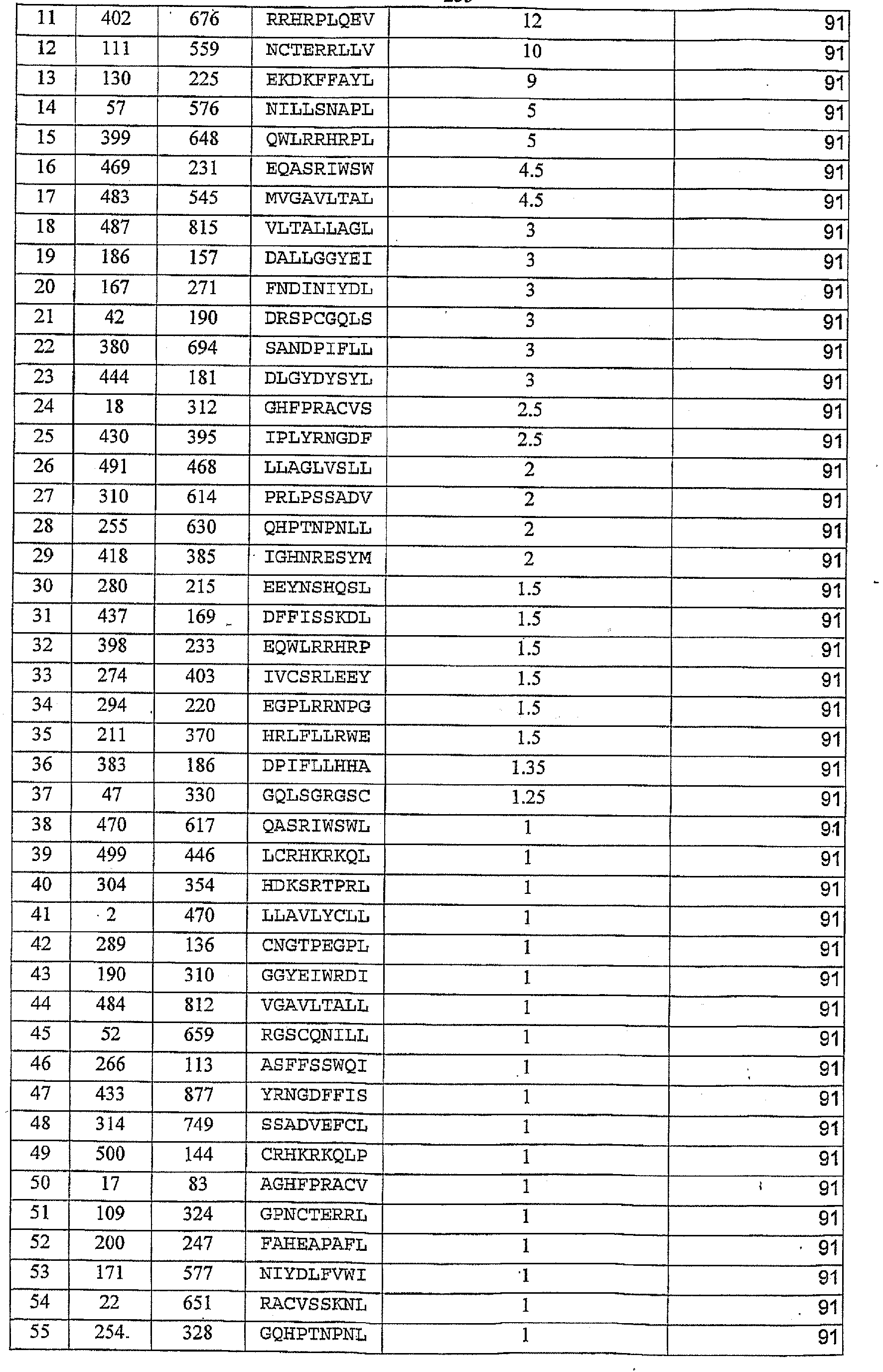

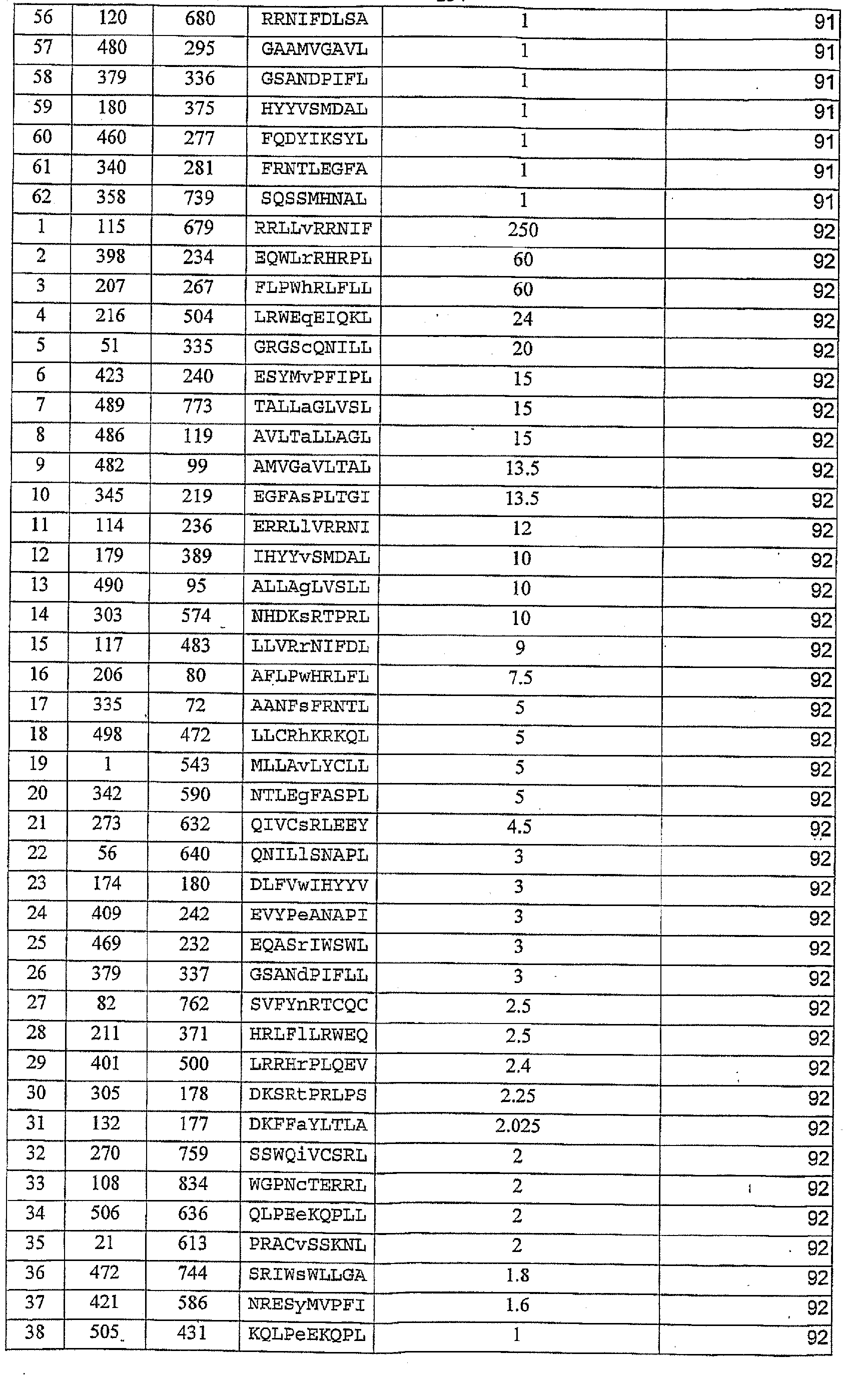

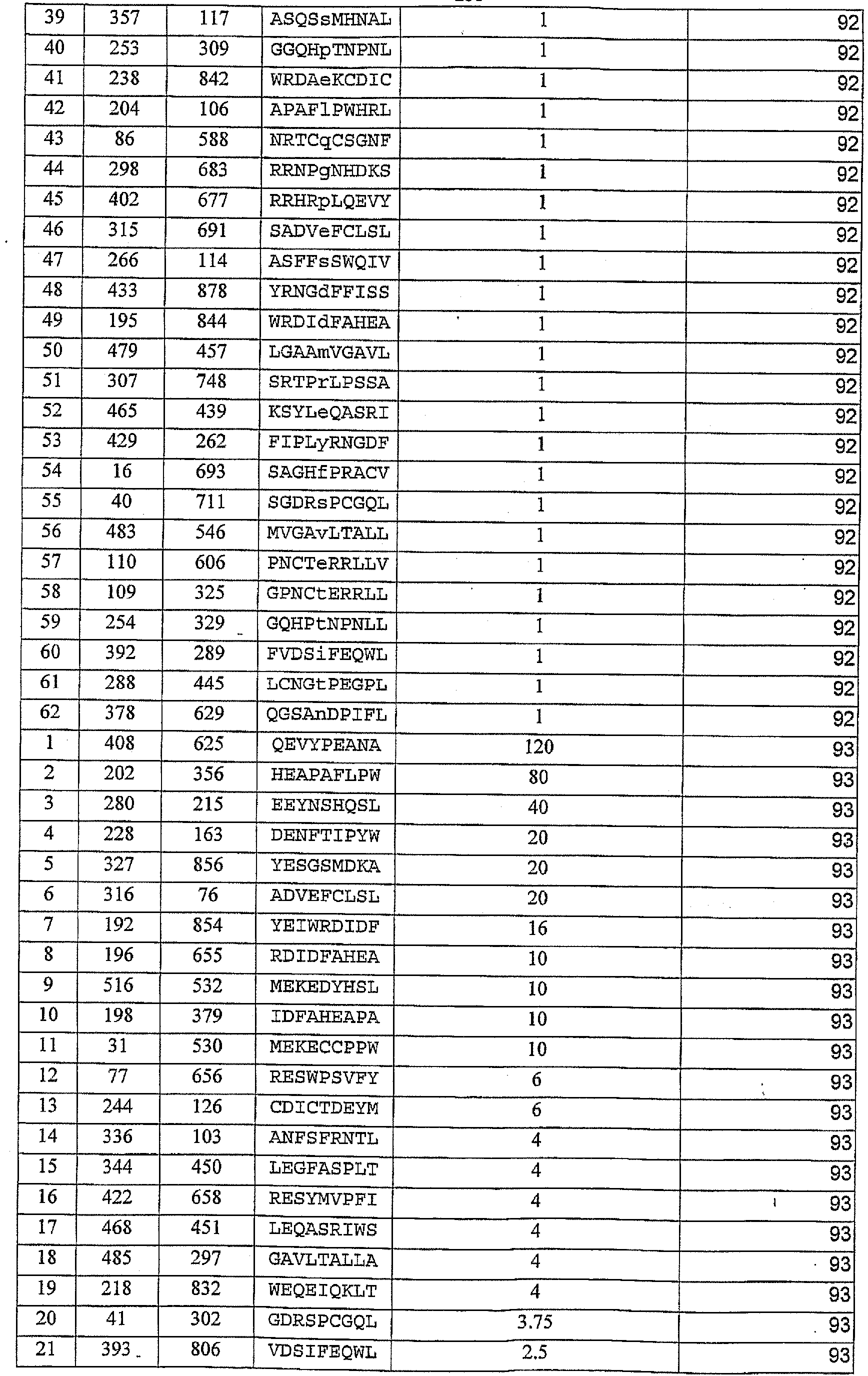

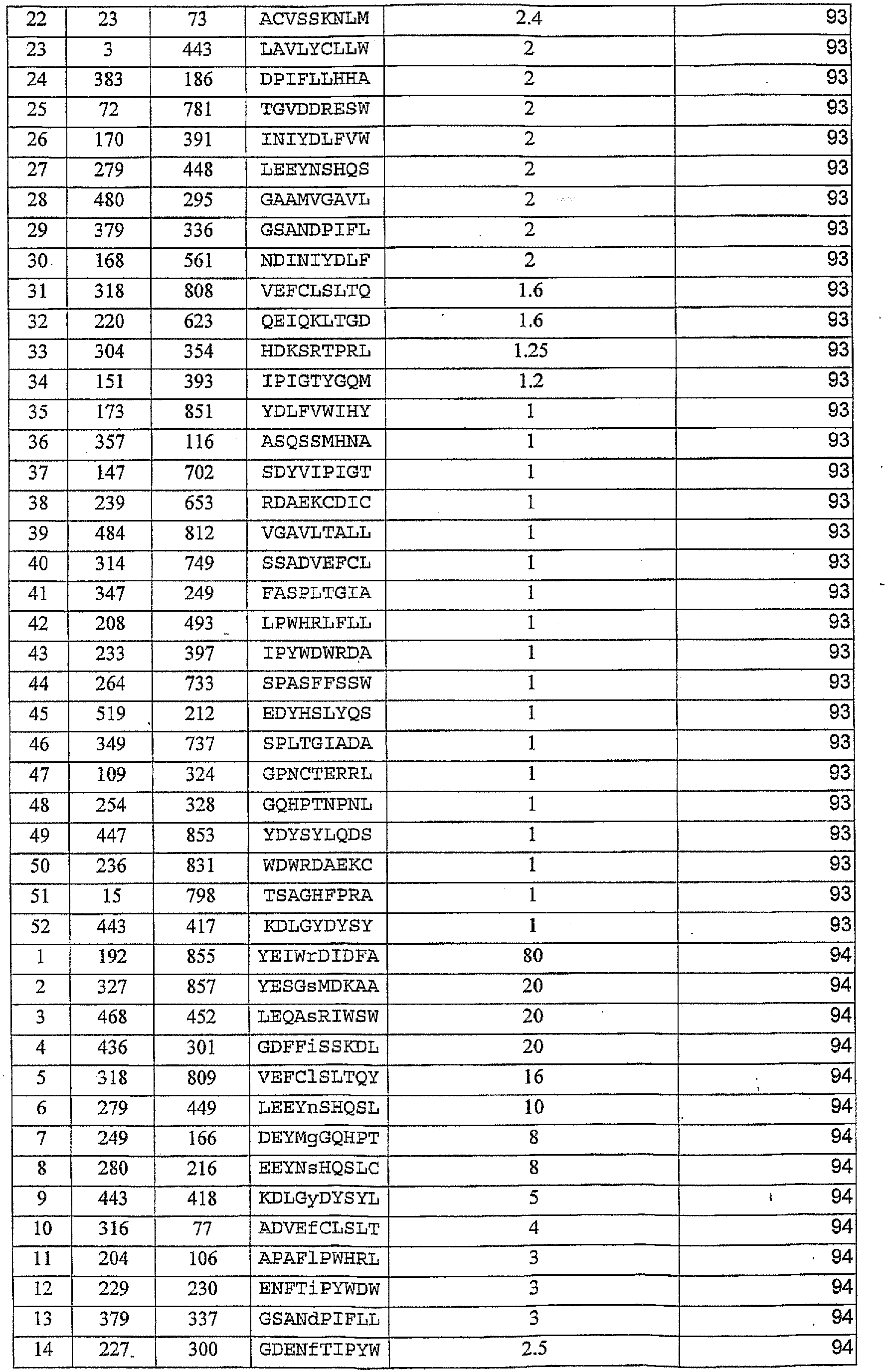

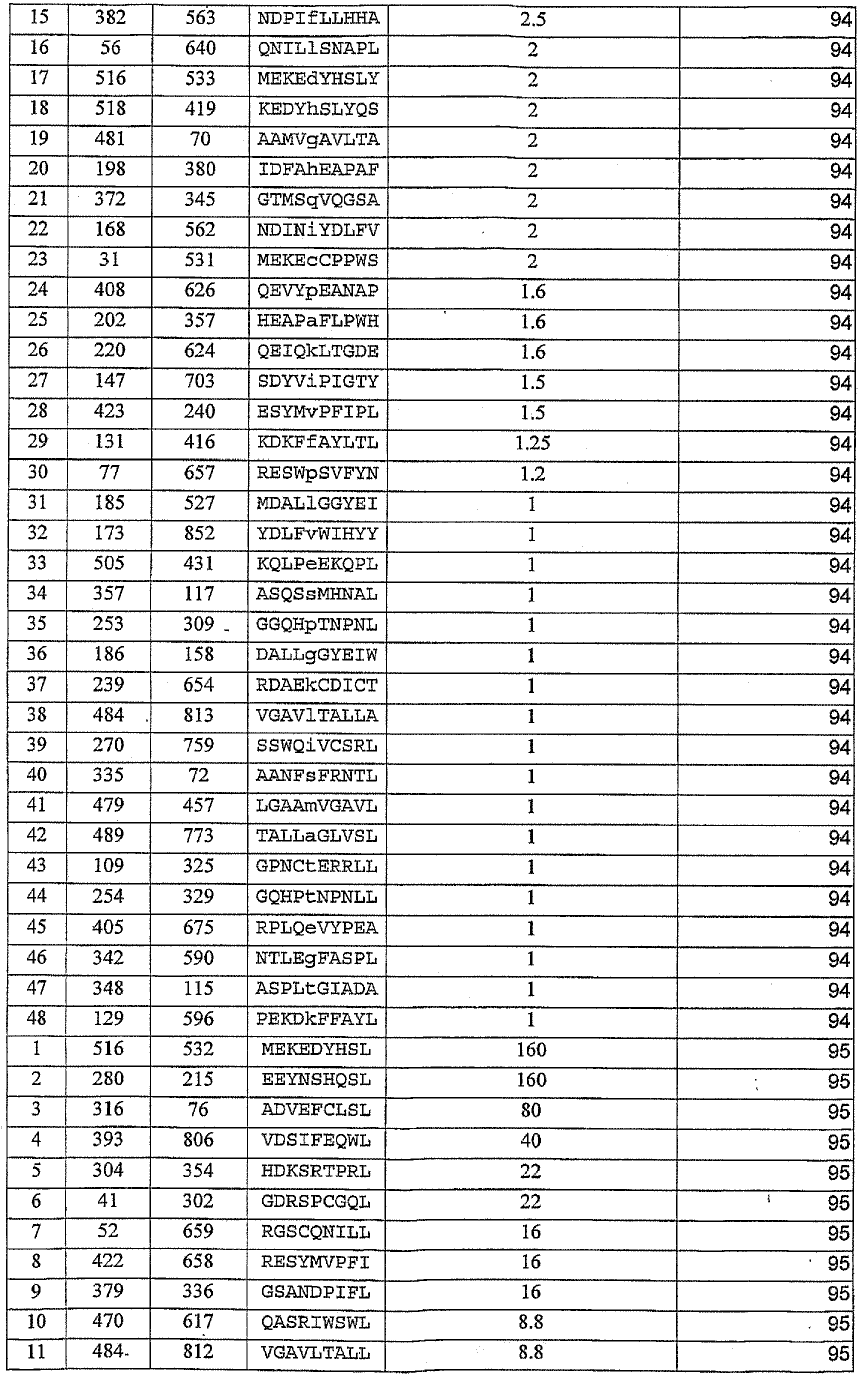

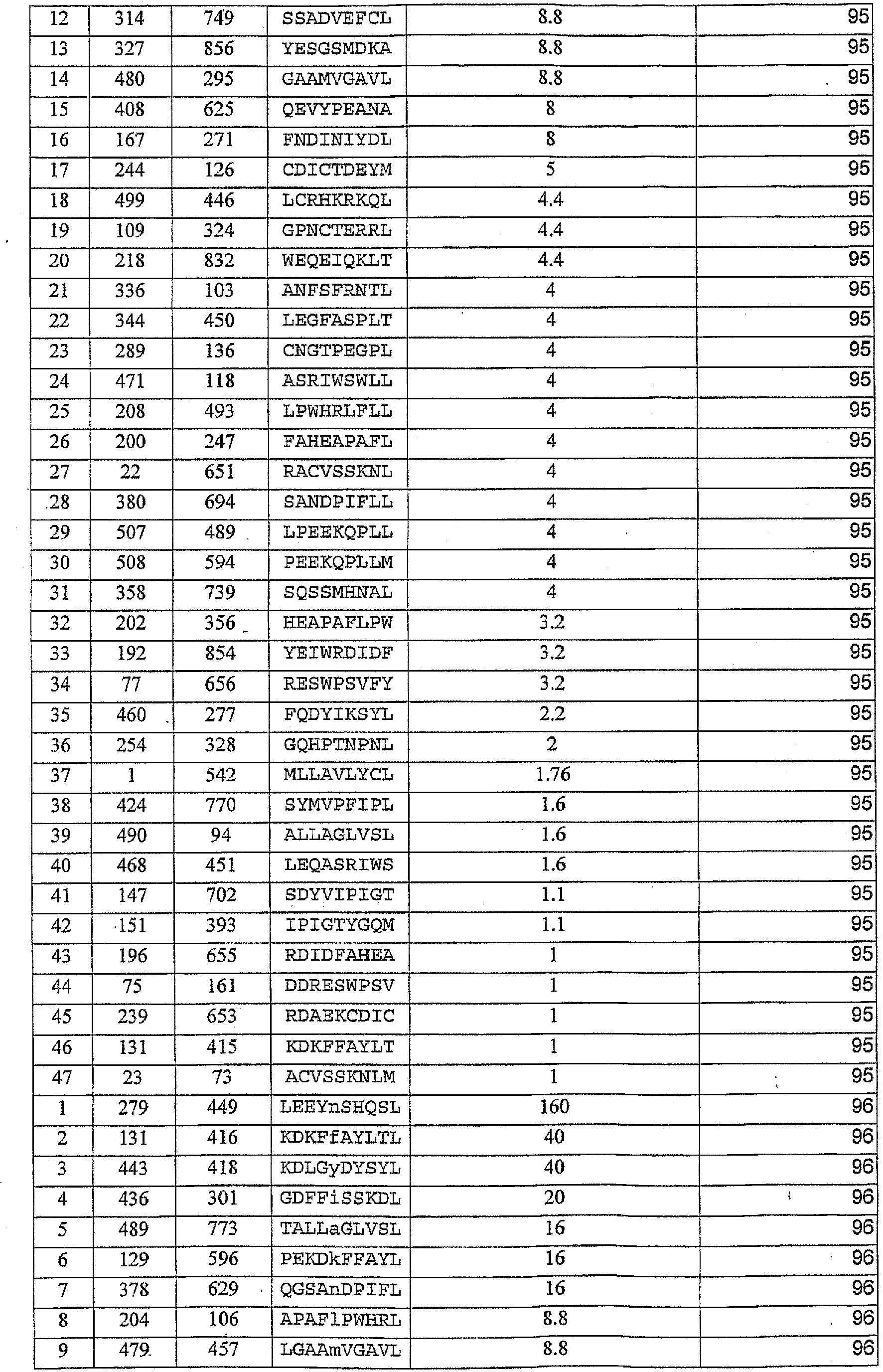

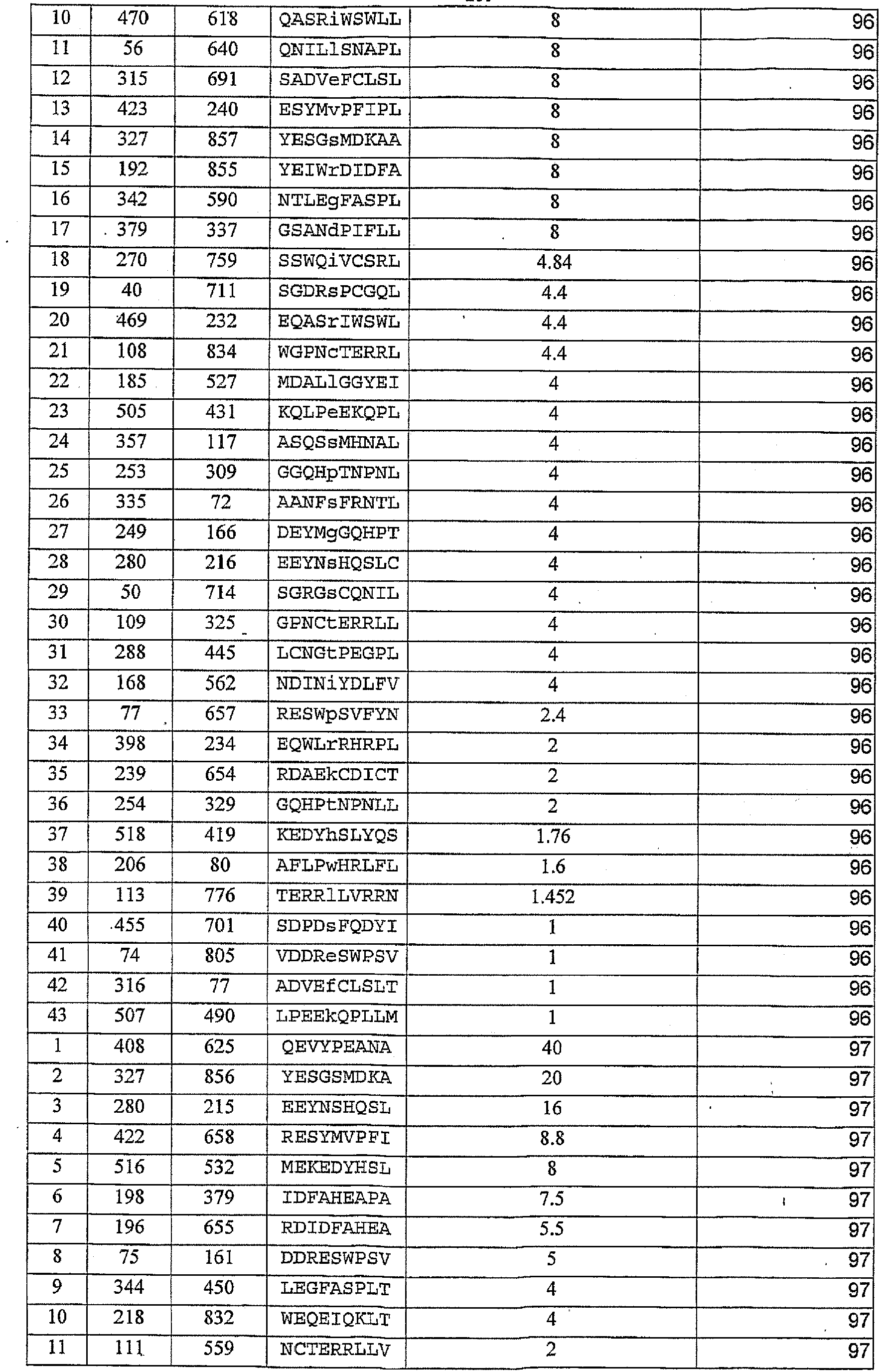

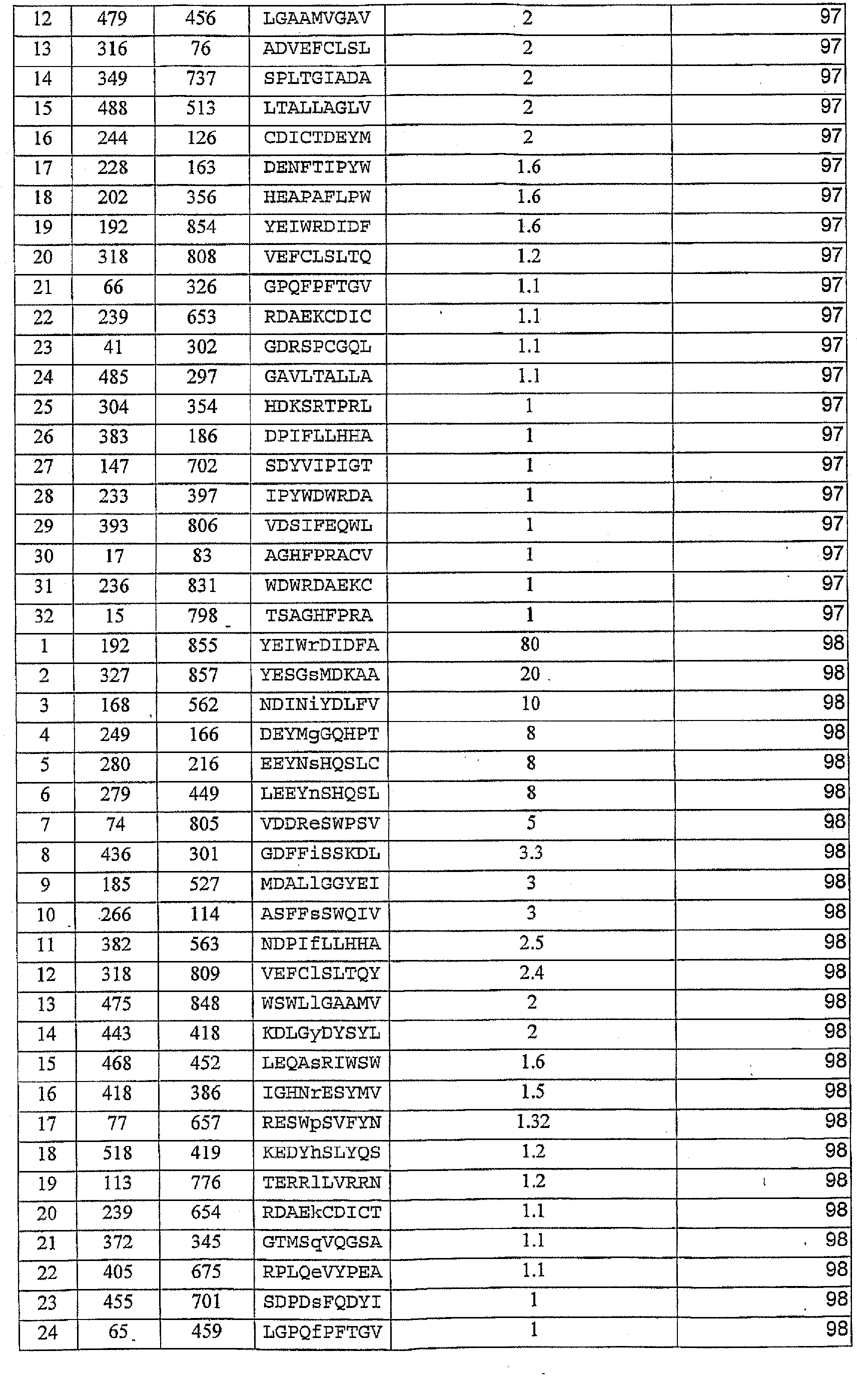

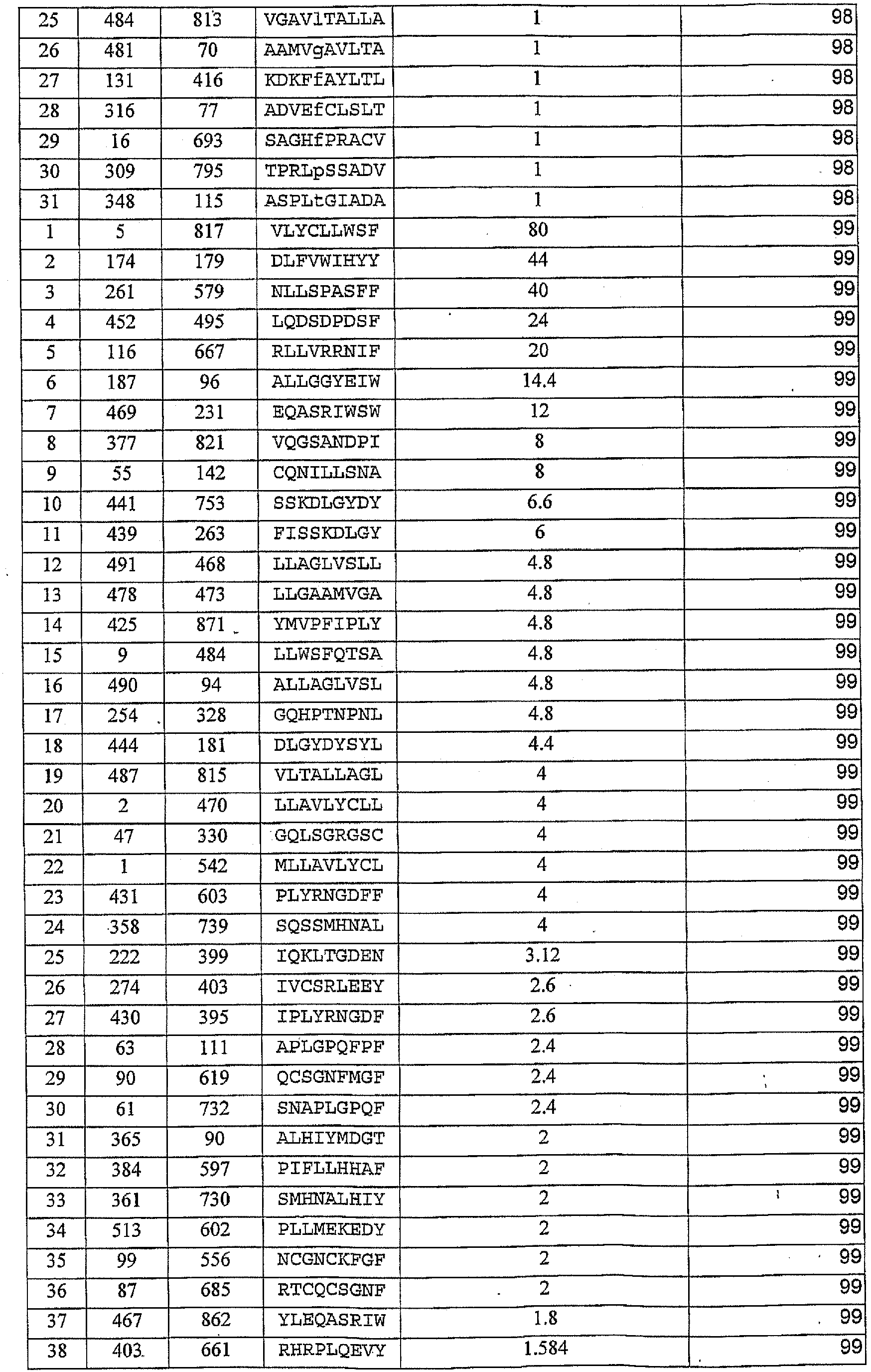

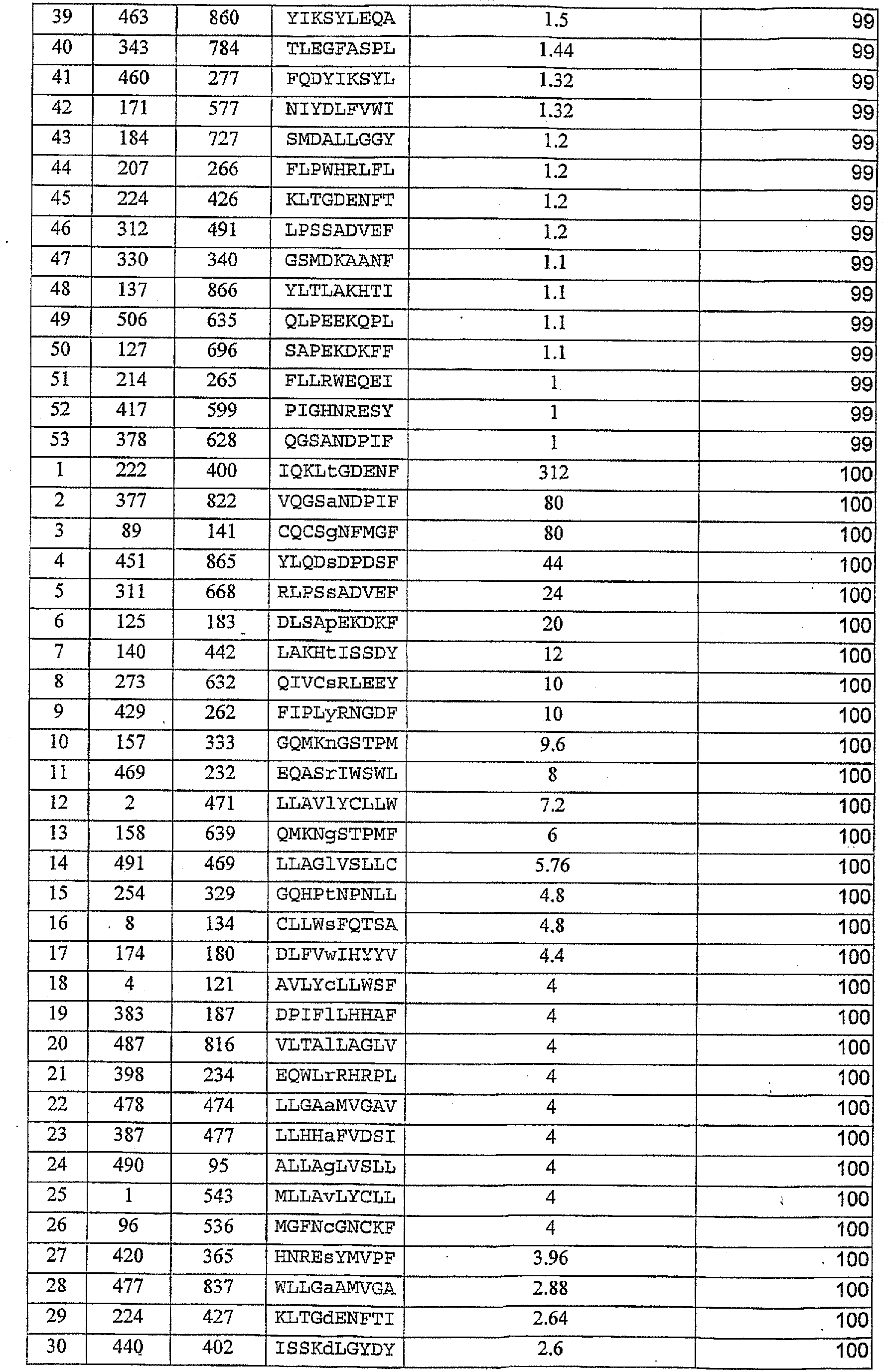

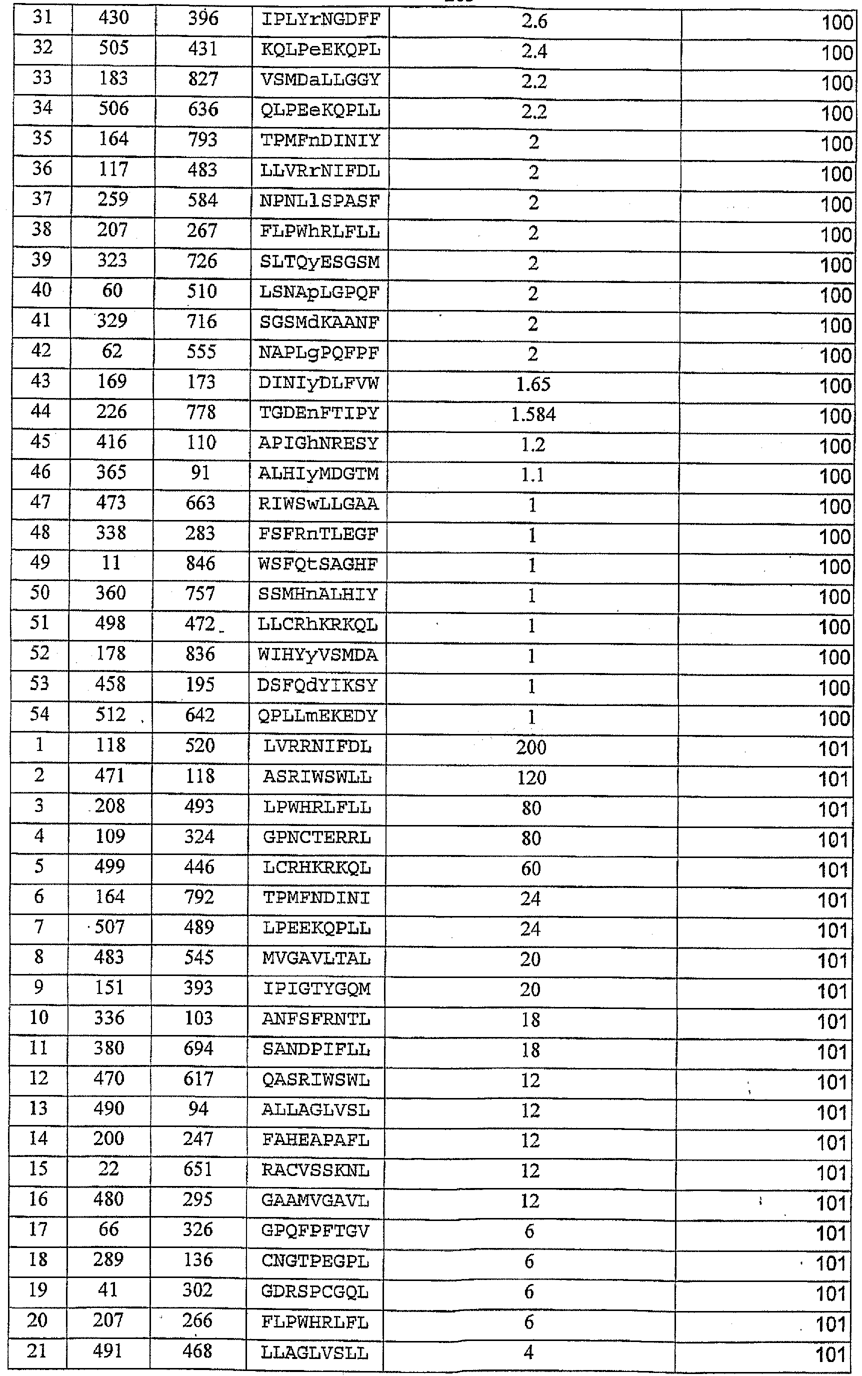

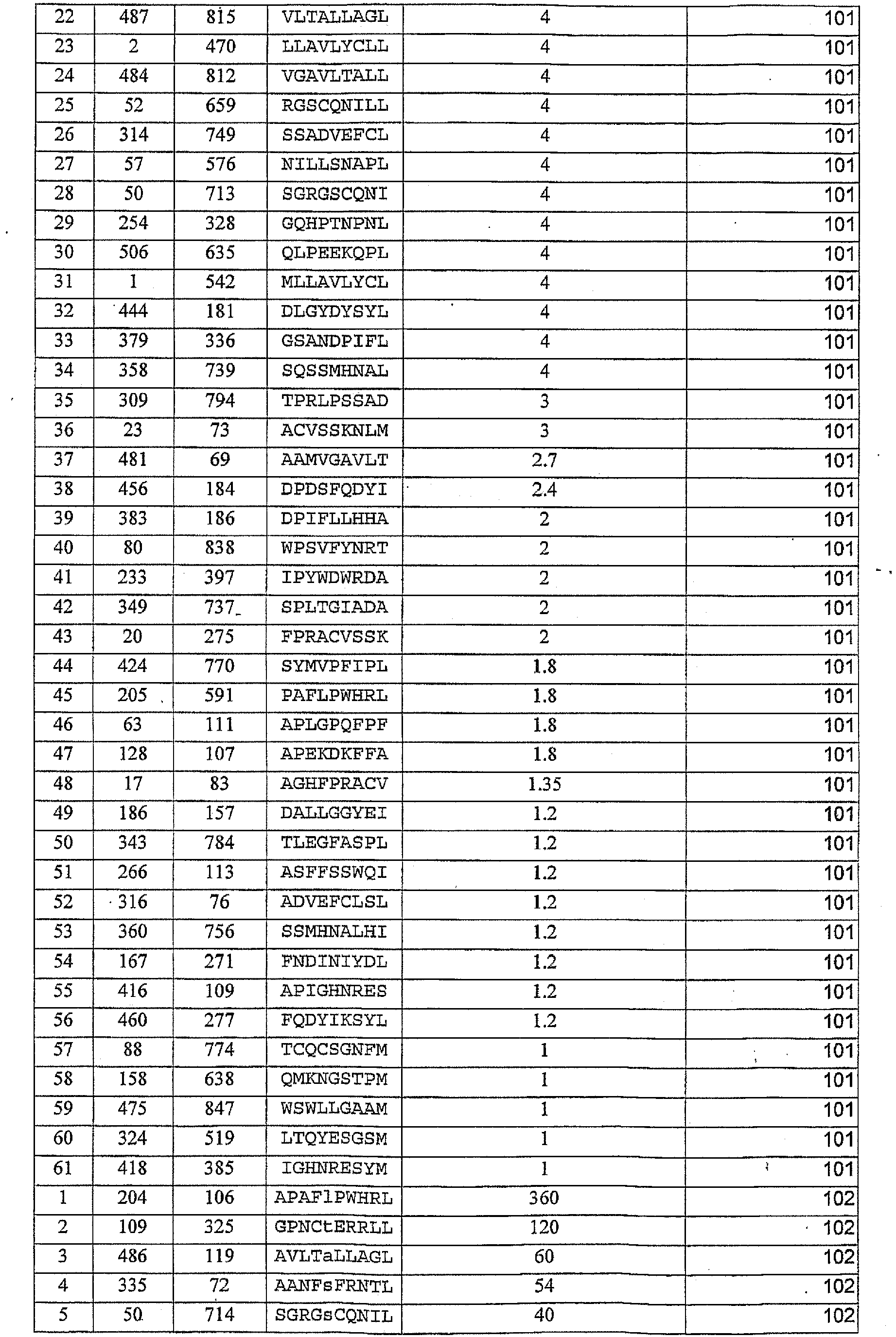

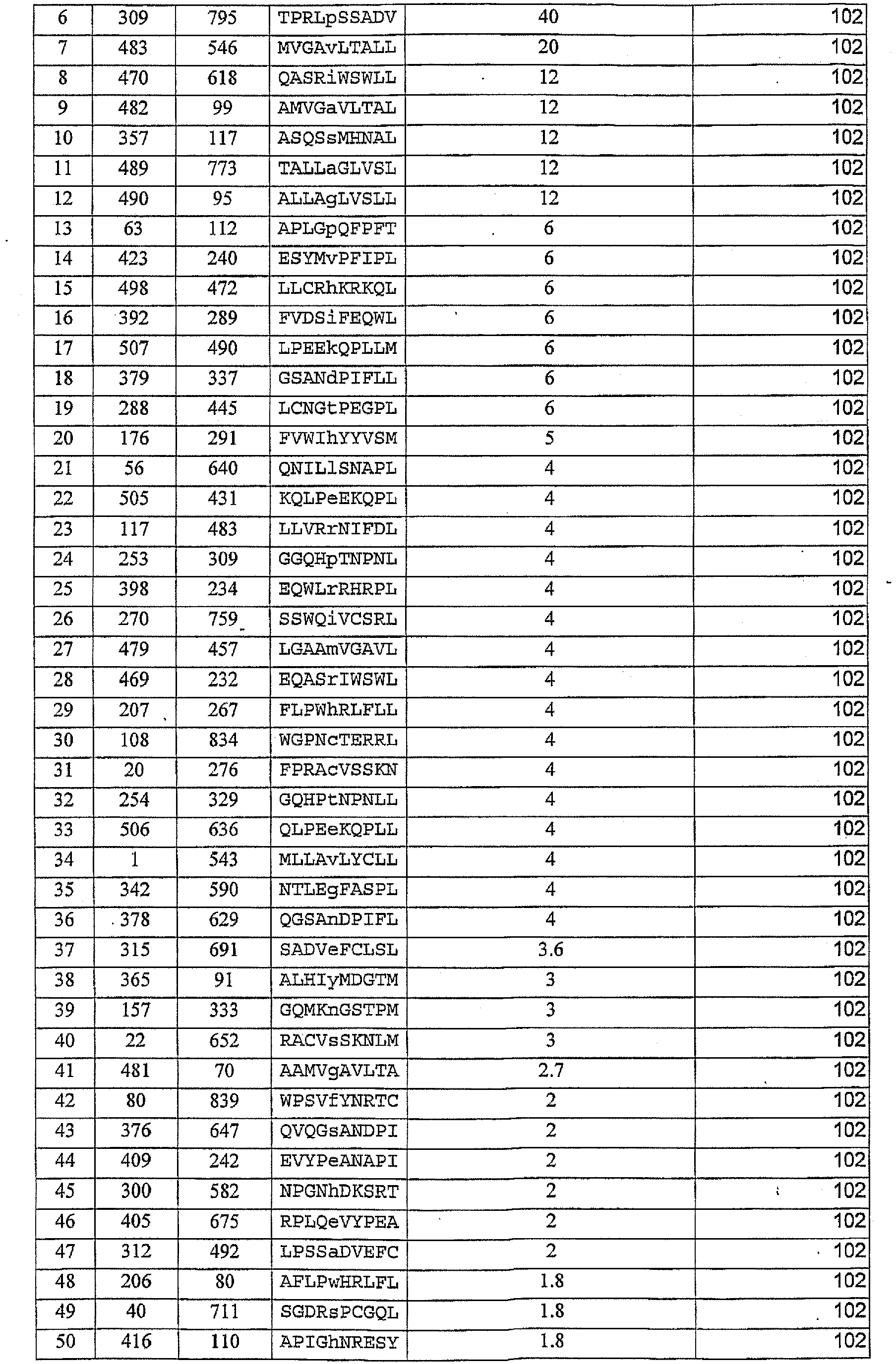

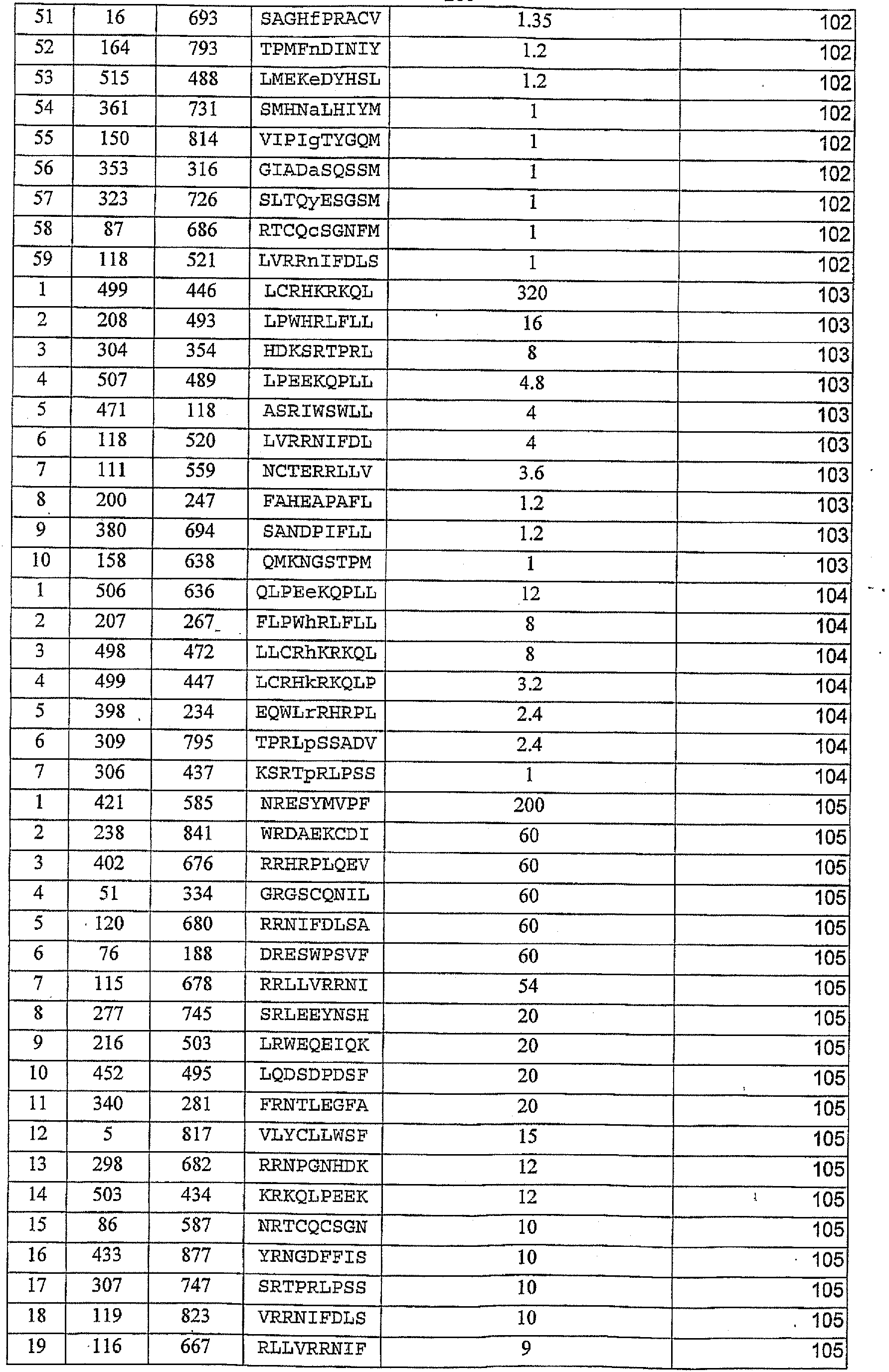

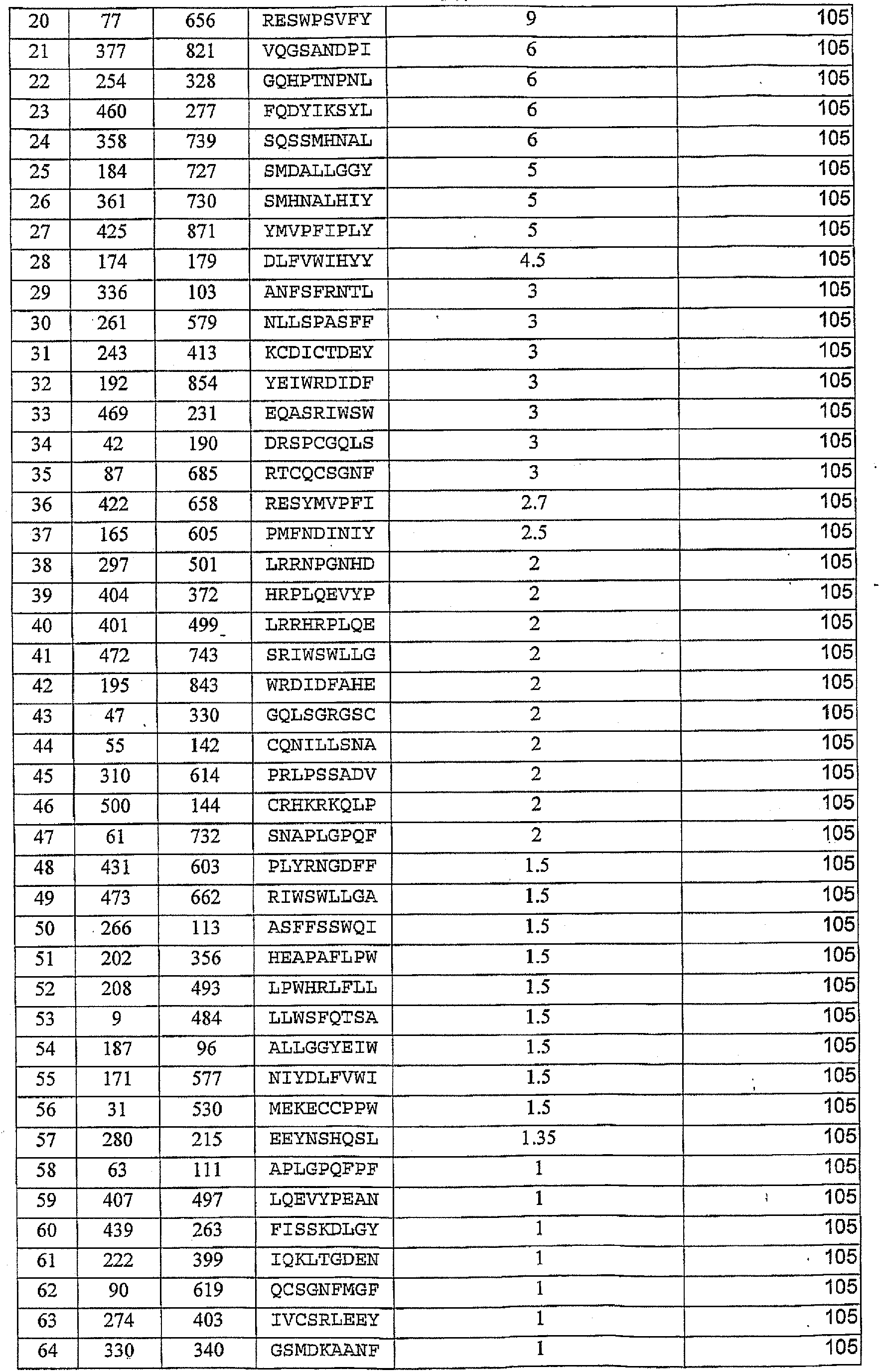

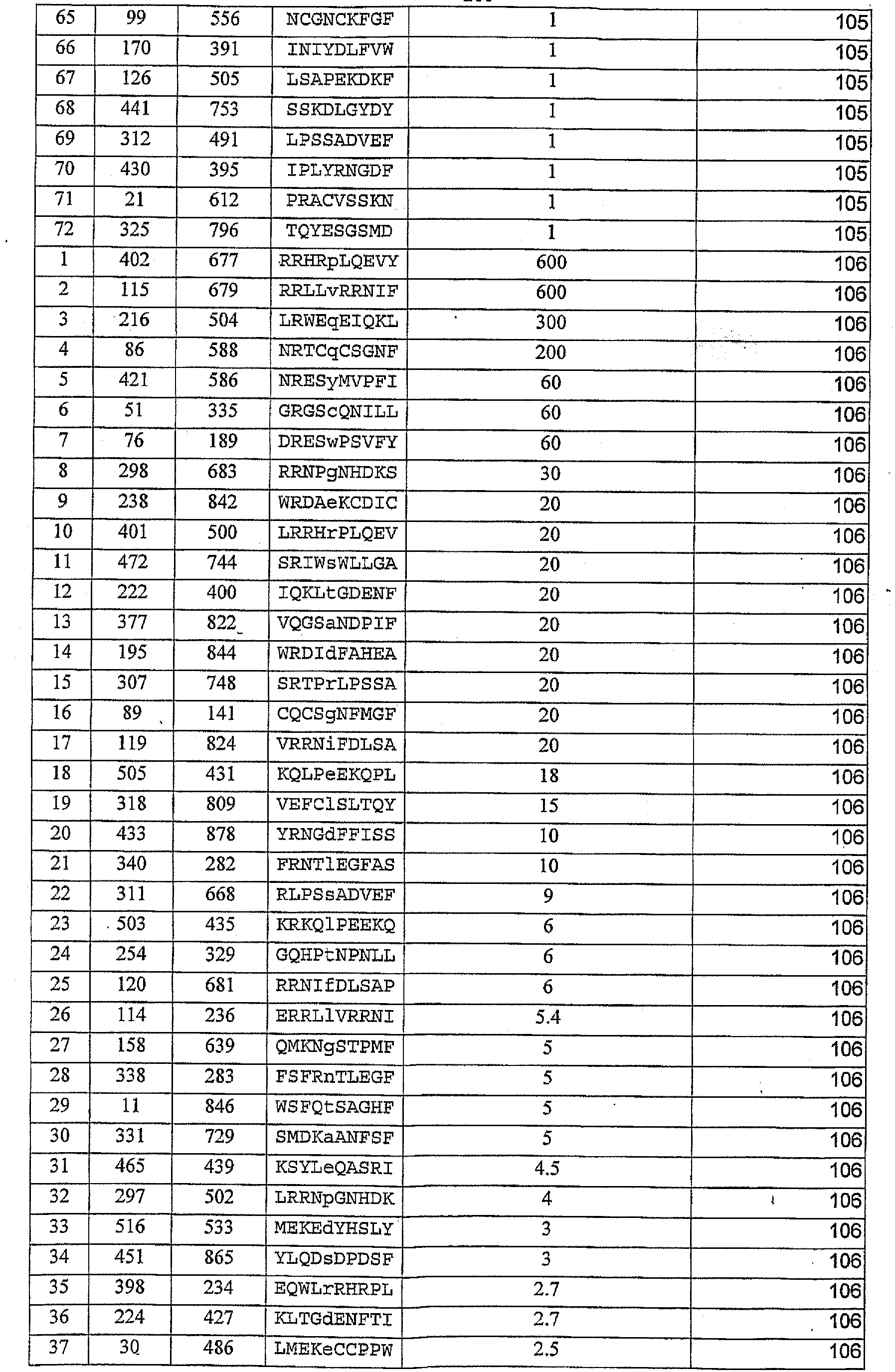

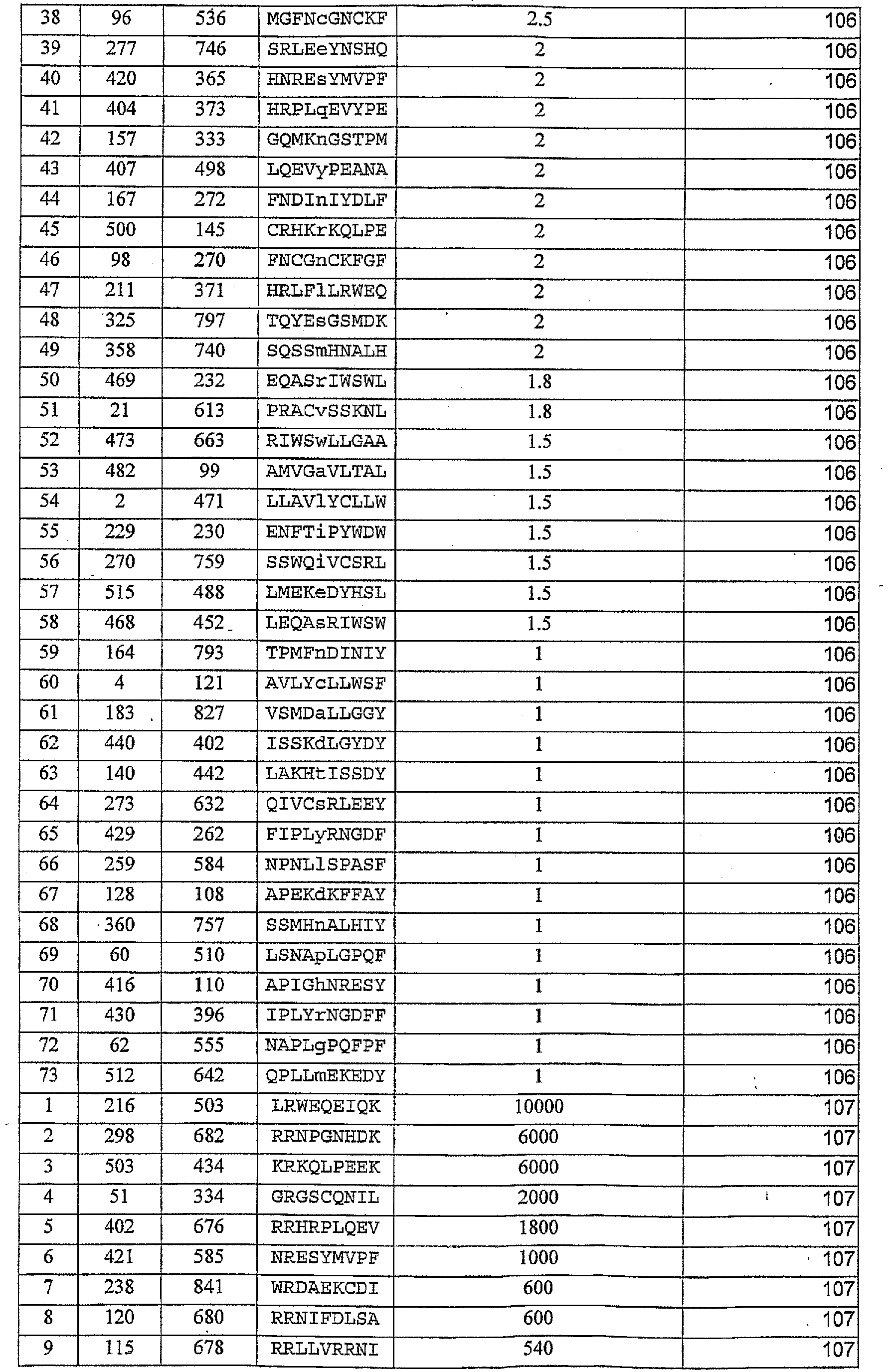

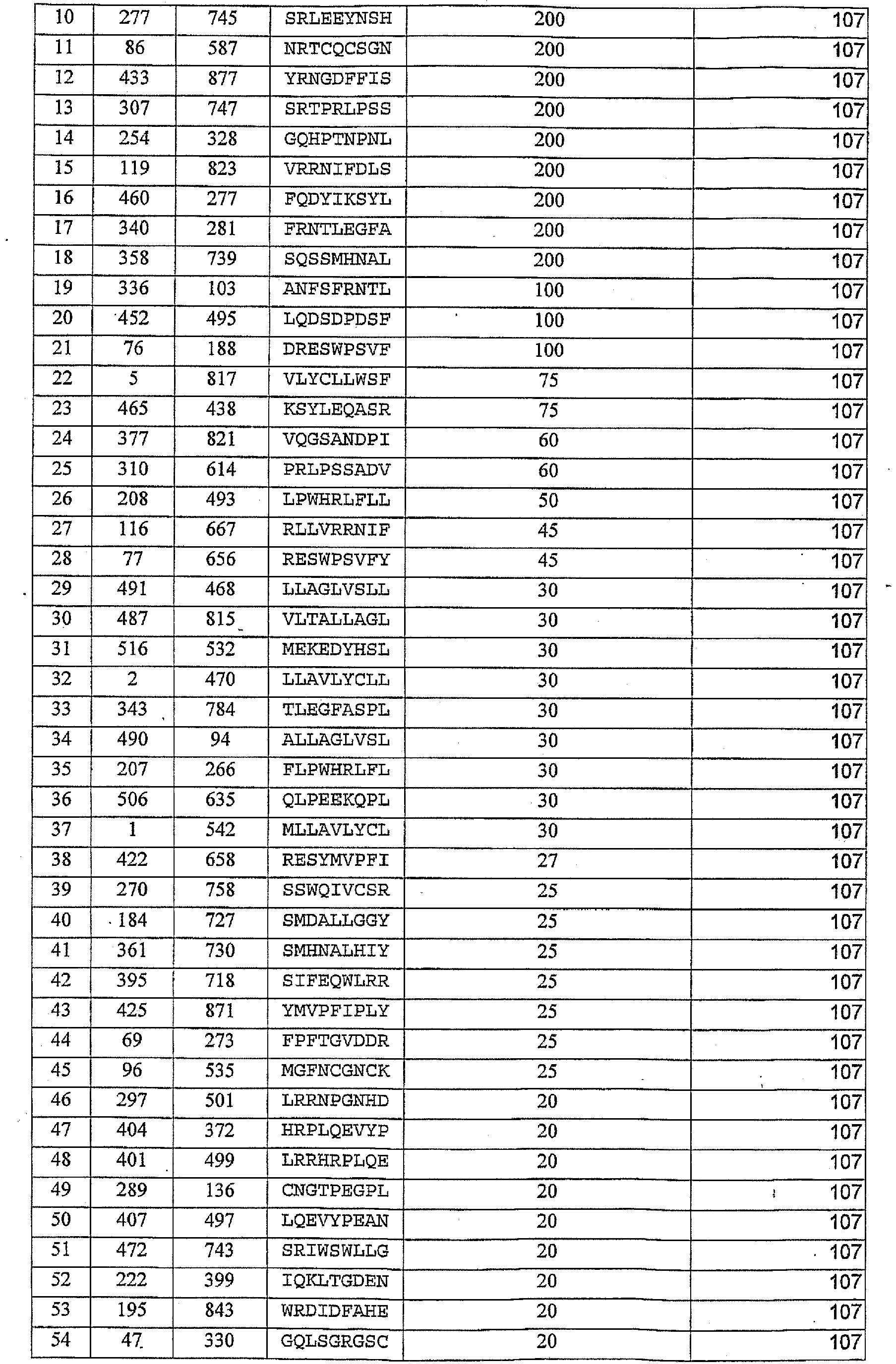

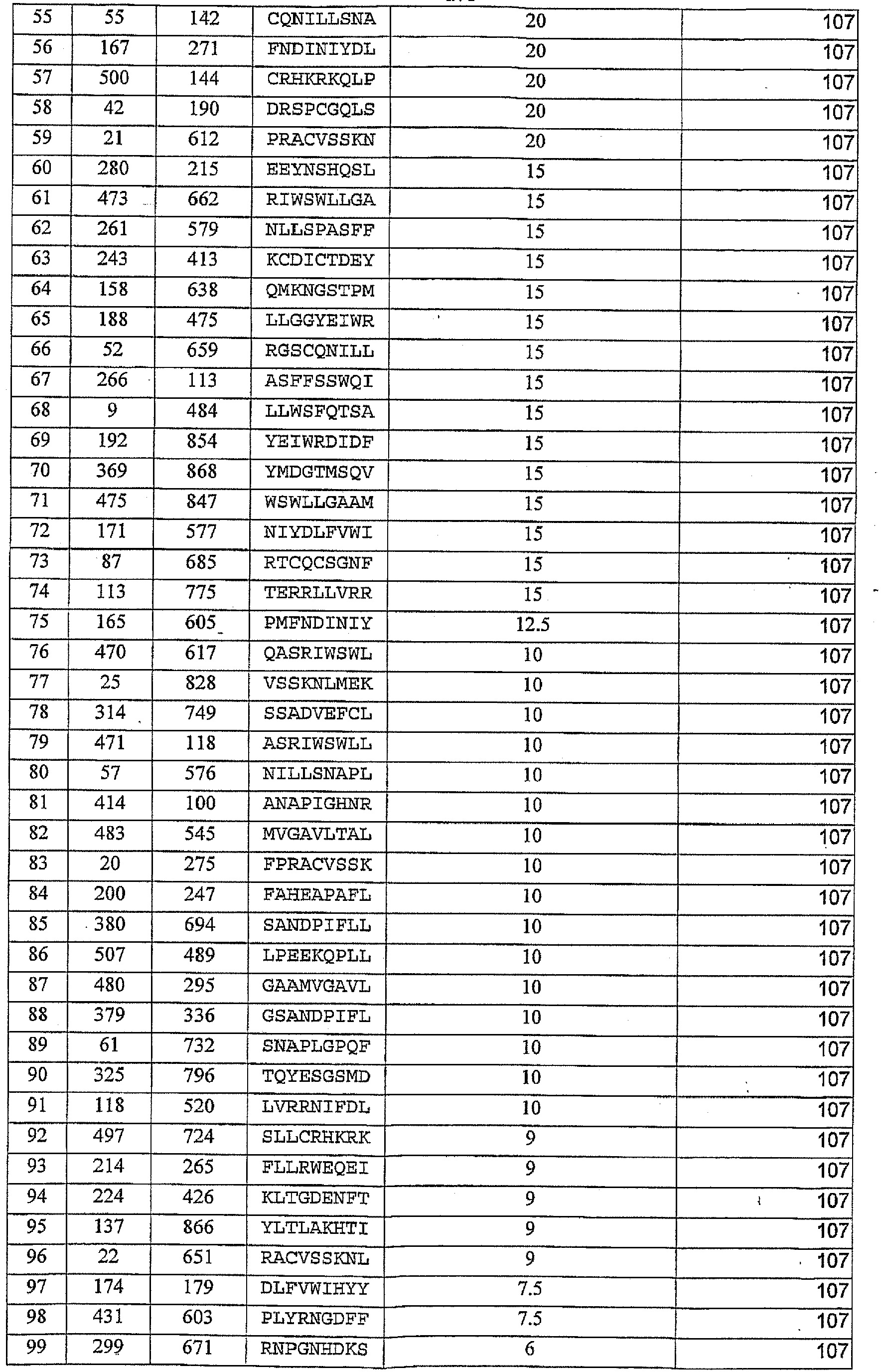

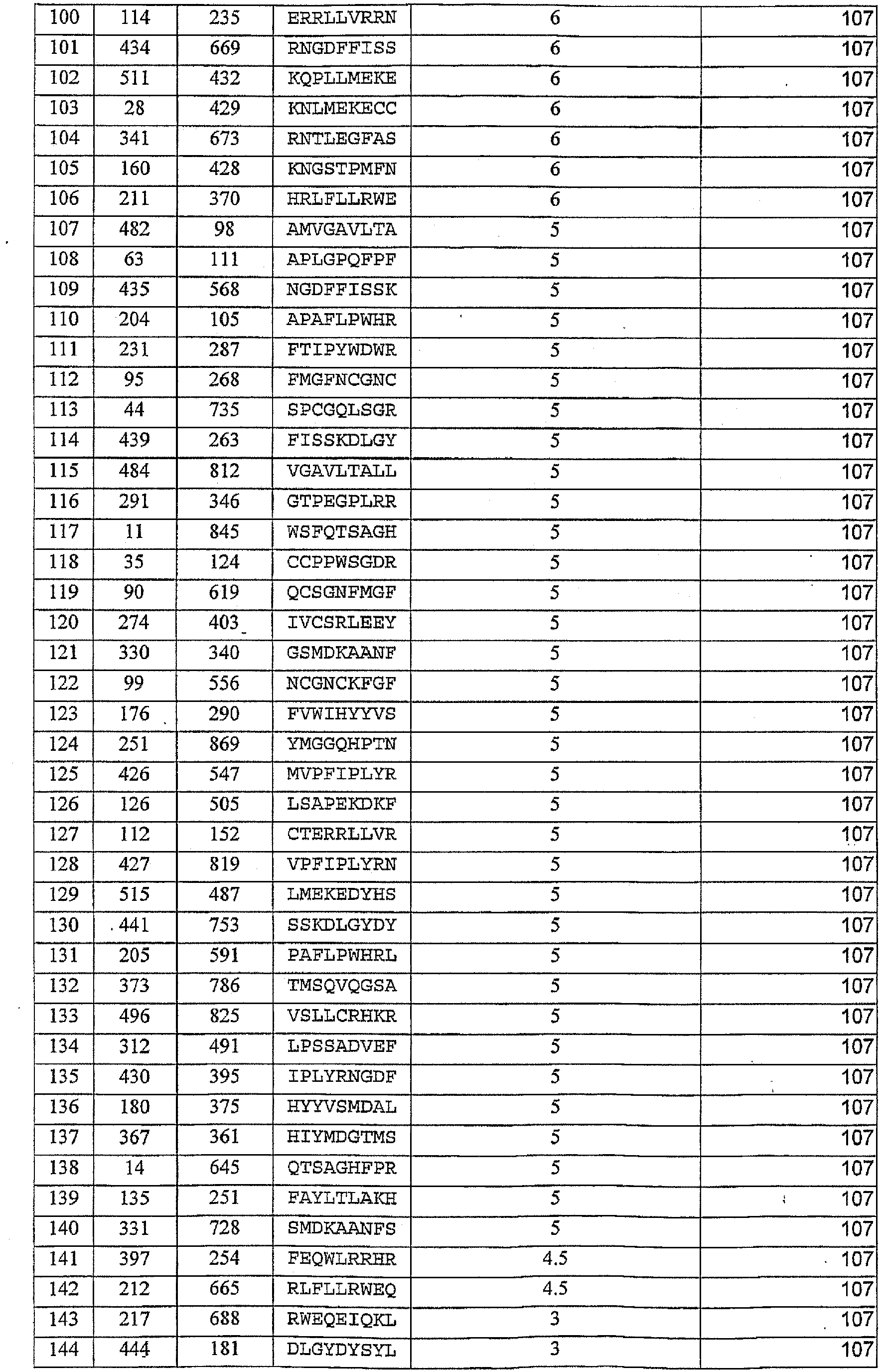

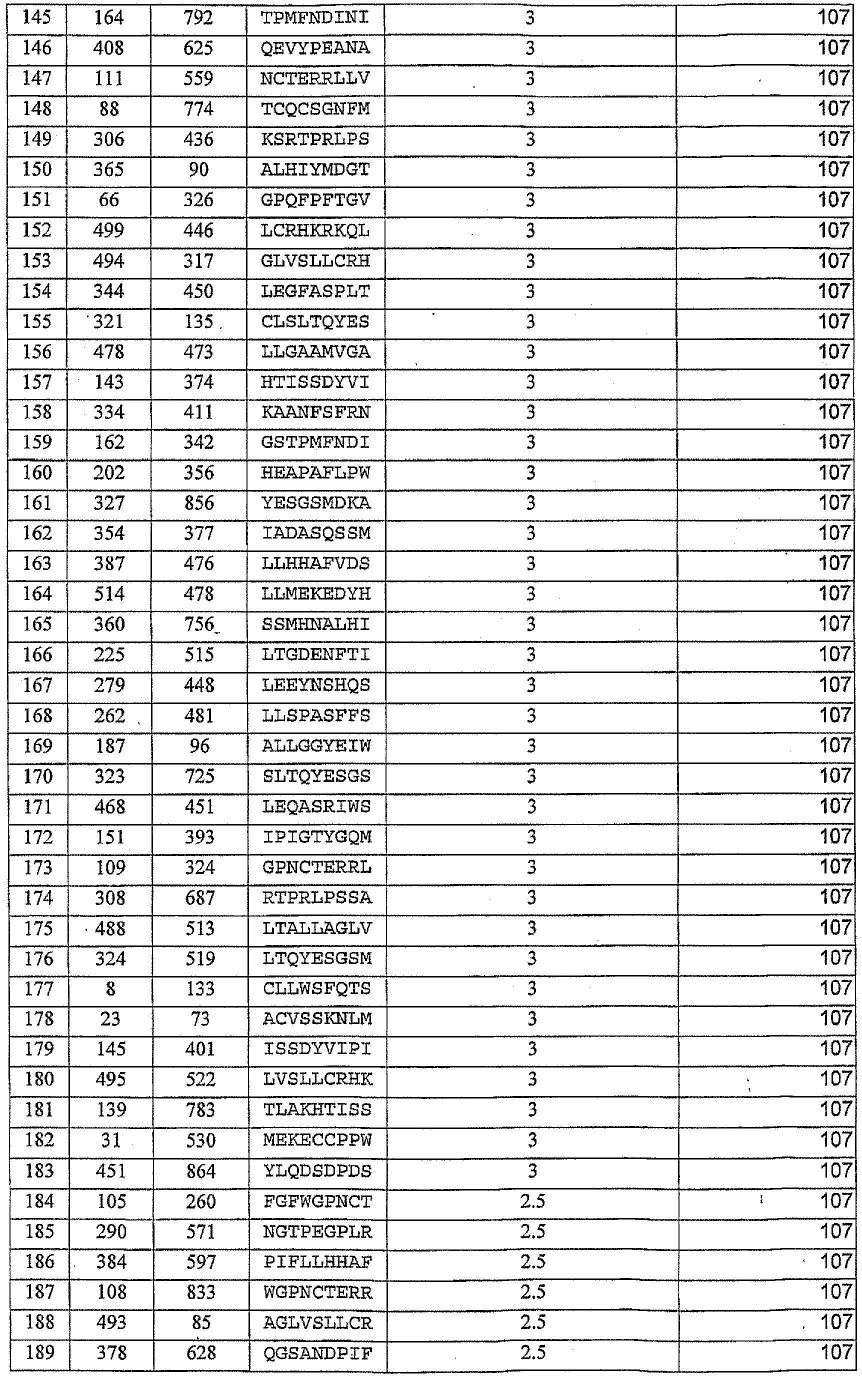

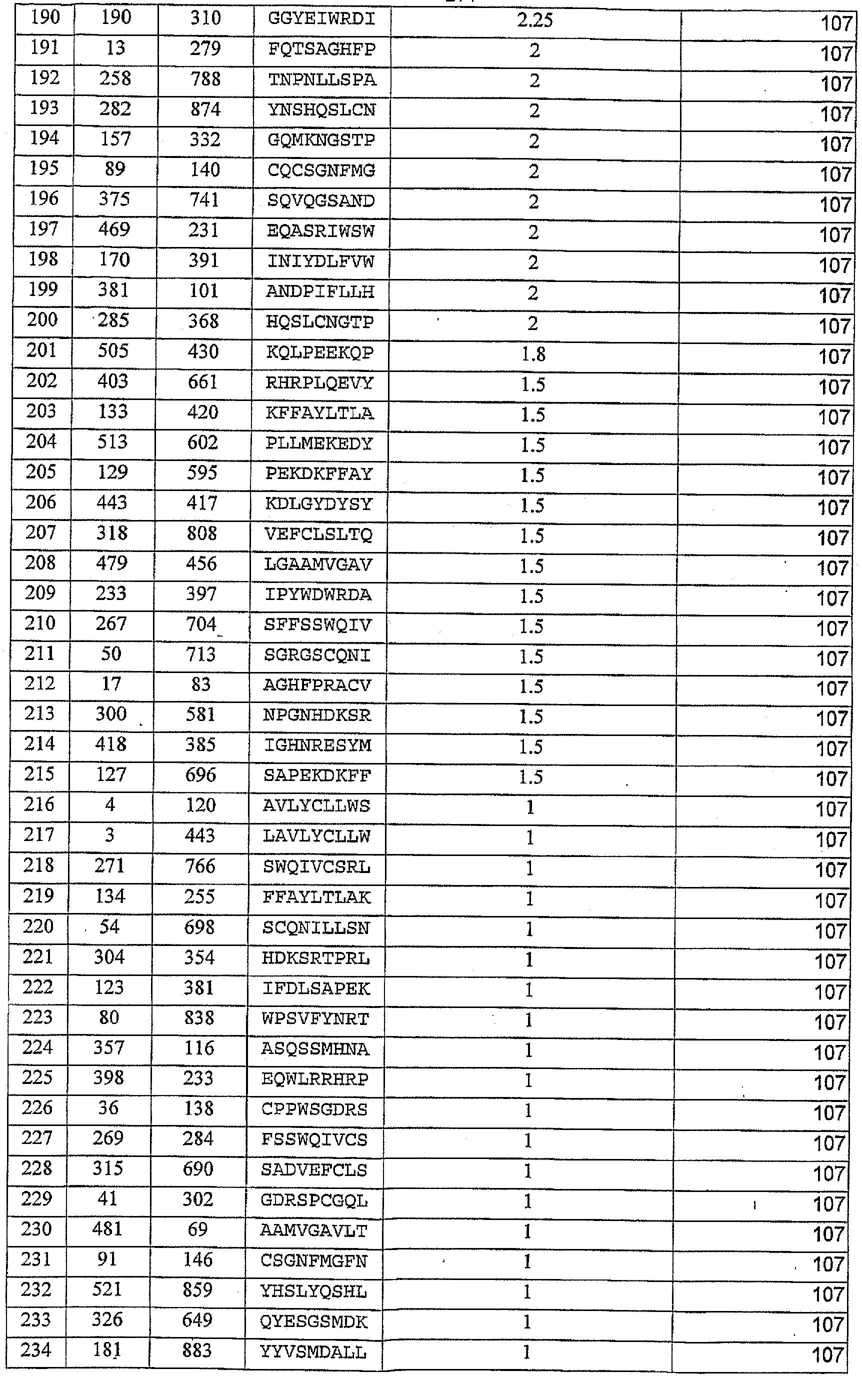

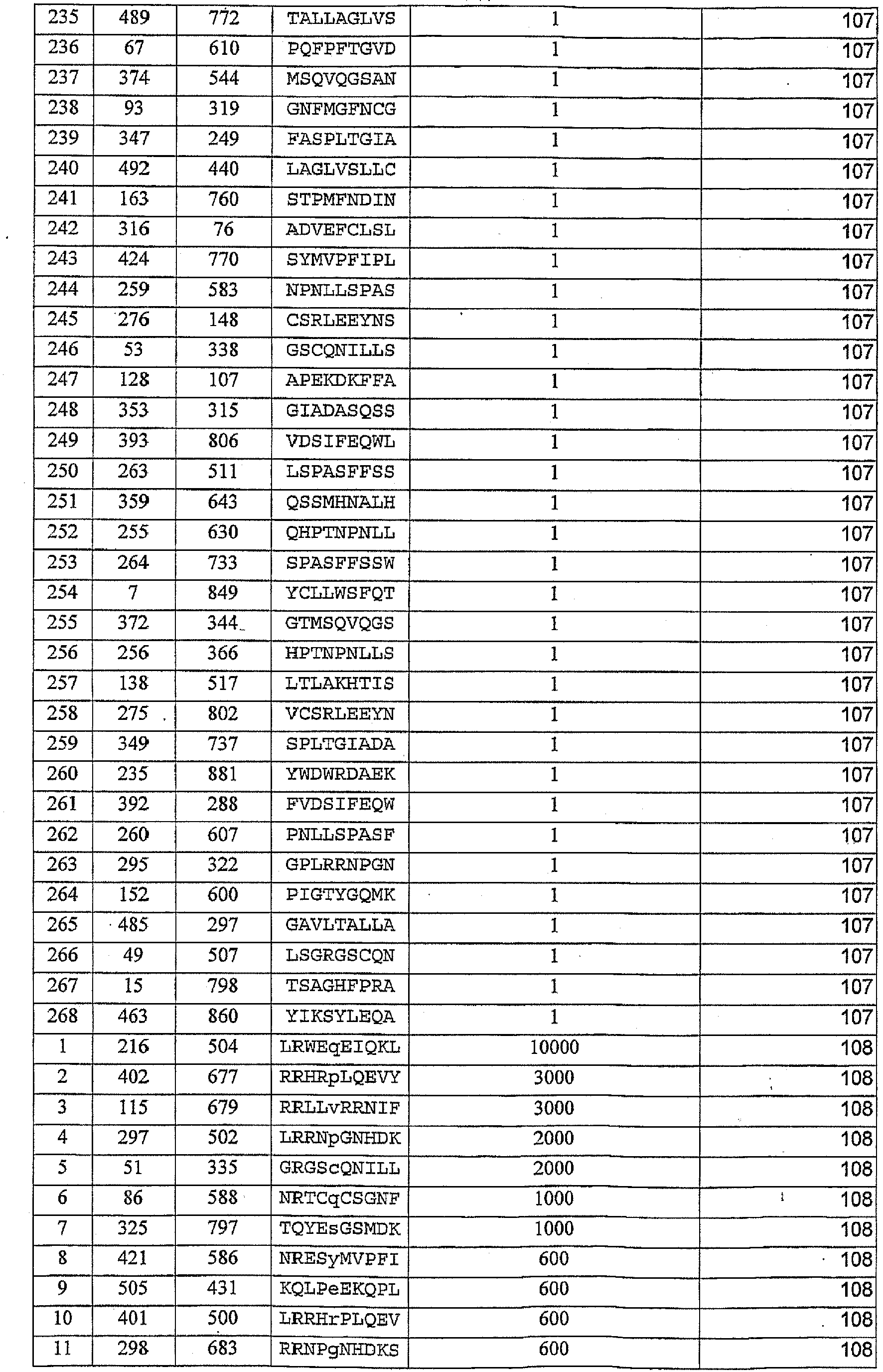

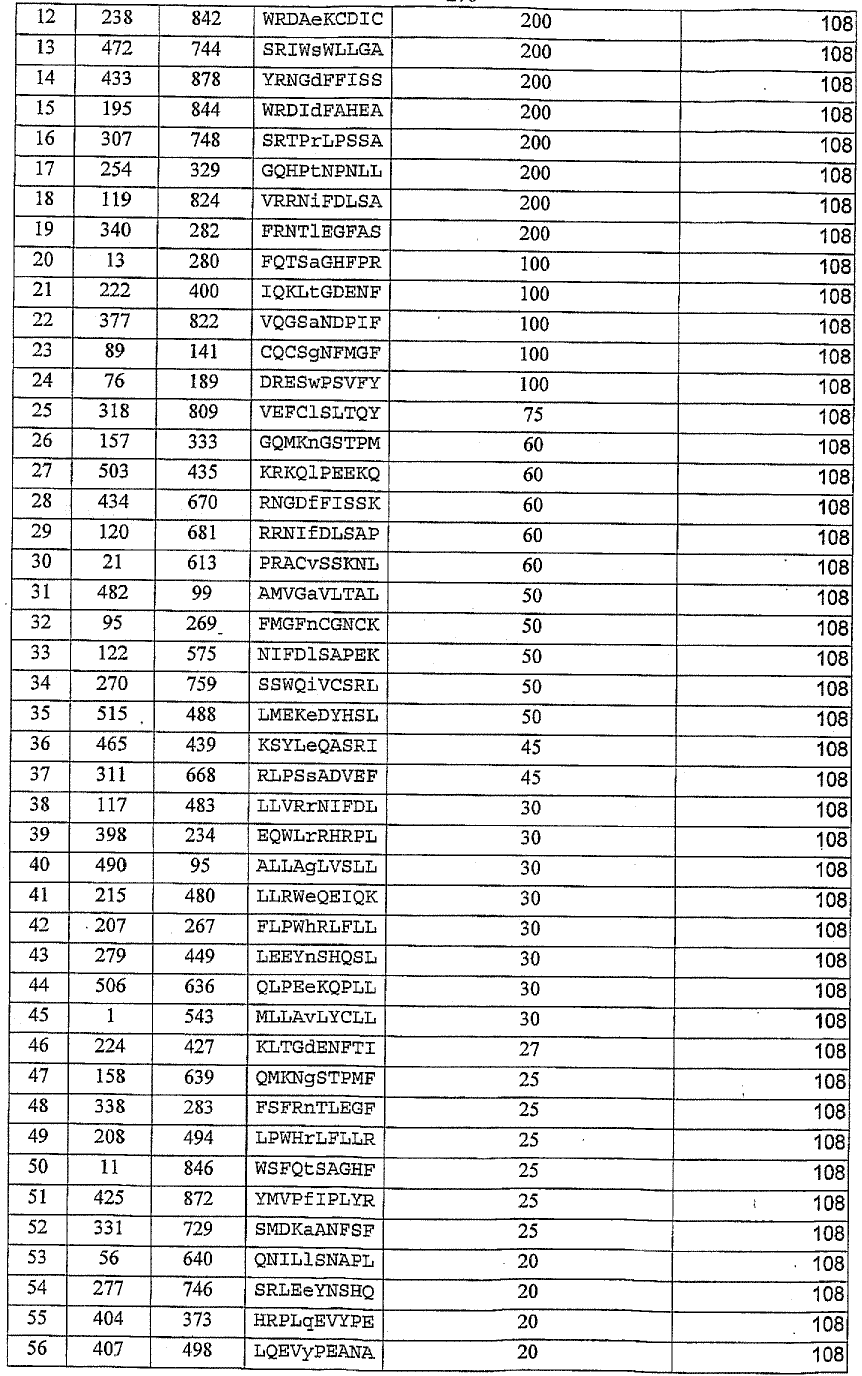

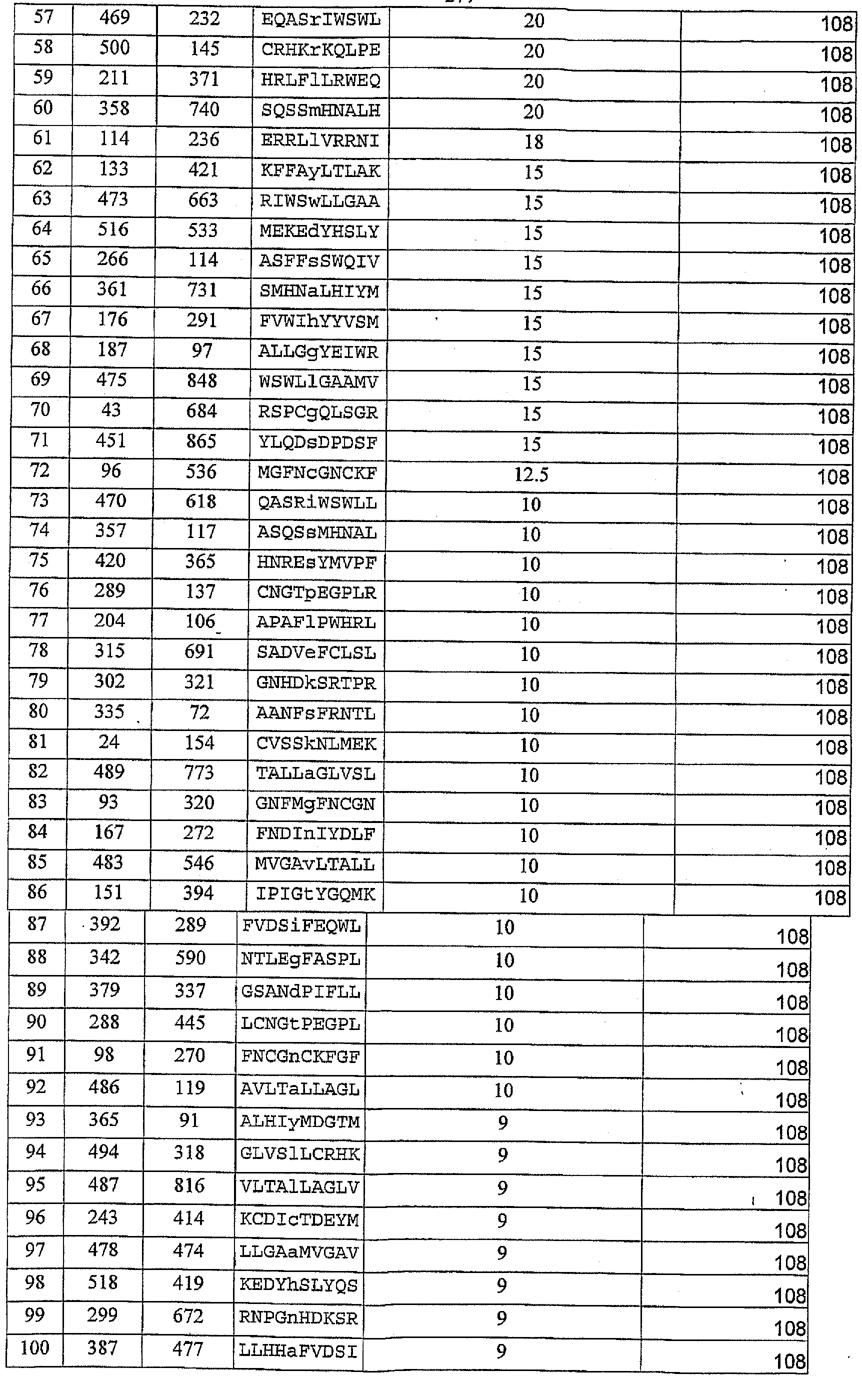

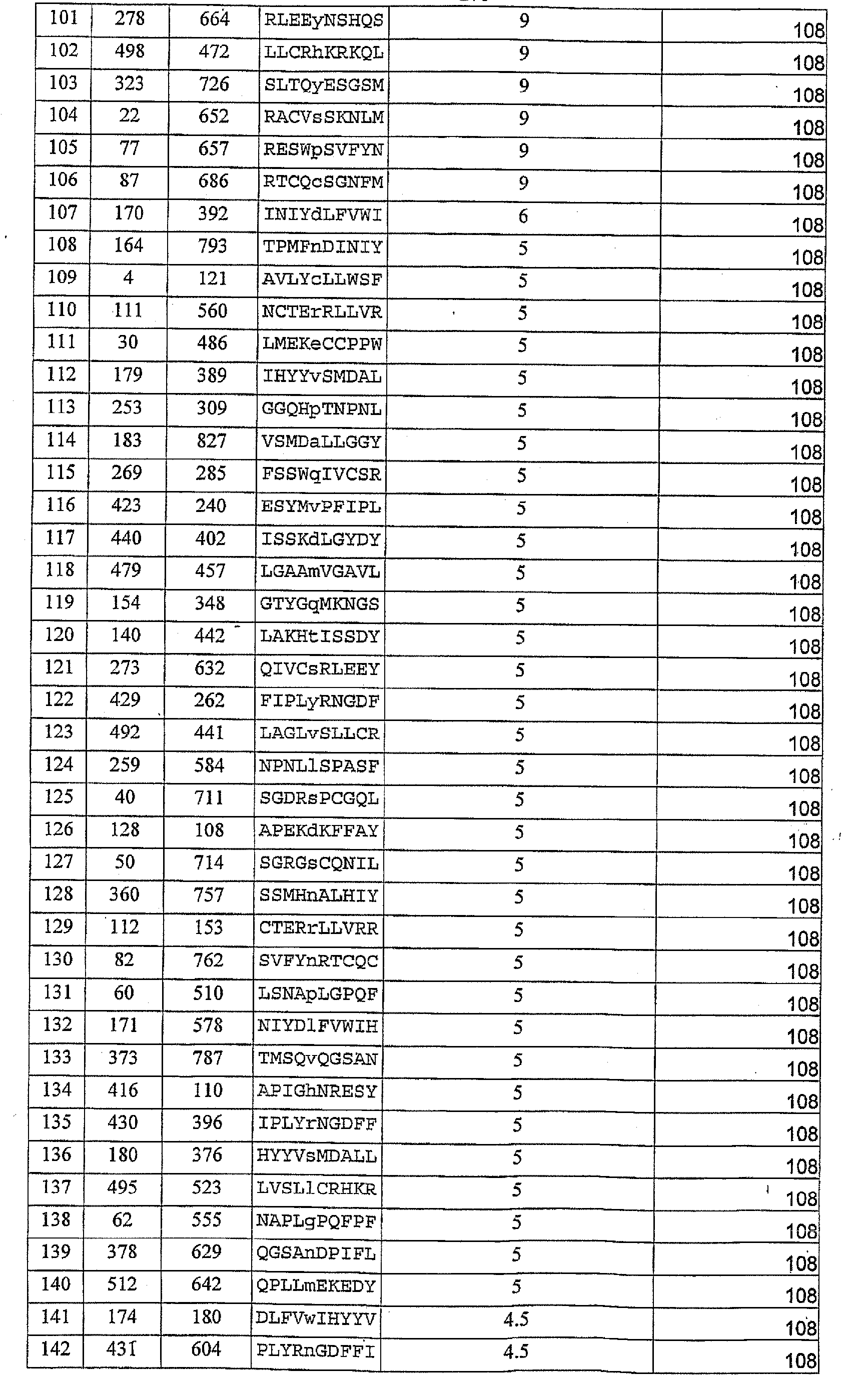

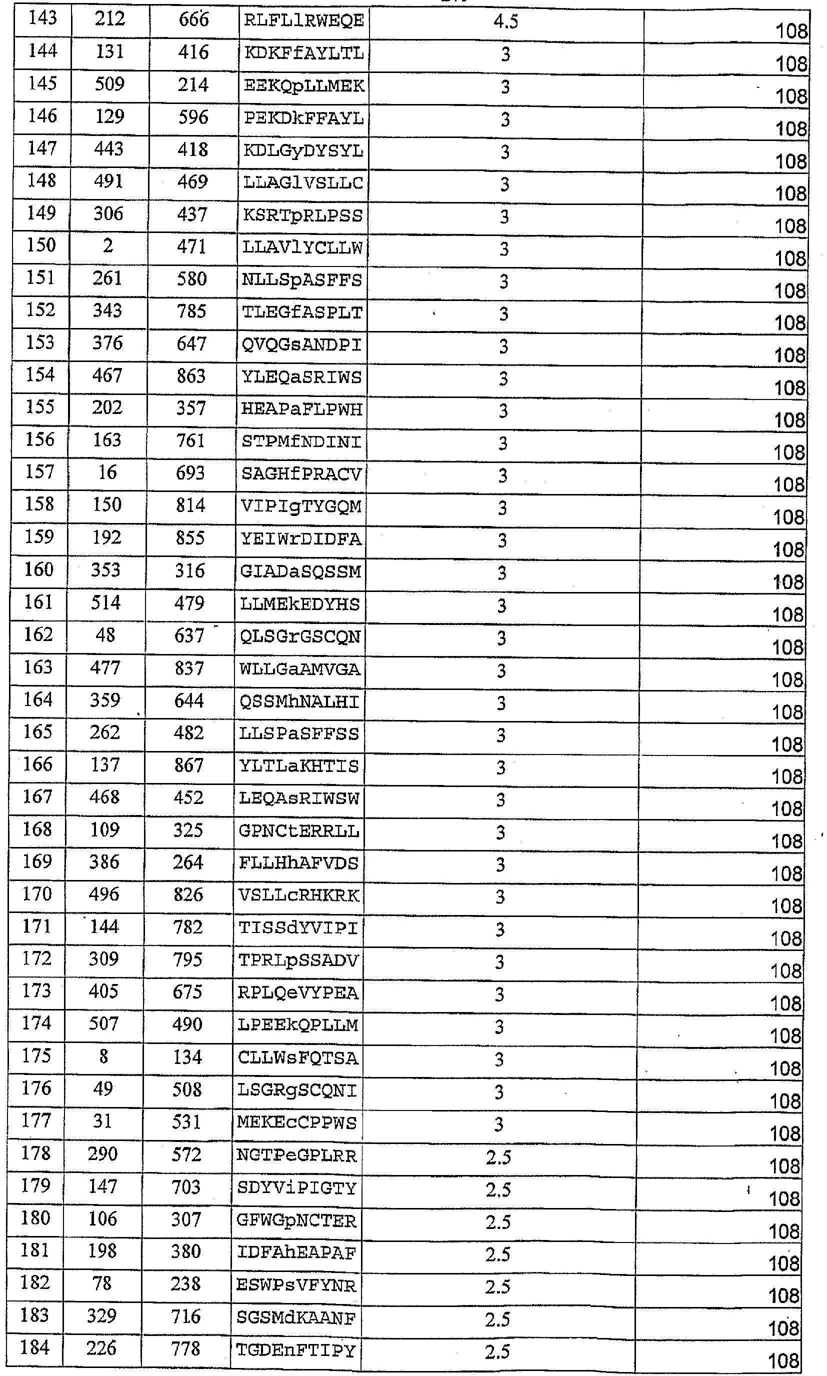

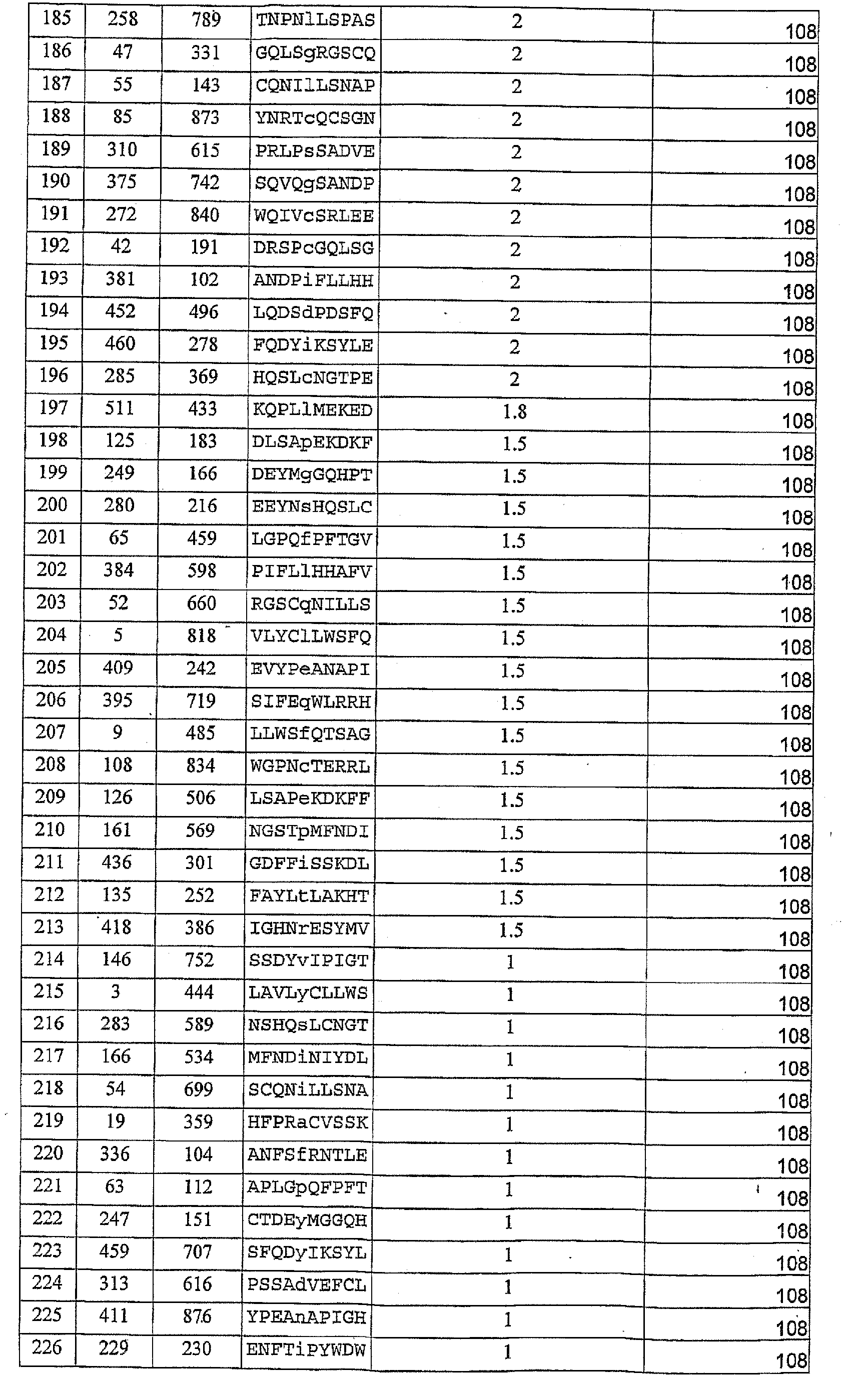

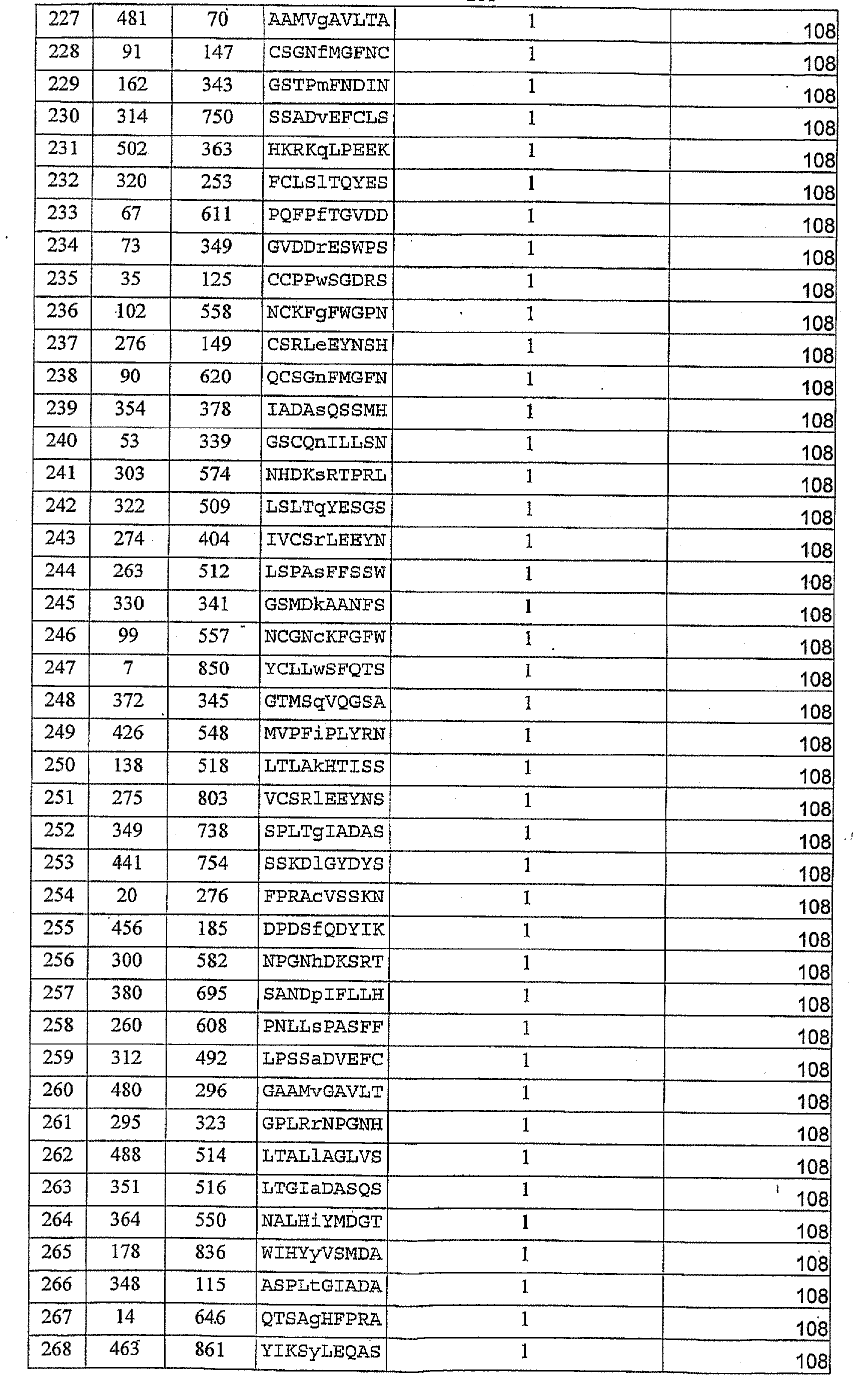

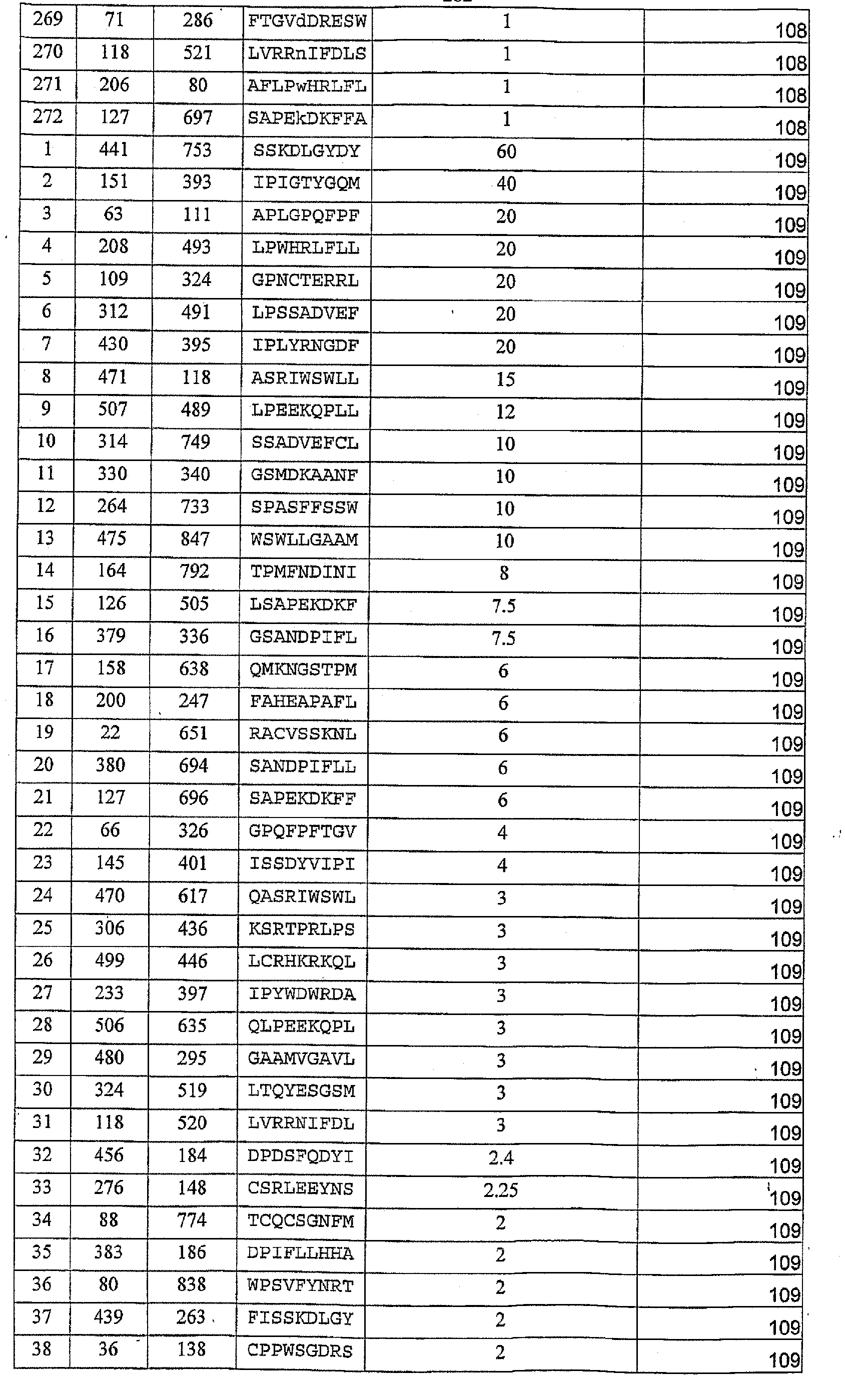

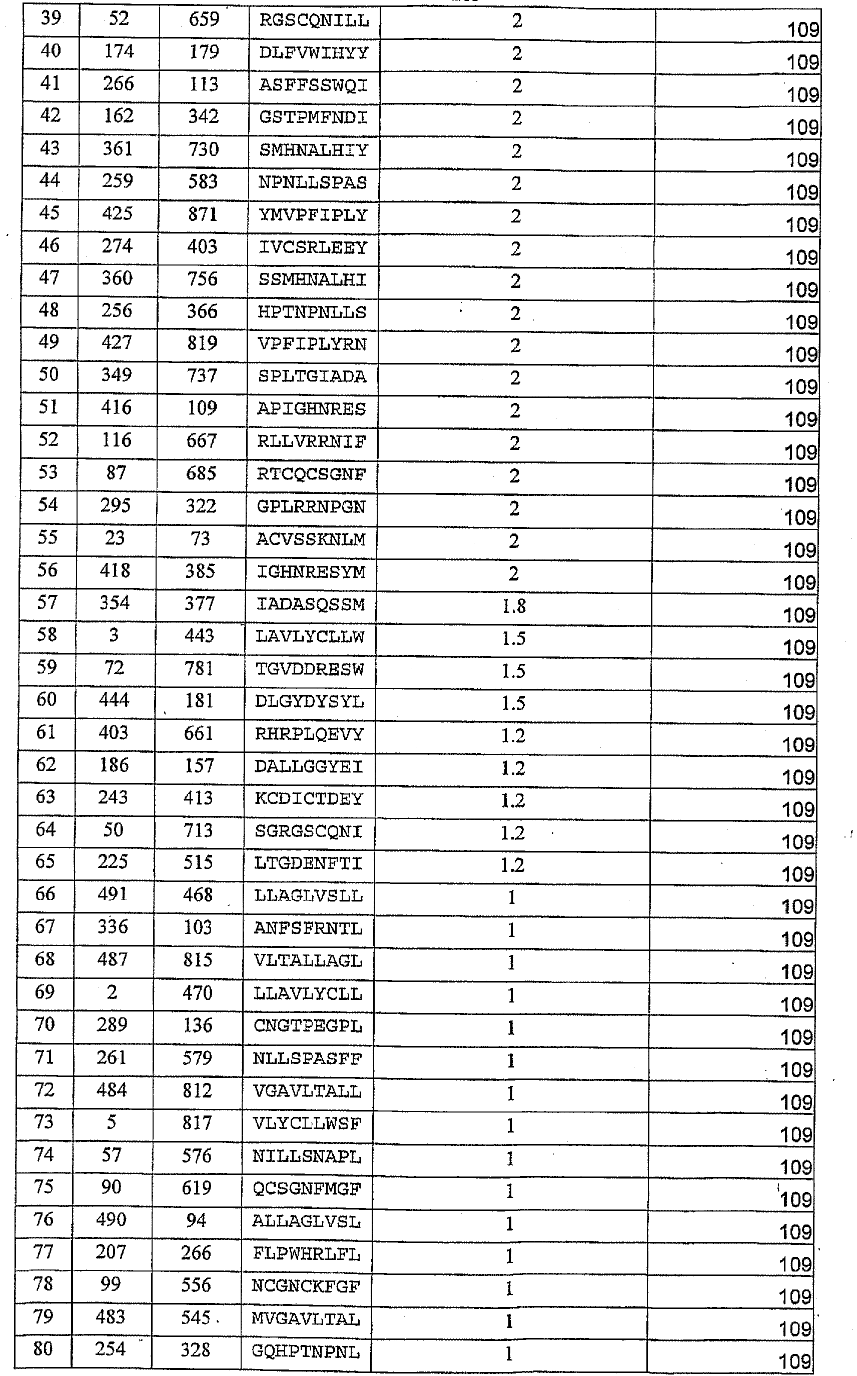

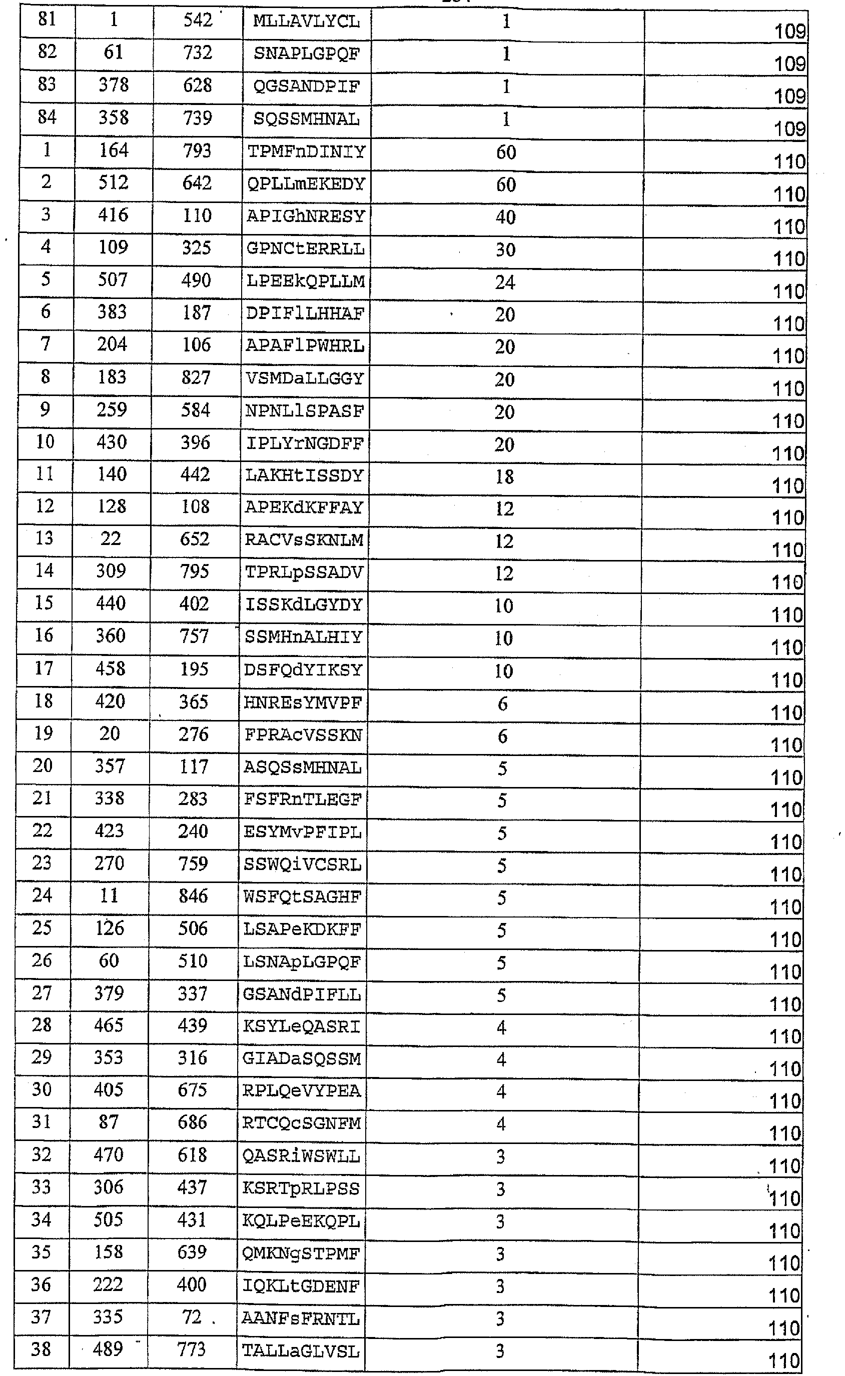

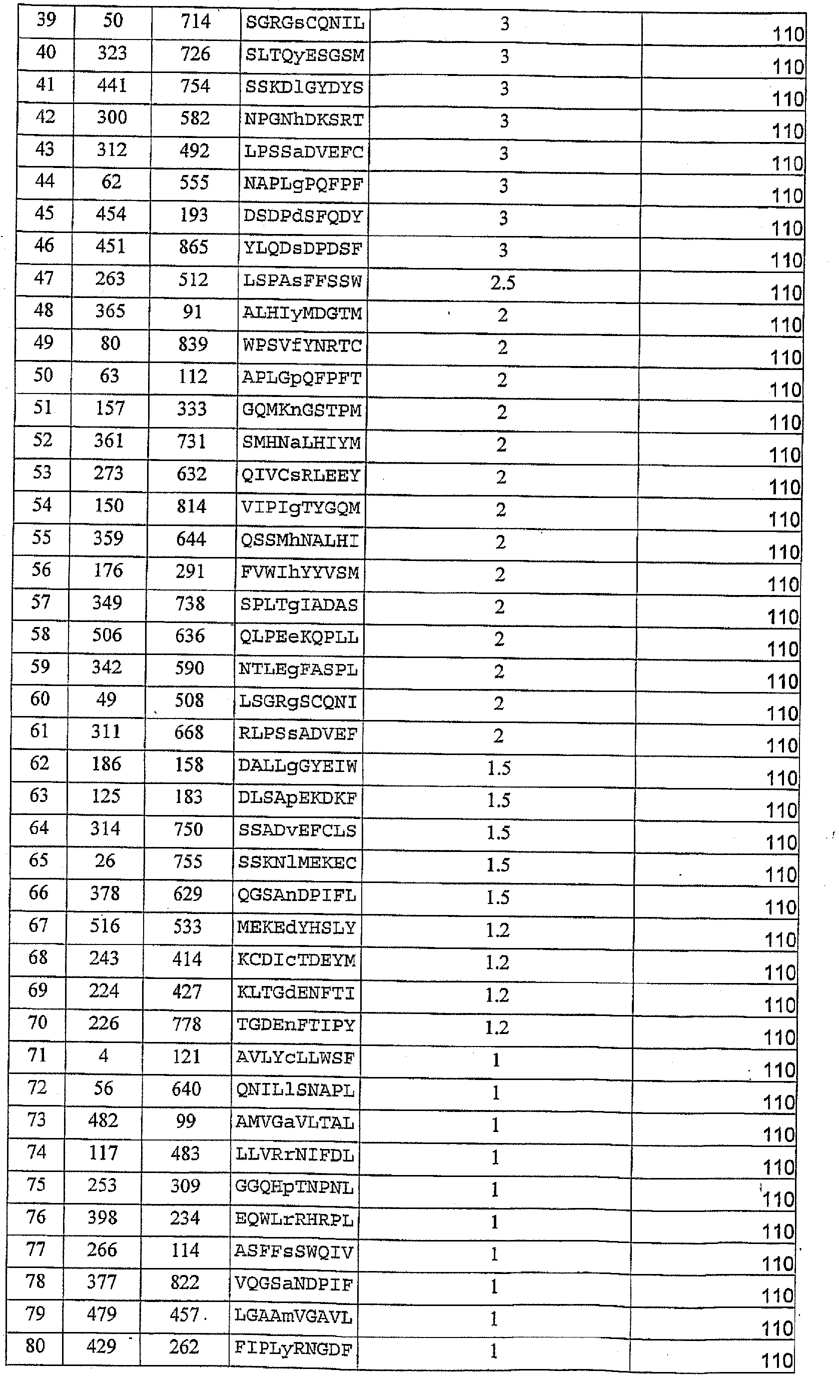

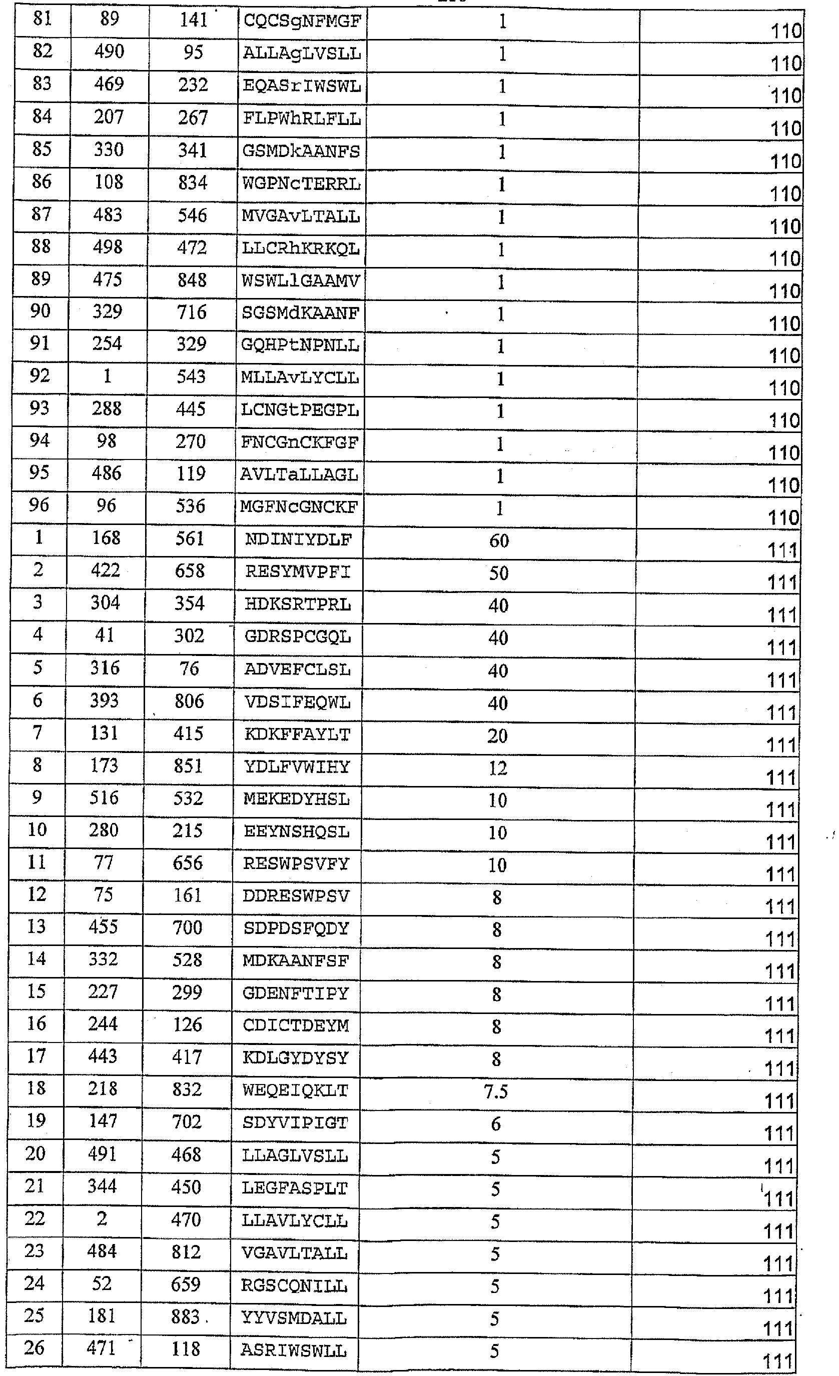

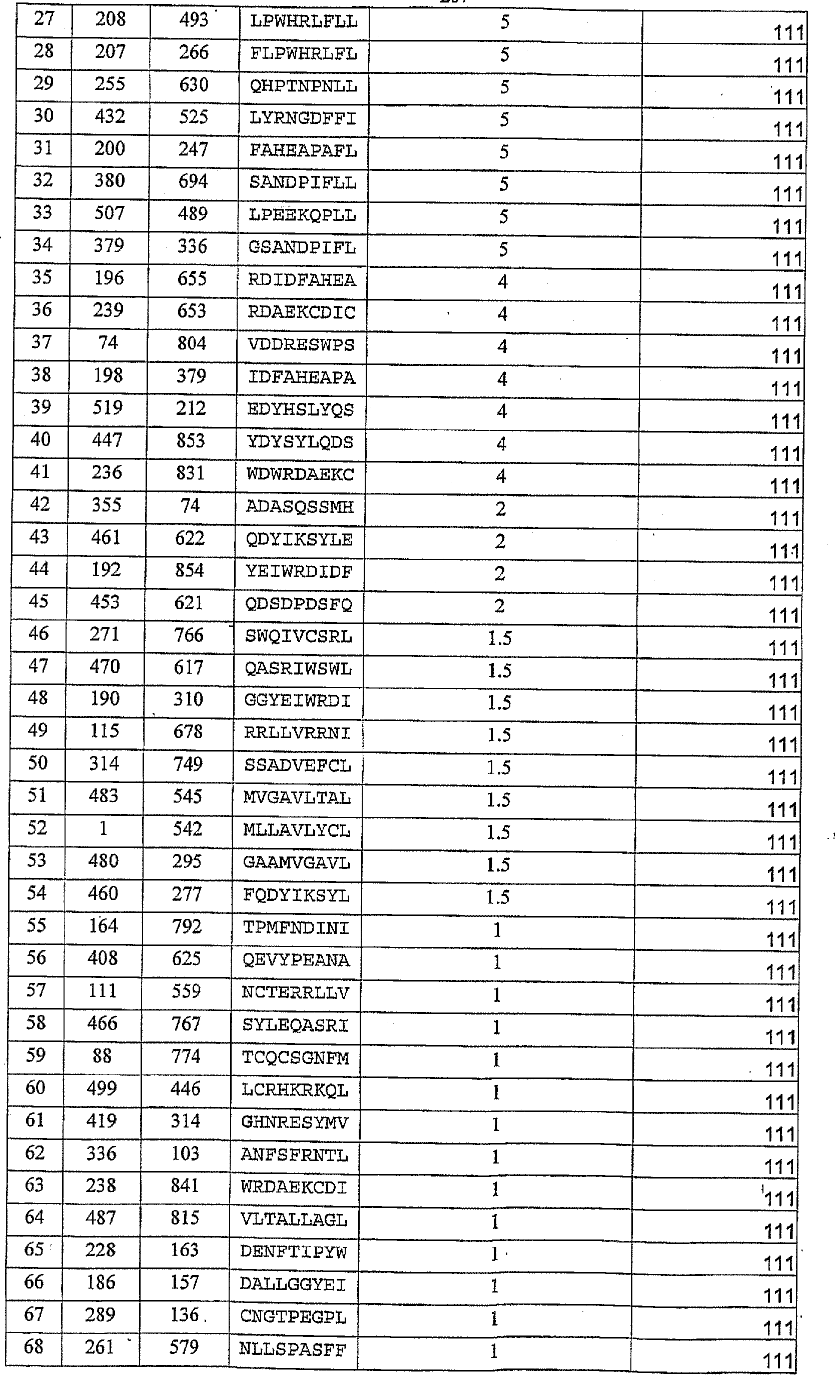

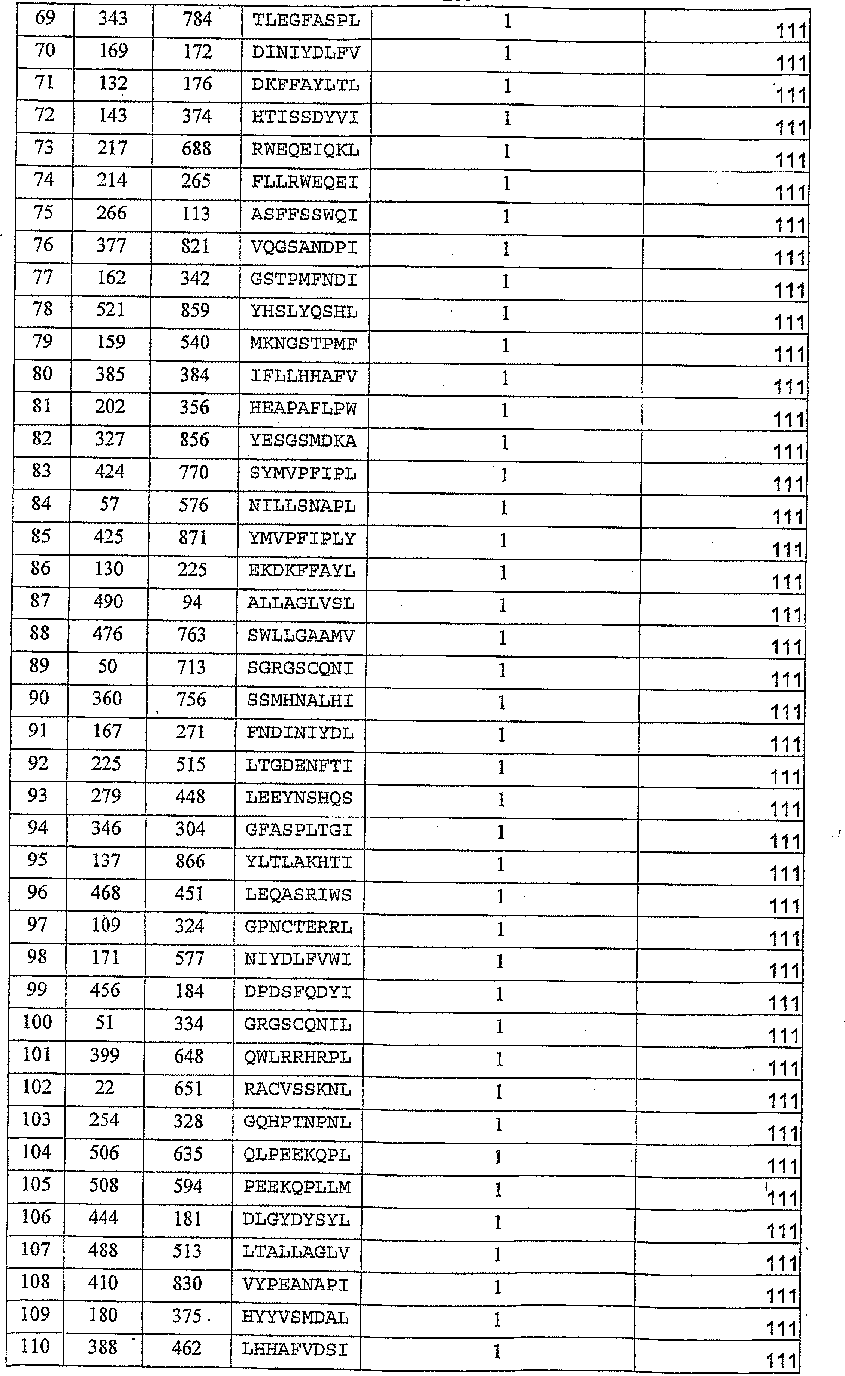

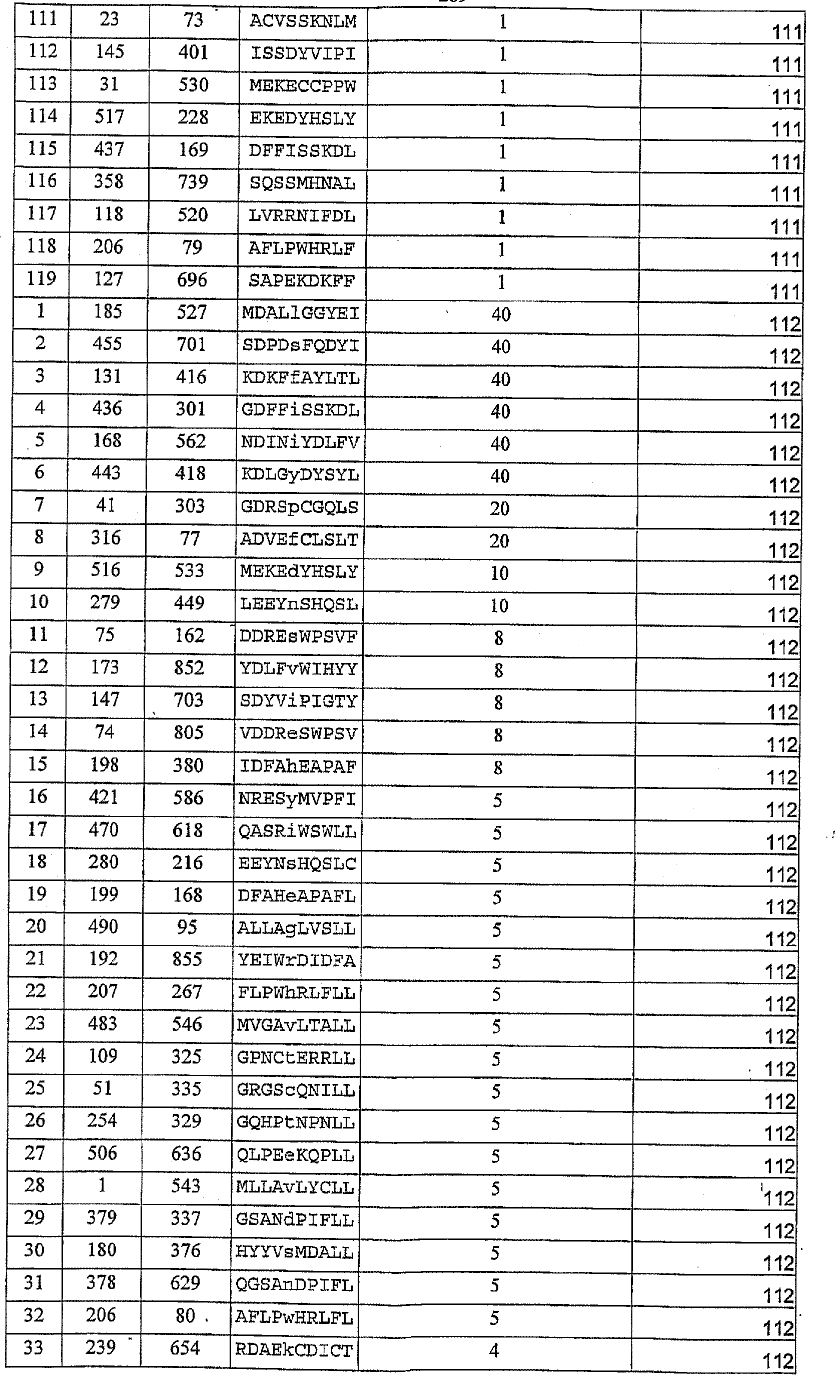

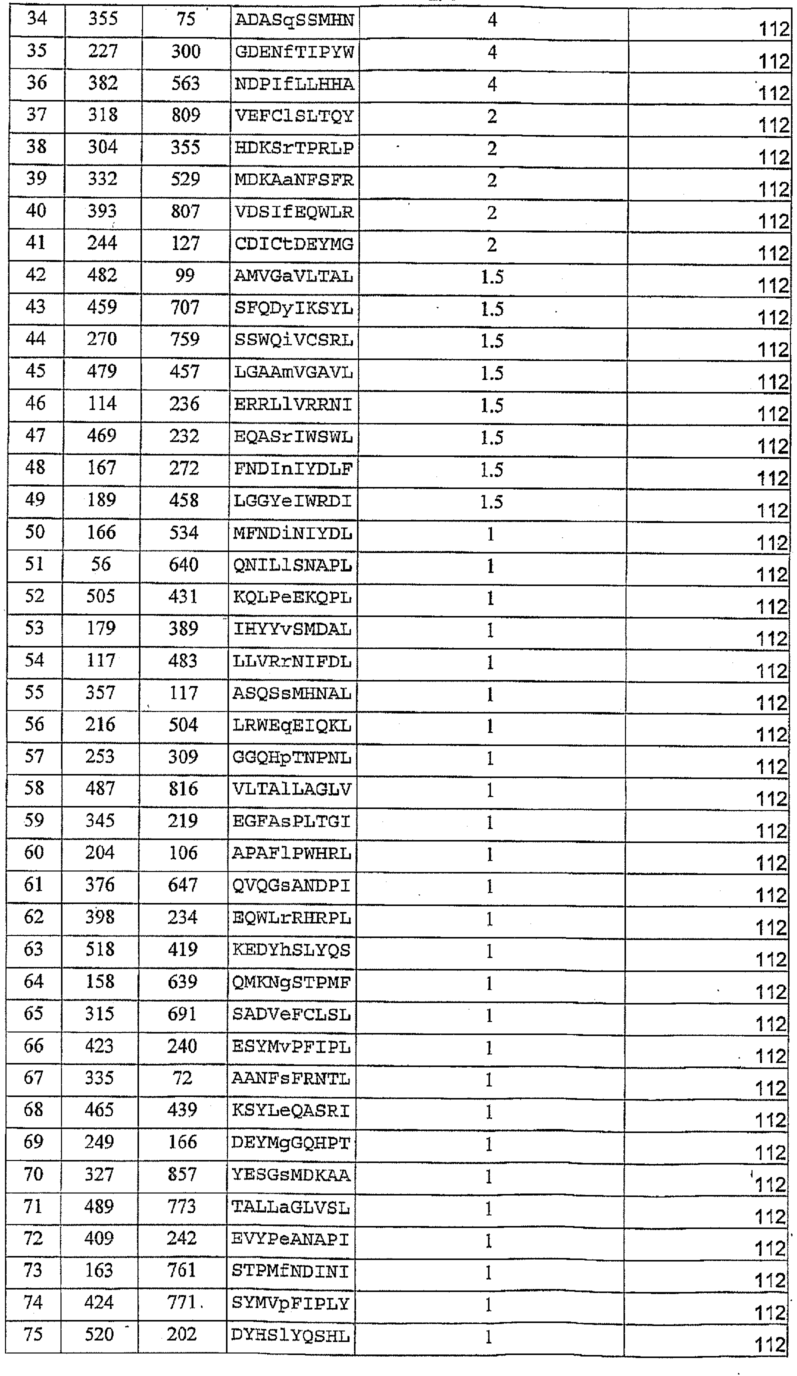

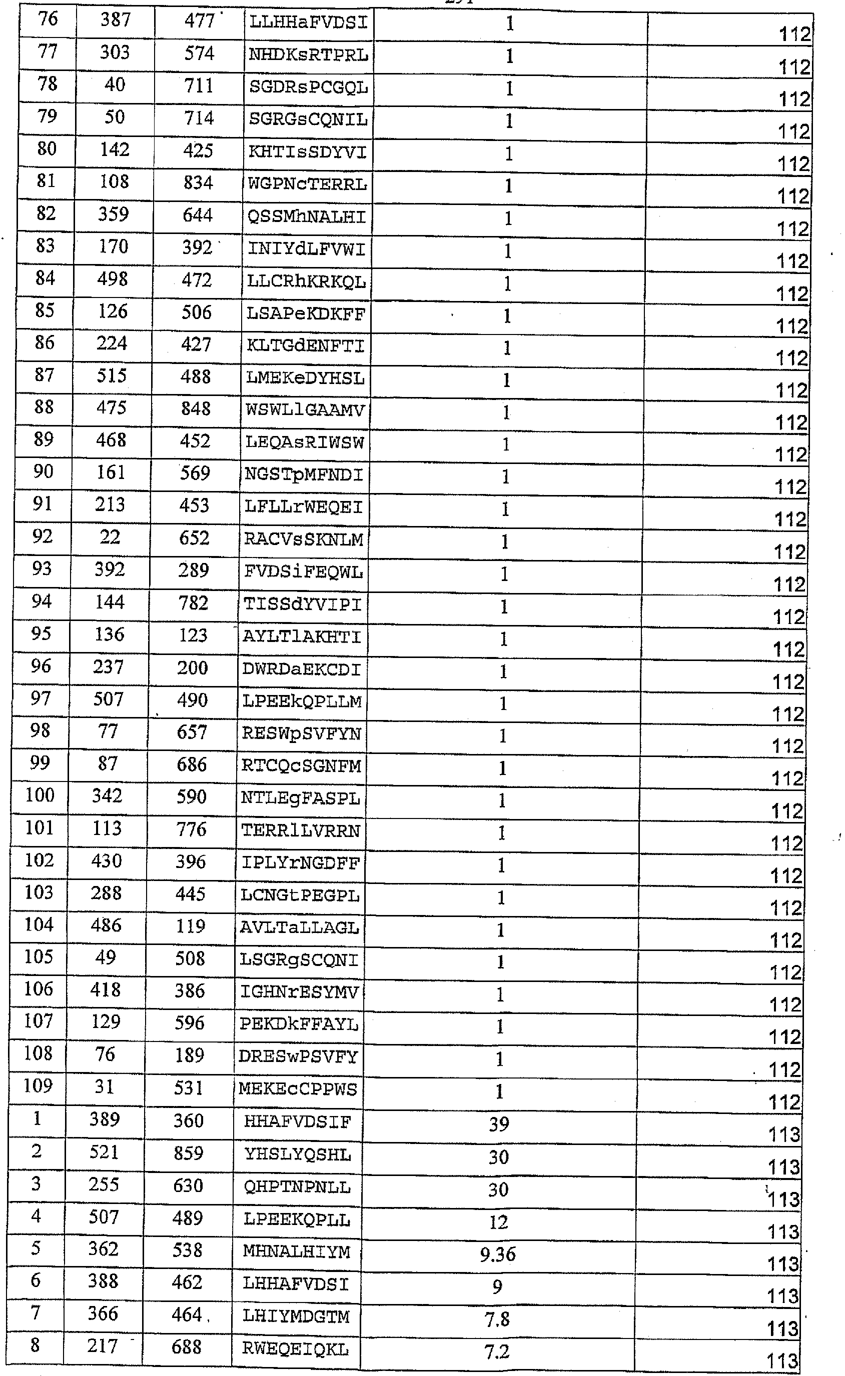

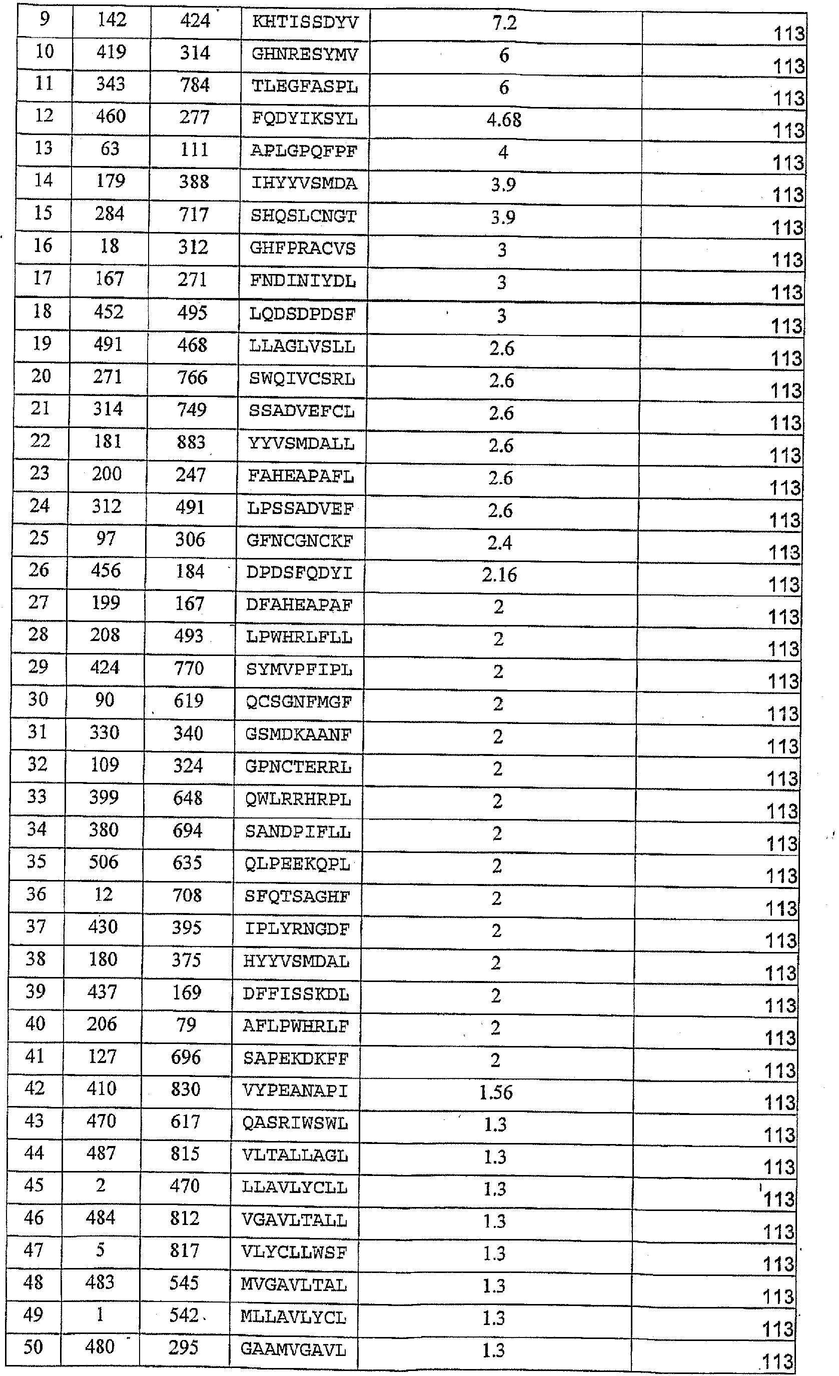

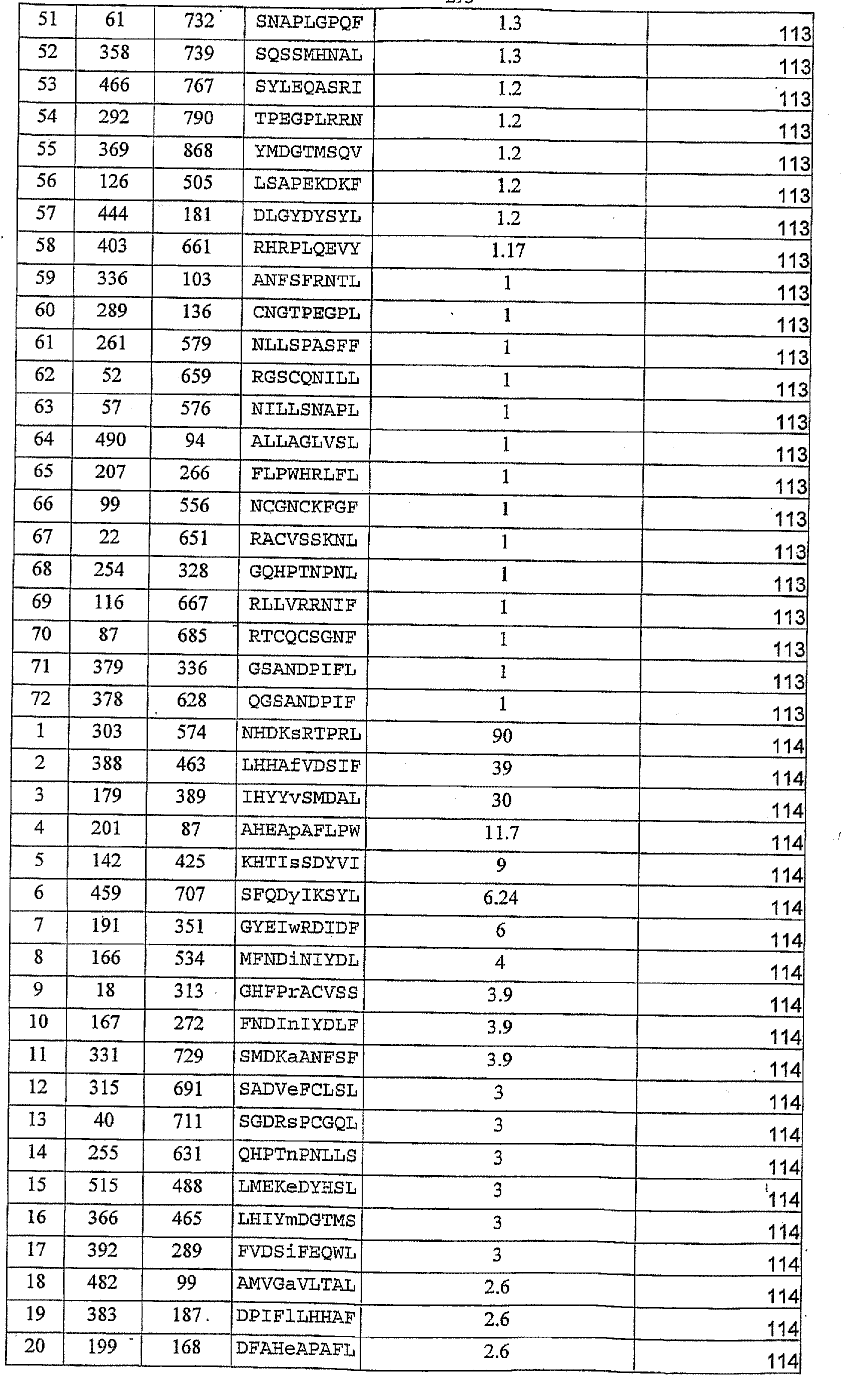

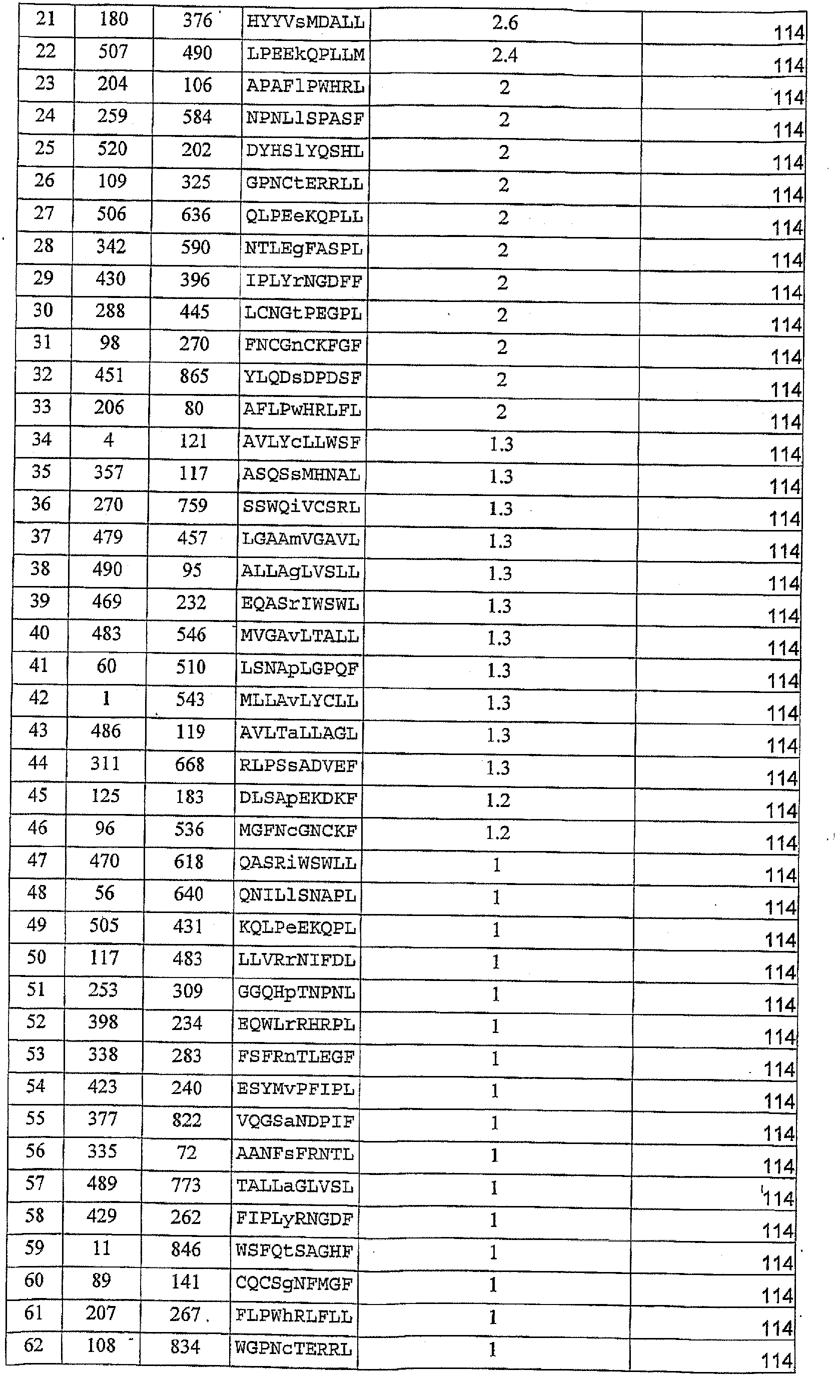

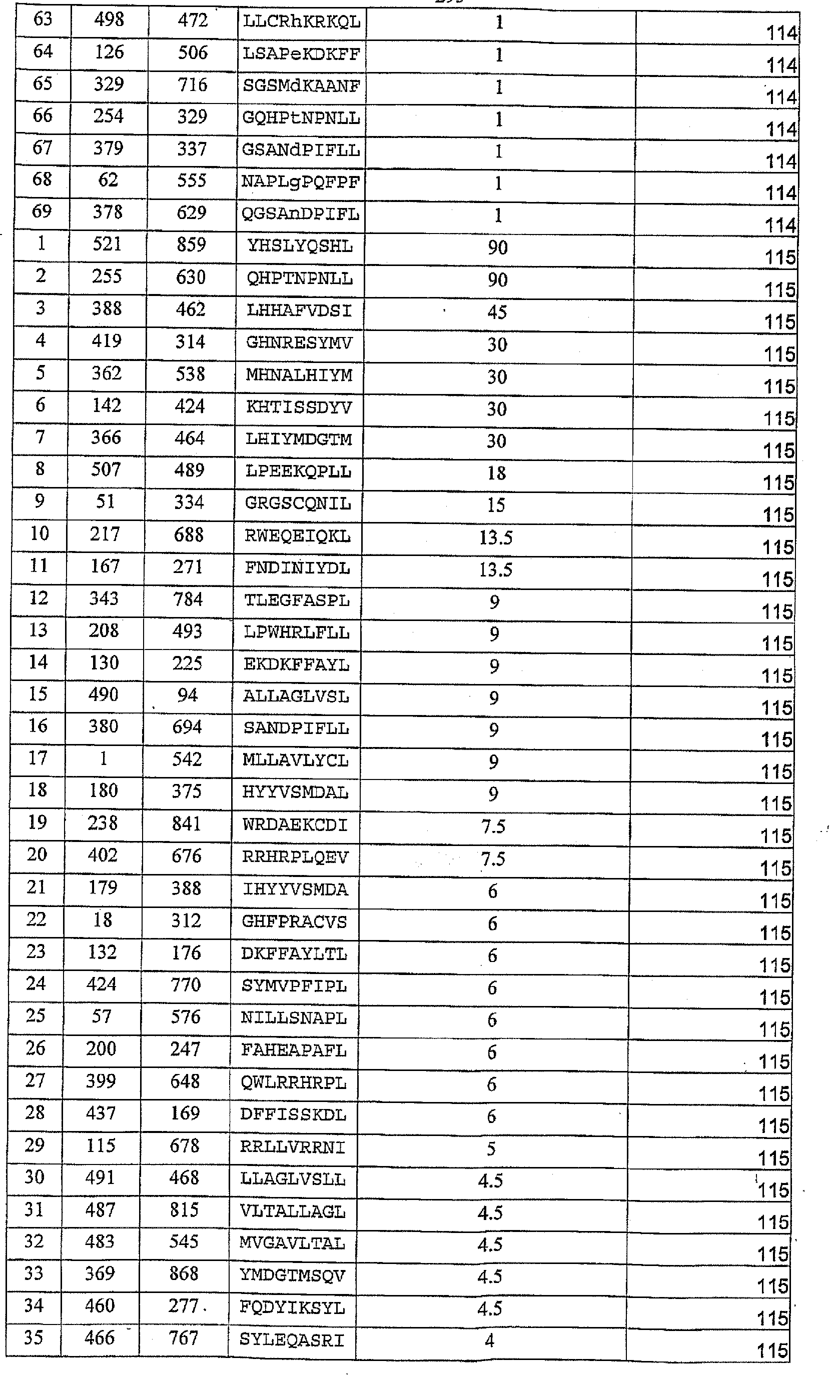

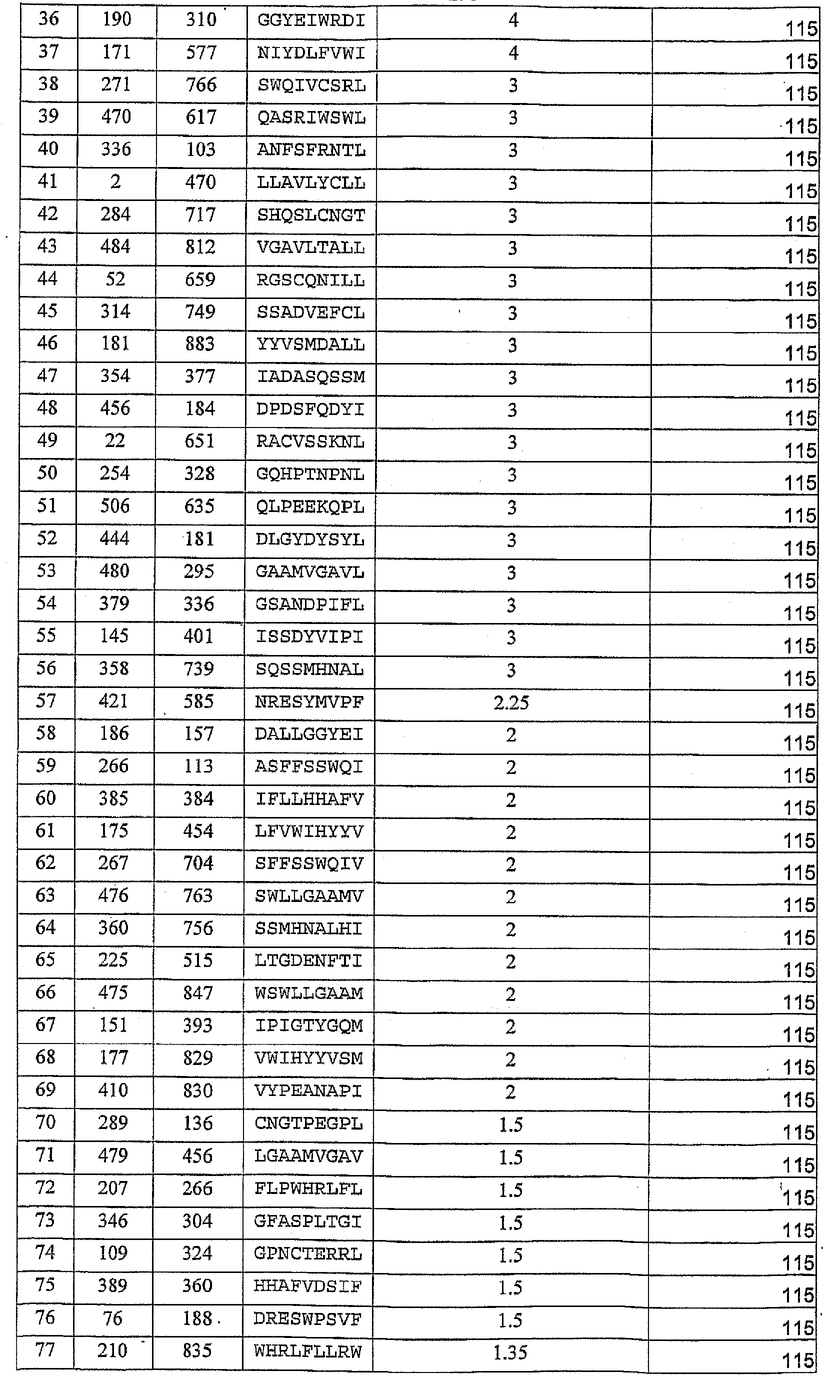

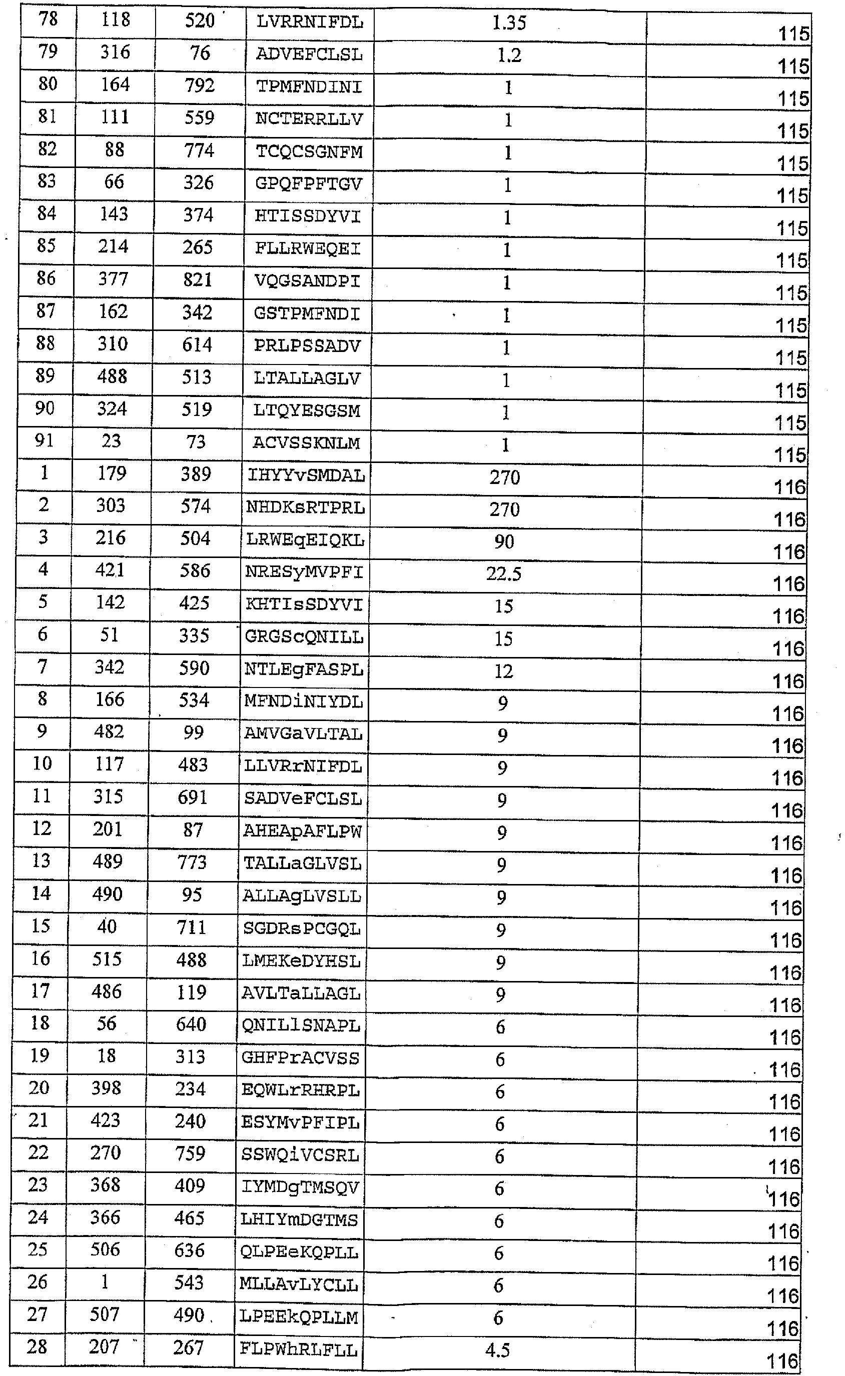

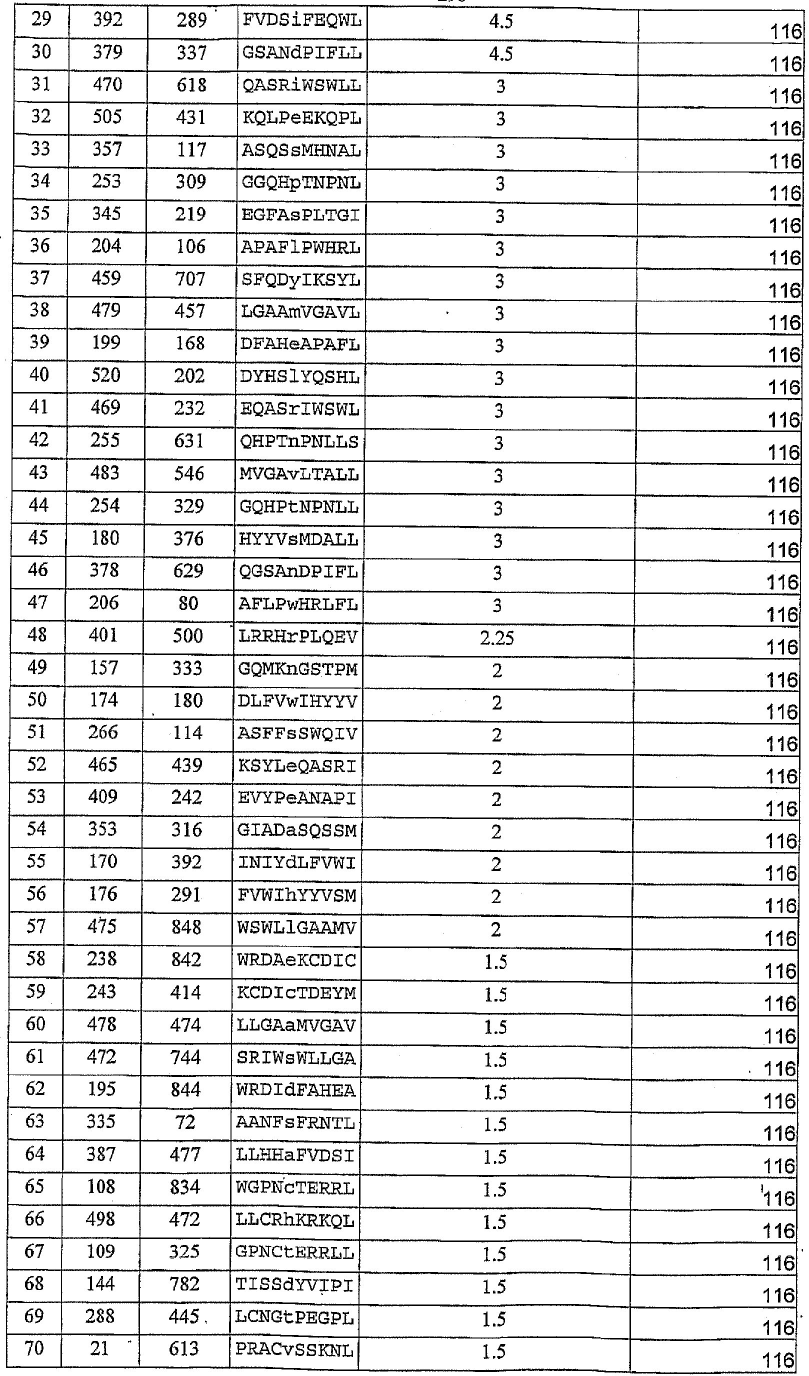

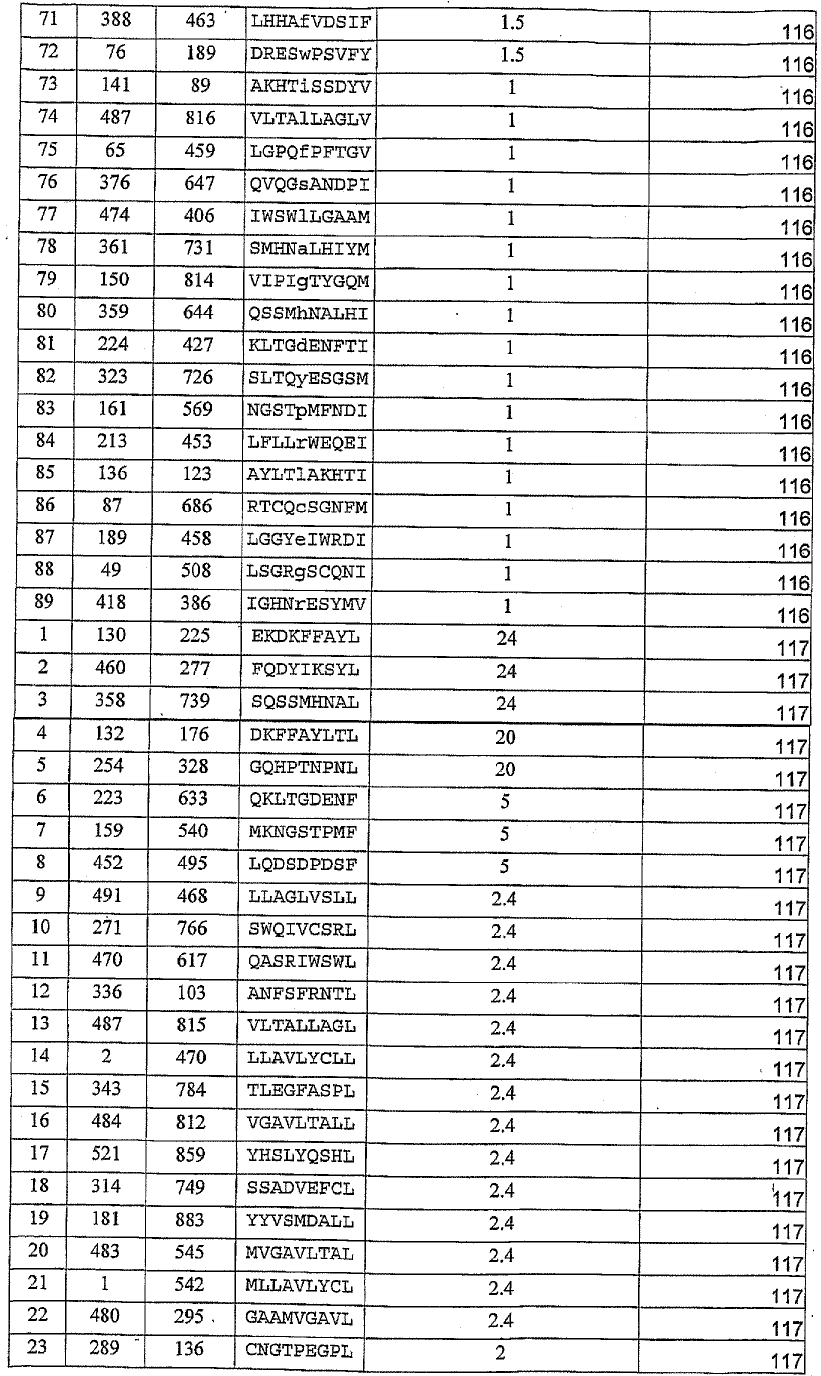

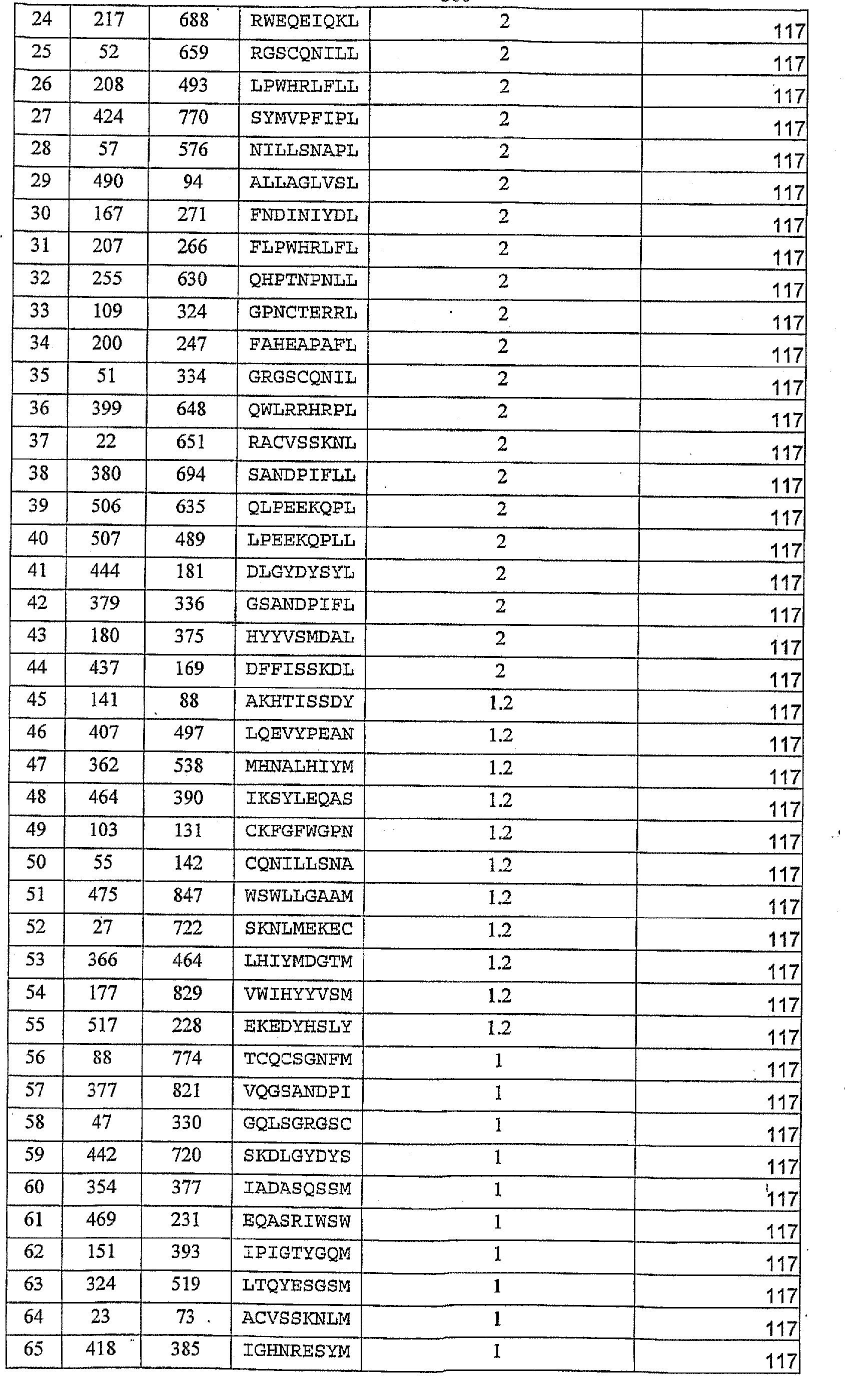

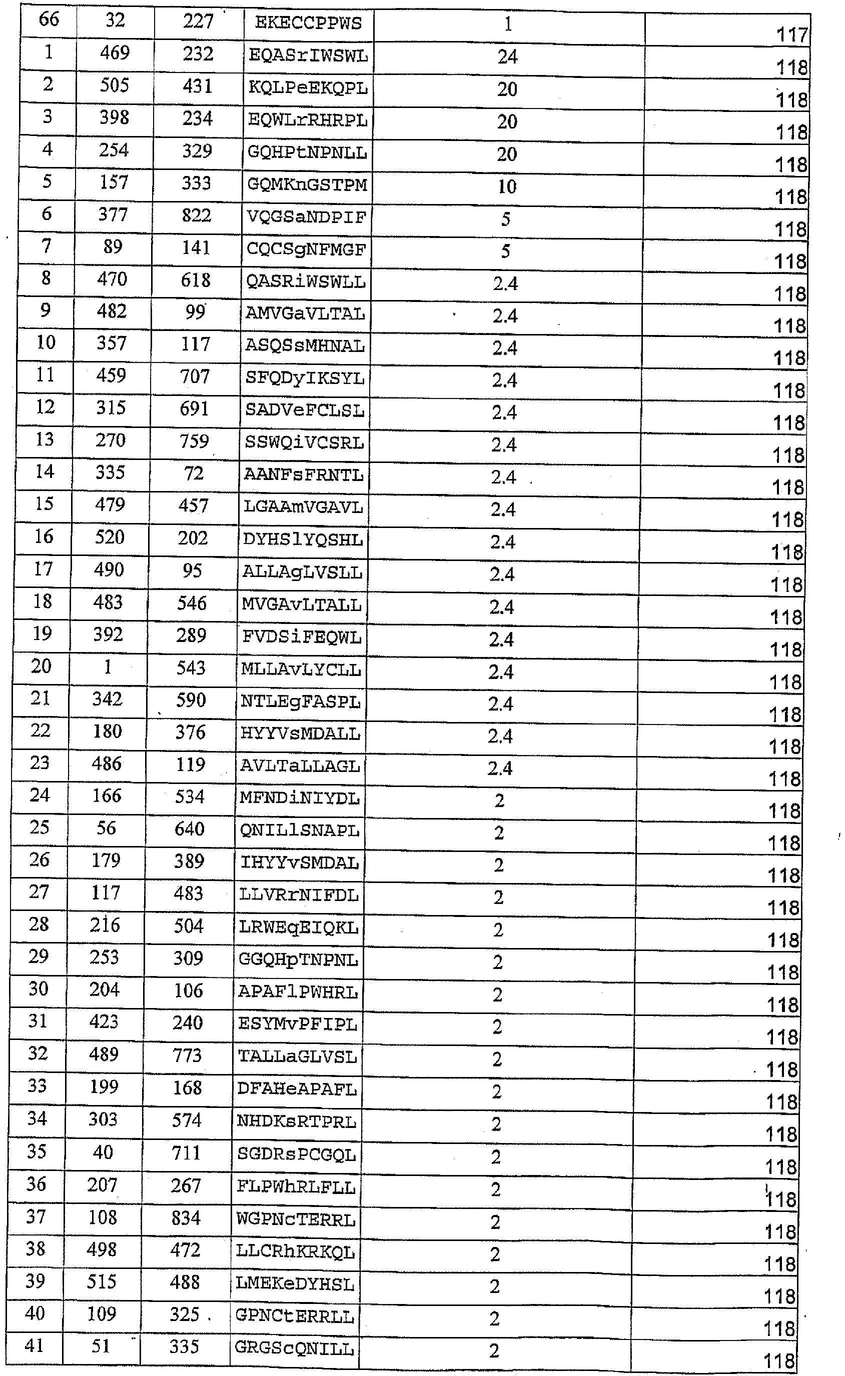

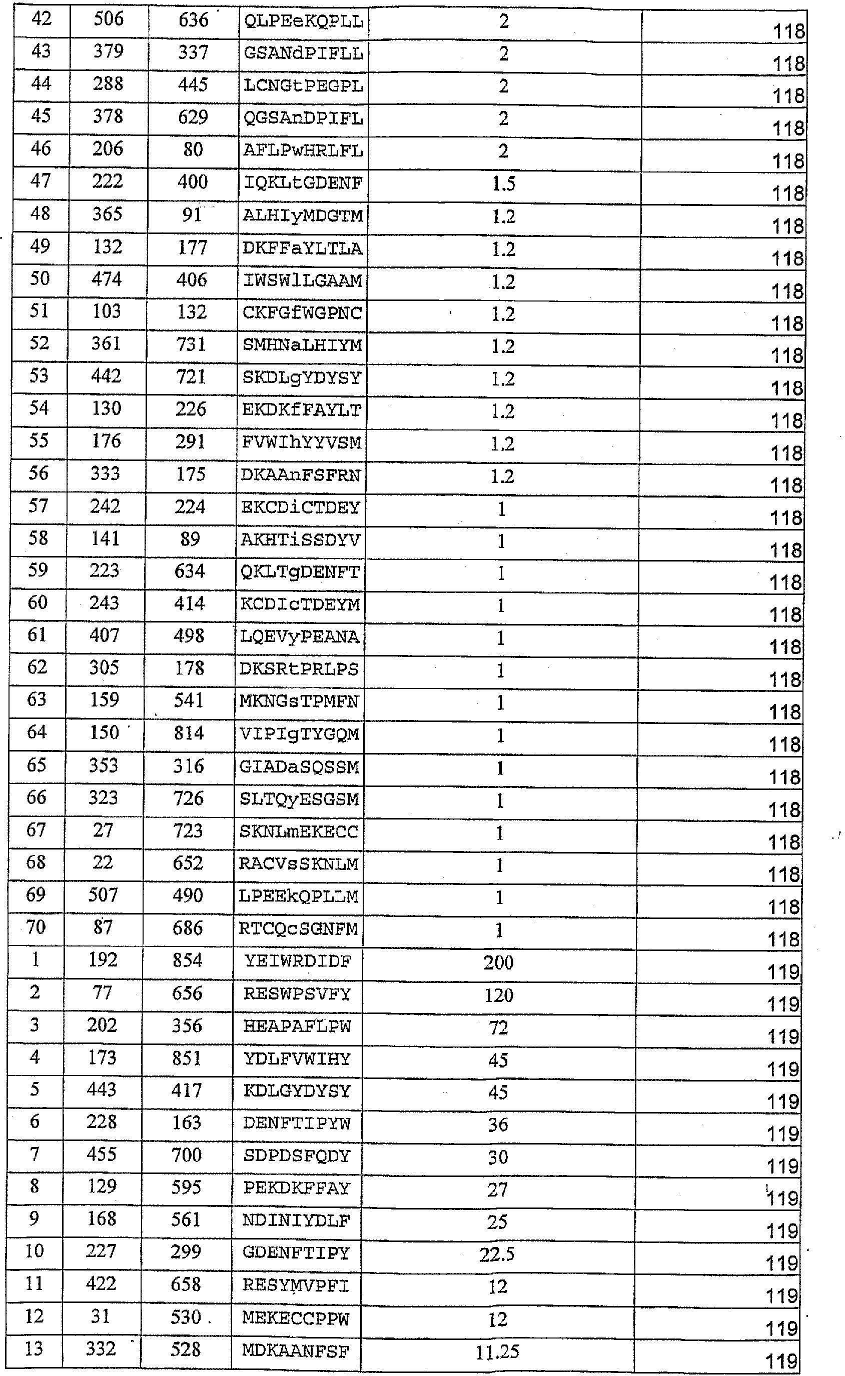

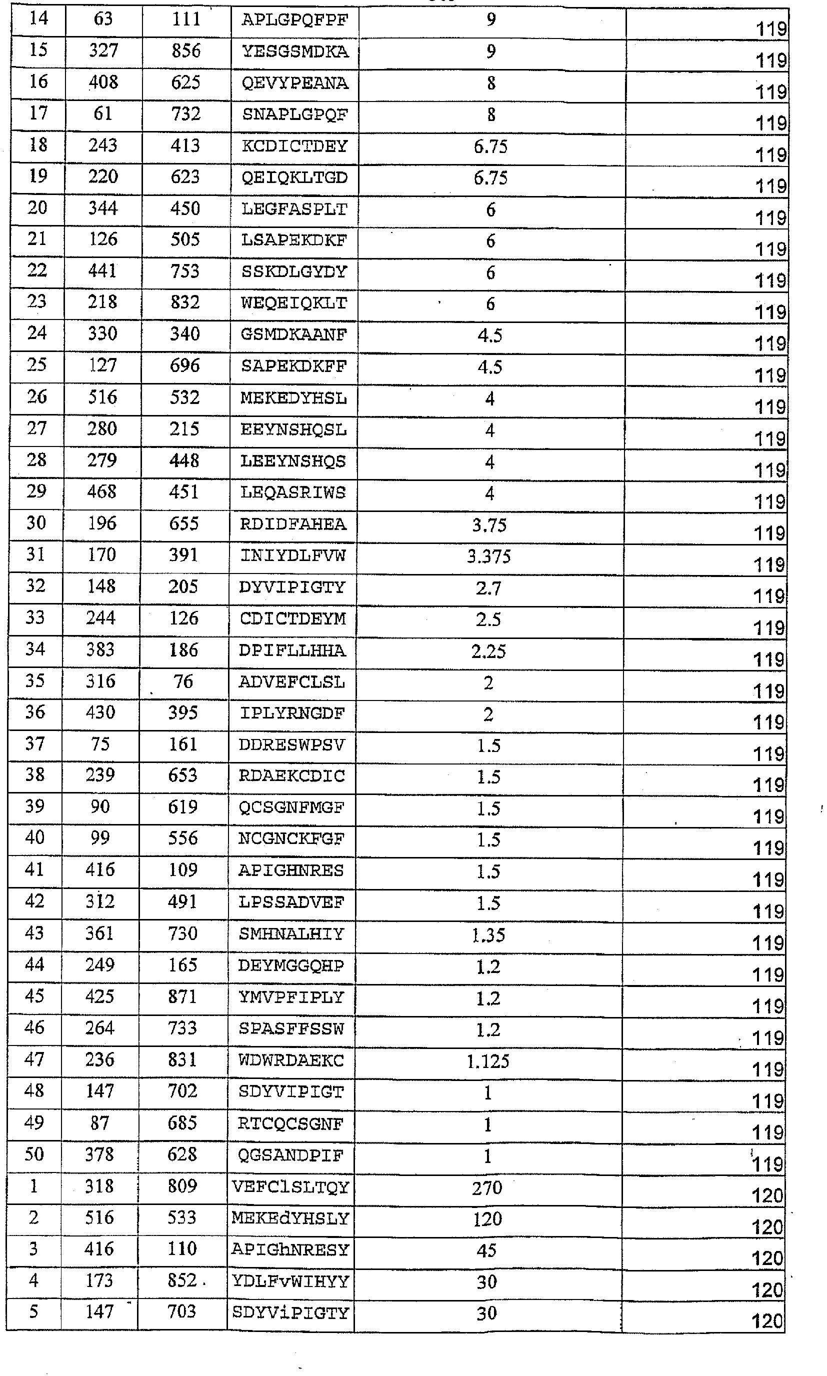

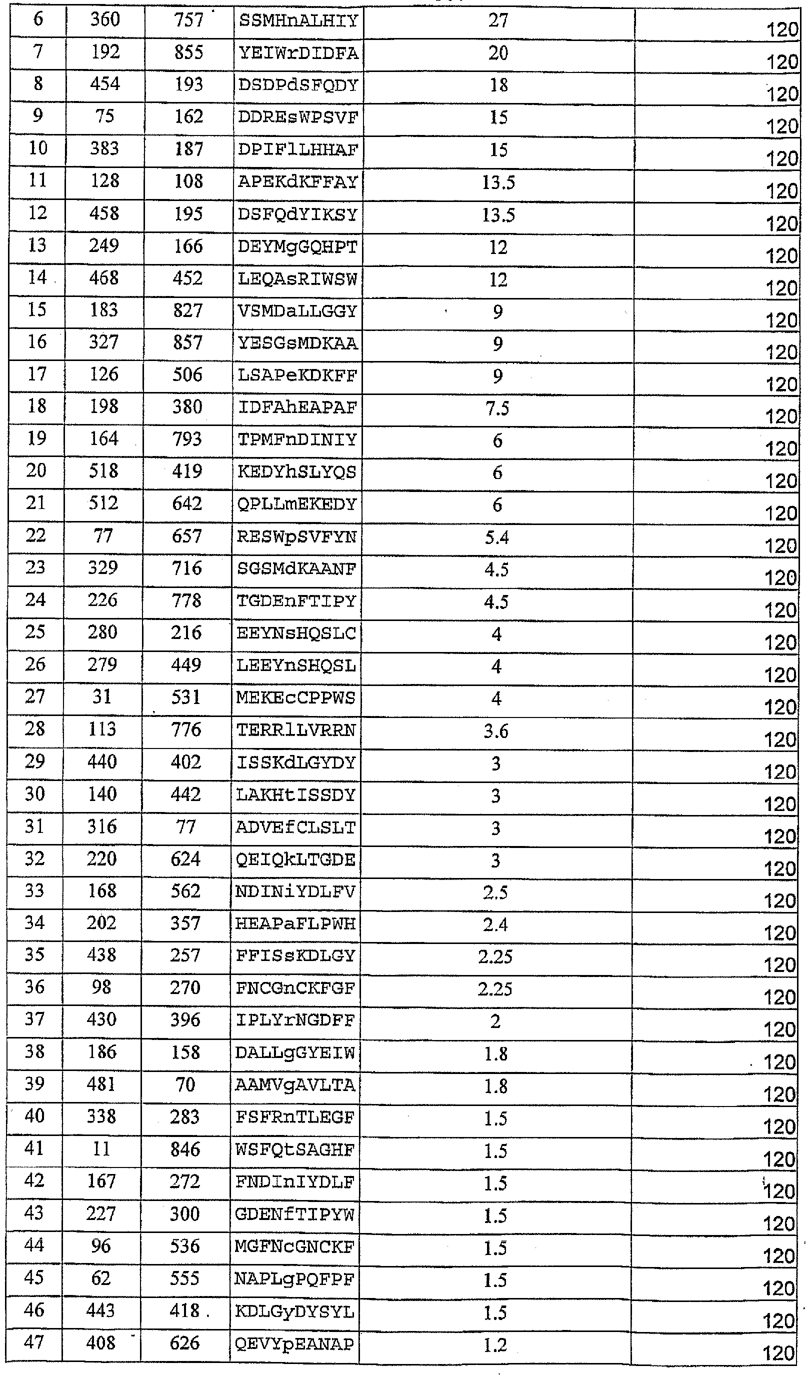

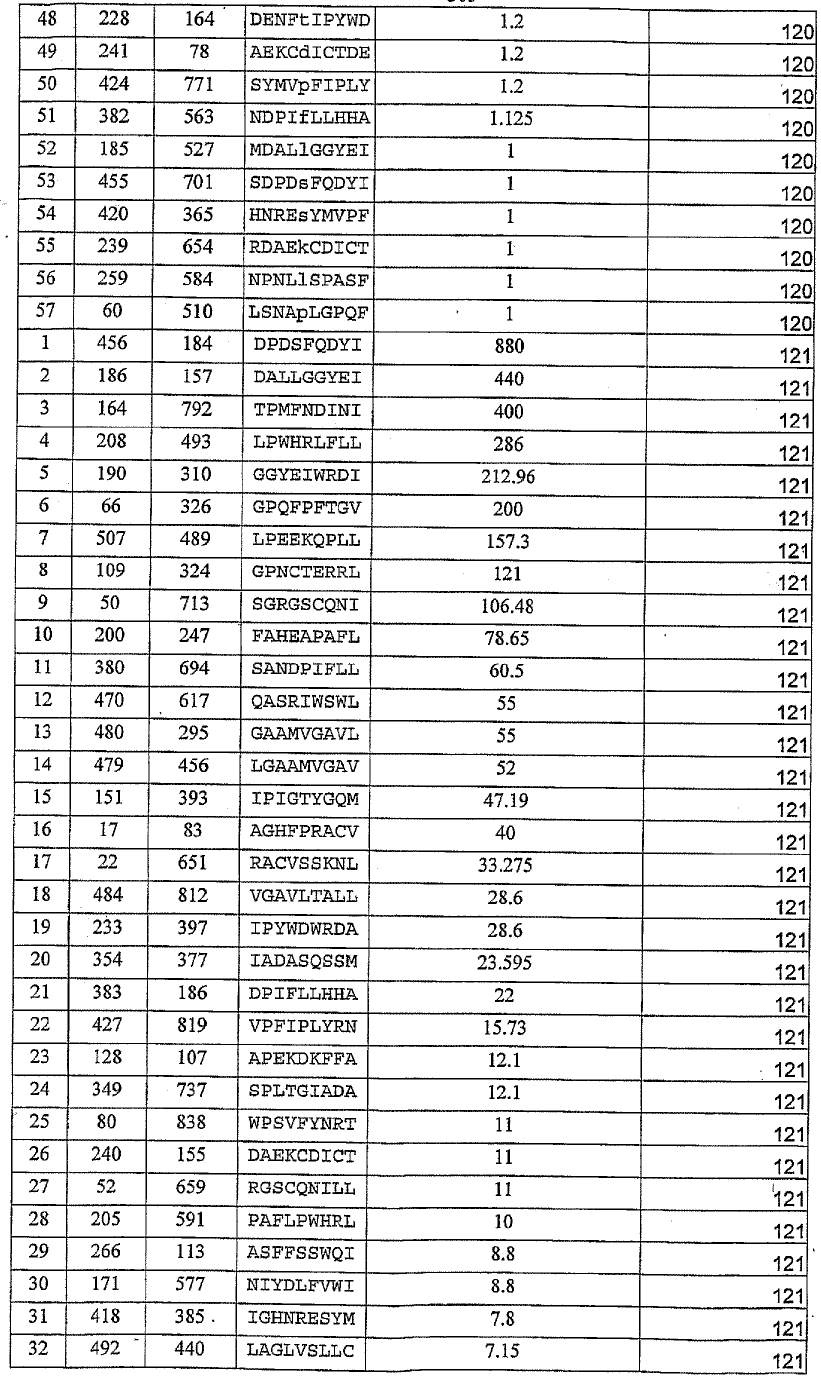

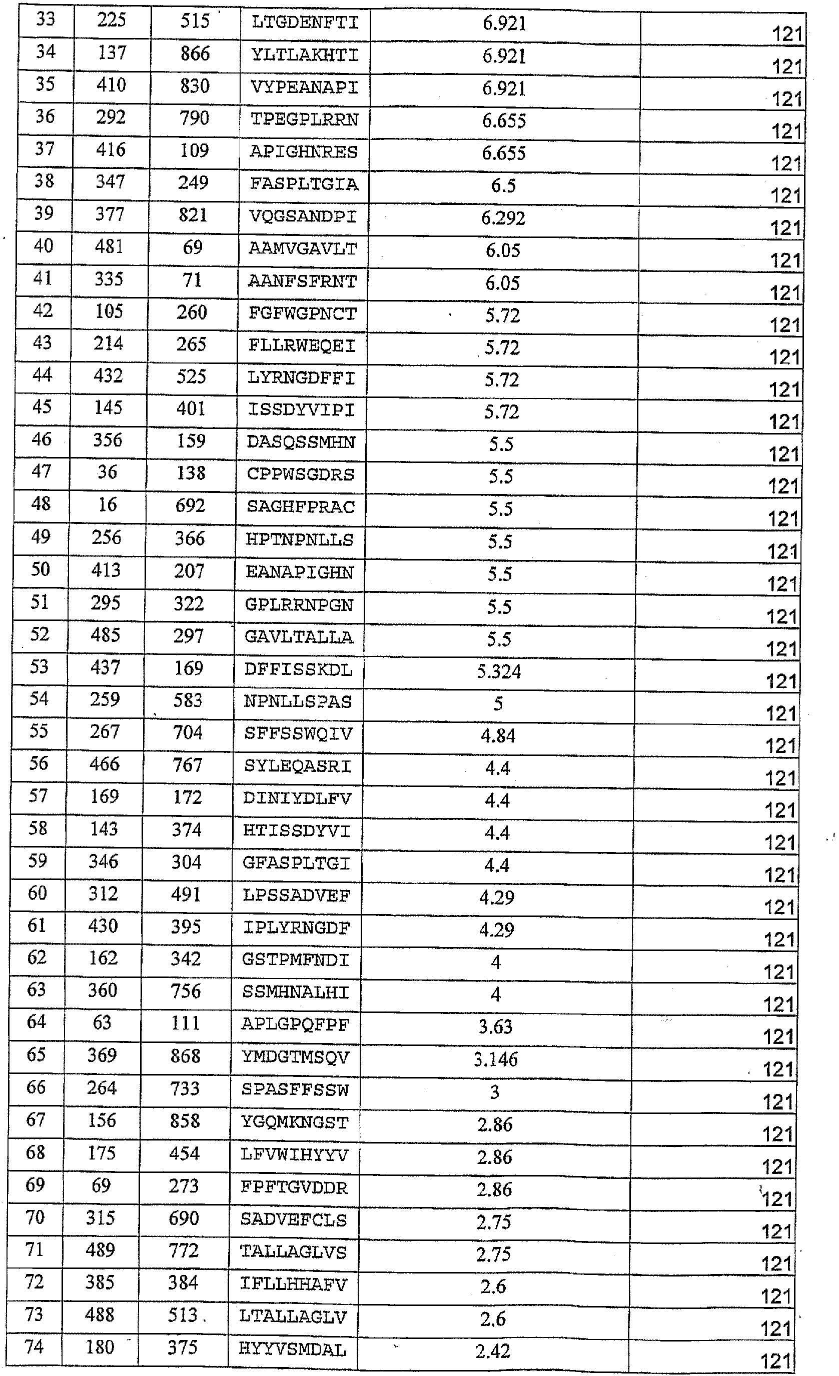

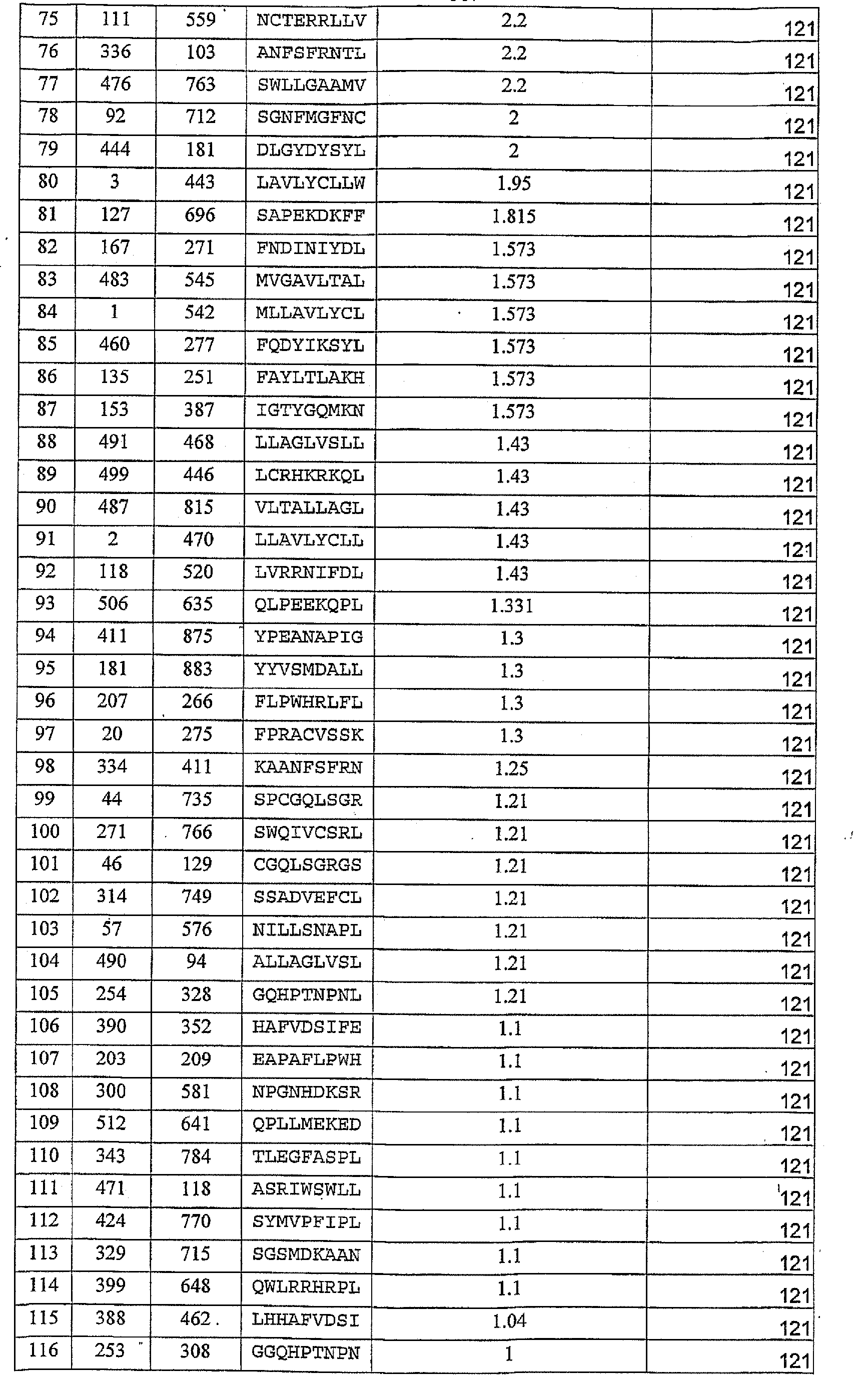

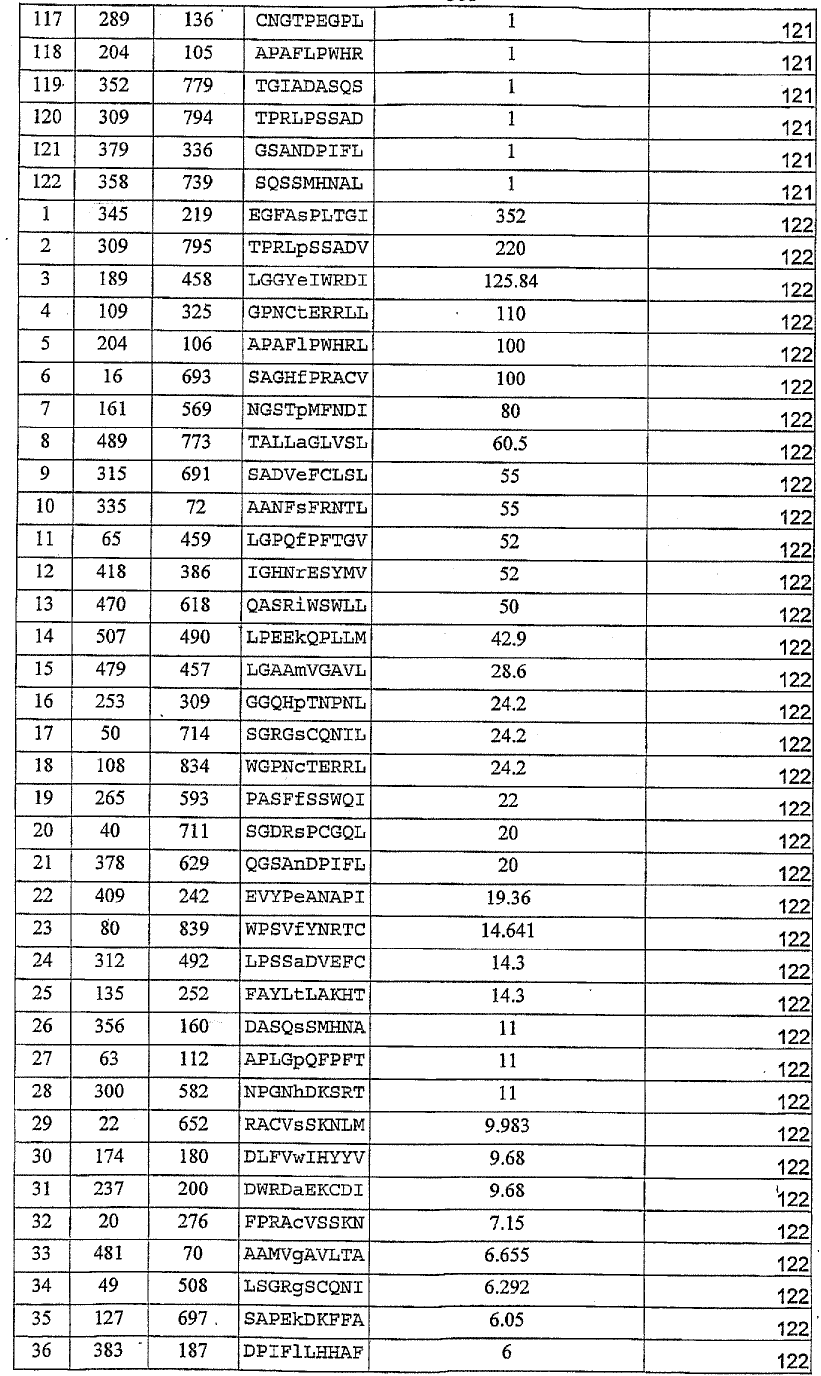

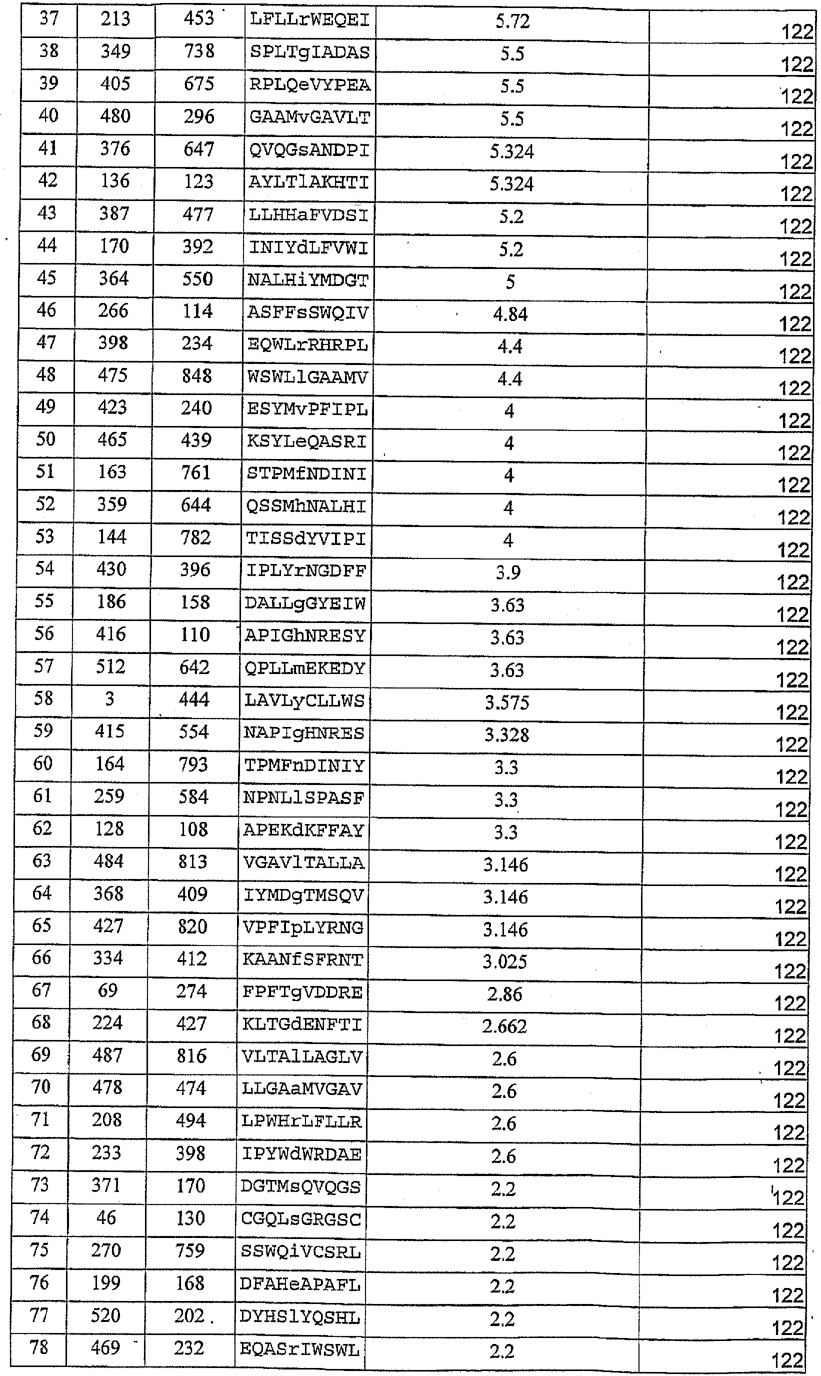

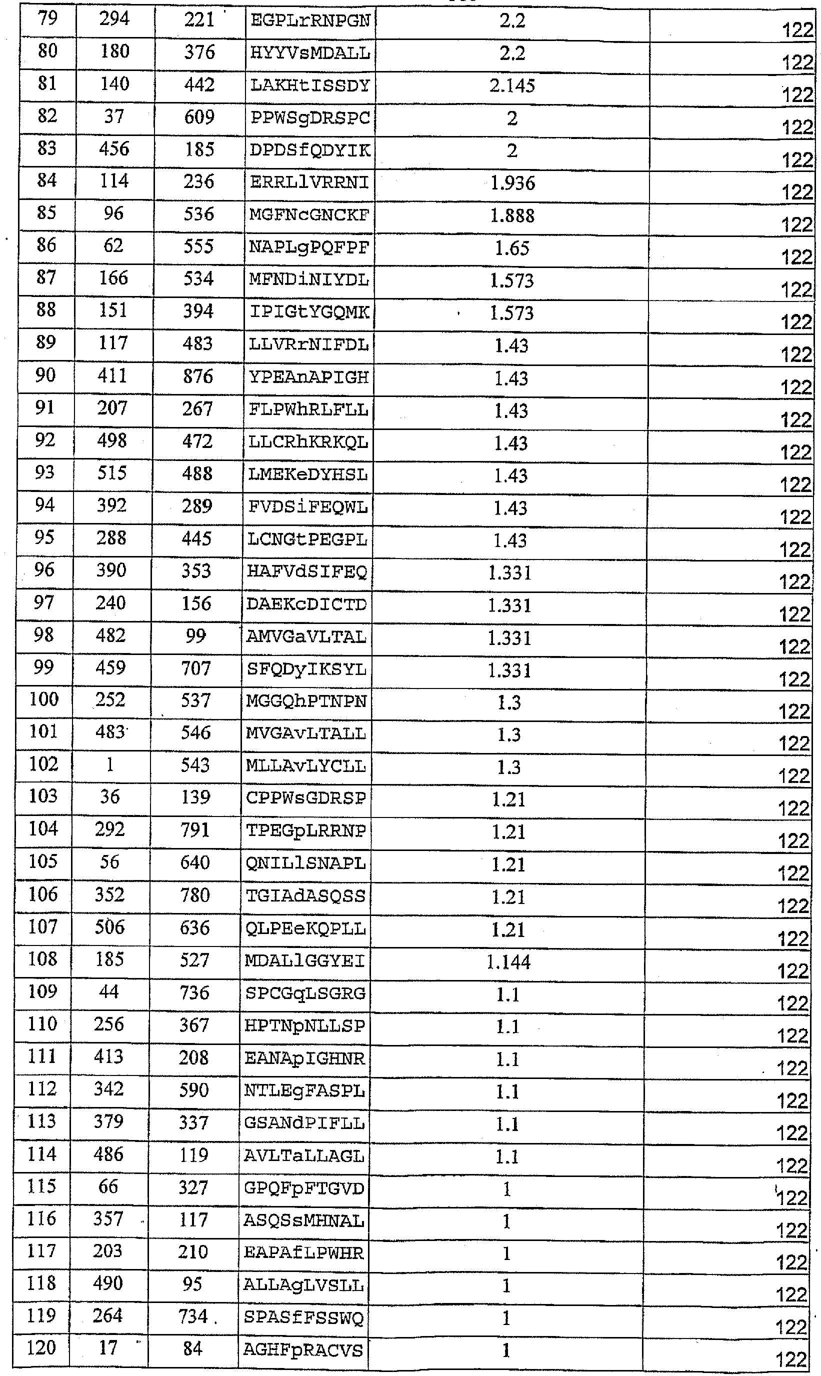

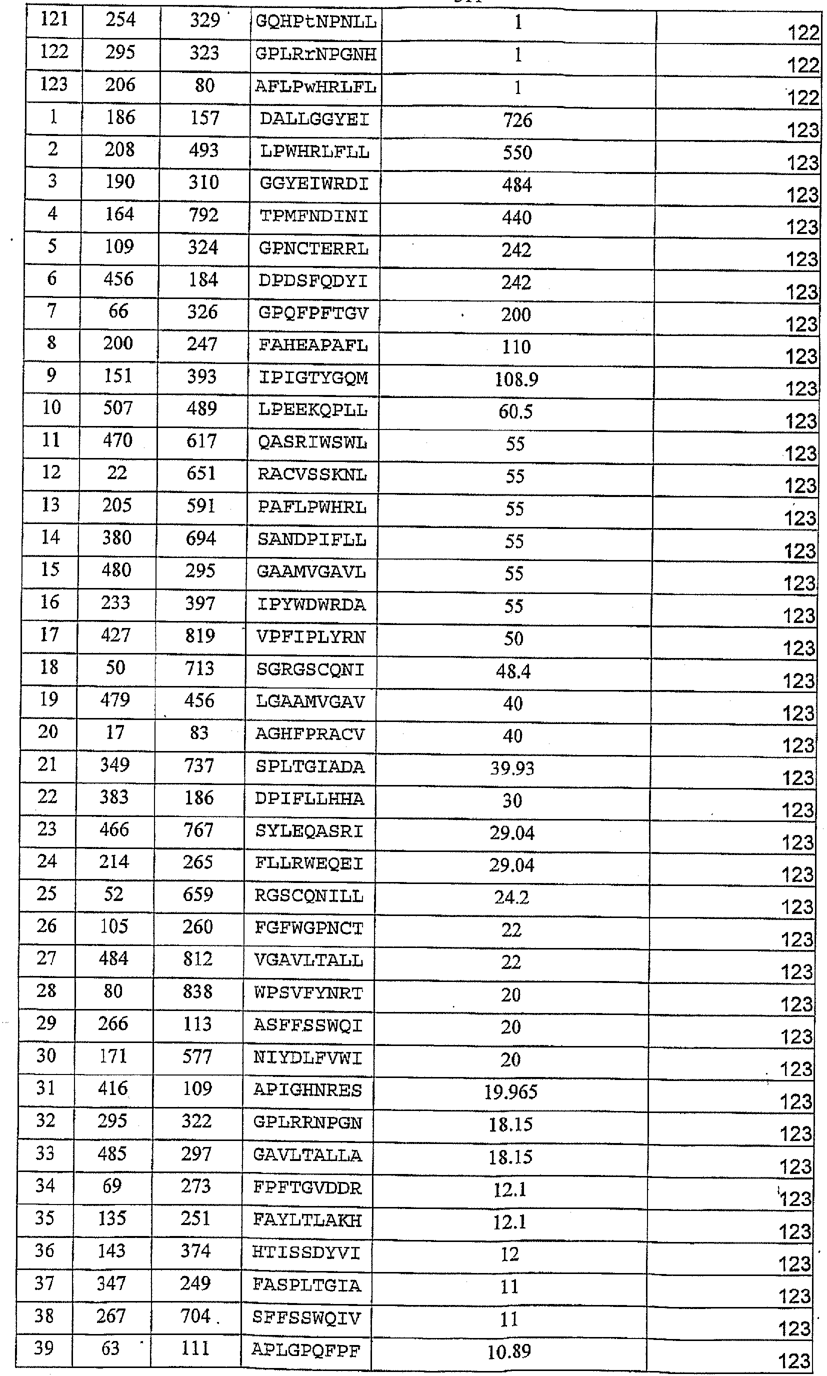

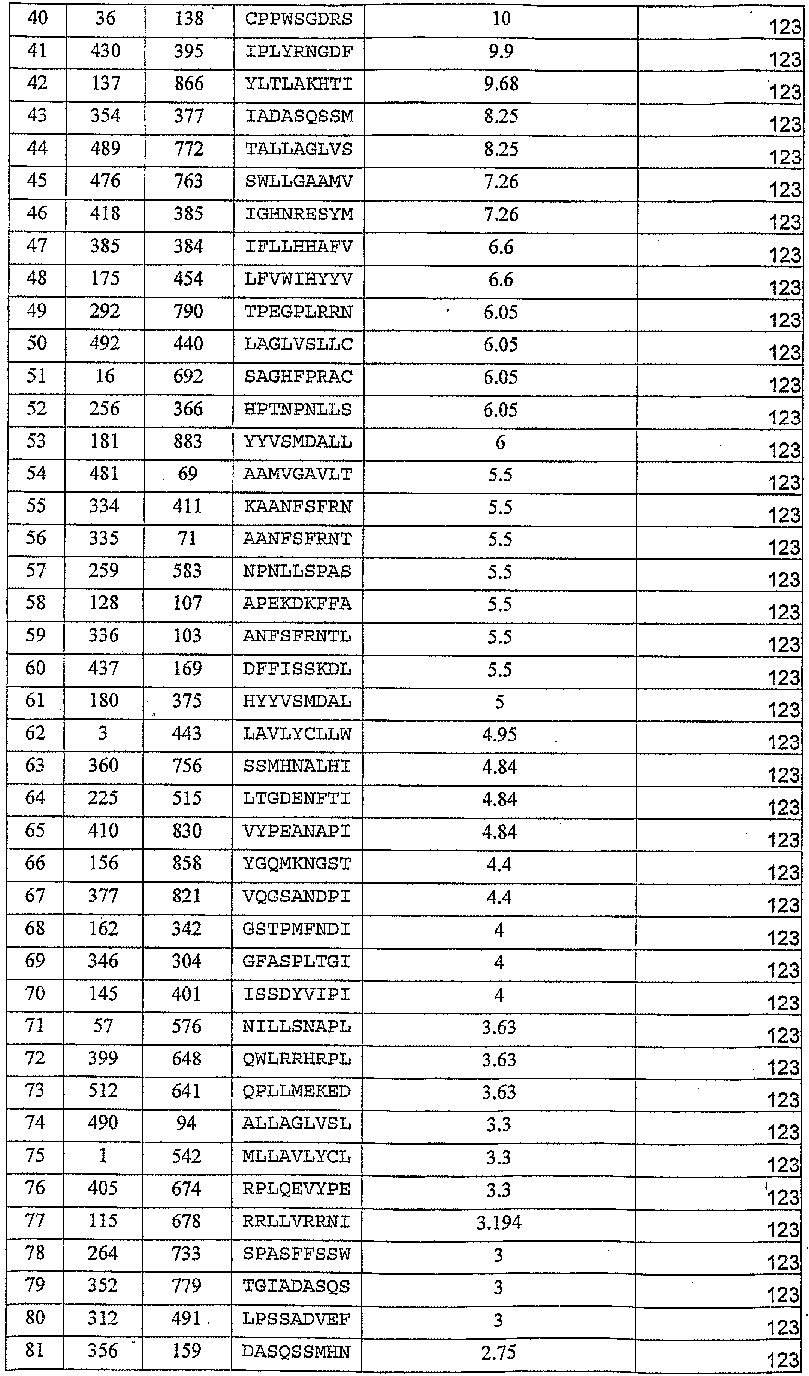

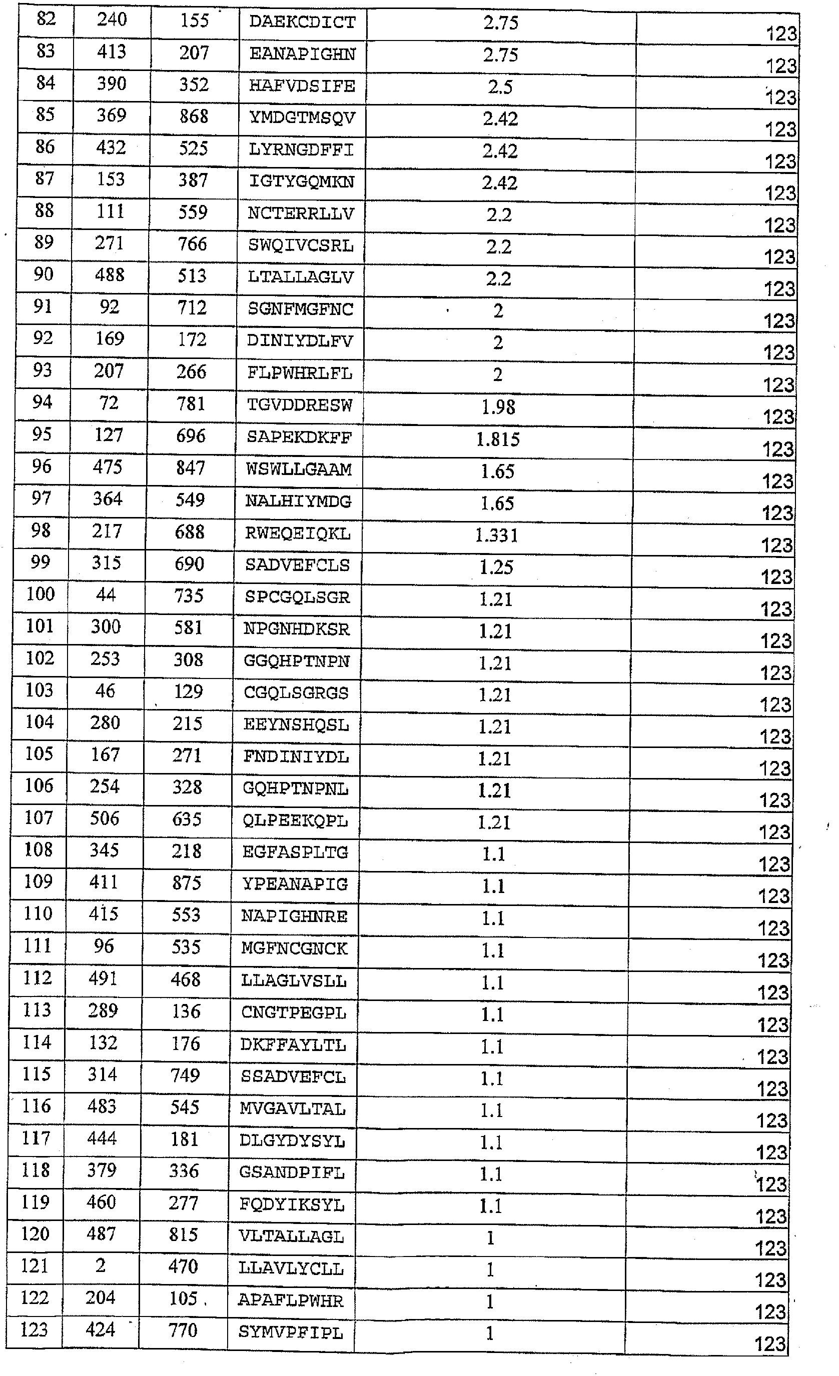

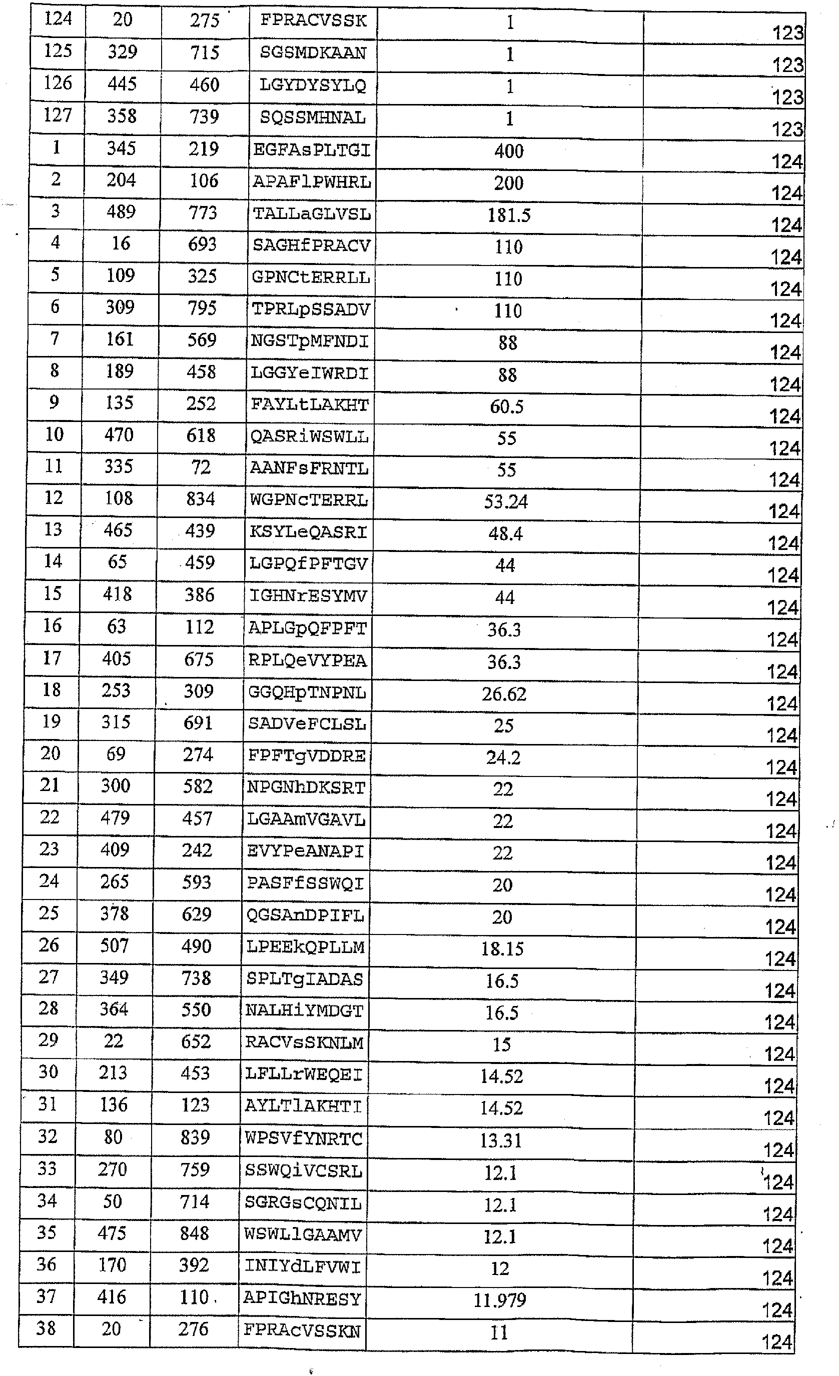

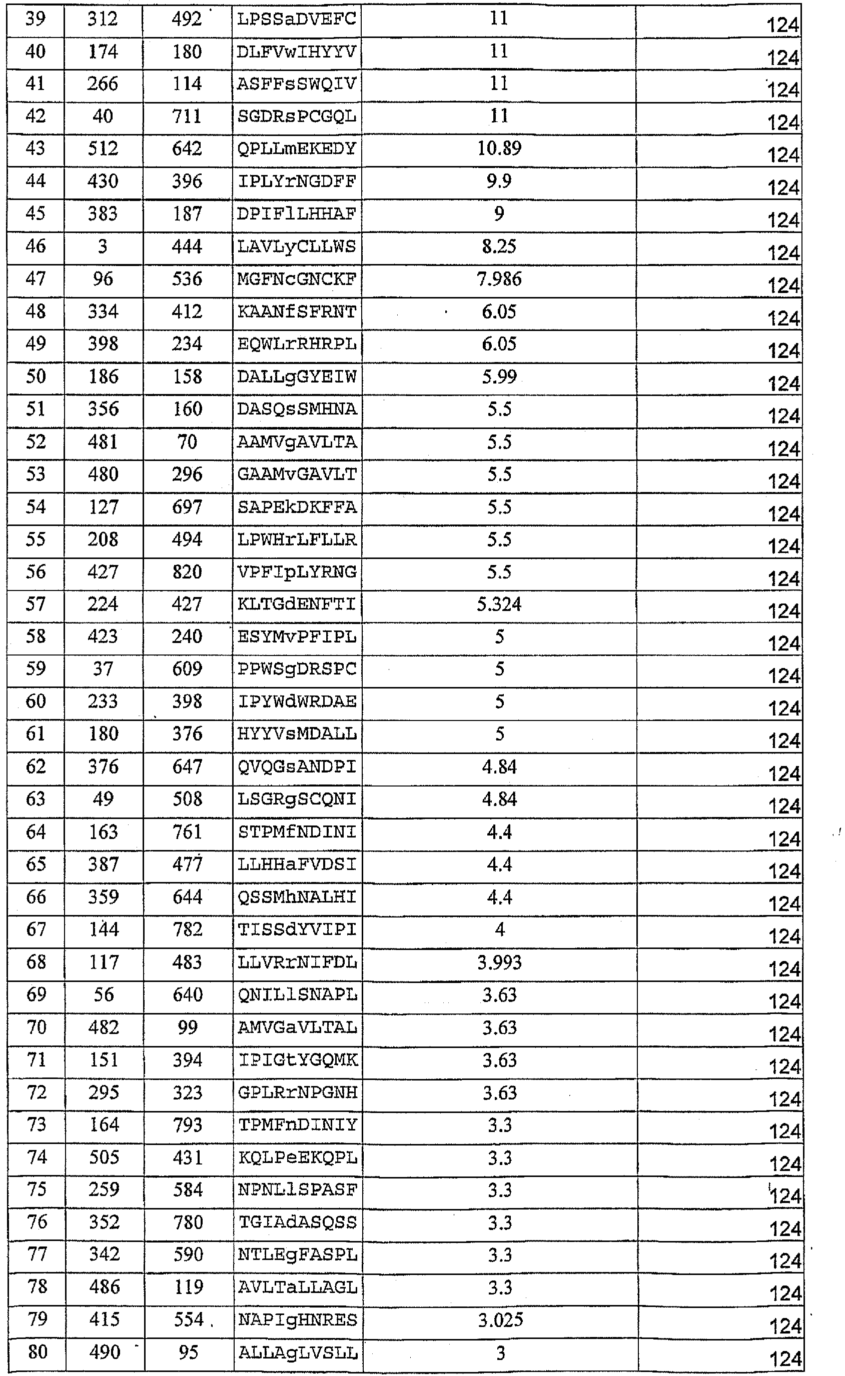

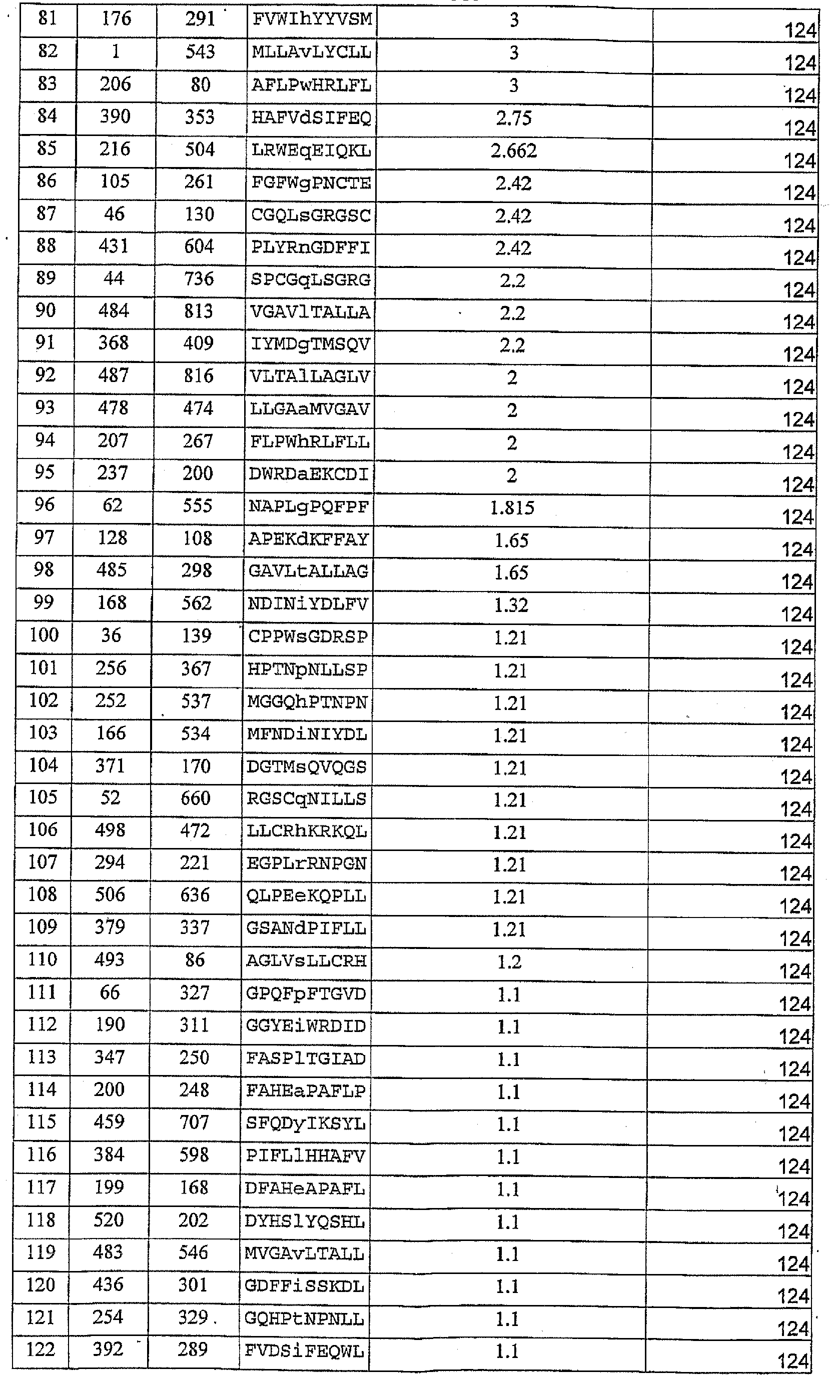

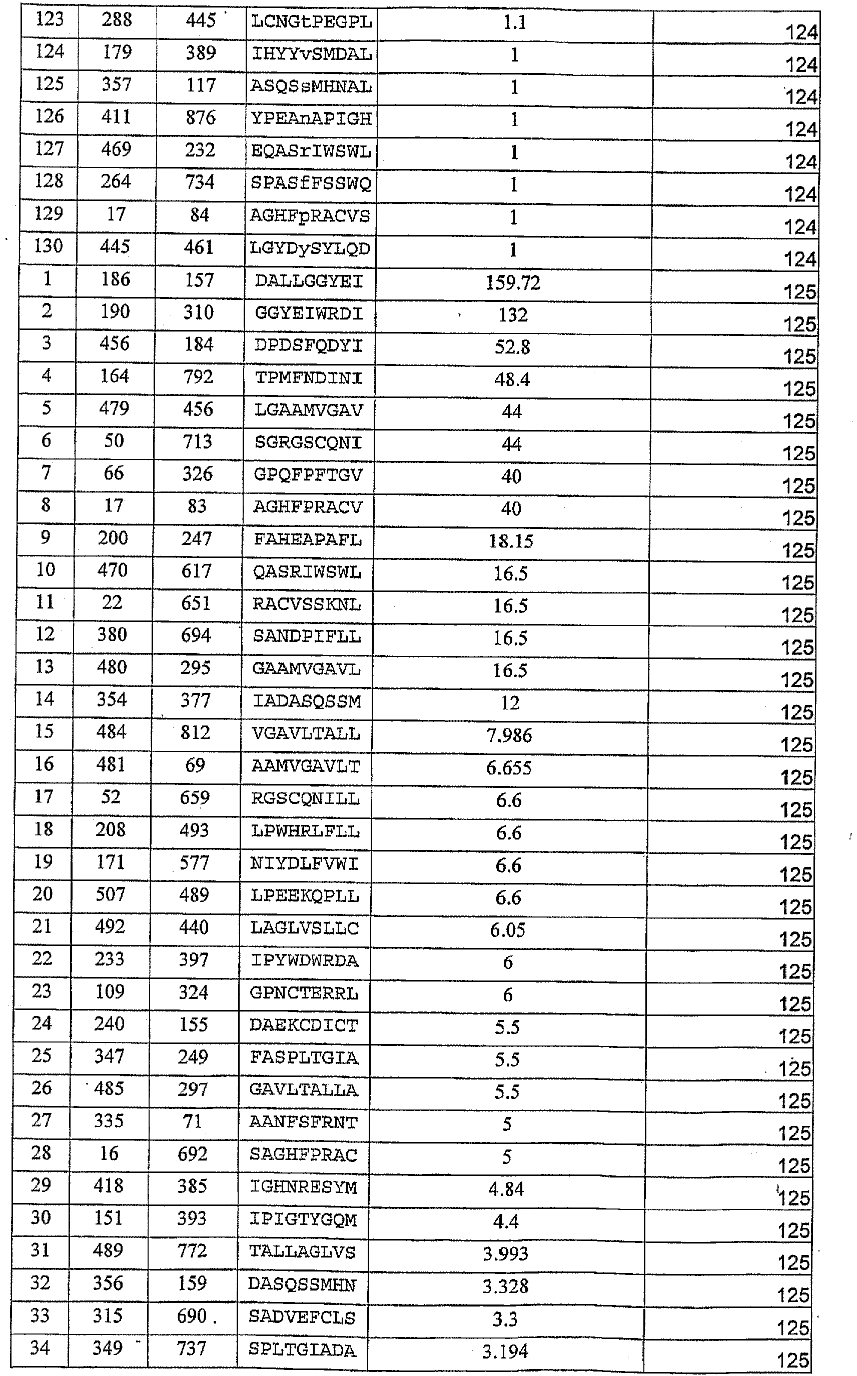

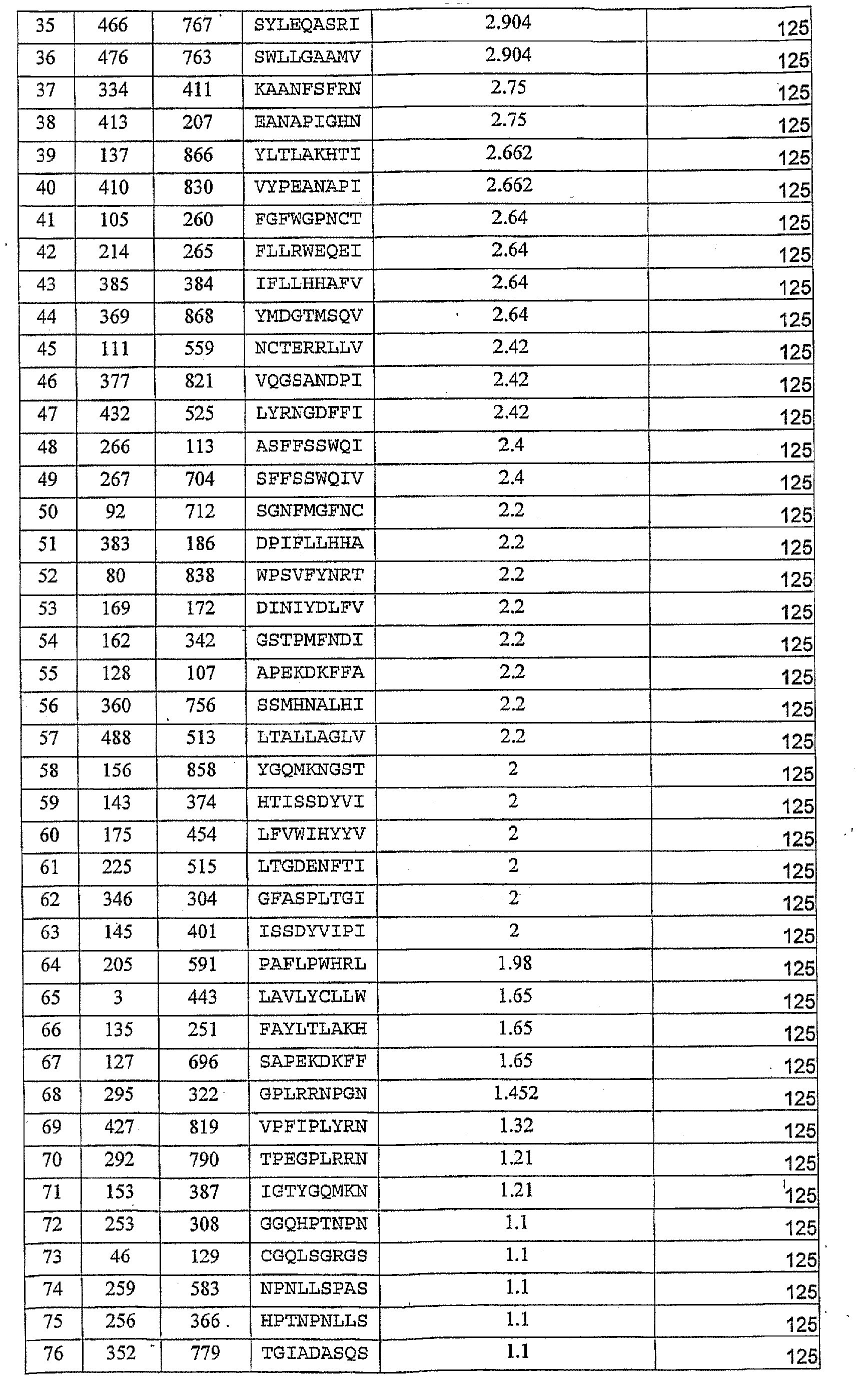

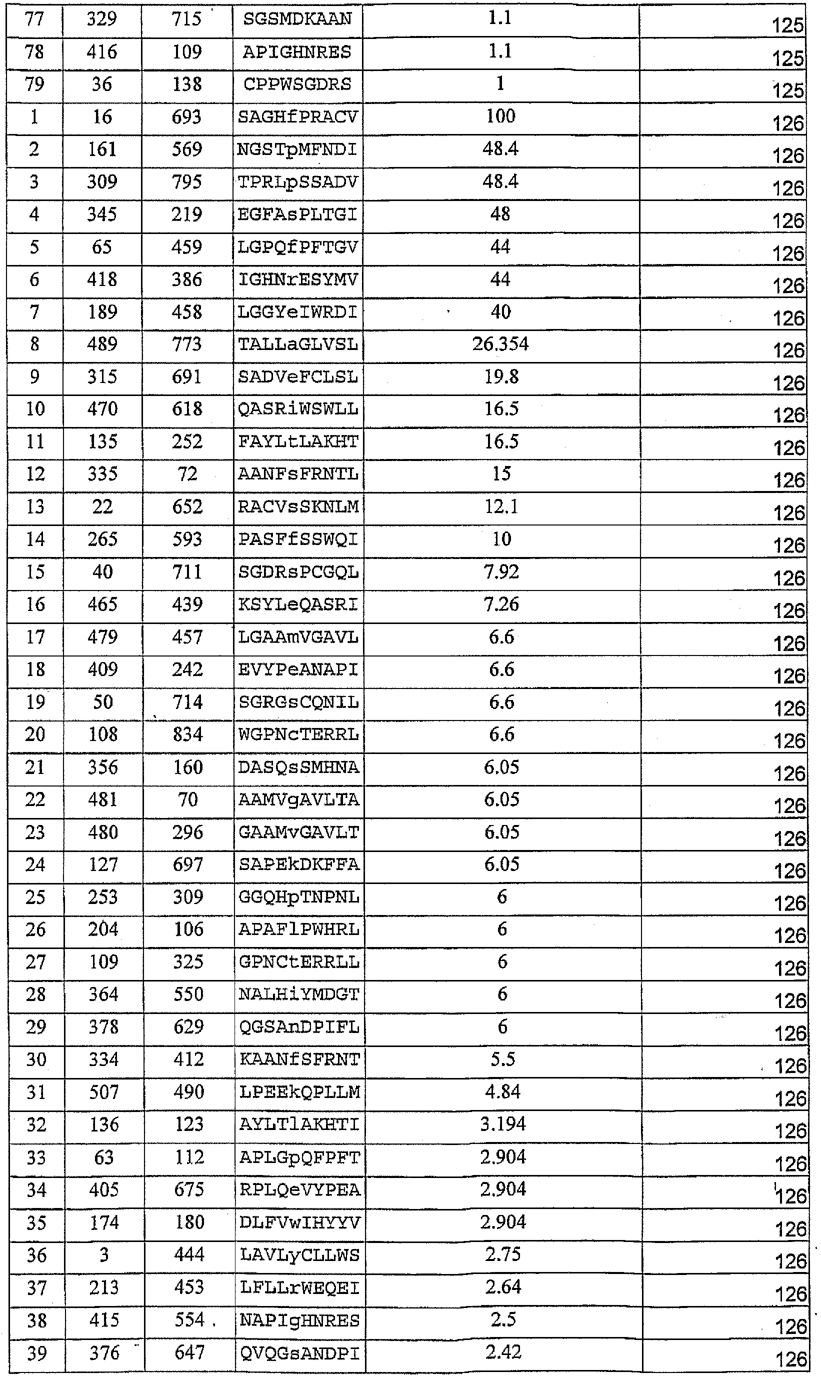

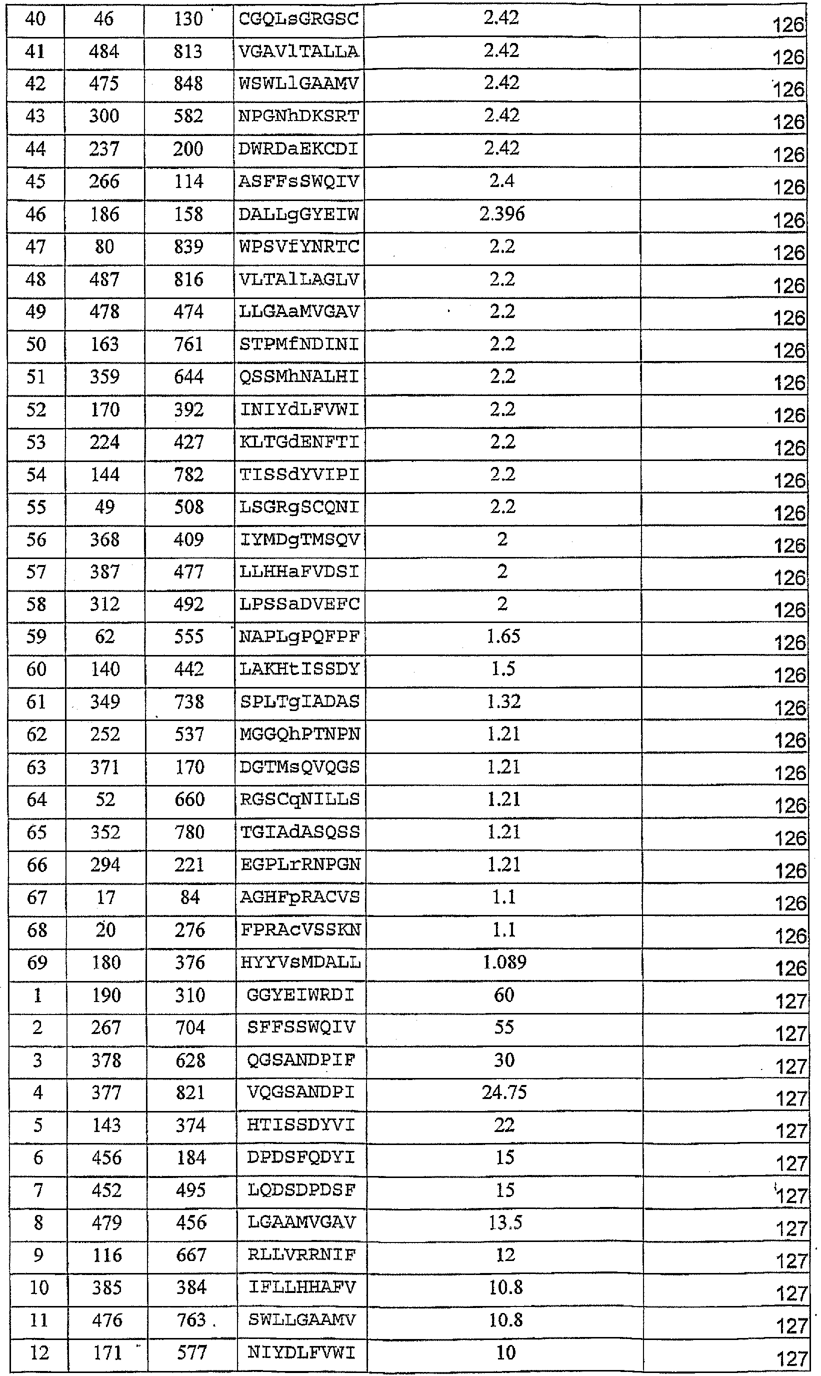

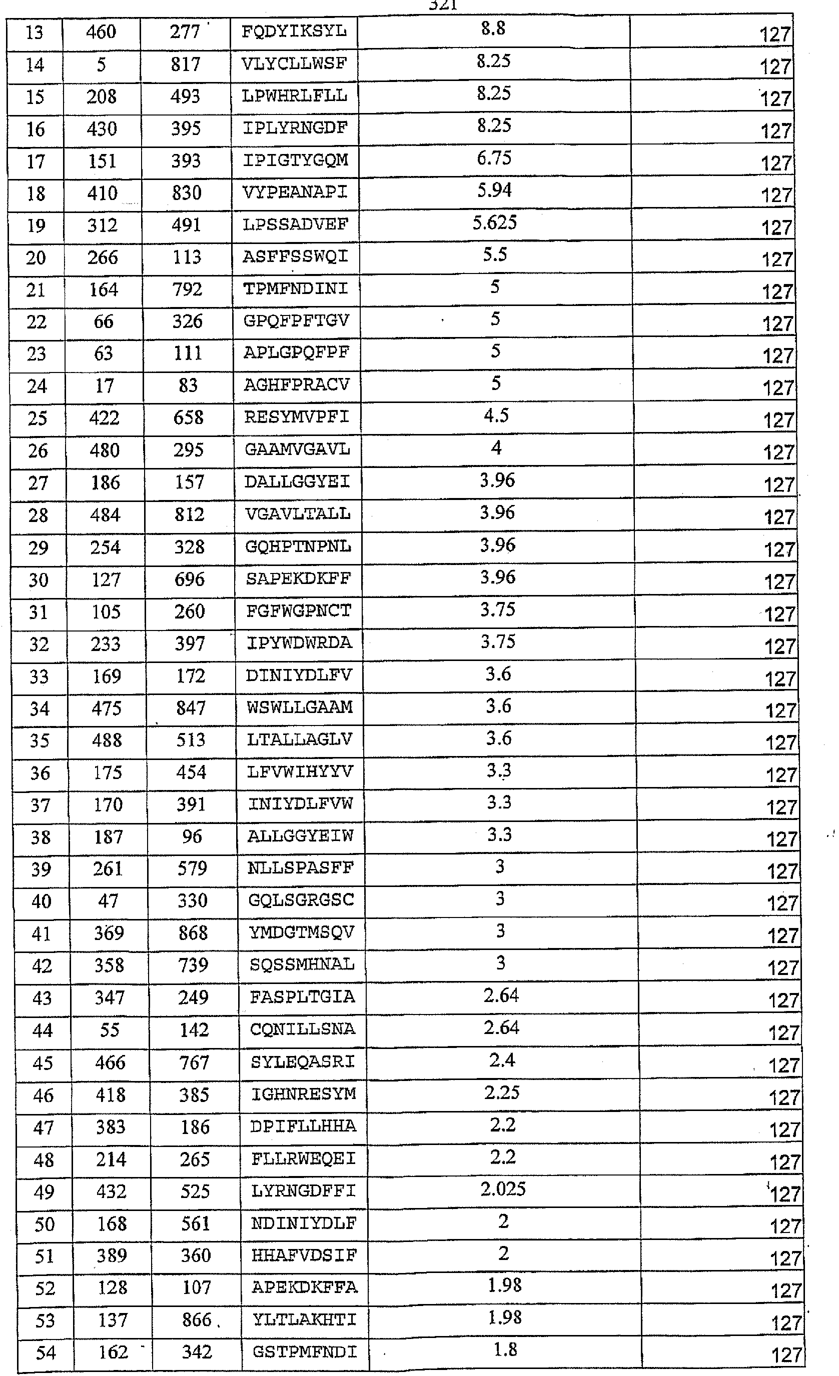

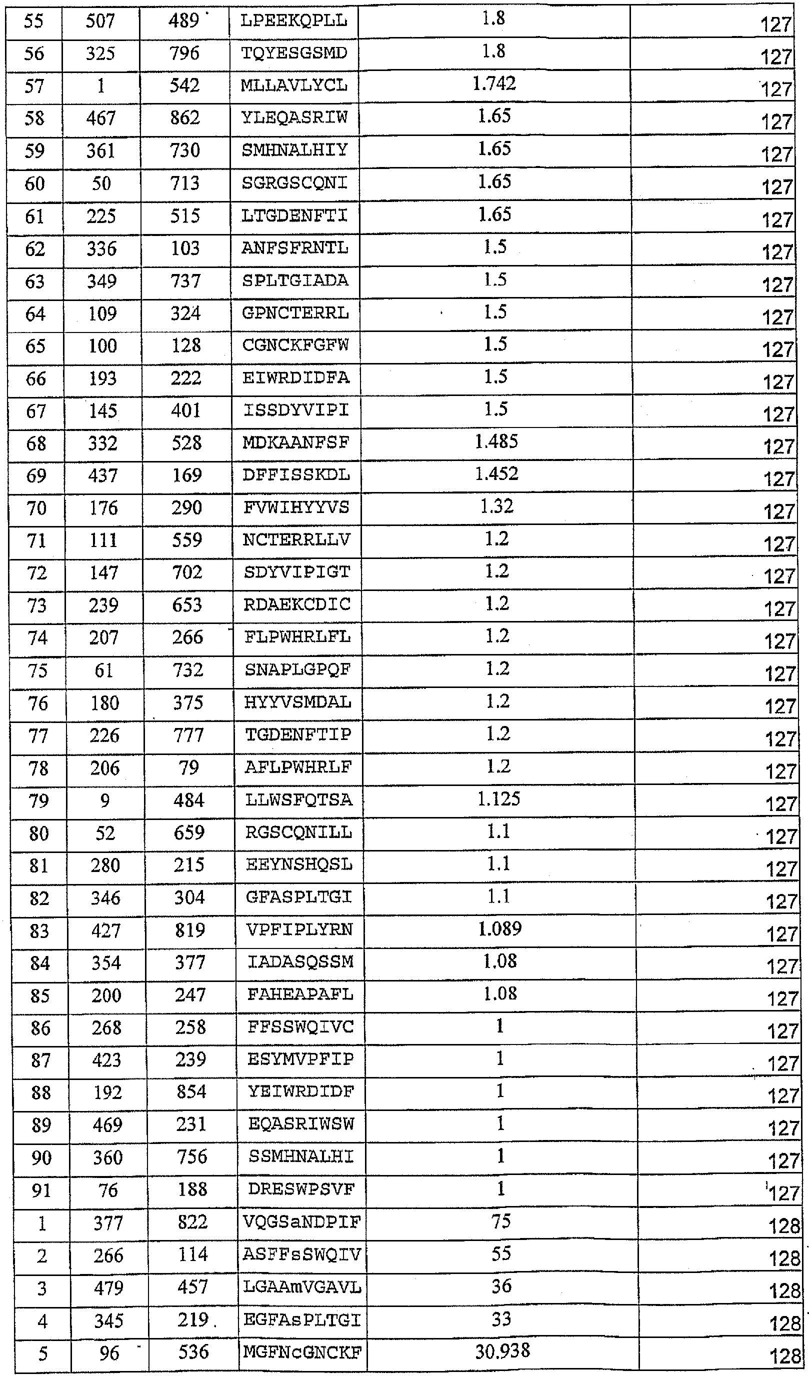

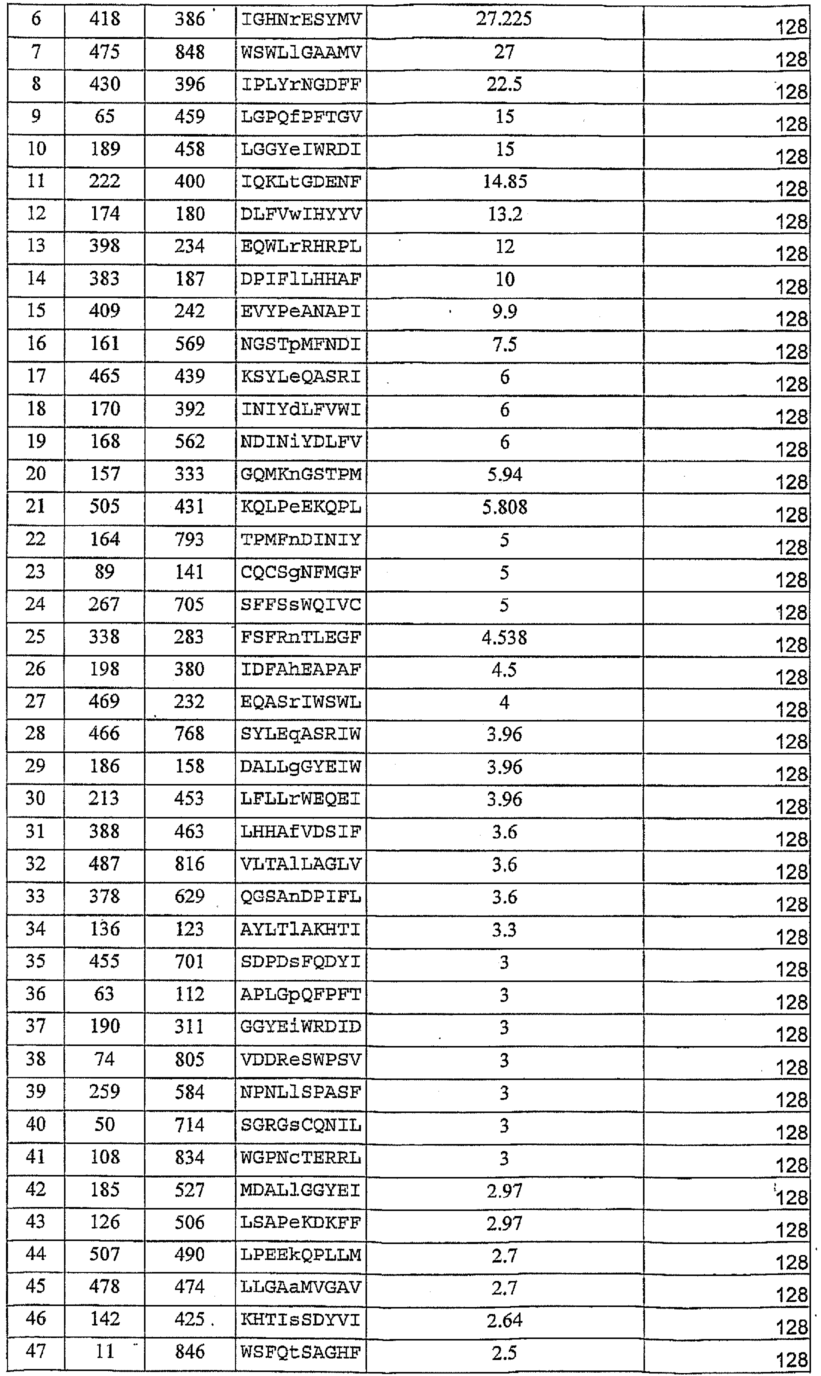

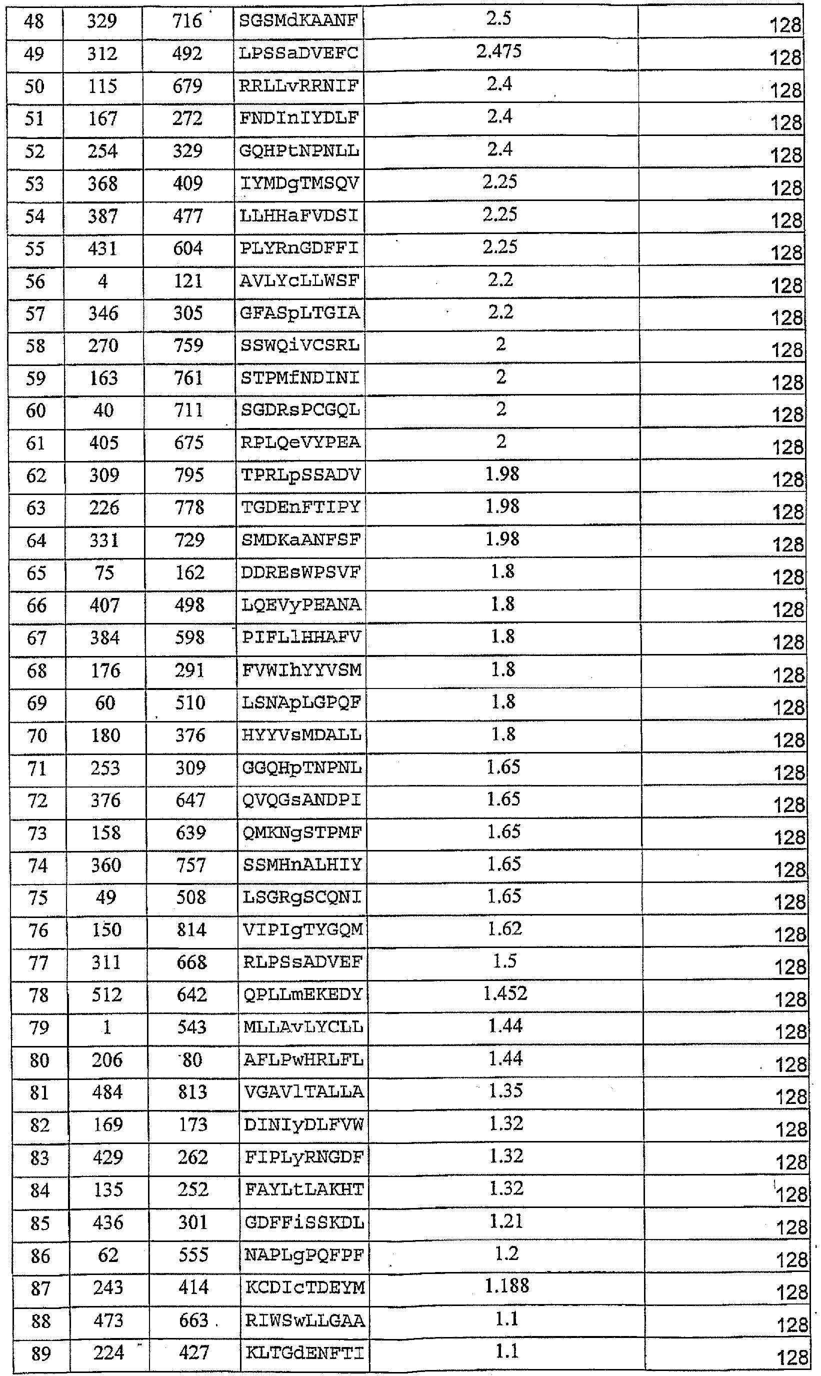

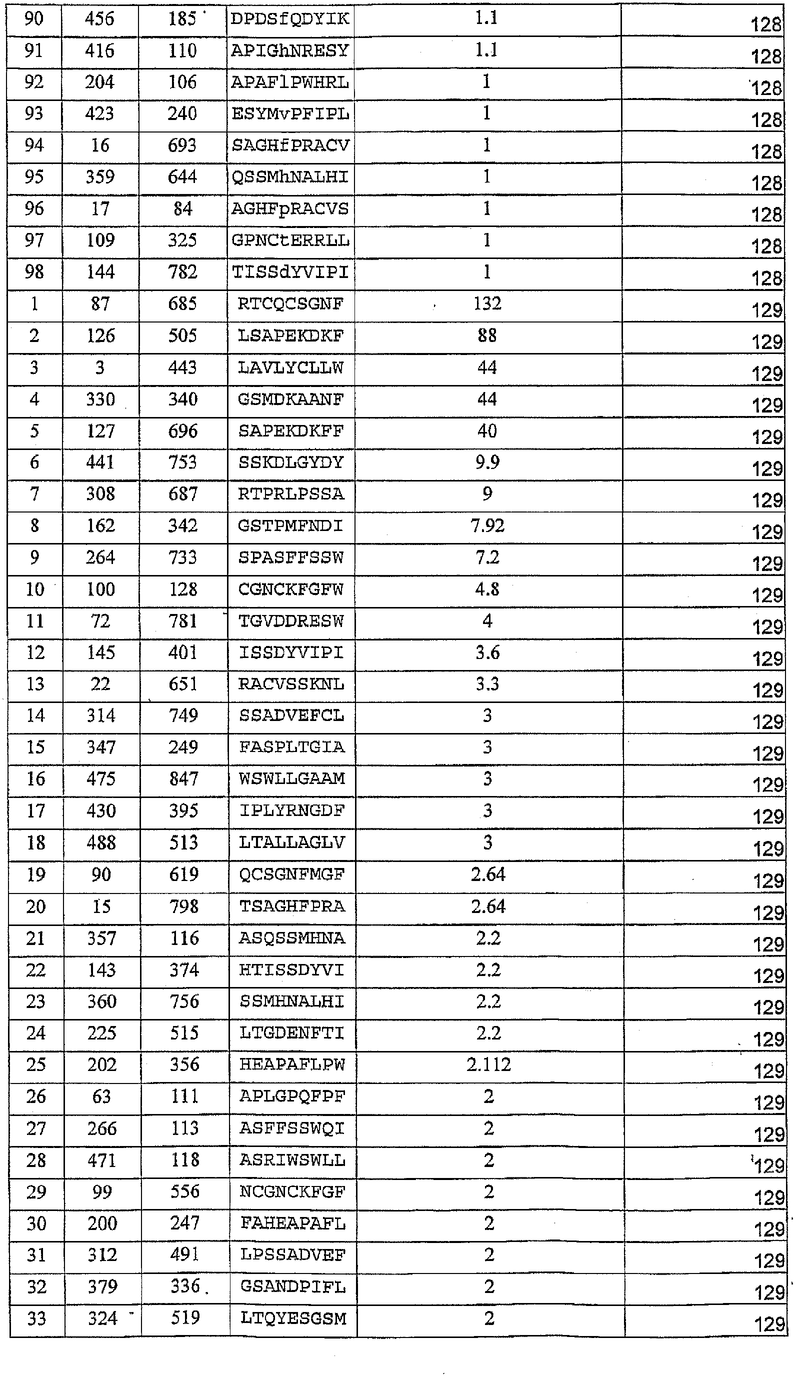

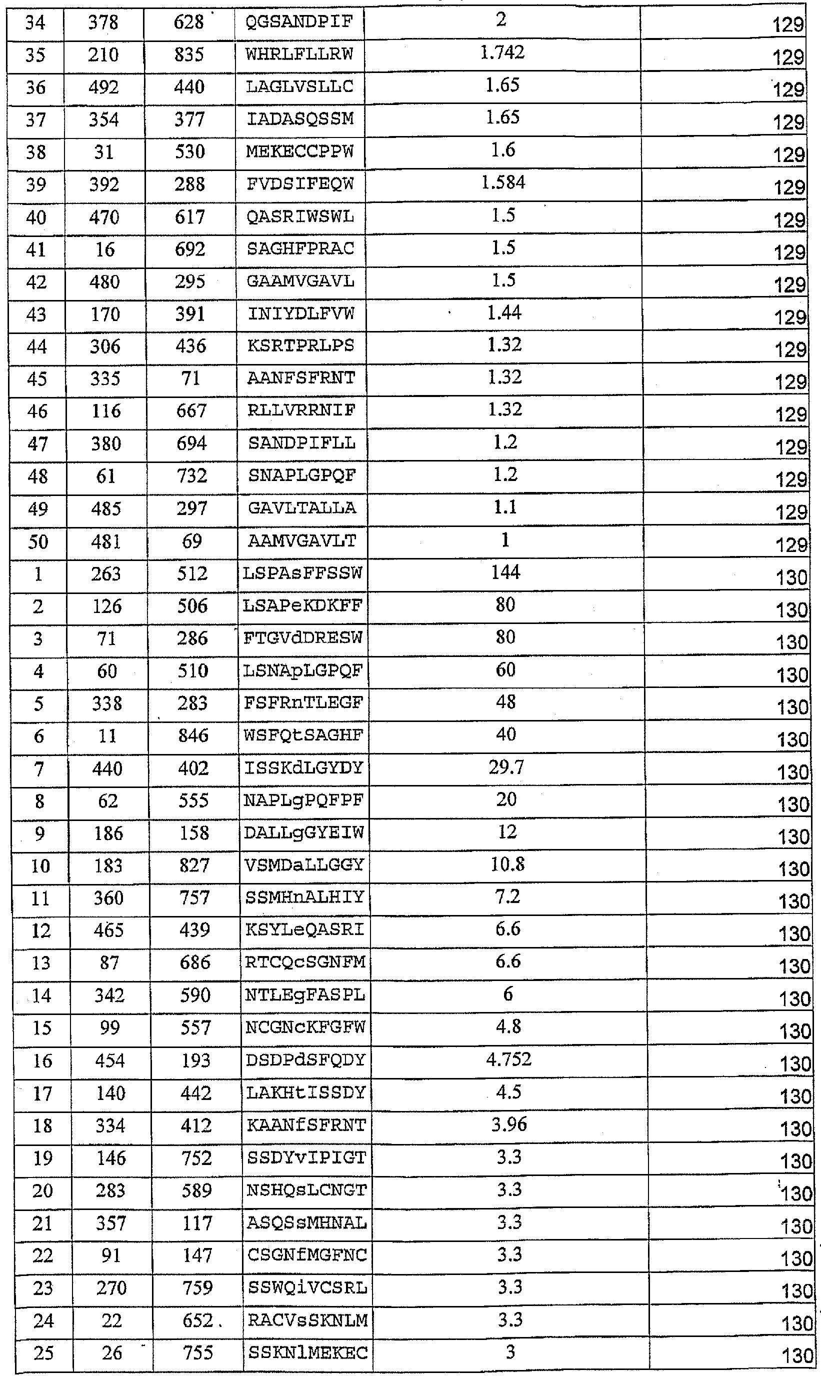

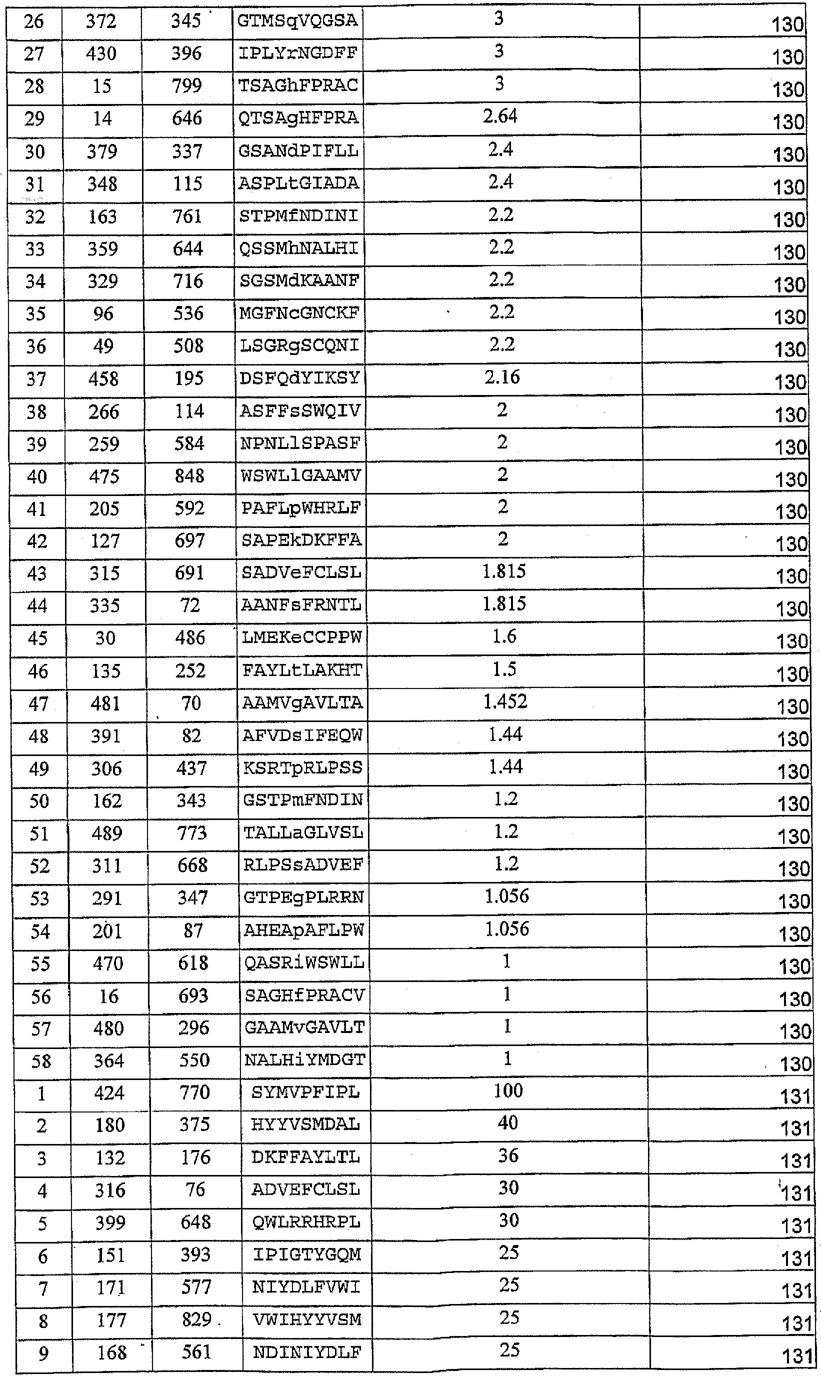

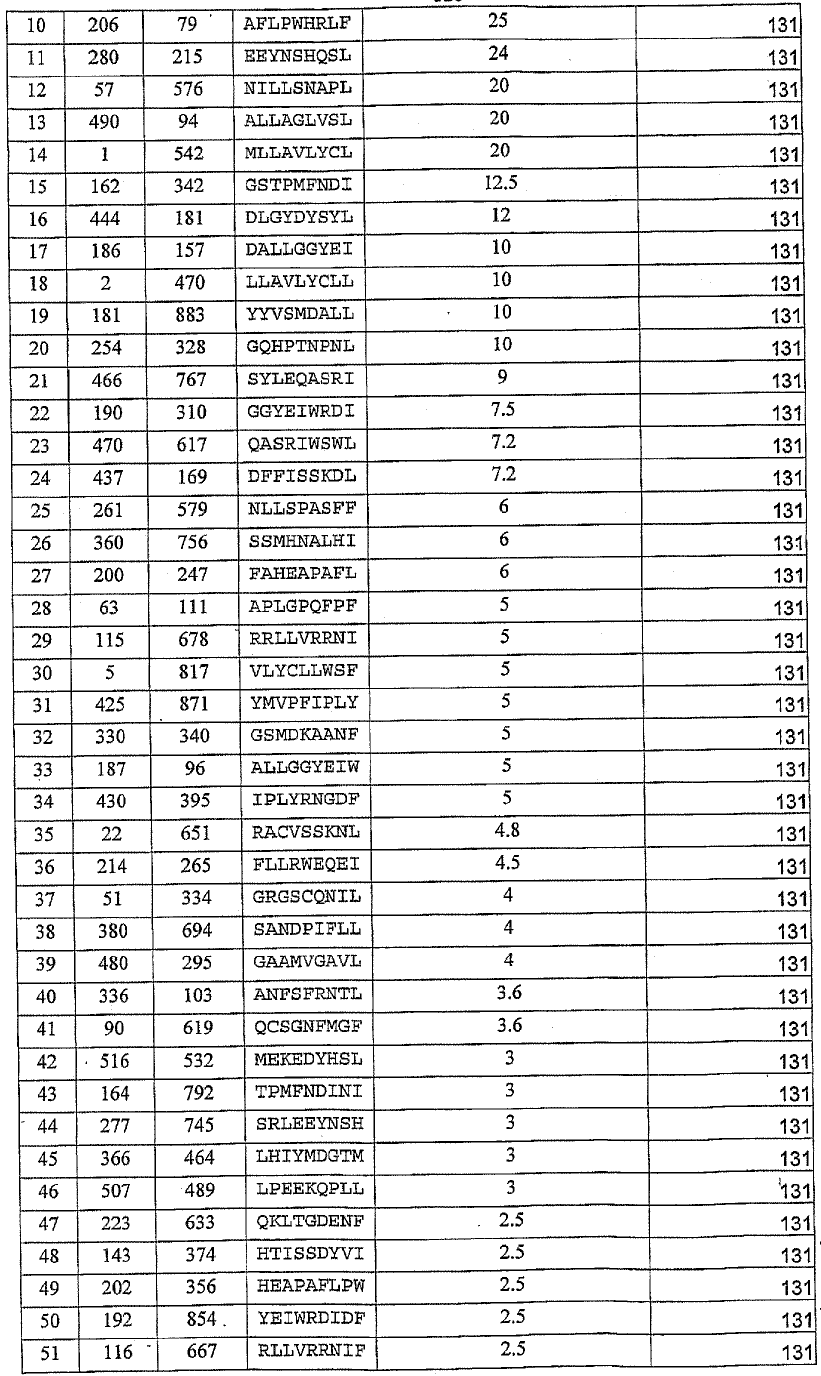

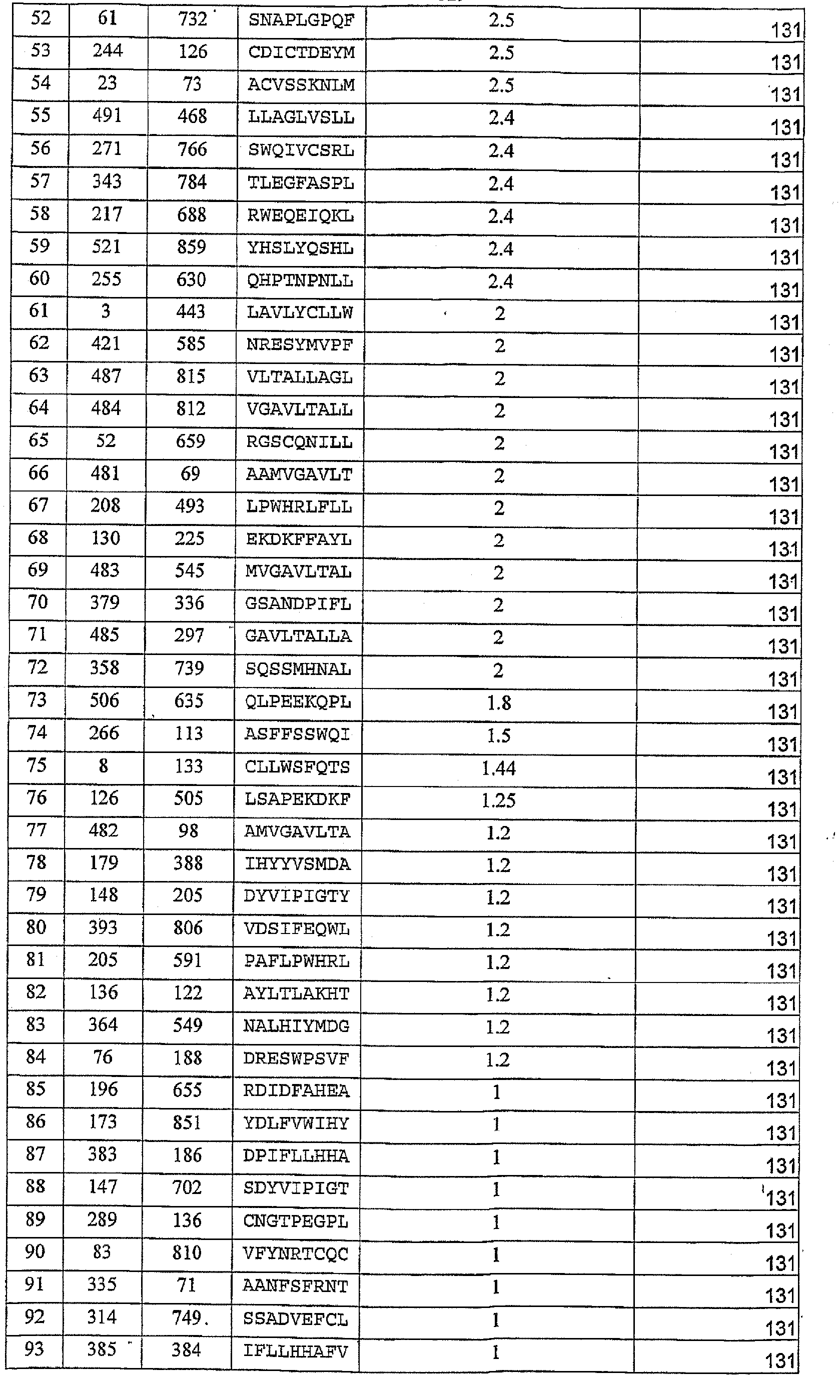

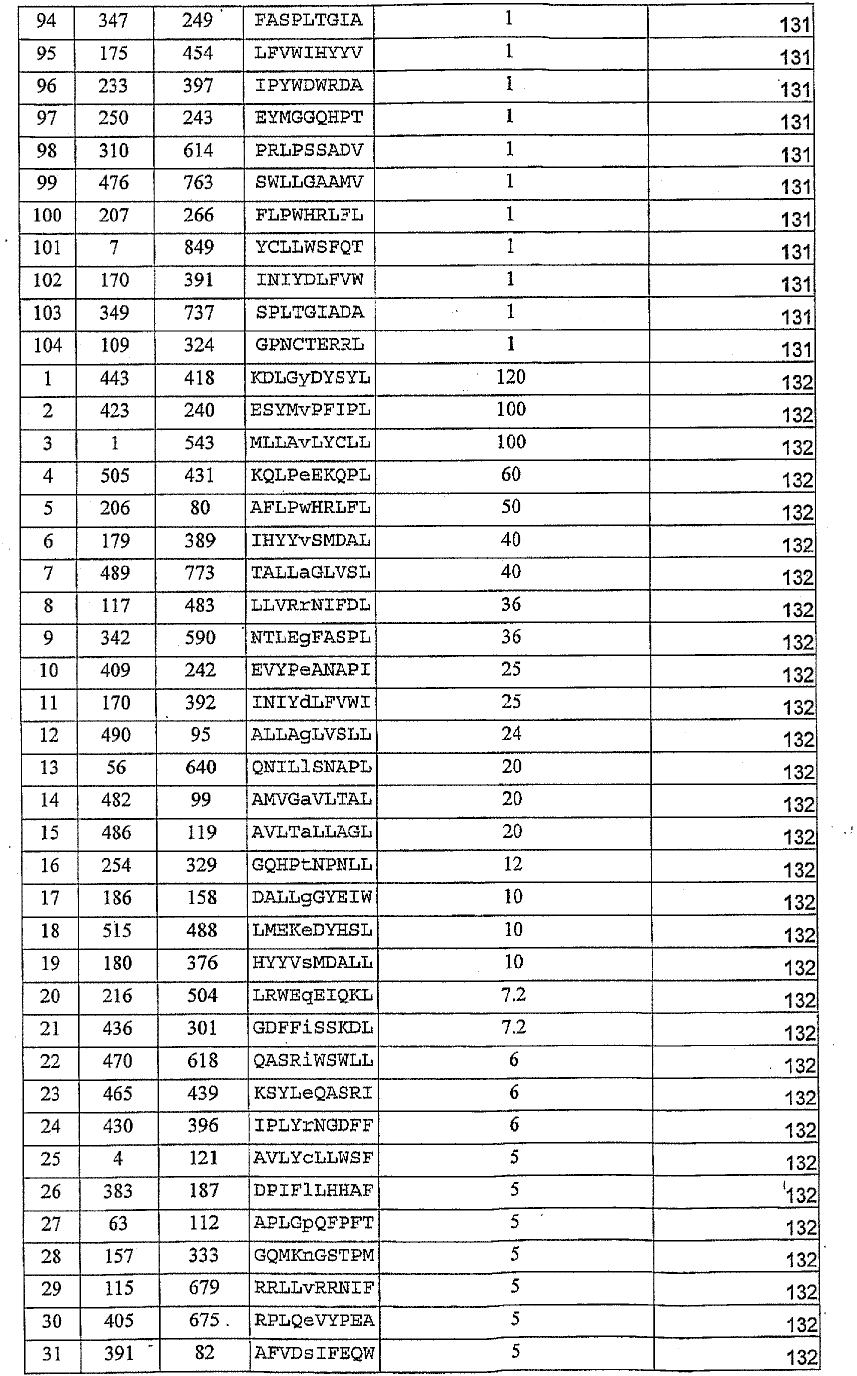

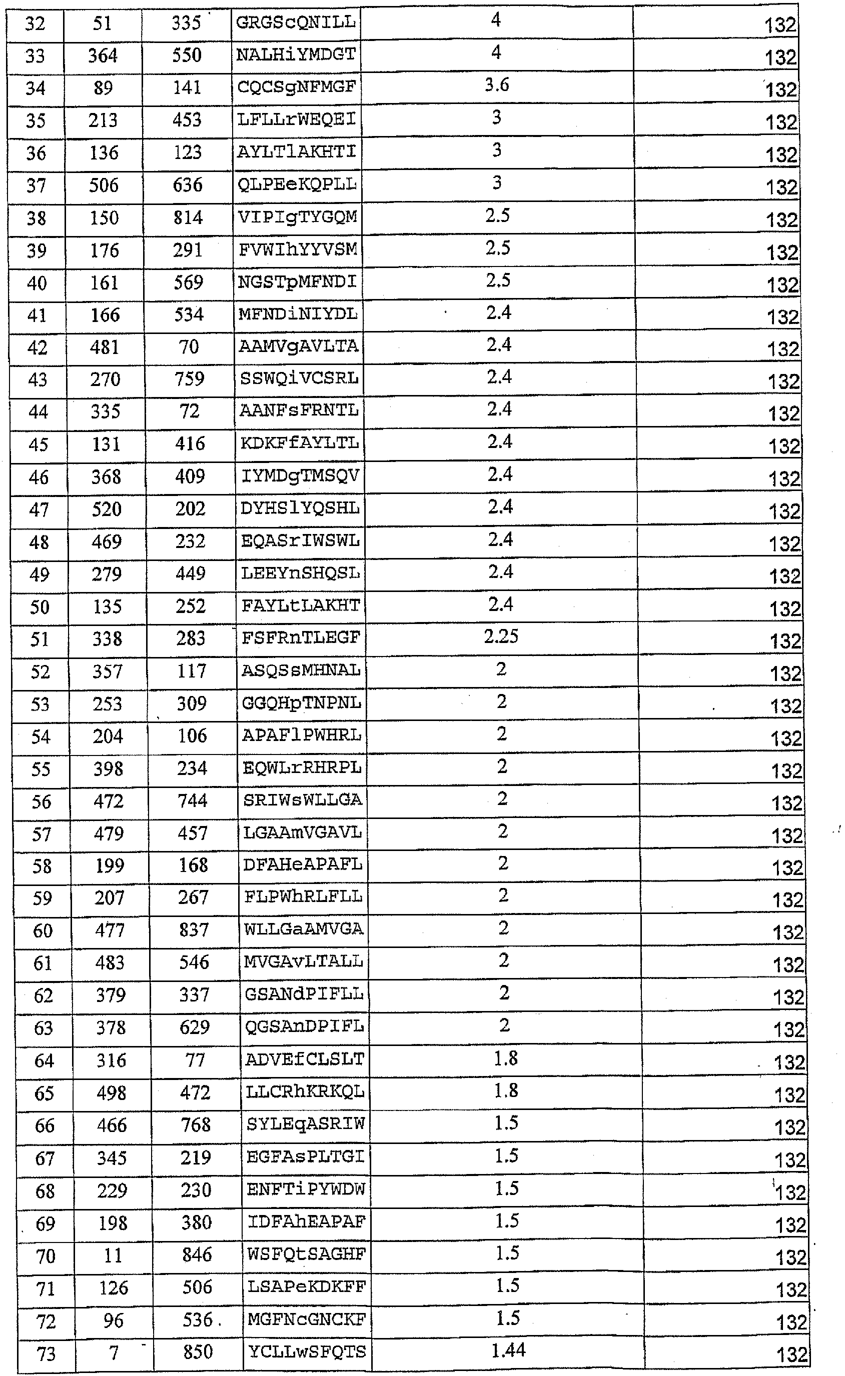

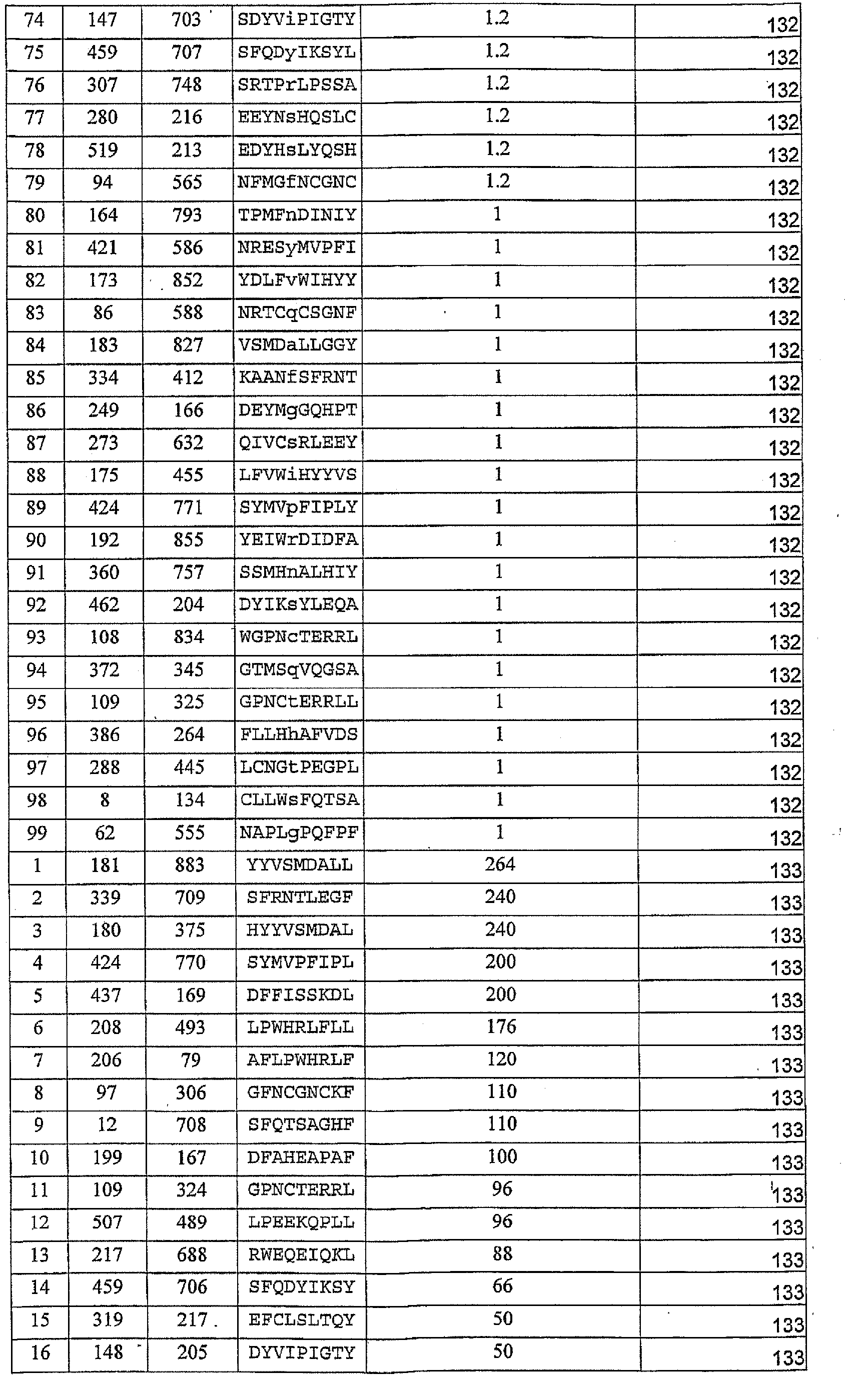

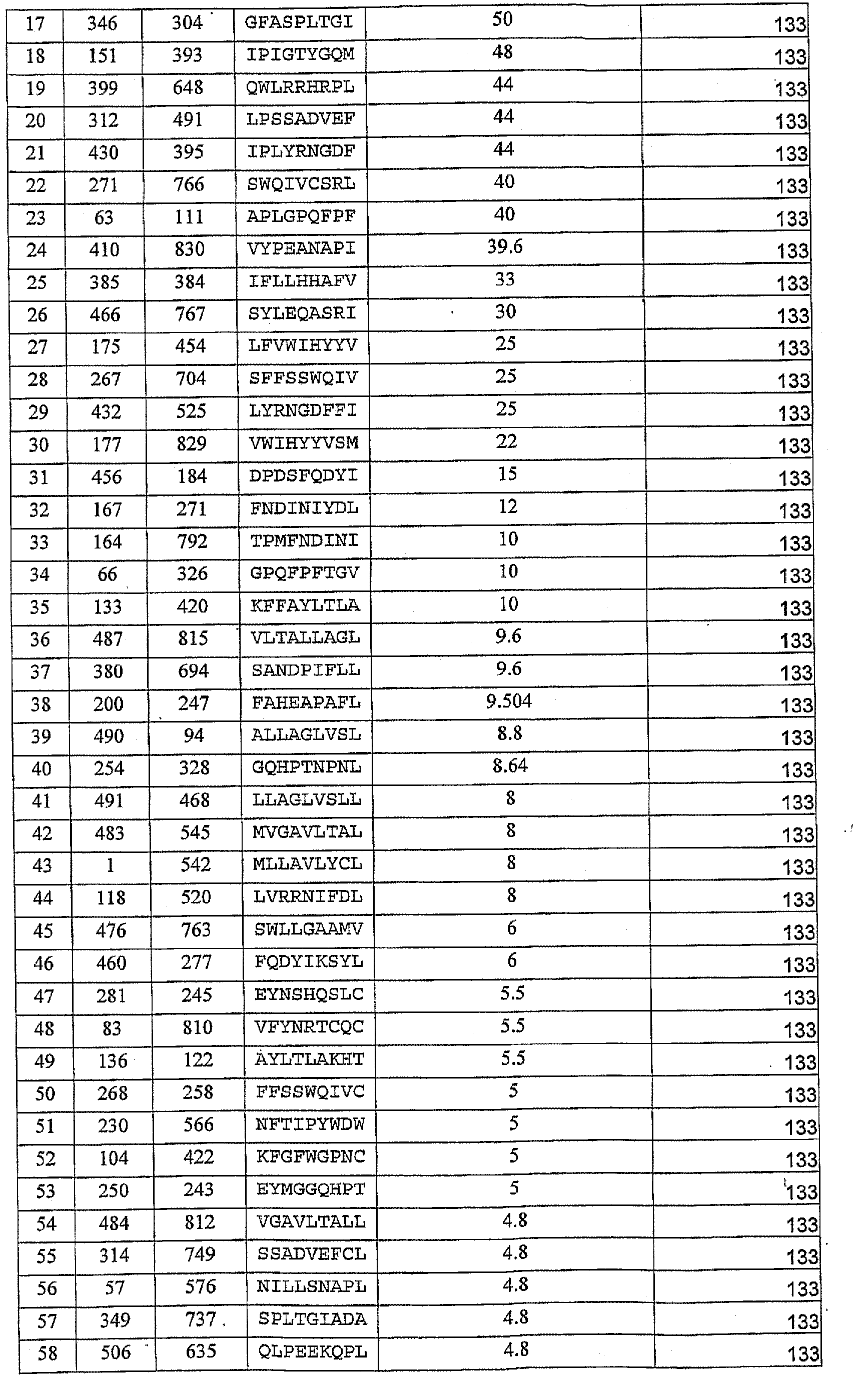

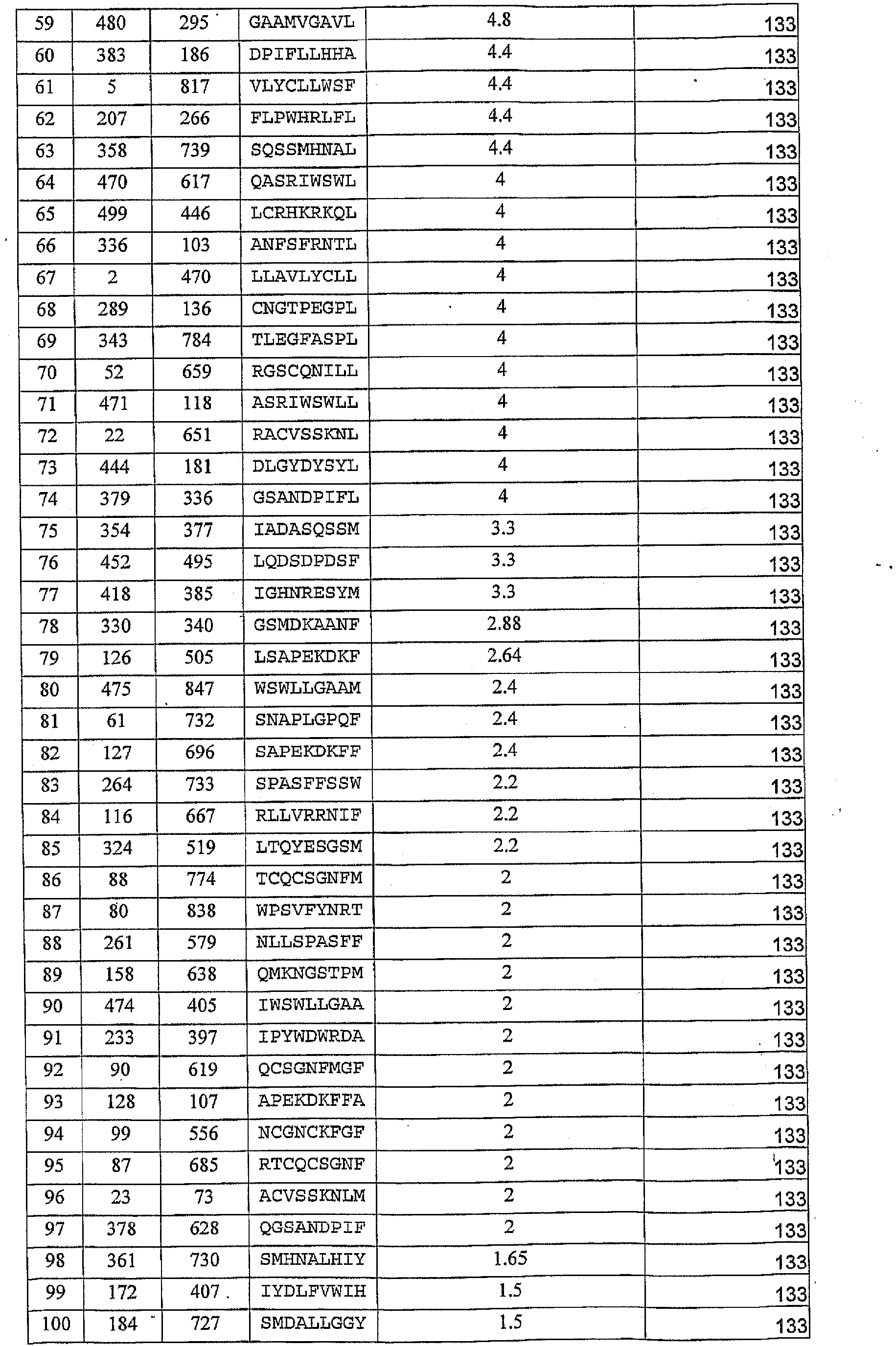

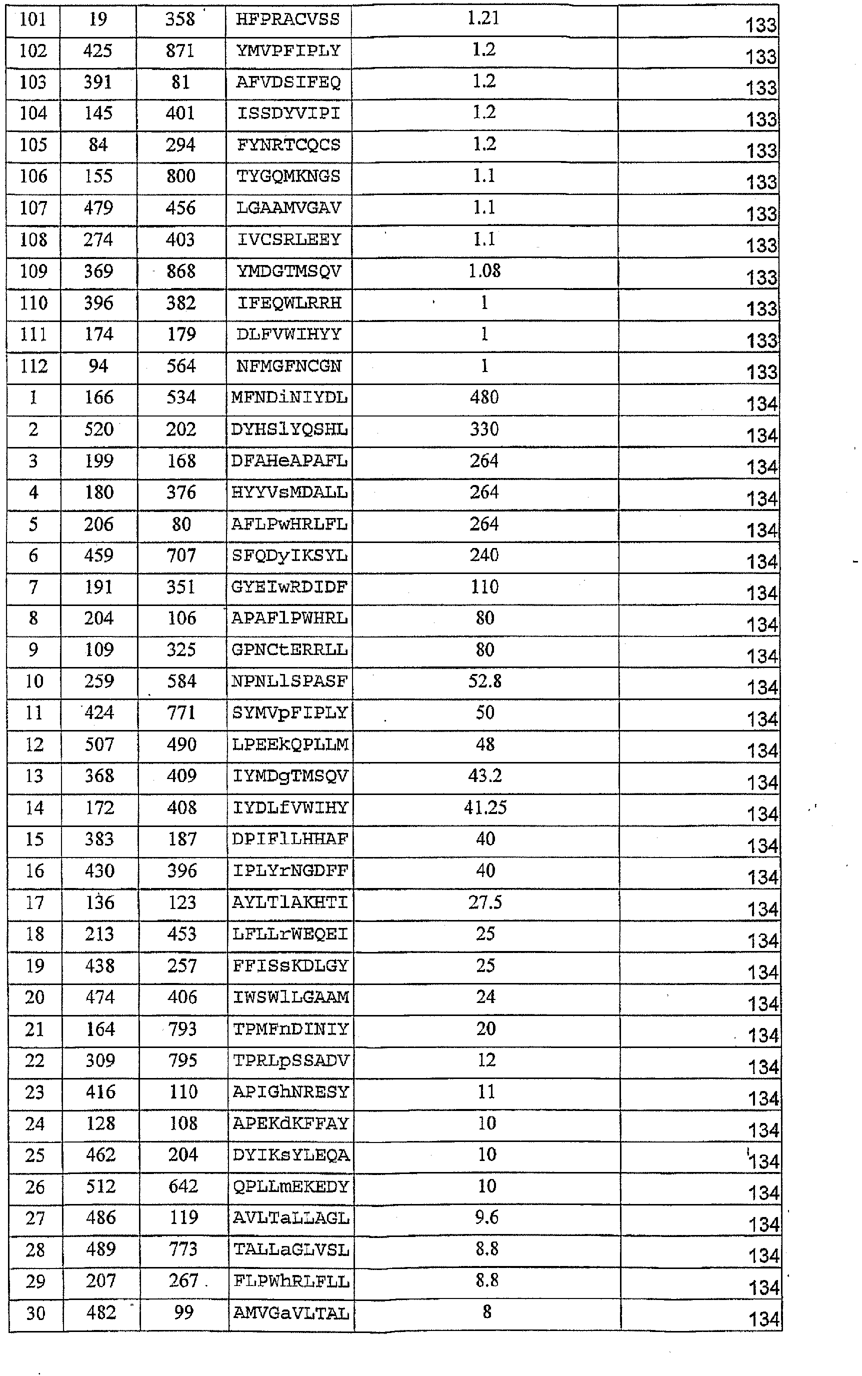

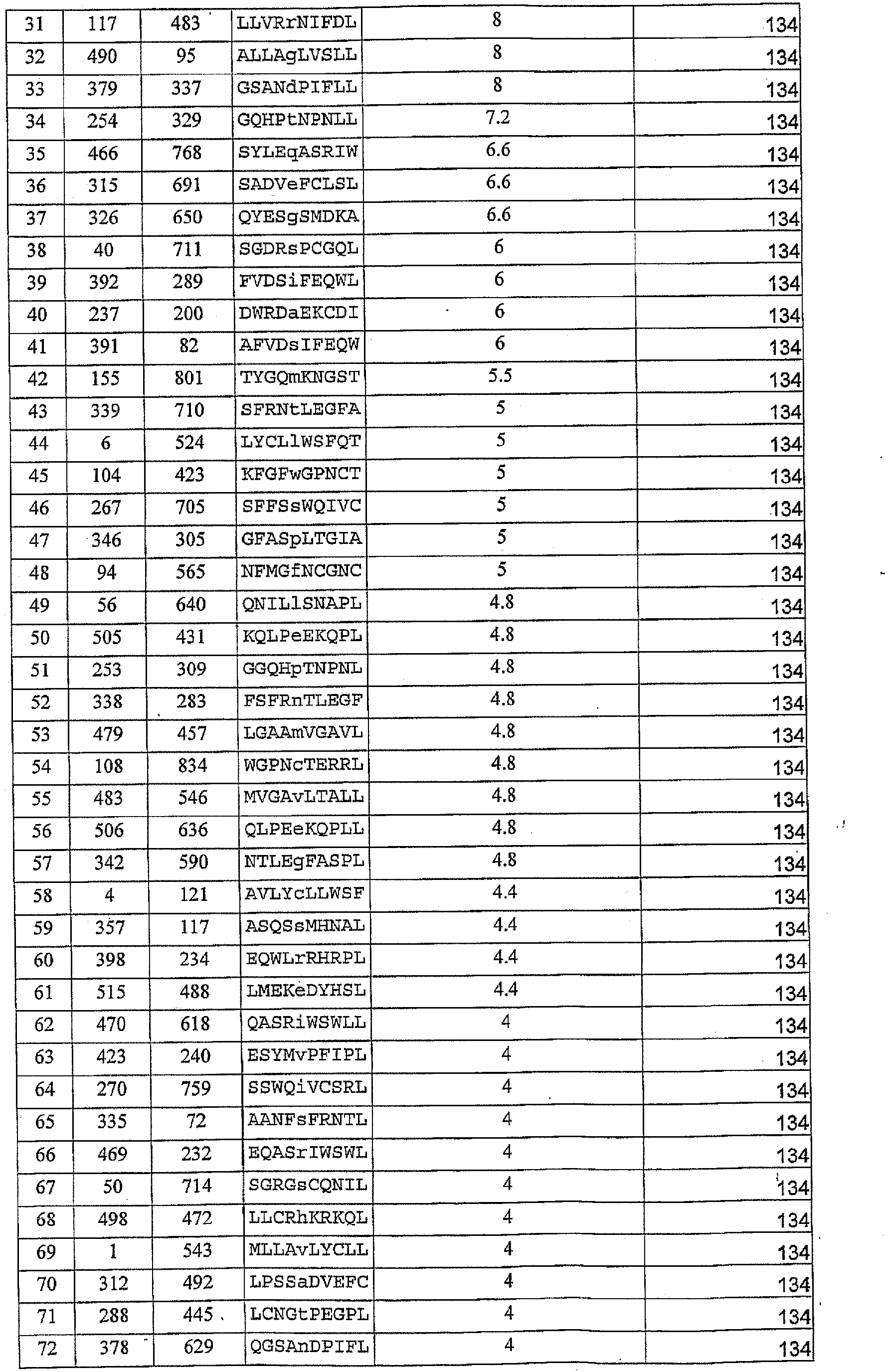

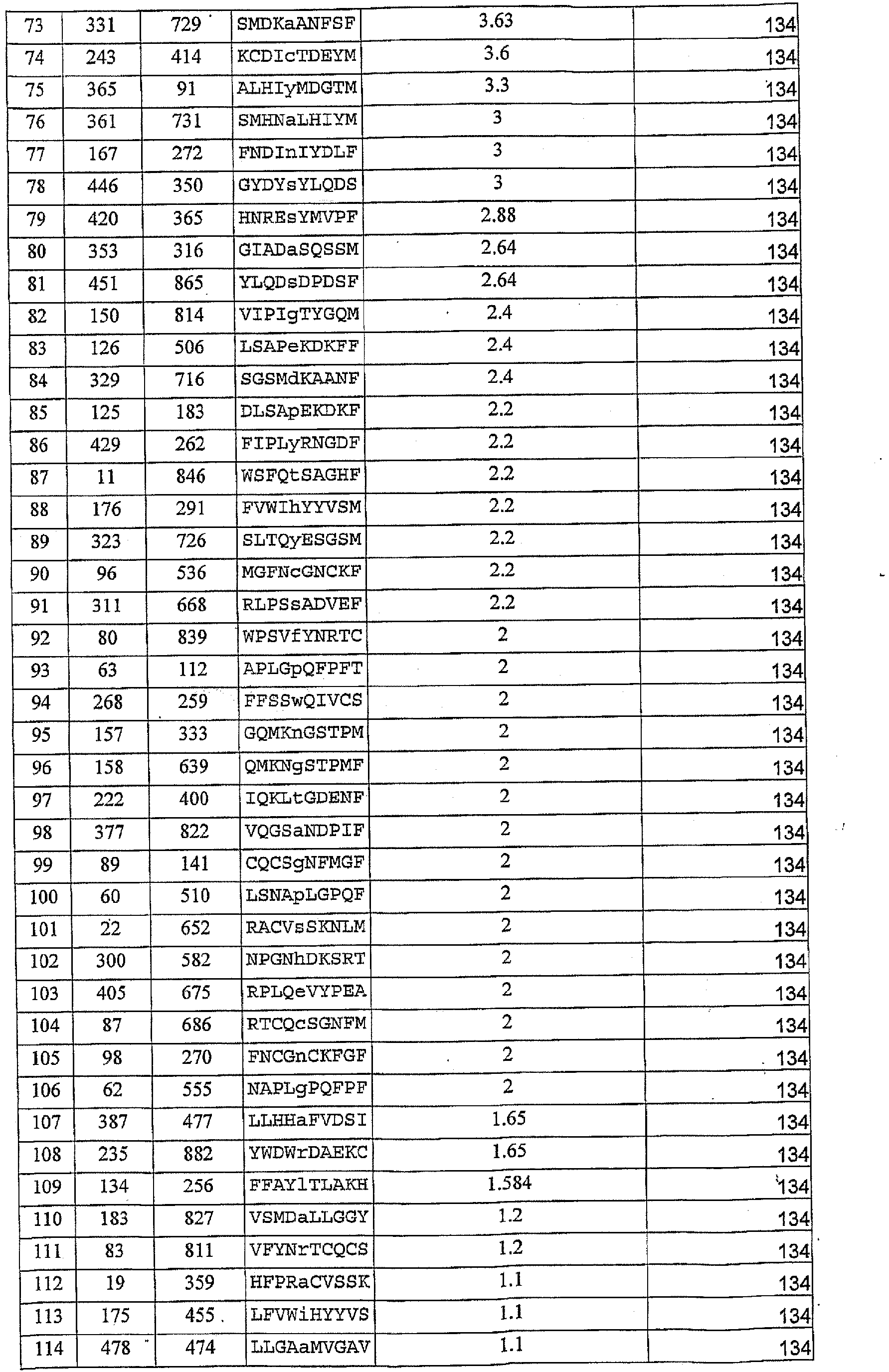

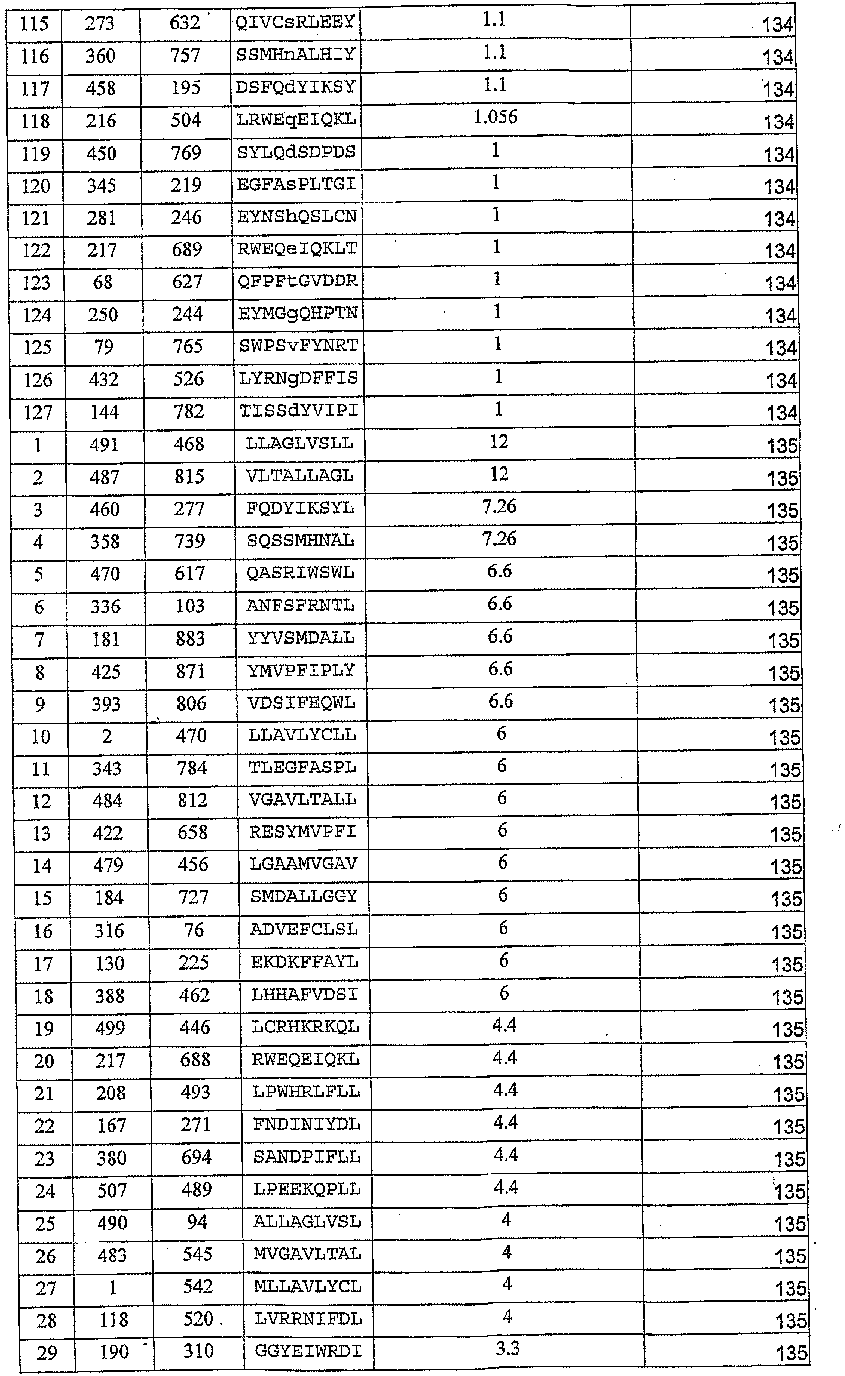

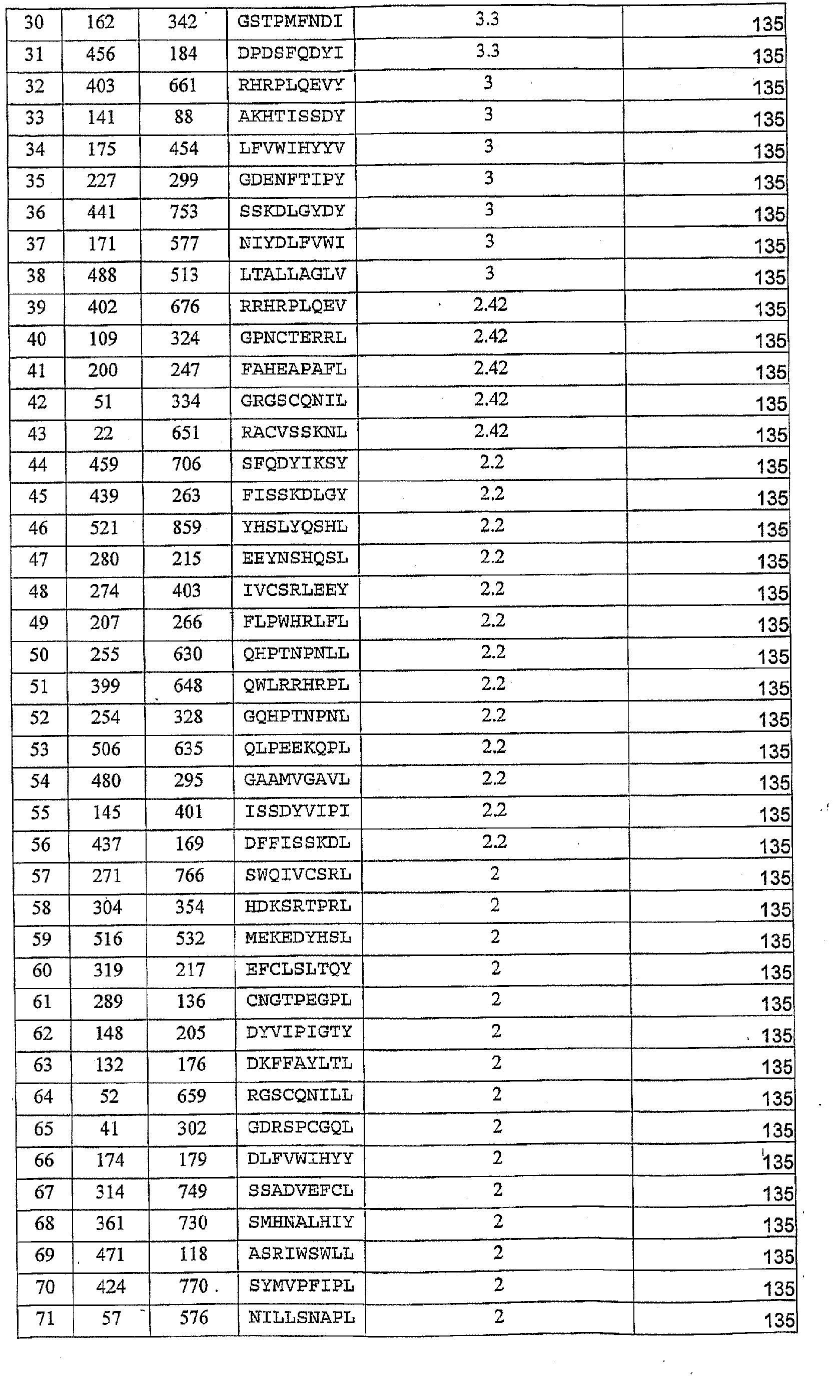

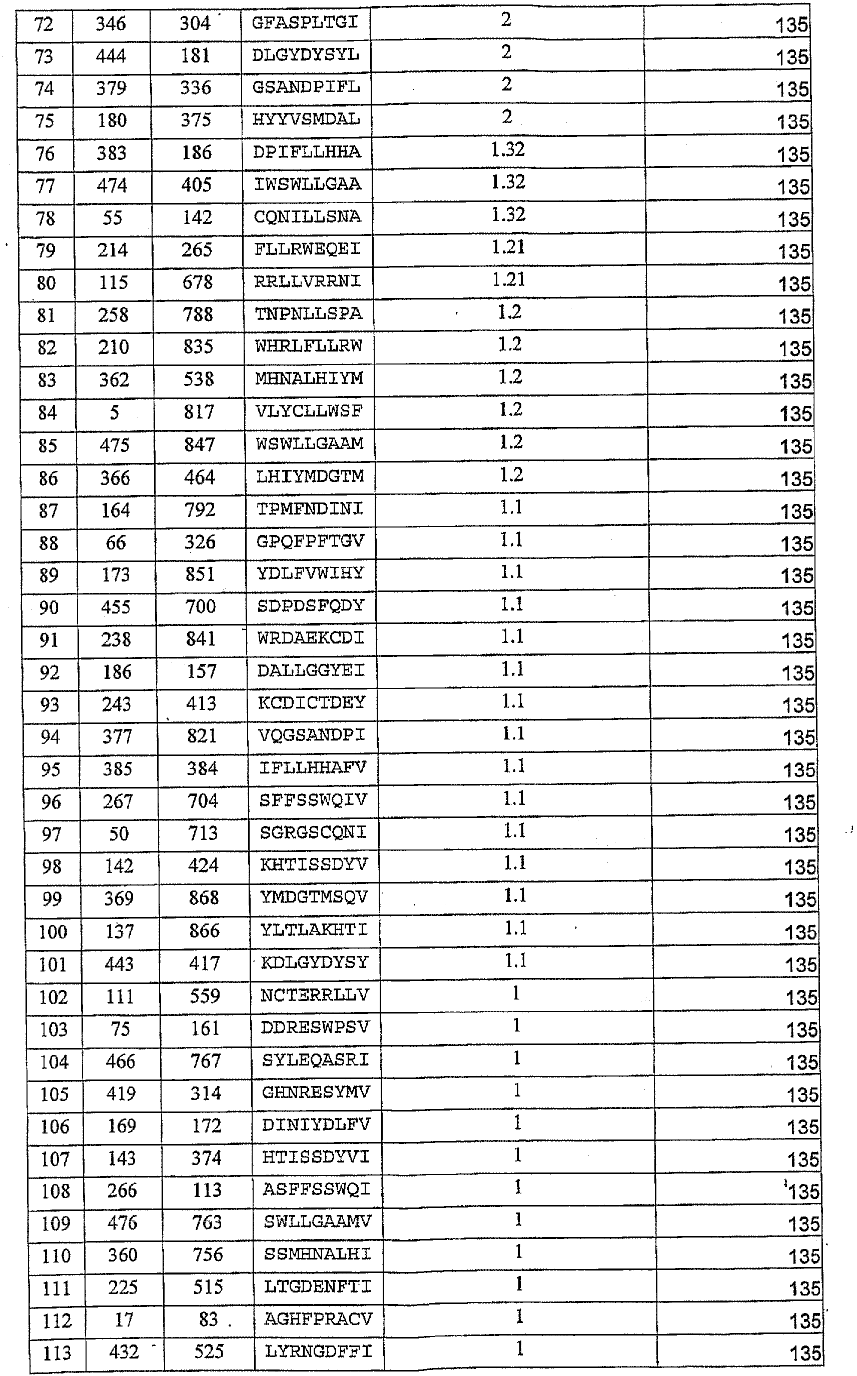

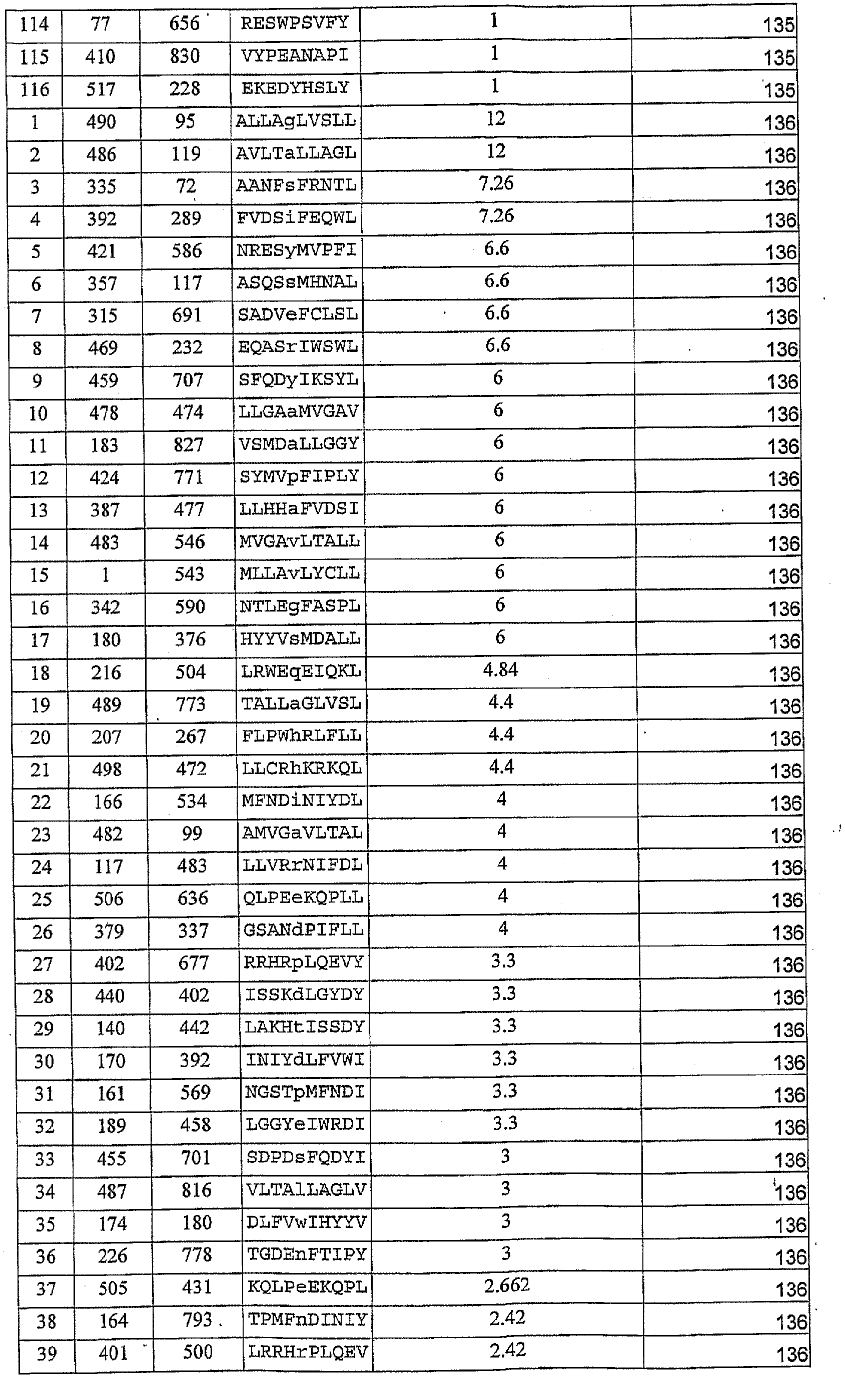

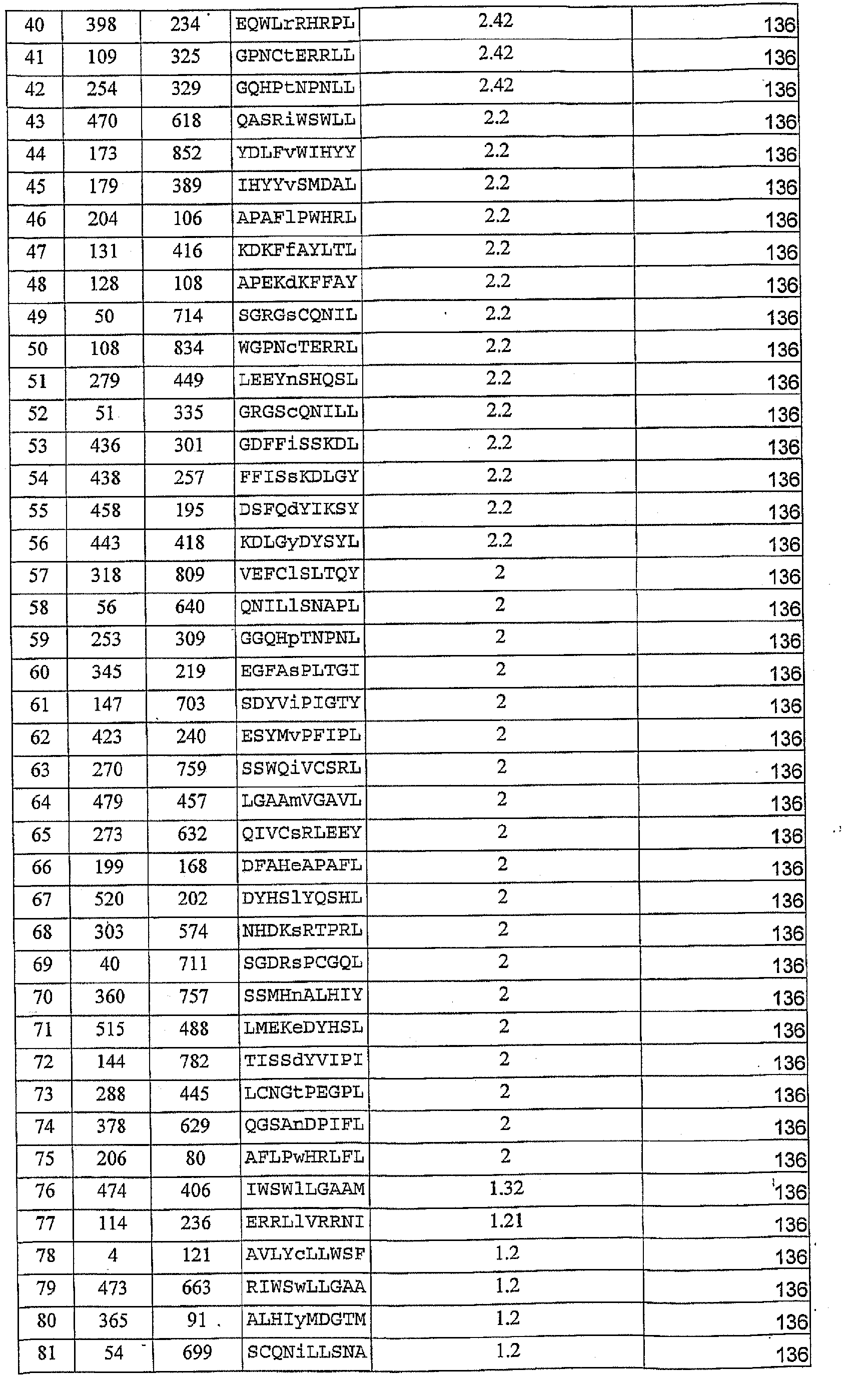

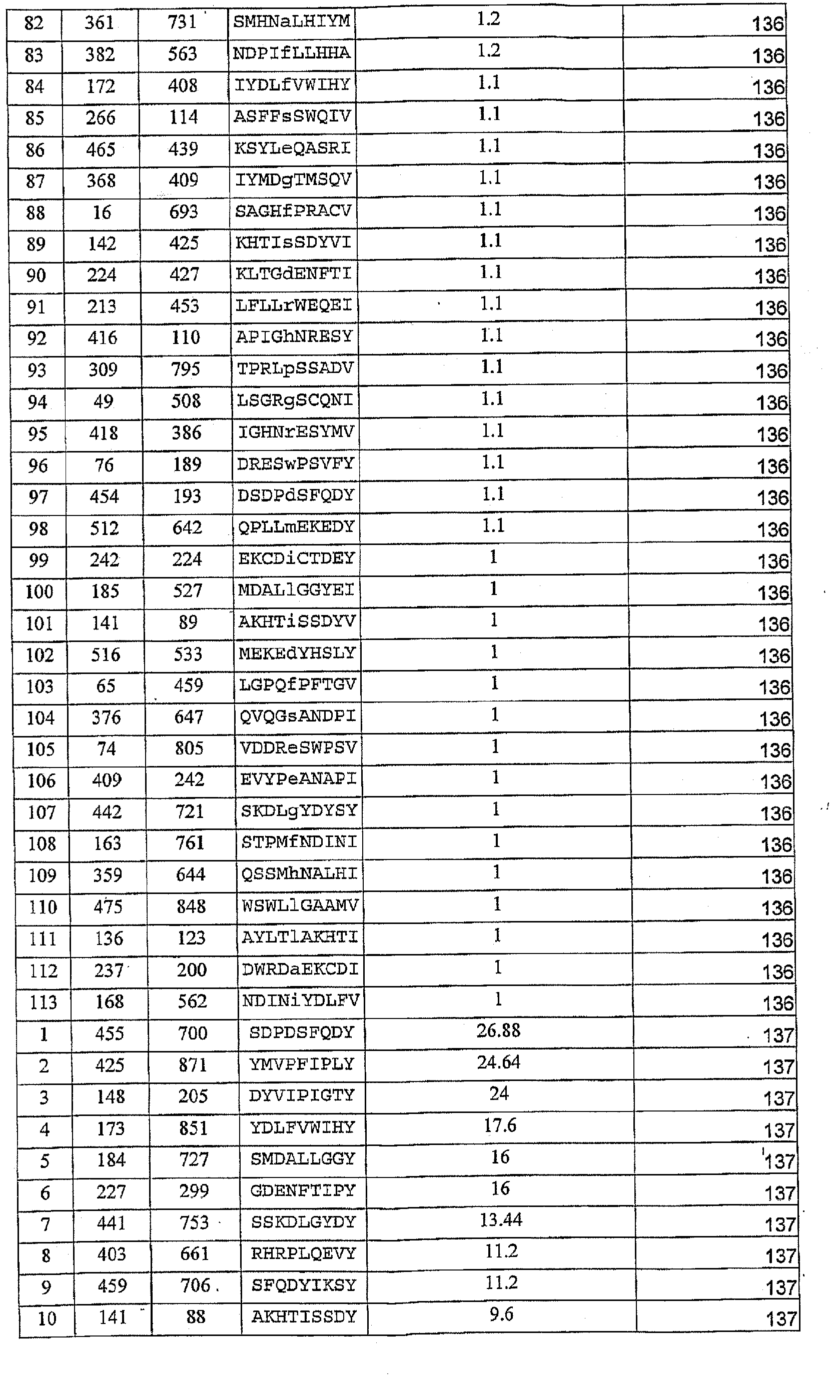

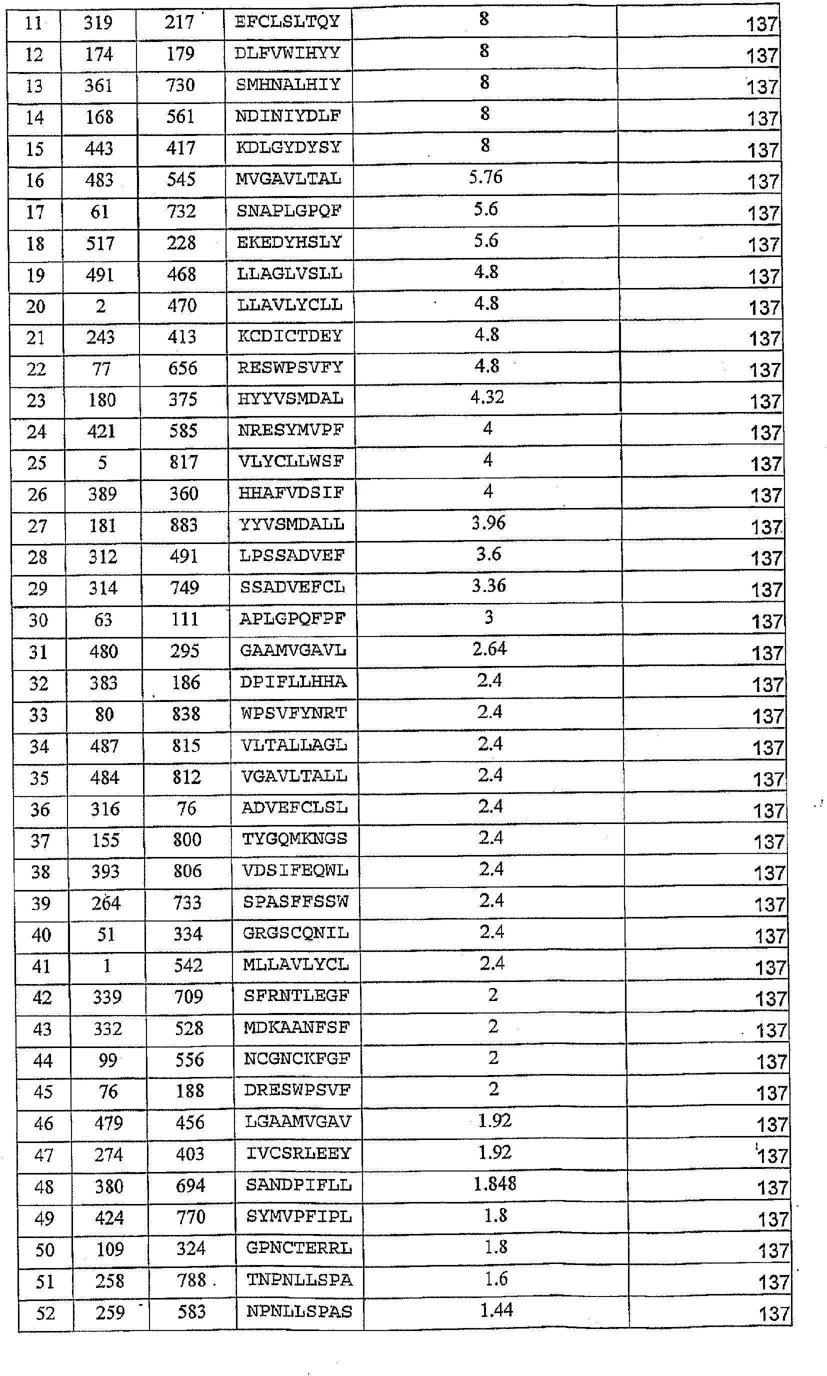

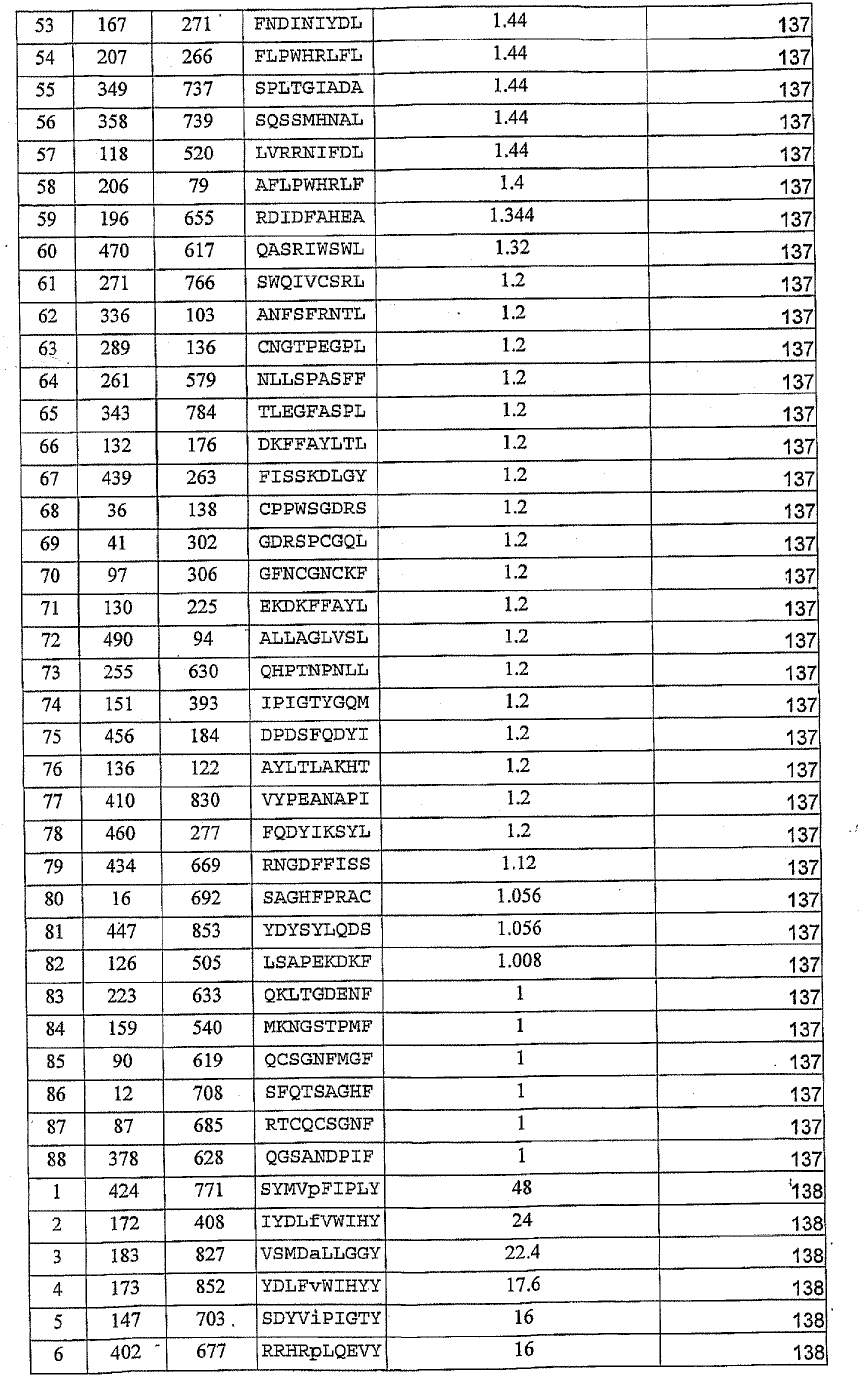

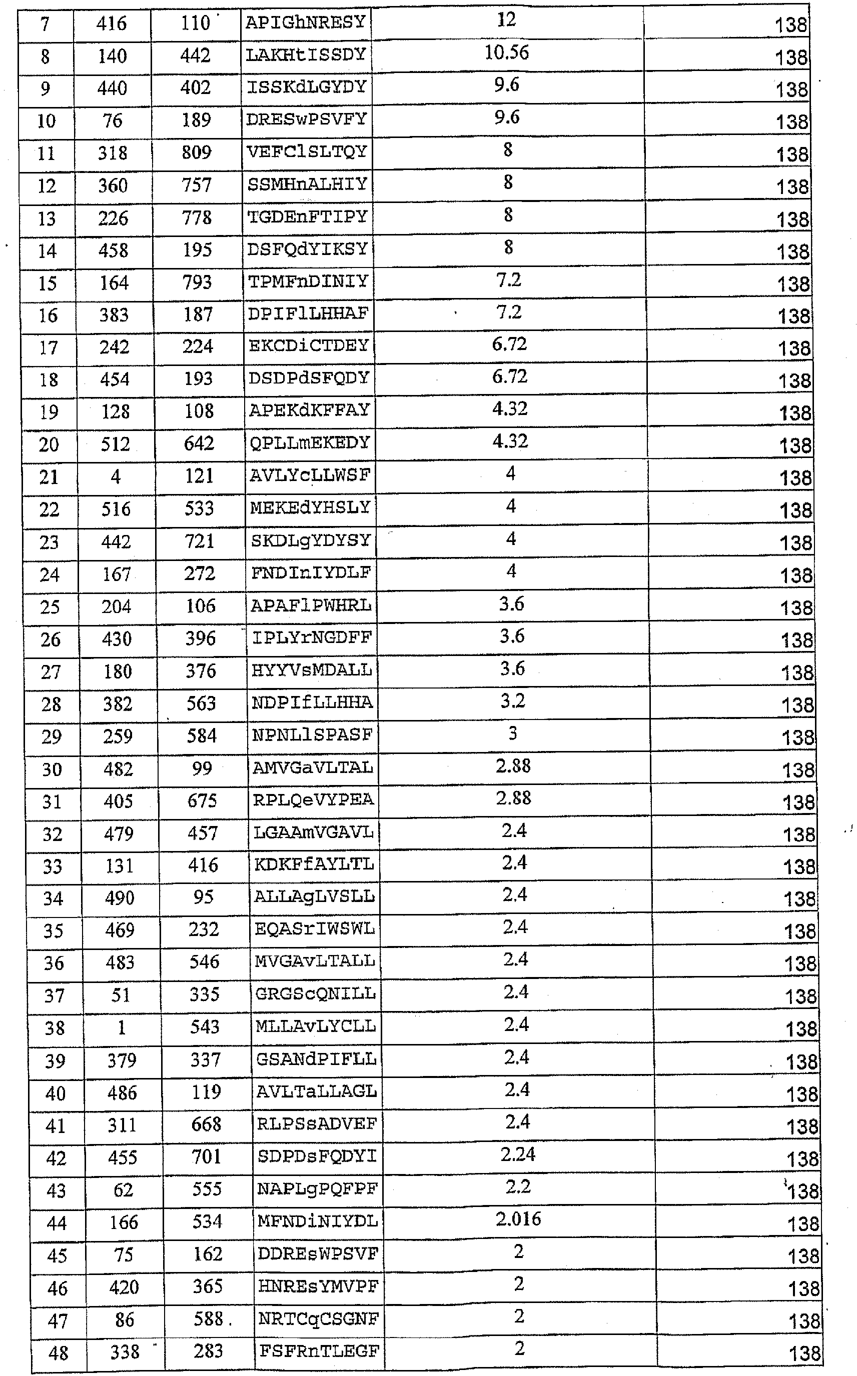

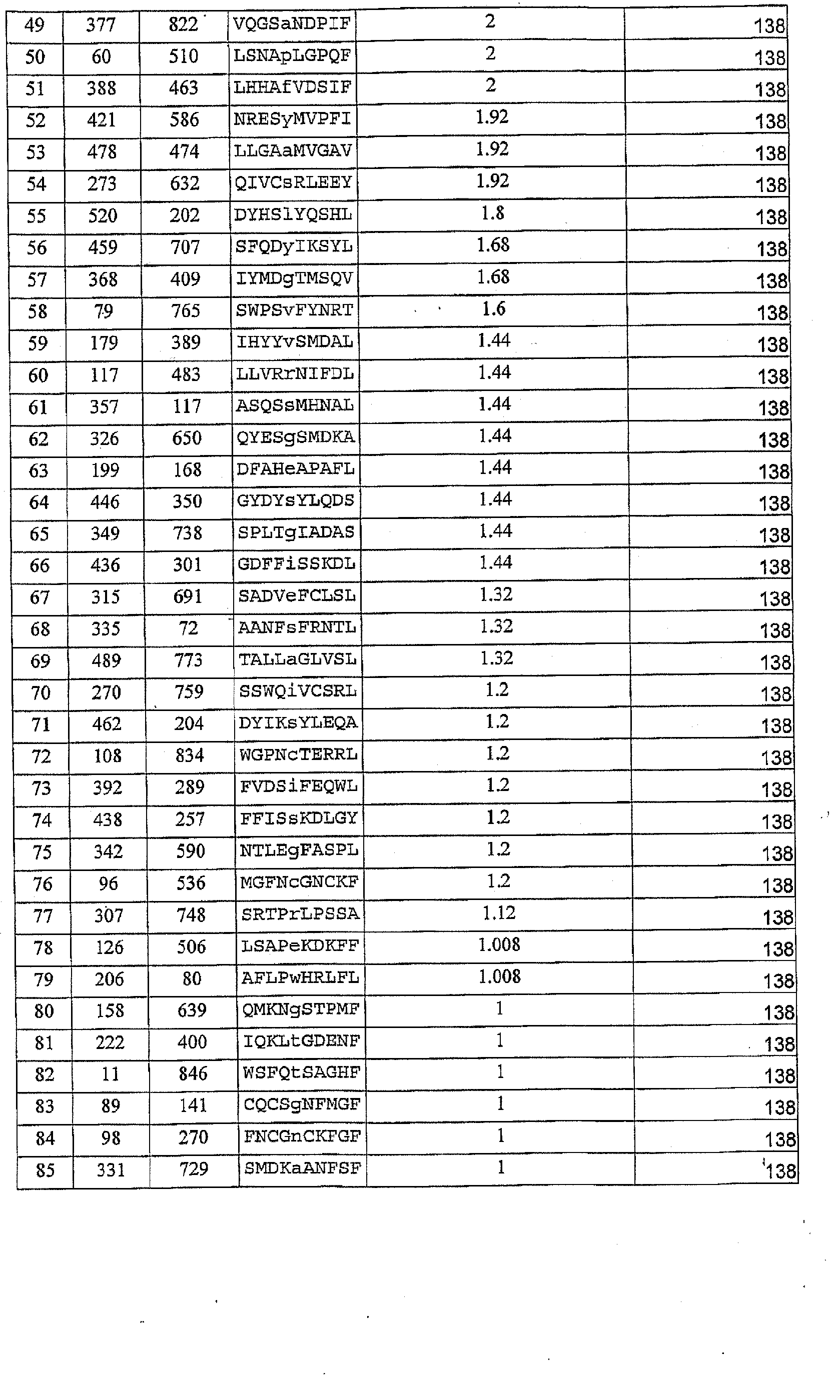

### EXAMPLE 12

### GENERATION OF ADDITIONAL ANTIBODIES WITH A SPECIFICITY TO THE

### HLA-A2/TYROSINASE₃₆₉₋₃₇₇ COMPLEX

### MATERIALS AND METHODS

***Production of Biotinylated scMHC*/*peptide Complexes:* To construct scMHC-**BirA plasmid, a peptide sequence for site specific biotinylation (LHHILDAQ**K**MVWNHR, (SEQ ID NO: 900) the lysine residue undergoing biotinylation by the BirA biotin ligase enzyme is marked) was fused at the C-terminus of the HLA-A2. These construct was subcloned into a pET-based expression vector for efficient expression in *E. Coli.*

***Folding and Purification of recombinant MHC*/*peptide complexes, and recombinant fusion molecule:*** Dithioerithriol was added to a final concentration of 65 mM (10 mg/ml) to the solubilized inclusion bodies of scMHC, or fusion molecule which were incubated for >2 hours. The reduced inclusion bodies were diluted x100 with refolding buffer (0.1M Tris-HCl pH=8, 0.5M Arginine, 0.09mM Oxidized Glutathione, 2mM EDTA, 0.2mM PMSF). 5 or 10 fold molar excess of peptide (usually 1 mg / 100 ml refolding buffer) was added to scMHC and the molecule containing HLA-A2, previously diluted in H₂O or DMSO, and incubated at 4-10 °C for 48 hours. After refolding, the protein was dialyzed against 100 mM Urea, 20 mM Tris-HCl pH=8, and concentrated by a Minisette system using a 10K cutoff cassette to a final volume of 200 ml. The protein was loaded on Q Sepharose anion exchange column. The column was washed with buffer A containing 5 mM NaCl, 20 mM Tris HCl pH=8, 1 mM EDTA. Relevant fractions corresponding to correctly folded MHC/peptide or fusion molecule monomers were poured on to a centricon device (30 kDa cut off) (Amicon, Beverly MA) and concentrated to volume 0.3-1.0 ml (usually no more than 2 mg/ml to avoid protein agregation).

The clean fractions were frozen at -70 °C at this step, until further use.

***Biotinylation of MHC*/*peptide complexes:*** The buffer was exchanged (using the centricon) with 10 mM Tris-HCl, pH=8, 50 mM NaCl. The final protein concentration was brought to 1-2 mg/ml (25-50 µM). Enzymatic biotinylation was performed at a specific lysine residue in the heavy chain C-terminal tag using biotin protein ligase - Bir A enzyme (AVIDITY, Denver, CO) for 16 hours at 25 °C, in presence of protease inhibitors cocktail (0.1 mM PMSF, 1 µg/ml Leupeptin, 1 µg/ml Pepstatin). The buffer was exchanged and the excess biotin was removed from the biotinylated complexes using centricon 30 ultrafiltration or G-25. The MHC/peptide biotinylated monomers were frozen at -70°C.

***Selection of Phage-Antibodies on Biotinylated Complexes:*** A large human Fab library containing 3.7 x 10¹⁰ different Fab clones was used for the selection [de Haard, H. J., et al., (1999) J.Biol.Chem. 274, 18218-18230]. Phage (10¹³) was first preincubated for 1 hour at room temperature in PBS containing 2 % nonfat dry milk with streptavidin-coated paramagnetic beads (200µl; Dynal, Oslo) to deplete streptavidin binders. Streptavidin-coated paramagnetic beads (200µl; Dynal, Oslo) were also incubated in PBS+2 % milk for 1hour at room temperature. The remaining phages were subsequently incubated for 1 hour with decreasing amounts of biotinylated scMHC-peptide complexes. Streptavidin magnetic beads were added, and the mixture was incubated for 15 minutes with continuous rotation. A magnetic force was applied to pull down phages bound to biotinylated complexes. After 10 washes of the streptavidin-bound complexes with MPBS 0.1 % Tween and 2 washes with PBS, bound phages were eluted by incubation for 7 minutes with 1 ml of Triethylamine (TEA) (100 mM). In this procedure, 3-4 rounds of selection were performed, the instant paragraph describing only one round of selection. In the first round 20 µg biotinylated scMHC-peptide complexe were used, and in the following rounds 5µg.

A second protocol of Off-Rate selection was assessed and included 42 washes of the streptavidin-bound complexes with MPBS 0.1 % Tween and 2 washes with PBS each time for 15 minutes. The elution mixture was neutralized by the addition of 100 µl of Tris-HCl (1M, pH 7.4) and used to infect *E.coli* TG1 cells (OD600=0.5) for 30 minutes at 37 °C. Selected phages were rescued using M13KO7 helper phage (5x10¹¹ cfu).

For both selection protocols, the diversity of the selected antibodies was determined by DNA fingerprinting. The Fab DNA of different clones was PCR-amplified using the primers pUC-reverse (5'-AGCGGATAACAATTTCACACAGG-3' - SEQ ID NO: 901) and fd-tet-seq24 (5'-TTTGTCGTCTTTCCAGACGTTAGT-3' - SEQ ID NO: 902). The resulting PCR fragments were digested with BstNI (NEB) (2 hours, 37 °C) and analyzed by agarose gel electrophoresis.

***Cell lines:*** JY (EBV-transformed B-lymphoblast), were maintained in RPMI-1640 supplemented with 10 % FCS, 2 mM glutamine, Penicillin (100 units/ml) and Streptomycin (100 µg/ml) at 37 °C with 5 % CO₂. The melanoma, leukemia, and glioblastoma cell lines were maintained in RPMI-1640 supplemented with 10 % FCS, 2 mM glutamine, Penicillin (100 units/ml) and Streptomycin (100 µg/ml) at 37 °C with 5 % CO₂.

***Expression and purification of soluble recombinant Fab antibodies:*** The Fab antibody was expressed in BL21 λDE3 cells as previously described (1) and purified from the periplasmic fraction by metal-ion affinity chromatography using the hexahistidine tag: 4 µl of miniprep DNA was transformed to 100 µl BL 21 *E*. *coli* competent cells and plated the bacteria on 2YT/A/G agar plates and incubated at 37 °C, over night. Inoculated plates into Superbroth supplemented with 12ml/liter 40gr/liter MgSO₄, 5ml/liter 20 % Glucose, and 100 µg/ml Ampicillin. For each liter of Superbroth, 5 plates were used (filled with colonies) which were grown to OD600nm=0.8-1.0 and induced to express the recombinant Fab antibody by the addition of 1mM IPTG for 3 hours at 30 °C. The cells were centrifuged and the pellet was resuspended in 5 ml of a B-PER solution (Pierce) to release periplasmatic content. After 30 minutes of rotated incubation at RT, the solution was centrifuged (15000 rpm, 15 minutes) and the supernatant was incubated with 0.5 ml of prewashed TALON beads suspension (Clontech) for 45 minutes at RT. The solution was applied onto a BioRad disposable column (Cat No. 731/1550), and after sedimentation the beads were washed three times with 10 ml of PBS/0.1 % Tween20 (pH 8.0). The bound Fabs were eluted using 0.5ml of 100 mM Imidazole in PBS. The eluted Fabs were dialyzed twice against PBS (overnight, 4 °C) to remove residual imidazole. The homogeneity and purity of the purified Fabs was determined by analysis on non-reduced and reduced SDS-PAGE.

***ELISA with purified Fab antibodies:*** The binding specificity of individual soluble Fab fragments was determined by ELISA using biotinylated scMHC-peptide complexes. ELISA plates (Falcon) were coated overnight with BSA-biotin (1µg/well). After having been washed, the plates were incubated (1 hour, RT) with streptavidin (1µg/well), washed extensively and further incubated (1 hour, RT) with 0.5 µg of MHC/peptide complexes. Plates were blocked for 30 minutes at RT with PBS 2 % BSA and subsequently were incubated for 1 hour at RT with various concentrations of soluble purified Fab, and after washing, with 1:1000 HRP-conjugated/anti-human antibody. Detection was performed using TMB reagent (Sigma). The HLA-A2-restricted peptides used for specificity studies of the purified Fab antibodies were: the Human Tyrosinase peptide369-377 (YMDGTMSQV - SEQ ID NO: 1) and non-specific peptides MART1 (ELAGIGILTV - SEQ ID NO: 27).

***Flow Cytometry:*** The EBV-transformed B-lymphoblast JY cells or virus infected cells as indicated were used to determine the reactivity of the recombinant TCRs with cell surface-expressed HLA-A2/peptide complexes About 10⁶ JY cells were washed with serum-free RPMI and incubated overnight at 37 °C in medium containing 100 µM of the peptide. The cells were incubated for 60 minutes at 4 °C with recombinant Fab antibodies or Fab-PE (10 -100 µg/ml) in 100 µl. After three washes the cells were incubated with rabbit anti-PE polyclonal antibody or followed by washing twice with PBS and incubated for 60 minutes with FITC labeled anti-rabbit IgG(for Fab-PE) or with FITC-labeled anti-human Fab or with PE-labeled anti-human Fab (Jackson) (for Fab Abs). After a final wash, the cells were resuspended in ice-cold PBS.

The melanoma, leukemia, and glioblastoma HLA-A2⁺/hla-a2⁻ Tyr⁺/tyr⁻ positive cell lines were incubated for 60 minutes at 4 °C with recombinant Fab antibodies or Fab-PE (10 -100 µg/ml) in 100 µl. After three washes the cells were incubated with rabbit anti-PE polyclonal antibody followed by washing twice with PBS and incubated for 60 minutes with FITC labeled anti rabbit IgG (for Fab-PE) or with FITC-labeled anti-human Fab or with PE- labeled anti-human Fab (Jackson) (for Fab Abs). After a final wash, the cells were resuspended in ice-cold PBS.

***Binding Affinity determination:*** A SPR real-time kinetic interaction analysis system (BioRad Israel) was used to determine association (kon) and dissociation (koff) constants of the TA2, B2 (SEQ ID NOs; 892 and 893) and MC1 (SEQ ID NOs: 884 and 885) Fab antibodies. A biosensor chip (BioRad, Inc.) was activated according to the manufacturer's instructions and coupled with 86-300 RUs (response units) of Fab anti Fab in 10 mM sodium acetate (pH 4.5). Unreacted groups were blocked with 1 M ethanolamine. The kinetics of the Fabs binding to complex were measured with serial dilutions beginning with 1000 nM to 62.5 nM complex in running buffer (PBS, 0.05% (v/v) Tween, 0.01%). Binding measurements were recorded at 25 °C. Data were fit to a 1:1 Langmuir binding model using Biorad evaluation software, which calculated kon koff rates. An equilibrium constant, KD, was calculated from koff/kon.

*Expression and purification of Fab-PE38 fusion protein:* The genes encoding the light and heavy chain of Fab TA2, MC1 were cloned separately into a T7-promotor pET-based expression vector Puli9 (KDEL version). The heavy chain gene was engineered to contain the PE38 recognition sequence at the COOH terminus (heavy-PE38). The genes encoding the variable heavy and light chain of TA2, MC1 Fab were cloned as scFv-PE-38 into a T7-promotor pET-based expression vector Puli9. (V_{H}-V_{L}-PE38).

These constructs were expressed separately in the Fab version or as one construct in the scFv version in *E. coli* BL21 cells and upon induction with IPTG, intracellular inclusion bodies which contain large amounts of the recombinant protein accumulated. Inclusion bodies of both chains of the Fab and the scFv construct were purified, reduced, and subsequently refolded, using a heavy PE38:light ratio of 2.5:1 for the Fab version, in a redox-shuffling buffer system containing 0.1 M Tris, 0.5 M Arginine, 0.09 mM Oxidized Glutathione (pH 7.4). Correctly folded Fab and scFv were then isolated and purified by ion-exchange chromatography Sepharose and MonoQ (Pharmacia).

***Cytotoxicity assays:*** Melanoma cells were incubated with increasing concentrations of immunotoxin and the inhibition of protein synthesis was determined by measuring the uptake of ³H-Leucine into cellular proteins, as previously described [Reiter Y, et al., (1994) Int.J.Cancer 58, 142-149]. IC₅₀ was determined as the concentration of immunotoxin required to inhibit protein synthesis by 50 %.

### RESULTS

***Selection of TCR-like recombinant antibodies recognizing HLA-A2*/*Tyr₃₆₉₋₃₇₇:*** Recombinant peptide-HLA-A2 complexes that present the *Tyr₃₆₉₋₃₇₇* Tyr derived peptide were generated using a scMHC construct that was described previously [Denkberg, G., Cohen, C. J., Segal, D., Kirkin, A. F., and Reiter, Y. (2000) Eur.J.Immunol. 30, 3522-3532]. In this construct, the extracellular domains of HLA-A2 are connected into a single chain molecule with ß2m using a 15-aa flexible linker. The sc-MHC-peptide complexes were produced by in vitro refolding of inclusion bodies in the presence of the Tyr-derived *Tyr₃₆₉₋₃₇₇* peptide. The refolded scHLA-A2/Tyr complexes were found to be very pure, homogenous, and monomeric by SDS-PAGE and size exclusion chromatography analyses (data not shown). Recombinant scMHC-peptide complexes generated by this strategy had been previously characterized in detail for their biochemical, biophysical, and biological properties, and were found to be correctly folded and functional [Denkberg, G., Cohen, C. J., Segal, D., Kirkin, A. F., and Reiter, Y. (2000) Eur.J.Immunol. 30, 3522-3532].

For selection of TCR-like Abs, a large Ab phage library was used, consisting of a repertoire of 3.7 X10¹⁰ independent, human recombinant Fab clones. Initially, the library was depleted of streptavidin binders and subsequently subjected to selection in solution using soluble recombinant biotinylated scHLA-A2-peptide complexes containing the *Tyr₃₆₉₋₃₇₇* peptide. In the conventional selection method, a 133-fold enrichment in phage titer was observed after three rounds of selection. In the off-rate selection the clones were picked after 2-rounds of selection where a 10-fold decrease in overall phage titer was observed between the second round and the first.

The specifcity of the selected phage Abs was determined by a differential ELISA analysis on streptavidin-coated wells incubated with biotinylated scMHC HLA-A2 complexes containing either the *Tyr₃₆₉₋₃₇₇* peptide or control complexes containing other HLA-A2-restricted peptides. Phage clones analyzed after the third round of selection in the conventional screening method exhibited two types of binding patterns toward the scHLA-A2- peptide complex. One class of Abs consisted of pan-MHC binders that could not differentiate between the various MHC-peptide complexes; the second type consisted of Abs that bound the MHC peptide complex in a peptide-specific manner. The ELISA screen revealed that 27 of 94 randomly selected clones screened (28 %) from the third round of panning appeared to be fully peptide dependent and specific for the peptide/MHC used in the selection (i.e., the scHLA-A2/Tyr complex). A representative monoclonal ELISA analysis of TCR-like Fab clones is shown in Figure 40a. The diversity within the selected TCR-like Fabs was assessed by DNA fingerprint analysis; 5 different antibodies with TCR-like specificity were revealed, indicating the selection of several different Abs with TCR-like specificity.

The specifcity of the selected phage Abs isolated from the Off -Rate selection was determined by a differential ELISA analysis after the second round of selection on streptavidin-coated wells incubated with biotinylated scMHC HLA-A2 complexes containing either the *Tyr₃₆₉₋₃₇₇* peptide or control complexes containing other HLA-A2-restricted peptides. Phage clones analyzed after the seconed round of selection exhibited the same two types of binding patterns toward the scHLA-A2- peptide complex. One class of Abs consisted of pan-MHC binders that could not differentiate between the various MHC-peptide complexes; the second type consisted of Abs that bound the MHC-peptide complex in a peptide-specific manner. The ELISA screen revealed that 1 of 94 randomly selected clones screened (1 %) from the seconed round of panning appeared to be fully peptide dependent and specific for the peptide/MHC used in the selection (i.e., the scHLA-A2/Tyr complex).

A representative monoclonal ELISA analysis of TCR-like Fab clones isolated from the off-rate selection is shown in Figure 40b. The diversity within the selected TCR-like Fab was assessed by DNA fingerprint analysis; 1 additional antibody with TCR-like specificity was revealed.

***Characterization of Recombinant Soluble Fab Antibodies with TCR-Like Specificity.*** Of the five Fab clones recognizing the HLA-A2―Tyr*₃₆₉₋₃₇₇* complex obtained by the conventional selection method, the one that exhibited the most specific peptide-dependent and TCR-like binding pattern as analyzed by the phage ELISAs, and the one positive clone from the off-rate selsection, were subjected to further analysis.

The Fab clone-MC1 (from the conventional selection method) and B2 (from the off-rate selection) were sequenced and produced in a soluble form in *E. coli* TG1 or BL21 cells and were purified by immobilized metal ion affinity chromatography (IMAC). Yields were 2-4 mg of pure material from 1 liter of bacterial culture. SDS_PAGE analysis revealed a homogenous and pure population of Fabs with the expected molecular size (data not shown).

MC1 and B2 Fabs were sequenced. The B2 heavy chain belongs to subgroup III of VH and the light chain belongs to the human kappa subgroup II family. The MC1 heavy chain belongs to subgroup II of VH and the light chain belongs to the human kappa subgroup II family (Figures 41a-d).

The binding affinity of the purified Fab fragments MC1, B2, and TA2 that was previously isolated was determined by SPR analysis. Surface plasmon resonance (SPR) was used to measure the binding kinetics of Fabs to HLA-A2/Tyr complex on a Biorad instrument (as descriebed in material and methods). Kinetics data for Fabs binding to HLA-A2/Tyr complex at 25 °C temperature are shown in Figures 41e-g. The Fab variants generally had very similar association constants but different dissociation rate constants, kₒₙ and k_{off} respectively. As a result, the mutants have different KD values of 1000 nM (TA2), 70 nM (B2) and 4 nM (MC1). The new screening methods revealed two Fab clones with improved affinity as compared to the TA2. B2 Fab had improved KD with an improvement over TA2 of about 10 fold. This improvement resulted primarily from a slower dissociation rate constant, k_{off} (Table 140, herein below, representing a mean of five experiments). MC1 Fab had improved KD with an improvement over TA2 of about 100 fold. This improvement resulted primarily from a slower dissociation rate constant, k_{off} (Table 140 herein below).

**Table 140**

| | ***ka*** | ***kd*** | ***KD*** | ***Rmax*** |
|---|---|---|---|---|
| Antibody | 1/Ms | 1/s | M | RU |
| TA2 | 2.37E+05 | 0.24 | 1.02E-06 (1000 nM) | 317.63 |
| B2 | 7.45E+04 | 4.98E-03 | 6.69E-08 (70 nM) | 174.65 |
| MC1 | 1.32E+05 | 5.47E-04 | 4.15E-09 (4 nM) | 231.22 |

***Binding of TA2, B2, MC1Fab Antibodies to the surface of melanoma cells:*** To explore whether the HLA-A2/Tyrosinase TCR-like Fab Abs have different binding pattern to endogenously derived MHC-Tyrosinase complexes on the surface of tumor cells due to their different affinites, flow cytometry analysis was performed on lines derived from melanoma, leukemia and glioblastoma patients. Cells were incubated with TA2, B2, MC1 anti-Tyrosinase 369-377 Ab in different concentrations followed by incubation with PE-labeled anti-human antibody. As shown in Figures 42a-k, 43a-k and 44a-k, the MC1, B2, TA2 Fab respectively recognized Tyrosinase positive and HLA-A2 positive cells with a very high intensity corresponding to their improved affinity. The detection limit of TA2 was 10 µg/ml on positive cells while B2 had detection limit of 1 µg/ml on positive cells and MC1 reacted in 0.1 µg/ml on positive cells (Figure 45a-d).

***Binding of TA2, MC1 Fab-PE38 melanoma cells:*** To demonstrate that purified TA2 and MC1 Fab-PE38 can bind the specific MHC-peptide complex as expressed on the cell surface of Tyr-expressing tumor cells, these cells were incubated with the fusion proteins and were tested by monitoring the reactivity of anti-PE38 antibodies. As shown in Figure 46a-d, the TA2 and MC1 Fab-toxin fusion proteins reacted specifically with HLA-A2 positive and Tyr positive 501A melanoma cells, but not with 1938 melanoma cells which express HLA-A2 but are Tyr negative.

These results demonstrate that the TA2 and MC1 Fab/scFv-PE38 retains their specificity to Tyr/HLA-A2 peptide complexes expressed on the surface of cells.

***Cytotoxicity of TA2 and MC1 Fab- or scFv-PE38 towards tumor cells displaying the Tyr derived epitopes:*** The ability of the TA2 and MC1 Fab- or scFv-PE38 (KDEL version) to inhibit protein synthesis was used as a measure to test the specificity and biological activity of the TCR-like TA2 and MC1 Fab or scFv fusion molecules. Because the cell binding domain in the toxin was deleted, cytotoxicity induced by internalization of the Fab or scFv toxin fusion molecules reflects antigen-specific binding. To test this activity, HLA-A2 and Tyr positive 501A and 624.38 melanoma cells were incubated with increasing concentrations of the TA2 and MC1 Fab- or scFv-fusion proteins, and protein synthesis was tested by measuring incorporation of [3H]-leucine into cellular proteins. As controls HLA-A2 positive and Tyr negative 1938 melanoma cells were used.

As shown in Figures 47a-d, the TA2 and MC1 Fab- or scFv-PE38 fusions inhibited (in a dose dependent manner) protein synthesis and were cytotoxic to 501A and 624.38 cells but not to control 1938 cells. The cytotoxic activity correlated the reactivity of the TA2 and MC1 TCR-like Fab; 501A cells reacted well with the antibodies and were killed more efficiently by the MC1 as compared to TA2.

A 100-fold increase in the IC50 of the MC1 Fab-PE38 and a 10-fold increase in the IC50 of the MC1 scFv-PE was observed compered to TA2 Fab/scFv -PE38 resulting from their higher affinity.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications and GenBank Accession numbers mentioned in this specification are herein incorporated in their entirety by reference into the specification, to the same extent as if each individual publication, patent or patent application or GenBank Accession number was specifically and individually indicated to be incorporated herein by reference. In addition, citation or identification of any reference in this application shall not be construed as an admission that such reference is available as prior art to the present invention.

### REFERENCES

### (Additional references are cited in text)

1. I. Komenaka, H. Hoerig, and H. L. Kaufman, "Immunotherapy for melanoma," Clin. Dermatol. 22(3), 251 (2004).
2. G. Parmiani, et al., "Immunotherapy of melanoma," Semin. Cancer Biol. 13(6), 391 (2003).
3. P. Romero, et al., "Antigenicity and immunogenicity of Melan-A/MART-1 derived peptides as targets for tumor reactive CTL in human melanoma," Immunol. Rev. 188, 81 (2002).
4. V. H. Engelhard, et al., "Antigens derived from melanocyte differentiation proteins: self-tolerance, autoimmunity, and use for cancer immunotherapy," Immunol. Rev. 188, 136 (2002).
5. T. Wolfel, et al., "Two tyrosinase nonapeptides recognized on HLA-A2 melanomas by autologous cytolytic T lymphocytes," Eur. J. Immunol. 24(3), 759 (1994).
6. M. E. Dudley and S. A. Rosenberg, "Adoptive-cell-transfer therapy for the treatment of patients with cancer," Nat. Rev. Cancer 3(9), 666 (2003).
7. C. J. Cohen, et al., "Recombinant antibodies with MHC-restricted, peptide-specific, T-cell receptor-like specificity: new tools to study antigen presentation and TCR-peptide-MHC interactions," J. Mol. Recognit. 16(5), 324 (2003).
8. G. Denkberg, et al., "Selective targeting of melanoma and APCs using a recombinant antibody with TCR-like specificity directed toward a melanoma differentiation antigen," J. Immunol. 171(5), 2197 (2003).
9. A. Lev, et al., "Isolation and characterization of human recombinant antibodies endowed with the antigen-specific, major histocompatibility complex-restricted specificity of T cells directed toward the widely expressed tumor T-cell epitopes of the telomerase catalytic subunit," Cancer Res. 62(11), 3184 (2002).
10. C. J. Cohen, et al., "Direct phenotypic analysis of human MHC class I antigen presentation: visualization, quantitation, and in situ detection of human viral epitopes using peptide-specific, MHC-restricted human recombinant antibodies," J. Immunol. 170(8), 4349 (2003).
11. J. Golay, et al., "Effect of alemtuzumab on neoplastic B cells," Haematologica 89(12), 1476 (2004).
12. H. Mellstedt, "Monoclonal antibodies in human cancer," Drugs Today (Barc. ) 39 Suppl C, 1 (2003).
13. H. Modjtahedi, et al., "Targeting of cells expressing wild-type EGFR and type-III mutant EGFR (EGFRvIII) by anti-EGFR MAb ICR62: a two-pronged attack for tumour therapy," Int. J. Cancer 105(2), 273 (2003).
14. N. Prang, et al., "Cellular and complement-dependent cytotoxicity of Ep-CAM-specific monoclonal antibody MT201 against breast cancer cell lines," Br. J. Cancer 92(2), 342 (2005).
15. H. Takeuchi, et al., "Expression of differentiation melanoma-associated antigen genes is associated with favorable disease outcome in advanced-stage melanomas," Cancer Res. 63(2), 441 (2003).
16. S. Reinke, et al., "Differential expression of MART-1, tyrosinase, and SM5-1 in primary and metastatic melanoma," Am. J. Dermatopathol. 27(5), 401 (2005).
17. V. Brichard, et al., "The tyrosinase gene codes for an antigen recognized by autologous cytolytic T lymphocytes on HLA-A2 melanomas," J. Exp. Med. 178(2), 489 (1993).
18. J. C. Skipper, et al., "An HLA-A2-restricted tyrosinase antigen on melanoma cells results from posttranslational modification and suggests a novel pathway for processing of membrane proteins," J. Exp. Med. 183(2), 527 (1996).
19. C. A. Mosse, et al., "The class I antigen-processing pathway for the membrane protein tyrosinase involves translation in the endoplasmic reticulum and processing in the cytosol," J. Exp. Med. 187(1), 37 (1998).
20. K. Jimbow, et al., "Assembly, target-signaling and intracellular transport of tyrosinase gene family proteins in the initial stage of melanosome biogenesis," Pigment Cell Res. 13(4), 222 (2000).
21. V. H. Engelhard, A. G. Brickner, and A. L. Zarling, "Insights into antigen processing gained by direct analysis of the naturally processed class I MHC associated peptide repertoire," Mol. Immunol. 39(3-4), 127 (2002).
22. R. Halaban, et al., "Abnormal acidification of melanoma cells induces tyrosinase retention in the early secretory pathway," J. Biol. Chem. 277(17), 14821 (2002).
23. R Halaban, et al., "Proper folding and endoplasmic reticulum to golgi transport of tyrosinase are induced by its substrates, DOPA and tyrosine," J. Biol. Chem. 276(15), 11933 (2001).
24. G. Denkberg, et al., "Recombinant human single-chain MHC-peptide complexes made from E. coli By in vitro refolding: functional single-chain MHC-peptide complexes and tetramers with tumor associated antigens," Eur. J. Immunol. 30(12), 3522 (2000).
25. W. J. Storkus, et al., "Identification of T-cell epitopes: rapid isolation of class I-presented peptides from viable cells by mild acid elution," J. Immunother. 14(2), 94 (1993).
26. J. C. Skipper, et al., "Mass-spectrometric evaluation of HLA-A*0201-associated peptides identifies dominant naturally processed forms of CTL epitopes from MART-1 and gp100," Int. J. Cancer 82(5), 669 (1999).
27. R. Halaban, et al., "Aberrant retention of tyrosinase in the endoplasmic reticulum mediates accelerated degradation of the enzyme and contributes to the dedifferentiated phenotype of amelanotic melanoma cells," Proc. Natl. Acad. Sci. U. S. A 94(12), 6210 (1997).
28. D. Rimoldi, et al., "Subcellular localization of the melanoma-associated protein Melan-AMART-1 influences the processing of its HLA-A2-restricted epitope," J. Biol. Chem. 276(46), 43189 (2001).
29. Y. Kawakami, et al., "Identification of the immunodominant peptides of the MART-1 human melanoma antigen recognized by the majority of HLA-A2-restricted tumor infiltrating lymphocytes," J. Exp. Med. 180(1), 347 (1994).
30. Y. Kawakami, et al., "Recognition of shared melanoma antigens in association with major HLA-A alleles by tumor infiltrating T lymphocytes from 123 patients with melanoma," J. Immunother. 23(1), 17 (2000).
31. D. Valmori, et al., "Analysis of the cytolytic T lymphocyte response of melanoma patients to the naturally HLA-A*0201-associated tyrosinase peptide 368-376," Cancer Res. 59(16), 4050 (1999).
32. B. Rocha, A. Grandien, and A. A. Freitas, "Anergy and exhaustion are independent mechanisms of peripheral T cell tolerance," J. Exp. Med. 181(3), 993 (1995).
33. L. S. Taams, Eden W. van, and M. H. Wauben, "Dose-dependent induction of distinct anergic phenotypes: multiple levels of T cell anergy," J. Immunol. 162(4), 1974 (1999).
34. R. H. Schwartz, "T cell anergy," Annu. Rev. Immunol. 21, 305 (2003);
35. W. R. Heath, et al., "Cross-presentation, dendritic cell subsets, and the generation of immunity to cellular antigens," Immunol. Rev. 199, 9 (2004).
36. W. L. Redmond, B. C. Marincek, and L. A. Sherman, "Distinct requirements for deletion versus anergy during CD8 T cell peripheral tolerance in vivo," J. Immunol. 174(4), 2046 (2005).
37. J. F. Miller, et al., "Induction of peripheral CD8+ T-cell tolerance by cross-presentation of self antigens," Immunol. Rev. 165,267 (1998).
38. R. H. Schwartz, "T cell anergy," Annu. Rev. Immunol. 21, 305 (2003).
39. T. R. Mosmann and S. Sad, "The expanding universe of T-cell subsets: Th1, Th2 and more," Immunol. Today 17(3), 138 (1996).
40. F. Sallusto, et al., "Two subsets of memory T lymphocytes with distinct homing potentials and effector functions," Nature 401(6754), 708 (1999).
41. D. J. Irvine, et al., "Direct observation of ligand recognition by T cells," Nature 419(6909), 845 (2002).
42. A. Grakoui, et al., "The immunological synapse: a molecular machine controlling T cell activation," Science 285(5425), 221 (1999).
43. Baarle D. van, et al., "Significance of senescence for virus-specific memory T cell responses: rapid ageing during chronic stimulation of the immune system," Immunol. Lett. 97(1), 19 (2005).
44. J. Zhou, et al., "Selective growth, in vitro and in vivo, of individual T cell clones from tumor-infiltrating lymphocytes obtained from patients with melanoma," J. Immunol. 173(12), 7622 (2004).
45. A. Lev, et al., "Tumor-specific Ab-mediated targeting of MHC-peptide complexes induces regression of human tumor xenografts in vivo," Proc. Natl. Acad. Sci. U. S. A 101(24), 9051 (2004).
46. C. J. Cohen, et al., "Recombinant antibodies with MHC-restricted, peptide-specific, T-cell receptor-like specificity: new tools to study antigen presentation and TCR-peptide-MHC interactions," J. Mol. Recognit. 16(5), 324 (2003).
47. G. Denkberg, et al., "Selective targeting of melanoma and APCs using a recombinant antibody with TCR-like specificity directed toward a melanoma differentiation antigen," J. Immunol. 171(5), 2197 (2003).
48. T. R. Mempel, S. E. Henrickson, and U. H. Von Andrian, "T-cell priming by dendritic cells in lymph nodes occurs in three distinct phases," Nature 427(6970), 154 (2004).
49. S. C. Jameson, K. A. Hogquist, and M. J. Bevan, "Specificity and flexibility in thymic selection," Nature 369(6483), 750 (1994).
50. M. Krogsgaard, et al., "Agonist/endogenous peptide-MHC heterodimers drive T cell activation and sensitivity," Nature 434(7030), 238 (2005).
51. C. J. Fox, P. S. Hammerman, and C. B. Thompson, "Fuel feeds function: energy metabolism and the T-cell response," Nat. Rev. Immunol. 5(11), 844 (2005).
52. G. Dennis, Jr., et al., "DAVID: Database for Annotation, Visualization, and Integrated Discovery," Genome Biol. 4(5), 3 (2003).
53. D. A. Hosack, et al., "Identifying biological themes within lists of genes with EASE," Genome Biol. 4(10), R70 (2003).
54. F. Ramsdell and B. J. Fowlkes, "Maintenance of in vivo tolerance by persistence of antigen," Science 257(5073), 1130 (1992).

The invention furthermore comprises the following items:
1. An antibody comprising an antigen recognition domain capable of binding to an MHC-I molecule being complexed with a tyrosinase peptide comprising an amino acid sequence as set forth in SEQ ID NO: 1, wherein the antibody does not bind said MHC-I in the absence of said complexed peptide, and wherein the antibody does not bind said peptide in an absence of said MHC.
2. The antibody of item 1, comprising an antigen recognition domain which comprise complementarity determining region (CDR) amino acid sequences as set forth in SEQ ID NOs: 59-64.
3. The antibody of item 1, capable of binding to an MHC-I molecule being complexed with a tyrosinase peptide with a disassociation constant less than 100 nM.
4. The antibody of item 3, comprising an antigen recognition domain which comprises complementarity determining region (CDR) amino acid sequences as set forth in SEQ ID NOs: 886-891.
5. The antibody of item 3, comprising an antigen recognition domain which comprises complementarity determining region (CDR) amino acid sequences as set forth in SEQ ID NOs: 894-899.
6. The antibody of item 1 capable of binding to an MHC-I molecule being complexed with a tyrosinase peptide with a disassociation constant less than 10 nM.
7. The antibody of any of items 1-6, being an IgG1 antibody.
8. The antibody of any of items 1-6, being conjugated to a therapeutic moiety.
9. The antibody of item 8, wherein said therapeutic moiety is selected from the group consisting of a cytotoxic moiety, a toxic moiety, a cytokine moiety and a bi-specific antibody moiety.
10. The antibody of item9, wherein said toxic moiety is PE38KDEL.
11. The antibody of any of items1-6, attached to a detectable moiety.
12. The antibody of item 11, wherein said detectable moiety is a fluorescent protein or an enzyme.
13. The antibody of any of items1-6, being an antibody fragment.
14. The antibody of any of items 1-6, wherein said antibody fragment is selected from the group consisting of an Fab fragment, an F(ab')₂ fragment and a single chain Fv fragment.
15. A pharmaceutical composition comprising the antibody of any of items1-10, 13, and 14.
16. A method of detecting a melanoma cell, comprising contacting the cell with an antibody comprising an antigen recognition domain capable of binding to an MHC-I molecule being complexed with a tyrosinase peptide, wherein the antibody does not bind said MHC-I in the absence of said complexed peptide, and wherein the antibody does not bind said peptide in an absence of said MHC, under conditions which allow immunocomplex formation, wherein a presence of said immunocomplex or level thereof is indicative of the melanoma cell.
17. A method of diagnosing a melanoma in a subject in need thereof, comprising contacting a cell of the subject with an antibody comprising an antigen recognition domain capable of binding to an MHC-I molecule being complexed with a tyrosinase peptide, wherein the antibody does not bind said MHC-I in the absence of said complexed peptide, and wherein the antibody does not bind said peptide in an absence of said MHC, under conditions which allow immunocomplex formation, wherein a presence of said immunocomplex or level thereof is indicative of the melanoma.
18. The method of item17, wherein said cell is a skin cell.
19. The method of item 16 or 17, wherein said tyrosinase peptide comprises an amino acid sequence as set forth in SEQ ID NO: 1.
20. The method of item19, wherein said antibody comprises complementarity determining region (CDR) amino acid sequences as set forth in SEQ ID NOs: 59-64.
21. The method of item 19, wherein said antibody comprises complementarity determining region (CDR) amino acid sequences as set forth in SEQ ID NOs: 886-891.
22. The method of item19, wherein said antibody comprises complementarity determining region (CDR) amino acid sequences as set forth in SEQ ID NOs: 894-899.
23. The method of item 19, wherein said antibody comprises an antigen recognition domain capable of binding to an MHC-I molecule being complexed with a tyrosinase peptide with a disassociation constant less than 100 nM, wherein said antibody does not bind said MHC-I in the absence of said complexed peptide, and wherein said antibody does not bind said peptide in an absence of said MHC.
24. The method of item 16 or 17, wherein said antibody is attached to a detectable moiety.
25. The method of item 24, wherein said detectable moiety is a fluorescent protein or an enzyme.
26. A method of identifying if a subject is suitable for TCRL-based epitope directed therapy, comprising determining a level of epitope presentation on at least one cell of the subject using an antibody comprising an antigen recognition domain capable of binding to an MHC-I molecule being complexed with a peptide fragment of said antigen, wherein the antibody does not bind said MHC-I in the absence of said complexed peptide fragment of said antigen, and wherein the antibody does not bind said peptide fragment of said antigen in an absence of said MHC, wherein a level value higher than a predetermined threshold is indicative of an individual being suitable for TCRL-based epitope directed therapy.
27. The method of item 26, wherein a level value below a predetermined threshold is indicative of an individual not being suitable for TCRL-based epitope directed therapy.
28. A method of identifying if a subject is suitable for CTL-based epitope directed therapy, comprising determining a level of epitope presentation on at least one cell of the subject using an antibody comprising an antigen recognition domain capable of binding to an MHC-I molecule being complexed with a peptide fragment of said antigen, wherein the antibody does not bind said MHC-I in the absence of said complexed peptide fragment of said antigen, and wherein the antibody does not bind said peptide fragment of said antigen in an absence of said MHC, wherein a level value lower than a predetermined threshold is indicative of an individual being suitable for CTL-based epitope directed therapy.
29. A method of treating a melanoma, comprising administering to a subject in need thereof a therapeutically effective amount of an antibody comprising an antigen recognition domain capable of binding to an MHC-I molecule being complexed with a tyrosinase peptide, wherein said antibody does not bind said MHC-I in the absence of said complexed tyrosinase peptide, and wherein said antibody does not bind said tyrosinase peptide in an absence of said MHC, thereby treating the melanoma.
30. The method of item 29, wherein said tyrosinase peptide comprises an amino acid sequence as set forth in SEQ ID NO: 1.
31. The method of item 29, wherein said antibody comprises complementarity determining region (CDR) amino acid sequences as set forth in SEQ ID NOs: 59-64.
32. The method of item 29, wherein said antibody comprises complementarity determining region (CDR) amino acid sequences as set forth in SEQ ID NOs: 886-891.
33. The method of item 29, wherein said antibody comprises complementarity determining region (CDR) amino acid sequences as set forth in SEQ ID NOs: 894-899.
34. The method of item 29, wherein said antibody comprises an antigen recognition domain capable of binding to an MHC-I molecule being complexed with a tyrosinase peptide with a disassociation constant less than 100 nM, wherein said antibody does not bind said MHC-I in the absence of said complexed peptide, and wherein said antibody does not bind said peptide in an absence of said MHC.
35. The method of item 30, wherein said antibody is conjugated to a therapeutic moiety.
36. The method of item 35, wherein said therapeutic moiety is selected from the group consisting of a cytotoxic moiety, a toxic moiety, a cytokine moiety and a bi-specific antibody moiety.
37. The method of i tem36, wherein said toxic moiety is PE38KDEL.
38. A method of killing or ablating a cell displaying a tyrosinase peptide on a surface MHC molecule, the method comprising contacting the target cell with an antibody comprising an antigen recognition domain capable of binding to an MHC-I molecule being complexed with a tyrosinase peptide, wherein said antibody does not bind said MHC-I in the absence of said complexed tyrosinase peptide, and wherein said antibody does not bind said tyrosinase peptide in an absence of said MHC, thereby killing or ablating the cell.
39. The method of item 38, wherein said tyrosinase peptide comprises an amino acid sequence as set forth in SEQ ID NO: 1.
40. The method of item 38, wherein said antibody comprises
   complementarity determining region (CDR) amino acid sequences as set forth in SEQ ID NOs: 59-64.
41. The method of item 38, wherein said antibody comprises complementarity determining region (CDR) amino acid sequences as set forth in SEQ ID NOs: 886-891.
42. The method of item 38, wherein said antibody comprises complementarity determining region (CDR) amino acid sequences as set forth in SEQ ID NOs: 894-899.
43. The method of item 38, wherein said antibody comprises an antigen recognition domain capable of binding to an MHC-I molecule being complexed with a tyrosinase peptide with a disassociation constant less than 100 nM, wherein said antibody does not bind said MHC-I in the absence of said complexed peptide, and wherein said antibody does not bind said peptide in an absence of said MHC.
44. The method of item 38, wherein said antibody is conjugated to a therapeutic moiety.
45. The method of i t em 44, wherein said therapeutic moiety is selected from the group consisting of a cytotoxic moiety, a toxic moiety, a cytokine moiety and a bi-specific antibody moiety.
46. The method of item 45, wherein said toxic moiety is PE38KDEL.
47. An antibody comprising an antigen recognition domain capable of binding to an MHC-I molecule being complexed with a MART-1 peptide comprising an amino acid sequence as set forth in SEQ ID NO: 21, wherein the antibody does not bind said MHC-I in the absence of said complexed peptide, and wherein the antibody does not bind said peptide in an absence of said MHC.
48. The antibody of item 47, comprising an antigen recognition domain which comprise complementarity determining region (CDR) amino acid sequences as set forth in SEQ ID NOs: 47-52.
49. The antibody of i t em 47, comprising an antigen recognition domain which comprises complementarity determining region (CDR) amino acid sequences as set forth in
   SEQ ID NOs: 53-58.

## Claims

1. A method of identifying if a subject is suitable for TCRL-based epitope directed therapy, comprising determining a level of epitope presentation on at least one cell of the subject using an antibody comprising an antigen recognition domain capable of binding to an MHC-I molecule being complexed with a peptide fragment of said antigen, wherein the antibody does not bind said MHC-I in the absence of said complexed peptide fragment of said antigen, and wherein the antibody does not bind said peptide fragment of said antigen in an absence of said MHC, wherein a level value higher than a predetermined threshold is indicative of an individual being suitable for TCRL-based epitope directed therapy.

2. The method of claim 1, wherein a level value below a predetermined threshold is indicative of an individual not being suitable for TCRL-based epitope directed therapy.

3. A method of identifying if a subject is suitable for CTL-based epitope directed therapy, comprising determining a level of epitope presentation on at least one cell of the subject using an antibody comprising an antigen recognition domain capable of binding to an MHC-I molecule being complexed with a peptide fragment of said antigen, wherein the antibody does not bind said MHC-I in the absence of said complexed peptide fragment of said antigen, and wherein the antibody does not bind said peptide fragment of said antigen in an absence of said MHC, wherein a level value lower than a predetermined threshold is indicative of an individual being suitable for CTL-based epitope directed therapy.

4. The method of claim 1, wherein said peptide is a tyrosinase peptide.

5. The method of claim 4, wherein said tyrosinase peptide is as set forth in SEQ ID NO: 1.

6. The method of claim 3, wherein said peptide is a tyrosinase peptide.

7. The method of claim 6, wherein said tyrosinase peptide is as set forth in SEQ ID NO: 1.

8. The method of claim 1, wherein said subject has a melanoma.

9. The method of claim 1, wherein said subject has a melanoma.

10. The method of claim 8 or 9, wherein said MHC class I molecule is HLA-A2 and whereas said peptide fragment is as set forth in SEQ ID NO: 1.
